(19) Europäisches Patentamt / European Patent Office / Office européen des brevets

(11) EP 3 390 384 B1

(12) **EUROPEAN PATENT SPECIFICATION**

(45) Date of publication and mention of the grant of the patent:
**15.09.2021 Bulletin 2021/37**

(21) Application number: **16815960.6**

(22) Date of filing: **15.12.2016**

(51) Int Cl.:
*C07D 401/14* (2006.01) *C07D 413/14* (2006.01)
*C07D 215/20* (2006.01) *C07D 215/54* (2006.01)
*C07D 401/04* (2006.01) *C07D 401/12* (2006.01)
*C07D 405/12* (2006.01) *C07D 413/12* (2006.01)
*C07D 417/12* (2006.01) *C07D 491/048* (2006.01)
*A61K 31/4709* (2006.01) *A61P 21/00* (2006.01)

(86) International application number:
**PCT/IB2016/057676**

(87) International publication number:
**WO 2017/103851 (22.06.2017 Gazette 2017/25)**

(54) **QUINOLINE-3-CARBOXAMIDES AS H-PGDS INHIBITORS**

CHINOLIN-3-5-CARBOXAMIDE ALS H-PGDS-INHIBITOREN

QUINOLÉINE-3-CARBOXAMIDES UTILISÉS COMME INHIBITEURS DE H-PGDS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.12.2015 US 201562268630 P**

(43) Date of publication of application:
**24.10.2018 Bulletin 2018/43**

(73) Proprietors:
- **Astex Therapeutics Limited**
  **Cambridge Cambridgeshire CB4 0QA (GB)**
- **GlaxoSmithKline Intellectual Property Development Limited**
  **Brentford**
  **Middlesex TW8 9GS (GB)**

(72) Inventors:
- **CADILLA, Rodolfo**
  **Research Triangle Park**
  **North Carolina 27709 (US)**
- **DEATON, Dave Norman**
  **King of Prussia**
  **Pennsylvania 19406 (US)**
- **HANCOCK, Ashley Paul**
  **Stevenage**
  **Hertfordshire SG1 2NY (GB)**
- **HOBBS, Heather**
  **Stevenage**
  **Hertfordshire SG1 2NY (GB)**
- **HODGSON, Simon Teanby**
  **Stevenage**
  **Hertfordshire SG1 2NY (GB)**
- **LARKIN, Andrew L.**
  **King of Prussia**
  **Pennsylvania 19406 (US)**
- **LE, Joelle**
  **Stevenage**
  **Hertfordshire SG1 2NY (GB)**
- **MORTENSON, Paul N.**
  **Cambridge**
  **Cambridgeshire CB4 0QA (GB)**
- **PRICE, Daniel J.**
  **Waltham**
  **Massachusetts 02451 (US)**
- **SAXTY, Gordon**
  **Cambridge**
  **Cambridgeshire CB4 0QA (GB)**
- **SCHALLER, Lee T.**
  **Research Triangle Park**
  **North Carolina 27709 (US)**
- **SCHULTE, Christie**
  **King of Prussia**
  **Pennsylvania 19406 (US)**
- **SMITH, Ian Edward David**
  **Stevenage**
  **Hertfordshire SG1 2NY (GB)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **THOMSON, Stephen Andrew**
  **Del Mar**
  **California 92014 (US)**
- **WILSON, Joseph Wendell**
  **Hillsborough**
  **North Carolina 27278 (US)**

(74) Representative: **Ahern, Jenna Marie**
**GlaxoSmithKline**
**Global Patents, CN925.1**
**980 Great West Road**
**Brentford, Middlesex TW8 9GS (GB)**



(56) References cited:
**WO-A1-00/73283** **WO-A1-2009/076631**
**WO-A1-2009/153721** **WO-A2-2014/012050**

- **AMIR HANNA-ELIAS ET AL: "Synthesis of Quinoline Derivatives as 5-HT 4 Receptor Ligands", AUSTRALIAN JOURNAL OF CHEMISTRY: AN INTERNATIONAL JOURNAL FOR CHEMICAL SCIENCE, vol. 62, no. 2, 1 January 2009 (2009-01-01), page 150, XP0055337093, AU ISSN: 0004-9425, DOI: 10.1071/CH08505**

## Description

## FIELD OF THE INVENTION

The present invention relates to novel compounds, to the use of the compounds as Hematopoietic Prostaglandin D Synthase (H-PGDS) inhibitors, to pharmaceutical compositions comprising the compounds and to the use of the compounds in therapy, especially in the treatment of conditions for which a H-PGDS inhibitor is indicated, such as neurodegenerative diseases and musculoskeletal diseases including Duchenne Muscular Dystrophy, where $PGD_2$ is considered to play a pathological role, for the use of a compound in the manufacture of a medicament for the treatment of conditions in which an inhibitor of H-PGDS is indicated, and a method for the treatment of disorders in which inhibition of H-PGDS is indicated, in a human.

## BACKGROUND OF THE INVENTION

Prostaglandin $D_2$ ($PGD_2$) is a product of arachidonic acid metabolism, and is the major prostanoid mediator synthesised by mast cells in response to stimulation via multiple mechanisms and cellular activation pathways, including allergen-mediated crosslinking of high affinity IgE receptors (Lewis et al. (1982) Prostaglandin D2 generation after activation of rat and human mast cells with anti-IgE. J Immunol, 129, 1627-1631). Other cells such as dendritic cells, $T_h2$ cells, and epithelial cells also produce $PGD_2$, but at lower levels than mast cells. $PGD_2$ mediates its effects via activation of the specific G-protein coupled receptors $DP_1$ (Boie et al (1995) Molecular cloning and characterization of the human prostanoid DP receptor. (J Biol Chem, 270, 18910-18916) and $DP_2$ (CRTH2)(Abe et al (1999), Molecular cloning, chromosome mapping and characterization of the mouse CRTH2 gene, a putative member of the leukocyte chemo-attractant receptor family. (Gene, 227, 71-77) and also acts via the receptor for thromboxane $A_2$ ($TXA_2$), the TP receptor, on target cells.

Prostaglandin D synthase (PGDS) is the enzyme responsible for the catalytic isomerase conversion of prostaglandin endoperoxide $PGH_2$ to $PGD_2$. $PGD_2$ is generated by the action of either H-PGDS (hematopoietic-type or H-type) or L-PGDS or (lipocalin-type or L-type) enzymes (Urade et al., (2000) Prostaglandin D synthase structure and function. Vitamins and hormones, 58, 89-120). H-PGDS activity is dependent on glutathione and plays an important role in the generation of $PGD_2$ by immune and inflammatory cells, including mast cells, antigen-presenting cells (e.g. dendritic cells), macrophages, and $T_h2$ cells, which are all key cells in the pathology of allergic disease. In contrast, L-type is glutathione-independent and is primarily located in the central nervous system, genital organs, and heart. These two isoforms of PGDS appear to have distinct catalytic properties, tertiary structure, and cellular and tissue distribution.

Using the inhibitor HQL-79, H-PGDS has also been implicated to play a role not only in allergic disease, but also other diseases such as Duchenne Muscular Dystrophy (Nakagawa et al. (2013) A prostaglandin D2 metabolite is elevated in the urine of Duchenne muscular dystrophy subjects and increases further from 8 years old, Clinica Chimica Acta 423, 10-14) and (Mohri et al. (2009), Inhibition of prostaglandin D synthase suppresses muscular necrosis, Am J Pathol 174, 1735-1744) and (Okinaga et al. (2002), Induction of hematopoietic prostaglandin D synthase in hyalinated necrotic muscle fibers: its implication in grouped necrosis, Acta Neuropathologica 104, 377-84), spinal cord contusion injury (Redensek et al. (2011) Expression and detrimental role of hematopoietic prostaglandin D synthase in spinal cord contusion injury, Glia 59, 603-614), neuroinflammation (Mohri et al. (2006) Prostaglandin D2-mediated microglia/astrocyte interaction enhances astrogliosis and demyelination in twitcher. J Neurosci 26, 4383-4393), and neurodegenerative disease (Ikuko et al. (2007) Hematopoietic prostaglandin D synthase and DP1 receptor are selectively upregulated in microglia and astrocytes within senile plaques from human subjects and in a mouse model of Alzheimer disease. J Neuropath Exp Neur 66, 469-480). H-PGDS has also been implicated to play a role in metabolic diseases such as diabetes and obesity, since $PGD_2$ is converted to 15-deoxy-$\Delta^{12,14}PGJ_2$, a potent ligand for PPARγ which is able to drive adipogenesis (Tanaka et al (2011) Mast cells function as an alternative modulator of adipogenesis through 15-deoxy-delta-12, 14-prostaglandin J2. Am J Physiol Cell Physiol 301, C1360-C1367). $PGD_2$ has been implicated to play a role in niacin-induced skin flushing (Papaliodis et al (2008) Niacin-induced "flush" involves release of prostaglandin D2 from mast cells and serotonin from platelets: Evidence from human cells in vitro and an animal model. JPET 327:665-672**).**

Weber et al. (2010), Identification and characterisation of new inhibitors for the human hematopoietic prostaglandin D2 synthase. European Journal of Medicinal Chemistry 45, 447-454, Carron et al. (2010), Discovery of an Oral Potent Selective Inhibitor of Hematopoietic Prostaglandin D Synthase (H-PGDS). ACS Med Chem Lett. 1, 59-63; Christ et al. (2010), Development and Characterization of New Inhibitors of the Human and Mouse Hematopoietic Prostaglandin D2 Synthases, J Med Chem , 53, 5536-5548; and Hohwy et al. (2008), Novel Prostaglandin D Synthase Inhibitors Generated by Fragment-Based Drug Design. J Med Chem, 51, 2178-2186 are also of interest.

Based on this evidence, chemical inhibitors of H-PGDS which inhibit $PGD_2$ formation, simultaneously inhibit the biological actions of $PGD_2$ and its metabolites at multiple receptors and offer the potential for therapeutic benefit in the treatment of a range of diseases where $PGD_2$ is considered to play a pathological role.

International Patent Applications WO2005/094805, WO2007/007778, WO2007/041634, 2008/121670, WO2008/122787, WO2009/153720, WO2009/153721, WO2010/033977, WO2010/104024, WO2011/043359, WO2011044307, WO2011/090062, Japanese Patent Application 2007-51121 and US Patent Application 2008/0146569 disclose certain H-PGDS inhibitors and their use in the treatment of diseases associated with the activity of H-PGDS.

It is an object of the invention to provide further H-PGDS inhibitors, suitably for the treatment of Muscular Dystrophy.

## SUMMARY OF THE INVENTION

In a first aspect, the invention is directed to compounds according to Formula XI:

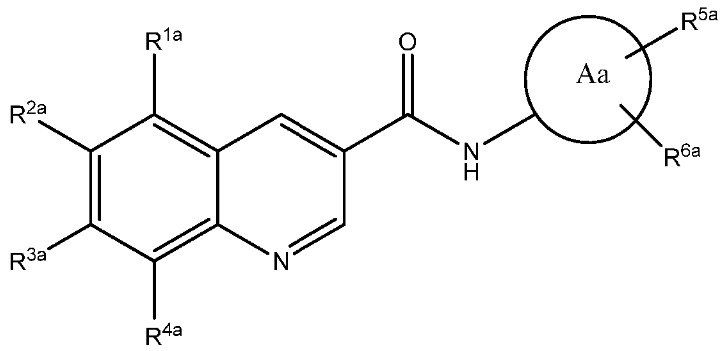

wherein $R^{1a}$, $R^{2a}$, $R^{3a}$, $R^{4a}$, $R^{5a}$, $R^{6a}$, and Aa are as defined in claim 1.

Compounds of Formula (XI) as defined in claim 1 and their pharmaceutically acceptable salts have H-PGDS activity and are believed to be of potential use for the treatment or prophylaxis of certain disorders.

Accordingly, in another aspect of the invention there is provided a pharmaceutical composition comprising a compound of Formula (XI) according to the first aspect, or a pharmaceutically acceptable salt thereof and one or more pharmaceutically acceptable carriers or excipients.

In a further aspect, the invention provides a compound of Formula (XI) or a pharmaceutically acceptable salt thereof according to the first aspect of the invention for use in therapy.

This invention also relates to a method of treating Duchenne muscular dystrophy, which comprises administering to a subject in need thereof an effective amount of a H-PGDS inhibiting compound of Formula (XI).

This invention also relates to a method of treating congenital myotonia, which comprises administering to a subject in need thereof an effective amount of a H-PGDS inhibiting compound of Formula (XI).

This invention also relates to a method of treating muscle injury, which comprises administering to a subject in need thereof an effective amount of a H-PGDS inhibiting compound of Formula (XI).

This invention also relates to a method of treating muscle lacerations, which comprises administering to a subject in need thereof an effective amount of a H-PGDS inhibiting compound of Formula (XI).

This invention also relates to a method of treating chronic muscle strains, which comprises administering to a subject in need thereof an effective amount of a H-PGDS inhibiting compound of Formula (XI).

This invention also relates to a method of treating Myotonic dystrophy type I, which comprises administering to a subject in need thereof an effective amount of a H-PGDS inhibiting compound of Formula (XI).

This invention also relates to a method of treating myotonic dystrophy type II, which comprises administering to a subject in need thereof an effective amount of a H-PGDS inhibiting compound of Formula (XI).

This invention also relates to a method of treating asthma, which comprises administering to a subject in need thereof an effective amount of a H-PGDS inhibiting compound of Formula (XI).

This invention also relates to a method of treating chronic obstructive pulmonary disease, which comprises administering to a subject in need thereof an effective amount of a H-PGDS inhibiting compound of Formula (XI).

This invention also relates to a method of treating rheumatoid arthritis, which comprises administering to a subject in need thereof an effective amount of a H-PGDS inhibiting compound of Formula (XI).

This invention also relates to a method of treating inflammatory bowel disease, which comprises administering to a subject in need thereof an effective amount of a H-PGDS inhibiting compound of Formula (XI).

This invention also relates to a method of treating osteoarthritis, which comprises administering to a subject in need thereof an effective amount of a H-PGDS inhibiting compound of Formula (XI).

This invention also relates to a method of treating psoriasis, which comprises administering to a subject in need thereof an effective amount of a H-PGDS inhibiting compound of Formula (XI).

This invention also relates to a method of treating a muscle degenerative disorder, which comprises administering to a subject in need thereof an effective amount of a H-PGDS inhibiting compound of Formula (XI).

This invention also relates to a method of treating muscular dystrophy, which comprises administering to a subject in need thereof an effective amount of a H-PGDS inhibiting compound of Formula (XI).

Also included in the present invention are methods of co-administering the presently invented H-PGDS inhibiting compounds with further active ingredients.

The invention also relates to a compound of Formula (XI) or a pharmaceutically acceptable salt thereof for use in the treatment of Duchenne muscular dystrophy.

The invention also relates to a compound of Formula (XI) or a pharmaceutically acceptable salt thereof for use in the treatment of congenital myotonia.

The invention also relates to a compound of Formula (XI) or a pharmaceutically acceptable salt thereof for use in the treatment of muscle injury.

The invention also relates to a compound of Formula (XI) or a pharmaceutically acceptable salt thereof for use in the treatment of muscle lacerations.

The invention also relates to a compound of Formula (XI) or a pharmaceutically acceptable salt thereof for use in the treatment of chronic muscle strains.

The invention also relates to a compound of Formula (XI) or a pharmaceutically acceptable salt thereof for use in the treatment of Myotonic dystrophy type I.

The invention also relates to a compound of Formula (XI) or a pharmaceutically acceptable salt thereof for use in the treatment of myotonic dystrophy type II.

The invention also relates to a compound of Formula (XI) or a pharmaceutically acceptable salt thereof for use in the treatment of asthma.

The invention also relates to a compound of Formula (XI) or a pharmaceutically acceptable salt thereof for use in the treatment of chronic obstructive pulmonary disease.

The invention also relates to a compound of Formula (XI) or a pharmaceutically acceptable salt thereof for use in the treatment of rheumatoid arthritis.

The invention also relates to a compound of Formula (XI) or a pharmaceutically acceptable salt thereof for use in the treatment of inflammatory bowel disease.

The invention also relates to a compound of Formula (XI) or a pharmaceutically acceptable salt thereof for use in the treatment of osteoarthritis.

The invention also relates to a compound of Formula (XI) or a pharmaceutically acceptable salt thereof for use in the treatment of psoriasis.

The invention also relates to a compound of Formula (XI) or a pharmaceutically acceptable salt thereof for use in the treatment of a muscle degenerative disorder.

The invention also relates to a compound of Formula (XI) or a pharmaceutically acceptable salt thereof for use in the treatment of muscular dystrophy.

A compound of Formula (XI) may be prepared by methods described herein.

## BRIEF DESCRIPTION OF THE FIGURES

**Figure 1.** Figure 1 depicts the protection and acceleration of functional repair dose response curves of PGDS Inhibition using the compound of Example 6 following limb muscle injury in normal mice.

**Figure 2.** Figure 2 depicts the protection and acceleration of functional repair dose response curves of PGDS Inhibition using the compound of Example 48 following limb muscle injury in *mdx* mice.

## DETAILED DESCRIPTION OF THE INVENTION

This invention is defined in the amended claims. Certain embodiments for illustrative purpose are disclosed in the following.

One such illustrative embodiment relates to a compound of formula (XI):

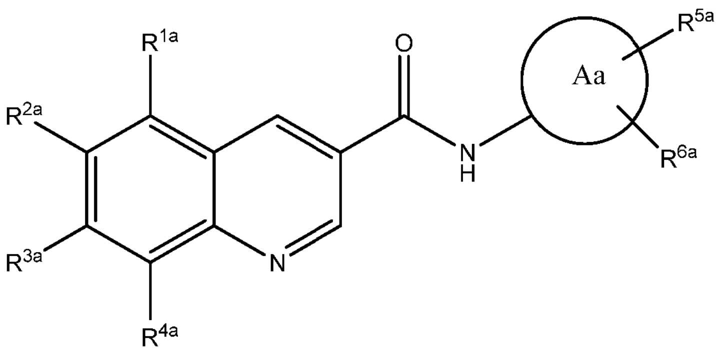

(XI)

wherein:

$R^{1a}$ is selected from:

H,
F,
Cl,
-OH,
$-OCH_3$, and
$C_1$alkoxy substituted 1 to 3 times with fluoro;

$R^{2a}$ is selected from:

H,
F,
Cl,
Br,
I,
-OH,
$-C(O)OC(CH_3)_3$,
-COOH,
$-C(O)C_{1-4}$alkyl,
$-C(O)C_{1-4}$alkyl substituted with from 1 to 5 substituents independently selected from: fluoro, chloro, bromo, oxo, -OH and -CN,
$-SC_{1-4}$alkyl,
$-SC_{1-4}$alkyl, substituted with from 1 to 5 substituents independently selected from: fluoro, chloro, bromo, oxo, -OH and -CN,
$-S(O)C_{1-4}$alkyl,
$-S(O)C_{1-4}$alkyl substituted with from 1 to 5 substituents independently selected from: fluoro, chloro, bromo, oxo, -OH and -CN,
$-N_3$,
-C=N,
$C_{1-4}$alkoxy,
$C_{1-4}$alkoxy substituted with from 1 to 5 substituents independently selected from: fluoro, chloro, bromo, oxo, -OH and -CN,
$C_{1-6}$alkyl,
$C_{1-6}$alkyl substituted with from 1 to 5 substituents Independently selected from: fluoro, chloro, bromo, iodo, oxo, $C_{1-4}$alkoxy, -OH, -COOH, $-NH_2$, $-N(H)C_{1-4}$alkyl, $-N(C_{1-4}alkyl)_2$ and -CN,
cyclopropyl,
cyclobutyl,
2,2-difluorocyclopropyl,
pyrrolidinyl,
azetidinyl, and
azetidinyl substituted with one or two substituents independently selected from halogen and methyl;

$R^{3a}$ is selected from:

H,
F,
Cl,
Br,
I,
-OH,
$-C(O)OC(CH_3)_3$,
-COOH,
$-C(O)C_{1-4}$alkyl,
$-C(O)C_{1-4}$alkyl substituted with from 1 to 5 substituents independently selected from: fluoro, chloro, bromo, oxo, -OH and -CN,
$-SC_{1-4}$alkyl,
$-SC_{1-4}$alkyl, substituted with from 1 to 5 substituents independently selected from: fluoro, chloro, bromo, oxo, -OH and -CN,
$-S(O)C_{1-4}$alkyl,
$-S(O)C_{1-4}$alkyl substituted with from 1 to 5 substituents independently selected from: fluoro, chloro, bromo, oxo, -OH and -CN,
$-NH_2$,
$-N(H)C_{1-3}$alkyl,
$-N(C_{1-3}alkyl)_2$,
-N(H)cyclopropyl,
$-N_3$,
-C=N,
$C_{1-4}$alkoxy,
$C_{1-4}$alkoxy substituted with from 1 to 5 substituents independently selected from: fluoro, chloro, bromo, oxo, -OH and -CN,
$C_{1-6}$alkyl,
$C_{1-6}$alkyl substituted with from 1 to 5 substituents Independently selected from: fluoro, chloro, bromo, iodo, oxo, $C_{1-4}$alkoxy, -OH, -COOH, $-NH_2$, $-N(H)C_{1-4}$alkyl, $-N(C_{1-4}alkyl)_2$ and -CN,
azido,
cyclopropyl,
cyclobutyl,
2,2-difluorocyclopropyl,
pyrrolidinyl,
azetidinyl, and
azetidinyl substituted with one or two substituents independently selected from F, Cl, Br, I and methyl;

$R^{4a}$ is selected from:

H,
F,
Cl,
-OH,
-C=N,
$C_{1-4}$alkoxy,
$C_{1-4}$alkoxy substituted from 1 to 5 times by fluoro,
$C_{1-3}$alkyl, and
$C_{1-3}$alkyl substituted from 1 to 5 times by fluoro,
where:
at least one substituent selected from $R^{1a}$, $R^{2a}$, $R^{3a}$, and $R^{4a}$ is not H;

or $R^{1a}$ and $R^{4a}$ are H and $R^{2a}$ and $R^{3a}$ join to form a 1,4-dioxanyl ring or a 1,3-dioxolanyl ring;

or $R^{1a}$ and $R^{2a}$ are H and $R^{3a}$ and $R^{4a}$ join to form a tetrahydrofuranyl ring;

Aa is selected from:

$C_{4-7}$cycloalkyl,
a 4-, 5-, or 6-membered heterocycloalkyl containing one or two heteroatoms independently selected from O and N,
and
a 5-12 membered heteroaryl containing one or two heteroatoms, wherein at least one heteroatom is nitrogen and the second heteroatom, if present, is selected from N and S;

$R^{5a}$ and $R^{6a}$ are independently selected from:

hydrogen,
$-OS(O)_2NH_2$,
$-S(O)_2CH_3$,
-OH,
-C=N,
F,
Cl,
Br,
I,
tetrazolyl,
methyl-tetrazolyl,
ethyl-tetrazolyl,
cycloalkyl,
cyclopropyl substituted with one or two substituents independently selected from; -OH, $-OCH_3$, and $-CH_3$,
morpholinyl,
azetidinyl,
azetidinyl substituted with one or two substituents independently selected from: fluoro, chloro, bromo, iodo, -OH, $-CF_3$, and $-CH_3$, pyridinyl,
pyridinyl substituted with -C=N,
oxazolyl,
oxazolyl substituted with $-C(O)OCH_2CH_3$,
oxazolyl substituted with -C=N,
-N(H)oxazolyl,
-N(H)oxazolyl substituted with $-C(O)OCH_2CH_3$,
-N(H)oxazolyl substituted with -C=N,
$-N(H)S(O)_2CH_3$,
oxo,
$C_{1-8}$alkyl,
$C_{1-8}$alkyl substituted with from one to six substituents independently selected from: -OH, oxo, fluoro, chloro, bromo, iodo, $C_{1-4}$alkoxy, cycloalkyl, morpholinyl, methylpiperazinyl, $-NH_2$, $-N(H)C_{1-4}$alkyl, $-N(H)C_{1-4}$alkyl where alkyl is subitituted with from 1 to 5 fluoro, $-N(C_{1-4}alkyl)_2$, and $-N(C_{1-4}alkyl)_2$ where the alkyls are independently subitituted with from 1 to 7 fluoro,
$C_{1-8}$alkoxy,
$C_{1-8}$alkoxy subsitituted with from one to six substituents independently selected from: -OH, oxo, fluoro, chloro, bromo, iodo, $C_{1-4}$alkoxy, cycloalkyl, $-NH_2$, $-N(H)C_{1-4}$alkyl, $-N(H)C_{1-4}$alkyl where the alkyl is subitituted with from 1 to 5 fluoro, $-N(C_{1-4}alkyl)_2$, $-N(C_{1-4}alkyl)_2$ where the alkyls are independently subitituted with from 1 to 7 fluoro, $-S(O)_2CH_3$, $-S(O)_2NH_2$, and $-S(O)_2N(H)C_{1-4}$alkyl,
dimethylamine oxide,
$N(C_{1-6}alkyl)_2$, where each alkyl is optionally subsitituted with from one to six substituents independently selected from: -OH, oxo, fluoro, chloro, bromo, iodo, and $-S(O)_2CH_3$,
$N(H)C_{1-6}$alkyl,
$N(H)C_{1-6}$alkyl subsitituted with from one to six substituents independently selected from: -OH, oxo, fluoro, chloro, bromo, iodo, and $-S(O)_2CH_3$;
and salts thereof.

This disclosure also relates to pharmaceutically acceptable salts of the compounds of Formula (XI).

Included in the present disclosure are compounds of Formula (XII):

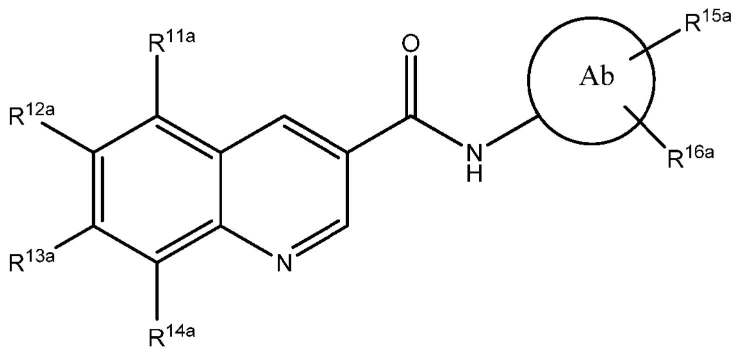

(XII)

wherein:

$R^{11a}$ is selected from:

H, F, Cl,
-OH, and
$-OCH_3$;

$R^{12a}$ is selected from:

H,
$C_{1-6}$alkyl,
F,
Cl,
Br,
-C=N, and
$C_{1-4}$alkoxy;

$R^{13a}$ is selected from:

H,
F,
Cl,
Br,
I,
-OH,
$-C(O)OC(CH_3)_3$,
-COOH,
$-C(O)C_{1-4}$alkyl,
$-C(O)C_{1-4}$alkyl substituted with from 1 to 5 substituents independently selected from: fluoro, chloro, bromo, oxo, -OH and -CN,
$-SC_{1-4}$alkyl,
$-SC_{1-4}$alkyl, substituted with from 1 to 5 substituents independently selected from: fluoro, chloro, bromo, oxo, -OH and -CN,
$-S(O)C_{1-4}$alkyl,
$-S(O)C_{1-4}$alkyl substituted with from 1 to 5 substituents independently selected from: fluoro, chloro, bromo, oxo, -OH and -CN,
$-NH_2$,
$-N(H)C_{1-3}$alkyl,
$-N(C_{1-3}alkyl)_2$,
-N(H)cyclopropyl,
$-N_3$,
-C=N,
$C_{1-4}$alkoxy,
$C_{1-4}$alkoxy substituted with from 1 to 5 substituents independently selected from: fluoro, chloro, bromo, oxo,

-OH and -CN,
$C_{1-6}$alkyl,
$C_{1-6}$alkyl substituted with from 1 to 5 substituents Independently selected from: fluoro, chloro, bromo, iodo, oxo, $C_{1-4}$alkoxy, -OH, -COOH, $-NH_2$, $-N(H)C_{1-4}$alkyl, $-N(C_{1-4}alkyl)_2$ and -CN,
cyclopropyl,
cyclobutyl,
2,2-difluorocyclopropyl,
pyrrolidinyl,
azetidinyl, and
azetidinyl substituted with one or two substituents independently selected from F, Cl, Br, I, and methyl;

$R^{14a}$ is selected from:

H,
F,
Cl,
-OH,
-C=N,
$C_{1-4}$alkoxy, and
$C_{1-3}$alkyl;
where:
at least one substituent selected from $R^{11a}$, $R^{12a}$, $R^{13a}$, and $R^{14a}$ is not H;

or $R^{11a}$ and $R^{14a}$ are H and $R^{12a}$ and $R^{13a}$ join to form a 1,4-dioxanyl ring or a 1,3-dioxoianyl ring;

or $R^{11a}$ and $R^{12a}$ are H and $R^{13a}$ and $R^{14a}$ join to form a tetrahydrofuranyl ring;

Ab is selected from:

$C_{4-7}$cycloalkyl,
a 4-, 5-, or 6-membered heterocycloalkyl containing one or two heteroatoms independently selected from O and N,
and
a 5-12 membered heteroaryl containing one or two heteroatoms, wherein at least one heteroatom is nitrogen and the second heteroatom, if present, is selected from N and S;

$R^{15a}$ and $R^{16a}$ are independently selected from:

H,
$-OS(O)_2NH_2$,
$-S(O)_2CH_3$,
-OH,
-CN,
F,
tetrazolyl,
methyl-tetrazolyl,
ethyl-tetrazolyl,
cyclopropyl,
cyclopropyl substituted with one or two substituents independently selected from; -OH, $-OCH_3$, and $-CH_3$,
morpholinyl,
azetidinyl,
azetidinyl substituted with one or two substituents independently selected from: fluoro, chloro, bromo, iodo, -OH, $-CF_3$, and $-CH_3$,
pyridinyl,
pyridinyl substituted with -C=N,
oxazolyl,
oxazolyl substituted with $-C(O)OCH_2CH_3$,
oxazolyl substituted with -C=N,

-N(H)oxazolyl,
-N(H)oxazolyl substituted with $-C(O)OCH_2CH_3$,
-N(H)oxazolyl substituted with -C=N,
$-N(H)S(O)_2CH_3$,
oxo,
$C_{1-8}$alkyl,
$C_{1-8}$alkyl substituted with from one to six substituents independently selected from: -OH, oxo, fluoro, chloro, bromo, iodo, $C_{1-4}$alkoxy, cyclopropyl cyclopentyl, cyclobutyl, morpholinyl, methylpiperazinyl, $-NH_2$, $-N(H)C_{1-4}$alkyl, $-N(H)C_{1-4}$alkyl where alkyl is subitituted with from 1 to 5 fluoro, $-N(C_{1-4}alkyl)_2$, and $-N(C_{1-4}alkyl)_2$ where the alkyls are independently subitituted with from 1 to 7 fluoro,
$C_{1-8}$alkoxy,
$C_{1-8}$alkoxy subsitituted with from one to six substituents independently selected from: -OH, oxo, fluoro, chloro, bromo, iodo, $C_{1-4}$alkoxy, cycloalkyl, $-NH_2$, $-N(H)C_{1-4}$alkyl, $-N(H)C_{1-4}$alkyl where the alkyl is subitituted with from 1 to 5 fluoro, $-N(C_{1-4}alkyl)_2$, $-N(C_{1-4}alkyl)_2$ where the alkyls are independently subitituted with from 1 to 7 fluoro, $-S(O)_2CH_3$, $-S(O)_2NH_2$, and $-S(O)_2N(H)C_{1-4}$alkyl, dimethylamine oxide,
$N(H)C_{1-6}$alkyl,
$N(H)C_{1-6}$alkyl subsitituted with from one to six substituents independently selected from: -OH, oxo, fluoro, chloro, bromo, iodo, and $-S(O)_2CH_3$;

and salts thereof.

This disclosure also relates to pharmaceutically acceptable salts of the compounds of Formula (XII).

Included in the disclosure are compounds of Formula (XIII):

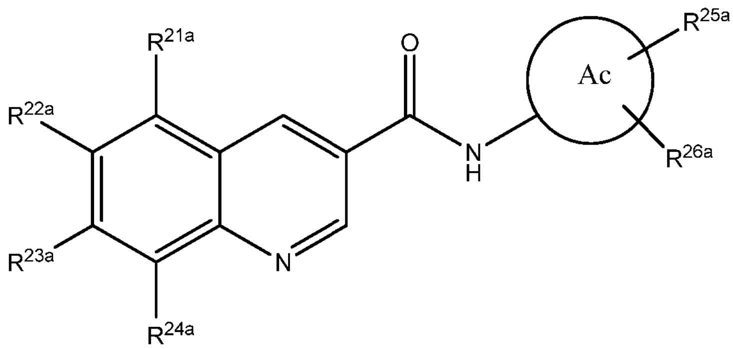

(XIII)

wherein:

$R^{21a}$ is selected from:

H, F,
-OH, and
$-OCH_3$;

$R^{22a}$ is selected from:

H, $C_{1-6}$alkyl, F, Cl, Br,
-C=N, and
$C_{1-4}$alkoxy;

$R^{23a}$ is selected from:

H,
F,
Cl,
Br,
-OH,

$-C(O)OC(CH_3)_3$,
-COOH,
$-C(O)C_{1-4}$alkyl,
$-C(O)C_{1-4}$alkyl substituted with from 1 to 5 substituents independently selected from: fluoro, chloro, bromo, oxo, -OH and -CN,
$-SC_{1-4}$alkyl,
$-SC_{1-4}$alkyl, substituted with from 1 to 5 substituents independently selected from: fluoro, chloro, bromo, oxo, -OH and -CN,
$-S(O)C_{1-4}$alkyl,
$-S(O)C_{1-4}$alkyl substituted with from 1 to 5 substituents independently selected from: fluoro, chloro, bromo, oxo, -OH and -CN,
$-NH_2$,
$-N(H)C_{1-3}$alkyl,
$-N(C_{1-3}alkyl)_2$,
-N(H)cyclopropyl,
$-N_3$,
-C=N,
$C_{1-4}$alkoxy,
$C_{1-4}$alkoxy substituted with from 1 to 5 substituents independently selected from: fluoro, chloro, bromo, oxo, -OH and -CN,
$C_{1-6}$alkyl,
$C_{1-6}$alkyl substituted with from 1 to 5 substituents Independently selected from: fluoro, chloro, bromo, iodo, oxo, $C_{1-4}$alkoxy, -OH, -COOH, $-NH_2$, $-N(H)C_{1-4}$alkyl, $-N(C_{1-4}alkyl)_2$ and -CN,
cyclopropyl,
cyclobutyl,
2,2-difluorocyclopropyl,
pyrrolidinyl,
azetidinyl, and
azetidinyl substituted with one or two substituents independently selected from F, Cl, Br, I, and methyl;

$R^{24a}$ is selected from:

H,
F,
Cl,
-OH,
-C=N,
$C_{1-2}$alkoxy, and
$C_{1-2}$alkyl;
where:
at least one substituent selected from $R^{21a}$, $R^{22a}$, $R^{23a}$, and $R^{24a}$ is not H;

or $R^{21a}$ and $R^{24a}$ are H and $R^{22a}$ and $R^{23a}$ join to form a 1,4-dioxanyl ring or a 1,3-dioxolanyl ring;

or $R^{21a}$ and $R^{22a}$ are H and $R^{23a}$ and $R^{24a}$ join to form a tetrahydrofuranyl ring;

Ac is selected from:

$C_{4-7}$cycloalkyl,
a 4-, 5-, or 6-membered heterocycloalkyl containing one or two heteroatoms independently selected from O and N,
and
a 5-12 membered heteroaryl containing one or two heteroatoms, wherein at least one heteroatom is nitrogen and the second heteroatom, if present, is selected from N and S;

$R^{25a}$ and $R^{26a}$ are independently selected from:

H,

$-OS(O)_2NH_2$,
$-S(O)_2CH_3$,
-OH,
-CN,
F,
methyl-tetrazolyl,
ethyl-tetrazolyl,
cyclopropyl,
cyclopropyl substituted with one or two substituents independently selected from; -OH, and $-OCH_3$,
morpholinyl,
azetidinyl,
azetidinyl substituted with one or two substituents independently selected from: fluoro, chloro, bromo, -OH, $-CF_3$, and $-CH_3$,
pyridinyl,
pyridinyl substituted with -C=N,
oxazolyl,
oxazolyl substituted with $-C(O)OCH_2CH_3$,
oxazolyl substituted with -C=N,
-N(H)oxazolyl,
-N(H)oxazolyl substituted with $-C(O)OCH_2CH_3$,
-N(H)oxazolyl substituted with -C=N,
$-N(H)S(O)_2CH_3$,
oxo,
$C_{1-8}$alkyl,
$C_{1-8}$alkyl substituted with from one to six substituents independently selected from: -OH, oxo, fluoro, chloro, bromo, iodo, $C_{1-4}$alkoxy, cyclopropyl, cyclopentyl, morpholinyl, methylpiperazinyl, $-NH_2$, $-N(H)C_{1-4}$alkyl, $-N(H)C_{1-4}$alkyl where alkyl is subitituted with from 1 to 5 fluoro, $-N(C_{1-4}alkyl)_2$, and $-N(C_{1-4}alkyl)_2$ where the alkyls are independently subitituted with from 1 to 7 fluoro,
$C_{1-8}$alkoxy,
$C_{1-8}$alkoxy subsitituted with from one to six substituents independently selected from: -OH, oxo, fluoro, chloro, bromo, iodo, $C_{1-4}$alkoxy, cyclopropyl, $-NH_2$, $-N(H)C_{1-4}$alkyl, $-N(H)C_{1-4}$alkyl where the alkyl is subitituted with from 1 to 5 fluoro, $-N(C_{1-4}alkyl)_2$, $-N(C_{1-4}alkyl)_2$ where the alkyls are independently subitituted with from 1 to 7 fluoro, $-S(O)_2CH_3$, $-S(O)_2NH_2$, and $-S(O)_2N(H)C_{1-4}$alkyl, dimethylamine oxide,
$N(H)C_{1-6}$alkyl,
$N(H)C_{1-6}$alkyl subsitituted with from one to six substituents independently selected from: -OH, oxo, fluoro, chloro, bromo, iodo, and $-S(O)_2CH_3$;

and salts thereof.

This disclosure also relates to pharmaceutically acceptable salts of the compounds of Formula (XIII).

Included are compounds of Formula (XIV):

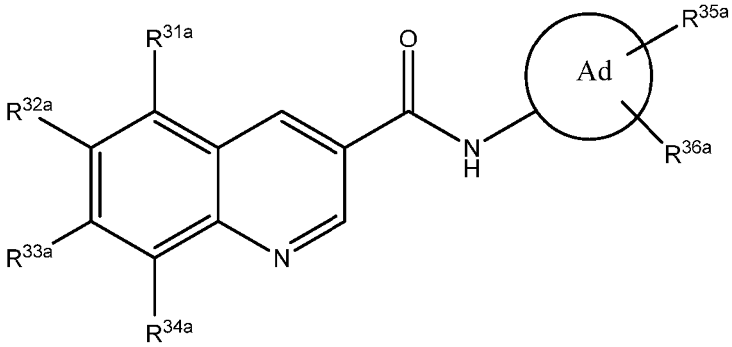

(XIV)

wherein:

$R^{31a}$ is selected from: H, F, and $-OCH_3$;

$R^{32a}$ is selected from: H, -C≡N, F, Cl, Br, $-OCH_3$, and $-CH_3$;

$R^{33a}$ is selected from: H, methyl, ethyl, $-CHCH_2$, $-OCH_3$, $-NH_2$, $-N(H)CH_3$, $-N(CH_3)_2$, -N(H)isopropyl, -N(H)cyclopropyl, $-SCH_3$, $-SCHF_2$,$-C(O)CHF_2$, $-C(O)CH_3$, $-N_3$, $-S(O)CHF_2$, $-OCH_2CH_3$, $-OCH(CH_3)_2$, $-OCHF_2$,$-OCF_3$, $-CF(CH_3)_2$,$-C(CH_3)F_2$, $-CF_3$, F, Cl, Br, cyclopropyl, cyclobutyl, 2,2-difluorocyclopropyl, pyrrolidinyl, azetidinyl or azetidinyl substituted with one or two substituents independently selected from F, Cl, Br, I, and methyl;
$R^{34a}$ is selected from: H, F, Cl, $-OCH_3$, methyl, ethyl, ethoxy, and -C=N;

where at least one substituent selected from $R^{31a}$, $R^{32a}$, $R^{33a}$, and $R^{34a}$ is not H;

or $R^{31a}$ and $R^{34a}$ are H and $R^{32a}$ and $R^{33a}$ join to form a 1,4-dioxanyl ring or a 1,3-dioxolanyl ring;

or $R^{31a}$ and $R^{32a}$ are H and $R^{33}$ and $R^{34}$ join to form a tetrahydrofuranyl ring;

and

either:

Ad is $C_{4-7}$cycloalkyl,
$R^{35a}$ is selected from: H, methyl, ethyl, -C≡N, $-CH_2OCH_2C≡CH$, $-C(CH_3)_2OH$, $-CH(CH_3)OH$, $-CH(CF_3)OH$, $-CH_2C(O)OCH_2CH_3$, $-CH_2NHCH(CH_3)CHF_2$, $-C(O)N(CH_3)_2$, $-C(O)OCH_3$, =O, $-OCH_3$, $-OCH_2C(O)NH_2$, $-CF_3$, $-CF_2$, $-OCH_2CH(CH_3)OH$, $-OCH_2C(CH_3)_2OH$, $-OCH_2C(CH_3)(CF_3)OH$, $-OCH_2CH(OH)CH(CH_3)_2$, $-OCH_2CH_2S(O)_2CH_3$, $-OCH_2C(O)NHCH_2C≡CH$, $-OCH_2C(O)NHCH_2CH_2C≡CH$, $-OCH_2C(CH_3)(CF_3)OH$, $-O(CH_2)_3NH_2$, $-NHS(O)_2CH_3$, $-NHCH(CH_3)CF_3$, $-NHCH(CH_3)CHF_2$, $-NHCH(CF_3)CH_2OH$, $-NHC(CH_3)_2CF_3$, $-NHCH_2CF_3$, $-NHCH_2CHF_2$, $-OS(O)_2NH_2$, -C(O)morpholinyl, -C(O)methylpiperazinyl,dimethylamine oxide, cyclopropanol, cyclopropanolmethoxy, morpholinyl, azetidinyl, azetidinyl substituted with one or two groups independently selected from: halogen, -OH, and $-CF_3$, $-C(OH)R^7R^8$ where $R^7$ is H and $R^8$ is selected from: cyclopropyl, $-CH_3$, and $-CF_3$, or $R^7$ and $R^8$ join together to form cyclopropyl ring, and $-NHR^9$ where $R^9$ is oxazolyl substituted with $-C(O)OCH_2CH_3$, and
$R^{36a}$ is selected from: H, and -OH,
where:
at least one substituent selected from $R^{35a}$, and $R^{36a}$ is not H;
or
Ad is a 4-, 5-, or 6-membered heterocycloalkyl containing one or two heteroatoms independently selected from O and N;
$R^{35a}$ is selected from: H, methyl, $-CH_2CF_3$, $-CH_2CHF_2$, $-C(CH_3)_2OH$, $-CH(CH_3)OH$, $-CH(CF_3)OH$, $-C(O)N(CH_3)_2$, =O, $-S(O)_2CH_3$, $-OS(O)_2NH_2$, ethyl-1*H*-tetrazolyl, methyl-1*H*-tetrazolyl, pyridinyl, pyridinyl substituted with -C=N, and oxazolyl substituted with $-C(O)OCH_2CH_3$ or -C=N, and
$R^{36a}$ is selected from: H, methyl, isopropyl, $-CH_2CH(CH_3)_2$, cyclopropylmethylene, or cyclopentylmethylene;
or
Ad is a 5-12 membered heteroaryl containing one or two heteroatoms, wherein at least one heteroatom is nitrogen and the second heteroatom, if present, is selected from N and S,
$R^{35a}$ is selected from: H, methyl or =O, and
$R^{36a}$ is selected from: H, and cyclopropyl;

and salts thereof.

This disclosure also relates to pharmaceutically acceptable salts of the compounds of Formula (XIV).

Suitably, in the compounds of Formula (XI), $R^{1a}$ is hydrogen.

Suitably, in the compounds of Formula (XI), $R^{2a}$ is hydrogen or halogen.

Suitably, in the compounds of Formula (XI), $R^{2a}$ is fluorine, or chlorine.

Suitably, in the compounds of Formula (XI), $R^{3a}$ is fluoromethoxy, difluoromethoxy, trifluoromethoxy, cyclopropyl, cyclobutyl, azetidinyl, methoxy, ethyl, methyl, bromine or chlorine, and $R^{4a}$ is hydrogen, fluorine or methoxy.

Suitably, in the compounds of Formula (XI), $R^{3a}$ is difluoromethoxy, azetidinyl, cyclobutyl, or cyclopropyl and $R^{4a}$ is hydrogen, fluorine or methoxy.

Suitably, in the compounds of Formula (XI), $R^{3a}$ is difluoromethoxy or azetidinyl and $R^{4a}$ is hydrogen, fluorine or methoxy.

Suitably, in the compounds of Formula (XI), $R^{4a}$ is hydrogen.

Suitably, in the compounds of Formula (XI), Aa is $C_{4-7}$cycloalkyl.

Suitably, in the compounds of Formula (XI), Aa is a 4-, 5-, or 6-membered heterocycloalkyl containing one or two heteroatoms independently selected from O and N.

Suitably, in the compounds of Formula (XI), Aa is a 5-12 membered heteroaryl containing one or two heteroatoms, wherein at least one heteroatom is nitrogen and the second heteroatom, if present, is selected from N and S;

Suitably, in the compounds of Formula (XI),

$R^{5a}$ is selected from: H, methyl, ethyl, -C≡N, $-CH_2OCH_2C≡CH$, $-C(CH_3)_2OH$, $-CH(CH_3)OH$, $-CH(CF_3)OH$, $-CH_2C(O)OCH_2CH_3$, $-CH_2NHCH(CH_3)CHF_2$, $-C(O)N(CH_3)_2$, $-C(O)OCH_3$, =O, $-OCH_3$, $-OCH_2C(O)NH_2$, $-CF_3$, $-CF_2$, $-OCH_2CH(CH_3)OH$, $-OCH_2C(CH_3)_2OH$, $-OCH_2C(CH_3)(CF_3)OH$, $-OCH_2CH(OH)CH(CH_3)_2$, $-OCH_2CH_2S(O)_2CH_3$, $-OCH_2C(O)NHCH_2C≡CH$, $-OCH_2C(O)NHCH_2CH_2C≡CH$, $-OCH_2C(CH_3)(CF_3)OH$, $-O(CH_2)_3NH_2$, $-NHS(O)_2CH_3$, $-NHCH(CH_3)CF_3$, $-NHCH(CH_3)CHF_2$, $-NHCH(CF_3)CH_2OH$, $-NHC(CH_3)_2CF_3$, $-NHCH_2CF_3$, $-NHCH_2CHF_2$, $-OS(O)_2NH_2$, -C(O)morpholinyl, -C(O)methylpiperazinyl,dimethylamine oxide, cyclopropanol, cyclopropanolmethoxy, morpholinyl, azetidinyl, azetidinyl substituted with one or two groups independently selected from: halogen, -OH, and $-CF_3$, $-C(OH)R^7R^8$ where $R^7$ is H and $R^8$ is selected from: cyclopropyl, $-CH_3$, and $-CF_3$, or $R^7$ and $R^8$ join together to form cyclopropyl ring, and $-NHR^9$ where $R^9$ is oxazolyl substituted with $-C(O)OCH_2CH_3$, and
$R^{6a}$ is selected from: H, and -OH,
where:
at least one substituent selected from $R^{5a}$, and $R^{6a}$ is not H.

A further illustrative disclosure relates to compounds of Formula (I):

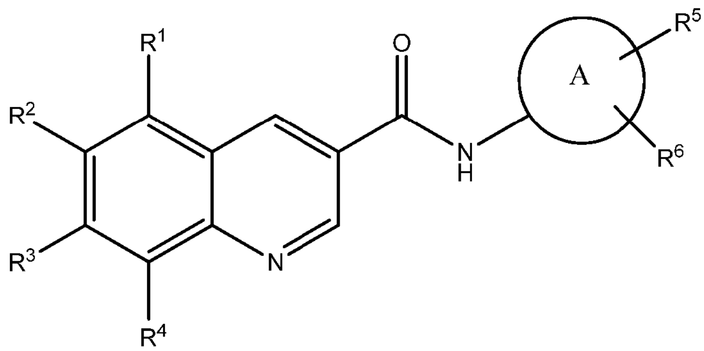

(I)

wherein:

$R^1$ is selected from:

H,
F,
Cl,
-OH,
$-OCH_3$, and
$C_1$alkoxy substituted 1 to 3 times with fluoro;

$R^2$ is selected from:

H,
F,
Cl,
Br,
I,
-OH,
$-C(O)OC(CH_3)_3$,
-COOH,
$-C(O)C_{1-4}$alkyl,
$-C(O)C_{1-4}$alkyl substituted with from 1 to 5 substituents independently selected from: fluoro, chloro, bromo, oxo, -OH and -CN,

-$SC_{1-4}$alkyl,
-$SC_{1-4}$alkyl, substituted with from 1 to 5 substituents independently selected from: fluoro, chloro, bromo, oxo, -OH and -CN,
-$S(O)C_{1-4}$alkyl,
-$S(O)C_{1-4}$alkyl substituted with from 1 to 5 substituents independently selected from: fluoro, chloro, bromo, oxo, -OH and -CN,
-$N_3$,
-C=N,
$C_{1-4}$alkoxy,
$C_{1-4}$alkoxy substituted with from 1 to 5 substituents independently selected from: fluoro, chloro, bromo, oxo, -OH and -CN,
$C_{1-6}$alkyl,
$C_{1-6}$alkyl substituted with from 1 to 5 substituents Independently selected from: fluoro, chloro, bromo, iodo, oxo, $C_{1-4}$alkoxy, -OH, -COOH, -$NH_2$, -$N(H)C_{1-4}$alkyl, -$N(C_{1-4}alkyl)_2$ and -CN,
azido,
cyclopropyl,
cyclobutyl,
2,2-difluorocyclopro pyl,
pyrrolidinyl,
azetidinyl, and
azetidinyl substituted with one or two substituents independently selected from halogen and methyl;

$R^3$ is selected from:

H,
F,
Cl,
Br,
I,
-OH,
-$C(O)OC(CH_3)_3$,
-COOH,
-$C(O)C_{1-4}$alkyl,
-$C(O)C_{1-4}$alkyl substituted with from 1 to 5 substituents independently selected from: fluoro, chloro, bromo, oxo, -OH and -CN,
-$SC_{1-4}$alkyl,
-$SC_{1-4}$alkyl, substituted with from 1 to 5 substituents independently selected from: fluoro, chloro, bromo, oxo, -OH and -CN,
-$S(O)C_{1-4}$alkyl,
-$S(O)C_{1-4}$alkyl substituted with from 1 to 5 substituents independently selected from: fluoro, chloro, bromo, oxo, -OH and -CN,
-$N_3$,
-C=N,
$C_{1-4}$alkoxy,
$C_{1-4}$alkoxy substituted with from 1 to 5 substituents independently selected from: fluoro, chloro, bromo, oxo, -OH and -CN,
$C_{1-6}$alkyl,
$C_{1-6}$alkyl substituted with from 1 to 5 substituents Independently selected from: fluoro, chloro, bromo, iodo, oxo, $C_{1-4}$alkoxy, -OH, -COOH, -$NH_2$, -$N(H)C_{1-4}$alkyl, -$N(C_{1-4}alkyl)_2$ and -CN,
azido,
cyclopropyl,
cyclobutyl,
2,2-difluorocyclopropyl,
pyrrolidinyl,
azetidinyl, and
azetidinyl substituted with one or two substituents independently selected from halogen and methyl;

$R^4$ is selected from:

H,
F,
Cl,
-OH,
-C=N,
$C_{1-4}$alkoxy,
$C_{1-4}$alkoxy substituted from 1 to 5 times by fluoro,
$C_{1-3}$alkyl, and
$C_{1-3}$alkyl substituted from 1 to 5 times by fluoro,

where:
at least one substituent selected from $R^1$, $R^2$, $R^3$, and $R^4$ is not H;

or $R^1$ and $R^4$ are H and $R^2$ and $R^3$ join to form a 1,4-dioxanyl ring,

or $R^1$ and $R^2$ are H and $R^3$ and $R^4$ join to form a tetrahydrofuranyl ring,

A is selected from:

$C_{4-7}$cycloalkyl,
a 4-, 5-, or 6-membered heterocycloalkyl containing one or two heteroatoms independently selected from O and N, and
a 5-10 membered heteroaryl containing one or two heteroatoms, wherein at least one heteroatom is nitrogen and the second heteroatom, if present, is selected from N and S;
$R^5$ and $R^6$ are independently selected from:

hydrogen,
$-OS(O)_2NH_2$,
$-S(O)_2CH_3$,
-OH,
-C=N,
F,
Cl,
Br,
I,
tetrazole,
methyl-tetrazole,
cycloalkyl,
morpholinyl,
azetidinyl,
azetidinyl substituted with one or two substituents independently selected from: fluoro, chloro, bromo, iodo, -OH, $-CF_3$, and $-CH_3$,
pyridinyl,
pyridinyl substituted with -C=N,
oxazolyl,
oxazolyl substituted with $-C(O)OCH_2CH_3$,
oxazolyl substituted with -C=N,
-N(H)oxazolyl,
-N(H)oxazolyl substituted with $-C(O)OCH_2CH_3$,
-N(H)oxazolyl substituted with -C=N,
$-N(H)S(O)_2CH_3$,
oxo,
$C_{1-8}$alkyl,
$C_{1-8}$alkyl substituted with from one to six substituents independently selected from: -OH, oxo, fluoro, chloro, bromo, iodo, $C_{1-4}$alkoxy, cycloalkyl, $-NH_2$, $-N(H)C_{1-4}$alkyl, $-N(H)C_{1-4}$alkyl where alkyl is subitituted with from 1 to 5 fluoro, $-N(C_{1-4}alkyl)_2$, and $-N(C_{1-4}alkyl)_2$ where the alkyls are independently subitituted with from 1 to 7 fluoro, $C_{1-8}$alkoxy,
$C_{1-8}$alkoxy subsitituted with from one to six substituents independently

selected from: -OH, oxo, fluoro, chloro, bromo, iodo, $C_{1-4}$alkoxy, cycloalkyl, $-NH_2$, $-N(H)C_{1-4}$alkyl, $-N(H)C_{1-4}$alkyl where the alkyl is subitituted with from 1 to 5 fluoro, $-N(C_{1-4}alkyl)_2$, $-N(C_{1-4}alkyl)_2$ where the alkyls are independently subitituted with from 1 to 7 fluoro,
$-S(O)_2CH_3$, $-S(O)_2NH_2$, and $-S(O)_2N(H)C_{1-4}$alkyl, $N(C_{1-6}alkyl)_2$, where each alkyl is optionally subsitituted with from one to six substituents independently selected from: -OH, oxo, fluoro, chloro, bromo, iodo, and $-S(O)_2CH_3$,
$N(H)C_{1-6}$alkyl,
$N(H)C_{1-6}$alkyl subsitituted with from one to six substituents independently selected from: -OH, oxo, fluoro, chloro, bromo, iodo, and $-S(O)_2CH_3$;

and salts thereof.

This disclosure also relates to pharmaceutically acceptable salts of the compounds of Formula (I).
Included in the disclosure are compounds of Formula (II):

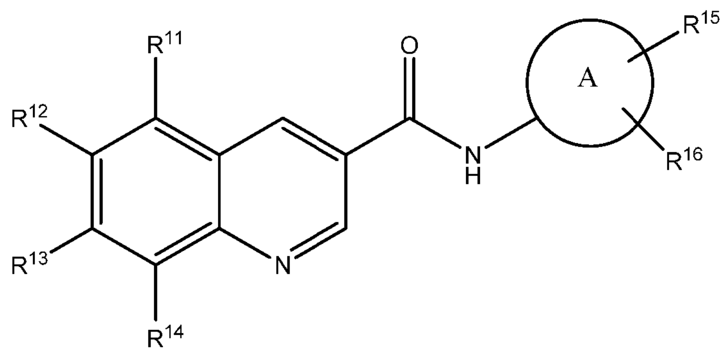

(II)

wherein:

$R^{11}$ is selected from:

H,
F,
Cl,
-OH, and
$-OCH_3$;

$R^{12}$ is selected from:

H,
$C_{1-6}$alkyl,
F,
Cl,
Br,
-C=N, and
$C_{1-4}$alkoxy;

$R^{13}$ is selected from:

H,
F,
Cl,
Br,
I,
-OH,
$-C(O)OC(CH_3)_3$,
-COOH,
$-C(O)C_{1-4}$alkyl,

$-C(O)C_{1-4}$alkyl substituted with from 1 to 5 substituents independently selected from: fluoro, chloro, bromo, oxo, -OH and -CN,
$-SC_{1-4}$alkyl,
$-SC_{1-4}$alkyl, substituted with from 1 to 5 substituents independently selected from: fluoro, chloro, bromo, oxo, -OH and -CN,
$-S(O)C_{1-4}$alkyl,
$-S(O)C_{1-4}$alkyl substituted with from 1 to 5 substituents independently selected from: fluoro, chloro, bromo, oxo, -OH and -CN,
$-N_3$,
-C=N,
$C_{1-4}$alkoxy,
$C_{1-4}$alkoxy substituted with from 1 to 5 substituents independently selected from: fluoro, chloro, bromo, oxo, -OH and -CN,
$C_{1-6}$alkyl,
$C_{1-6}$alkyl substituted with from 1 to 5 substituents Independently selected from: fluoro, chloro, bromo, iodo, oxo, $C_{1-4}$alkoxy, -OH, -COOH, $-NH_2$, $-N(H)C_{1-4}$alkyl, $-N(C_{1-4}alkyl)_2$ and -CN,
azido,
cyclopropyl,
cyclobutyl,
2,2-difluorocyclopropyl,
pyrrolidinyl,
azetidinyl, and
azetidinyl substituted with one or two substituents independently selected from halogen and methyl;

$R^{14}$ is selected from:

H,
F,
Cl,
-OH,
-C=N,
$C_{1-4}$alkoxy, and
$C_{1-3}$alkyl;

where:
at least one substituent selected from $R^{11}$, $R^{12}$, $R^{13}$, and $R^{14}$ is not H;

or $R^{11}$ and $R^{14}$ are H and $R^{12}$ and $R^{13}$ join to form a 1,4-dioxanyl ring;

or $R^{11}$ and $R^{12}$ are H and $R^{13}$ and $R^{14}$ join to form a tetrahydrofuranyl ring;

A is selected from:

$C_{4-7}$cycloalkyl,
a 4-, 5-, or 6-membered heterocycloalkyl containing one or two heteroatoms independently selected from O and N,
and
a 5-10 membered heteroaryl containing one or two heteroatoms, wherein at least one heteroatom is nitrogen and the second heteroatom, if present, is selected from N and S;

$R^{15}$ and $R^{16}$ are independently selected from:

H,
$-OS(O)_2NH_2$,
$-S(O)_2CH_3$,
-OH,
-CN,
F,

tetrazole,
methyl-tetrazole,
cyclopropyl,
morpholinyl,
azetidinyl,
azetidinyl substituted with one or two substituents independently selected from: fluoro, chloro, bromo, iodo, -OH, $-CF_3$, and $-CH_3$,
pyridinyl,
pyridinyl substituted with -C=N,
oxazolyl,
oxazolyl substituted with $-C(O)OCH_2CH_3$,
oxazolyl substituted with -C=N,
-N(H)oxazolyl,
-N(H)oxazolyl substituted with $-C(O)OCH_2CH_3$,
-N(H)oxazolyl substituted with -C=N,
$-N(H)S(O)_2CH_3$,
oxo,
$C_{1-8}$alkyl,
$C_{1-8}$alkyl substituted with from one to six substituents independently selected from: -OH, oxo, fluoro, chloro, bromo, iodo, $C_{1-4}$alkoxy, cyclopropyl cyclopentyl, cyclobutyl, $-NH_2$, $-N(H)C_{1-4}$alkyl, $-N(H)C_{1-4}$alkyl where alkyl is subitituted with from 1 to 5 fluoro, $-N(C_{1-4}alkyl)_2$, and $-N(C_{1-4}alkyl)_2$ where the alkyls are independently subitituted with from 1 to 7 fluoro,
$C_{1-8}$alkoxy,
$C_{1-8}$alkoxy subsitituted with from one to six substituents independently selected from: -OH, oxo, fluoro, chloro, bromo, iodo, $C_{1-4}$alkoxy, cycloalkyl, $-NH_2$, $-N(H)C_{1-4}$alkyl, $-N(H)C_{1-4}$alkyl where the alkyl is subitituted with from 1 to 5 fluoro, $-N(C_{1-4}alkyl)_2$, $-N(C_{1-4}alkyl)_2$ where the alkyls are independently subitituted with from 1 to 7 fluoro, $-S(O)_2CH_3$, $-S(O)_2NH_2$, and $-S(O)_2N(H)C_{1-4}$alkyl,
$N(H)C_{1-6}$alkyl,
$N(H)C_{1-6}$alkyl subsitituted with from one to six substituents independently selected from: -OH, oxo, fluoro, chloro, bromo, iodo, and $-S(O)_2CH_3$;

and salts thereof.

This disclosure also relates to pharmaceutically acceptable salts of the compounds of Formula (II).

Included in the disclosure are compounds of Formula (III):

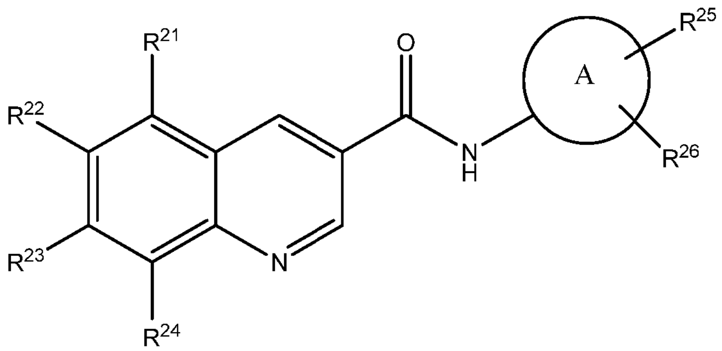

(III)

wherein:

$R^{21}$ is selected from:

H,
F,
-OH, and
$-OCH_3$;

$R^{22}$ is selected from:

H,
$C_{1-6}$alkyl,
F,
Cl,
Br,
-C=N, and
$C_{1-4}$alkoxy;

$R^{23}$ is selected from:

H,
F,
Cl,
Br,
-OH,
-C(O)OC$(CH_3)_3$,
-COOH,
-C(O)$C_{1-4}$alkyl,
-C(O)$C_{1-4}$alkyl substituted with from 1 to 5 substituents independently selected from: fluoro, chloro, bromo, oxo, -OH and -CN,
-S$C_{1-4}$alkyl,
-S$C_{1-4}$alkyl, substituted with from 1 to 5 substituents independently selected from: fluoro, chloro, bromo, oxo, -OH and -CN,
-S(O)$C_{1-4}$alkyl,
-S(O)$C_{1-4}$alkyl substituted with from 1 to 5 substituents independently selected from: fluoro, chloro, bromo, oxo, -OH and -CN,
-$N_3$,
-C=N,
$C_{1-4}$alkoxy,
$C_{1-4}$alkoxy substituted with from 1 to 5 substituents independently selected from: fluoro, chloro, bromo, oxo, -OH and -CN,
$C_{1-6}$alkyl,
$C_{1-6}$alkyl substituted with from 1 to 5 substituents Independently selected from: fluoro, chloro, bromo, iodo, oxo, $C_{1-4}$alkoxy, -OH, -COOH, -$NH_2$, -N(H)$C_{1-4}$alkyl, -N($C_{1-4}$alkyl)$_2$ and -CN,
azido,
cyclopropyl,
cyclobutyl,
2,2-difluorocyclopropyl,
pyrrolidinyl,
azetidinyl, and
azetidinyl substituted with one or two substituents independently selected from halogen and methyl;

$R^{24}$ is selected from:

H,
F,
Cl,
-OH,
-C=N,
$C_{1-2}$alkoxy, and
$C_{1-2}$alkyl;

where:
at least one substituent selected from $R^{21}$, $R^{22}$, $R^{23}$, and $R^{24}$ is not H;

or $R^{21}$ and $R^{24}$ are H and $R^{22}$ and $R^{23}$ join to form a 1,4-dioxanyl ring;

or $R^{21}$ and $R^{22}$ are H and $R^{23}$ and $R^{24}$ join to form a tetrahydrofuranyl ring;

A is selected from:

$C_{4-7}$cycloalkyl,
a 4-, 5-, or 6-membered heterocycloalkyl containing one or two heteroatoms independently selected from O and N,
and
a 5-10 membered heteroaryl containing one or two heteroatoms, wherein at least one heteroatom is nitrogen and the second heteroatom, if present, is selected from N and S;

$R^{25}$ and $R^{26}$ are independently selected from:

H,
$-OS(O)_2NH_2$,
$-S(O)_2CH_3$,
-OH,
-CN,
F,
methyl-tetrazole,
cyclopropyl,
morpholinyl,
azetidinyl,
azetidinyl substituted with one or two substituents independently selected from: fluoro, chloro, bromo, -OH, $-CF_3$, and $-CH_3$,
pyridinyl,
pyridinyl substituted with -C=N,
oxazolyl,
oxazolyl substituted with $-C(O)OCH_2CH_3$,
oxazolyl substituted with -C=N,
-N(H)oxazolyl,
-N(H)oxazolyl substituted with $-C(O)OCH_2CH_3$,
-N(H)oxazolyl substituted with -C=N,
$-N(H)S(O)_2CH_3$,
oxo,
$C_{1-8}$alkyl,
$C_{1-8}$alkyl substituted with from one to six substituents independently selected from: -OH, oxo, fluoro, chloro, bromo, iodo, $C_{1-4}$alkoxy, cyclopropyl, cyclopentyl, $-NH_2$, $-N(H)C_{1-4}$alkyl, $-N(H)C_{1-4}$alkyl where alkyl is subitituted with from 1 to 5 fluoro, $-N(C_{1-4}alkyl)_2$, and $-N(C_{1-4}alkyl)_2$ where the alkyls are independently subitituted with from 1 to 7 fluoro,
$C_{1-8}$alkoxy,
$C_{1-8}$alkoxy subsitituted with from one to six substituents independently selected from: -OH, oxo, fluoro, chloro, bromo, iodo, $C_{1-4}$alkoxy, cyclopropyl, $-NH_2$, $-N(H)C_{1-4}$alkyl, $-N(H)C_{1-4}$alkyl where the alkyl is subitituted with from 1 to 5 fluoro, $-N(C_{1-4}alkyl)_2$, $-N(C_{1-4}alkyl)_2$ where the alkyls are independently subitituted with from 1 to 7 fluoro, $-S(O)_2CH_3$, $-S(O)_2NH_2$, and $-S(O)_2N(H)C_{1-4}$alkyl,
$N(H)C_{1-6}$alkyl,
$N(H)C_{1-6}$alkyl subsitituted with from one to six substituents independently selected from: -OH, oxo, fluoro, chloro, bromo, iodo, and $-S(O)_2CH_3$;

and salts thereof.

This disclosure also relates to pharmaceutically acceptable salts of the compounds of Formula (III).

Included in the disclosure are compounds of Formula (IV):

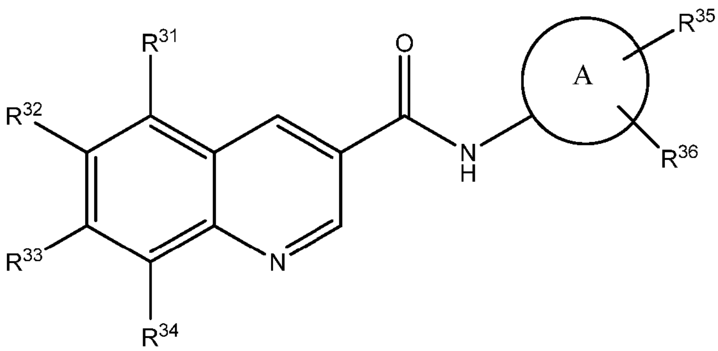

(IV)

wherein:

$R^{31}$ is selected from: H, F, and $-OCH_3$;

$R^{32}$ is selected from: H, -C≡N, F, Cl, Br, I, $-OCH_3$, and $-CH_3$;

$R^{33}$ is selected from: H, methyl, ethyl, $-OCH_3$, $-NH_2$, $-SCH_3$, $-SCHF_2$,$-C(O)CHF_2$, $-C(O)CH_3$, $-N(CH_3)_2$, $-N_3$, $-S(O)CHF_2$, $-OCH_2CH_3$, $-OCH(CH_3)_2$,$-OCHF_2$, $-OCF_3$, $-CF_3$, halogen, azido, cyclopropyl, cyclobutyl, 2,2-difluorocyclopropyl, pyrrolidinyl, azetidinyl or azetidinyl substituted with one or two substituents independently selected from halogen, and methyl;
$R^{34}$ is selected from: H, F, Cl, $-OCH_3$, methyl, ethyl, ethoxy, and -C=N;

where at least one substituent selected from $R^{31}$, $R^{32}$, $R^{33}$, and $R^{34}$ is not H;

or $R^{31}$ and $R^{34}$ are H and $R^{32}$ and $R^{33}$ join to form a 1,4-dioxanyl ring;

or $R^{31}$ and $R^{32}$ are H and $R^{33}$ and $R^{34}$ join to form a tetrahydrofuranyl ring;

and

either:

A is $C_{4-7}$cycloalkyl,
$R^{35}$ is selected from: H, methyl, ethyl, -C≡N, $-CH_2OCH_2C{\equiv}CH$, $-C(CH_3)_2OH$, $-CH(CH_3)OH$, $-CH(CF_3)OH$, $-CH_2C(O)OCH_2CH_3$, $-CH_2NHCH(CH_3)CHF_2$, $-C(O)N(CH_3)_2$, $-C(O)OCH_3$, =O, $-OCH_3$, $-OCH_2C(O)NH_2$, $-OCH_2CH(CH_3)OH$, $-OCH_2C(CH_3)_2OH$, $-OCH_2C(CH_3)(CF_3)OH$, $-OCH_2CH(OH)CH(CH_3)_2$, $-OCH_2CH_2S(O)_2CH_3$, $-OCH_2C(O)NHCH_2C{=}CH$, $-OCH_2C(O)NHCH_2CH_2C{=}CH$, $-OCH_2C(CH_3)(CF_3)OH$, $-O(CH_2)_3NH_2$,$-NH_2$, $-NHS(O)_2CH_3$, $-NHCH(CH_3)CF_3$, $-NHCH(CH_3)CHF_2$, $-NHCH(CF_3)CH_2OH$, $-NHC(CH_3)_2CF_3$, $-NHCH_2CF_3$, $-NHCH_2CHF_2$, $-OS(O)_2NH_2$, cyclopropanol, cyclopropanolmethoxy, morpholinyl, azetidinyl, azetidinyl substituted with one or two groups independently selected from: halogen, -OH, and $-CF_3$, $-C(OH)R^7R^8$ where $R^7$ is H and $R^8$ is selected from: cyclopropyl, $-CH_3$, and $-CF_3$, or $R^7$ and $R^8$ join together to form cyclopropyl ring, and $-NHR^9$ where $R^9$ is oxazolyl substituted with $-C(O)OCH_2CH_3$, and
$R^{36}$ is selected from: H, and -OH,
where:

at least one substituent selected from $R^{35}$, and $R^{36}$ is not H;
or

A is a 4-, 5-, or 6-membered heterocycloalkyl containing one or two heteroatoms independently selected from O and N;
$R^{35}$ is selected from: H, methyl, $-CH_2CF_3$, $-CH_2CHF_2$, $-C(CH_3)_2OH$, $-CH(CH_3)OH$, $-CH(CF_3)OH$, $-C(O)N(CH_3)_2$, =O, $-S(O)_2CH_3$, $-OS(O)_2NH_2$, ethyl-1*H*-tetrazolyl, methyl-1*H*-tetrazolyl, pyridinyl, pyridinyl substituted with -C=N, and oxazolyl substituted with $-C(O)OCH_2CH_3$ or -C≡N, and $R^{36}$ is selected from: H, methyl, isopropyl, $-CH_2CH(CH_3)_2$, cyclopropylmethylene, or cyclopentylmethylene;
or

A is a 5-10 membered heteroaryl containing one or two heteroatoms, wherein at least one heteroatom is nitrogen and the second heteroatom, if present, is selected from N and S,
$R^{35}$ is selected from: H, methyl or =O, and
$R^{36}$ is selected from: H, and cyclopropyl;

and salts thereof.

This disclosure also relates to pharmaceutically acceptable salts of the compounds of Formula (IV).
Suitably, in the compounds of Formula (I), $R^1$ is hydrogen.
Suitably, in the compounds of Formula (I), $R^2$ is hydrogen or halogen.
Suitably, in the compounds of Formula (I), $R^2$ is fluorine, or chlorine.
Suitably, in the compounds of Formula (I), $R^3$ is fluoromethoxy, difluoromethoxy, trifluoromethoxy, cyclopropyl, cyclobutyl, azetidinyl, methoxy, ethyl, methyl, bromine or chlorine, and $R^4$ is hydrogen, fluorine or methoxy.
Suitably, in the compounds of Formula (I), $R^3$ is difluoromethoxy, azetidinyl, cyclobutyl, or cyclopropyl and $R^4$ is hydrogen, fluorine or methoxy.
Suitably, in the compounds of Formula (I), $R^3$ is difluoromethoxy or azetidinyl and $R^4$ is hydrogen, fluorine or methoxy.
Suitably, in the compounds of Formula (I), $R^4$ is hydrogen.
Included in the illustrative disclosure are compounds of Formula (XI) selected from:

7-Methoxy-N-(1-(1-methyl-1*H*-tetrazol-5-yl)piperidin-4-yl)quinoline-3-carboxamide;
(S)-7-Methoxy-N-(2-oxopyrrolidin-3-yl)quinoline-3-carboxamide;
7-(Difluoromethoxy)-N-(1-(1-methyl-1H-tetrazol-5-yl)piperidin-4-yl)quinoline-3-carboxamide;
(S)-7-(Difluoromethoxy)-N-(2-oxopyrrolidin-3-yl)quinoline-3-carboxamide;
7-(Difluoromethoxy)-N-((trans)-4-(dimethylcarbamoyl)cyclohexyl)quinoline-3-carboxamide;
7-(Difluoromethoxy)-N-((trans)-4-(2-hydroxypropan-2-yl)cyclohexyl)quinoline-3-carboxamide;
7-(Difluoromethoxy)-N-(1-(methylsulfonyl)piperidin-4-yl)quinoline-3-carboxamide;
7-(Difluoromethoxy)-N-(3-methyl-1H-pyrazol-5-yl)quinoline-3-carboxamide;
7-(Difluoromethoxy)-N-(2-oxo-1,2,3,4-tetrahydroquinolin-4-yl)quinoline-3-carboxamide;
7-(Difluoromethoxy)-N-(thiazol-2-yl)quinoline-3-carboxamide;
7-(Difluoromethoxy)-N-(trans-3-(2-hydroxypropan-2-yl)cyclobutyl)quinoline-3-carboxamide;
7-(Difluoromethoxy)-N-(trans-4-(2-hydroxy-2-methylpropoxy)cyclohexyl)quinoline-3-carboxamide;
7-(Difluoromethoxy)-N-(trans-4-(-1-hydroxyethyl)cyclohexyl)quinoline-3-carboxamide;
N-(trans-4-(-Cyclopropyl(hydroxy)methyl)cyclohexyl)-7-(difluoromethoxy)quinoline-3-carboxamide;
7-(Difluoromethoxy)-N-(trans-4-(2,2,2-trifluoro-1-hydroxyethyl)cyclohexyl)quinoline-3-carboxamide;
7-(Difluoromethoxy)-N-(trans-4-(2-hydroxypropoxy)cyclohexyl)quinoline-3-carboxamide;
7-(Difluoromethoxy)-N-((3R,6S)-6-(2,2,2-trifluoro-1-hydroxyethyl)tetrahydro-2H-pyran-3-yl)quinoline-3-carboxamide;
7-(Difluoromethoxy)-N-(trans-4-(1-hydroxycyclopropyl)cyclohexyl)quinoline-3-carboxamide;
7-(Difluoromethoxy)-N-(trans-4-(3,3,3-trifluoro-2-hydroxy-2-methylpropoxy)cyclohexyl)quinoline-3-carboxamide;
7-(Difluoromethoxy)-N-(trans-4-((*R*)-2,2,2-trifluoro-1-hydroxyethyl)cyclohexyl)quinoline-3-carboxamide;
7-(Difluoromethoxy)-N-(trans-4-((S)-2,2,2-trifluoro-1-hydroxyethyl)cyclohexyl)quinoline-3-carboxamide;
7-(Difluoromethoxy)-N-((3s,6*r*)-6-(2-hydroxypropan-2-yl)tetrahydro-2H-pyran-3-yl)quinoline-3-carboxamide;
N-(trans-4-((3-Aminopropoxy)methyl)cyclohexyl)-7-(difluoromethoxy)quinoline-3-carboxamide;
6-Cyano-7-cyclopropyl-N-(1-(1-methyl-1*H*-tetrazol-5-yl)piperidin-4-yl)quinoline-3-carboxamide;
(S)-6-Cyano-7-cyclopropyl-N-(2-oxopyrrolidin-3-yl)quinoline-3-carboxamide;
6-Cyano-7-cyclopropyl-N-(2-oxo-1,2,3,4-tetrahydroquinolin-4-yl)quinoline-3-carboxamide;
7-Bromo-6-chloro-N-(1-(1-methyl-1*H*-tetrazol-5-yl)piperidin-4-yl)quinoline-3-carboxamide;
8-Methoxy-N-(thiazol-2-yl)quinoline-3-carboxamide;
7-Methoxy-6-methyl-N-(1-(1-methyl-1*H*-tetrazol-5-yl)piperidin-4-yl)quinoline-3-carboxamide;
5-Fluoro-7-methoxy-N-(1-(1-methyl-1*H*-tetrazol-5-yl)piperidin-4-yl)quinoline-3-carboxamide;
7-Chloro-8-methoxy-N-(1-(1-methyl-1*H*-tetrazol-5-yl)piperidin-4-yl)quinoline-3-carboxamide;
6-Chloro-7-methoxy-N-(1-(1-methyl-1*H*-tetrazol-5-yl)piperidin-4-yl)quinoline-3-carboxamide;
7,8-Dimethoxy-N-(1-(1-methyl-1*H*-tetrazol-5-yl)piperidin-4-yl)quinoline-3-carboxamide;
6-Chloro-7-cyclopropyl-N-(1-(1-methyl-1*H*-tetrazol-5-yl)piperidin-4-yl)quinoline-3-carboxamide;
(S)-6-Chloro-7-cyclopropyl-N-(2-oxopyrrolidin-3-yl)quinoline-3-carboxamide;
(S)-6-Chloro-7-cyclopropyl-N-(2-oxopiperidin-3-yl)quinoline-3-carboxamide;
6-Chloro-7-cyclopropyl-N-(2-oxo-1,2,3,4-tetrahydroquinolin-4-yl)quinoline-3-carboxamide;

(S)-6-Chloro-7-cyclopropyl-N-(3-oxoisoxazolidin-4-yl)quinoline-3-carboxamide;
6-Chloro-7-cyclopropyl-N-(trans)-4-(2-hydroxypropan-2-yl)cyclohexyl)quinoline-3-carboxamide;
6-Chloro-7-cyclopropyl-N-((1s,3s)-3-hydroxy-3-methylcyclobutyl)quinoline-3-carboxamide;
(S)-7-Cyclopropyl-6-fluoro-N-(2-oxopyrrolidin-3-yl)quinoline-3-carboxamide;
7-Cyclopropyl-6-fluoro-N-(trans-4-(2-hydroxypropan-2-yl)cyclohexyl)quinoline-3-carboxamide;
7-Cyclopropyl-N-(*trans*-4-((2,2-difluoroethyl)amino)cyclohexyl)-6-fluoroquinoline-3-carboxamide;
7-Cyclopropyl-N-(1-(1-methyl-1*H*-tetrazol-5-yl)piperidin-4-yl)quinoline-3-carboxamide;
(S)-7-Cyclopropyl-N-(2-oxopyrrolidin-3-yl)quinoline-3-carboxamide;
6-Fluoro-7-methoxy-N-(1-(1-methyl-1*H*-tetrazol-5-yl)piperidin-4-yl)quinoline-3-carboxamide;
8-Fluoro-7-methoxy-N-(thiazol-2-yl)quinoline-3-carboxamide;
(S)-7-(Difluoromethoxy)-6-fluoro-N-(2-oxopyrrolidin-3-yl)quinoline-3-carboxamide;
N-(*trans*-4-((2,2-Difluoroethyl)amino)cyclohexyl)-7-(difluoromethoxy)-6-fluoroquinoline-3-carboxamide;
(S)-7-(Difluoromethoxy)-6-fluoro-N-(1-isobutyl-2-oxopyrrolidin-3-yl)quinoline-3-carboxamide;
7-(Difluoromethoxy)-6-fluoro-N-(trans-4-(2-hydroxypropan-2-yl)cyclohexyl)quinoline-3-carboxamide;
7-(Difluoromethoxy)-6-fluoro-N-((1S,2R)-2-hydroxycyclopentyl)quinoline-3-carboxamide;
(S)-N-(1-(Cyclopropylmethyl)-2-oxopyrrolidin-3-yl)-7-(difluoromethoxy)-6-fluoroquinoline-3-carboxamide;
7-(Difluoromethoxy)-6-fluoro-N-(2-oxo-1,2,3,4-tetrahydroquinolin-4-yl)quinoline-3-carboxamide;
7-(Difluoromethoxy)-6-fluoro-N-(trans-4-methoxycyclohexyl)quinoline-3-carboxamide;
7-(Difluoromethoxy)-6-fluoro-N-(trans-4-(methylsulfonamido)cyclohexyl)quinoline-3-carboxamide;
7-(Diflluoromethoxy)-6-flluoro-N-(*trans*-4-((1,1,1-triflluoropropan-2-yl)amino)cyclohexyl)quinoline-3-carboxamide;
7-(Difluoromethoxy)-6-fluoro-N-(1-(2,2,2-trifluoroethyl)piperidin-4-yl)quinoline-3-carboxamide;
7-(Difluoromethoxy)-6-fluoro-N-(*trans*-3-((2,2,2-trifluoroethyl)amino)cyclobutyl)quinoline-3-carboxamide;
7-(Difluoromethoxy)-6-fluoro-N-(trans-4-(2-hydroxy-2-methylpropoxy)cyclohexyl)quinoline-3-carboxamide;
7-(Difluoromethoxy)-6-fluoro-N-(6-((2,2,2-trifluoroethyl)amino)spiro[3.3]heptan-2-yl)quinoline-3-carboxamide;
7-(Difluoromethoxy)-6-fluoro-N-(trans-4-(((R)-1,1,1-trifluoropropan-2-yl)amino)cyclohexyl)quinoline-3-carboxamide;
7-(Difluoromethoxy)-6-fluoro-N-(trans-4-(((S)-1,1,1-trifluoropropan-2-yl)amino)cyclohexyl)quinoline-3-carboxamide;
7-(Difluoromethoxy)-6-fluoro-N-((1R,3R)-3-((2,2,2-trifluoroethyl)amino)cyclopentyl)quinoline-3-carboxamide;
N-(1-(2,2-Difluoroethyl)piperidin-4-yl)-7-(difluoromethoxy)-6-fluoroquinoline-3-carboxamide;
7-(Difluoromethoxy)-6-fluoro-N-(*trans*-4-((1,1,1-trifluoro-2-methylpropan-2-yl)amino)cyclohexyl)quinoline-3-carboxamide;
7-(Difluoromethoxy)-N-(*trans*-4-((1,1-difluoropropan-2-yl)amino)cyclohexyl)-6-fluoroquinoline-3-carboxamide;
7-(Difluoromethoxy)-6-fluoro-N-(4-morpholinocyclohexyl)quinoline-3-carboxamide;
7-(Difluoromethoxy)-N-(trans-4-(((R)-1,1-difluoropropan-2-yl)amino)cyclohexyl)-6-fluoroquinoline-3-carboxamide;
7-(Difluoromethoxy)-N-(trans-4-(((S)-1,1-difluoropropan-2-yl)amino)cyclohexyl)-6-fluoroquinoline-3-carboxamide;
N-(*trans*-4-(3,3-Difluoroazetidin-1-yl)cyclohexyl)-7-(difluoromethoxy)-6-fluoroquinoline-3-carboxamide;
Ethyl 2-(4-(7-(difluoromethoxy)-6-fluoroquinoline-3-carboxamido)piperidin-1-yl)oxazole-5-carboxylate;
7-(Difluoromethoxy)-N-((1S,3r)-3-(((S)-1,1-difluoropropan-2-yl)amino)cyclobutyl)-6-fluoroquinoline-3-carboxamide;
7-(Difluoromethoxy)-N-((1R,3r)-3-(((R)-1,1-difluoropropan-2-yl)amino)cyclobutyl)-6-fluoroquinoline-3-carboxamide;
7-(Difluoromethoxy)-N-(*trans*-4-(((1,1-difluoropropan-2-yl)amino)methyl)cyclohexyl)-6-fluoroquinoline-3-carboxamide;
7-(Difluoromethoxy)-6-fluoro-N-(trans-4-(3,3,3-trifluoro-2-hydroxy-2-methylpropoxy)cyclohexyl)quinoline-3-carboxamide;
7-(Difluoromethoxy)-6-fluoro-N-(*trans*-4-(((R)-1,1,1-trifluoro-3-hydroxypropan-2-yl)amino)cyclohexyl)quinoline-3-carboxamide;
(S)-N-(1-(Cyclopentylmethyl)-2-oxopyrrolidin-3-yl)-7-(difluoromethoxy)-6-fluoroquinoline-3-carboxamide;
7-(Difluoromethoxy)-6-fluoro-N-(trans-4-(2-hydroxy-3-methylbutoxy)cyclohexyl)quinoline-3-carboxamide;
(S)-Ethyl 2-(3-(7-(difluoromethoxy)-6-fluoroquinoline-3-carboxamido)pyrrolidin-1-yl)oxazole-5-carboxylate;
7-(Difluoromethoxy)-N-((1r,4r)-4-ethyl-4-hydroxycyclohexyl)-6-fluoroquinoline-3-carboxamide;
Ethyl 2-(3-(7-(difluoromethoxy)-6-fluoroquinoline-3-carboxamido)azetidin-1-yl)oxazole-4-carboxylate;
Ethyl 2-((trans-3-(7-(difluoromethoxy)-6-fluoroquinoline-3-carboxamido)cyclobutyl)amino)oxazole-5-carboxylate;
Ethyl 2-(3-(7-(difluoromethoxy)-6-fluoroquinoline-3-carboxamido)azetidin-1-yl)oxazole-5-carboxylate;
7-(Difluoromethoxy)-6-fluoro-N-(*trans*-4-(((S)-1,1,1-trifluoro-3-hydroxypropan-2-yl)amino)cyclohexyl)quinoline-3-carboxamide
*trans*-Methyl 4-(7-(difluoromethoxy)-6-fluoroquinoline-3-carboxamido)cyclohexanecarboxylate;

N-(*trans*-4-Cyanocyclohexyl)-7-(difluoromethoxy)-6-fluoroquinoline-3-carboxamide;
Ethyl 2-(*trans*-4-(7-(difluoromethoxy)-6-fluoroquinoline-3-carboxamido)cyclohexyl)acetate;
7-(Difluoromethoxy)-6-fluoro-N-(*cis*-4-(3-fluoroazetidin-1-yl)cyclohexyl)quinoline-3-carboxamide;
7-(Difluoromethoxy)-6-fluoro-N-(*trans*-4-(3-fluoroazetidin-1-yl)cyclohexyl)quinoline-3-carboxamide;
7-(Diflluoromethoxy)-6-flluoro-N-(*cis*-4-(3-hydroxy-3-(trifluoromethyl)azetidin-1-yl)cyclohexyl)quinoline-3-carboxamide;
7-(Diflluoromethoxy)-6-flluoro-N-(*trans*-4-(3-hydroxy-3-(trifluoromethyl)azetidin-1-yl)cyclohexyl)quinoline-3-carboxamide;
7-(Difluoromethoxy)-6-fluoro-N-((1s,3s)-3-hydroxy-3-methylcyclobutyl)quinoline-3-carboxamide;
7-(Difluoromethoxy)-6-fluoro-N-(trans-4-(2-(methylsulfonyl)ethoxy)cyclohexyl)quinoline-3-carboxamide;
7-(Difluoromethoxy)-6-fluoro-N-(1-(pyridin-2-yl)piperidin-4-yl)quinoline-3-carboxamide;
7-(Difluoromethoxy)-6-fluoro-N-(1-(pyridin-3-yl)piperidin-4-yl)quinoline-3-carboxamide;
N-(1-(5-Cyanooxazol-2-yl)piperidin-4-yl)-7-(difluoromethoxy)-6-fluoroquinoline-3-carboxamide;
N-(1-(5-Cyanopyridin-2-yl)azetidin-3-yl)-7-(difluoromethoxy)-6-fluoroquinoline-3-carboxamide;
7-(Difluoromethoxy)-N-(*trans*-4-((((R)-1,1-difluoropropan-2-yl)amino)methyl)cyclohexyl)-6-fluoroquinoline-3-carboxamide;
7-(Difluoromethoxy)-N-(*trans*-4-((((S)-1,1-difluoropropan-2-yl)amino)methyl)cyclohexyl)-6-fluoroquinoline-3-carboxamide;
7-(Difluoromethoxy)-6-fluoro-N-(trans-4-((prop-2-yn-1-yloxy)methyl)cyclohexyl)quinoline-3-carboxamide;
7-(Difluoromethoxy)-6-fluoro-N-(trans-4-(2-oxo-2-(prop-2-yn-1-ylamino)ethoxy)cyclohexyl)quinoline-3-carboxamide;
N-(trans-4-(2-(But-3-yn-1-ylamino)-2-oxoethoxy)cyclohexyl)-7-(difluoromethoxy)-6-fluoroquinoline-3-carboxamide;
(S)-N-(2-Oxopyrrolidin-3-yl)-7-(trifluoromethoxy)quinoline-3-carboxamide;
8-Ethyl-N-(thiazol-2-yl)quinoline-3-carboxamide;
6-Chloro-N-(1-(1-methyl-1H-tetrazol-5-yl)piperidin-4-yl)quinoline-3-carboxamide;
N-(1-(1-Methyl-1*H*-tetrazol-5-yl)piperidin-4-yl)-7-(trifluoromethyl)quinoline-3-carboxamide;
7-Bromo-N-(1-(1-methyl-1*H*-tetrazol-5-yl)piperidin-4-yl)quinoline-3-carboxamide;
7-Chloro-N-(1-(1-methyl-1*H*-tetrazol-5-yl)piperidin-4-yl)quinoline-3-carboxamide;
7-Ethyl-N-(1-(1-methyl-1*H*-tetrazol-5-yl)piperidin-4-yl)quinoline-3-carboxamide;
N-(1-(1-Methyl-1H-tetrazol-5-yl)piperidin-4-yl)-2,3-dihydro-[1,4]dioxino[2,3-g]quinoline-8-carboxamide;
N-(1-(1-Methyl-1 H-tetrazol-5-yl)piperidin-4-yl)-2,3-dihydrofuro[3,2-h]quinoline-7-carboxamide;
N-(trans-4-(2-Hydroxypropan-2-yl)cyclohexyl)-2,3-dihydrofuro[3,2-h]quinoline-7-carboxamide;
6-Cyano-7-methoxy-N-(1-(1-methyl-1*H*-tetrazol-5-yl)piperidin-4-yl)quinoline-3-carboxamide;
7-Isopropoxy-N-(1-(1-methyl-1*H*-tetrazol-5-yl)piperidin-4-yl)quinoline-3-carboxamide;
(6-Chloro-7-(difluoromethoxy)-N-(trans-4-(2-hydroxypropan-2-yl)cyclohexyl)quinoline-3-carboxamide;
(S)-6-Chloro-7-(difluoromethoxy)-N-(2-oxopyrrolidin-3-yl)quinoline-3-carboxamide;
6-Chloro-7-(difluoromethoxy)-N-(trans-3-(2-hydroxypropan-2-yl)cyclobutyl)quinoline-3-carboxamide;
(S)-6-Chloro-7-(difluoromethoxy)-N-(1-isopropyl-2-oxopyrrolidin-3-yl)quinoline-3-carboxamide;
(S)-6-Chloro-N-(1-(cyclopropylmethyl)-2-oxopyrrolidin-3-yl)-7-(difluoromethoxy)quinoline-3-carboxamide;
6-Chloro-7-(difluoromethoxy)-N-(trans-4-(1-hydroxycyclopropyl)cyclohexyl)quinoline-3-carboxamide;
6-Chloro-7-(difluoromethoxy)-N-(trans-4-(2-hydroxy-2-methylpropoxy)cyclohexyl)q uinoline-3-carboxamide;
6-Chloro-7-(difluoromethoxy)-N-(trans-4-((1-hydroxycyclopropyl)methoxy)cyclohexyl)quinoline-3-carboxamide;
6-Chloro-7-(difluoromethoxy)-N-((3*r*,6*s*)-6-(2-hydroxypropan-2-yl)tetrahydro-2H-pyran-3-yl)quinoline-3-carboxamide;
6-Chloro-7-(difluoromethoxy)-N-(*trans*-4-((1,1-difluoropropan-2-yl)amino)cyclohexyl)quinoline-3-carboxamide;
6-Chloro-7-(difluoromethoxy)-N-((3*r*,6*s*)-6-(dimethylcarbamoyl)tetrahydro-2H-pyran-3-yl)quinoline-3-carboxamide;
6-Chloro-7-(difluoromethoxy)-N-(trans-4-(2-hydroxy-3-methylbutoxy)cyclohexyl)quinoline-3-carboxamide;
6-Chloro-N-(3-cyclopropyl-1-methyl-1H-pyrazol-5-yl)-7-(difluoromethoxy)quinoline-3-carboxamide;
6-Chloro-7-(difluoromethoxy)-N-(*trans*-4-(((R)-1,1-difluoropropan-2-yl)amino)cyclohexyl)quinoline-3-carboxamide;
6-Chloro-7-(difluoromethoxy)-N-(*trans*-4-(((S)-1,1-difluoropropan-2-yl)amino)cyclohexyl)quinoline-3-carboxamide;
6-Chloro-7-(difluoromethoxy)-N-(*trans*-4-(((S)-1,1,1-trifluoro-3-hydroxypropan-2-yl)amino)cyclohexyl)quinoline-3-carboxamide;
6-Chloro-7-(difluoromethoxy)-N-(*trans*-4-(3-fluoroazetidin-1-yl)cyclohexyl)quinoline-3-carboxamide;
6-Chloro-7-(difluoromethoxy)-N-((3S)-5-methyl-2-oxopyrrolidin-3-yl)quinoline-3-carboxamide;
6-Chloro-7-(difluoromethoxy)-N-(*trans*-4-(3-hydroxy-3-(trifluoromethyl)azetidin-1-yl)cyclohexyl)quinoline-3-carboxamide;
6-Chloro-7-(difluoromethoxy)-N-(*trans*-4-(((R)-1,1,1-trifluoro-3-hydroxypropan-2-yl)amino)cyclohexyl)quinoline-3-carboxamide;

N-(trans-4-(2-Amino-2-oxoethoxy)cyclohexyl)-6-chloro-7-(difluoromethoxy)quinoline-3-carboxamide;
6-Chloro-7-(difluoromethoxy)-N-((1r,3r)-3-hydroxy-3-methylcyclobutyl)quinoline-3-carboxamide;
6-Chloro-7-(difluoromethoxy)-N-((1s,3s)-3-hydroxy-3-methylcyclobutyl)quinoline-3-carboxamide;
6-Chloro-7-(difluoromethoxy)-N-(trans-4-(2-(methylsulfonyl)ethoxy)cyclohexyl)quinoline-3-carboxamide;
6-Chloro-7-(difluoromethoxy)-N-(*trans*-3-hydroxycyclobutyl)quinoline-3-carboxamide;
trans-4-(6-Chloro-7-(difluoromethoxy)quinoline-3-carboxamido)cyclohexyl sulfamate;
6-Chloro-7-(difluoromethoxy)-N-(3-oxocyclobutyl)quinoline-3-carboxamide;
6-Chloro-7-(difluoromethoxy)-N-((1r,4r)-4-hydroxy-4-methylcyclohexyl)quinoline-3-carboxamide;
6-Chloro-7-(difluoromethoxy)-N-((3S,4R)-4-methyl-2-oxopyrrolidin-3-yl)quinoline-3-carboxamide;
6-Chloro-7-(difluoromethoxy)-N-(3-hydroxy-3-methylcyclopentyl)quinoline-3-carboxamide;
6-Chloro-7-(difluoromethoxy)-N-((1S,3S)-3-hydroxy-3-methylcyclopentyl)quinoline-3-carboxamide;
6-Chloro-7-(difluoromethoxy)-N-((1s,4s)-4-hydroxy-4-methylcyclohexyl)quinoline-3-carboxamide;
6-Chloro-7-(difluoromethoxy)-N-(tetrahydro-2H-pyran-4-yl)quinoline-3-carboxamide;
6-Chloro-7-(difluoromethoxy)-N-(tetrahydro-2H-pyran-3-yl)quinoline-3-carboxamide;
6-Chloro-7-(difluoromethoxy)-N-(2,2-dimethyltetrahydro-2H-pyran-4-yl)quinoline-3-carboxamide;
7-(2,2-Difluorocyclopropyl)-6-fluoro-N-(trans-4-(2-hydroxypropan-2-yl)cyclohexyl)quinoline-3-carboxamide;
6-Bromo-7-(difluoromethoxy)-N-(*trans*-4-(2-hydroxypropan-2-yl)cyclohexyl)quinoline-3-carboxamide;
6-Bromo-7-(difluoromethoxy)-N-(*trans*-4-(((S)-1,1,1-trifluoro-3-hydroxypropan-2-yl)amino)cyclohexyl)quinoline-3-carboxamide;
(S)-6-Bromo-7-(difluoromethoxy)-N-(2-oxopyrrolidin-3-yl)quinoline-3-carboxamide;
6-Bromo-7-(difluoromethoxy)-N-(*trans*-4-(((R)-1,1,1-trifluoro-3-hydroxypropan-2-yl)amino)cyclohexyl)quinoline-3-carboxamide;
6-Bromo-7-(difluoromethoxy)-N-(*trans*-4-((1,1-difluoropropan-2-yl)amino)cyclohexyl)quinoline-3-carboxamide;
6-Bromo-7-(difluoromethoxy)-N-((1s,3s)-3-hydroxy-3-methylcyclobutyl)quinoline-3-carboxamide;
6-Bromo-7-(difluoromethoxy)-N-(*trans*-4-(((R)-1,1-difluoropropan-2-yl)amino)cyclohexyl)quinoline-3-carboxamide;
6-Bromo-7-(difluoromethoxy)-N-(*trans*-4-(((S)-1,1-difluoropropan-2-yl)amino)cyclohexyl)quinoline-3-carboxamide;
7-(Dimethylamino)-6-fluoro-N-(*trans*-4-(2-hydroxypropan-2-yl)cyclohexyl)quinoline-3-carboxamide;
6-Fluoro-N-(*trans*-4-(2-hydroxypropan-2-yl)cyclohexyl)-7-(methylthio)quinoline-3-carboxamide;
7-(Azetidin-1-yl)-6-fluoro-N-(*trans*-4-(2-hydroxypropan-2-yl)cyclohexyl)quinoline-3-carboxamide;
6-Fluoro-N-(*trans*-4-(2-hydroxypropan-2-yl)cyclohexyl)-7-(pyrrolidin-1-yl)quinoline-3-carboxamide;
6-Fluoro-7-(3-fluoroazetidin-1-yl)-N-(*trans*-4-(2-hydroxypropan-2-yl)cyclohexyl)quinoline-3-carboxamide;
6-Fluoro-7-(3-fluoro-3-methylazetidin-1-yl)-N-(trans-4-(2-hydroxypropan-2-yl)cyclohexyl)quinoline-3-carboxamide;
(*S*)-7-(Azetidin-1-yl)-6-chloro-N-(2-oxopyrrolidin-3-yl)quinoline-3-carboxamide;
7-(Azetidin-1-yl)-6-chloro-N-(3-hydroxy-3-methylcyclobutyl)quinoline-3-carboxamide;
7-Azido-6-chloro-N-(trans-4-(2-hydroxypropan-2-yl)cyclohexyl)quinoline-3-carboxamide;
(S)-6-Chloro-7-(3-fluoroazetidin-1-yl)-N-(2-oxopyrrolidin-3-yl)quinoline-3-carboxamide;
6-Fluoro-N-(*trans*-4-(2-hydroxypropan-2-yl)cyclohexyl)-7-(2-methylazetidin-1-yl)quinoline-3-carboxamide;
6-Fluoro-7-(2-methylazetidin-1-yl)-N-((S)-2-oxopyrrolidin-3-yl)quinoline-3-carboxamide;
7-Acetyl-6-chloro-N-(trans-4-(2-hydroxypropan-2-yl)cyclohexyl)quinoline-3-carboxamide;
7-Amino-6-fluoro-N-(*trans*-4-(2-hydroxypropan-2-yl)cyclohexyl)quinoline-3-carboxamide;
6-Fluoro-N-(trans-4-(2-hydroxypropan-2-yl)cyclohexyl)-7-((S)-2-methylazetidin-1-yl)quinoline-3-carboxamide;
6-Fluoro-N-(trans-4-(2-hydroxypropan-2-yl)cyclohexyl)-7-((R)-2-methylazetidin-1-yl)quinoline-3-carboxamide;
6-Fluoro-7-((S)-2-methylazetidin-1-yl)-N-((S)-2-oxopyrrolidin-3-yl)quinoline-3-carboxamide;
7-(3,3-Difluoroazetidin-1-yl)-6-fluoro-N-(*trans*-4-(2-hydroxypropan-2-yl)cyclohexyl)quinoline-3-carboxamide;
7-Ethoxy-6-fluoro-N-(*trans*-4-(2-hydroxypropan-2-yl)cyclohexyl)quinoline-3-carboxamide;
6-Chloro-7-cyclobutyl-N-(trans-4-(2-hydroxypropan-yl)cyclohexyl)quinoline-3-carboxamide;
6-Chloro-7-cyclobutyl-N-((1s,3s)-3-hydroxy-3-methylcyclobutyl)quinoline-3-carboxamide;
(S)-6-Chloro-7-cyclobutyl-N-(2-oxopyrrolidin-3-yl)quinoline-3-carboxamide;
6-Chloro-7-(2-methylazetidin-1-yl)-N-((S)-2-oxopyrrolidin-3-yl)quinoline-3-carboxamide;
6-Fluoro-N-(*trans*-4-(2-hydroxypropan-2-yl)cyclohexyl)-7-(3-methylazetidin-1-yl)quinoline-3-carboxamide;
7-((Difluoromethyl)thio)-6-fluoro-N-(*trans*-4-(2-hydroxypropan-2-yl)cyclohexyl)quinoline-3-carboxamide;
7-((Difluoromethyl)sulfinyl)-6-fluoro-N-(*trans*-4-(2-hydroxypropan-2-yl)cyclohexyl)quinoline-3-carboxamide;
N-(trans-4-(2-Hydroxypropan-2-yl)cyclohexyl)-7-methylquinoline-3-carboxamide;
6-Chloro-N-(trans-4-(2-hydroxypropan-2-yl)cyclohexyl)-7-((S)-2-methylazetidin-1-yl)quinoline-3-carboxamide;
6-Chloro-7-((S)-2-methylazetidin-1-yl)-N-((S)-2-oxopyrrolidin-3-yl)quinoline-3-carboxamide;
N-(trans-4-(2-Hydroxypropan-2-yl)cyclohexyl)-8-methoxyquinoline-3-carboxamide;
N-(trans-4-(2-Hydroxypropan-2-yl)cyclohexyl)-7-(trifluoromethyl)quinoline-3-carboxamide;
7-Bromo-N-(trans-4-(2-hydroxypropan-2-yl)cyclohexyl)quinoline-3-carboxamide;

7-Cyclopropyl-N-(trans-4-(2-hydroxypropan-2-yl)cyclohexyl)quinoline-3-carboxamide;
7-Fluoro-N-(trans-4-(2-hydroxypropan-2-yl)cyclohexyl)quinoline-3-carboxamide;
7-(Azetidin-1-yl)-N-(trans-4-(2-hydroxypropan-2-yl)cyclohexyl)quinoline-3-carboxamide;
N-(trans-4-(2-Hydroxypropan-2-yl)cyclohexyl)-[1,3]dioxolo[4,5-g]quinoline-7-carboxamide;
7-(Dimethylamino)-N-(trans-4-(2-hydroxypropan-2-yl)cyclohexyl)quinoline-3-carboxamide;
7-Cyclobutyl-N-(trans-4-(2-hydroxypropan-2-yl)cyclohexyl)quinoline-3-carboxamide;
N-((1r,4r)-4-Hydroxy-4-methylcyclohexyl)-7-methoxyquinoline-3-carboxamide;
N-(6-(2-Hydroxypropan-2-yl)spiro[3.3]heptan-2-yl)-7-methoxyquinoline-3-carboxamide;
N-(trans-4-(2-Hydroxy-2-methylpropoxy)cyclohexyl)-7-methoxyquinoline-3-carboxamide;
7-Methoxy-N-(trans-4-((2,2,2-trifluoroethyl)amino)cyclohexyl)quinoline-3-carboxamide;
8-Fluoro-N-(trans-4-(2-hydroxypropan-2-yl)cyclohexyl)-7-methoxyquinoline-3-carboxamide;
6,7-Dichloro-N-(trans-4-(2-hydroxypropan-2-yl)cyclohexyl)quinoline-3-carboxamide;
N-((1s,3s)-3-Hydroxy-3-methylcyclobutyl)-7-methoxyquinoline-3-carboxamide;
N-((1r,3r)-3-Hydroxy-3-methylcyclobutyl)-7-methoxyquinoline-3-carboxamide;
7-Chloro-N-(trans-4-(2-hydroxypropan-2-yl)cyclohexyl)quinoline-3-carboxamide;
6-Chloro-N-((3S,4R)-4-methyl-2-oxopyrrolidin-3-yl)-7-((S)-2-methylazetidin-1-yl)quinoline-3-carboxamide;
6-Fluoro-7-(3-fluoroazetidin-1-yl)-N-((3S,4R)-4-methyl-2-oxopyrrolidin-3-yl)quinoline-3-carboxamide;
6-Fluoro-N-((3S,4R)-4-methyl-2-oxopyrrolidin-3-yl)-7-(3-methylazetidin-1-yl)quinoline-3-carboxamide;
trans-4-(6-Chloro-7-(difluoromethoxy)quinoline-3-carboxamido)-N,N-dimethylcyclohexanamine oxide;
6-Chloro-7-fluoro-N-((trans)-4-(2-hydroxypropan-2-yl)cyclohexyl)quinoline-3-carboxamide;
6-Chloro-7-(difluoromethoxy)-N-((trans)-4-(morpholine-4-carbonyl)cyclohexyl)quinoline-3-carboxamide;
6-Chloro-7-(difluoromethoxy)-N-((trans)-4-(4-methylpiperazine-1-carbonyl)cyclohexyl)quinoline-3-carboxamide;
6-Chloro-N-((trans)-4-(2-hydroxypropan-2-yl)cyclohexyl)-7-(isopropylamino)quinoline-3-carboxamide;
6-Chloro-7-(cyclopropylamino)-N-((trans)-4-(2-hydroxypropan-2-yl)cyclohexyl)quinoline-3-carboxamide;
6-Chloro-7-(difluoromethoxy)-N-((trans)-3-(3-hydroxy-3-(trifluoromethyl)azetidin-1-yl)cyclobutyl)quinoline-3-carboxamide;
8-Chloro-N-((*trans*)-4-(2-hydroxypropan-2-yl)cyclohexyl)-7-(trifluoromethoxy)quinoline-3-carboxamide;
6-Chloro-N-((*trans*)-4-(2-hydroxypropan-2-yl)cyclohexyl)-7-(trifluoromethoxy)quinoline-3-carboxamide;
8-Chloro-7-(difluoromethoxy)-N-((*trans*)-4-(2-hydroxypropan-2-yl)cyclohexyl)quinoline-3-carboxamide;
6,8-Dichloro-7-(difluoromethoxy)-N-((*trans*)-4-(2-hydroxypropan-2-yl)cyclohexyl)quinoline-3-carboxamide;
8-Chloro-7-ethoxy-N-(trans-4-(2-hydroxypropan-2-yl)cyclohexyl)quinoline-3-carboxamide;
6-Chloro-7-(difluoromethoxy)-N-(1-(2-hydroxy-2-methylpropanoyl)piperidin-4-yl)quinoline-3-carboxamide;
6-Chloro-7-(difluoromethoxy)-N-(1-(2-hydroxy-2-methylpropanoyl)azetidin-3-yl)quinoline-3-carboxamide;
6-Chloro-7-(2,2-difluorocyclopropyl)-N-((trans)-4-(2-hydroxypropan-2-yl)cyclohexyl)quinoline-3-carboxamide;
6-Chloro-7-(difluoromethoxy)-N-((cis)-4-hydroxy-4-(trifluoromethyl)cyclohexyl)quinoline-3-carboxamide;
6-Chloro-7-(difluoromethoxy)-N-(4-(2-hydroxy-2-methylpropanoyl)piperazin-1-yl)quinoline-3-carboxamide;
6-Chloro-N-((trans)-4-(2-hydroxypropan-2-yl)cyclohexyl)-7-vinylquinoline-3-carboxamide;
6-Chloro-7-(difluoromethoxy)-N-(trans-3-(2-hydroxy-2-methylpropanamido)cyclobutyl)quinoline-3-carboxamide;
6-Chloro-7-(difluoromethoxy)-N-((trans)-4-hydroxy-4-(trifluoromethyl)cyclohexyl)quinoline-3-carboxamide;
8-Chloro-7-(difluoromethoxy)-N-((*trans*)-4-(2-hydroxypropan-2-yl)cyclohexyl)-6-methylquinoline-3-carboxamide;
6-Chloro-7-(difluoromethoxy)-N-((*trans*)-4-(2-hydroxypropan-2-yl)cyclohexyl)-8-methylquinoline-3-carboxamide;
6-Chloro-7-(2-fluoropropan-2-yl)-N-((trans)-4-(2-hydroxypropan-2-yl)cyclohexyl)quinoline-3-carboxamide;
6-Chloro-7-(1,1-difluoroethyl)-N-((trans)-4-(2-hydroxypropan-2-yl)cyclohexyl)quinoline-3-carboxamide;
6-Chloro-7-(difluoromethoxy)-N-((1s,3s)-3-hydroxy-3-(trifluoromethyl)cyclobutyl)quinoline-3-carboxamide;
6-Chloro-7-(difluoromethoxy)-N-((1s,4s)-4-(difluoromethyl)-4-hydroxycyclohexyl)quinoline-3-carboxamide;
6-Chloro-7-(difluoromethoxy)-N-((1r,4r)-4-(difluoromethyl)-4-hydroxycyclohexyl)quinoline-3-carboxamide;
8-Chloro-7-(difluoromethoxy)-6-fluoro-N-(trans-4-(2-hydroxypropan-2-yl)cyclohexyl)quinoline-3-carboxamide;
6-Chloro-7-(difluoromethoxy)-8-fluoro-N-(trans-4-(2-hydroxypropan-2-yl)cyclohexyl)quinoline-3-carboxamide; and
6-Chloro-7-(difluoromethoxy)-N-(2-oxo-2,3,4,5-tetrahydro-1H-benzo[b]azepin-3-yl)quinoline-3-carboxamide;
and salts thereof including pharmaceutically acceptable salts thereof.

The skilled artisan will appreciate that salts, including pharmaceutically acceptable salts, of the compounds according to Formula (XI) may be prepared. Indeed, in certain embodiments of the invention, salts including pharmaceutically-acceptable salts of the compounds according to Formula (XI) may be preferred over the respective free or unsalted compound. Accordingly, the invention is further directed to salts, including pharmaceutically-acceptable salts, of the compounds according to Formula X(I).

The salts, including pharmaceutically acceptable salts, of the compounds of the invention are readily prepared by those of skill in the art.

Representative pharmaceutically acceptable acid addition salts include, but are not limited to, 4-acetamidobenzoate, acetate, adipate, alginate, ascorbate, aspartate, benzenesulfonate (besylate), benzoate, bisulfate, bitartrate, butyrate, calcium edetate, camphorate, camphorsulfonate (camsylate), caprate (decanoate), caproate (hexanoate), caprylate (octanoate), cinnamate, citrate, cyclamate, digluconate, 2,5-dihydroxybenzoate, disuccinate, dodecylsulfate (estolate), edetate (ethylenediaminetetraacetate), estolate (lauryl sulfate), ethane-1,2-disulfonate (edisylate), ethanesulfonate (esylate), formate, fumarate, galactarate (mucate), gentisate (2,5-dihydroxybenzoate), glucoheptonate (gluceptate), gluconate, glucuronate, glutamate, glutarate, glycerophosphorate, glycolate, hexylresorcinate, hippurate, hydrabamine (*N,N'*-di(dehydroabietyl)-ethylenediamine), hydrobromide, hydrochloride, hydroiodide, hydroxynaphthoate, isobutyrate, lactate, lactobionate, laurate, malate, maleate, malonate, mandelate, methanesulfonate (mesylate), methylsulfate, mucate, naphthalene-1,5-disulfonate (napadisylate), naphthalene-2-sulfonate (napsylate), nicotinate, nitrate, oleate, palmitate, *p*-aminobenzenesulfonate, *p*-aminosalicyclate, pamoate (embonate), pantothenate, pectinate, persulfate, phenylacetate, phenylethylbarbiturate, phosphate, polygalacturonate, propionate, *p*-toluenesulfonate (tosylate), pyroglutamate, pyruvate, salicylate, sebacate, stearate, subacetate, succinate, sulfamate, sulfate, tannate, tartrate, teoclate (8-chlorotheophyllinate), thiocyanate, triethiodide, undecanoate, undecylenate, and valerate.

Representative pharmaceutically acceptable base addition salts include, but are not limited to, aluminium, 2-amino-2-(hydroxymethyl)-1,3-propanediol (TRIS, tromethamine), arginine, benethamine (*N*-benzylphenethylamine), benzathine (*N,N'*-dibenzylethylenediamine), *bis*-(2-hydroxyethyl)amine, bismuth, calcium, chloroprocaine, choline, clemizole (1-*p* chlorobenzyl-2-pyrrolildine-1'-ylmethylbenzimidazole), cyclohexylamine, dibenzylethylenediamine, diethylamine, diethyltriamine, dimethylamine, dimethylethanolamine, dopamine, ethanolamine, ethylenediamine, L-histidine, iron, isoquinoline, lepidine, lithium, lysine, magnesium, meglumine (*N*-methylglucamine), piperazine, piperidine, potassium, procaine, quinine, quinoline, sodium, strontium, *t*-butylamine, and zinc.

The compounds according to Formula (XI) may contain one or more asymmetric centers (also referred to as a chiral center) and may, therefore, exist as individual enantiomers, diastereomers, or other stereoisomeric forms, or as mixtures thereof. Chiral centers, such as chiral carbon atoms, may be present in a substituent such as an alkyl group. Where the stereochemistry of a chiral center present in a compound of Formula (XI), or in any chemical structure illustrated herein, if not specified the structure is intended to encompass all individual stereoisomers and all mixtures thereof. Thus, compounds according to Formula (XI) containing one or more chiral centers may be used as racemic mixtures, enantiomerically enriched mixtures, or as enantiomerically pure individual stereoisomers.

The compounds according to Formula (XI) may also contain double bonds or other centers of geometric asymmetry. Where the stereochemistry of a center of geometric asymmetry present in Formula (XI), or in any chemical structure illustrated herein, is not specified, the structure is intended to encompass the trans (E) geometric isomer, the cis (Z) geometric isomer, and all mixtures thereof. Likewise, all tautomeric forms are also included in Formula (XI) whether such tautomers exist in equilibrium or predominately in one form.

The compounds of Formula (XI) or salts, including pharmaceutically acceptable salts, thereof may exist in solid or liquid form. In the solid state, the compounds of the invention may exist in crystalline or noncrystalline form, or as a mixture thereof. For compounds of the invention that are in crystalline form, the skilled artisan will appreciate that pharmaceutically acceptable solvates may be formed wherein solvent molecules are incorporated into the crystalline lattice during crystallization. Accordingly, the compounds of Formula (XI) and pharmaceutically acceptable salts thereof may exist in solvated and unsolvated forms.

The skilled artisan will further appreciate that certain compounds of Formula (XI) or salts, including pharmaceutically acceptable salts thereof that exist in crystalline form, including the various solvates thereof, may exhibit polymorphism (i.e. the capacity to occur in different crystalline structures). These different crystalline forms are typically known as "polymorphs." Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. The skilled artisan will appreciate that different polymorphs may be produced, for example, by changing or adjusting the reaction conditions or reagents, used in making the compound. For example, changes in temperature, pressure, or solvent may result in polymorphs. In addition, one polymorph may spontaneously convert to another polymorph under certain conditions. Accordingly, the compounds of Formula (XI) and pharmaceutically acceptable salts thereof may exist in a single crystalline form or in different polymorphic forms.

As used herein, when referring to Formula (XI) it will be appreciated that any one of the other Formulas are also referenced unless the context dictates otherwise.

## Definitions

It will be appreciated that the following definitions apply to each of the aforementioned formulae and to all instances of these terms, unless the context dictates otherwise.

**"Alkyl"** refers to a hydrocarbon chain having the specified number of "member atoms". For example, $C_1$-$C_6$alkyl and $C_{1-6}$alkyl refers to an alkyl group having from 1 to 6 member atoms. Alkyl groups may be saturated, unsaturated, straight or branched. Representative branched alkyl groups have one, two, or three branches. Alkyl includes methyl, ethyl, ethylene, propyl (n-propyl and isopropyl), butene, butyl (n-butyl, isobutyl, and t-butyl), pentyl and hexyl.

**"Alkoxy"** refers to an -O-alkyl group wherein "alkyl" is as defined herein. For example, $C_1$-$C_4$alkoxy refers to an alkoxy group having from 1 to 4 member atoms. Representative branched alkoxy groups have one, two, or three branches. Examples of such groups include methoxy, ethoxy, propoxy, and butoxy.

**"Cycloalkyl",** unless otherwise defined, refers to a saturated or unsaturated non aromatic hydrocarbon ring system having from three to seven carbon atoms. Cycloalkyl groups are monocyclic or bicyclic ring systems. For example, $C_3$-$C_7$ cycloalkyl refers to a cycloalkyl group having from 3 to 7 member atoms. Examples of cycloalkyl as used herein include: cyclopropyl, cyclobutyl, cyclopentyl, cyclohexyl, cyclobutenyl, cyclopentenyl, cyclohexenyl, cycloheptyl and spiro heptane. Suitably cycloalkyl is selected from: cyclopropyl, cyclobutyl, cyclopentyl, cyclohexyl and spiro heptane.

**"Halogen"**refers to the halogen radicals fluoro, chloro, bromo, and iodo.

**"Heteroaryl"** refers to a monocyclic or bicyclic aromatic 5 to 10 member ring system containing from 1 to 7 carbon atoms and containing from 1 to 4 heteroatoms, provided that when the number of carbon atoms is 3, the aromatic ring contains at least two heteroatoms. When the heteroaryl is bicyclic, at least one ring is aromatic. Heteroaryl groups containing more than one heteroatom may contain different heteroatoms. Heteroaryl includes: pyrrolyl, pyrazolyl, imidazolyl, oxazolyl, isoxazolyl, thiazolyl, isothiazolyl, furanyl, furazanyl, thienyl, triazolyl, pyridinyl, pyrimidinyl, pyridazinyl, pyrazinyl, triazinyl, tetrazinyl, tetrazolyl, tetrahydrobenzazepinyl, tetrahydroquinolinyl. Suitably, "heteroaryl" is selected from: pyrazolyl, thiazolyl, tetrazolyl, tetrahydrobenzazepinyl, and tetrahydroquinolinyl.

**"Heterocycloalkyl"** refers to a saturated or unsaturated non-aromatic monocyclic ring system containing 4 to 6 member atoms, of which 1 to 5 are carbon atoms and from 1 to 4 are heteroatoms. Heterocycloalkyl groups containing more than one heteroatom may contain different heteroatoms. Heterocycloalkyl includes: pyrrolidinyl, tetrahydrofuranyl, dihydrofuranyl, pyranyl, tetrahydropyranyl, dihydropyranyl, tetrahydrothienyl, pyrazolidinyl, oxazolidinyl, oxetanyl, thiazolidinyl, piperidinyl, homopiperidinyl, piperazinyl, morpholinyl, thiamorpholinyl, 1,3-dioxolanyl, 1,3-dioxanyl, 1,4-dioxanyl, 1,3-oxathiolanyl, 1,3-oxathianyl, 1,3-dithianyl, and azetidinyl. Suitably, "heterocycloalkyl" is selected from: pyrrolidinyl, tetrahydropyranyl, oxazolidinyl, morpholinyl, piperidinyl, piperazinyl, and azetidinyl.

**"Heteroatom"** refers to a nitrogen, sulphur or oxygen atom.

## COMPOUND PREPARATION

The compounds according to Formula (XI) are prepared using conventional organic synthetic methods. A suitable synthetic route is depicted below in the following general reaction schemes. All of the starting materials are commercially available or are readily prepared from commercially available starting materials by those of skill in the art.

The skilled artisan will appreciate that if a substituent described herein is not compatible with the synthetic methods described herein, the substituent may be protected with a suitable protecting group that is stable to the reaction conditions. The protecting group may be removed at a suitable point in the reaction sequence to provide a desired intermediate or target compound. Suitable protecting groups and the methods for protecting and de-protecting different substituents using such suitable protecting groups are well known to those skilled in the art; examples of which may be found in T. Greene and P. Wuts, Protecting Groups in Organic Synthesis (4th ed.), John Wiley & Sons, NY (2006). In some instances, a substituent may be specifically selected to be reactive under the reaction conditions used. Under these circumstances, the reaction conditions convert the selected substituent into another substituent that is either useful as an intermediate compound or is a desired substituent in a target compound.

As used in the Schemes, "r" groups represent corresponding groups on any of the aforementioned formulae.

In one method of preparation, the 3-quinoline carboxamides may be prepared from anilines as shown in Scheme 1. First, conjugation addition of suitable anilines to a $\alpha,\beta$-unsaturated diester in the Michael fashion provide vinylogous carbamates, which upon heating with phosphorus oxychloride give 4-chloro-3-quinoline esters. Subsequent removal of the 4-chloro moieties and ester hydrolyses afford 3-quinoline carboxylic acids. Then, coupling of the acids with suitable amines yield the desired 3-quinoline carboxamides.

Scheme 1

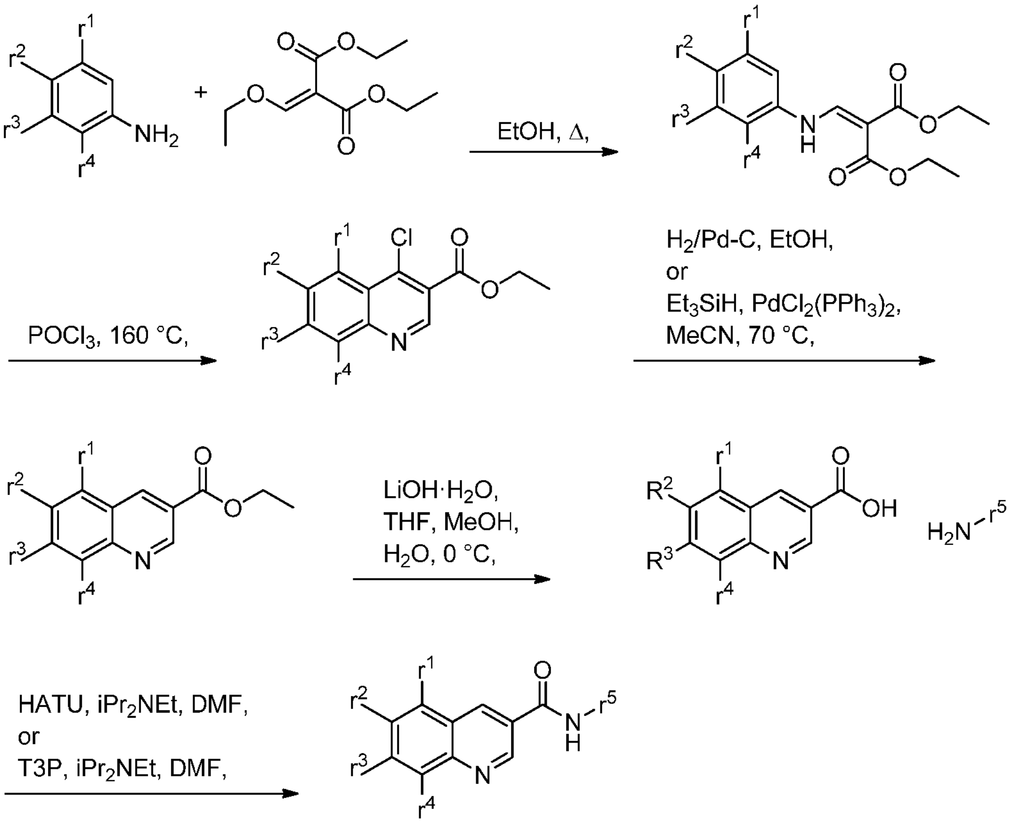


In an alternative method of preparation, the 3-quinoline carboxamides may be synthesized from aldehydes as shown in Scheme 2. First, nitration of suitable aromatic aldehydes provide the arylnitrates. Then, reduction to the anilines with tin(II) chloride, followed by addition to ethyl 3,3-diethoxypropanoate and intramolecular aldol condensation give 3-quinoline esters. Subsequent ester hydrolyses afford 3-quinoline carboxylic acids, which upon coupling to suitable amines yield the desired 3-quinoline carboxamides.

Scheme 2

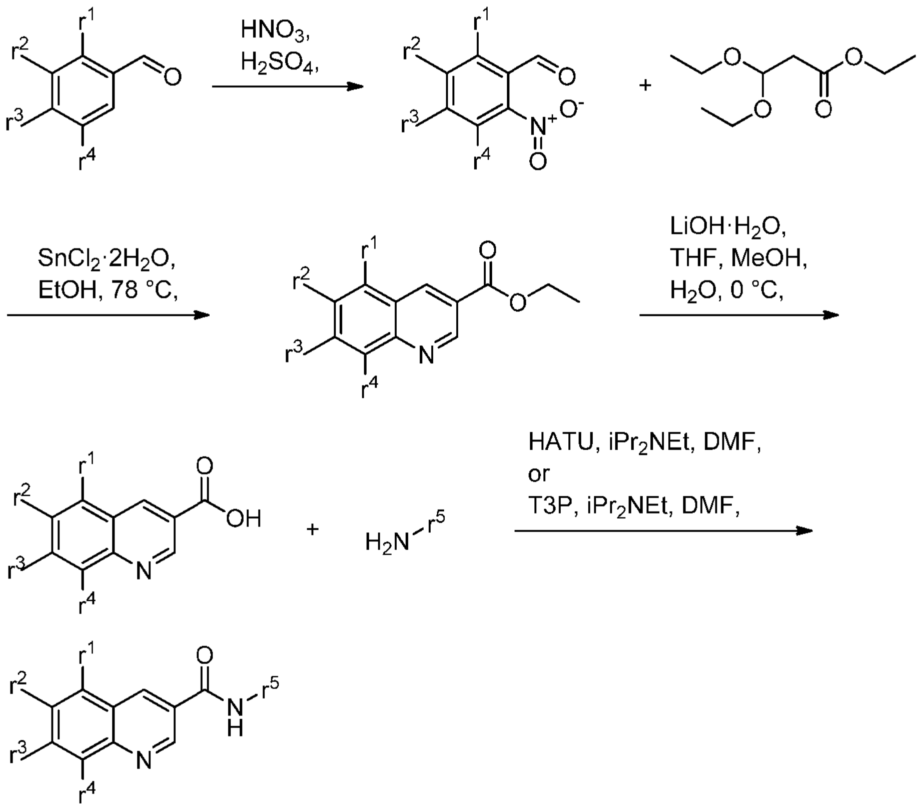


In another method of preparation, the 3-quinoline carboxamides may be derived from suitably protected 7-halo-3-quinolone carboxylates as shown in Scheme 3. After suitable protection, *ipso* deplacement of the 7-halo substituents by suitable nucleophiles afford the 7-substituted quinolones. Then, conversion to the 4-chloroquinolines and 4-chloro group removal provide the 3-quinoline esters. Subsequent ester hydrolyses afford 3-quinoline carboxylic acids, which upon coupling to suitable amines yield the desired 3-quinoline carboxamides.

Scheme 3

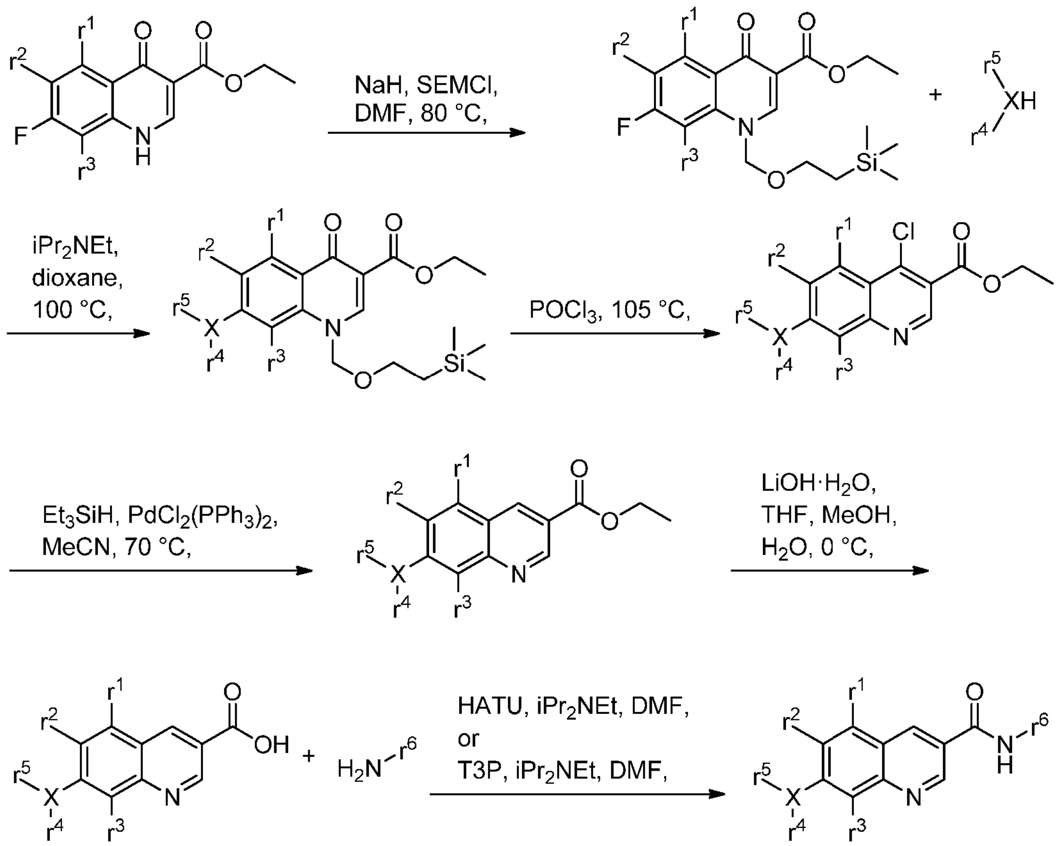


In an additional method of preparation, the 3-quinoline carboxamides may be made from 7-halo-3-quinoline carboxylates as shown in Scheme 4. First, *ipso* deplacement of the 7-halo substituents by suitable nucleophiles afford the 7-substituted quinolines. Then, ester hydrolyses provide 3-quinoline carboxylic acids, which upon coupling to suitable amines give the desired 3-quinoline carboxamides.

Scheme 4

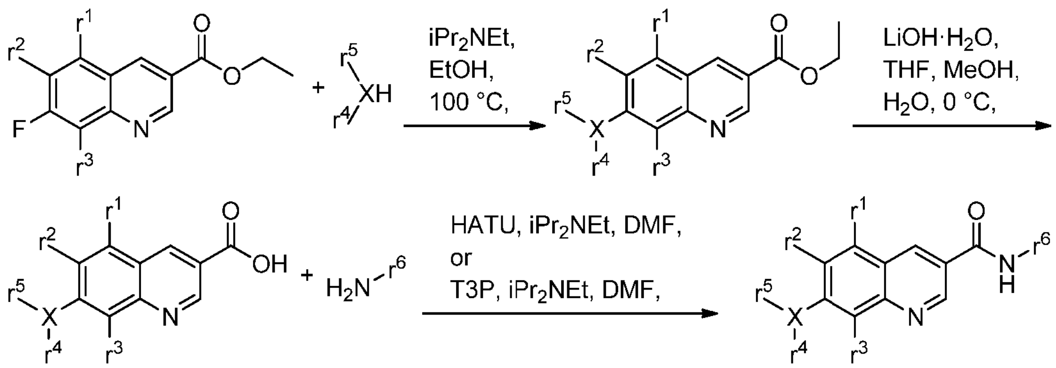


## METHODS OF USE

The inventors have shown that inhibitors of Hematopoietic Prostaglandin D Synthase (H-PGDS), reduce muscle damage and preserve muscle function when administered prior to muscle damage in an *in vivo* assay for muscle function. Furthermore, the inventors have shown that when an H-PGDS inhibitor is administered after muscle damage in the same assay, recovery of muscle function is enhanced. These results support a role for the use of H-PGDS inhibitors in the treatment of muscle degenerative disorders and muscle injury.

In one aspect, the invention provides a method of treating a muscle degenerative disorder comprising administering to a human an inhibitor of H-PGDS.

In one aspect, the invention provides a method of treating a muscle degenerative disorder comprising administering to a human an H-PGDS inhibitor of claim 1 or a pharmaceutically acceptable salt thereof.

In particular embodiments, the muscle degenerative disorder is muscular dystrophy, myotonic dystrophy, polymyositis, or dermatomyositis.

For example, the compounds of claim 1 or a pharmaceutically acceptable salt thereof may be used to treat a muscular dystrophy disorder selected from Duchenne MD, Becker MD, Congenital MD (Fukuyama), Emery Dreifuss MD, Limb girdle MD, and Fascioscapulohumeral MD.

The compounds of claim 1 or a pharmaceutically acceptable salt thereof may be used to treat myotonic dystrophy type I (DM1 or Steinert's), myotonic dystrophy type II (DM2 or proximal myotonic myopathy), or congenital myotonia.

In another aspect, the invention provides a method of treating a muscle injury comprising administering to a human an inhibitor of H-PGDS as defined in claim 1.

In some embodiments, the muscle injury is a surgery-related muscle injury, a traumatic muscle injury, a work-related skeletal muscle injury, or an overtraining-related muscle injury.

Non-limiting examples of surgery-related muscle injuries include muscle damage due to knee replacement, anterior cruciate ligament (ACL) repair, plastic surgery, hip replacement surgery, joint replacement surgery, tendon repair surgery, surgical repair of rotator cuff disease and injury, and amputation.

In one embodiment, the muscle injury is a surgery-related muscle injury and the treatment method provides for administration of at least one dose of an H-PGDS inhibitor of Formula (XI) or a pharmaceutically acceptable salt thereof prior to the surgery (for example, within one day before the surgery) followed by periodic administration of a dose of a H-PGDS inhibitor during the recovery period.

In another embodiment, the muscle injury is a surgery-related muscle injury and the treatment method provides for administration of at least one high dose of a H-PGDS inhibitor of Formula (XI) or a pharmaceutically acceptable salt thereof within one day to one week following the surgery.

In yet another embodiment, the muscle injury is a surgery-related muscle injury and the treatment method provides for administration of at least one high dose of a H-PGDS inhibitor of Formula (XI) or a pharmaceutically acceptable salt thereof within one day to one week following the surgery, followed by periodic administration of a dose of a H-PGDS inhibitor during the recovery period.

Non-limiting examples of traumatic muscle injuries include battlefield muscle injuries, auto accident-related muscle injuries, and sports-related muscle injuries. Traumatic injury to the muscle can include lacerations, blunt force contusions, shrapnel wounds, muscle pulls or tears, burns, acute strains, chronic strains, weight or force stress injuries, repetitive stress injuries, avulsion muscle injury, and compartment syndrome.

In one embodiment, the muscle injury is a traumatic muscle injury and the treatment method provides for administration of at least one dose of a H-PGDS inhibitor of Formula (XI) or a pharmaceutically acceptable salt thereof immediately after the traumatic injury (for example, within one day of the injury) followed by periodic administration of a dose of a H-PGDS inhibitor during the recovery period.

Non-limiting examples of work-related muscle injuries include injuries caused by highly repetitive motions, forceful motions, awkward postures, prolonged and forceful mechanical coupling between the body and an object, and vibration.

Overtraining-related muscle injuries include unrepaired or under-repaired muscle damage coincident with a lack of recovery or lack of an increase of physical work capacity.

In an additional embodiment, the muscle injury is exercise or sports-induced muscle damage including exercise-induced delayed onset muscle soreness (DOMS).

In some embodiments, the invention encompasses a therapeutic combination in which the H-PGDS inhibitor of claim 1 or a pharmaceutically acceptable salt thereof is administered in a subject in combination with the implantation of a biologic scaffold (e.g. a scaffold comprising extracellular matrix) that promotes muscle regeneration. Such scaffolds are known in the art. See, for example, Turner and Badylack (2012) Cell Tissue Res. 347(3):759-74 and US Patent No. 6,576,265. Scaffolds comprising non-crosslinked extracellular matrix material are preferred.

In another aspect, the invention provides a method of treating tendon damage where the method comprises administering a compound of claim 1 or a pharmaceutically acceptable salt thereof to a subject in need thereof. In a particular embodiment, the invention includes a method of enhancing the formation of a stable tendon-bone interface. In a related embodiment, the invention provides a method of increasing the stress to failure of tendons, for example surgically-repaired tendons. In an additional embodiment, the invention provides a method of reducing fibrosis at the repair site for surgically-repaired tendons. In a particular embodiment, the invention provides a method of treating tendon damage associated with rotator cuff injury, or tendon damage associated with surgical repair of rotator cuff injury.

In another aspect, the invention provides a method of treating a disease state selected from: allergic diseases and other inflammatory conditions such as asthma, aspirinexacerbated respiratory disease (AERD), cough, chronic obstructive pulmonary disease (including chronic bronchitis and emphysema), bronchoconstriction, allergic rhinitis (seasonal or perennial), vasomotor rhinitis, rhinoconjuctivitis, allergic conjunctivitis, food allergy, hypersensitivity lung diseases, eosinophilic syndromes including eosinophilic asthma, eosinophilic pneumonitis, eosinophilic oesophagitis, eosinophilic granuloma, delayed-type hypersensitivity disorders, atherosclerosis, rheumatoid arthritis, pancreatitis, gastritis, inflammatory bowel disease, osteoarthritis, psoriasis, sarcoidosis, pulmonary fibrosis, respiratory distress syndrome, bronchiolitis, sinusitis, cystic fibrosis, actinic keratosis, skin dysplasia, chronic urticaria, eczema and all types of dermatitis including atopic dermatitis or contact dermatitis in a subject in need thereof comprising administering to the subject a therapeutically effective amount of a compound of claim 1 or a pharmaceutically acceptable salt thereof.

By the term "treating" and derivatives thereof as used herein, in reference to a condition means: (1) to ameliorate or prevent the condition or one or more of the biological manifestations of the condition, (2) to interfere with (a) one or more points in the biological cascade that leads to or is responsible for the condition or (b) one or more of the biological manifestations of the condition, (3) to alleviate one or more of the symptoms or effects associated with the condition, or (4) to slow the progression of the condition or one or more of the biological manifestations of the condition.

The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof.

As used herein, the term "effective amount" and derivatives thereof means that amount of a drug or pharmaceutical agent that will elicit the biological or medical response of a tissue, system, animal or human that is being sought, for instance, by a researcher or clinician. Furthermore, the term "therapeutically effective amount" and derivatives thereof means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

The subject to be treated in the methods of the invention is typically a mammal in need of such treatment, preferably a human in need of such treatment.

## COMPOSITIONS

The pharmaceutically active compounds within the scope of this invention are useful as inhibitors of H-PGDS in mammals, particularly humans, in need thereof.

The present invention therefore provides a method of treating muscle injury, muscle degenerative disorders and other conditions requiring H-PGDS inhibition, which comprises administering an effective amount of a compound of claim 1 or a pharmaceutically acceptable salt thereof. The compounds of claim 1 also provide for a method of treating the above indicated disease states because of their demonstrated ability to act as H-PGDS inhibitors. The drug may be administered to a subject in need thereof by any conventional route of administration, including, but not limited to, intravenous, intramuscular, oral, topical, subcutaneous, intradermal, intraocular and parenteral. Suitably, a H-PGDS inhibitor may be delivered directly to the brain by intrathecal or intraventricular route, or implanted at an appropriate anatomical location within a device or pump that continuously releases the H-PGDS inhibitor drug.

The pharmaceutically active compounds of the present invention are incorporated into convenient dosage forms such as capsules, tablets, or injectable preparations. Solid or liquid pharmaceutical carriers are employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation may be in the form of a syrup, elixir, emulsion, soft gelatin capsule, sterile injectable liquid such as an ampoule, or an aqueous or nonaqueous liquid suspension.

The pharmaceutical compositions are made following conventional techniques of a pharmaceutical chemist involving mixing, granulating, and compressing, when necessary, for tablet forms, or mixing, filling and dissolving the ingredients, as appropriate, to give the desired oral or parenteral products.

Pharmaceutical compositions adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert excipient such as ethanol, glycerol, water and the like. Powders are prepared by reducing the compound to a suitable fine size and mixing with a similarly prepared pharmaceutical excipient such as an edible carbohydrate, as, for example, starch or mannitol. Flavouring, preservative, dispersing and colouring agent can also be present.

Capsules are made by preparing a powder mixture, as described above, and filling formed gelatin sheaths. Excipients including glidants and lubricants such as colloidal silica, talc, magnesium stearate, calcium stearate or solid

polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, excipients including suitable binders, glidants, lubricants, sweetening agents, flavours, disintegrating agents and colouring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrants include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with a free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Compositions for inhaled administration include aqueous, organic or aqueous/organic mixtures, dry powder or crystalline compositions administered to the respiratory tract by pressurised pump or inhaler, for example, reservoir dry powder inhalers, unit-dose dry powder inhalers, pre-metered multi-dose dry powder inhalers, nasal inhalers or pressurised aerosol inhalers, nebulisers or insufflators. Suitable compositions contain water as the diluent or carrier for this purpose and may be provided with conventional excipients such as buffering agents, tonicity modifying agents and the like. Aqueous compositions may also be administered to the nose and other regions of the respiratory tract by nebulisation. Such compositions may be aqueous solutions or suspensions or aerosols delivered from pressurised packs, such as a metered dose inhaler, with the use of a suitable liquefied propellant.

Dry powder compositions for topical delivery to the lung by inhalation may, for example, be presented in capsules and cartridges of for example gelatine, or blisters of for example laminated aluminium foil, for use in an inhaler or insufflator. Powder blend compositions generally contain a powder mix for inhalation of the compound of Formula (I) or a pharmaceutically acceptable salt thereof and a suitable powder base (carrier/diluent/excipient substance) such as mono-, di-, or polysaccharides (for example lactose or starch). Dry powder compositions may also include, in addition to the drug and carrier, a further excipient (for example a ternary agent such as a sugar ester for example cellobiose octaacetate, calcium stearate, or magnesium stearate.

In one embodiment, a composition suitable for inhaled administration may be incorporated into a plurality of sealed dose containers provided on medicament pack(s) mounted inside a suitable inhalation device. The containers may be rupturable, peelable, or otherwise openable one-at-a-time and the doses of the dry powder composition administered by inhalation on a mouthpiece of the inhalation device, as known in the art. The medicament pack may take a number of different forms, for instance a disk-shape or an elongate strip. Representative inhalation devices are the DISKHALER™ and DISKUS™ devices, marketed by GlaxoSmithKline.

A dry powder inhalable composition may also be provided as a bulk reservoir in an inhalation device, the device then being provided with a metering mechanism for metering a dose of the composition from the reservoir to an inhalation channel where the metered dose is able to be inhaled by a subject inhaling at a mouthpiece of the device. Exemplary marketed devices of this type are TURBUHALER™ (AstraZeneca), TWISTHALER™ (Schering) and CLICKHALER™ (Innovata.)

A further delivery method for a dry powder inhalable composition is for metered doses of the composition to be provided in capsules (one dose per capsule) which are then loaded into an inhalation device, typically by the subject on demand. The device has means to rupture, pierce or otherwise open the capsule so that the dose is able to be entrained into the subject's lung when they inhale at the device mouthpiece. As marketed examples of such devices there may be mentioned ROTAHALER™ (GlaxoSmithKline) and HANDIHALER™ (Boehringer Ingelheim.)

Pressurised aerosol compositions suitable for inhalation can be either a suspension or a solution and may contain a compound of Formula (I) or a pharmaceutically acceptable salt thereof and a suitable propellant such as a fluorocarbon or hydrogen-containing chlorofluorocarbon or mixtures thereof, particularly hydrofluoroalkanes, especially 1,1,1,2-tetrafluoroethane, 1,1,1,2,3,3,3-heptafluoro-n-propane or a mixture thereof. The aerosol composition may optionally contain additional composition excipients well known in the art such as surfactants e.g. oleic acid, lecithin or an oligolactic acid or derivative thereof e.g. as described in WO 94/21229 and WO 98/34596 (Minnesota Mining and Manufacturing Company) and co-solvents e.g. ethanol. Pressurised compositions will generally be retained in a canister (e.g. an aluminium canister) closed with a valve (e.g. a metering valve) and fitted into an actuator provided with a mouthpiece.

Doses of the presently invented pharmaceutically active compounds in a pharmaceutical dosage unit as described above will be an efficacious, nontoxic quantity preferably selected from the range of 0.001 - 500 mg/kg of active compound, preferably 0.001 - 100 mg/kg. When treating a human subject in need of a H-PGDS inhibitor, the selected dose is administered preferably from 1-6 times daily, orally or parenterally. Preferred forms of parenteral administration include topically, rectally, transdermally, by injection and continuously by infusion. Oral dosage units for human administration preferably contain from 0.05 to 3500 mg of active compound. Oral administration, which uses lower dosages, is preferred. Parenteral administration, at high dosages, however, also can be used when safe and convenient for the subject.

Optimal dosages to be administered may be readily determined by those skilled in the art, and will vary with the particular H-PGDS inhibitor in use, the strength of the preparation, the mode of administration, and the advancement of the disease condition. Additional factors depending on the particular subject being treated will result in a need to adjust dosages, including subject age, weight, diet, and time of administration.

The method of this invention of inducing H-PGDS inhibitory activity in mammals, including humans, comprises administering to a subject in need of such activity an effective H-PGDS inhibiting amount of a pharmaceutically active compound of the present invention.

The invention also provides for the use of a compound of claim 1 or a pharmaceutically acceptable salt thereof in the manufacture of a medicament for use as a H-PGDS inhibitor.

The invention also provides for the use of a compound of claim 1 or a pharmaceutically acceptable salt thereof in the manufacture of a medicament for use in therapy.

The invention also provides for the use of a compound of claim 1 or a pharmaceutically acceptable salt thereof in the manufacture of a medicament for use in treating musculoskeletal diseases such as Duchenne Muscular Dystrophy, spinal cord contusion injury, neuroinflammatory diseases such as Multiple Sclerosis or neurodegenerative diseases such as Alzheimer's disease or amyotrophic lateral sclerosis (ALS).

The invention also provides for a pharmaceutical composition for use as a H-PGDS inhibitor which comprises a compound of claim 1 or a pharmaceutically acceptable salt thereof and a pharmaceutically acceptable carrier or excipient.

The invention also provides for a pharmaceutical composition for use in the treatment of muscle injury and muscle degenerative disorders which comprises a compound of claim 1 or a pharmaceutically acceptable salt thereof and a pharmaceutically acceptable carrier or excipient.

In addition, the pharmaceutically active compounds of the present invention can be co-administered with further active ingredients, such as other compounds known to treat any of the diseases or conditions disclosed herein, or compounds known to have utility when used in combination with a H-PGDS inhibitor.

By the term "co-administration" as used herein is meant either simultaneous administration or any manner of separate sequential administration of a H-PGDS inhibiting compound, as described herein, and a further active agent or agents, known to be useful in the treatment of conditions in which a H-PGDS inhibitor is indicated, including the conditions disclosed herein. The term "further active agent or agents", as used herein, includes any compound or therapeutic agent known to or that demonstrates advantageous properties when administered to a subject having a condition in which a H-PGDS inhibitor is indicated, including the conditions disclosed herein. Preferably, if the administration is not simultaneous, the compounds are administered in a close time proximity to each other. Furthermore, it does not matter if the compounds are administered in the same dosage form, e.g. one compound may be administered by injection and another compound may be administered orally.

The invention also relates to the use of a compound of claim 1 or a pharmaceutically acceptable salt thereof in the preparation of a medicament for the treatment of neurodegenerative diseases, musculoskeletal diseases and diseases in which H-PGDS inhibition is indicated.

The invention also provides a pharmaceutical composition comprising from 0.5 to 1,000 mg of a compound of claim 1 or pharmaceutically acceptable salt thereof and from 0.5 to 1,000 mg of a pharmaceutically acceptable excipient.

Without further elaboration, it is believed that one skilled in the art can, using the preceding description, utilize the present invention to its fullest extent. The following Examples are, therefore, to be construed as merely illustrative and not a limitation of the scope of the present invention in any way.

## ABBREVIATIONS

As used herein the symbols and conventions used in these processes, schemes and examples are consistent

with those used in the contemporary scientific literature, for example, the *Journal of the American Chemical Society* or the *Journal of Biological Chemistry.* Standard single-letter or three-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:

Ac (acetyl);
$Ac_2O$ (acetic anhydride);
ACN (acetonitrile);
AIBN (azobis(isobutyronitrile));
BINAP (2,2'-bis(diphenylphosphino)-1,1'-binaphthyl);
BMS (borane - dimethyl sulphide complex);
Bn (benzyl);
Boc (tert-Butoxycarbonyl);
$Boc_2O$ (di-*tert*-butyl dicarbonate);
BOP (Benzotriazole-1-yl-oxy-tris-(dimethylamino)-phosphonium hexafluorophosphate);
CAN (cerric ammonium nitrate);
Cbz (benzyloxycarbonyl);
CSI (chlorosulfonyl isocyanate);
CsF (cesium fluoride);
DABCO (1,4-Diazabicyclo[2.2.2]octane);
DAST (Diethylamino)sulfur trifluoride);
DBU (1,8-Diazabicyclo[5.4.0]undec-7-ene);
DCC (Dicyclohexyl Carbodiimide);
DCE (1,2-dichloroethane);
DCM (dichloromethane);
DDQ (2,3-Dichloro-5,6-dicyano-1,4-benzoquinone);
ATP (adenosine triphosphate);
Bis-pinacolatodiboron (4,4,4',4',5,5,5',5'-Octamethyl-2,2'-bi-1,3,2-dioxaborolane);
BSA (bovine serum albumin);
C18 (refers to 18-carbon alkyl groups on silicon in HPLC stationary phase);
$CH_3CN$ (acetonitrile);
Cy (cyclohexyl);
DCM (dichloromethane);
DIEA (Hünig's base, *N,N*-Diisopropylethylamine, *N*-ethyl-*N*-(1-methylethyl)-2-propanamine);
Dioxane (1,4-dioxane);
DMAP (4-dimethylaminopyridine);
DME (1,2-dimethoxyethane);
DMEDA (*N,N'*-dimethylethylenediamine);
DMF (*N,N*-dimethylformamide);
DMSO (dimethylsulfoxide);
DPPA (diphenyl phosphoryl azide);
EDC (*N*-(3-dimethylaminopropyl)-*N'*ethylcarbodiimide);
EDTA (ethylenediaminetetraacetic acid);
EtOAc (ethyl acetate);
EtOH (ethanol);
$Et_2O$ (diethyl ether);
HEPES (4-(2-hydroxyethyl)-1-piperazine ethane sulfonic acid);
HATU (O-(7-Azabenzotriazol-1-yl)-*N,N,N',N'*-tetramethyluronium hexafluorophosphate);
HOAt (1-hydroxy-7-azabenzotriazole);
HOBt (1-hydroxybenzotriazole);
HOAc (acetic acid);
HPLC (high pressure liquid chromatography);
HMDS (hexamethyldisilazide);
IPA (isopropyl alcohol);
Indoline (2,3-dihydro-1*H*-indole) ;
KHMDS (potassium hexamethyldisilazide) ;
LAH (lithium aluminum hydride) ;

LDA (lithium diisopropylamide) ;
LHMDS (lithium hexamethyldisilazide)
MeOH (methanol);
MTBE (methyl tert-butyl ether);
mCPBA (m- chloroperoxybenzoic acid);
NaHMDS (sodium hexamethyldisilazide);
NBS (*N*-bromosuccinimide);
PE (petroleum ether);
$Pd_2(dba)_3$ (Tris(dibenzylideneacetone)dipalladium(0);
$Pd(dppf)Cl_2$.DCM Complex([1,1'-Bis(diphenylphosphino)ferrocene]dichloropalladium(II).dichloromethane complex);
PyBOP (benzotriazol-1-yl-oxytripyrrolidinophosphonium hexafluorophosphate);
PyBrOP (bromotripyrrolidinophosphonium hexafluorophosphate);
RP-HPLC (reverse phase high pressure liquid chromatography);
RT (room temperature);
Sat. (saturated)
SFC (supercritical fluid chromatography);
SGC (silica gel chromatography);
SM (starting material);
TLC (thin layer chromatography);
TEA (triethylamine);
TEMPO (2,2,6,6-Tetramethylpiperidine 1-oxyl, free radical);
TFA (trifluoroacetic acid); and
THF (tetrahydrofuran).

All references to ether are to diethyl ether and brine refers to a saturated aqueous solution of NaCl.

## EXPERIMENTAL DETAILS

### Mass Directed Auto-Preparative HPLC (MDAP)

Mass Directed Auto-Preparative HPLC is undertaken under the conditions given below. Detection is by absorption over the wavelength range 210 nm to 350 nm and mass spectra are recorded on a mass spectrometer using alternate-scan positive and negative mode electrospray ionization.

Method A

Method A is conducted on a Waters SunFire C18 column (typically 150 mm x 30 mm i.d. with 5 micron particle size) at ambient temperature. The solvents employed are:

A = 0.1% v/v solution of formic acid in water
B = 0.1% v/v solution of formic acid in acetonitrile.

Method B

Method B is conducted on a Waters XBridge C18 column (typically 100 mm x 30 mm i.d. with 5 micron particle size) at ambient temperature. The solvents employed are:

A = 10 mM aqueous ammonium bicarbonate adjusted to pH 10 with ammonia solution.
B = acetonitrile.

Method C

Method C is conducted on a Waters SunFire C18 column (typically 150mm x 30mm i.d. with 5 micron particle size) at ambient temperature. The solvents employed are:

A = 0.1% v/v solution of trifluoroacetic acid in water
B = 0.1% v/v solution of trifluoroacetic acid in acetonitrile.

## EXAMPLES

The following Examples illustrate the invention. These examples are not intended to limit the scope of the present invention, but rather to provide guidance to the skilled artisan to prepare and use the compounds, compositions, and methods of the present invention. While particular embodiments of the present invention are described, the skilled artisan will appreciate that various changes and modifications can be made within the scope of the claims.

It is further noted that examples not following under the scope of the claims are to be regarded as reference compounds only.

The compounds in the application were named using ChemDraw.

## INTERMEDIATES

### Intermediate 1: Triethylammonium 7-methoxyquinoline-3-carboxylate

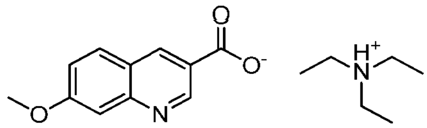

A solution of ethyl 7-methoxyquinoline-3-carboxylate (4.15 g, 17.95 mmol) (commercially available, but prepared by a similar route to intermediate 2) in MeOH (150 mL) was treated with a solution of lithium hydroxide (0.860 g, 35.9 mmol) in water (20 mL). The reaction mixture was stirred at 60 °C for 48 h prior to concentration *in vacuo*. The sample was purified by column chromatography on silica using a 0-30 % MeOH (+ 1 % $Et_3N$) in DCM gradient. Appropriate fractions were combined and evaporated to give the title compound (4.0237 g). $^1H$ NMR (400 MHz, $CD_3OD$) δ 1.33 (t, *J* = 7 Hz, 9 H), 3.22 (q, *J* = 7 Hz, 6 H), 4.00 (s, 3 H), 7.31 (dd, *J* = 9, 2 Hz, 1 H), 7.42 (d, *J* = 2 Hz, 1 H), 7.94 (d, *J* = 9 Hz, 1 H), 8.78 (d, *J* = 2 Hz, 1 H), 9.31 (d, *J* = 2 Hz, 1 H); LCMS ES+ve m/z 204 $[M+H]^+$.

### Intermediate 2: 7-(Difluoromethoxy)quinoline-3-carboxylic acid

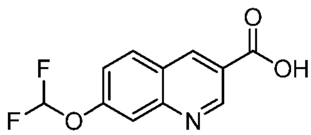

**A. Diethyl 2-(((3-(difluoromethoxy)phenyl)amino)methylene)malonate**

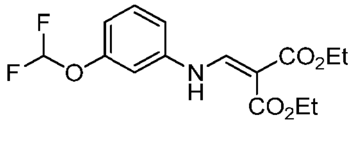

A mixture of 3-(difluoromethoxy)aniline (2.04 g, 12.8 mmol) and diethyl 2-(ethoxymethylene)malonate (2.77 g, 12.8 mmol) in EtOH (50 mL) was heated at reflux for 3 h. The cooled mixture was evaporated to dryness to afford the title compound (4.2 g). ES+ve *m/z* 330 $[M+H]^+$.

**B. Ethyl 4-chloro-7-(difluoromethoxy)quinoline-3-carboxylate**

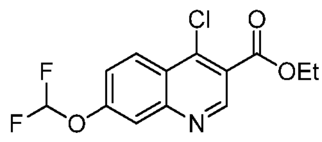

A mixture of diethyl 2-(((3-(difluoromethoxy)phenyl)amino)methylene)malonate (4.2 g, 12.8 mmol) and phosphorus oxychloride (10 mL, 107 mmol) was heated in a sealed vial by microwave at 150 °C for 30 min. The cooled mixture was evaporated to dryness and the residue was taken up in DCM (150 mL). The solution was treated cautiously with water (150 mL) and the aqueous phase was adjusted to ~pH 7 by the addition of 2 M aqueous sodium hydroxide solution. The organic phase was collected and the aqueous phase was extracted with additional DCM (100 mL). The combined organics were evaporated to dryness and the product was purified by column chromatography on silica using a 0-50 % EtOAc in cyclohexane gradient to afford the title compound (1.2 g). LCMS ES+ve *m/z* 302 $[M+H]^+$.

**C. Ethyl 7-(difluoromethoxy)quinoline-3-carboxylate**

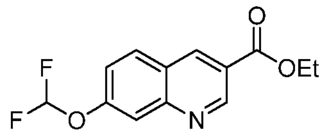

A mixture of ethyl 4-chloro-7-(difluoromethoxy)quinoline-3-carboxylate (1.1 g, 3.65 mmol) and triethylamine (2 mL, 14.4 mmol) in EtOH (120 mL) was added to palladium on carbon (Degussa type, 10 % palladium) (110 mg) and stirred vigorously under an atmosphere of hydrogen for 1 h. The reaction mixture was filtered through Celite® and then evaporated to dryness. The residue was purified by column chromatography on silica using 0-25 % EtOAc in cyclohexane to afford the title compound (910 mg). $^1H$ NMR (400 MHz, $CDCl_3$) $\delta$ 1.49 (t, *J* = 7 Hz, 3 H), 4.51 (q, *J* = 7 Hz, 2 H), 6.76 (t, *J* = 73 Hz, 1 H), 7.44 (dd, *J* = 9, 2 Hz, 1 H), 7.83 (s, 1 H), 7.97 (d, *J* = 9 Hz, 1 H), 8.84 (d, *J* = 2 Hz, 1 H), 9.47 (s, 1 H). ES+ve *m/z* 268 $[M+H]^+$.

**D. 7-(Difluoromethoxy)quinoline-3-carboxylic acid**

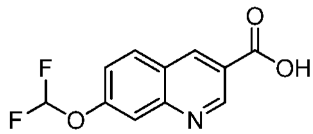

A suspension of ethyl 7-(difluoromethoxy)quinoline-3-carboxylate (870 mg, 3.26 mmol) in MeOH (10 mL) was treated with a solution of sodium hydroxide (0.197 g, 4.93 mmol) in water (3 mL) and the mixture was heated at 50 °C for 1 h. The cooled mixture was evaporated to dryness and the residue was taken up in water (60 mL) and acidified to pH 6 with 2 M aqueous hydrochloric acid. The precipitated product was filtered off, washed with water and dried *in vacuo* to afford the title compound (655 mg). $^1H$ NMR (400 MHz, $CD_3SOCD_3$) $\delta$ 7.35 - 7.76 (m, 2 H), 7.79 (d, *J* = 2 Hz, 1 H), 8.29 (d, *J* = 9 Hz, 1 H), 8.99 (d, *J* = 2 Hz, 1 H), 9.33 (d, *J* = 2 Hz, 1 H). ES+ve *m/z* 240 $[M+H]^+$.

**Intermediate 3: 6-cyano-7-cyclopropylquinoline-3-carboxylic acid**

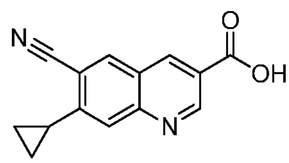

**A. 4-Amino-2-cyclopropylbenzonitrile**

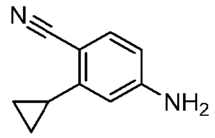

A mixture of 4-amino-2-chlorobenzonitrile (3 g, 19.7 mmol), cyclopropylboronic acid (5.07 g, 59.0 mmol), tricyclohexylphosphine (1.10 g, 3.93 mmol) and potassium phosphate (12.5 g, 59.0 mmol) in toluene (250 mL) was treated with water (6 mL) and then degassed by the repeated application of vacuum followed by nitrogen pressure. The mixture was treated with palladium(II) acetate (0.44 g, 1.97 mmol) and then heated at 95 °C under an atmosphere of nitrogen for 6 h. The cooled mixture was treated with saturated aqueous sodium bicarbonate solution (150 mL). The separated organic layer was dried over magnesium sulfate, filtered, and evaporated to dryness. The residue was purified by column chromatography on silica using a 0-50 % TBME in cyclohexane gradient to afford the title compound (3.0 g). ES+ve m/z 159 [M+H]+.

**B. Diethyl 2-(((4-cyano-3-cyclopropylphenyl)amino)methylene)malonate**

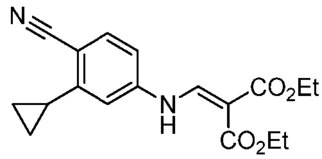

A mixture of 4-amino-2-cyclopropylbenzonitrile (3 g, 19.0 mmol) and diethyl 2-(ethoxymethylene)malonate (3.80 mL, 19.0 mmol) in EtOH (20 mL) was heated at reflux for 3 h. The cooled mixture was evaporated to dryness to afford the title compound (6.2 g). ES+ve *m/z* 329 $[M+H]^+$.

**C. Ethyl 4-chloro-6-cyano-7-cyclopropylquinoline-3-carboxylate**

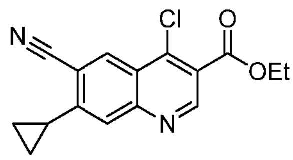

A mixture of diethyl 2-(((4-cyano-3-cyclopropylphenyl)amino)methylene)malonate (6.2 g, 18.9 mmol) and phosphorus oxychloride (30 mL, 322 mmol) was split between two reaction vessels. The vessels were sealed and heated by microwave at 160 °C for 45 min. The combined reaction mixtures were evaporated to dryness and the residue was taken up in DCM (150 mL). The solution was treated cautiously with water (150 mL) and the aqueous phase was adjusted to ~pH 7 by the addition of 2 M aqueous sodium hydroxide solution. The organic phase was collected and the aqueous phase was extracted with additional DCM (100 mL). The combined organics were evaporated to dryness and the residue was purified by column chromatography on silica using a 0-50 % EtOAc in cyclohexane gradient to afford the title compound (2.2 g). ES+ve *m/z* 301 $[M+H]^+$.

**D. Ethyl 6-cyano-7-cyclopropylquinoline-3-carboxylate**

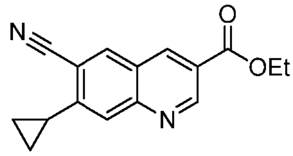

A mixture of ethyl 4-chloro-6-cyano-7-cyclopropylquinoline-3-carboxylate (2.2 g, 7.32 mmol) and triethylamine (3 mL, 21.5 mmol) in EtOH (60 mL) was added to palladium on carbon (Degussa type, 10 % palladium) (150 mg) and stirred vigorously under an atmosphere of hydrogen for 1 h. The reaction mixture was filtered through Celite® and then evaporated to dryness. The residue was purified by column chromatography on silica using a 0-25 % EtOAc in cyclohexane gradient to afford the title compound (1.18 g). ES+ve *m/z* 268 $[M+H]^+$.

**E. 6-cyano-7-cyclopropylquinoline-3-carboxylic acid**

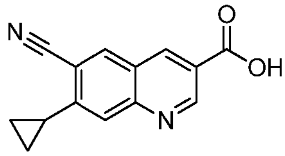

A suspension of ethyl 6-cyano-7-cyclopropylquinoline-3-carboxylate (1 g, 3.76 mmol) in MeOH (10 mL) was treated with a solution of sodium hydroxide (0.45 g, 11.3 mmol) in water (3 mL) and the mixture was heated at 50 °C for 1 h. The cooled mixture was evaporated to dryness and the residue was taken up in water (60 mL) and acidified to pH 6 using 2 M aqueous hydrochloric acid. The precipitated product was filtered off, washed with water and dried *in vacuo* to afford the title compound (758 mg). ES+ve *m/z* 239 $[M+H]^+$.

**Intermediate 4: 7-Bromo-6-chloroquinoline-3-carboxylic acid**

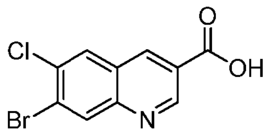

**A. 4-Bromo-5-chloro-2-nitrobenzaldehyde**

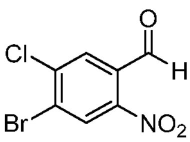

Ice-cooled, stirred concentrated sulfuric acid (15 mL, 281 mmol) was cautiously treated dropwise with concentrated nitric acid (70 %) (1.9 mL, 29.8 mmol) and the mixture was treated portionwise over 10 min with 4-bromo-3-chlorobenzaldehyde (3.2 g, 14.6 mmol). The cooling was removed and the mixture was allowed to warm to RT and was stirred for 3 h. The mixture was cautiously added to ice-water (100 mL) and extracted with DCM (3 x 60 mL). The combined extracts were evaporated to dryness and the residue was purified by column chromatography on silica using a 0-25 % TBME in cyclohexane gradient to afford the title compound (2.55 g). $^1H$ NMR ($CDCl_3$) δ 8.03 (s, 1 H), 8.43 (s, 1 H), 10.41 (s, 1 H).

**B. Ethyl 7-bromo-6-chloroquinoline-3-carboxylate**

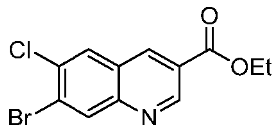

A suspension of 4-bromo-5-chloro-2-nitrobenzaldehyde (2.41 g, 9.11 mmol) in EtOH (140 mL) was warmed to 50 °C to effect a solution and then cooled to ambient temperature. The solution was treated with ethyl 3,3-diethoxypropanoate (4.05 mL, 22.8 mmol) and tin(II) chloride dihydrate (8.22 g, 36.5 mmol) and heated at reflux for 6 h. The cooled mixture was evaporated to dryness and the residue was taken up in EtOAc (200 mL) and added to saturated aqueous sodium bicarbonate (400 mL). The resulting emulsion was stirred for 30 min and then filtered through a pad of Celite®. The filtrate layers were separated and the aqueous phase was extracted with additional EtOAc (100 mL). The combined organics were dried over magnesium sulfate, filtered, and evaporated to dryness. The residue was purified by column chromatography on silica using a 0-25% EtOAc in cyclohexane gradient to afford the title compound (2.25 g). $^{1}$H NMR (400 MHz, $CDCl_3$) $\delta$ 1.49 (t, *J* = 7 Hz, 3 H), 4.51 (q, *J* = 7 Hz, 2 H), 8.07 (s, 1 H), 8.53 (s, 1 H), 8.76 (d, *J* = 2Hz, 1 H), 9.46 (d, *J* =2 Hz, 1 H). ES+ve *m*/*z* 314/316 [M+H]+.

**C. 7-Bromo-6-chloroquinoline-3-carboxylic acid**

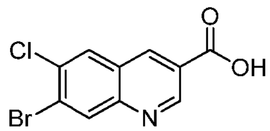

A suspension of ethyl 7-bromo-6-chloroquinoline-3-carboxylate (500 mg, 1.59 mmol) in MeOH (10 mL) was treated with a solution of sodium hydroxide (127 mg, 3.18 mmol) in water (3 mL) and the mixture was heated at 50 °C for 1 h. The cooled mixture was evaporated to dryness and the residue was taken up in water (60 mL) and acidified to pH 6 using 2 M aqueous hydrochloric acid. The precipitated product was filtered off, washed with water and dried *in vacuo* to afford the title compound (378 mg). ES+ve *m*/*z* 286/288 $[M+H]^+$.

Large scale synthesis of **Ethyl 7-bromo-6-chloroquinoline-3-carboxylate:**

**A. 4-bromo-5-chloro-2-nitrobenzaldehyde**

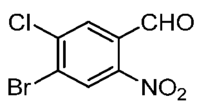

A 6 L JLR was charged with conc. sulfuric acid (2500 mL, 46.9 mol) and cooled to 2 °C (internal temp). Conc nitric acid (224 mL, 3510 mmol) was added *via* dropping addition funnel at a rate such that the internal temp remained ≤ 10 °C (ca. 30 min). The mixture was cooled to *ca.* 2 °C (internal temp) and 4-bromo-3-chlorobenzaldehyde (514 g, 2340 mmol) was added portionwise over 15 min. After 15 min jacket temperature was raised to 10 °C. Jacket cooling was discontinued after 3 h and the mixture was allowed to warm to RT with stirring overnight. The reactor was carefully drained into 20 L crushed ice with stirring. After ca. 30 min, precipitated solids were collected by filtration. The cake was washed 2 x 5 L water and dried on the filter over a weekend affording 614 g pale yellow solid. EtOAc (620 mL) and the crude solid were added to a 6 L JLR and jacket temperature was set to 60 °C with stirring, affording an orange solution within ca. 15 min. Heptane (3.8 L) was added over 90 min, during which time the internal temperature was raised gradually to 75 °C. After ca. 30 min, jacket heating was discontinued and the mixture was allowed to cool to RT overnight. Precipitated solids were collected by filtration. The cake was washed with the mother liquor, then 2 x 750 mL heptane

and dried on the filter affording the title compound (285.4 g, 1079 mmol, 46 % yield) as a light yellow solid. $^1$H NMR indicated only the desired regioisomer was present. $^1$H NMR (400 MHz, $CDCl_3$) δ ppm 8.02 (s, 1 H), 8.42 (s, 1 H), 10.40 (s, 1 H).

**B. ethyl 7-bromo-6-chloroquinoline-3-carboxylate**

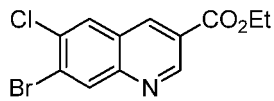

A 16 L JLR was charged with EtOH (10 L) and warmed to 40 °C (internal temp). 4-Bromo-5-chloro-2-nitrobenzaldehyde (720 g, 2723 mmol) and ethyl 3,3-diethoxypropanoate (1.324 L, 6806 mmol) were added and the mixture was stirred until dissolution of solids occurred. Jacket temp was set to 10 °C and tin chloride dihydrate (2457 g, 10.9 mol) was added over *ca.* 15 min (internal temp rose to *ca.* 50 °C). The mixture was stirred ca. 15 min and jacket temperature was increased to 70 °C. The mixture was stirred 7.5 h, jacket heating was discontinued and the mixture was stirred at RT over a weekend. The slurry was drained from the reactor and concentrated using a 20 L rotary evaporator (ca. 9.5 L EtOH removed). The residue was transfered back into the reactor as a slurry in DCM (ca. 2 L), and diluted with DCM (10 L total). Jacket temperature was set to 0 °C and the mixture was cooled to ca. 5 °C (internal). Triethylamine (2.66 L, 19.1 mol) was added *via* cannula over *ca.* 90 min (internal temp ≤ 20 °C). Jacket cooling was discontinued and the slurry was allowed to warm to RT with stirring overnight. Precipitated solids were removed by filtration, and the cake was washed 2 x 1 L DCM. The filtrate was transfered back into the reactor and 25% w/v $K_3PO_4$ (4 L) was added. The contents were stirred ca. 1 h. Additional 25% $K_3PO_4$ (4 L) was added and stirring continued overnight. Additional 25% $K_3PO_4$ (4 L) was added, jacket temperature was set at 35 °C and stirring rate was set to 15 RPM. Most solids had dissolved within a few hours. Additional 25% $K_3PO_4$ (2 L) and water (1 L) were added and stirring continued ca. 3 h. Jacket temp was set to 20 °C and stirring was discontinued. The organic layer was drained and the aqueous layer was extracted with DCM (2 x 2 L). Combined organics were dried over $MgSO_4$ and concentrated to near dryness with a 20 L rotary evaporator (2 x 2 L MTBE chase). The residue was slurried in MTBE (3 L) and aged overnight. Heptane (9 L) was added with stirring over 2 h and the mixture was cooled in an ice bath. Precipitated solids were collected by filtration, washed with the mother liquor and heptane (2 x 1 L) and dried on the filter over a weekend affording ethyl 7-bromo-6-chloroquinoline-3-carboxylate (552.3 g, 1756 mmol, 65 % yield) as a pale yellow solid. $^1$H NMR (400 MHz, $CDCl_3$) δ ppm 1.47 (t, *J* = 7 Hz, 3 H), 4.49 (q, *J* = 7 Hz, 2 H), 8.04 (s, 1 H), 8.50 (s, 1 H), 8.74 (d, *J* = 2 Hz, 1 H), 9.43 (d, *J* = 2 Hz, 1 H). MS (ESI) *m*/*z* 314, 316 (M+H, Br isotopes).

**Alternate preparation of ethyl 7-bromo-6-chloroquinoline-3-carboxylate**

A 6 L JLR was charged with EtOH (1.2 L) and 4-bromo-5-chloro-2-nitrobenzaldehyde (86.4 g, 327 mmol) and stirred at 250 RPM. The jacket temp was set at 45 °C and the mixture was stirred until dissolution of solids occurred (ca. 15 min). Ethyl 3,3-diethoxypropanoate (0.159 L, 817 mmol) was added and jacket temp was set to 10 °C. Upon cooling to *ca.* 20 °C (internal), tin(II) chloride dihydrate (295 g, 1307 mmol) was added in portions over ca. 10 min. Jacket temp was ramped to 70 °C over 1 h. Heating was discontinued after 5 h and the mixture was stirred at RT over a weekend. Heating was resumed (jacket temp 70 °C), jacket temp was increased to 80 °C after 1.5 h. The mixture was stirred 2.5 h at 80 °C, cooled to ca. 40 °C (internal temp) and quenched by addition of 25% w/v $K_3PO_4$ (3.5 L, 4130 mmol). No exotherm noted during addition of $K_3PO_4$. Jacket temp was set to 45 °C and the mixture was stirred 2 h. Jacket temp was set to 20 °C and 2 L DCM + 2 L water were added. The mixture was stirred vigorously ca. 5 min and allowed to partition. Layers were separated and the aqueous layer was extracted with DCM (1 L). Combined organics were returned to the JLR and washed with water (2 L). The organic layer was drained, dried over $Na_2SO_4$, and concentrated *in vacuo* (2 x 1 LTBME chase). The crude solid was slurried in TBME (250 mL) and slowly diluted with heptane (750 mL). The slurry was stirred in an ice bath ca. 30 min and solids were collected by filtration. The cake was washed with the liquor and a small amount of heptane and dried on the filter over a weekend affording ethyl 7-bromo-6-chloroquinoline-3-carboxylate (64.9 g, 206 mmol, 63 % yield) as a pale yellow solid (v. fine needles). $^1$H NMR / LCMS were found to be identical to material prepared according to step B above.

<u>**Intermediate 5: Triethylammonium 8-methoxyquinoline-3-carboxylate**</u>

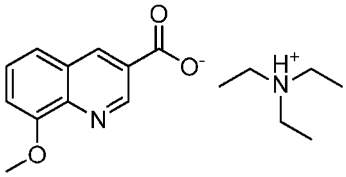

**A. Ethyl 4-hydroxy-8-methoxyquinoline-3-carboxylate**

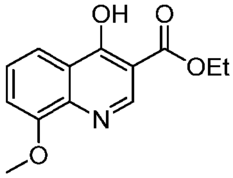

A mixture of o-anisidine (2.53 mL, 22 mmol) and diethyl 2-(ethoxymethylene)malonate (4.36 mL, 22 mmol) were heated at 100 °C for 2 h under nitrogen prior to cooling. Diphenyl ether (3.50 mL, 22.00 mmol) was added and the reaction mixture was heated at 250 °C for 2 h prior to cooling. The mixture was purified by column chromatography on silica using a 0-100 % EtOAc in cyclohexane gradient. Appropriate fractions were combined and evaporated *in vacuo* to give the title compound (6.45 g). ES+ve m/z 247 $[M+H]^+$.

**B. Ethyl 4-chloro-8-methoxyquinoline-3-carboxylate**

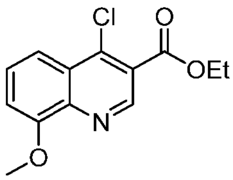

Ethyl 4-hydroxy-8-methoxyquinoline-3-carboxylate (6.45 g, 26.1 mmol) was treated with $POCl_3$ (4.13 mL, 44.3 mmol) and the reaction mixture was stirred at 100 °C for 24 h under nitrogen. The reaction mixture was added portion wise to 5 N NaOH (40 mL) at 0 °C, allowing each portion to react prior to addition of the next. The internal reaction temperature was kept below 35 °C. The reaction mixture was extracted with DCM (2 x 100 mL) and the organic extracts concentrated *in vacuo.* The residue was purified by column chromatography on silica using a 0-100% EtOAc in cyclohexane gradient. Appropriate fractions were combined and evaporated *in vacuo* to give the title compound (4.17 g). $^1H$ NMR (400 MHz, $CDCl_3$) $\delta$ 1.48 (t, *J* = 7 Hz, 3 H), 4.13 (s, 3 H), 4.52 (q, *J* = 7 Hz, 2 H), 7.21 (d, *J* = 7 Hz, 1 H), 7.64 (t, *J* = 8 Hz, 1 H), 7.99 (dd, *J* = 9, 1 Hz, 1 H), 9.19 (s, 1 H). ES+ve m/z 266 $[M+H]^+$.

**C. Ethyl 8-methoxy-3,4-dihydroquinoline-3-carboxylate**

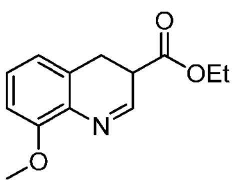

A hydrogenation flask containing 10 % Pd/C (0.334 g, 3.14 mmol) was flushed three times with nitrogen and a solution of ethyl 4-chloro-8-methoxyquinoline-3-carboxylate (4.17 g, 15.69 mmol) in EtOH (250 mL) and triethylamine (6.56 mL, 47.1 mmol) was added under vacuum. The flask was flushed three more times with nitrogen prior to stirring

under hydrogen for 2 h. The required volume of hydrogen had been consumed. The reaction mixture was flushed with nitrogen three times prior to filtration through Celite® under a blanket of nitrogen. The filtrate was concentrated *in vacuo* and the sample was purified by column chromatography on silica using a 0-100 % EtOAc in cyclohexane gradient. Appropriate fractions were combined and evaporated *in vacuo* to give the title compound (2.15 g). $^1$H NMR (400 MHz, $CDCl_3$) δ 1.32 (t, *J* = 7 Hz, 3 H), 3.78 (s, 2 H), 3.86 (s, 3 H), 4.22 (q, *J* = 7 Hz, 2 H), 6.51 (br s, 1 H), 6.67 (m, 2 H), 6.87 (m, 1 H), 7.38 (d, *J* = 6 Hz, 1 H); LCMS ES+ve m/z 234 $[M+H]^+$.

**D. Triethylammonium 8-methoxyquinoline-3-carboxylate**

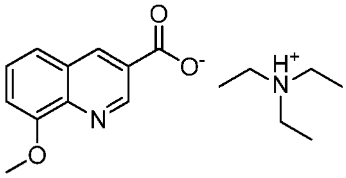

A solution of ethyl 8-methoxy-3,4-dihydroquinoline-3-carboxylate (2.15 g, 9.22 mmol) in MeOH (150 mL) was treated with a solution of lithium hydroxide (0.441 g, 18.43 mmol) in water (20 mL). The reaction mixture was stirred at 21 °C for 2 h in air. Further lithium hydroxide (0.441 g, 18.43 mmol) and water (20 mL) were added and the reaction mixture was stirred at 60 °C for 48 h prior to concentration *in vacuo.* The residue was purified by column chromatography on silica using a 0-30 % MeOH (+ 1 % $Et_3N$) in DCM gradient. Appropriate fractions were combined and evaporated *in vacuo* to give the title compound containing excess triethylamine (2.41 g). $^1$H NMR (400 MHz, $CD_3OD$) δ 1.34 (t, *J* = 7 Hz, 60 H), 3.23 (q, *J* = 7 Hz, 40 H), 4.09 (s, 3 H), 7.27 (dd, *J* = 6, 3 Hz, 1 H), 7.58 (m, 2 H), 8.79 (d, *J* = 2 Hz, 1 H), 9.35 (d, *J* = 2 Hz, 1 H). MS ES+ve m/z 204 $[M+H]^+$.

**Intermediate 6: Triethylammonium 7-methoxy-6-methylquinoline-3-carboxylate**

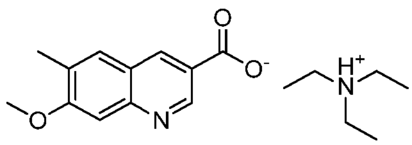

**A. Diethyl 2-(((3-methoxy-4-methylphenyl)amino)methylene)malonate**

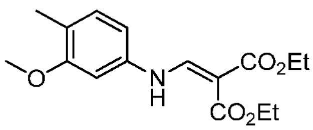

3-Methoxy-4-methylaniline (1 g, 7.29 mmol) and diethyl 2-(ethoxymethylene)malonate (1.576 g, 7.29 mmol) were heated by microwave at 100 °C for 2 h. The mixture was purified by column chromatography on silica using a 0-100 % EtOAc in cyclohexane gradient. Appropriate fractions were combined and evaporated *in vacuo* to give the title compound (2.1709 g). $^1$H NMR (400 MHz, $CDCl_3$) δ 1.22 - 1.51 (2 x t, 6 H), 2.20 (s, 3 H), 3.86 (s, 3 H), 4.15 - 4.42 (2 x q, 4 H), 6.58 (d, *J* = 2 Hz, 1 H), 6.68 (dd, *J* = 8, 2 Hz, 1 H), 7.12 (d, *J* = 8 Hz, 1 H), 8.52 (d, *J* = 14 Hz, 1 H), 11.02 (d, *J* = 14 Hz, 1 H); LCMS ES+ve *m/z* 308 $[M+H]^+$.

**B. Ethyl 4-chloro-7-methoxy-6-methylquinoline-3-carboxylate**

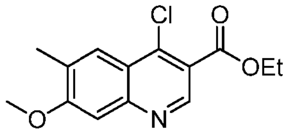

Diethyl 2-(((3-methoxy-4-methylphenyl)amino)methylene)malonate (2.1709 g, 7.06 mmol) and $POCl_3$ (32.5 g, 212 mmol) were heated in the microwave to 160 °C for 25 min. The reaction was quenched by dropwise addition to ice cold 5N NaOH solution (150 mL). The temperature was not allowed to exceed 40 °C during the addition. The product was recovered by filtration and washed with distilled water. The material was pre-absorbed onto Florisil® and purified by column chromatography on silica using a 0-100 % EtOAc in cyclohexane gradient. Appropriate fractions were combined and evaporated *in vacuo* to give the title compound (1.69 g). $^1H$ NMR (400 MHz, $CDCl_3$) δ 1.43 - 1.48 (t, *J* = 7 Hz, 3 H), 2.40 (s, 3 H), 3.98 (s, 3 H), 4.47 (q, *J* = 7 Hz, 2 H), 7.33 (s, 1 H), 8.05 (d, *J* = 1 Hz, 1 H), 9.08 (s, 1 H). MS ES+ve *m/z* 280 $[M+H]^+$.

**C. Ethyl 7-methoxy-6-methylquinoline-3-carboxylate**

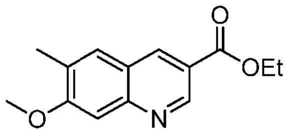

A hydrogenation flask containing 10 wt % palladium on carbon (0.125 g, 1.173 mmol) was flushed three times with nitrogen and a solution of ethyl 4-chloro-7-methoxy-6-methylquinoline-3-carboxylate (1.64 g, 5.86 mmol) and triethylamine (4.90 mL, 35.2 mmol) in EtOH (300 mL) was added under vacuum. The flask was flushed three more times with nitrogen prior to stirring under hydrogen for 3 h. The required volume of hydrogen (140 mL) was consumed. The reaction mixture was flushed with nitrogen three times prior to filtration through Celite® under a blanket of nitrogen. The filtrate was concentrated *in vacuo* giving a residue. The material was purified by reverse-phase column chromatography on C18 modified silica using a 30-80 % acetonitrile in water (with 0.1 % v/v formic acid) gradient. Evaporation of the solvent from appropriate fractions gave the title compound (530 mg). $^1H$ NMR (400 MHz, $CDCl_3$) δ 1.47 (t, *J* = 7 Hz, 3 H), 2.42 (s, 3 H), 4.03 (s, 3 H), 4.48 (q, *J* = 7 Hz, 2 H), 7.44 (s, 1 H), 7.65 (s, 1 H), 8.69 (d, *J* = 2 Hz, 1 H), 9.34 (d, *J* = 2 Hz, 1 H). MS ES+ve *m/z* 246 $[M+H]^+$.

**D. Triethylammonium 7-methoxy-6-methylquinoline-3-carboxylate**

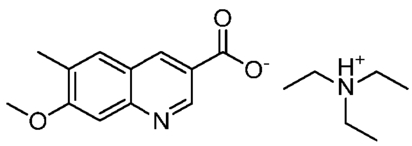

Ethyl 7-methoxy-6-methylquinoline-3-carboxylate (450 mg, 1.835 mmol) and lithium hydroxide (176 mg, 7.34 mmol) were dissolved in MeOH (15 mL) and water (2 mL) and heated by microwave in a sealed vial at 65 °C for 2 h. The reaction mixture was concentrated *in vacuo* and purified by column chromatography on silica using a 0-30 % MeOH (+ 1 % $Et_3N$) in DCM gradient to yield the title compound (448.3 mg). $^1H$ NMR (400 MHz, $CD_3OD$) δ 1.31 (t, *J* = 7 Hz, 9 H), 3.18 (q, *J* = 7 Hz, 6 H), 3.37 (s, 3 H), 4.03 (s, 3 H), 7.36 (s, 1 H), 7.73 (s, 1 H), 8.69 (d, *J* = 2 Hz, 1 H), 9.26 (d, *J* = 2 Hz, 1 H). MS ES+ve *m/z* 218 $[M+H]^+$.

**Intermediate 7: Triethylammonium 5-fluoro-7-methoxyquinoline-3-carboxylate**

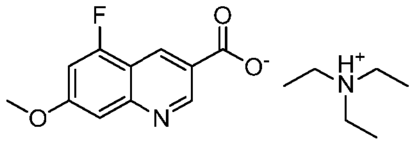

**A. Diethyl 2-(((3-fluoro-5-methoxyphenyl)amino)methylene)malonate**

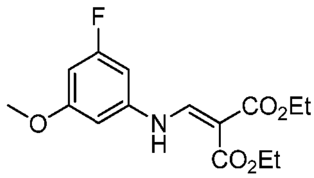

3-Fluoro-5-methoxyaniline (1 g, 7.09 mmol) and diethyl 2-(ethoxymethylene)malonate (1.532 g, 7.09 mmol) were heated by microwave in a sealed vial at 100 °C for 2 h. The mixture was purified by column chromatography on silica using a 0-100 % EtOAc in cyclohexane gradient. Appropriate fractions were combined and evaporated *in vacuo* to give the title compound (2.318 g). $^1$H NMR (400 MHz, $CDCl_3$) δ 1.25 - 1.51 (m, 6 H), 3.83 (s, 3 H), 4.20 - 4.41 (m, 4 H), 6.32 - 6.56 (m, 3 H), 8.44 (d, *J* = 14 Hz, 1 H), 10.96 (d, *J* = 13 Hz, 1 H). MS ES+ve *m/z* 312 $[M+H]^+$.

**B. Ethyl 4-chloro-5-fluoro-7-methoxyquinoline-3-carboxylate**

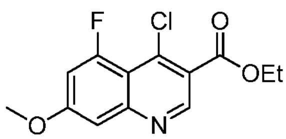

Diethyl 2-(((3-fluoro-5-methoxyphenyl)amino)methylene)malonate (2.3180 g, 7.45 mmol) and $POCl_3$ (20.82 mL, 223 mmol) were heated by microwave in a sealed vial at 160 °C for a total of 20 min. The reaction was quenched by dropwise addition of the mixture to ice cold 5 N NaOH solution (150 mL). The temperature was not allowed to exceed 40 °C. The product was recovered by filtration and washed with distilled water. The material was pre-absorbed onto Florisil® and purified by column chromatography on silica using a 0-100 % EtOAc in cyclohexane gradient. Appropriate fractions were combined and evaporated *in vacuo* to give the title compound (650 mg). $^1$H NMR (400 MHz, $CDCl_3$) δ 1.45 (t, *J* = 7 Hz, 3 H), 3.98 (s, 3 H), 4.49 (q, *J* = 7 Hz, 2 H), 7.00 (dd, *J* = 14, 3 Hz, 1 H), 7.28 (m, 1 H), 9.02 (s, 1 H). MS ES+ve *m/z* 284 $[M+H]^+$.

**C. Ethyl 5-fluoro-7-methoxyquinoline-3-carboxylate**

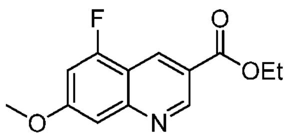

A hydrogenation flask containing 10 % Pd/C (450 mg) was flushed three times with nitrogen and a solution of ethyl 4-chloro-5-fluoro-7-methoxyquinoline-3-carboxylate (600 mg, 2.115 mmol) in EtOH (300 mL) and triethylamine (1284 mg, 12.69 mmol) was added under vacuum. The flask was flushed three more times with nitrogen prior to stirring under hydrogen for 3 h. The reaction mixture was flushed with nitrogen three times prior to filtration through Celite® under a blanket of nitrogen. The filtrate was concentrated *in vacuo* and the residue was purified by column chromatography on silica using a 0-100 % EtOAc in cyclohexane gradient. Appropriate fractions were combined and evaporated *in vacuo*

to give the title compound (403 mg). $^1H$ NMR (400 MHz, $CDCl_3$) δ 1.48 (t, *J* = 7 Hz, 3 H), 4.00 (s, 3 H), 4.49 (q, *J* = 7 Hz, 2 H), 6.97 (dd, *J* = 11, 2 Hz, 1 H), 7.31 (d, *J* = 2 Hz, 1 H), 8.98 (d, *J* = 2 Hz, 1 H), 9.42 (d, *J* = 2 Hz, 1 H). MS ES+ve *m*/*z* 250 $[M+H]^+$.

**D. Triethylammonium 5-fluoro-7-methoxyquinoline-3-carboxylate**

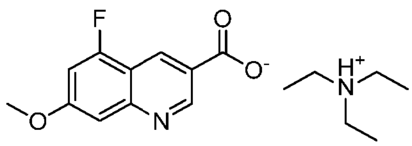

Ethyl 5-fluoro-7-methoxyquinoline-3-carboxylate (400 mg, 1.605 mmol) and lithium hydroxide (154 mg, 6.42 mmol) were dissolved in MeOH (15 mL) and water (2 mL) and heated by microwave in a sealed vial at 65 °C for 2 hr. The reaction mixture was concentrated *in vacuo* and was purified by column chromatography on silica using a 0-30 % MeOH (+ 1 % $Et_3N$) in DCM gradient to yield the title compound (310.2 mg). $^1H$ NMR (400 MHz, $CD_3OD$) δ 1.33 (t, *J* = 7 Hz, 9 H), 3.23 (q, *J* = 7 Hz, 6 H), 4.00 (s, 3 H), 7.05 (dd, J = 11, 2 Hz, 1 H), 7.28 (d, *J* = 2 Hz, 1 H), 8.94 (d, *J* = 2 Hz, 1 H), 9.36 (d, *J* = 2 Hz, 1 H). MS ES+ve *m*/*z* 222 $[M+H]^+$.

**Intermediate 8: Triethylammonium 7-chloro-8-methoxyquinoline-3-carboxylate**

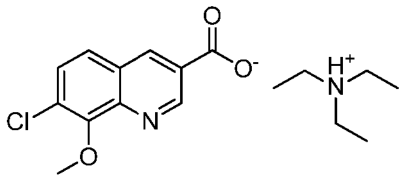

**A. Diethyl 2-(((3-chloro-2-methoxyphenyl)amino)methylene)malonate**

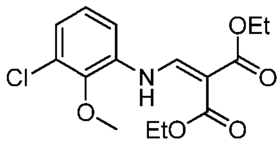

3-Chloro-2-methoxyaniline (1 g, 6.35 mmol) and diethyl 2-(ethoxymethylene)malonate (1.372 g, 6.35 mmol) were heated by microwave in a sealed vial at 100 °C for 2 h to give a crude sample of the title compound (2.08 g). MS ES+ve *m*/*z* 328 $[M+H]^+$.

**B. Ethyl 4,7-dichloro-8-methoxyquinoline-3-carboxylate**

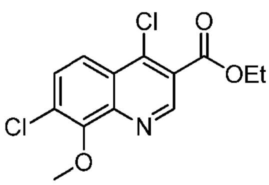

Diethyl 2-(((3-chloro-2-methoxyphenyl)amino)methylene)malonate (2.08 g, 6.35 mmol) and $POCl_3$ (19.46 g,

127 mmol) were heated by microwave at 160 °C for 20 min. The reaction was quenched by dropwise addition of the mixture to ice-cold 5 N NaOH solution (150 mL). The temperature was not allowed to exceed 40 °C. The product was then collected by filtration and washed with distilled water. The material was preabsorbed onto Florisil® and purified by column chromatography on silica using a 0-100 % EtOAc in cyclohexane gradient. Appropriate fractions were combined and evaporated *in vacuo* to give the title compound (628.2 mg). $^1$H NMR (400 MHz, $CDCl_3$) δ 1.48 (t, *J* = 7 Hz, 3 H), 4.20 (s, 3 H), 4.52 (q, *J* = 7 Hz, 2 H), 7.69 (d, *J* = 9 Hz, 1 H), 8.13 (d, *J* = 9 Hz, 1 H), 9.25 (s, 1 H). MS ES+ve *m/z* 300 $[M+H]^+$.

**C. Ethyl 7-chloro-8-methoxyquinoline-3-carboxylate**

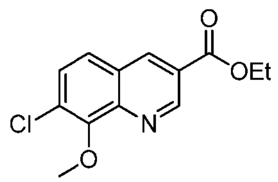

A hydrogenation flask containing 10 % palladium on carbon (40.3 mg) was flushed three times with nitrogen and a solution of ethyl 4,7-dichloro-8-methoxyquinoline-3-carboxylate (568.2 mg, 1.893 mmol) in EtOH (200 mL) and triethylamine (1.6 mL, 11.36 mmol) was added under vacuum. The flask was flushed three more times with nitrogen prior to stirring under hydrogen for 1 h. The reaction mixture was flushed with nitrogen three times prior to filtration through Celite® under a blanket of nitrogen. The filtrate was concentrated *in vacuo* and the residue was purified by reverse phase column chromatography on C18 modified silica using a 30-80 % acetonitrile in water (+ 0.1% v/v formic acid) gradient to yield the title compound (140 mg). $^1$H NMR (400 MHz, $CDCl_3$) δ 1.48 (t, *J* = 7 Hz, 4 H), 4.23 (s, 3 H), 4.51 (q, *J* = 7 Hz, 2 H), 7.64 (m, 2 H), 8.84 (d, *J* = 2 Hz, 1 H), 9.51 (d, *J* = 2 Hz, 1 H). MS ES+ve *m/z* 266 $[M+H]^+$.

**D. Triethylammonium 7-chloro-8-methoxyquinoline-3-carboxylate**

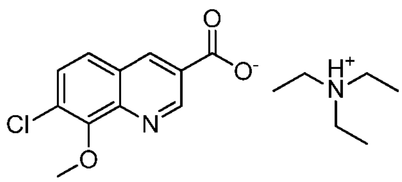

Ethyl 7-chloro-8-methoxyquinoline-3-carboxylate (120 mg, 0.452 mmol) and lithium hydroxide (43.3 mg, 1.807 mmol) were dissolved in MeOH (15 mL) and water (2 mL) and heated by microwave in a sealed vial at 65 °C for 2 h. The reaction mixture was concentrated *in vacuo* and purified by column chromatography on silica using a 0-30 % MeOH (+ 1 % $Et_3N$) in DCM gradient to yield the title compound (118.9 mg). $^1$H NMR (400 MHz, $CD_3OD$) δ 1.32 (t, *J* = 7 Hz, 9 H), 3.22 (q, *J* = 7 Hz, 6 H), 4.01 (s, 3 H), 7.49 (d, *J* = 9 Hz, 1 H), 7.62 (d, *J* = 9 Hz, 1 H), 8.82 (s, 1 H), 9.44 (s, 1 H). MS ES+ve *m/z* 238 $[M+H]^+$.

**Intermediate 9: 6-chloro-7-methoxyquinoline-3-carboxylic** acid

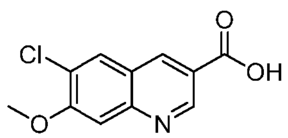

**A. Diethyl 2-(((4-chloro-3-methoxyphenyl)amino)methylene)malonate**

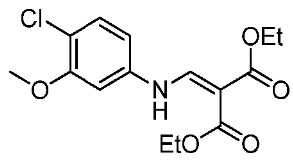

4-Chloro-3-methoxyaniline (1 g, 6.35 mmol) and diethyl 2-(ethoxymethylene)malonate (1.372 g, 6.35 mmol) were heated by microwave in a sealed vial at 100 °C for 2 h. The mixture was purified by column chromatography on silica using a 0-100 % EtOAc in cyclohexane gradient. Appropriate fractions were combined and evaporated *in vacuo* to give the title compound (1.8913 g). $^1$H NMR (400 MHz, $CDCl_3$) $\delta$ 1.32 - 1.47 (m, 6 H), 3.94 (s, 3 H), 4.24 - 4.37 (m, 4 H), 6.67 (d, *J* = 2 Hz, 1 H), 6.72 (dd, J = 8, 2 Hz, 1 H), 7.36 (d, *J* = 8 Hz, 1 H), 8.48 (d, *J* = 14 Hz, 1 H), 11.04 (d, *J* = 14 Hz, 1 H). MS ES+ve *m/z* 328 $[M+H]^+$.

**B. Ethyl 4,6-dichloro-7-methoxyquinoline-3-carboxylate**

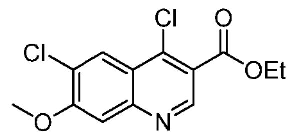

Diethyl 2-(((4-chloro-3-methoxyphenyl)amino)methylene)malonate (1.8913 g, 5.77 mmol) and $POCl_3$ (26.5 g, 173 mmol) were heated by microwave in a sealed vial at 160 °C for 25 min. The reaction was quenched by dropwise addition of the mixture to ice cold 5N NaOH solution (150 mL). The temperature was not allowed to exceed 40 °C. The product was collected by filtration and washed with distilled water. The material was preabsorbed onto Florisil® and purified by column chromatography on silica using a 0-100 % EtOAc in cyclohexane gradient. Appropriate fractions were combined and evaporated *in vacuo* to give the title compound (1.56 g). $^1$H NMR (400 MHz, $CDCl_3$) $\delta$ 1.47 (t, *J* = 7 Hz, 3 H), 4.08 (s, 3 H), 4.49 (q, *J* = 7 Hz, 2 H), 7.48 (s, 1 H), 8.39 (s, 1 H), 9.15 (s, 1 H). MS ES+ve *m/z* 300 $[M+H]^+$.

**C. Ethyl 6-chloro-7-methoxyquinoline-3-carboxylate**

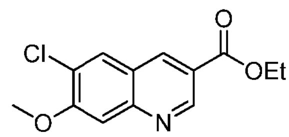

A hydrogenation flask containing 10 % palladium on carbon (106 mg) was flushed three times with nitrogen and a solution of ethyl 4,6-dichloro-7-methoxyquinoline-3-carboxylate (1.5 g, 5.00 mmol) in EtOH (300 mL) and triethylamine (4.18 mL, 30.0 mmol) was added under vacuum. The flask was flushed three more times with nitrogen prior to stirring under hydrogen for 3 h. The reaction mixture was flushed with nitrogen three times prior to filtration through Celite® under a blanket of nitrogen. The filtrate was concentrated *in vacuo* and the residue was purified by reverse phase preparative HPLC (Waters XBridge Shield RP18, 100 mm x 19 mm i.d., 5 m,65-100% $CH_3CN$ /water + 10 mM aqueous bicarbonate, 20 mL/min) to yield the title compound (227 mg). $^1$H NMR (400 MHz, $CD_3SOCD_3$) $\delta$ 1.38 (t, *J* = 7 Hz, 3 H), 4.07 (s, 3 H), 4.41 (q, *J* = 7 Hz, 2 H), 7.64 (s, 1 H), 8.40 (s, 1 H), 8.90 (d, *J* = 2 Hz, 1 H), 9.26 (d, *J* = 2 Hz, 1 H). MS ES+ve *m/z* 266 $[M+H]^+$.

**D. 6-chloro-7-methoxyquinoline-3-carboxylic acid**

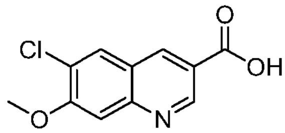

Ethyl 6-chloro-7-methoxyquinoline-3-carboxylate (227 mg, 0.854 mmol) and lithium hydroxide (82 mg, 3.42 mmol) were dissolved in MeOH (15 mL) and water (2 mL) and heated by microwave in a sealed vial at 65 °C for 2 h. The reaction mixture was concentrated *in vacuo* and purified by column chromatography on silica using a 0-30 % MeOH (+ 1 % $Et_3N$) in DCM gradient to yield the title compound (190 mg, 0.8 mmol). $^1H$ NMR (400 MHz, $CD_3SOCD_3$) δ 4.01 (s, 3 H), 7.53 (s, 1 H), 8.17 (s, 1 H), 8.57 (d, *J* = 2 Hz, 1 H), 9.29 (d, *J* = 2 Hz, 1 H). MS ES+ve *m/z* 238 $[M+H]^+$.

**Intermediate** 10: **Lithium 7,8-dimethoxyquinoline-3-carboxylate**

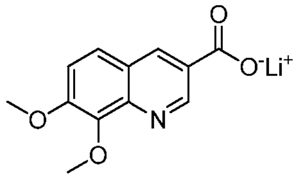

**A. Ethyl 4-chloro-7,8-dimethoxyquinoline-3-carboxylate**

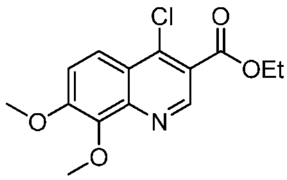

2,3-Dimethoxyaniline (1 g, 6.53 mmol) and diethyl 2-(ethoxymethylene)malonate (1.412 g, 6.53 mmol) were heated by microwave in a sealed vial at 100 °C for 2 h. The resulting crude diethyl 2-(((2,3-dimethoxyphenyl)amino)methylene)malonate (2.111 g, 6.53 mmol) and $POCl_3$ (30.0 g, 196 mmol) were heated by microwave at 160 °C for 25 min. The reaction was quenched by dropwise addition of the mixture to ice cold 5 N NaOH solution (150 mL). The temperature was not allowed to exceed 40 °C. The product was collected by filtration and washed with water. The material was pre-absorbed onto Florisil® and purified by column chromatography on silica using a 0-100 % EtOAc in cyclohexane gradient. Appropriate fractions were combined and evaporated *in vacuo* to give the title compound (1.32 g). $^1H$ NMR (400 MHz, $CDCl_3$) δ 1.47 (t, J = 7 Hz, 3 H), 4.09 (s, 3 H), 4.13 (s, 3 H), 4.50 (q, *J* = 7 Hz, 2 H), 7.50 (d, *J* = 9 Hz, 1 H), 8.19 (d, *J* = 9 Hz, 1 H), 9.23 (s, 1 H). MS ES+ve *m/z* 296 $[M+H]^+$.

**B. Ethyl 7,8-dimethoxyquinoline-3-carboxylate**

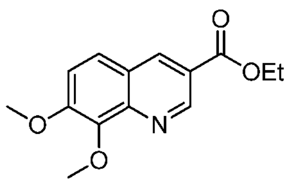

A hydrogenation flask containing 10 % palladium on carbon (95 mg, 0.893 mmol) was flushed three times with nitrogen and a solution of ethyl 4-chloro-7,8-dimethoxyquinoline-3-carboxylate (1.32 g, 4.46 mmol) in EtOH (300 mL) and triethylamine (3.73 mL, 26.8 mmol) were added under vacuum. The flask was flushed three more times with nitrogen

prior to stirring under hydrogen for 3 h. The reaction mixture was flushed with nitrogen three times prior to filtration through Celite® under a blanket of nitrogen. The filtrate was concentrated *in vacuo* to yield the title compound (0.77 g). $^1$H NMR (400 MHz, $CDCl_3$) δ 1.48 (t, *J* = 7 Hz, 4 H), 4.09 (s, 3 H), 4.17 (s, 3 H), 4.49 (d, *J* = 7 Hz, 2 H), 7.45 (d, *J* = 9 Hz, 1 H), 7.72 (d, *J* = 9 Hz, 1 H), 8.79 (d, *J* = 2 Hz, 1 H), 9.47 (d, *J* = 2 Hz, 1 H). MS ES+ve *m*/*z* 262 $[M+H]^+$.

**C. Lithium 7,8-dimethoxyquinoline-3-carboxylate**

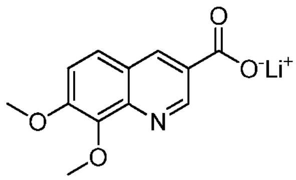

Ethyl 7,8-dimethoxyquinoline-3-carboxylate (0.72 g, 2.76 mmol) and lithium hydroxide (0.264 g, 11.02 mmol) were dissolved in MeOH (15 mL) and water (2 mL) and heated by microwave in a sealed vial at 65 °C for 2 h. The reaction mixture was concentrated *in vacuo* to give a crude sample of the title compound (0.8 g). MS ES+ve *m*/*z* 234 $[M+H]^+$.

**Intermediate 11: 6-Chloro-7-cyclopropylquinoline-3-carboxylic acid**

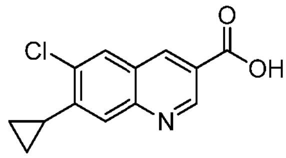

**A. Ethyl 6-chloro-7-cyclopropylquinoline-3-carboxylate**

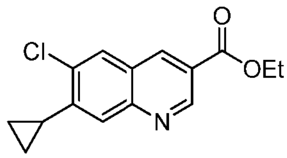

Under an atmosphere of nitrogen, a mixture of ethyl 7-bromo-6-chloroquinoline-3-carboxylate (Intermediate 4, Step B) (1.2 g, 3.81 mmol), cyclopropylboronic acid (0.75 g, 8.67 mmol), tricyclohexylphosphine (0.195 g, 0.694 mmol), palladium(II) acetate (0.078 g, 0.347 mmol), and potassium phosphate (2.2 g, 10.4 mmol) in a mixture of toluene (120 mL) and water (2.8 mL) was heated at 95 °C for 3 h. The cooled mixture was treated with EtOAc (150 mL) and water (80 mL), the layers were separated and the organic phase was dried over magnesium sulfate, filtered, and evaporated to dryness. The residue was purified by column chromatography on silica using a 0-100 % TBME in cyclohexane gradient to afford the title compound (730 mg). $^1$H NMR (400 MHz, $CDCl_3$) δ 0.92 (m, 2 H), 1.19 (m, 2 H), 1.48 (t, *J* = 7 Hz, 3 H), 2.39 (tt, *J* = 8, 5 Hz, 1 H), 4.50 (q, *J* = 7 Hz, 2 H), 7.75 (s, 1 H), 7.97 (s, 1 H), 8.73 (d, *J* = 2 Hz, 1 H), 9.40 (d, *J* = 2 Hz, 1 H). MS ES+ve *m*/*z* 276 $[M+H]^+$.

**B. 6-Chloro-7-cyclopropylquinoline-3-carboxylic acid**

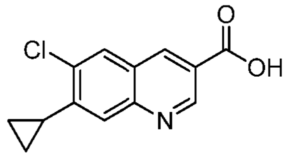

A suspension of ethyl 6-chloro-7-cyclopropylquinoline-3-carboxylate (260 mg, 0.943 mmol) in MeOH (10 mL) was treated with a solution of sodium hydroxide (113 mg, 2.83 mmol) in water (3 mL) and the mixture was heated at 50 °C for 1 h. The cooled mixture was evaporated to dryness; the residue was taken up in water (60 mL) and acidified to pH 6 using 2 M aqueous HCl. The precipitated product was filtered off, washed with water and dried *in vacuo* to afford the title compound (226 mg). MS ES+ve *m*/*z* 248 $[M+H]^+$.

**Intermediate 12: 7-Cyclopropyl-6-fluoroquinoline-3-carboxylic acid**

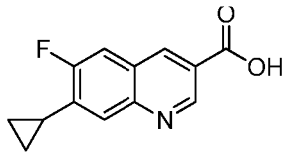

**A. 3-Cyclopropyl-4-fluoroaniline**

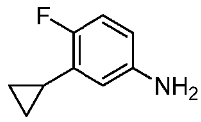

Under an atmosphere of nitrogen, a mixture of 3-bromo-4-fluoroaniline (2.7 g, 14.21 mmol), cyclopropylboronic acid (2.77 g, 32.3 mmol), tricyclohexylphosphine (0.725 g, 2.58 mmol) and potassium phosphate (8.23 g, 38.8 mmol) in a mixture of toluene (180 mL) and water (4 mL) was treated with palladium(II) acetate (0.29 g, 1.292 mmol) and then heated at 95 °C for 3 h. The cooled mixture was treated with water (80 mL), the layers separated and the organic phase was dried over magnesium sulfate, filtered, evaporated to dryness. The crude product was purified by column chromatography on silica using a 0-50 % EtOAc in cyclohexane gradient to afford the title compound (1.93 g). $^1$H NMR (400 MHz, $CDCl_3$) δ 0.70 (m, 2 H), 0.96 (m, 2 H), 2.04 (m, 1 H), 3.33 (br s, 2 H), 6.21 (dd, *J* = 6, 3 Hz, 1 H), 6.43 (m, 1 H), 6.81 (dd, *J* = 10, 9 Hz, 1 H). MS ES+ve *m*/*z* 152 $[M+H]^+$.

**B. Diethyl 2-(((3-cyclopropyl-4-fluorophenyl)amino)methylene)malonate**

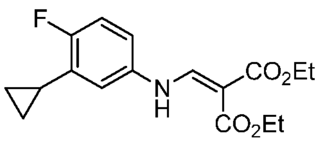

A mixture of 3-cyclopropyl-4-fluoroaniline (2.5 g, 16.5 mmol) and diethyl 2-(ethoxymethylene)malonate (3.48 mL, 17.4 mmol) was heated with stirring at 100 °C in an open vessel for 1 h and then cooled to RT to afford the title compound (5.31 g). MS ES+ve m/z 322 [M+H]+.

**C. Ethyl 4-chloro-7-cyclopropyl-6-fluoroquinoline-3-carboxylate**

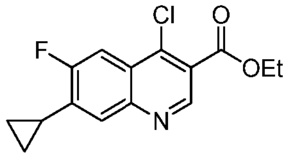

A mixture of diethyl 2-(((3-cyclopropyl-4-fluorophenyl)amino)methylene)malonate (5.3 g, 16.49 mmol) and phosphorus oxychloride (10 mL, 107 mmol) in a sealed vial was heated by microwave at 160 °C for 45 min. The mixture was evaporated to dryness and the residue was taken up in DCM (150 mL). The solution was treated cautiously with water (150 mL) and the aqueous phase was adjusted to pH 7-8 by the addition of 2 M aqueous sodium hydroxide. The organic phase was collected and the aqueous phase was extracted with additional DCM (100 mL). The combined organics were evaporated to dryness and the residue was purified by column chromatography on silica using a 0-25 % EtOAc in cyclohexane gradient to afford the title compound (1.4 g). MS ES+ve *m*/*z* 294 $[M+H]^+$.

**D. Ethyl 7-cyclopropyl-6-fluoroquinoline-3-carboxylate**

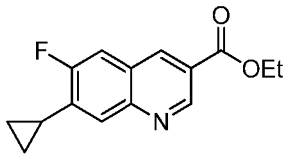

A mixture of ethyl 4-chloro-7-cyclopropyl-6-fluoroquinoline-3-carboxylate (1.4 g, 4.77 mmol) and triethylamine (2 mL, 14.4 mmol) in EtOH (60 mL) was added to palladium on carbon (Degussa type, 10 % palladium) (140 mg) and stirred vigorously under an atmosphere of hydrogen for 3 h. The reaction mixture was filtered through Celite® and then evaporated to dryness. The residue was purified by column chromatography on silica using 0-25 % EtOAc in cyclohexane gradient to afford the title compound (0.85 g). $^1H$ NMR (400 MHz, $CDCl_3$ $\delta$ 0.97 (m, 2 H), 1.18 (m, 2 H), 1.48 (t, *J* = 7 Hz, 4 H), 2.29 (m, 1 H), 4.49 (q, *J* = 7 Hz, 2 H), 7.51 (d, J = 10 Hz, 1 H), 7.69 (d, *J* = 7 Hz, 1 H), 8.74 (d, *J* = 2 Hz, 1 H), 9.37 (d, *J* = 2 Hz, 1 H). MS ES+ve *m*/*z* 260 $[M+H]^+$.

**E. 7-Cyclopropyl-6-fluoroquinoline-3-carboxylic acid**

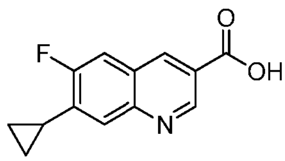

A suspension of ethyl 7-cyclopropyl-6-fluoroquinoline-3-carboxylate (0.8 g, 3.09 mmol) in MeOH (20 mL) was treated with a solution of sodium hydroxide (0.37 g, 9.26 mmol) in water (5 mL) and the mixture was heated at 50 °C for 1 h. The cooled mixture was evaporated to dryness and the residue was taken up in water (60 mL) and acidified to pH 5 using 2 M aqueous HCl. The precipitated product was filtered off, washed with water and dried *in vacuo* to afford the title compound (0.662 g). MS ES+ve *m*/*z* 232 $[M+H]^+$.

**Intermediate 13: Triethylammonium 6-fluoro-7-methoxyquinoline-3-carboxylate**

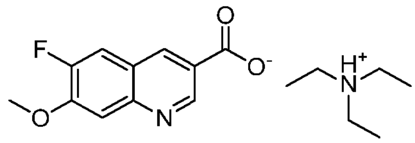

A. Diethyl 2-(((4-fluoro-3-methoxyphenyl)amino)methylene)malonate

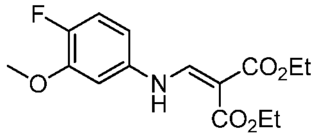

A mixture of 4-fluoro-3-methoxyaniline (0.850 mL, 7.08 mmol) and diethyl 2-(ethoxymethylene)malonate (1.403 mL, 7.08 mmol) was heated at 100 °C for 2 h under nitrogen prior to cooling. The mixture was purified by column chromatography on silica using a 0-100 % EtOAc in cyclohexane gradient. Appropriate fractions were combined and evaporated *in vacuo* to give the title compound (1.763 g). MS ES+ve *m*/*z* 312 $[M+H]^+$.

**B. Ethyl 4-chloro-6-fluoro-7-methoxyquinoline-3-carboxylate**

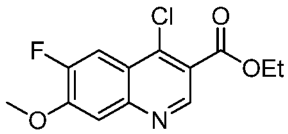

A mixture of diethyl 2-(((4-fluoro-3-methoxyphenyl)amino)methylene)malonate (12.17 g, 39.1 mmol) and phosphorus oxychloride (25 mL, 268 mmol) was heated by microwave in a sealed vial at 130 °C for 30 min. The mixture was evaporated to dryness and the residue taken up in DCM (150 mL). The solution was added slowly to an ice-cooled mixture of ice-water (~150 mL) and concentrated aqueous ammonia (100 mL). The mixture was filtered, the organic phase was separated and the aqueous phase was extracted with additional DCM (100 mL). The combined organics were evaporated to dryness and the residue was purified by column chromatography on silica using a 0-100 % EtOAc in cyclohexane gradient to afford the title compound (7.64 g). $^1H$ NMR (400 MHz, $CDCl_3$) δ 1.48 (t, *J* = 7 Hz, 3 H), 4.09 (s, 3 H), 4.51 (q, *J* = 7 Hz, 2 H), 7.56 (d, *J* = 8 Hz, 1 H), 8.04 (d, *J* = 12 Hz, 1 H), 9.16 (s, 1 H). MS ES+ve m/z 284 $[M+H]^+$.

**C. Ethyl 6-fluoro-7-methoxyquinoline-3-carboxylate**

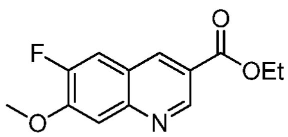

A mixture of ethyl 4-chloro-6-fluoro-7-methoxyquinoline-3-carboxylate (7.6 g, 26.8 mmol) and triethylamine (11.20 mL, 80 mmol) in EtOH (350 mL) was added to palladium on carbon (10 %, Degussa type) (760 mg, 0.714 mmol) and stirred vigorously in an atmosphere of hydrogen for 30 min. The reaction mixture was filtered through Celite®, the filtered solid was washed with DCM and the combined filtrate and washings were then evaporated to dryness. The residue was partitioned between DCM (150 mL) and water (100 mL). The organic phase was evaporated to dryness to afford the title compound (6.6 g). $^1H$ NMR (400 MHz, $CD_3OD$) δ 1.33 (t, *J* = 7 Hz, 3 H), 3.23 (q, *J* = 7 Hz, 2 H), 4.09 (s, 3 H), 7.56 (d, *J* = 8 Hz, 1 H), 7.74 (d, *J* = 11 Hz, 1 H), 8.78 (d, *J* = 2 Hz, 1 H), 9.29 (d, *J* = 2 Hz, 1 H). MS ES+ve m/z 250 $[M+H]^+$.

**D. Triethylammonium 6-fluoro-7-methoxyquinoline-3-carboxylate**

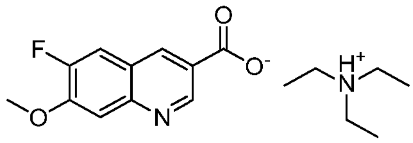

A solution of ethyl 6-fluoro-7-methoxyquinoline-3-carboxylate (1.405 g, 2.90 mmol) in MeOH (150 mL) was treated with a solution of lithium hydroxide (0.139 g, 5.80 mmol) in water (20 mL) and the reaction mixture stirred at 60 °C for 24 h. The mixture was concentrated *in vacuo* and the residue was pre-absorbed onto Florisil® and purified by column chromatography on silica using a 0-30 % MeOH (+1 % $Et_3N$) in DCM gradient. Appropriate fractions were combined and evaporated *in vacuo* to give the title compound (0.88 g). $^1H$ NMR (400 MHz, $CD_3OD$) $\delta$ 1.33 (t, *J* = 7 Hz, 9 H), 3.23 (q, *J* = 7 Hz, 6 H), 4.09 (s, 3 H), 7.56 (d, *J* = 8 Hz, 1 H), 7.74 (d, *J* = 11 Hz, 1 H), 8.78 (d, *J* = 2 Hz, 1 H), 9.29 (d, *J* = 2 Hz, 1 H). MS ES+ve *m/z* 222 $[M+H]^+$.

**Intermediate 14: Triethylammonium 8-fluoro-7-methoxyquinoline-3-carboxylate**

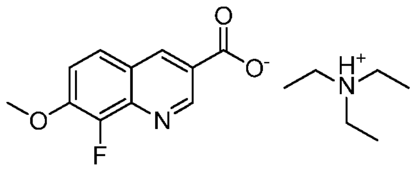

**A. Tert-butyl (2-fluoro-3-methoxyphenyl)carbamate**

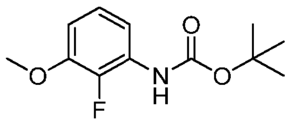

Diphenylphosphorylazide (3.80 mL, 17.63 mmol) was added dropwise to a solution of 2-fluoro-3-methoxybenzoic acid (2 g, 11.75 mmol), triethylamine (2.54 mL, 18.22 mmol) and tert-butanol (1.686 mL, 17.63 mmol) in toluene (30 mL). The resulting mixture was heated at 120 °C under nitrogen for 12 h. The reaction mixture was cooled to RT, treated with water (50 mL) and extracted with EtOAc (3 x 35 mL). The combined organic extracts were washed with brine (70 mL), dried over $MgSO_4$, and evaporated to give an oil. This material was purified by column chromatography on silica using a 0-50 % EtOAc in cyclohexane gradient. Appropriate fractions were combined and evaporated *in vacuo* to give the title compound (2.006 g). $^1H$ NMR (400 MHz, $CDCl_3$) $\delta$ 1.55 (s, 9 H), 3.90 (s, 3 H), 6.66 (dt, J = 8, 1 Hz, 1 H), 6.73 (br s, 1 H), 7.03 (dt, J = 8, 2 Hz, 1 H), 7.70 (t, *J* = 7 Hz, 1 H). MS ES+ve *m/z* 259 $[M+NH_4]^+$.

**B. 2-Fluoro-3-methoxyaniline hydrochloride**

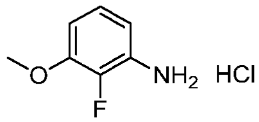

4M HCl in 1,4-dioxane (14.69 mL, 58.8 mmol) was added dropwise to tert-butyl (2-fluoro-3-methoxyphenyl)carbamate (2.006 g, 8.31 mmol) and the mixture was stirred at RT over the weekend. The solvent was evaporated *in vacuo*

to give the title compound (1.41 g). $^1H$ NMR (400 MHz, $CD_3OD$) δ 3.96 (s, 3 H), 7.03 (m, 1 H), 7.26 (m, 2 H). MS ES+ve *m*/*z* 142 $[M+H]^+$.

**C. Diethyl 2-(((2-fluoro-3-methoxyphenyl)amino)methylene)malonate**

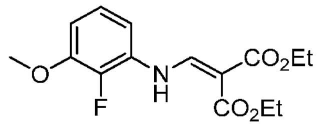

2-Fluoro-3-methoxyaniline hydrochloride (1.41 g, 7.94 mmol), triethylamine (2.434 mL, 17.47 mmol), and diethyl 2-(ethoxymethylene)malonate (1.717 g, 7.94 mmol) were stirred in toluene (5 mL) at 100 °C under nitrogen for 2 h. The solvent was evaporated *in vacuo* and the residue was purified by column chromatography on silica using a 0-100 % EtOAc in cyclohexane gradient. Appropriate fractions were combined and evaporated *in vacuo* to give the title compound (2 g). $^1H$ NMR (400 MHz, $CD_3OD$) δ 1.34 (m, 6 H), 3.92 (s, 3 H), 4.25 (q, *J* = 7 Hz, 2 H), 4.32 (q, *J* = 7 Hz, 2 H), 6.93 (dt, *J* = 8, 1 Hz, 1 H), 7.04 (dt, J = 8, 1 Hz, 1 H), 7.14 - 7.20 (m, 1 H), 8.56 (s, 1 H). MS ES+ve *m*/*z* 312 $[M+H]^+$.

**D. Ethyl 4-chloro-8-fluoro-7-methoxyquinoline-3-carboxylate**

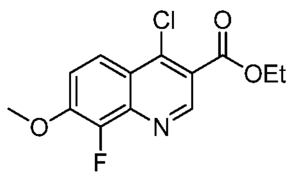

$POCl_3$ (17.97 mL, 193 mmol) was added to diethyl 2-(((2-fluoro-3-methoxyphenyl)amino)methylene)malonate (2 g, 6.42 mmol) and the mixture was heated at 100 °C for 6 h under nitrogen. Additional $POCl_3$ (17.97 mL, 193 mmol) was added and the reaction mixture was left overnight. The mixture was cooled to RT and added dropwise to ice-cold 5 N NaOH solution (600 mL) with stirring ensuring the temperature remained below 35 °C. The resulting solution was diluted with 2N NaOH (1 L) and extracted with DCM (3 x 1 L). The combined organic extracts were dried over $MgSO_4$, filtered, and evaporated *in vacuo* to give the title compound (776 mg). MS ES+ve *m*/*z* 284 $[M+H]^+$.

**E. Ethyl 8-fluoro-7-methoxyquinoline-3-carboxylate**

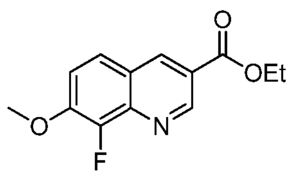

A hydrogenation flask containing 10 % Pd/C (58.2 mg, 0.547 mmol) was flushed three times with nitrogen and a solution of ethyl 4-chloro-8-fluoro-7-methoxyquinoline-3-carboxylate (776 mg, 2.74 mmol) in EtOH (30 mL) and triethylamine (2.288 mL, 16.41 mmol) was added under vacuum. The flask was flushed three more times with nitrogen prior to stirring under an atmosphere of hydrogen for 3 h. The required volume of hydrogen had been consumed. The reaction mixture was flushed with nitrogen three times prior to filtration through Celite® under a blanket of nitrogen. The filtrate was concentrated *in vacuo* and the residue was pre-absorbed onto Florisil® and purified by column chromatography on silica using a 0-100 % EtOAc in cyclohexane gradient. Appropriate fractions were combined and evaporated *in vacuo* to give the title compound (421 mg). $^1H$ NMR (400 MHz, $CDCl_3$) δ 1.48 (t, *J* = 7 Hz, 3 H), 4.13 (s, 3 H), 4.50 (q, *J* = 7 Hz, 2 H), 7.46 (dd, *J* = 9, 7 Hz, 1 H), 7.74 (dd, *J* = 9, 2 Hz, 1 H), 8.81 (t, *J* = 2 Hz, 1 H), 9.47 (d, *J* = 2 Hz, 1 H). MS ES+ve

*m/z* 250 [M+H]+.

**F. Triethylammonium 8-fluoro-7-methoxyquinoline-3-carboxylate**

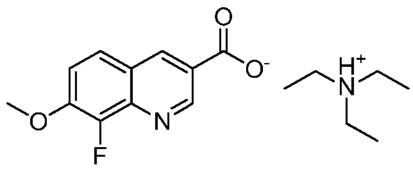

A solution of ethyl 8-fluoro-7-methoxyquinoline-3-carboxylate (0.42 g, 1.685 mmol) in MeOH (20 mL) was treated with a solution of lithium hydroxide (0.161 g, 6.74 mmol) in water (2.67 mL). The reaction mixture was stirred at 60 °C for 6 h prior to concentration *in vacuo.* The residue was purified by column chromatography on silica using a 0-30 % MeOH (+ 1 % $Et_3N$) in DCM gradient. Appropriate fractions were combined and evaporated *in vacuo* to give the title compound (474 mg). $^1H$ NMR (400 MHz, $CD_3OD$) δ 1.33 (t, *J* = 7 Hz, 9 H), 3.23 (q, *J* = 7 Hz, 6 H), 4.10 (s, 3 H), 7.63 (dd, J = 9, 7 Hz, 1 H), 7.86 (dd, J = 9, 2 Hz, 1 H), 8.83 (t, *J* = 2 Hz, 1 H), 9.37 (d, *J* = 2 Hz, 1 H). MS ES+ve *m/z* 222 [M+H]+.

**Intermediate 15: 7-(Difluoromethoxy)-6-fluoroquinoline-3-carboxylic acid hydrochloride**

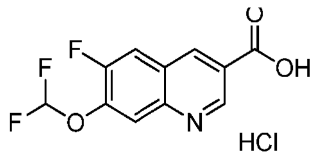

A. **6-Fluoro-7-hydroxyquinoline-3-carboxylic acid hydrobromide**

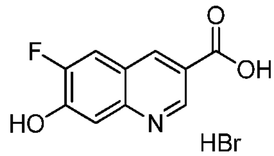

Ethyl 6-fluoro-7-methoxyquinoline-3-carboxylate (Intermediate 13, Step C) (6.6 g, 26.5 mmol) was treated with aqueous hydrobromic acid (48 %) (120 mL, 2210 mmol) and the mixture was heated at 140 °C for 18 h. The cooled solution was evaporated to dryness and the residue was suspended in water and filtered. The recovered solid was washed with water and dried *in vacuo* to afford the title compound (5.33 g). MS ES+ve m/z 288 [M+H]+.

B. **7-(Difluoromethoxy)-6-fluoroquinoline-3-carboxylic acid hydrochloride**

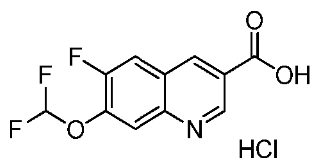

A mixture of 6-fluoro-7-hydroxyquinoline-3-carboxylic acid hydrobromide (2 g, 6.96 mmol), sodium 2-chloro-2,2-difluoroacetate (2.124 g, 13.93 mmol) and potassium carbonate (3.85 g, 27.9 mmol) was treated with DMF (25 mL)

and water (5 mL) and the mixture was heated at 100 °C for 90 min and then cooled. The mixture was cautiously adjusted to pH 1 with 2 M aqueous hydrochloric acid. The mixture was evaporated to dryness and the residue was taken up in water (150 mL) and filtered. The recovered solid was washed with 1 M hydrochloric acid (50 mL). The combined filtrate and washings were evaporated to dryness and the residue was purified by reverse-phase column chromatography on C18-modified silica using a 10-50 % acetonitrile (+ 0.1 % formic acid) in water (+ 0.1 % formic acid) gradient. Product-containing fractions were combined and evaporated to dryness to afford the title compound (940 mg). $^1H$ NMR (400 MHz, $CD_3SOCD_3$) δ 7.60 (t, *J* = 73 Hz ,1 H), 8.00 (d, *J* = 8 Hz, 1 H), 8.25 (d, *J* = 11 Hz, 1 H), 8.99 (d, *J* = 2 Hz, 1 H), 9.31 (d, *J* = 2 Hz, 1 H), 13.57 (br s, 1 H). MS ES+ve m/z 238 $[M+H]^+$.

**Intermediate 16: 7-(Trifluoromethoxy)quinoline-3-carboxylic acid**

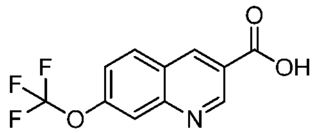

**A. Diethyl 2-(((3-(trifluoromethoxy)phenyl)amino)methylene)malonate**

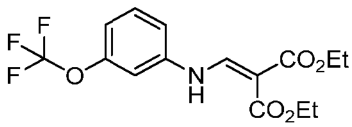

A mixture of 3-(trifluoromethoxy)aniline (1.52 g, 8.58 mmol) and diethyl 2-(ethoxymethylene)malonate (1.699 mL, 8.58 mmol) was heated at 100 °C for 1 h under nitrogen. After cooling the reaction mixture was concentrated *in vacuo* to afford the title compound (2.98 g). MS ES+ve m/z 348 $[M+H]^+$.

**B. Ethyl 4-chloro-7-(trifluoromethoxy)quinoline-3-carboxylate**

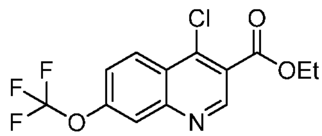

Diethyl 2-(((3-(trifluoromethoxy)phenyl)amino)methylene)malonate (2.98g, 8.58 mmol) was treated with $POCl_3$ (0.800 mL, 8.58 mmol), then transferred to a microwave vial, sealed and heated by microwave at 150 °C for 1 h. The cooled mixture was added to 5 N NaOH (20 mL) which was maintained at between 20 °C and 40 °C with ice-bath cooling. The resulting suspension was partitioned between water (150 mL) and DCM (200 mL). The aqueous phase was re-extracted with DCM (250 mL) and the combined organics were concentrated *in vacuo.* The residue was purified by column chromatography on silica using a 0-100 % EtOAc in cyclohexane gradient. Appropriate fractions were combined and evaporated *in vacuo* to give the title compound (833 mg). $^1H$ NMR (400 MHz, $CDCl_3$) δ 1.47 (t, *J* = 7 Hz, 3 H), 4.52 (q, *J* = 7 Hz, 2 H), 7.55 (dd, *J* = 9, 2 Hz, 1 H), 7.98 (d, *J* = 1 Hz, 1 H), 8.46 (d, *J* = 9 Hz, 1 H), 9.24 (s, 1 H). MS ES+ve m/z 320 $[M+H]^+$.

**C. 7-(Trifluoromethoxy)quinoline-3-carboxylic acid**

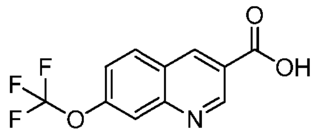

A solution of ethyl 4-chloro-7-(trifluoromethoxy)quinoline-3-carboxylate (833 mg, 2.61 mmol) and triethylamine (2.179 mL, 15.64 mmol) in EtOH (25 mL) was hydrogenated using an H-Cube® system (settings: 21 °C, full hydrogen, 1 mL/min flow rate) and a 10 % Pd/C CatCart 30 catalyst cartridge. LCMS analysis indicated ~10 % remaining starting material. The solution was hydrogenated a second time by the same method. The reaction mixture was concentrated *in vacuo* to afford a crude sample of ethyl 7-(trifluoromethoxy)-3,4-dihydroquinoline-3-carboxylate (478 mg). Lithium hydroxide (319 mg, 13.31 mmol) was suspended in water (75 mL) and was added to a solution of the crude ethyl 7-(trifluoromethoxy)-3,4-dihydroquinoline-3-carboxylate (478 mg) in MeOH (75 mL). THF (75 mL) was added, followed by an additional portion of lithium hydroxide (319 mg, 13.31 mmol) in water (75 mL). The reaction mixture was heated at 70 °C for 24 h. The reaction mixture was concentrated *in vacuo* to remove the organic solvents and the remaining mixture was acidified with 2 N HCl (100 mL). The suspension was extracted with DCM (2 x 200 mL) and the combined extracts were dried over $MgSO_4$ and concentrated *in vacuo*. The residue was purified by column chromatography on silica using a 0-50 % MeOH in DCM gradient. Appropriate fractions were combined and evaporated *in vacuo* to give the title compound (33 mg). $^1H$ NMR (400 MHz, $CD_3OD$) $\delta$ 7.58 (d, *J* = 9 Hz, 1 H), 7.92 (s, 1 H), 8.17 (d, *J* = 9 Hz, 1 H), 8.92 (s, 1 H), 9.47 (d, *J* = 2 Hz, 1 H). MS ES+ve m/z 258 $[M+H]^+$.

**Intermediate 17: Lithium 2,3-dihydro-[1,41dioxino[2,3-g]quinoline-8-carboxylate**

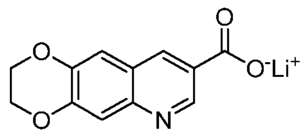

**A. Diethyl 2-(((2,3-dihydrobenzo[b][1,4]dioxin-6-yl)amino)methylene)malonate**

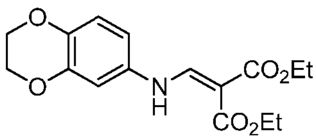

2,3-dihydrobenzo[b][1,4]dioxin-6-amine (1 g, 6.62 mmol) and diethyl 2-(ethoxymethylene)malonate (1.430 g, 6.62 mmol) were heated with microwave irradiation at 100°C for 2 h. The reaction mixture was cooled to RT, dissolved in DCM and purified by flash column chromatography (0-100% EtOAc in cyclohexane) to yield the title compound (2.126 g, 6.62 mmol, 100% yield). MS ES+ve *m/z* 322 $[M+H]^+$.

**B. Ethyl 9-chloro-2,3-dihydro-[1,4]dioxino[2,3-g]quinoline-8-carboxylate**

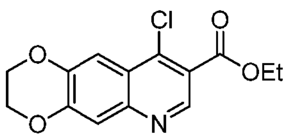

Diethyl 2-(((2,3-dihydrobenzo[b][1,4]dioxin-6-yl)amino)methylene)malonate (2.126 g, 6.62 mmol) and $POCl_3$ (30.4 g, 198 mmol) were heated with microwave irradiation to 160°C for 20 min.The reaction was quenched by dropwise

addition to ice cold NaOH solution (150 mL, 5 N), being careful not to allow the temperature to exceed 40°C. The product was then filtered via a Büchner funnel and washed with distilled water. The sample was loaded preabsorbed on Florosil® and purified by silica gel chromatography using 0-100% EtOAc in cyclohexane gradient. The appropriate fractions were combined and *evaporated in vacuo* to give the title compound (1.35 g, 4.60 mmol, 69.5 % yield) as a yellow solid. $^1H$ NMR (400 MHz, $CDCl_3$) δ 1.48 (t, *J* = 7 Hz, 3 H), 4.46-4.57 (m, 6 H), 7.33 (d, *J* = 9 Hz, 1 H), 7.94 (d, *J* = 9 Hz, 1 H), 9.17 (s, 1 H). MS ES+ve *m/z* 294 $[M+H]^+$.

**C. Ethyl 2,3-dihydro-[1,4]dioxino[2,3-g]quinoline-8-carboxylate**

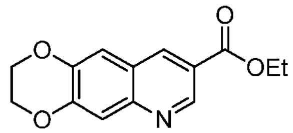

A hydrogenation flask containing Palladium on carbon (0.098 g, 0.919 mmol) was flushed three times with nitrogen and a solution of ethyl 9-chloro-2,3-dihydro-[1,4]dioxino[2,3-g]quinoline-8-carboxylate (1.35 g, 4.60 mmol) and triethylamine (2.79 g, 27.6 mmol) in EtOH (300 mL) was added under vacuum. The flask was flushed three more times with nitrogen prior to stirring under $H_2$ (g) for 1 h. The reaction mixture was then flushed with nitrogen three times prior to isolation via filtration through Celite® under a blanket of nitrogen. The filtrate was concentrated in vacuo and purified by reverse phase column chromatography (120 g C18 column, 5-95% MeCN in water + 0.1% formic acid) to yield the title compound (0.544 g, 2.10 mmol, 45.7 % yield) as a white solid. $^1H$ NMR (400 MHz, $CDCl_3$) δ 1.47 (t, *J* = 7 Hz, 3 H), 4.46-4.57 (m, 6 H), 7.27 (d, *J* = 9 Hz, 1 H), 7.47 (d, *J* = 9 Hz, 1 H), 8.76 (d, *J* = 2 Hz, 1 H), 9.41 (d, J = 2 Hz, 1 H). MS ES+ve *m/z* 260 $[M+H]^+$.

**D. Lithium 2,3-dihydro-[1,4]dioxino[2,3-g]quinoline-8-carboxylate**

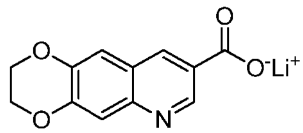

Ethyl 2,3-dihydro-[1,4]dioxino[2,3-g]quinoline-8-carboxylate (0.5 g, 1.929 mmol) and lithium hydroxide (0.185 g, 7.71 mmol) were dissolved in MeOH (15 mL) and water (2 mL) and heated to 65 °C for 2 h. The reaction mixture was concentrated *in vacuo* to afford the title compound as the lithium salt (0.459 g, 1.929 mmol, 100% yield). The material was used without further purification. MS ES+ve *m/z* 232 $[M+H]^+$.

**Intermediate 18: 2,3-dihydrofuro[3,2-h]quinoline-7-carboxylic acid**

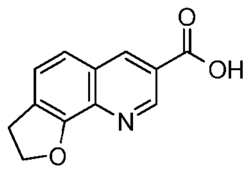

**A. Diethyl 2-(((2,3-dihydrobenzofuran-7-yl)amino)methylene)malonate**

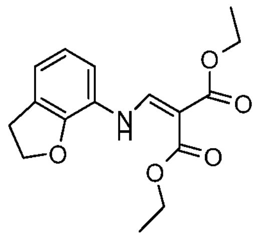

A mixture of 2,3-dihydrobenzofuran-7-amine (500 mg, 3.70 mmol) and diethyl 2-(methoxymethylene)malonate (785 mg, 3.88 mmol) in EtOH (10 mL) was sealed and heated in a microwave at 150 °C for 2 h. The cooled mixture was evaporated to dryness and used for the next reaction without further isolation.

**B. Ethyl 6-chloro-2,3-dihydrofuro[3,2-h]quinoline-7-carboxylate**

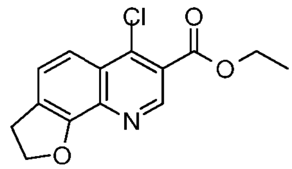

To a microwave tube was added phosphorus oxychloride (6.84 mL, 73.4 mmol) followed by diethyl 2-(((2,3-dihydrobenzofuran-7-yl)amino)methylene)malonate (1.12 g, 3.67 mmol). The reaction was heated at 120 °C for 45 min. The solution was diluted with EtOAc (150 mL), cooled to 0 °C in an ice bath, and the pH was adjusted to 7 with saturated $NaHCO_3$. The organic layer was separated, and the aqueous layer re-extracted with EtOAc (2X100 mL). The combined organic layers were absorbed on silica gel and purified by silica gel chromatography (5-40% EtOAc/hexanes) to provide the title compound (478 mg, 47% yield). MS (ESI) *m/z* 278(M+1).

**C. Ethyl 2,3-dihydrofuro[3,2-h]quinoline-7-carboxylate**

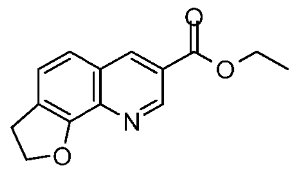

To an $N_2$ degassed solution of ethyl 6-chloro-2,3-dihydrofuro[3,2-h]quinoline-7-carboxylate (400 mg, 1.440 mmol) in acetonitrile (30 mL) was added bis(triphenylphosphine)palladium(II) chloride (202 mg, 0.288mmol) followed by triethylsilane (0.690 mL, 4.32 mmol). After heating overnight at 70 °C, the crude reaction was absorbed onto silica gel and purified by silica gel chromatography (5-50% EtOAc/hexanes) to provide the title compound (231 mg, 66% yield). MS (ESI) *m/z* 244(M+1).

**D. 2,3-dihydrofuro[3,2-h]quinoline-7-carboxylic acid**

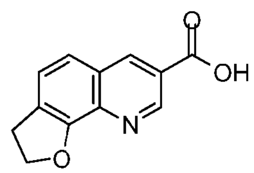

A suspension of lithium hydroxide (68.2 mg, 2.85 mmol) in THF (5 mL), and EtOH (5 mL) was treated with a solution of ethyl 2,3-dihydrofuro[3,2-h]quinoline-7-carboxylate (231 mg, 0.950 mmol) in water (5 mL). The mixture was heated at 60 °C for 2 h. The cooled mixture was evaporated to dryness and the residue was taken up in water (60 mL) and acidified to pH 6 using 2 M aqueous HCl. The precipitated product was filtered off, washed with water and dried under high vacuum to afford the title compound as a white solid (200 mg, 98 % yield). MS (ESI) *m/z* 216(M+1).

**Intermediate 19: 7-Hydroxyquinoline-3-carboxylic acid**

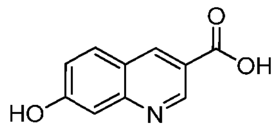

Triethylammonium 7-methoxyquinoline-3-carboxylate (Intermediate 1) (1 g, 4.92 mmol) and iodotrimethylsilane (5g, 24.99 mmol) were slurried in acetonitrile (10 mL) in a sealed vial and heated in the microwave to 180 °C for 10 min. An additional aliquot of iodotrimethylsilane (5g, 24.99 mmol) was added and the reaction was heated to 180 °C for an additional 10 min. The reaction mixture was quenched by the careful addition to water and EtOAc. The aqueous and organic layers were separated and the organic layer was washed with saturated sodium thiosulphate solution. The organic layer was concentrated in vacuo to yield the title compound (0.82 g, 88 % yield) which was used without further purification. $^1H$ NMR (400 MHz, $CD_3SOCD_3$) $\delta$ 7.24 (dd, *J* = 9, 2 Hz, 1 H), 7.31 (d, *J* = 2 Hz, 1 H), 8.02 (d, *J* = 9 Hz, 1 H), 8.77 (s, 1 H), 9.16 (s, 1 H), 10.55 (s, 1 H), 13.17 (br s, 1 H). MS ES+ve *m/z* 190 $[M+H]^+$.

**Intermediate 20: 6-Chloro-7-(difluoromethoxy)quinoline-3-carboxylic acid**

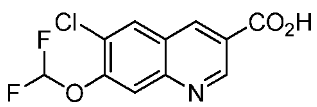

**A. Ethyl 6-chloro-7-(4,4,5,5-tetramethyl-1,3,2-dioxaborolan-2-yl)quinoline-3-carboxylate**

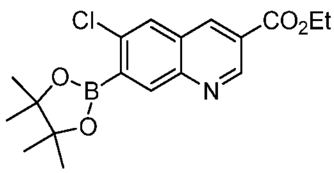

A 6L JLR was charged with ethyl 7-bromo-6-chloroquinoline-3-carboxylate (Intermediate 4, step B) (506 g, 1609 mmol), bis(pinacolato)diboron (490 g, 1930 mmol), $PdCl_2(dppf{\cdot}CH_2Cl_2$ (32.8 g, 40.2 mmol), KOAc (395 g, 4021 mmol), and dioxane (4 L) and the contents were sparged with nitrogen. Over the course of ca. 1 h the internal temperature was raised to 80 °C, at which point nitrogen sparging was ceased. N.B.: at some point over the next 4 h jacket heating inadvertantly stopped. Jacket heating was resumed and internal temperature was raised from 40 to 80 °C. Additional catalyst (16 g) was added after 30 min and stirring/heating maintained overnight. Additional KOAc (200 g), bis(pinacolato)diboron (245 g) and catalyst (16 g) were added after 23.5 h and stirring/heating maintained. Heating was discontinued after 59 h. Upon cooling, the mixture was diluted with EtOAc (4 L) and filtered through Celite® (EtOAc wash). The filtrate was concentrated *in vacuo* (4 x 1 L EtOH chase). The residue was slurried in EtOH (500 mL) and aged overnight. The mixture was cooled in an ice bath and solids were collected by filtration. The cake was washed with the liquor and dried on the filter affording the title compound (411 g, 71 % yield) as a beige solid. $^1H$ NMR (400 MHz, $CDCl_3$) $\delta$ ppm 1.42 (s, 12 H), 1.46 (t, *J* = 7 Hz, 3 H), 4.48 (q, *J* = 7 Hz, 2 H), 7.91 (s, 1 H), 8.51 (s, 1 H), 8.72 (d, *J* = 2 Hz, 1 H), 9.43 (d, *J* = 2 Hz, 1 H). MS (ESI) *m/z* 280 $(Ar\text{-}B(OH)_2 + H)$.

**B. Ethyl 6-chloro-7-hydroxyquinoline-3-carboxylate**

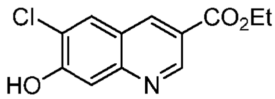

A 6 L JLR was charged with ethyl 6-chloro-7-(4,4,5,5-tetramethyl-1,3,2-dioxaborolan-2-yl)quinoline-3-carboxylate (328.1 g, 907 mmol), $K_2CO_3$ (125 g, 907 mmol), and EtOH (3 L) and cooled to *ca.* 2 °C (internal temp). 30% $H_2O_2$ (0.093 L, 910 mmol) was added *via* a dropping addition funnel at such a rate that the internal temp remained ≤ 20°C (ca. 25 min). After 15 min the jacket temperature was raised to 10 °C and the mixture was stirred 30 min. Jacket cooling was discontinued and the mixture was stirred at RT. Additional $H_2O_2$ (9.3 mL; 91 mmol) was added after stirring 3 h at RT. After 20 min the jacket temp was set to 5 °C and the mixture was stirred 15 min. The mixture was quenched by addition of 1 M $Na_2S_2O_3$ (181 mL; 181 mmol), stirred 10 min and 1 N HCl (910 mL) was added. Precipitated solids were collected by filtration, washed with water and dried on the Buchner funnel affording the title compound (198.3 g, 87 % yield) as a beige solid. $^1H$ NMR (400 MHz, $CD_3SOCD_3$) δ ppm 1.36 (t, *J* = 7 Hz, 3 H), 4.38 (q, *J* = 7 Hz, 2 H), 7.48 (s, 1 H), 8.31 (s, 1 H), 8.82 (d, *J* = 2 Hz, 1 H), 9.17 (d, *J* = 2 Hz, 1 H), 10.62 - 12.55 (m, 1 H). MS (ESI) *m/z* 252 (M+H).

**C. Ethyl 6-chloro-7-(difluoromethoxy)quinoline-3-carboxylate**

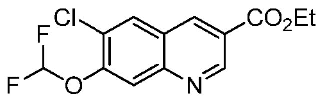

A 6 L JLR was charged with ethyl 6-chloro-7-hydroxyquinoline-3-carboxylate (207 g, 823 mmol), $K_2CO_3$ (341 g, 2470 mmol), DMF (2 L), and water (200 mL) and heated to 95 °C (internal temp). Meanwhile, sodium 2-chloro-2,2-difluoroacetate (251 g, 1645 mmol) was dissolved in DMF (300 mL) in a RB flask under nitrogen. The sodium 2-chloro-2,2-difluoroacetate solution was added dropwise *via* dropping addition funnel to the hot mixture over 90 min (no exotherm noted). An additional portion of sodium 2-chloro-2,2-difluoroacetate (37.6 g; 272 mmol) was added after 4 h and stirring continued. Jacket heating was discontinued after ca. 1 h and the mixture was allowed to cool to RT overnight. Water (9 L) was added and the mixture was stirred ca. 1 h. Solids were collected by filtration and the cake was washed with water (2 x 1 L). The cake was dried on the filter over a weekend affording ca. 210 g orange solid. The crude solid was slurried in TBME (350 mL) and heptane (1.2 L) was added slowly. The mixture was stirred ca. 1 h in an ice bath and solids were collected by filtration. The cake was washed with the liquor and a small amount of heptane and dried on the filter affording the title compound (204 g, 82 % yield) as a light orange powder, used without further purification. $^1H$ NMR (400 MHz, $CD_3SOCD_3$) δ ppm 1.38 (t, *J* = 7 Hz, 3 H), 4.42 (q, *J* = 7 Hz, 2 H), 7.66 (t, J = 72 Hz, 1 H), 7.96 (s, 1 H), 8.58 (s, 1 H), 9.01 (d, *J* = 2 Hz, 1 H), 9.33 (d, *J* = 2 Hz, 1 H). MS (ESI) *m/z* 302 (M+H).

**D. 6-Chloro-7-(difluoromethoxy)quinoline-3-carboxylic acid**

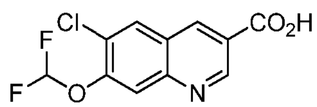

A 6L JLR was charged with 2-MeTHF (2 L), ethyl 6-chloro-7-(difluoromethoxy)quinoline-3-carboxylate (215.2 g, 713 mmol), LiOH·$H_2O$ (90 g, 2140 mmol), and water (100 mL) and the mixture was heated under reflux. After 40 min the mixture was cooled to 20 °C, water (5 L) and ether (1 L) were added and the mixture was stirred vigorously ca. 5 min. Stirring was slowed (40 RPM) and the mixture was allowed to partition. Layers were separated and the aqueous layer was extracted with ether (1 L). The aqueous layer was filtered and acidified to pH 5.5 by addition of 1 N HCl. Precipitated solids were collected by filtration. Aqueous filtrate pH had risen to ca. 6 on standing; additional 1N HCl was

added to pH 5.5 and precipitated solids were collected by filtration (combined with first crop). The cake was dried on the filter over a weekend, suspended in TBME (500 mL) and heptane (1 L) was slowly added with stirring. The mixture was cooled in an ice bath and solids were collected by filtration. The cake was dried in a vacuum oven overnight (65°C /20" Hg vacuum) affording the title compound (178 g, 91 % yield) as a beige solid, used without further purification. $^1H$ NMR (400 MHz, $CD_3SOCD_3$) δ ppm 7.64 (t, *J* = 72 Hz, 1 H), 7.95 (s, 1 H), 8.56 (s, 1 H), 8.98 (d, *J* = 2 Hz, 1 H), 9.33 (d, *J* = 2 Hz, 1 H), 13.66 (br s, 1 H). MS (ESI) *m/z* 274 (M+H).

**Intermediate 21: 2,2-Difluorocyclopropyl)-6-fluoroquinoline-3-carboxylic acid**

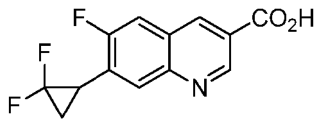

**A. Ethyl 6-fluoro-7-hydroxyquinoline-3-carboxylate**

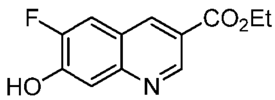

A mixture of 6-fluoro-7-hydroxyquinoline-3-carboxylic acid hydrochloride (Intermediate 15, step A) (33.57 g, 162 mmol) and $H_2SO_4$ (9.50 mL, 178 mmol) in EtOH (200 mL) was heated under reflux. Additional portions of $H_2SO_4$ (4 mL) were added after 4 h and 24 h. After 48 h the hot mixture was filtered through Celite® (EtOH wash). The filtrate was concentrated *in vacuo*. The residue was slurried in EtOAc and neutralized by slow addition of sat. $NaHCO_3$. The mixture was stirred 30 min and the whole was filtered, affording ca. 17 g crude product. Layers of the filtrate were separated and treated as follows: The aqueous layer was adjusted to ca. pH 6 by addition of conc HCl. Precipitated solids were collected by filtration and combined with the crude product obtained above. The solid was dissolved in refluxing 1:1 EtOAc/EtOH (ca. 500 mL) and the hot mixture was filtered. The filtrate was concentrated *in vacuo* and the residue was recrystallized from EtOAc/EtOH (1:1) affording 14.3 g of the title compound. The mother liquor was concentrated and the residue was triturated with $Et_2O$ affording a second crop (6.21 g) of the title compound (combined with the first crop). The organic layer was dried over $Na_2SO_4$ and concentrated *in vacuo.* The residue was purified by flash chromatography (silica gel, EtOAc / hexanes, gradient elution) affording an additional 2.18 g title compound which was combined with batch obtained above. Thus, total yield of the title compound was 22.7 g (96 mmol; 60%) as pale yellow needles. $^1H$ NMR (400 MHz, $CD_3SOCD_3$) δ ppm 1.36 (t, *J* = 7 Hz, 3 H), 4.38 (q, *J* = 7 Hz, 2 H), 7.48 (d, *J* = 8 Hz, 1 H), 7.99 (d, *J* = 11 Hz, 1 H), 8.83 (d, *J* = 2 Hz, 1 H), 9.15 (d, *J* = 2 Hz, 1 H), 11.35 (br s, 1 H). MS (ESI) *m/z* 236 (M+H).

**B. Ethyl 6-fluoro-7-(((trifluoromethyl)sulfonyl)oxy)quinoline-3-carboxylate**

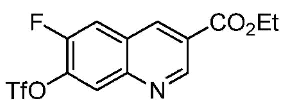

To a solution of ethyl 6-fluoro-7-hydroxyquinoline-3-carboxylate (2.857 g, 12.15 mmol) and $Et_3N$ (1.86 mL, 13.4 mmol) in DCM (100 mL) at 0 °C was added $Tf_2O$ (2.16 mL, 12.8 mmol), dropwise. After 90 min the mixture was poured into water and extracted with DCM (x3). Combined organics were washed (water, brine), dried over $Na_2SO_4$, and concentrated *in vacuo.* The residue was purified by flash chromatography (silica gel, EtOAc / hexanes, gradient elution) affording the title compound (3.06 g, 69 % yield) as a pale yellow solid. $^1H$ NMR (400 MHz, $CDCl_3$) δ ppm 1.48 (t, *J* = 7 Hz, 3 H), 4.51 (q, *J* = 7 Hz, 2 H), 7.78 (d, *J* = 9 Hz,1 H), 8.18 (d, *J* = 7 Hz, 1 H), 8.83 (d, *J* = 2 Hz, 1 H), 9.49 (d, *J* = 2 Hz, 1 H).

**C. Ethyl 6-fluoro-7-vinylquinoline-3-carboxylate**

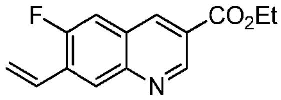

A 20 mL microwave vial was charged with ethyl 6-fluoro-7-(((trifluoromethyl)sulfonyl)oxy)quinoline-3-carboxylate (1 g, 2.72 mmol), potassium vinyltrifluoroborate (0.401 g, 3.00 mmol), and $PdCl_2(dppf)\cdot CH_2Cl_2$ (0.111 g, 0.136 mmol) and sealed with a rubber septum. The vial was evacuated/backfilled with nitrogen (3X). Degassed *i*-PrOH (10 mL) and water (5 mL) were added *via* syringe followed by DIEA (1.427 mL, 8.17 mmol). The septum was replaced with a crimp top and the mixture was subjected to microwave heating (100 °C) for 15 min. Upon cooling the mixture was diluted with water and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound (0.443 g, 1.81 mmol, 66 % yield) as a pale yellow solid. $^1H$ NMR (400 MHz, $CDCl_3$) $\delta$ ppm 1.47 (t, *J* = 7 Hz, 3 H), 4.48 (q, *J* = 7 Hz, 2 H), 5.62 (d, *J* = 11 Hz, 1 H), 6.14 (d, *J* = 18 Hz, 1 H), 7.02 (dd, *J* = 18, 11 Hz, 1 H), 7.53 (d, *J* = 10 Hz, 1 H), 8.27 (d, *J* = 7 Hz, 1 H), 8.73 (d, *J* = 2 Hz, 1 H), 9.39 (d, *J* = 2 Hz, 1 H). MS (ESI) *m/z* 246 (M+H).

**D. Ethyl 7-(2,2-difluorocyclopropyl)-6-fluoroquinoline-3-carboxylate**

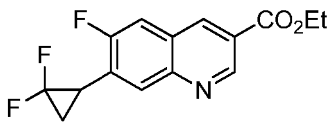

An oven-dried 20 mL microwave vial was charged with ethyl 6-fluoro-7-vinylquinoline-3-carboxylate (0.240 g, 0.979 mmol) and sodium iodide (0.029 g, 0.196 mmol), sealed with a rubberseptum and flushed with nitrogen. THF (4 mL) and trimethyl(trifluoromethyl)silane (0.362 mL, 2.446 mmol) were added *via* syringe and the septum was replaced with a crimp top. The mixture was stirred in an oil bath at 65 °C for 2 h. Upon cooling the mixture was poured into water and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) to give the title compound (0.0351 g, 12 % yield) as an orange solid. $^1H$ NMR (400 MHz, $CDCl_3$) $\delta$ ppm 1.47 (t, *J* = 7 Hz, 3 H), 1.83 - 1.94 (m, 1 H), 1.98 - 2.10 (m, 1 H), 2.97 - 3.10 (m, 1 H), 4.49 (q, *J* = 7 Hz, 2 H), 7.58 (d, *J* = 10 Hz, 1 H), 7.97 (d, *J* = 7 Hz, 1 H), 8.75 - 8.80 (m, 1 H), 9.40 (d, *J* = 2 Hz, 1 H). MS (ESI) *m/z* 296 (M+H).

**E. 7-(2,2-Difluorocyclopropyl)-6-fluoroquinoline-3-carboxylic acid**

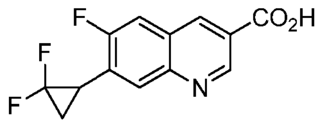

A mixture of ethyl 7-(2,2-difluorocyclopropyl)-6-fluoroquinoline-3-carboxylate (0.0651 g, 0.220 mmol) and $LiOH\cdot H_2O$ (0.046 g, 1.102 mmol) in THF (2 mL) and water (0.2 mL) was stirred in a heating block at 65 °C (sealed vial) for 2.5 h. Upon cooling the mixture was poured into water and extracted with $Et_2O$. The aqueous layer was acidified (ca. pH 2) by addition of 1 N $KHSO_4$ and extracted with EtOAc (3X). Combined organics were dried over $Na_2SO_4$ and concentrated *in vacuo* affording the title compound (0.0596 g, 101 % yield) as an orange solid which was used without further purification. MS (ESI) *m/z* 268 (M+H).

**Intermediate 22: 6-Bromo-7-(difluoromethoxy)quinoline-3-carboxylic acid**

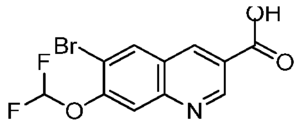

A. 2-Amino-5-bromo-4-methoxybenzoic acid

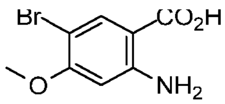

A 250 mL reaction vessel equipped with overhead stirrer was charged with 2-amino-4-methoxybenzoic acid (11.35 g, 67.9 mmol) in DMF (100 mL). The solution was cooled to 0 °C and N-bromosuccinimide (12.08 g, 67.9 mmol) was added such that the temperature remains less than 10 °C. After the addition was complete, the ice bath was removed and the reaction mixture was stirred at RT. The reaction was monitored by LCMS and after 10 min the reaction had progressed to completion. The mixture was poured over ice water and the solid filtered off and dried overnight in a vacuum oven with nitrogen purge at 60 °C to give the title compound as an off white solid (16.50 g, 99 % yield). $^1$H NMR (400 MHz, $CD_3SOCD_3$) δ ppm 3.80 (s, 3 H), 6.42 (s, 1 H), 7.77 (s, 1 H).

B. (2-Amino-5-bromo-4-methoxyphenyl)methanol

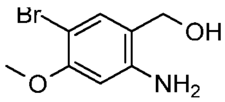

A 500 mL reaction vessel equipped with overhead stirrer was charged with lithium aluminum hydride (35.6 mL, 71.1 mmol, 2 M in THF) and THF (50 mL). The contents were cooled to 0 °C in an ice bath under a nitrogen flow and then 2-amino-5-bromo-4-methoxybenzoic acid (10 g, 40.6 mmol) in THF (100 mL) was added slowly maintaining a temperature less than 10 °C over 15 min. The reaction mixture was stirred at RT for 4 h. The reaction was diluted with $Et_2O$ (50 mL) and cooled to 0 °C to this was then added; first 2.70 mL water, followed by 2.70 mL of 15% NaOH, and then 8.09 mL water, while maintaining an internal temp below 10 °C. The mixture was stirred for 1 h and then filtered. The filtrate was concentrated to a thick amber oil which solidified upon standing. The solid was dissolved in a minimal amount of iPrOAc and then toluene added until cloudy. The crude material was sonicated until precipitate formed and was then filtered to give the title compound as an off-white solid (7.15 g, 76 % yield). $^1$H NMR (400 MHz, $CD_3SOCD_3$) δ ppm 3.72 (s, 3 H), 4.30 (d, *J* = 5 Hz, 2 H), 4.98 (t, *J* = 5 Hz, 1 H), 5.12 (s, 2 H), 6.39 (s, 1 H), 7.16 (s, 1 H).

C. 2-Amino-5-bromo-4-methoxybenzaldehyde

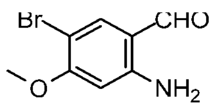

A 100 mL reaction vessel equipped with magnetic stirrer was charged with (2-amino-5-bromo-4-methoxyphenyl)methanol (3.140 g, 13.53 mmol) in DCM (30 mL) and manganese dioxide (7.06 g, 81 mmol) was added. The reaction mixture was stirred for 3 h at RT and then filtered through a Celite® plug. The cake was washed several times with DCM and then a final rinse with EtOAc to assure that all the material was washed off the $MnO_2$ and the filtrate concentrated to a solid. This was recrystallized from EtOH to give the title compound as a yellowish-brown solid (2.56 g, 82 % yield). $^1$H NMR (400 MHz, $CD_3SOCD_3$) δ ppm 3.83 (s, 3 H), 6.40 (s, 1 H), 7.32 (br s, 2 H), 7.72 (s, 1 H), 9.62 (s, 1 H).

**D. Ethyl 6-bromo-7-methoxyquinoline-3-carboxylate**

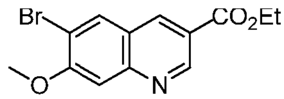

A 100 mL reaction vessel equipped with magnetic stirrer was charged with 2-amino-5-bromo-4-methoxybenzaldehyde (2.710 g, 11.78 mmol), and ethyl 3,3-diethoxypropanoate (5.60 g, 29.4 mmol) in toluene (30 mL). To this was added p-toluenesulfonic acid monohydrate (0.224 g, 1.178 mmol) and the mixture was heated at a gentle reflux. After 4 h the mixture was allowed to cool to RT with the precipitation of solid. This was filtered and dried to a pale yellow solid and used without further purification (2.56 g, 70% yield). $^1H$ NMR (400 MHz, $CD_3SOCD_3$) $\delta$ ppm 1.38 (t, *J* = 7 Hz, 3 H), 4.05 (s, 3 H), 4.40 (q, *J* = 7 Hz, 2 H), 7.58 (s, 1 H), 8.56 (s, 1 H), 8.90 (s, 1 H), 9.26 (s, 1 H). MS (ESI) *m*/*z* 310, 312 (M+1).

**E. Ethyl 6-bromo-7-hdroxuinoline-3-carboxlate**

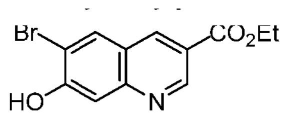

A 50 mL reaction vessel equipped with magnetic stirrer was charged with ethyl 6-bromo-7-methoxyquinoline-3-carboxylate (2.50 g, 8.06 mmol) in 1,2-dichloroethane (25 mL) and to this was added aluminum chloride (4.84 g, 36.3 mmol) portion-wise. The reaction was warmed to 55 °C. After 1.5 h the mixture was cooled and diluted with DCM (50 mL) and poured into ice water. The mixture was stirred for 3 h and the layers separated. The DCM layer was washed with 2 M $Na_2CO_3$. The aqueous phase was acidified with conc. HCl and then extracted with EtOAc (2 x 150 mL). The organics were dried ($MgSO_4$), filtered, and concentrated to give the title compound as a white solid (2.03 g, 85% yield). $^1H$ NMR (400 MHz, $CD_3SOCD_3$) $\delta$ ppm 1.37 (t, *J* = 7 Hz, 3 H), 4.39 (q, *J* = 7 Hz, 2 H), 7.47 (s, 1 H), 8.51 (s, 1 H), 8.85 (s, 1 H), 9.19 (s, 1 H), 11.62 (br s, 1 H).

**F. Ethyl 6-bromo-7-(difluoromethoxy)quinoline-3-carboxylate**

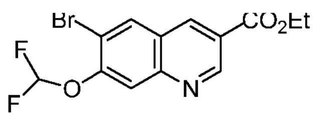

To a solution of potassium carbonate (1.059 g, 7.66 mmol) in DMF (5 mL), heated to 90 °C was added a slurry of sodium 2-chloro-2,2-difluoroacetate (1.946 g, 12.77 mmol) and ethyl 6-bromo-7-hydroxyquinoline-3-carboxylate (1.890 g, 6.38 mmol) in DMF (10.00 mL) over a period of 10 min maintaining an internal temp of 90 °C. The stirred mixture was kept at 90 °C for 30 min and then cooled to RT. The reaction was diluted with water and the solid filtered off and dried. This was triturated from a mixture of 10 mL MTBE/Heptane (4:1 v/v) to give the title compound as a tan solid (1.35 g, 61%). $^1H$ NMR (400 MHz, $CD_3SOCD_3$) $\delta$ ppm 1.39 (t, *J* = 7 Hz, 3 H), 4.42 (q, *J* = 7 Hz, 2 H), 7.66 (t, *J* = 74 Hz, 1 H), 7.92 (s, 1 H), 8.75 (s, 1 H), 9.01 (br s, 1 H), 9.34 (s, 1 H). MS (ESI) *m*/*z* 346, 348 (M+1).

**G. 6-Bromo-7-(difluoromethoxy)quinoline-3-carboxylic acid**

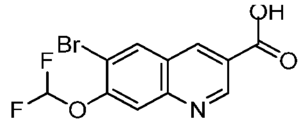

To a solution of ethyl 6-bromo-7-(difluoromethoxy)quinoline-3-carboxylate (1.25 g, 3.61 mmol) in THF (4 mL), MeOH (1 mL), and water (1 mL) was added lithium hydroxide mono-hydrate (0.455 g, 10.83 mmol) and the reaction warmed to 45 °C with a water bath until the mixture became a homogeneous solution. The mixture was concentrated and the residue was taken back up into water and to this was added $NaH_2PO_3$ until reaching a pH of 5.5-6. This was stirred for 1 h and then filtered and the solids dried in vacuo at 55 °C to give the title compound as a white solid (0.51 g, 43 % yield). A second crop of product was also isolated (0.489 g, 41 % yield). $^1H$ NMR (400 MHz, $CD_3SOCD_3$) δ ppm 7.64 (t, *J* = 74 Hz, 1 H), 7.90 (s, 1 H), 8.71 (s, 1 H), 8.97 (s, 1 H), 9.33 (s, 1 H). MS (ESI) *m/z* 318, 320 (M+1).

**Intermediate 23: 7-(Dimethylamino)-6-fluoroquinoline-3-carboxylic acid ammonia salt**

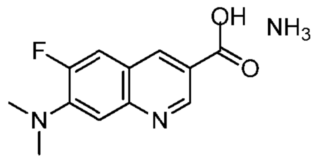

**A. Ethyl 6,7-difluoro-4-oxo-1-((2-(trimethylsilyl)ethoxy)methyl)-1,4-dihydroquinoline-3-carboxylate**

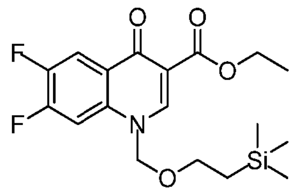

Sodium hydride (0.337 g, 8.42 mmol) was added to ethyl 6,7-difluoro-4-oxo-1,4-dihydroquinoline-3-carboxylate (2.03 g, 8.02 mmol) in DMF (38.5 mL) at RT, and then heated to 80 °C. Then, (2-(chloromethoxy)ethyl)trimethylsilane (1.56 mL, 8.82 mmol) was added and the reaction mixture was stirred for 16 h at 80 °C. The reaction mixture was poured into water, extracted with EtOAc, and concentrated. The residue was purified by silica gel chromatography, eluting with EtOAc:hexanes (3:2) to give the title compound (2.73 g, 78 % yield), containing a small amount of impurity of the same mass (8:1) that is presumably the O-alkylation product or an alkylated isomeric impurity in the starting material. $^1H$ NMR (400 MHz, $CD_3SOCD_3$) δ -0.10 (s, 9 H), 0.85 (t, *J* = 8 Hz, 2 H), 1.28 (t, *J* = 7 Hz, 3 H), 3.58 (t, *J* = 8 Hz, 2 H), 4.23 (q, *J* = 7 Hz, 2 H), 5.71 (s, 2 H), 7.92 (dd, *J* = 13, 7 Hz, 1 H), 8.08 (dd, *J* = 11, 9 Hz, 1 H), 8.82 (s, 1 H); LC-MS (LC-ES) M+H = 384.

**B. Ethyl 7-(dimethylamino)-6-fluoro-4-oxo-1-((2-(trimethylsilyl)ethoxy)methyl)-1,4-dihydroquinoline-3-carboxylate**

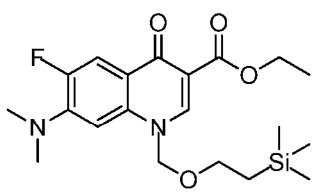

Dimethylamine (1.069 mL, 2.139 mmol, 2 M in THF) was added to ethyl 6,7-difluoro-4-oxo-1-((2-(trimethylsilyl)ethoxy)methyl)-1,4-dihydroquinoline-3-carboxylate (0.388 mL, 1.426 mmol) at RT, then DIEA (0.747 mL, 4.28 mmol) was added and the reaction mixture was heated at 80 °C for 66 h. The reaction mixture was poured into saturated sodium bicarbonate, extracted with DCM, dried over magnesium sulfate, filtered, and concentrated. The residue was purified by silica gel chromatography, eluting with EtOAc:hexanes (7:3) to give the title compound (0.2933 g, 47.8 % yield). $^1H$ NMR (400 MHz, $CD_3SOCD_3$) δ -0.09 (s, 9 H), 0.86 (t, *J* = 8 Hz, 2 H), 1.27 (t, *J* = 7 Hz, 3 H), 2.97 (s, 3 H), 2.98 (s, 3 H), 3.58 (t, *J* = 8 Hz, 2 H), 4.21 (q, *J* = 7 Hz, 2 H), 5.69 (s, 2 H), 6.99 (d, *J* = 8 Hz, 1 H), 7.71 (d, *J* = 14 Hz, 1 H), 8.67 (s, 1 H); LC-MS (LC-ES) M+H = 409.

**C. Ethyl 4-chloro-7-(dimethylamino)-6-fluoroquinoline-3-carboxylate**

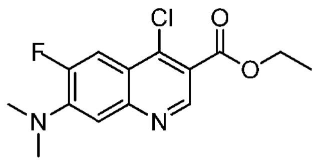

Phosphorus oxychloride (6.69 mL, 71.8 mmol) was added to ethyl 7-(dimethylamino)-6-fluoro-4-oxo-1-((2-(trimethylsilyl)ethoxy)methyl)-1,4-dihydroquinoline-3-carboxylate (0.208 mL, 0.718 mmol) at RT, then the reaction mixture was heated at 105 °C for 16 h. The reaction mixture was poured into ice, quenched with 5 N sodium hydroxide, extracted with DCM, dried over magnesium sulfate, filtered, and concentrated. The residue was purified by silica gel chromatography, eluting with EtOAc:hexanes (2:3) to give the title compound(0.1180 g, 52.6 % yield). $^1H$ NMR (400 MHz, $CD_3SOCD_3$) δ 1.36 (t, *J* = 7 Hz, 3 H), 3.06 (s, 3 H), 3.06 (s, 3 H), 4.38 (q, *J* = 7 Hz, 2 H), 7.32 (d, *J* = 9 Hz, 1 H), 7.93 (d, *J* = 15 Hz, 1 H), 9.00 (s, 1 H); LC-MS (LC-ES) M+H = 296.

**D. Ethyl 7-(dimethylamino)-6-fluoroquinoline-3-carboxylate**

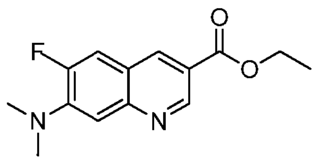

Bis(triphenylphosphine)palladium(II) chloride (0.013 g, 0.018 mmol) was added to ethyl 4-chloro-7-(dimethylamino)-6-fluoroquinoline-3-carboxylate (0.078 mL, 0.369 mmol) in acetonitrile (3.5 mL) at RT, then triethylsilane (0.082 mL, 0.516 mmol) was added and the reaction mixture was heated at 70 °C for 16 h. The reaction mixture was poured into saturated sodium bicarbonate, extracted with DCM, dried over magnesium sulfate, filtered, and concentrated. The residue was purified by silica gel chromatography, eluting with EtOAc:hexanes (1:2) to give the title compound (0.0652 g, 0.236 mmol, 64.1 % yield). $^1H$ NMR (400 MHz, $CD_3SOCD_3$) δ 1.36 (t, *J* = 7 Hz, 3 H), 3.02 (s, 3 H), 3.02 (s, 3 H), 4.37 (q, *J* = 7 Hz, 2 H), 7.30 (d, *J* = 9 Hz, 1 H), 7.89 (d, *J* = 14 Hz, 1 H), 8.76 (d, *J* = 2 Hz, 1 H), 9.13 (d, *J* = 2 Hz, 1 H); LC-MS (LC-ES) M+H = 263.

**E. 7-(Dimethylamino)-6-fluoroquinoline-3-carboxylic acid ammonia salt**

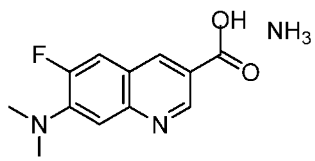

Lithium hydroxide (0.016 g, 0.652 mmol) was added to ethyl 7-(dimethylamino)-6-fluoroquinoline-3-carboxylate

(0.0570 g, 0.217 mmol) in MeOH (1.93 mL) and water (0.24 mL) at RT and the reaction mixture was stirred 2 h at 60 °C. The reaction mixture was concentrated. The reaction mixture was purified by RP HPLC eluting with acetonitrile:water with 0.1% ammonium hydroxide (5:95-100:0) to give the title compound (0.0503 g, 0.190 mmol, 88 % yield). $^1$H NMR (400 MHz, $CD_3SOCD_3$) δ 2.99 (s, 3 H), 2.99 (s, 3 H), 3.28 (br s, 4 H), 7.30 (d, *J* = 9 Hz, 1 H), 7.83 (d, *J* = 14 Hz, 1 H), 8.68 (d, *J* = 2 Hz, 1 H), 9.13 (d, *J* = 2 Hz, 1 H); LC-MS (LC-ES) M-H = 233.

**Intermediate 24: 6-Fluoro-7-(methylthio)quinoline-3-carboxylic acid ammonia salt**

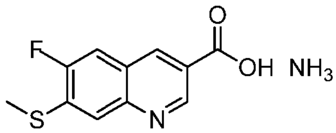

**A. Ethyl 6-fluoro-7-(methylthio)-4-oxo-1-((2-(trimethylsilyl)ethoxy)methyl)-1,4-dihydroquinoline-3-carboxylate**

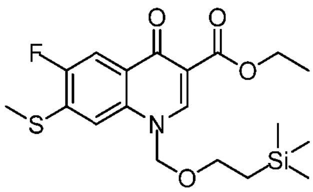

Sodium methanethiolate (0.163 mL, 1.964 mmol) was added to ethyl 6,7-difluoro-4-oxo-1-((2-(trimethylsilyl)ethoxy)methyl)-1,4-dihydroquinoline-3-carboxylate (Intermediate 23, step A) (0.356 mL, 1.309 mmol) at RT, then DIEA (0.457 mL, 2.62 mmol) was added and the reaction mixture was heated at 100 °C for 2 h. The reaction mixture was poured into saturated sodium bicarbonate, extracted with DCM, dried over magnesium sulfate, filtered, and concentrated. The residue was purified by silica gel chromatography, eluting with EtOAc:hexanes (3:2) to give the title compound (0.3645 g, 64.3 % yield). $^1$H NMR (400 MHz, $CD_3SOCD_3$) δ -0.09 (s, 9 H), 0.86 (t, *J* = 8 Hz, 2 H), 1.27 (t, *J* = 7 Hz, 3 H), 2.61 (s, 3 H), 3.59 (t, *J* = 8 Hz, 2 H), 4.23 (q, *J* = 7 Hz, 2 H), 5.79 (s, 2 H), 7.54 (d, *J* = 6 Hz, 1 H), 7.81 (d, *J* = 10 Hz, 1 H), 8.67 (s, 1 H); LC-MS (LC-ES) M+H = 412.

**B. Ethyl 4-chloro-6-fluoro-7-(methylthio)quinoline-3-carboxylate**

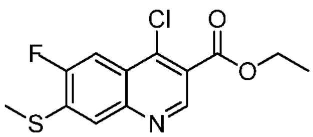

Phosphorus oxychloride (4.13 mL, 44.3 mmol) was added to ethyl 6-fluoro-7-(methylthio)-4-oxo-1-((2-(trimethylsilyl)ethoxy)methyl)-1,4-dihydroquinoline-3-carboxylate (0.259 mL, 0.886 mmol) at RT, then the reaction mixture was heated at 105 °C for 16 h. The reaction mixture was poured into ice, quenched with 5 N sodium hydroxide, extracted with DCM, dried over magnesium sulfate, filtered, and concentrated. The reaction mixture was purified by RP HPLC eluting with acetonitrile:water with 0.1% ammonium hydroxide (5:95-100:0) to give the title compound (0.0898 g, 32.1 % yield). $^1$H NMR (400 MHz, $CD_3SOCD_3$) δ 1.37 (t, *J* = 7 Hz, 3 H), 2.69 (s, 3 H), 4.41 (q, *J* = 7 Hz, 2 H), 7.94 (d, *J* = 8 Hz, 1 H), 8.06 (d, *J* = 11 Hz, 1 H), 9.11 (s, 1 H); LC-MS (LC-ES) M+H = 300.

**C. Ethyl 6-fluoro-7-(methylthio)quinoline-3-carboxylate**

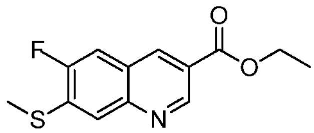

Bis(triphenylphosphine)palladium(II) chloride (9.78 mg, 0.014 mmol) was added to ethyl 4-chloro-6-fluoro-7-(methylthio)quinoline-3-carboxylate (0.059 mL, 0.279 mmol) in acetonitrile (2.79 mL) at RT, then triethylsilane (0.062 mL, 0.390 mmol) was added and the reaction mixture was heated at 70 °C for 16 h. The reaction mixture was poured into saturated sodium bicarbonate, extracted with DCM, dried over magnesium sulfate, filtered, and concentrated. The residue was purified by silica gel chromatography, eluting with EtOAc:hexanes (3:7) to give the title compound (0.0325 g, 38.7 % yield). $^1H$ NMR (400 MHz, $CD_3SOCD_3$) $\delta$ 1.37 (t, *J* = 7 Hz, 3 H), 2.67 (s, 3 H), 4.40 (q, *J* = 7 Hz, 2 H), 7.89 (d, *J* = 7 Hz, 1 H), 8.03 (d, *J* = 11 Hz, 1 H), 8.93 (d, *J* = 2 Hz, 1 H), 9.25 (d, *J* = 2 Hz, 1 H); LC-MS (LC-ES) M+H = 266.

**D. 6-Fluoro-7-(methylthio)quinoline-3-carboxylic acid ammonia salt**

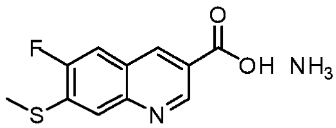

Lithium hydroxide (7.61 mg, 0.318 mmol) was added to ethyl 6-fluoro-7-(methylthio)quinoline-3-carboxylate (0.0281 g, 0.106 mmol) in MeOH (0.94 mL) and water (0.12 mL) at RT and the reaction mixture was stirred 2 h at 60 °C. The reaction mixture was concentrated. The reaction mixture was purified by RP HPLC eluting with acetonitrile:water with 0.1% ammonium hydroxide (5:95-100:0) to give the title compound (0.0195 g, 68.8 % yield). $^1H$ NMR (400 MHz, $CD_3SOCD_3$) $\delta$ 2.65 (s, 3 H), 7.84 (d, *J* = 7 Hz, 1 H), 7.93 (d, *J* = 11 Hz, 1 H), 8.76 (s, 1 H), 9.23 (d, *J* = 2 Hz, 1 H), 13.58 (br s, 1 H); LC-MS (LC-ES) M-H = 236.

**Intermediate 25: 6-Fluoro-7-(pyrrolidin-1-yl)quinoline-3-carboxylic acid, ammonia salt**

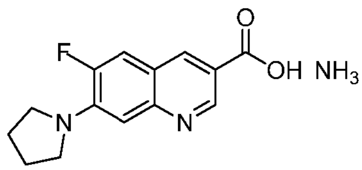

**A. Ethyl 6-fluoro-4-oxo-7-(pyrrolidin-1-yl)-1-((2-(trimethylsilyl)ethoxy)methyl)-1,4-dihydroquinoline-3-carboxylate**

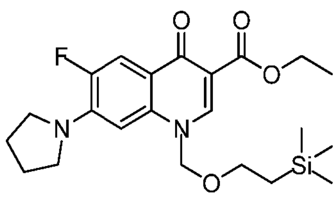

DIEA (1.38 mL, 7.90 mmol) was added to ethyl 6,7-difluoro-4-oxo-1-((2-(trimethylsilyl)ethoxy)methyl)-1,4-dihydroquinoline-3-carboxylate (Intermediate 23, step A) (0.716 mL, 2.63 mmol) in 1,4-dioxane (8.78 mL) at RT, then pyrrolidine (0.436 mL, 5.27 mmol) was added and the reaction mixture was heated at 100 °C for 17 h. The reaction mixture

was poured into saturated sodium bicarbonate, extracted with DCM, dried over magnesium sulfate, filtered, and concentrated. The residue was purified by silica gel chromatography, eluting with EtOAc:hexanes (7:3 to 1:0) to give the title compound (0.8540 g, 70.9 % yield). $^1H$ NMR (400 MHz, $CD_3SOCD_3$) δ -0.07 (s, 9 H), 0.87 (t, *J* = 8 Hz, 2 H), 1.27 (t, *J* = 7 Hz, 3 H), 1.92-1.98 (m, 4 H), 3.46-3.56 (m, 4 H), 3.58 (t, *J* = 8 Hz, 2 H), 4.21 (q, *J* = 7 Hz, 2 H), 5.66 (s, 2 H), 6.74 (d, *J* = 8 Hz, 1 H), 7.67 (d, *J* = 15 Hz, 1 H), 8.62 (s, 1 H); LC-MS (LC-ES) M+H = 435.

**B. Ethyl 4-chloro-6-fluoro-7-(methylthio)quinoline-3-carboxylate**

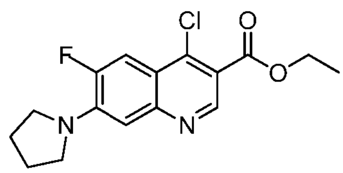

Phosphorus oxychloride (5.50 mL, 59.0 mmol) was added to ethyl 6-fluoro-4-oxo-7-(pyrrolidin-1-yl)-1-((2-(trimethylsilyl)ethoxy)methyl)-1 ,4-dihydroquinoline-3-carboxylate (0.854 g, 1.965 mmol) at RT, then the reaction mixture was heated at 105 °C for 16 h. The reaction mixture was poured into ice, quenched with 5 N sodium hydroxide, extracted with DCM, dried over magnesium sulfate, filtered, and concentrated. The residue was purified by silica gel chromatography, eluting with EtOAc:hexanes (1:2) to give the title compound (0.4299 g, 64.4 % yield). $^1H$ NMR (400 MHz, $CD_3SOCD_3$) δ 1.35 (t, *J* = 7 Hz, 3 H), 1.94-2.00 (m, 4 H), 3.54-3.60 (m, 4 H), 4.36 (q, *J* = 7 Hz, 2 H), 7.05 (d, *J* = 9 Hz, 1 H), 7.87 (d, *J* = 15 Hz, 1 H), 8.94 (s, 1 H); LC-MS (LC-ES) M+H = 323.

**C. Ethyl 6-fluoro-7-(pyrrolidin-1-yl)quinoline-3-carboxylate**

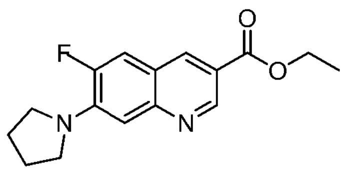

Bis(triphenylphosphine)palladium(II) chloride (0.046 g, 0.065 mmol) was added to ethyl 4-chloro-6-fluoro-7-(pyrrolidin-1-yl)quinoline-3-carboxylate (0.4215 g, 1.306 mmol) in acetonitrile (6.53 mL) at RT, then triethylsilane (0.29 mL, 1.83 mmol) was added and the reaction mixture was heated at 70 °C for 16 h. The reaction mixture was poured into saturated sodium bicarbonate, extracted with DCM, dried over magnesium sulfate, filtered, and concentrated. The residue was purified by RP HPLC eluting with acetonitrile:water with 0.1% ammonium hydroxide (5:95-100:0) to give the title compound (0.1750 g, 44.2 % yield). $^1H$ NMR (400 MHz, $CD_3SOCD_3$) δ 1.35 (t, *J* = 7 Hz, 3 H), 1.96 (dt, *J* = 5, 3 Hz, 4 H), 3.55 (dt, *J* = 7, 3 Hz, 4 H), 4.35 (q, *J* = 7 Hz, 2 H), 7.03 (d, *J* = 9 Hz, 1 H), 7.83 (d, *J* = 15 Hz, 1 H), 8.68 (d, *J* = 2 Hz, 1 H), 9.07 (d, *J* = 2 Hz, 1 H); LC-MS (LC-ES) M+H = 289.

**D. 6-Fluoro-7-(pyrrolidin-1-yl)quinoline-3-carboxylic acid, ammonia salt**

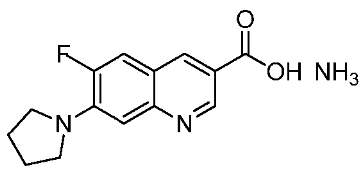

Lithium hydroxide (0.044 g, 1.821 mmol) was added to ethyl 6-fluoro-7-(pyrrolidin-1-yl)quinoline-3-carboxylate (0.175 g, 0.607 mmol) in MeOH (5.40 mL) and water (0.674 mL) at RT and the reaction mixture was stirred 16 h at 60

°C. The reaction mixture was concentrated. The reaction mixture was purified by RP HPLC eluting with acetonitrile:water with 0.1% ammonium hydroxide (5:95-100:0) to give the title compound (0.1759 g, 99 % yield). $^1H$ NMR (400 MHz, $CD_3SOCD_3$) δ 1.95 (dt, *J* = 7, 4 Hz, 4 H), 3.46 (dt, *J* = 7, 3 Hz, 4 H), 7.01 (d, *J* = 9 Hz, 1 H), 7.62 (d, *J* = 15 Hz, 1 H), 8.41 (d, *J* = 2 Hz, 1 H), 9.10 (d, *J* = 2 Hz, 1 H); LC-MS (LC-ES) M-H = 259.

**Intermediate 26: 6-Fluoro-7-(pyrrolidin-1-yl)quinoline-3-carboxylic acid, ammonia salt**

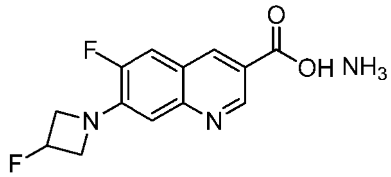

**A. Ethyl 4-chloro-6,7-difluoroquinoline-3-carboxylate**

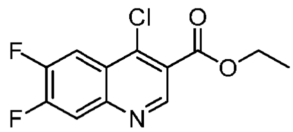

Phosphorus oxychloride (3.79 mL, 40.7 mmol) was added to ethyl 6,7-difluoro-4-oxo-1,4-dihydroquinoline-3-carboxylate (1.03 g, 4.07 mmol) at RT, then the reaction mixture was heated at 105 °C for 4 h. The reaction mixture was poured into ice, quenched with 5 N sodium hydroxide, extracted with DCM, dried over magnesium sulfate, filtered, and concentrated. The residue was purified by silica gel chromatography, eluting with MeOH:DCM (0:1 to 1:9) to give the title compound (1.15 g, 99 % yield). $^1H$ NMR (400 MHz, $CD_3SOCD_3$) δ 1.37 (t, *J* = 7 Hz, 3 H), 4.43 (q, *J* = 7 Hz, 2 H), 8.25 (dd, *J* = 11, 8 Hz, 1 H), 8.36 (dd, *J* = 12, 9 Hz, 1 H), 9.17 (s, 1 H); LC-MS (LC-ES) M+H = 272.

**B. Ethyl 6,7-difluoroquinoline-3-carboxylate**

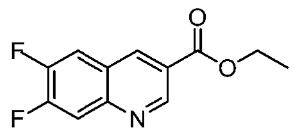

Bis(triphenylphosphine)palladium(II) chloride (0.297 g, 0.423 mmol) was added to ethyl 4-chloro-6,7-difluoroquinoline-3-carboxylate (1.15 g, 4.23 mmol) in acetonitrile (21.17 mL) at RT, then triethylsilane (0.947 mL, 5.93 mmol) was added and the reaction mixture was heated at 70 °C for 4 h. The reaction mixture was poured into saturated sodium bicarbonate, extracted with DCM, dried over magnesium sulfate, filtered, and concentrated. The residue was purified by silica gel chromatography, eluting with MeOH:DCM (1:49 to 1:9) to give the title compound (0.8825 g, 80 % yield). $^1H$ NMR (400 MHz, $CD_3SOCD_3$) δ 1.38 (t, *J* = 7 Hz, 3 H), 4.41 (q, *J* = 7 Hz, 2 H), 8.15 (dd, *J* = 11, 8 Hz, 1 H), 8.34 (dd, *J* = 11, 9 Hz, 1 H), 9.03 (d, *J* = 2 Hz, 1 H), 9.31 (d, *J* = 2 Hz, 1 H); LC-MS (LC-ES) M+H = 238.

**C. Ethyl 6-fluoro-7-(3-fluoroazetidin-1-yl)quinoline-3-carboxylate**

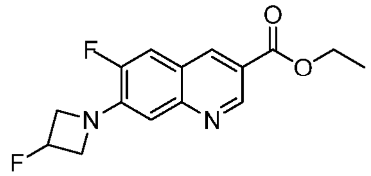

DIEA (0.896 mL, 5.13 mmol) was added to ethyl 6,7-difluoroquinoline-3-carboxylate (0.304 g, 1.282 mmol) in EtOH (6.41 mL) at RT. Then, 3-fluoroazetidine hydrochloride (0.358 g, 3.21 mmol) was added and the reaction mixture was heated at 100 °C in the microwave for 8 h and concentrated. The reaction mixture was dissolved in DCM, washed with saturated sodium bicarbonate, dried over magnesium sulfate, filtered, and concentrated. The residue was purified by silica gel chromatography, eluting with EtOAc:hexanes (1:2) to give the title compound (0.2297 g, 58.2 % yield). $^1$H NMR (400 MHz, $CD_3SOCD_3$) δ 1.35 (t, *J* = 7 Hz, 3 H), 4.23 (dd, *J* = 24, 14 Hz, 2 H), 4.36 (q, *J* = 7 Hz, 2 H), 4.44-4.56 (m, 2 H), 5.54 (ddt, *J* = 58, 5, 3 Hz, 1 H), 6.98 (d, *J* = 9 Hz, 1 H), 7.87 (d, *J* = 13 Hz, 1 H), 8.75 (d, *J* = 2 Hz, 1 H), 9.11 (d, *J* = 2 Hz, 1 H); LC-MS (LC-ES) M+H = 293.

**D. 6-Fluoro-7-(3-fluoroazetidin-1-yl)quinoline-3-carboxylic acid ammonia salt**

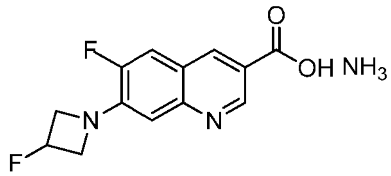

Lithium hydroxide (0.055 g, 2.281 mmol) was added to ethyl 6-fluoro-7-(3-fluoroazetidin-1-yl)quinoline-3-carboxylate (0.222 g, 0.760 mmol) in MeOH (6.76 mL) and water (0.845 mL) at RT and the reaction mixture was stirred 16 h at 60 °C. The reaction mixture was concentrated. The reaction mixture was purified by RP HPLC eluting with acetonitrile:water with 0.1% ammonium hydroxide (5:95-100:0) to the title compound (0.1283 g, 57.0% yield). $^1$H NMR (400 MHz, $CD_3SOCD_3$) δ 4.04-4.18 (m, 2 H), 4.32-4.44 (m, 2 H), 5.51 (dtt, *J* = 58, 6, 3 Hz, 1 H), 6.93 (d, *J* = 9 Hz, 1 H), 7.66 (d, *J* = 13 Hz, 1 H), 8.42 (d, *J* = 2 Hz, 1 H), 9.12 (d, *J* = 2 Hz, 1 H); LC-MS (LC-ES) M-H = 263.

**Intermediate 27: 6-Fluoro-7-(3-fluoro-3-methylazetidin-1-yl)quinoline-3-carboxylic acid ammonia salt**

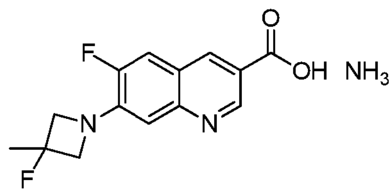

**A. Ethyl 6-fluoro-7-(3-fluoro-3-methylazetidin-1-yl)quinoline-3-carboxylate**

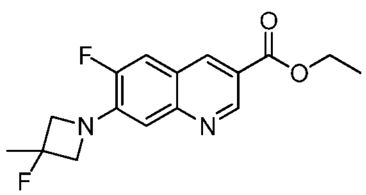

DIEA (0.611 mL, 3.50 mmol) was added to ethyl 6,7-difluoroquinoline-3-carboxylate (Intermediate 26, step B) (0.2073 g, 0.874 mmol) in EtOH (4.37 mL) at RT. Then, 3-fluoro-3-methylazetidine hydrochloride (0.274 g, 2.185 mmol) was added and the reaction mixture was heated at 100 °C in the microwave for 5 h and concentrated. The reaction mixture was dissolved in DCM, washed with saturated sodium bicarbonate, dried over magnesium sulfate, filtered, and concentrated. The residue was purified by silica gel chromatography, eluting with EtOAc:hexanes (1:2) to give the title compound (0.1632 g, 50.0 % yield). $^1$H NMR (400 MHz, $CD_3SOCD_3$) δ 1.35 (t, *J* = 7 Hz, 3 H), 1.65 (d, *J* = 22 Hz, 3 H), 4.18-4.34 (m, 4 H), 4.36 (q, *J* = 7 Hz, 2 H), 6.98 (d, *J* = 9 Hz, 1 H), 7.88 (d, *J* = 13 Hz, 1 H), 8.75 (d, *J* = 2 Hz, 1 H), 9.11 (d, *J* = 2 Hz, 1 H); LC-MS (LC-ES) M+H = 307.

**B. 6-Fluoro-7-(3-fluoro-3-methylazetidin-1-yl)quinoline-3-carboxylic acid ammonia salt**

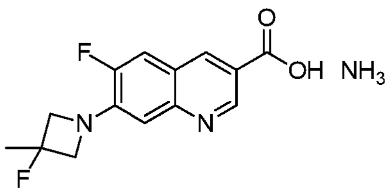

Lithium hydroxide (0.036 g, 1.519 mmol) was added to ethyl 6-fluoro-7-(3-fluoro-3-methylazetidin-1-yl)quinoline-3-carboxylate (0.1551 g, 0.506 mmol) in MeOH (4.50 mL) and water (0.563 mL) at RT and the reaction mixture was stirred 16 h at 60 °C. The reaction mixture was concentrated. The reaction mixture was purified by RP HPLC eluting with acetonitrile:water with 0.1% ammonium hydroxide (5:95-100:0) to give the title compound (0.0754 g, 47.9% yield). $^1$H NMR (400 MHz, $CD_3SOCD_3$) δ 1.65 (d, *J* = 22 Hz, 3 H), 4.08-4.24 (m, 4 H), 6.94 (d, *J* = 9 Hz, 1 H), 7.69 (d, *J* = 13 Hz, 1 H), 8.47 (d, *J* = 2 Hz, 1 H), 9.12 (d, *J* = 2 Hz, 1 H); LC-MS (LC-ES) M-H = 277.

**Intermediate 28: 7-(azetidin-1-yl)-6-chloroquinoline-3-carboxylic acid**

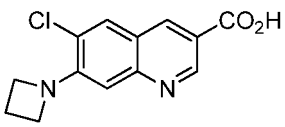

**A. Diethyl 2-(((4-chloro-3-fluorophenyl)amino)methylene)malonate**

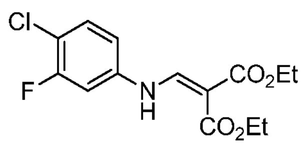

In a thick-walled glass pressure vessel, a solution of 4-chloro-3-fluoroaniline (24.83 g, 171 mmol) and diethyl 2-(ethoxymethylene)malonate (34.5 mL, 171 mmol) in toluene (100 mL) was stirred in an oil bath at 120 °C for 12 h. Volatiles were removed *in vacuo* affording a waxy orange solid. The crude solid was triturated with hexanes and dried on the Buchner funnel affording the title compound (51.41 g, 95 % yield) as an off-white powder which was used without further purification. $^1$H NMR (400 MHz, $CD_3SOCD_3$) δ ppm 1.25 (dt, *J* = 7, 6 Hz, 6 H), 4.13 (q, *J* = 7 Hz, 2 H), 4.21 (q, *J* = 7 Hz, 2 H), 7.24 - 7.31 (m, 1 H), 7.56 (t, *J* = 8 Hz, 1 H), 7.61 (dd, *J* = 11, 3 Hz, 1 H), 8.33 (d, *J* = 14 Hz, 1 H), 10.63 (d, *J* = 14 Hz, 1 H). MS (ESI) *m/z* 316 (M+H).

**B. Ethyl 6-chloro-7-fluoro-4-oxo-1,4-dihydroquinoline-3-carboxylate**

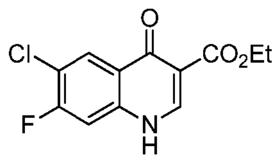

Diethyl 2-(((4-chloro-3-fluorophenyl)amino)methylene)malonate (5.00 g, 15.8 mmol) was added to refluxing diphenyl ether in 1 g portions over 10 min. Heating was maintained an additional 10 min, and the mixture was cooled in a water bath. Hexane (40 mL) was added and precipitated solids were collected by filtration. The filter cake was washed with hexane and diethyl ether and dried on the Buchner funnel affording the title compound (3.76 g, 13.9 mmol, 88 % yield), used without further purification. MS (ESI) *m/z* 270 (M+H).

**C. Ethyl 6-chloro-7-fluoro-4-oxo-1-((2-(trimethylsilyl)ethoxy)methyl)-1,4-dihydroquinoline-3-carboxylate**

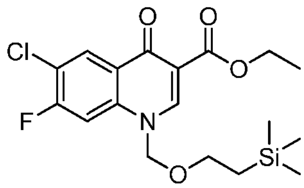

To a slurry of hexane-washed NaH (0.837 g, 20.9 mmol) in DMF (20 mL) at RT was added a hot slurry of ethyl 6-chloro-7-fluoro-4-oxo-1,4-dihydroquinoline-3-carboxylate (3.76 g, 13.9 mmol) in DMF (50 mL), portionwise over 15 min. The mixture was stirred 1 h at RT and SEM-Cl (2.72 mL, 15.3 mmol) was added *via* syringe. After 1.25 h an additional portion of NaH (0.140 g; 3.5 mmol) was added and the mixture was stirred 10 min. An additional portion of SEM-Cl (0.27 mL; 1.5 mmol) was added and stirring continued for 30 min. The mixture was poured into sat. $NaHCO_3$ (200 mL) and a small amount of water was added to re-dissolve precipitated inorganic salts. The mixture was stirred 30 min and precipitated solids were collected by filtration affording ca. 5.7 g orange solid. The crude solid was triturated with hot heptane affording the title compound (3.07 g, 55 % yield) as a tan solid. A second crop of the title compound (0.637 g, 11 % yield) was obtained from the filtrate on standing (combined with first crop). MS (ESI) *m/z* 400 (M+H).

**D. Ethyl 4,6-dichloro-7-fluoroquinoline-3-carboxylate**

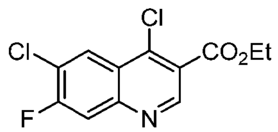

An oven-dried vial was charged with ethyl 6-chloro-7-fluoro-4-oxo-1-((2-(trimethylsilyl)ethoxy)methyl)-1,4-dihydroquinoline-3-carboxylate (0.630 g; 1.58 mmol), $POCl_3$ (0.16 mL; 1.7 mmol), and dioxane (10 mL) and sealed with a crimp top. The mixture was subjected to microwave heating at 80 °C for 30 min. Upon cooling the mixture was poured into sat. $NaHCO_3$ and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound (0.3395 g, 75 % yield) as a colorless solid, used without further purification. $^1H$ NMR (400 MHz, $CD_3SOCD_3$) $\delta$ ppm 1.38 (t, *J* = 7 Hz, 3 H), 4.43 (q, *J* = 7 Hz, 2 H), 8.17 (d, *J* = 10 Hz, 1 H),8.53 (d, *J* = 8 Hz, 1 H), 9.17 (s, 1 H). MS (ESI) *m/z* 288 (M+H).

**Alternate preparation of ethyl 4,6-dichloro-7-fluoroquinoline-3-carboxylate**

A thick-walled glass vessel was charged with ethyl 6-chloro-7-fluoro-4-oxo-1,4-dihydroquinoline-3-carboxylate (intermediate B above) (2.76 g, 10.24 mmol), dioxane (20 mL), and $POCl_3$ (1.049 mL, 11.26 mmol) and sealed with a teflon bushing. The mixture was stirred in an oil bath at 80 °C for 40 min, then at 100 °C for 30 min. Additional dioxane (10 mL) and $POCl_3$ (0.10 mL) were added, and heating continued at 120 °C for 5 h. Upon cooling, the dark solution was

poured into sat. $NaHCO_3$ (200 mL) and the mixture was stirred 30 min. Precipitated solids were collected by filtration, washed with water and dried on the Buchner funnel. The crude solid was purified by flash chromatography (EtOAc/hexanes, gradient elution) affording ethyl 4,6-dichloro-7-fluoroquinoline-3-carboxylate (2.39 g, 8.30 mmol, 81 % yield) as a colorless solid. $^1H$ NMR / LCMS were found to be identical with material prepared as described by steps C and D above.

**E. Ethyl 6-chloro-7-fluoroquinoline-3-carboxylate**

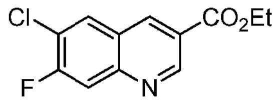

A 20 mL vial was charged with ethyl 4,6-dichloro-7-fluoroquinoline-3-carboxylate (0.329 g; 1.14 mmol), $PdCl_2(PPh_3)_2$ (0.040 g; 0.057 mmol) and MeCN (5 mL) and sealed with a rubber septum. The mixture was degassed by sparging with nitrogen for 10 min while immersed in an ultrasonic bath. $Et_3SiH$ (0.20 mL; 1.3 mmol) was added *via* syringe and the septum was replaced with a crimp top. The mixture was stirred in a heating block at 70 °C. An additional portion of $Et_3SiH$ (0.020 mL; 0.13 mmol) was added after 5 h and heating was resumed. After 21 h the mixture was cooled, poured into water and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound (0.205 g, 71 % yield) as a tan solid. $^1H$ NMR (400 MHz, $CD_3SOCD_3$) $\delta$ ppm 1.38 (t, *J* = 7 Hz, 3 H), 4.42 (q, *J* = 7 Hz, 2 H), 8.09 (d, *J* = 10 Hz, 1 H), 8.59 (d, *J* = 8 Hz, 1 H), 9.01 (dd, *J* = 2, 1 Hz, 1 H), 9.32 (d, *J* = 2 Hz, 1 H). MS (ESI) *m/z* 254 (M+H).

**F. Ethyl 7-(azetidin-1-yl)-6-chloroquinoline-3-carboxylate**

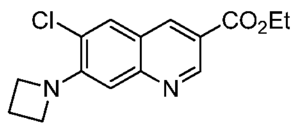

An oven-dried vial was charged with ethyl 6-chloro-7-fluoroquinoline-3-carboxylate (0.197 g, 0.777 mmol), MeCN (5 mL), DIEA (0.271 mL, 1.553 mmol), and azetidine (0.079 mL, 1.165 mmol), and sealed with a crimp top. The mixture was stirred in a heating block at 100 °C for 17 h. Additional portions of azetidine (0.027 mL; 0.78 mmol) and DIEA (0.14 mL; 0.8 mmol) were added and the mixture was heated to 100 °C for 1 h, then 120 °C for 1 h. Additional azetidine (0.060 mL; 0.89 mmol) was added and the mixture was heated to 120 °C for 1 h. Upon cooling the mixture was poured into water and extracted with EtOAc (3X). Combined organics were washed with brine, dried over $Na_2SO_4$, and concentrated *in vacuo.* The residue was purified by flash chromatography (silica gel, EtOAc / hexanes, gradient elution) affording the title compound (0.225 g, 100 % yield) as a yellow solid. $^1H$ NMR (400 MHz, $CDCl_3$) $\delta$ ppm 1.44 (t, *J* = 7 Hz, 3 H), 2.35 - 2.45 (m, 2 H), 4.30 (t, *J* = 7 Hz, 4 H), 4.44 (q, *J* = 7 Hz, 2 H), 6.97 (s, 1 H), 7.73 (s, 1 H), 8.54 (d, *J* = 2 Hz, 1 H), 9.26 (d, *J* = 2 Hz, 1 H). MS (ESI) *m/z* 291 (M+H).

**G. 7-(Azetidin-1-yl)-6-chloroquinoline-3-carboxylic acid**

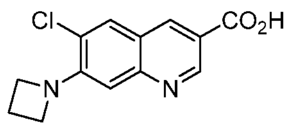

A mixture of ethyl 7-(azetidin-1-yl)-6-chloroquinoline-3-carboxylate (0.588 g, 2.022 mmol) and $LiOH{\cdot}H_2O$ (0.255 g, 6.07 mmol) in THF (10 mL) and water (0.5 mL) was heated under reflux for 15 h (flask went dry). The residue was resuspended in THF (10 mL), water (0.5 mL) and additional $LiOH{\cdot}H_2O$ (0.085g; 2.0 mmol) were added and heating resumed. After ca. 1 h, the mixture was poured into water and extracted with $Et_2O$ (2X). The aqueous layer was acidified

to ca. pH 5.5 by addition of 1 N HCl and precipitated solids were collected by filtration, affording the title compound (0.5261 g, 99 % yield) as a yellow solid. $^1H$ NMR (400 MHz, $CD_3SOCD_3$) δ ppm 2.26 - 2.38 (m, 2 H), 4.22 (t, *J* = 7 Hz, 4 H), 6.89 (s, 1 H), 8.11 (s, 1 H), 8.69 (d, *J* = 2 Hz, 1 H), 9.11 (d, *J* = 2 Hz, 1 H), 13.19 (br s, 1 H). MS (ESI) *m/z* 263 (M+H).

**Intermediate 29: 7-Azido-6-chloroquinoline-3-carboxylic acid**

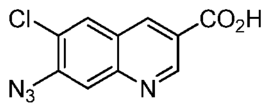

**A. Ethyl 7-azido-6-chloroquinoline-3-carboxylate**

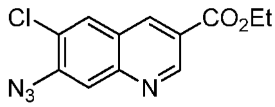

A microwave vial was charged with ethyl 6-chloro-7-fluoroquinoline-3-carboxylate (Intermediate 28, step E) (200 mg, 0.79 mmol) and DMSO (4 mL). To this was added sodium azide (103 mg, 1.577 mmol) and the reaction was heated in the microwave at 110 °C for 1 h. After cooling to RT the reaction was quenched into 20 mL water and extract with EtOAc (4x). The combined organics were dried over sodium sulfate and concentrated. The residue was purified by silica gel chromatography (0-35% EtOAc-hexanes gradient) to afford the title compound as a white solid (186 mg, 85% yield). $^1H$ NMR (400 MHz, $CD_3SOCD_3$) δ ppm 1.36 (t, *J* = 7 Hz, 3 H), 4.40 (q, *J* = 7 Hz, 2 H), 7.99 (s, 1 H), 8.46 (s, 1 H), 8.94 (d, *J* = 2 Hz, 1 H), 9.30 (d, *J* = 2 Hz, 1 H). MS (ESI) *m/z* 277 (M+1).

**B. 7-Azido-6-chloroquinoline-3-carboxylic acid**

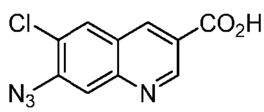

To ethyl 7-azido-6-chloroquinoline-3-carboxylate (180 mg, 0.651 mmol), in THF (3 mL) was added LiOH (82 mg, 1.95 mmol, mono-hydrate) dissolved in water (1 mL) and the mixture was heated at 45 °C overnight. To the mixture was added 5 mL of water, the pH was adjusted to 5.5 with 6N HCl, and it was extracted with EtOAc (4X). The combined organics were dried over sodium sulfate and concentrated to afford the title compound as a pale yellow solid (107 mg, 66% yield). $^1H$ NMR (400 MHz, $CD_3SOCD_3$) δ ppm 7.90 (s, 1 H), 8.30 (s, 1 H), 8.67 (s, 1 H), 9.31 (d, *J* = 2 Hz, 1 H). MS (ESI) *m/z* 249 (M+1).

**Intermediate 30: 6-Chloro-7-(3-fluoroazetidin-1-yl)quinoline-3-carboxylic** acid

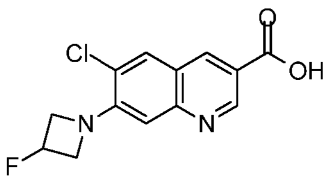

**A: Ethyl 6-chloro-7-(3-fluoroazetidin-1-yl)quinoline-3-carboxylate**

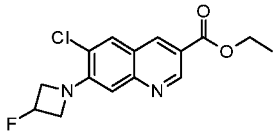

To a solution of ethyl 6-chloro-7-fluoroquinoline-3-carboxylate (Intermediate 28, step E) (300 mg, 1.183 mmol) in DMF (15 mL) was added 3-fluoroazetidine hydrochloride (396 mg, 3.55 mmol) followed by DIEA (1.24 mL, 7.10 mmol). The reaction was then heated at 90 °C for 5 h. The reaction was concentrated under reduced pressure, the residue was dissolved in DCM, and absorbed onto silica gel. The crude reaction was then purified by silica gel chromatography (5-30% EtOAc/hexanes) to afford the title compound (250 mg, 69%). MS (ESI) *m/z* 309(M+1).

**B: 6-chloro-7-(3-fluoroazetidin-1-yl)quinoline-3-carboxylic acid**

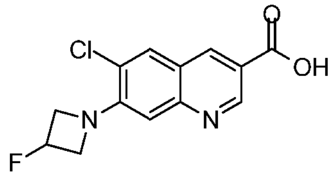

To a solution of ethyl 6-chloro-7-(3-fluoroazetidin-1-yl)quinoline-3-carboxylate (250 mg, 0.868 mmol) in THF (25 mL), MeOH (25 mL) and water (5 mL) was added lithium hydroxide (104 mg, 4.34 mmol). The reaction was heated at 50 °C for 3 h. The pH of the reaction mixture was adjusted to 7 with 1M HCl and the organic solvent was removed. The solid was filtered, washed with water, and dried overnight under high vacuum to afford the title compound (200 mg, 72%). MS (ESI) *m/z* 281 (M+1).

**Intermediate 31: 6-Fluoro-7-(2-methylazetidin-1-yl)quinoline-3-carboxylic acid ammonia salt**

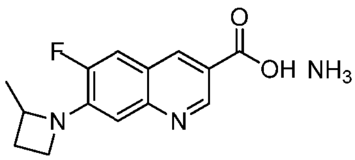

**A. Ethyl 6-fluoro-7-(2-methylazetidin-1-yl)quinoline-3-carboxylate**

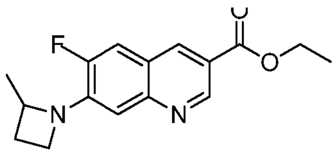

DIEA (1.22 mL, 6.98 mmol) was added to ethyl 6,7-difluoroquinoline-3-carboxylate (Intermediate 26, step B) (0.4142 g, 1.746 mmol) in EtOH (8.73 mL) at RT. Then, 2-methylazetidine hydrochloride (0.470 g, 4.37 mmol) was added and the reaction mixture was heated at 100 °C in the microwave for 2 h and concentrated. The reaction mixture was dissolved in DCM, washed with saturated sodium bicarbonate, dried over magnesium sulfate, filtered, and concentrated.

The residue was purified by silica gel chromatography, eluting with EtOAc:hexanes (3:7) to give the title compound (0.432 g, 82 % yield). $^1H$ NMR (400 MHz, $CD_3SOCD_3$) δ 1.35 (t, *J* = 7 Hz, 3 H), 1.44 (d, *J* = 6 Hz, 3 H), 1.96-2.08 (m, 1 H), 2.46-2.58 (m, 1 H), 3.95 (q, *J* = 8 Hz, 1 H), 4.14-4.24 (m, 1 H), 4.36 (q, *J* = 7 Hz, 2 H), 4.50 (h, *J* = 6 Hz, 1 H), 6.93 (d, *J* = 8 Hz, 1 H), 7.84 (d, *J* = 13 Hz, 1 H), 8.71 (d, *J* = 2 Hz, 1 H), 9.09 (d, *J* = 2 Hz, 1 H); LC-MS (LC-ES) M+H = 289.

**B. 6-Fluoro-7-(2-methylazetidin-1-yl)quinoline-3-carboxylic acid ammonia salt**

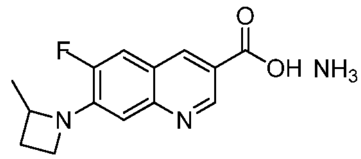

Lithium hydroxide (0.105 g, 4.37 mmol) was added to ethyl 6-fluoro-7-(2-methylazetidin-1-yl)quinoline-3-carboxylate (0.4199 g, 1.456 mmol) in MeOH (12.95 mL) and water (1.618 mL) at RT and the reaction mixture was stirred 2 h at 60 °C. The reaction mixture was concentrated. The reaction mixture was purified by RP HPLC eluting with acetonitrile:water with 0.1% ammonium hydroxide (5:95-100:0) to give the title compound (0.3709 g, 87 % yield). $^1H$ NMR (400 MHz, $CD_3SOCD_3$) δ 1.42 (d, *J* = 6 Hz, 3 H), 1.96-2.06 (m, 1 H), 2.46-2.54 (m, 1 H), 3.87 (q, *J* = 8 Hz, 1 H), 4.08-4.16 (m, 1 H), 4.43 (h, *J* = 6 Hz, 1 H), 6.92 (d, *J* = 9 Hz, 1 H), 7.71 (d, *J* = 13 Hz, 1 H), 8.54 (d, *J* = 2 Hz, 1 H), 9.08 (d, *J* = 2 Hz, 1 H); LC-MS (LC-ES) M-H = 259.

**Intermediate 32: 7-Acetyl-6-chloroquinoline-3-carboxylic acid**

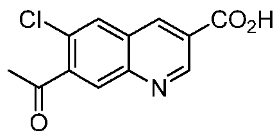

**A. Ethyl 7-acetyl-6-chloroquinoline-3-carboxylate**

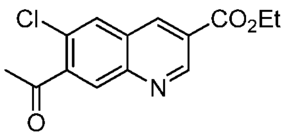

To a microwave vial charged with ethyl 7-bromo-6-chloroquinoline-3-carboxylate (Intermediate 4, step B) (200 mg, 0.636 mmol) and trifluorotoluene (6 mL) was added tributyl(1-ethoxyvinyl)stannane (253 mg, 0.70 mmol) and bis(triphenylphosphine)palladium(II) chloride (22 mg, 0.03 mmol). The reaction was heated in the microwave at 150 °C for 20 min, cooled to RT, and filtered through Celite®, washing with EtOAc. Combined organics were washed with water, dried over $Na_2SO_4$, filtered, and concentrated. The residue was dissolved in THF (5 mL) and 1 N HCl (5 mL) and stirred at RT for 2 h. The pH was adjusted to ca. 8 with $NaHCO_3$ and extracted with EtOAc (3X). The combined organics were dried over $Na_2SO_4$, filtered, and concentrated. The residue was purified by silica gel chromatography (0-65% EtOAc-hexanes gradient) to afford the title compound as a white solid (148 mg, 84% yield). $^1H$ NMR (400 MHz, $CDCl_3$) δ ppm 1.46 (t, *J* = 7 Hz, 3 H), 2.73 (s, 3 H), 4.49 (q, *J* = 7 Hz, 2 H), 8.00 (s, 1 H), 8.29 (s, 1 H), 8.76 (d, *J* = 2 Hz, 1 H), 9.47 (d, *J* = 2 Hz, 1 H). MS (ESI) *m*/*z* 278 (M+1).

**B. 7-Acetyl-6-chloroquinoline-3-carboxylic acid**

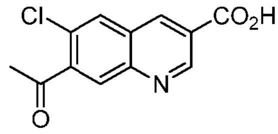

To ethyl 7-acetyl-6-chloroquinoline-3-carboxylate (135 mg, 0.486 mmol), in THF (3 mL) was added LiOH (61 mg, 1.46 mmol, mono-hydrate) dissolved in water (1 mL) and the mixture was heated at 45 °C overnight. To the mixture was added 5 mL of water and 10% citric acid, and it was extracted with EtOAc (4X). The combined organics were washed with water and brine, dried over sodium sulfate, filtered, and concentrated to afford the title compound as a off white solid (125 mg, 103 % yield). $^1$H NMR (400 MHz, $CD_3SOCD_3$) δ ppm 2.70 (s, 3 H), 8.41 (s, 1 H), 8.45 (s, 1 H), 8.99 (d, *J* = 2 Hz, 1 H), 9.36 (d, *J* = 2 Hz, 1 H). MS (ESI) *m/z* 250 (M+1).

**Intermediate 33: 7-(3,3-Difluoroazetidin-1-yl)-6-fluoroquinoline-3-carboxylic acid trifluoroacetate**

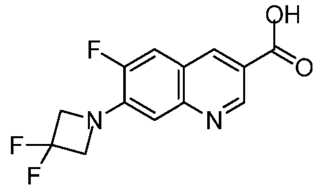 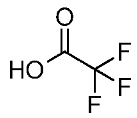

**A. Ethyl 7-(3,3-difluoroazetidin-1-yl)-6-fluoroquinoline-3-carboxylate and Ethyl 7-ethoxy-6-fluoroquinoline-3-carboxylate**

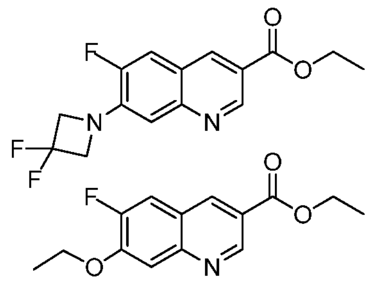

3,3-Difluoroazetidine hydrochloride (0.228 g, 1.758 mmol) was added to ethyl 6,7-difluoroquinoline-3-carboxylate (Intermediate 26, step B) (0.4171 g, 1.758 mmol) in DMSO (4.40 mL) at RT, then sodium hydride (0.141 g, 3.52 mmol) was added and the reaction mixture was heated at 100 °C in the microwave for 1 h. The reaction mixture was dissolved in DCM, washed with water, dried over magnesium sulfate, filtered, and concentrated. The residue was purified by silica gel chromatography, eluting with EtOAc:hexanes (3:7) to give ethyl 7-(3,3-difluoroazetidin-1-yl)-6-fluoroquinoline-3-carboxylate (0.0412 g, 7.2 % yield) and ethyl 7-ethoxy6-fluoroquinoline-3-carboxylate (0.0935 g, 19.2 % yield). The starting amine hydrochloride must be wet, leading to ester hydrolysis and ethoxide addition.

**Ethyl 7-(3,3-difluoroazetidin-1-yl)-6-fluoroquinoline-3-carboxylate:** $^1$H NMR (400 MHz, $CD_3SOCD_3$) δ 1.36 (t, *J* = 7 Hz, 3 H), 4.37 (q, *J* = 7 Hz, 2 H), 4.61 (dt, *J* = 12, 2 Hz, 4 H), 7.12 (d, *J* = 9 Hz, 1 H), 7.94 (d, *J* = 13 Hz, 1 H), 8.79 (d, *J* = 2 Hz, 1 H), 9.15 (d, *J* = 2 Hz, 1 H); LC-MS (LC-ES) M+H = 311.

**Ethyl 7-ethoxy-6-fluoroquinoline-3-carboxylate:** $^1$H NMR (400 MHz, $CD_3SOCD_3$) δ 1.38 (t, *J* = 7 Hz, 3 H), 1.45 (t, *J* = 7 Hz, 3 H), 4.34 (q, *J* = 7 Hz, 2 H), 4.40 (q, *J* = 7 Hz, 2 H), 7.66 (d, *J* = 8 Hz, 1 H), 8.06 (d, *J* = 12 Hz, 1 H), 8.89 (d, *J* = 2 Hz, 1 H), 9.23 (d, *J* = 2 Hz, 1 H); LC-MS (LC-ES) M+H = 264

**B. 7-(3,3-Difluoroazetidin-1-yl)-6-fluoroquinoline-3-carboxylic acid trifluoroacetate**

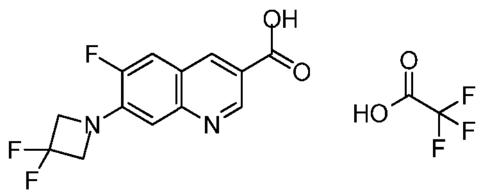

Lithium hydroxide (8.34 mg, 0.348 mmol) was added to ethyl 7-(3,3-difluoroazetidin-1-yl)-6-fluoroquinoline-3-carboxylate (0.0360 g, 0.116 mmol) in MeOH (2.06 mL) and water (0.25 mL) at RT and the reaction mixture was stirred 16 h at 60 °C. The reaction mixture was concentrated. The reaction mixture was purified by RP HPLC eluting with acetonitrile:water with 0.1% trifluoroacetic acid (0:100:100:0) to give the title compound (0.0422 g, 79 % yield). $^1H$ NMR (400 MHz, $CD_3SOCD_3$) δ 4.61 (dt, *J* = 12, 2 Hz, 4 H), 7.11 (d, *J* = 9 Hz, 1 H), 7.93 (d, *J* = 13 Hz, 1 H), 8.78 (d, *J* = 2 Hz, 1 H), 9.14 (d, *J* = 2 Hz, 1 H), 13.27 (br s, 1 H); LC-MS (LC-ES) M-H = 281.

**Intermediate 34: 7-Ethoxy-6-fluoroquinoline-3-carboxylic acid ammonia salt**

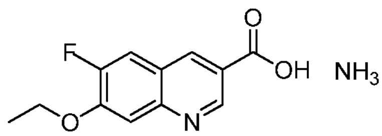

Lithium hydroxide (0.023 g, 0.975 mmol) was added to ethyl 7-ethoxy-6-fluoroquinoline-3-carboxylate (Intermediate 33, step A) (0.0856 g, 0.325 mmol) in MeOH (2.89 mL) and water (0.36 mL) at RT and the reaction mixture was stirred 64 h at 60 °C. The reaction mixture was concentrated. The reaction mixture was purified by RP HPLC eluting with acetonitrile:water with 0.1% ammonium hydroxide (5:95-100:0) to give the title compound (0.0682 g, 79 % yield). $^1H$ NMR (400 MHz, $CD_3SOCD_3$) δ 1.42 (t, *J* = 7 Hz, 3 H), 4.25 (q, *J* = 7 Hz, 2 H), 7.51 (d, *J* = 8 Hz, 1 H), 7.80 (d, *J* = 12 Hz, 1 H), 8.53 (d, *J* = 2 Hz, 1 H), 9.20 (d, *J* = 2 Hz, 1 H); LC-MS (LC-ES) M-H = 234.

**Intermediate 35: 6-Chloro-7-cyclobutylquinoline-3-carboxylic acid**

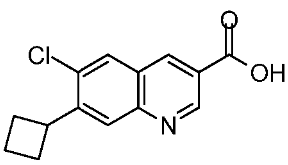

**A: Ethyl 6-chloro-7-cyclobutylquinoline-3-carboxylate**

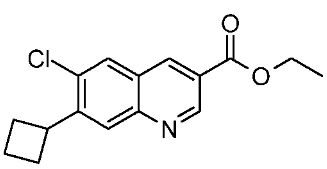

To $N_2$ degassed toluene (10 mL) was added $N_2$ degassed water (3 mL), ethyl 7-bromo-6-chloroquinoline-3-carboxylate (Intermediate 4, step B) (100 mg, 0.318 mmol),cesium carbonate (153 mg, 0.468 mmol), $PdCl_2$(dppf)-$CH_2Cl_2$ adduct (47.2 mg, 0.058 mmol), and cyclobutyltrifluoroborate, potassium salt (56.2 mg, 0.347 mmol). The resulting reaction mixture was heated at 95 °C for 3 h. The organic layer was removed, absorbed to silica gel and purified by silica gel

chromatography (2-20% EtOAc/ hexanes) to afford the title compound (30 mg, 36%). MS (ESI) *m/z* 290(M+1).

**B: 6-Chloro-7-cyclobutylquinoline-3-carboxylic acid**

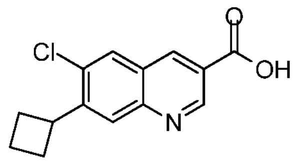

To a solution of ethyl 6-chloro-7-cyclobutylquinoline-3-carboxylate (100 mg, 0.345 mmol) in THF (15 mL), MeOH (15 mL), and water (3 mL) was added lithium hydroxide (41.3 mg, 1.726 mmol). The reaction was then allowed to heat at 50 °C for 1 h. The pH was adjusted to 5 with 1 M HCl and the solvent was removed. The resulting solid was dried under high vacuum to afford the title compound (80 mg, 89%). $^1$H NMR (400 MHz, $CD_3SOCD_3$) δ ppm 1.79 - 1.91 (m, 1 H) 2.00 - 2.13 (m, 1 H) 2.17 - 2.29 (m, 2 H) 2.42 - 2.48 (m, 2 H) 3.80 - 3.99 (m, 1 H) 8.00 (s, 1 H) 8.31 (s, 1 H) 8.91 (d, *J* = 2 Hz, 1 H) 9.28 (d, *J* = 2 Hz, 1 H) 13.54 (s, 1 H) MS (ESI) *m/z* 262(M+1).

**Intermediate 36: 6-Chloro-7-(2-methylazetidin-1-yl)quinoline-3-carboxylic acid**

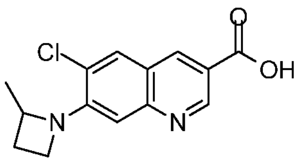

**A: Ethyl 6-chloro-7-(2-methylazetidin-1-yl)quinoline-3-carboxylate**

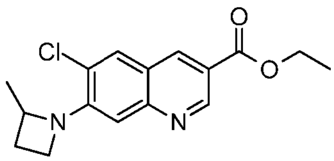

To a solution of ethyl 6-chloro-7-fluoroquinoline-3-carboxylate (Intermediate 28, step E) (50 mg, 0.197 mmol) in DMF (15 mL) was added 2-methylazetidine hydrochloride (63.6 mg, 0.591 mmol), followed by DIEA (0.207 mL, 1.183 mmol). The reaction was heated at 90 °C for 3 h. The reaction was concentrated under reduced pressure, the residue was dissolved in DCM, and absorbed onto silica gel. The crude reaction was then purified by silica gel chromatography (5-30% EtOAc/hexanes) to afford the title compound (50 mg, 83%). MS (ESI) *m/z* 305(M+1).

**B: 6-Chloro-7-(2-methylazetidin-1-yl)quinoline-3-carboxylic acid**

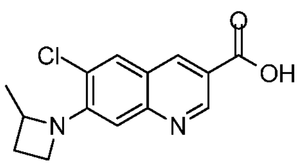

To a solution of ethyl 6-chloro-7-(2-methylazetidin-1-yl)quinoline-3-carboxylate (150 mg, 0.492 mmol) in THF (15 mL), MeOH (15 mL), and water (3 mL) was added lithium hydroxide (58.9 mg, 2.461 mmol). The reaction was heated at 50 °C for 3 h. The pH was adjusted to 7 with 1 M HCl and the organic solvent was removed. The solid was filtered, washed with water, and dried overnight under high vacuum to afford the title compound (110 mg, 81%). MS (ESI) *m*/*z* 277(M+1).

**Intermediate 37: 6-Fluoro-7-(3-methylazetidin-1-yl)quinoline-3-carboxylic acid ammonia salt**

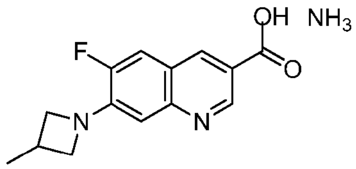

**A. Ethyl 6-fluoro-7-(3-methylazetidin-1-yl)quinoline-3-carboxylate**

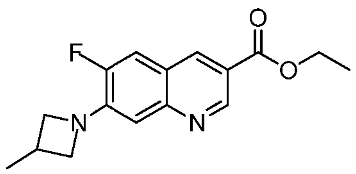

DIEA (0.913 mL, 5.23 mmol) was added to ethyl 6,7-difluoroquinoline-3-carboxylate (Intermediate 26, Step B) (0.3099 g, 1.306 mmol) in EtOH (6.53 mL) at RT. Then, 3-methylazetidine hydrochloride (0.351 g, 3.27 mmol) was added and the reaction mixture was heated at 100 °C in the microwave for 2 h and concentrated. The residue was dissolved in DCM, washed with saturated sodium bicarbonate, dried over magnesium sulfate, filtered, and concentrated. The residue was purified by silica gel chromatography, eluting with EtOAc:hexanes (3:7) to give the title compound (0.3266 g, 82 % yield). $^1$H NMR (400 MHz, $CD_3SOCD_3$) δ 1.26 (d, *J* = 7 Hz, 3 H), 1.35 (t, *J* = 7 Hz, 3 H), 2.86 (o, *J* = 7 Hz, 1 H), 3.73 (dt, *J* = 6, 1 Hz, 2 H), 4.27 (dt, *J* = 8, 2 Hz, 2 H), 4.35 (q, *J* = 7 Hz, 2 H), 6.83 (d, *J* = 9 Hz, 1 H), 7.82 (d, *J* = 13 Hz, 1 H), 8.70 (d, *J* = 2 Hz, 1 H), 9.08 (d, *J* = 2 Hz, 1 H); LC-MS (LC-ES) M+H = 289.

**B. 6-Fluoro-7-(3-methylazetidin-1-yl)quinoline-3-carboxylic acid ammonia salt**

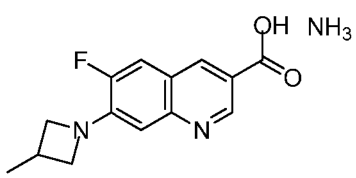

Lithium hydroxide (0.080 g, 3.33 mmol) was added to ethyl 6-fluoro-7-(3-methylazetidin-1-yl)quinoline-3-carboxylate (0.3196 g, 1.109 mmol) in MeOH (8.87 mL) and water (2.22 mL) at RT and the reaction mixture was stirred 16 h at 60 °C. The reaction mixture was concentrated. The reaction mixture was purified by RP HPLC eluting with acetonitrile:water with 0.1% ammonium hydroxide (5:95-100:0) to give the title compound (0.3059 g, 95 % yield). $^1$H NMR (400 MHz, $CD_3SOCD_3$) δ 1.25 (d, *J* = 7 Hz, 3 H), 2.82 (o, *J* = 7 Hz, 1 H), 3.64 (dt, *J* = 7, 1 Hz, 2 H), 4.19 (dt, *J* = 8, 2 Hz, 2 H), 6.81 (d, *J* = 9 Hz, 1 H), 7.63 (d, *J* = 13 Hz, 1 H), 8.45 (d, *J* = 2 Hz, 1 H), 9.09 (d, *J* = 2 Hz, 1 H); LC-MS (LC-ES) M-H = 259.

**Intermediate 38: 7-((Difluoromethyl)thio)-6-fluoroquinoline-3-carboxylic acid ammonia salt**

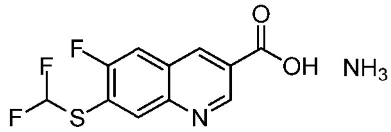

**A. Ethyl 7-(benzhydrylthio)-6-fluoroquinoline-3-carboxylate**

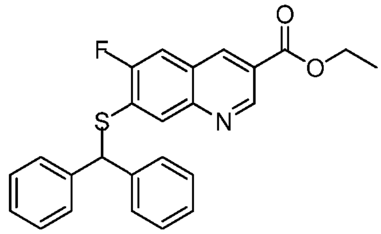

DIEA (1.192 mL, 6.82 mmol) was added to ethyl 6,7-difluoroquinoline-3-carboxylate (Intermediate 26, step B) (0.4046 g, 1.706 mmol) in EtOH (8.53 mL) at RT, then diphenylmethanethiol (0.410 g, 2.047 mmol) was added and the reaction mixture was heated at 100 °C for 4 h. The reaction mixture was poured into saturated sodium bicarbonate and extracted with DCM, dried over magnesium sulfate, filtered, and concentrated. The residue was purified by silica gel chromatography, eluting with EtOAc:hexanes (1:4) to give the title compound (0.4237 g, 56.5 % yield). $^1$H NMR (400 MHz, $CD_3SOCD_3$) δ 1.35 (t, *J* = 7 Hz, 3 H), 4.38 (q, *J* = 7 Hz, 2 H), 6.36 (s, 1 H), 7.25 (t, *J* = 7 Hz, 2 H), 7.36 (t, *J* = 7 Hz, 4 H), 7.60 (d, *J* = 7 Hz, 4 H), 7.89 (d, *J* = 7 Hz, 1 H), 8.02 (d, *J* = 10 Hz, 1 H), 8.88 (d, *J* = 2 Hz, 1 H), 9.16 (d, *J* = 2 Hz, 1 H). LC-MS (LC-ES) M+H = 418.

**B. Ethyl 6-fluoro-7-mercaptoquinoline-3-carboxylate**

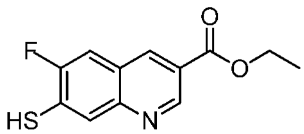

Phenol (0.231 g, 2.456 mmol) was added to ethyl 7-(benzhydrylthio)-6-fluoroquinoline-3-carboxylate (0.4102 g, 0.983 mmol), then 2,2,2-trifluoroacetic acid (3.78 mL, 49.1 mmol) was added at RT and the reaction mixture was stirred at 40 °C for 64 h. The reaction mixture was poured into saturated sodium bicarbonate and extracted with DCM, dried over magnesium sulfate, filtered, and concentrated. The residue was purified by silica gel chromatography, eluting with MeOH:EtOAc (1:49) to give the title compound (0.1414 g, 43.0 % yield). $^1$H NMR (400 MHz, $CD_3SOCD_3$) δ 1.36 (t, *J* = 7 Hz, 3 H), 4.38 (q, *J* = 7 Hz, 2 H), 7.88 (br s, 1 H), 8.04 (br s, 1 H), 8.91 (br s, 1 H), 9.13 (br s, 1 H); LC-MS (LC-ES) M+H = 252.

**C. Ethyl 7-((difluoromethyl)thio)-6-fluoroquinoline-3-carboxylate**

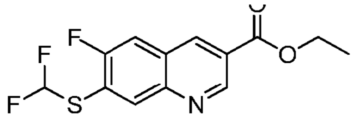

Sodium 2-chloro-2,2-difluoroacetate (0.172 g, 1.125 mmol) was added to ethyl 6-fluoro-7-mercaptoquinoline-3-carboxylate (0.1414 g, 0.563 mmol) in DMF (5.63 mL) and the solution was added dropwise to potassium carbonate (0.117 g, 0.844 mmol) in DMF (5.63 mL) at 90 °C and the reaction mixture was stirred for 3 h. The reaction mixture was quenched with water, extracted with DCM, dried over magnesium sulfate, filtered, and concentrated. The reaction mixture was purified by silica gel chromatography, eluting with EtOAc:hexanes (1:6) to give the title compound (0.0739 g, 41.4 % yield). $^1H$ NMR (400 MHz, $CD_3SOCD_3$) δ 1.38 (t, *J* = 7 Hz, 3 H), 4.42 (q, *J* = 7 Hz, 2 H), 7.74 (t, *J* = 55 Hz, 1 H), 8.24 (d, *J* = 10 Hz, 1 H), 8.40 (d, *J* = 9 Hz, 1 H), 9.04 (d, *J* = 2 Hz, 1 H), 9.33 (d, *J* = 2 Hz, 1 H); LC-MS (LC-ES) M+H = 302.

**D. 7-((Difluoromethyl)thio)-6-fluoroquinoline-3-carboxylic acid ammonia salt**

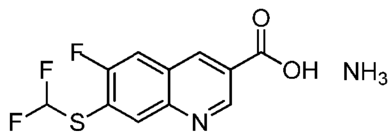

Lithium hydroxide (0.017 g, 0.695 mmol) was added to ethyl 7-((difluoromethyl)thio)-6-fluoroquinoline-3-carboxylate (0.0698 g, 0.232 mmol) in MeOH (1.853 mL) and water (0.463 mL) at RT and the reaction mixture was stirred 16 h at 60 °C. The reaction mixture was concentrated. The reaction mixture was purified by RP HPLC eluting with acetonitrile:water with 0.1% ammonium hydroxide (5:95-100:0) to give the title compound (0.0563 g, 80 % yield). $^1H$ NMR (400 MHz, $CD_3SOCD_3$) δ 7.64 (d, *J* = 56 Hz, 1 H), 8.02 (d, *J* = 10 Hz, 1 H), 8.27 (d, *J* = 7 Hz, 1 H), 8.64 (d, *J* = 2 Hz, 1 H), 9.30 (d, *J* = 2 Hz, 1 H); LC-MS (LC-ES) M-H = 272.

**Intermediate 39: 1-(1-Methyl-1*H*-tetrazol-5-yl)piperidin-4-amine hydrochloride**

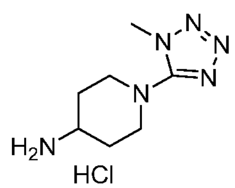

**A. tert-Butyl (1-(1-methyl-1*H*-tetrazol-5-yl)piperidin-4-yl)carbamate**

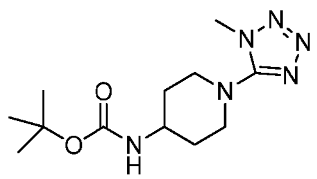

A mixture of tert-butyl piperidin-4-ylcarbamate (5.21 g, 26.0 mmol) and 5-bromo-1-methyl-1*H*-tetrazole (3.53 g, 21.7 mmol) in 2-propanol (40 mL) was treated with DIEA (5.7 mL, 32.5 mmol) and then heated at reflux for 3 h. The mixture was evaporated to dryness, treated with 5 % EtOH in EtOAc (80 mL) and washed with 5 % w/v aqueous citric acid solution (3 x 50 mL). The organic phase was dried over magnesium sulfate, filtered, and evaporated to dryness and the residue was purified by column chromatography on silica using a 0-25 % MeOH in DCM gradient to afford the title compound (3.55 g). MS ES+ve *m*/*z* 283 $[M+H]^+$.

**B. 1-(1-Methyl-1*H*-tetrazol-5-yl)piperidin-4-amine hydrochloride**

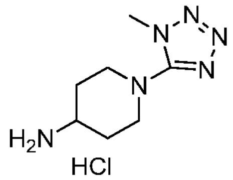

A solution of tert-butyl (1-(1-methyl-1*H*-tetrazol-5-yl)piperidin-4-yl)carbamate (1.59 g, 5.63 mmol) in DCM (30 mL) was treated with trifluoroacetic acid (5 mL) and the mixture was stirred at RT for 1 h. The mixture was evaporated to dryness and then re-evaporated twice to dryness with diethyl ether. The residual oil was treated with THF (20 mL) and then with 4 M HCl in 1,4-dioxane (5 mL). The mixture was stirred for 5 min and then evaporated to dryness to afford the title compound (0.67 g). $^1$H NMR ($CD_3SOCD_3$) δ 1.64-1.78 (m, 2 H), 1.96-2.03 (m, 2 H), 3.00-3.11 (m, 2H), 3.21-3.33 (m, 1 H), 3.62-3.68 (m, 2H), 3.87 (s, 3 H), 8.28 (br s, 2 H).

**Intermediate 40: (Trans)-4-amino-N,N-dimethylcyclohexanecarboxamide**

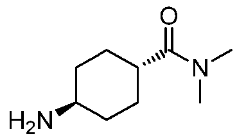

**A. Benzyl ((trans)-4-(dimethylcarbamoyl)cyclohexyl)carbamate**

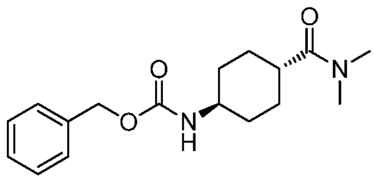

A mixture of (trans)-4-(((benzyloxy)carbonyl)amino)cyclohexanecarboxylic acid (400 mg, 1.44 mmol), dimethylamine hydrochloride (941 mg, 11.54 mmol) and HATU (823 mg, 2.16 mmol) were dissolved in DCM (7.5 mL). DIEA (3.02 mL, 17.31 mmol) was added and the mixture was stirred in a sealed flask at RT for 18 h. The solvent was evaporated under reduced pressure to give a yellow residue. The residue was dissolved in EtOAc (100 mL) and washed sequentially with 2 M hydrochloric acid, water, saturated sodium bicarbonate solution, and brine. The organic layer was dried over $MgSO_4$ and the solvent was evaporated under reduced pressure to give the title compound (425.8 mg). $^1$H NMR ($CD_3SOCD_3$) δ 1.15-1.44 (m, 4 H), 1.67 (m, 2 H), 1.83 (m, 2 H), 2.79 (s, 3 H), 2.99 (s, 3 H), 5.00 (s, 2 H), 7.18 (d, *J* = 7 Hz, 1 H), 7.28-7.42 (m, 5 H).

**B. (trans)-4-Amino-N,N-dimethylcyclohexanecarboxamide**

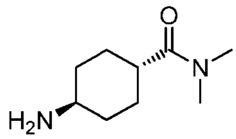

Benzyl ((trans)-4-(dimethylcarbamoyl)cyclohexyl)carbamate (400 mg, 1.314 mmol) was dissolved in EtOAc (15 mL) and added to 10 % palladium on carbon (300 mg, 0.282 mmol) in a hydrogenation flask. The reaction mixture was stirred at RT for 1 h under a hydrogen atmosphere. The mixture was filtered through Celite® and the solvent was evaporated from the filtrate to give the title compound (215.9 mg). $^1$H NMR ($CD_3SOCD_3$) δ 0.98-1.12 (m, 2 H), 1.26-1.41

(m, 2 H), 1.62 (m, 2 H), 1.77 (m, 2 H), 2.40-2.50 (m, 2 H), 2.78 (s, 3 H), 2.99 (s, 3 H).

**Intermediate 41: 2-(3-Aminocyclobutyl)propan-2-ol**

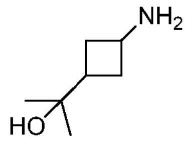

**A. 3-(2-Hydroxypropan-2-yl)cyclobutanol**

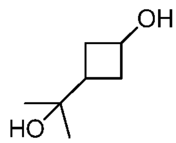

To a $Et_2O$ solution (30 mL) containing methyl magnesium bromide (7.63 mL of a 3.0 M $Et_2O$ solution) was added a $Et_2O$ solution (5 mL) containing ethyl 3- hydroxycyclobutane carboxylate (2.05 g, 6.94 mmol) dropwise at RT. The solution was stirred for 2 h at which time it was carefully quenched with 3 M aqueous HCl. The solution then had $MgSO_4$ added to it until the evolution of gas stopped. The solution was then filtered and the solvent removed *in vacuo* yielding a viscous oil. The oil was purified by silica gel chromatography (50-100% EtOAc/hexanes) to give the title compound (419 mg, 3.22 mmol). $^1H$ NMR ($CDCl_3$) $\delta$ 4.03 - 4.09 (m, 1H), 2.66 (br s, 1H), 2.23 - 2.39 (m, 2H), 1.74 - 1.86 (m, 4H), 1.13 (s, 6H).

**B. 3-(2-Hydroxypropan-2-yl)cyclobutyl4-methylbenzenesulfonate**

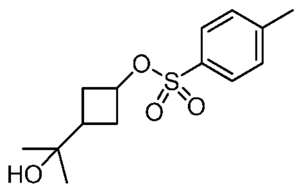

To a pyridine solution (15 mL) containing 3-(2-hydroxypropan-2-yl)cyclobutanol (415 mg, 3.19 mmoL) cooled to 0 °C was added p-toluenesulfonyl chloride (638 mg, 3.35 mmol). The reaction was slowly allowed to warm to RT overnight at which time the organics were taken up in $Et_2O$. The solution was washed with water, saturated $NaHCO_3$ and saturated $NaHSO_4$ followed by drying over $MgSO_4$. The solvent was removed *in vacuo* yielding the title compound (792 mg) as a viscous oil which was taken on crude. $^1H$ NMR ($CDCl_3$) $\delta$ 7.78 (d, *J* = 8 Hz, 2H), 7.33 (d, *J* = 8 Hz, 2H), 4.65 (quin, *J* = 8 Hz, 1H), 2.45 (s, 3H), 2.12 - 2.23 (m, 2H), 1.98 - 2.11 (m, 2H), 1.71 - 1.88 (m, 1H), 1.08 (s, 6H).

**C. 2-(3-Azidocyclobutyl)propan-2-ol**

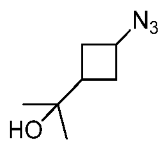

A DMF solution (40 mL) containing 3-(2-hydroxypropan-2-yl)cyclobutyl 4-methylbenzenesulfonate (2.5 g, 8.79 mmol) and sodium azide (686 mg, 10.6 mmol) was heated to 90 °C overnight. Upon cooling the organics were taken up in $Et_2O$ and washed with water (2x) and saturated $NaHCO_3$ followed by drying over $MgSO_4$. The solvent was carefully removed *in vacuo* yielding the title compound (1.19 g) as an oil which was taken on crude. $^1H$ NMR ($CDCl_3$) δ 3.87 - 4.01 (m, 1H), 2.35 - 2.44 (m, 1H), 2.26 - 2.34 (m, 2H), 2.03 - 2.16 (m, 2H), 1.14 (s, 6H).

**D. 2-(3-Aminocyclobutyl)propan-2-ol**

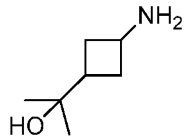

To an EtOH solution (25 mL) containing 10% Pd/C (809 mg, wet Degussa) was added an EtOH solution (5 mL) containing 2-(3-azidocyclobutyl)propan-2-ol (1.18 g, 7.60 mmol). The flask was then evacuated under vacuum and refilled with hydrogen via a balloon. This process was repeated twice more and then the reaction stirred under 1 atm of hydrogen overnight. The catalyst was removed under vacuum filtration though a plug of Celite®. The Celite® was rinsed with DCM and the solvent removed *in vacuo* yielding the title compound (920 mg) as an oil. $^1H$ NMR ($CDCl_3$) δ 3.40 - 3.52 (m, 1H), 2.27 - 2.42 (m, 1H), 2.16 - 2.28 (m, 2H), 1.66 - 1.77 (m, 2H), 1.12 (s, 6H).

**Intermediate 42: 1-((trans-4-Aminocyclohexyl)oxy)-2-methylpropan-2-ol**

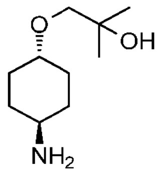

**A. trans-4-(Dibenzylamino)cyclohexanol**

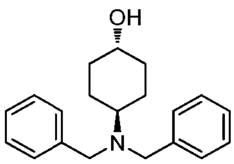

Benzyl bromide (60 g, 351 mmol) was added to (trans)-4-aminocyclohexanol hydrochloride (20 g, 174 mmol) and sodium bicarbonate (40 g, 476 mmol) in EtOH (400 mL) at RT. The reaction was then heated to reflux for 36 h. The reaction was filtered and concentrated to give a solid. To this was added hexanes and stirred overnight and filtered and air dried to afford the title compound (33.2 g, 65% yield). $^1H$ NMR ($CDCl_3$) δ 1.14 - 1.28 (m, 2 H), 1.30 (d, *J* = 5 Hz, 1 H), 1.38 - 1.52 (m, 2 H), 1.91 (d, *J* = 12 Hz, 2 H), 2.00 (br s, 1 H), 2.53 (tt, *J* = 12, 3 Hz, 1 H), 3.50 - 3.59 (m, 1 H), 3.62 (s, 4 H), 7.18 - 7.25 (m, 2 H), 7.27 - 7.32 (m, 4 H), 7.33 - 7.38 (m, 4 H).

**B. tert-Butyl 2-((trans-4-(dibenzylamino)cyclohexyl)oxy)acetate**

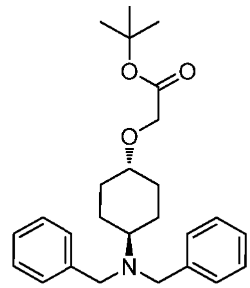

trans-4-(Dibenzylamino)cyclohexanol (1.0 g, 3.4 mmol) and tert-butyl 2-bromoacetate (1.0 mL, 6.8 mmol) were stirred in DMF (5 mL) at 55 °Cthen a 60% dispersion of NaH in mineral oil (0.27 g, 6.8 mmol) was added portion wise over 1 h. Additional tert-butyl 2-bromoacetate (1.00 mL, 6.8 mmol) and NaH (0.27 g, 6.8 mmol) were added portion wise over 1 h. The reaction was allowed to stir at 55 °C overnight, before careful quenching with water. The reaction was diluted with 1.0 N aq. NaOH and extracted with EtOAc. The organic layer was washed with water (2X), followed by brine. The combined aq. fractions were extracted with EtOAc and the combined organics were dried over sodium sulfate, filtered, and concentrated under reduced pressure. The residue was purified by silica gel chromatography eluting with a gradient from 2-15% (3:1 ratio of EtOAc:EtOH in hexanes). The appropriate fractions were concentrated under reduced pressure to afford the title compound as a colorless oil (497 mg, 1.21 mmol). $^1H$ NMR ($CDCl_3$) δ 1.43 - 1.46 (m, 4 H), 1.47 (s, 9 H), 1.92 (d, *J* = 12 Hz, 2 H), 2.07 - 2.14 (m, 2 H), 2.48 - 2.58 (m, 1 H), 3.24 - 3.30 (m, 1 H), 3.61 (s, 4 H), 3.96 (s, 2 H), 7.17 - 7.24 (m, 2 H), 7.26 - 7.32 (m, 4H), 7.34 - 7.38 (m, 4 H). LCMS: ES+ve *m/z* 410 $[M+H]^+$.

**C. 1-((trans-4-(Dibenzylamino)cyclohexyl)oxy)-2-methylpropan-2-ol**

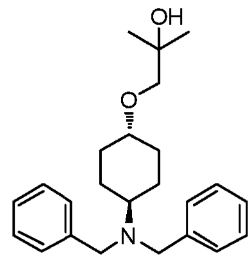

Tert-butyl 2-((trans-4-(dibenzylamino)cyclohexyl)oxy)acetate (0.40 g, 0.977 mmol) was stirred in THF (2.5 mL) under nitrogen at 0 °C, then a 3.0 M solution of methylmagnesium bromide (0.8 mL, 2.4 mmol) in diethyl ether was added. The reaction was stirred at RT for 3 h then quenched with sat. aq. $NH_4Cl$ (3 mL) and stirred at RT overnight. The reaction was diluted with EtOAc, then washed with saturated aqueous sodium bicarbonate, water (2X), and brine. The organics were dried over sodium sulfate, filtered, and concentrated under reduced pressure. The residue was purified by silica gel chromatography eluting with a gradient from 5-15% ((3:1 EtOAc:EtOH) in hexanes), providing a colorless oil that solidified over time to afford the title compound (0.168 g, 0.457 mmol). $^1H$ NMR ($CDCl_3$) δ 1.09 - 1.22 (m, 8 H), 1.33 - 1.47 (m, 2 H), 1.88 - 1.97 (m, 2 H), 2.04 - 2.11 (m, 2 H), 2.53 (tt, *J* = 12, 3 Hz, 1 H), 3.19 (tt, *J* = 11, 4 Hz, 1 H), 3.24 (s, 2 H), 3.61 (s, 4 H), 7.18 - 7.24 (m, 2 H), 7.26 - 7.33 (m, 4 H), 7.34 - 7.39 (m, 4 H). LCMS: ES+ve *m/z* 368 $[M+H]^+$.

**D. 1-((trans-4-Aminocyclohexyl)oxy)-2-methylpropan-2-ol**

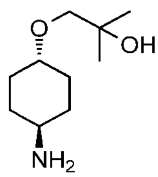

A mixture of 1-((trans-4-(dibenzylamino)cyclohexyl)oxy)-2-methylpropan-2-ol (620 mg, 1.69 mmol) and palladium hydroxide on carbon (300 mg, 2.14 mmol) in EtOH (5 mL) was hydrogenated at 55 psi (Fisher-Porter apparatus) for 2 h. The reaction mixture was filtered through a plug of Celite®. The catalyst was washed with MeOH and DCM. The filtrate was concentrated to dryness and dried under high vacuum to give the title compound as a grey solid (306 mg). $^1$H NMR ($CDCl_3$) δ 1.17 (s, 6 H), 1.07-1.35 (m, 4 H), 1.92 (m, 2 H), 2.02 (m, 2 H), 2.45 (br s, 3 H), 2.78 (m, 1 H), 3.25 (m, 3 H).

**Intermediate 43: (R,S)-1-((trans)-4-Aminocyclohexyl)ethano, Hydrochloride**

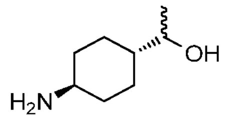

**A. (trans)-Methyl 4-((tertbutoxycarbonyl)amino)cyclohexanecarboxylate**

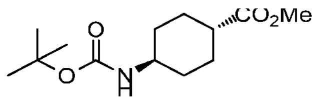

To (trans)-4-(methoxycarbonyl)cyclohexanecarboxylic acid (2.5 mg, 13.43 mmol) in tert-butanol (25 mL) was added diphenyl phosphorazidate (3880 mg, 14.10 mmol) and triethylamine (1.965 mL, 14.10 mmol). The mixture was heated at 60 °C for 1 h and then at reflux overnight. After cooling to RT, the mixture was quenched into ice water and extracted with EtOAc. The combined organics were washed with sat. $NaHCO_3$ and brine, dried over $Na_2SO_4$, filtered, and concentrated. The residue was dissolved in MeOH (6 mL) and to this was added water (18 mL). After stirring for ca. 1 h on ice, the resulting solid was collected by filtration, and washed with 3:1 water:MeOH, and hexane to give the title compound as a white solid (2.32 g, 67% yield). $^1$H NMR (400 MHz, $CD_3SOCD_3$) δ ppm 1.05 - 1.20 (m, 2 H), 1.25 - 1.44 (m, 11 H), 1.72 - 1.91 (m, 4H), 2.17 (tt, *J* = 12, 3 Hz, 1 H), 3.06 - 3.21 (m, 1 H), 3.56 (s, 3 H), 6.72 (d, *J* = 8 Hz, 1 H).

**B. tert-Butyl ((trans)-4-(hydroxymethyl)cyclohexyl)carbamate**

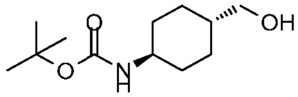

To a solution of (trans)-methyl 4-((tert-butoxycarbonyl)amino)cyclohexanecarboxylate (1.5 g, 5.83 mmol) in EtOH (24 mL) and THF (2.7 mL), cooled on ice, was added calcium chloride (1294 mg, 11.66 mmol) portion wise to give a milky suspension. $NaBH_4$ (882 mg, 23.32 mmol) was then added portion wise over ca. 25 min and the reaction was stirred on ice for 1 h. The bath was removed and the mixture was allowed to stir at RT overnight. The reaction was cooled to 10 °C and to this was added 5% aq. $K_2CO_3$ (5.4 mL) dropwise, to give a pH of ca. 11. A white ppt formed and was isolated by filtration. The solid was stirred with EtOAc (50 mL) and water (14 mL). The layers were separated and the organic layer was washed with 0.5 M aq. HCl (5 mL), water and brine, dried over $MgSO_4$, filtered, and concentrated to give the title compound (474 mg) as a white solid. The initial filtrate was concentrated, then dilute with sat. aq. $NH_4Cl$ and extracted with EtOAc (3X). Combined organics were washed with brine and dried over $Na_2SO_4$, filtered, and concentrated to give the title compound (724 mg) as a white solid. Total isolated product was 1189 mg (89% yield). $^1$H NMR (400 MHz, $CD_3SOCD_3$) δ ppm 0.78 - 0.93 (m, 2 H), 1.01 - 1.15 (m, 2 H), 1.35 (s, 9 H), 1.64 - 1.80 (m, 4 H), 3.10 (d, *J* = 8 Hz, 1 H), 3.16 (t, *J* = 6 Hz, 2 H), 4.33 (t, *J* = 5 Hz, 1 H), 6.64 (d, *J* = 8 Hz, 1 H).

**C. tert-Butyl ((trans)-4-formylcyclohexyl)carbamate**

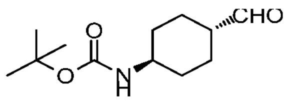

To a solution of tert-butyl ((trans)-4-(hydroxymethyl)cyclohexyl)carbamate (375 mg, 1.635 mmol) in DCM (9 mL) and DMSO (2.8 mL), cooled on ice, was added DIEA (1.142 mL, 6.54 mmol), followed by pyridine sulfur trioxide (1041 mg, 6.54 mmol) dissolved in DMSO (2.8 mL). The bath was removed and the reaction stirred at RT for 15 min. The mixture was partitioned between $Et_2O$ and 1N aq. HCl. The organic phase was washed with 1 N HCl, water, and brine, dried over $MgSO_4$, filtered, and concentrated to give the title compound as a white solid (343 mg, 92% yield), which was used without purification. $^1H$ NMR (400 MHz, $CD_3SOCD_3$) δ ppm 1.12 - 1.23 (m, 4 H), 1.35 (s, 9 H), 1.73 - 1.93 (m, 4 H), 2.07 - 2.19 (m, 1 H), 3.05 - 3.20 (m, 1H), 6.74 (d, $J$ = 7 Hz, 1 H), 9.53 (s, 1 H).

**D. tert-Butyl ((trans)-4-((R,S)-1-hydroxyethyl)cyclohexyl)carbamate**

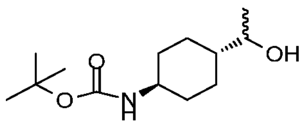

To a solution of tert-butyl ((trans)-4-formylcyclohexyl)carbamate (70 mg, 0.308 mmol) in THF (4 mL), cooled to -78°C, was added methylmagnesium iodide (0.226 mL, 0.678 mmol, 3 M in THF) dropwise over ca. 2 min and the reaction was stirred at -78 °C for 30 min.The reaction was poured into sat. $NH_4Cl$ and extracted with TBME. The combined organics were washed with brine, dried over $Na_2SO_4$, filtered, and concentrated. The residue was purified by silica gel chromatography (0-80% EtOAc-hexanes gradient) to afford the title compound as a glass (15 mg, 20% yield).

A second reaction was run with the following procedure.

To a solution of tert-butyl ((trans)-4-formylcyclohexyl)carbamate (100 mg, 0.440 mmol) in THF (4 mL), cooled to -78 °C, was added methylmagnesium bromide (0.183 mL, 0.55 mmol, 3M in THF) dropwise over ca. 2-3 mins, and the reaction was stirred at -78 °C for 30 min. The reaction was poured into sat. $NH_4Cl$ and extracted with TBME. The combined organics were washed with brine, dried over $Na_2SO_4$, filtered, and concentrated. The residue was purified by silica gel chromatography (0-80% EtOAc-hexanes gradient) to afford the title compound as a white foam (27 mg, 25% yield). This product and the above were combined to be used in the next step. $^1H$ NMR (400 MHz, $CD_3SOCD_3$) δ ppm 0.87 - 1.10 (m, 7 H), 1.35 (s, 9 H), 1.57 (d, $J$ = 10 Hz, 1 H), 1.67 - 1.85 (m, 4 H), 3.08 (d, $J$ = 7 Hz, 1 H), 3.25 - 3.46 (m, 2 H), 6.62 (d, $J$ = 8 Hz, 1 H).

**E. (R,S)-1-((trans)-4-Aminocyclohexyl)ethanol, Hydrochloride**

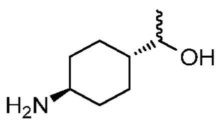

To tert-butyl ((trans)-4-((R,s)-1-hydroxyethyl)cyclohexyl)carbamate (40 mg, 0.164 mmol) was added 4 N HCl in dioxane (5 mL). The mixture was stirred at RT for 3.5 h and then concentrated to give the title compound as a glass (33 mg, 112% yield). $^1H$ NMR (400 MHz, $CD_3SOCD_3$) δ ppm 0.99 (d, $J$ = 6 Hz, 3 H), 1.06 (d, $J$ = 7 Hz, 2 H), 1.20 - 1.29 (m, 2 H), 1.63 (d, $J$ = 12 Hz, 1 H), 1.84 (d, $J$ = 13 Hz, 1 H), 1.93 (d, $J$ = 12 Hz, 2 H), 2.85 (d, $J$ = 5 Hz, 1 H), 3.46 (dd, $J$ = 12, 4 Hz, 1 H), 3.62 - 3.74 (m, 1 H).

**Intermediate 44: 1-((trans-4-Aminocyclohexyl)oxy)propan-2-ol**

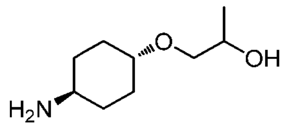

**A. 1-((trans-4-(Dibenzylamino)cyclohexyl)oxy)propan-2-yl hydrogen sulfate**

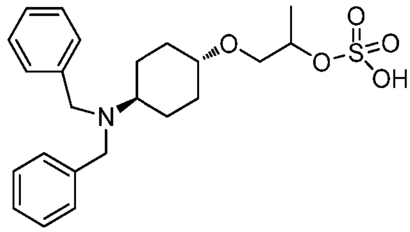

A 250 mL three neck flask equipped with overhead stirrer and an external $CH_3CN$ bath (at RT) was charged with trans-4-(dibenzylamino)cyclohexanol (Intermediate 42, step A) (10 g, 33.9 mmol) and 4-methyl-1,3,2-dioxathiolane 2,2-dioxide (6.5 g, 47.1 mmol) and DMF (50 mL) and stirred at RT under $N_2$. Small amounts of dry ice were added to the $CH_3CN$ cooling bath until the internal temp reached -25 °C (external bath temp ca. -30 °C) and solid sodium tert-pentoxide (5.5 g, 49.9 mmol) was added in 6 portions over 13 min maintaining the internal temp between -25 and -20 °C. Following the addition, the reaction mixture was maintained at -20 °C internal temp for 1 h and at ca. -15 °C for 30 min. The reaction mixture was warmed to 0 °C over 15 min and quenched by addition of ice water (140 mL). TBME (100 mL) were added, the mixture was poured into a separatory funnel shaken vigorously and the layers were separated. The aqueous layer was poured back into the reaction vessel and the solution was adjusted to pH ~7 with conc. HCl (ca. 3.7 mL). $N_2$ was blown over the top of the reaction mixture to help aid in evaporation of residual TBME and the solution was cooled to -5 °C and then aged overnight under $N_2$ with stirring and allowed to warm to RT. The resulting white slurry was cooled to -5 °C and filtered, washing with ice cold water, the white cake was air dried on the filter funnel under $N_2$ flow for 3 h and then dried under vacuum at RT to afford the title compound (11.9 g). $^1H$ NMR (400 MHz, $CD_3SOCD_3$) δ 0.88 - 1.05 (m, 2 H), 1.11 (m, 2 H), 1.40 (q, *J* = 12 Hz, 1 H), 1.68 - 1.85 (m, 2 H), 1.94 - 2.14 (m, 2 H), 2.20 (m, 1 H), 2.38 (m, 1 H), 3.03 - 3.31 (m, 3 H), 3.52 (m, 4 H), 4.17 (m, 2 H), 4.47 (dd, *J* = 13, 4 Hz, 1 H), 7.19 (m, 1 H), 7.31 (m, 3 H), 7.43 (m, 3 H), 7.52 (m, 2 H), 10.08 (br s, 1 H). LCMS: ES+ve *m/z* 434 $[M+H]^+$.

**B. 1-((trans-4-(Dibenzylamino)cyclohexyl)oxy)propan-2-ol**

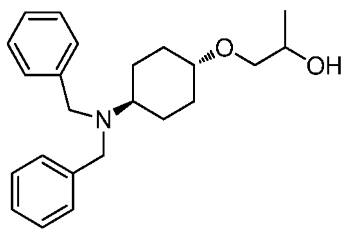

A 250 mL three neck flask was charged with MeOH (150 mL) and the solvent was cooled in an ice water bath under $N_2$. Acetyl chloride (20 mL) was added dropwise over 5 min maintaining the reaction temp below 25 °C. The cooling bath was removed and 1-((trans-4-(dibenzylamino)cyclohexyl)oxy)propan-2-yl hydrogen sulfate (11.9 g, 27.4 mmol) was added. The mixture was stirred under $N_2$ at RT for 3 h. The reaction mixture was concentrated by rotovap and the crude oil was partitioned between TBME (300 mL) and 5% aqueous $Na_2CO_3$ (300 mL) with shaking. The layers were separated and the aqueous layer was extracted with TBME (100 mL). The combined TBME layers were washed with 5% aqueous $Na_2CO_3$ (100 mL), dried over $MgSO_4$, and filtered through a millipore filter pad. The filtrate was concentrated by rotovap to afford the title compound as a colorless oil (8.8 g). $^1H$ NMR (400 MHz, $CD_3SOCD_3$) δ 0.96 (m, 5 H), 1.33 - 1.46 (m, 2 H), 1.81 (m, 2 H), 2.00 (m, 2 H), 2.39 (m, 1 H), 3.14 (m, 2 H), 3.25 (dd, *J* = 9, 6 Hz, 1 H), 3.56

(s, 4 H), 3.63 (dt, *J* = 11, 6 Hz, 1 H), 4.46 (d, *J* = 5 Hz, 1 H), 7.19 (m, 2 H), 7.31 (m, 8 H). LCMS: ES+ve *m/z* 354 $[M+H]^+$.

**C. 1-((trans-4-aminocyclohexyl)oxy)propan-2-ol**

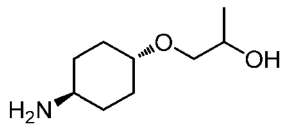

A 1L flask equipped with a magnetic stirrer was charged with a solution of 1-((trans-4-(dibenzylamino)cyclohexyl)oxy)propan-2-ol (8.7 g, 24.61 mmol) in EtOH (80 mL) and purged with $N_2$. Pearlman's catalyst (20 wt% $Pd(OH)_2$ on carbon; 50% wetted Dugussa type E101 NE/W) (2 g) was added. Using a Buchi Press flow gas controller apparatus, the stirred reaction mixture was evacuated and purged with $N_2$ (three cycles) and then with $H_2$ (three cycles) and the mixture was stirred under $H_2$ atmosphere for 3.5 h. The vessel was evacuated and purged with $N_2$ (three cycles) and catalyst was removed by filtration, washing with EtOH and the filtrate was concentrated by rotovap to afford the title compound as a colorless oil (4.2 g). $^1H$ NMR (400 MHz, $CD_3SOCD_3$) $\delta$ 0.92 - 1.22 (m, 7 H), 1.72 (m, 2 H), 1.89 (m, 2 H), 3.15 (m, 2 H), 3.27 (dd, *J* = 9, 6 Hz, 1 H), 3.65 (m, 1 H).

**Intermediate 45: Racemic (5-Aminotetrahydro-2Hpyran-2-yl)methanol, hydrochloride**

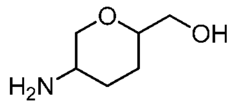

**A. tert-Butyl (1-hydroxy-4-(oxiran-2-yl)butan-2-yl)carbamate**

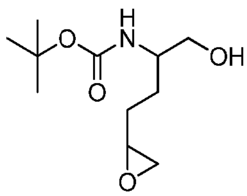

To a solution of tert-butyl (1-hydroxyhex-5-en-2-yl)carbamate (2570 mg, 11.94 mmol) (see; Organic Letters, 12(10), 2322-2325; 2010) in dichloroethane (66 mL), water (55 mL), and phosphate buffer pH 8.0 (33 mL) was added mCPBA (3.24 g, 13.13 mmol) (70% by wt) and the mixture was stirred vigorously overnight. The phases were separated and the aq. phase was extracted with DCM. The combined organics were washed with sat. $NaHCO_3$, dried over $Na_2SO_4$, filtered, and concentrated. The residue was purified by silica gel chromatography (0-100% EtOAc-hexanes gradient) to afford the title compound as a clear oil (1.85 g, 67% yield). $^1H$ NMR (400 MHz, $CDCl_3$) $\delta$ ppm 1.43 (s, 9 H), 1.51 - 1.75 (m, 4 H), 2.32 (br s, 1 H), 2.49 (dt, *J* = 5, 3 Hz, 1 H), 2.76 (q, *J* = 4 Hz, 1 H), 2.90 - 2.98 (m, 1 H), 3.50 - 3.72 (m, 3 H), 4.67 (br s, 1 H).

**B. tert-Butyl (6-(hydroxymethyl)tetrahydro-2Hpyran-3-yl)carbamate**

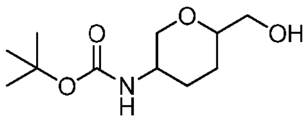

To a solution of tert-butyl (1-hydroxy-4-(oxiran-2-yl)butan-2-yl)carbamate (1.8 g, 7.78 mmol) was in DCM (40 mL) was added DL-10-camphorsulfonic acid (0.181 g, 0.778 mmol) and the mixture was stirred at RT for 4.5 h. The reaction was concentrated and the residue was purified by silica gel chromatography (30-100% EtOAc-hexanes gradient) to afford the title compound as a waxy solid (754 mg, 67% yield), which appeared to be a mixture of cis and trans isomers and was taken directly into the next step.

**C. Racemic (5-Aminotetrahydro-2Hpyran-2-yl)methanol, hydrochloride**

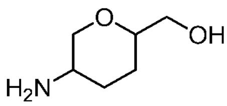

To a solution of tert-butyl (6-(hydroxymethyl)tetrahydro-2H-pyran-3-yl)carbamate (100 mg, 0.432 mmol) (mix of cis and trans) and 1,4-dioxane (2 mL) was added HCl (1.081 mL, 4.32 mmol) (4 M in dioxane) and the mixture was stirred at RT for overnight. The reaction was concentrated to give the title compound as a white solid (82 mg, 113% yield). MS (ESI) *m/z* 132 (M+1).

**Intermediate 46: 1-(trans-4-aminocyclohexyl)cyclopropanol**

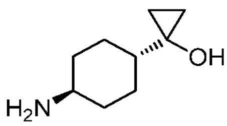

**A. (trans)-Methyl 4-(dibenzylamino)cyclohexanecarboxylate**

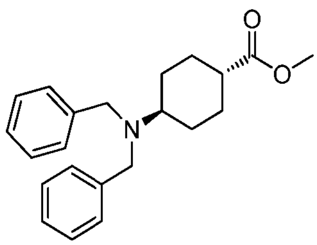

To a solution of (trans)-methyl 4-aminocyclohexanecarboxylate hydrochloride (1.7 g, 8.78 mmol), in acetonitrile (30 mL), was added $K_2CO_3$ (4.85 g, 35.1 mmol) and (bromomethyl)benzene (3.75 g, 21.94 mmol). The reaction was heated at 80 °C overnight, cooled to RT, filtered, and concentrated. The residue was purified by silica gel chromatography (0-20% EtOAc-hexanes gradient) to afford the title compound as a white solid (1.55 g, 52% yield). $^1H$ NMR (400 MHz, $CDCl_3$) δ ppm 1.29 - 1.42 (m, 4 H), 1.91 - 2.03 (m, 4 H), 2.19 (ddd, *J* = 12, 8, 3 Hz, 1 H), 2.51 (ddd, *J* = 11, 8, 3 Hz, 1 H), 3.62 (s, 4 H), 3.60 (s, 3 H), 7.14 - 7.22 (m, 2 H), 7.24 - 7.31 (m, 4H), 7.33 - 7.37 (m, 4 H).

**B. 1-((trans)-4-(Dibenzylamino)cyclohexyl)cyclopropanol**

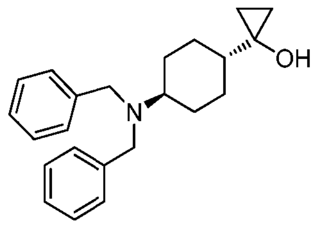

To a solution of (trans)-methyl 4-(dibenzylamino)cyclohexanecarboxylate (500 mg, 1.482 mmol) in THF (15 mL) was added titanium(IV) isopropoxide (0.608 mL, 2.074 mmol). To this mixture was added ethylmagnesium chloride (2.074 mL, 4.15 mmol, 2 M in THF)) dropwise over 30 min. The reaction was allowed to stir overnight. The reaction was quenched by the addition of water (25 mL) with stirring, filtered to remove the ppt and the filtrate extracted with $Et_2O$ (3X). The combined organics were washed with water and brine, dried over $Na_2SO_4$, filtered, and concentrated. The residue was purified by silica gel chromatography (0-35% EtOAc-hexanes gradient) to afford the title compound as a white solid (291 mg, 59% yield). $^1H$ NMR (400 MHz, $CDCl_3$) $\delta$ ppm 0.37 - 0.44 (m, 2 H), 0.64 - 0.69 (m, 2 H), 0.81 - 0.94 (m, 1 H), 1.11 - 1.25 (m, 2 H), 1.30 - 1.43 (m, 2 H), 1.59 - 1.66 (m, 1 H), 1.76 - 1.85 (m, 2 H), 1.91 - 2.01 (m, 2 H), 2.49 (tt, *J* = 12, 3 Hz, 1 H), 3.61 (s, 4 H), 7.16 - 7.22 (m, 2 H), 7.24 - 7.30 (m, 4 H), 7.33 - 7.38 (m, 4 H). MS (ESI) *m/z* 336 (M+1).

**C. 1-(trans-4-aminocyclohexyl)cyclopropanol**

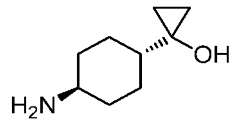

A Fisher-Porter bottle was flushed with $N_2$ and charged with 1-((trans)-4-(dibenzylamino)cyclohexyl)cyclopropanol (285 mg, 0.850 mmol) and EtOH (10 mL). Under a $N_2$ atm Pearlman's catalyst (59.7 mg, 0.085 mmol) was added. The vessel was evacuated and flushed with $N_2$, then stirred under 30 psi $H_2$ for 20 h. The vessel was evacuated and flushed with $N_2$, and the mixture was filtered through a pad of Celite®, washing with MeOH. The filtrate was concentrated to give the title compound as a white semi-solid (124 mg, 94% yield). $^1H$ NMR (400 MHz, $CDCl_3$) $\delta$ ppm 0.41 - 0.48 (m, 2 H), 0.67 - 0.77 (m, 2 H), 0.91 (tt, *J* = 12, 3 Hz, 1 H), 1.02 - 1.15 (m, 2 H), 1.36 (qd, *J* = 13, 3 Hz, 2 H), 1.79 (d, *J* = 12 Hz, 2 H), 1.93 (d, *J* = 13 Hz, 2 H), 2.64 (tt, *J* = 11, 4 Hz, 1 H).

**Intermediate 47: 3-((trans-4-Aminocyclohexyl)oxy)-1,1,1-trifluoro-2-methylpropan-2-ol**

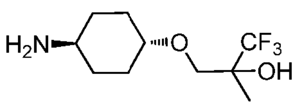

**A. 2-((trans-4-(Dibenzylamino)cyclohexyl)oxy)acetic acid hydrochloride**

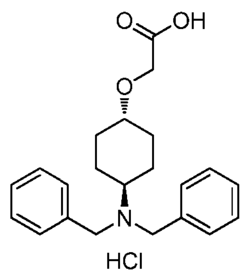

tert-Butyl 2-((trans-4-(dibenzylamino)cyclohexyl)oxy)acetate (Intermediate 42, step B) (2.0 g, 4.88 mmol) was stirred in DCM (5.0 mL) and TFA (3 mL) was added. The reaction was stirred at RT for 1 h before concentrating under reduced pressure. The residue was taken up in 1,4-dioxane (5 mL) and 4.0M HCl in dioxane (2 mL) was added and then stirred at 0 °C. Diethyl ether (20 mL) was added and the resulting precipitate was stirred at the same temperature for ~10 min before collecting by vacuum filtration. The solid was washed with diethyl ether providing the title compound as a white solid (2.03 g, 5.21 mmol). LCMS: ES+ve *m/z* 354 $[M+H]^+$.

**B. 2-((trans-4-(Dibenzylamino)cyclohexyl)oxy)-N-methoxy-N-methylacetamide**

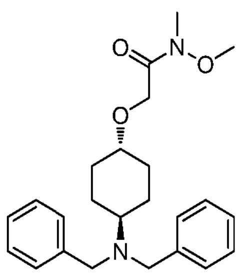

2-((trans-4-(Dibenzylamino)cyclohexyl)oxy)acetic acid hydrochloride (2.0 g, 5.13 mmol) was dissolved in DMF (50 mL) followed by the addition of DIEA (3 mL, 17.18 mmol) and HATU (2.34 g, 6.16 mmol). The reaction was stirred at RT for ca. 5 min, and then N,O-dimethylhydroxylamine hydrochloride (0.751 g, 7.69 mmol) was added. The reaction was allowed to stir at RT overnight. The reaction was diluted with EtOAc and washed with saturated aqueous sodium bicarbonate. The organics were separated and washed with brine, dried over sodium sulfate, filtered, and concentrated under reduced pressure. The residue was purified by silica gel chromatography eluting with a gradient from 50-90% EtOAc/heptanes. The appropriate fractions were concentrated under reduced pressure and solidified under high vacuum to afford the title compound as a yellow solid (1.735 g, 4.38 mmol). $^1H$ NMR ($CDCl_3$) δ 1.17 - 1.30 (m, 2 H), 1.34 - 1.46 (m, 2 H), 1.92 (d, *J* = 12 Hz, 2 H), 2.14 (d, *J* =11 Hz, 2 H), 2.49 - 2.60 (m, 1 H), 3.18 (s, 3 H), 3.27 - 3.37 (m, 1 H), 3.61 (s, 4 H), 3.68 (s, 3 H), 4.27 (s, 2 H), 7.18 - 7.40 (m, 10 H). LCMS: ES+ve *m/z* 398 $[M+H]^+$.

**C. 1-((trans-4-(Dibenzylamino)cyclohexyl)oxy)propan-2-one**

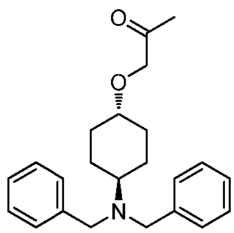

2-((trans-4-(Dibenzylamino)cyclohexyl)oxy)-N-methoxy-N-methylacetamide (1.73 g, 4.36 mmol) was stirred in THF (7 mL) at 0 °C then a 3.0 M solution in diethyl ether of methylmagnesium bromide (2 mL, 6.0 mmol) was slowly added. The reaction was allowed to stir at the same temperature for ca. 10 min then warmed to RT and stirred overnight. The reaction was quenched with saturated aqueous $NH_4Cl$ and extracted with EtOAc. The organics were washed with brine, dried over sodium sulfate, filtered, and concentrated under reduced pressure to afford the title compound as an orange oil (1.52 g, 4.32 mmol). $^1H$ NMR ($CDCl_3$) δ 1.16 - 1.28 (m, 2 H), 1.33 - 1.48 (m, 2 H), 1.94 (d, *J* = 12 Hz, 2 H), 2.04 - 2.12 (m, 2 H), 2.15 (s, 3 H), 2.50 - 2.60 (m, 1 H), 3.17 - 3.26 (m, 1 H), 3.61 (s, 4 H), 4.03 (s, 2 H), 7.18 - 7.24 (m, 2 H), 7.29 (m, 4 H), 7.34 - 7.39 (m, 4 H). LCMS: ES+ve *m/z* 352 $[M+H]^+$.

**D. 3-((trans-4-(Dibenzylamino)cyclohexyl)oxy)-1,1,1-trifluoro-2-methylpropan-2-ol**

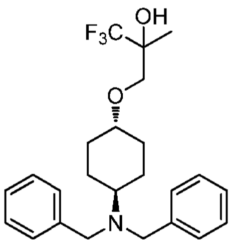

1-((trans-4-(Dibenzylamino)cyclohexyl)oxy)propan-2-one (1.0 g, 2.85 mmol) and cesium fluoride (0.432 g, 2.85 mmol) in THF (6.5 mL) were stirred at 0 °C, then trimethyl(trifluoromethyl)silane (1.2 mL, 8.12 mmol) was slowly added. The reaction was stirred at 0 °C for 1 h. A1.0 M solution in THF of TBAF (3.2 mL, 3.20 mmol) was added and the reaction was allowed to stir at 0 °C for 1 h. The reaction was diluted with EtOAc and washed with water, followed by brine. The organics were dried over sodium sulfate, filtered, and concentrated under reduced pressure to give an orange oil that was purified by silica gel chromatography eluting with 30% EtOAc/heptanes. The appropriate fractions were concentrated under reduced pressure to afford the title compound as a yellow solid. $^1$H NMR ($CDCl_3$) δ 1.14 - 1.24 (m, 2 H), 1.31 (s, 3 H), 1.34 - 1.47 (m, 2 H), 1.91 (br s, 2 H), 2.06 (d, *J* = 7 Hz, 2 H), 2.49 - 2.59 (m, 1 H), 3.19 - 3.28 (m, 1 H), 3.35 (dd, *J* = 10, 1 Hz, 1 H), 3.61 (s, 4 H), 3.67 (d, *J* = 10 Hz, 1 H), 7.19 - 7.24 (m, 2 H), 7.27 - 7.32 (m, 4 H), 7.33 - 7.39 (m, 4 H). LCMS: ES+ve *m/z* 422 $[M+H]^+$.

**E. 3-((trans-4-Aminocyclohexyl)oxy)-1,1,1-trifluoro-2-methylpropan-2-ol**

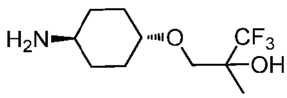

3-((trans-4-(Dibenzylamino)cyclohexyl)oxy)-1,1,1-trifluoro-2-methylpropan-2-ol (0.923 mL, 2.107 mmol) and 20 wt% Pearlman's catalyst (0.089 g, 0.632 mmol) was stirred in EtOH (20 mL) and flushed with hydrogen via balloon (3x) before being left to stir at RT overnight. The reaction was filtered through a pad of Celite® and washed with EtOAc. The filtrate was concentrated under reduced pressure to afford the title compound as a green oil (0.483 g, 2.00 mmol). $^1$H NMR ($CDCl_3$) δ 1.11 - 1.22 (m, 2 H), 1.28 - 1.41 (m, 5 H), 1.86 - 1.95 (m, 2 H), 2.02 (d, *J* = 10 Hz, 2 H), 2.71 - 2.81 (m, 1 H), 3.30 (m, 1 H), 3.39 (dd, *J* = 10, 1 Hz, 1 H), 3.70 (d, *J* = 10 Hz, 1 H). LCMS: ES+ve *m/z* 242 $[M+H]^+$.

**Intermediate 48: (R,S)-1-((trans)-4-Aminocyclohexyl)-2,2,2-trifluoroethanol, hydrochloride**

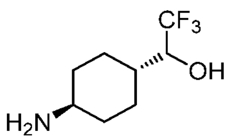

**A. tert-Butyl ((trans)-4-((R,S)-2,2,2-trifluoro-1-hydroxyethyl)cyclohexyl)carbamate**

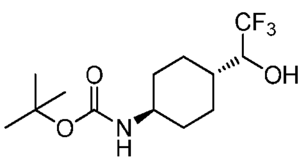

To an ice cold solution of tert-butyl (trans-4-formylcyclohexyl)carbamate (Intermediate 43, step C) (445 mg, 1.958 mmol) in THF (20 mL) was added cesium fluoride (892 mg, 5.87 mmol) followed by trimethyl(trifluoromethyl)silane

(1.736 mL, 11.75 mmol) . The reaction was stirred at 0 °C for 30 min, quenched into water and extract with EtOAc (2X). The combined organics were washed with brine, dried over $Na_2SO_4$, and concentrated. The residue was dissolved in THF (20 mL), cooled in a ice bath and tetrabutylammonium fluoride (3.92 mL, 3.92 mmol, 1 M in THF) was added dropwise. After stirring for 30 min, the mixture was quenched into sat. $NH_4Cl$ and extracted with EtOAc. The combined organic phases were washed with brine, dried over $Na_2SO_4$, filtered, and concentrated. The residue was purified by silica gel chromatography (0-50% EtOAc-hexanes gradient) to afford the title compound as a white solid (275 mg, 47% yield). $^1H$ NMR (400 MHz, $CDCl_3$) $\delta$ ppm 1.01 - 1.17 (m, 2 H), 1.28 - 1.39 (m, 2 H), 1.42 (s, 9 H), 1.60 - 1.82 (m, 1 H), 1.90 - 2.19 (m, 4 H), 2.30 - 2.43 (m, 1 H), 3.38 (br s, 1 H), 3.67 - 3.81 (m, 1 H), 4.38 (br s, 1 H).

**B. (R,S)-1-((trans)-4-aminocyclohexyl)-2,2,2-trifluoroethanol, hydrochloride**

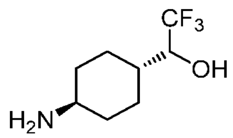

To tert-butyl ((trans)-4-((R,S)-2,2,2-trifluoro-1-hydroxyethyl)cyclohexyl)carbamate (270 mg, 0.908 mmol) under a $N_2$ atm was added 1,4-dioxane (10 mL), followed by HCl (2.270 mL, 9.08 mmol, 4 M in dioxane) . The mixture was stirred at RT overnight and concentrated to give the title compound as a white solid (235 mg, 109% yield). MS (ESI) *m/z* 198 (M+1).

**Intermediate 49: Racemic 2-((2S.5R)-5-Aminotetrahvdro-2Hpyran-2-yl)propan-2-ol hydrochloride**

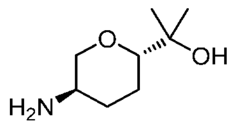

**A. N-Benzyl-6-(((tertbutyldiphenylsilyl)oxy)methyl)tetrahydro-2Hpyran-3-amine**

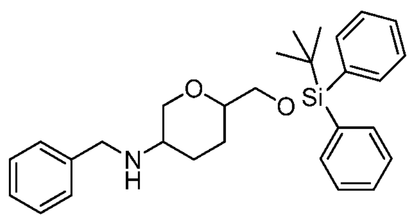

Benzylamine (3.17 mL, 32.6 mmol) was added to a solution of 6-(((tertbutyldiphenylsilyl)oxy)methyl)dihydro-2H-pyran-3(4H)-one (4 g, 10.85 mmol) (see; Biogranic and Medicinal Chemistry 14 (2006), 3953) in MeOH (30 mL) and the mixture was stirred at RT for 1 h. The mixture was cooled to -78 °C and $LiBH_4$ (5.97 mL, 11.94 mmol) was added. The reaction was maintained at -78 °C for 1 h and allowed to warm to RT slowly overnight. The mixture was partitioned between EtOAc and sat. $NaHCO_3$ and the aq layer was extract with EtOAc (2X). The combined organics were washed with brine, dried over $Na_2SO_4$, and concentrated. The residue was purified by silica gel chromatography (0-40% (3:1 EtOAc/EtOH)-hexanes gradient) to afford the title compound as a yellow oil (3.49 g, 70% yield) (mixture of cis and trans isomers). MS (ESI) *m/z* 460 (M+1).

**B. 6-(((tert-Butyldiphenylsilyl)oxy)methyl)tetrahydro-2Hpyran-3-amine**

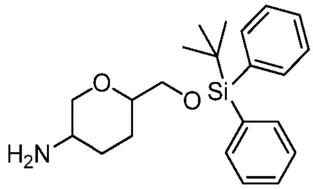

A Fisher-Porter bottle was flushed with $N_2$ and charged with N-benzyl-6-(((tertbutyldiphenylsilyl)oxy)methyl)tetrahydro-2H-pyran-3-amine (3.9 g, 8.48 mmol) and EtOH (80 mL). Under a $N_2$ atm Pearlman's catalyst (1489 mg, 2.121 mmol) was added and the vessel was evacuated and flushed with $N_2$ then stirred under 50 psi $H_2$ over 4 days. The vessel was evacuated and flushed with $N_2$ and filtered through a pad of Celite®, washing with MeOH. The filtrate was concentrated to give the title compound as a clear thick oil (2.98 g, 94%). MS (ESI) *m/z* 370 (M+1).

**C. tert-Butyl (6-(((tertbutyldiphenylsilyl)oxy)methyl)tetrahydro-2Hpyran-3-yl)carbamate**

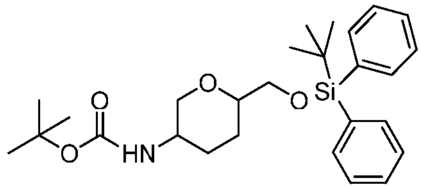

To a solution of 6-(((tert-butyldiphenylsilyl)oxy)methyl)tetrahydro-2H-pyran-3-amine (2.98 g, 8.06 mmol) and $Et_3N$ (1.686 mL, 12.10 mmol) in DCM (100 mL) at 0 °C was added $Boc_2O$ (2.153 mL, 9.27 mmol). The mixture was stirred on ice for 1 h then at RT overnight. The reaction was diluted with DCM and washed with water and brine. The aq phases were back extracted with DCM, and the combined organics were dried over $Na_2SO_4$, filtered, and concentrated to give a thick oil. The residue was purified by silica gel chromatography (0-20% (EtOAc-hexanes gradient) to afford the title compound as a thick oil (2.4 g, 70% yield). MS (ESI) *m/z* 470 (M+1).

**D. tert-Butyl (6-(hydroxymethyl)tetrahydro-2Hpyran-3-yl)carbamate**

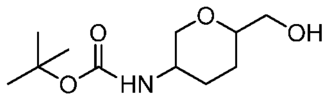

To a solution of tert-butyl (6-(((tert-butyldiphenylsilyl)oxy)methyl)tetrahydro-2H-pyran-3-yl)carbamate (2.4 g, 5.11 mmol) in THF (60 mL), cooled on ice, was added TBAF (10.22 mL, 10.22 mmol, 1M in THF). The bath was removed and the reaction was stirred at RT for 2 h. The mixture was concentrated to ca. half volume under reduced pressure, 15 mL of sat. $NaHCO_3$ was added, and it was extracted with EtOAc (2X). The combined organics were dried over $Na_2SO_4$, filtered, and concentrated to give an oil. The residue was purified by silica gel chromatography (0-20% (EtOAc-hexanes gradient) to afford the title compound as a white solid (1.12 g, 94% yield). This material was used directly in the next step.

**E. 5-((tert-Butoxycarbonyl)amino)tetrahydro-2Hpyran-2-carboxylic acid**

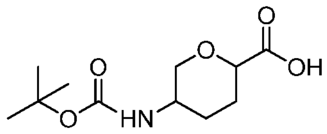

To an ice cooled solution of tert-butyl (6-(hydroxymethyl)tetrahydro-2H-pyran-3-yl)carbamate (1100 mg, 4.76 mmol) in DCM (10 mL), acetonitrile (10 mL), and water (15 mL), was added sodium periodate (4069 mg, 19.02 mmol) and ruthenium(III) chloride (99 mg, 0.476 mmol). The mixture was vigorously stirred for 3 h, then diluted with EtOAc (50 mL) and filtered to remove solids. To the filtrate was added 10 mL of MeOH and the mixture was filtered to remove solids. To the filtrate was added 20 mL of 10% aq. $NaHSO_3$ solution which resulted in decolorization. The pH was adjusted to ca. 2 with addition of 20 % aq. $NaHSO_4$ and the layers were separated. The aq. layer was extracted with EtOAc (3X) and the combined organics were dried over $MgSO_4$ and concentrated to give the title compound as a yellow foam (1.21 g, 104% yield), which was used without further purification. MS (ESI) *m*/*z* 244 (M-1).

**F. Methyl 5-((tert-butoxycarbonyl)amino)tetrahydro-2Hpyran-2-carboxylate**

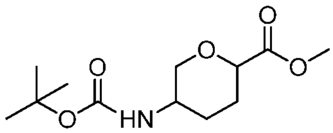

A solution of diazomethane (900 mg, 21.40 mmol) in DCM (generated from N-methyl-N-nitrosourea (2.5 g) added to 30% aq. NaOH (50 mL) and DCM (40 mL) on ice) was added slowly to an ice cold solution of5-((tert-butoxycarbonyl)amino)tetrahydro-2H-pyran-2-carboxylic acid (1050 mg, 4.28 mmol) in DCM (50 mL) and the reaction as stirred on ice for 30 mins.The bath was removed and the mixture flushed well with $N_2$ to remove excess diazomethane. To this was added $CH_3CO_2H$ (2 drops) with stirring (to quench any remaining diazomethane), and the solution was washed with sat. $NaHCO_3$. The mixture was dried over $Na_2SO_4$, filtered, and concentrated. The residue was purified by silica gel chromatography (0-40% (EtOAc-hexanes gradient) to afford the title compound as a white waxy solid (882 mg, 79% yield). $^1H$ NMR (400 MHz, $CDCl_3$) $\delta$ ppm 1.42 (s, 9 H), 1.71 - 1.82 (m, 1 H), 2.00 - 2.17 (m, 2 H), 3.13 (br s, 1 H), 3.66 (dd, *J* = 12, 2 Hz, 1 H), 3.76 (s, 3 H), 3.86 - 4.04 (m, 1 H), 4.17 (dd, *J* = 11, 3 Hz, 1 H), 4.35 (br s, 1 H).

**G. Methyl 5-aminotetrahydro-2H-pyran-2-carboxylate hydrochloride**

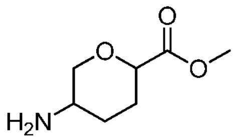

To a solution of methyl 5-((tert-butoxycarbonyl)amino)tetrahydro-2H-pyran-2-carboxylate (880 mg, 3.39 mmol) was added 1,4-dioxane (25 mL), followed by HCl (8.48 mL, 33.9 mmol, 4M in dioxane). The mixture was stirred at RT overnight. The reaction was concentrated to give the title compound as a white solid (670 mg, 101% yield). MS (ESI) *m*/*z* 160 (M+1).

**H. Racemic (2S,5R)-Methyl 5-(dibenzylamino)tetrahydro-2H-pyran-2-carboxylate**

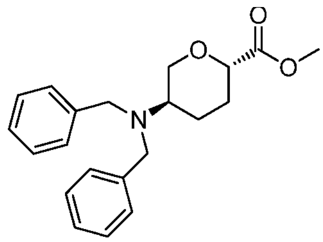

To a solution of methyl 5-aminotetrahydro-2H-pyran-2-carboxylate hydrochloride (670 mg, 3.42 mmol) in acetonitrile (25 mL), was added potassium carbonate (1893 mg, 13.70 mmol) and (bromomethyl)benzene (0.915 mL, 7.71 mmol) and the mixture was heated at 80 °C overnight. After cooling to RT, the mixture was filtered, and concentrated. The residue was purified by silica gel chromatography (0-40% (EtOAc-hexanes gradient) to afford the title compound as a clear oil (881 mg, 76% yield), which was assigned as the trans isomer by NMR. $^1H$ NMR (400 MHz, $CDCl_3$) $\delta$ ppm 1.54 - 1.75 (m, 2 H), 2.05 - 2.17 (m, 2 H), 2.83 (tt, *J* = 11, 4 Hz, 1 H), 3.42 (t, *J* = 11 Hz, 1 H), 3.58 - 3.74 (m, 7 H), 3.86 (dd, *J* = 12, 2 Hz, 1 H), 4.09 - 4.17 (m, 1 H), 7.11 - 7.48 (m, 10 H). MS (ESI) *m/z* 340 (M+1).

**I. Racemic 2-((2S,5R)-5-(Dibenzylamino)tetrahydro-2H-pyran-2-yl)propan-2-ol**

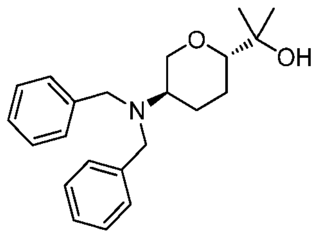

To a solution of racemic (2S,5R)-methyl 5-(dibenzylamino)tetrahydro-2H-pyran-2-carboxylate (535 mg, 1.576 mmol) in THF (18 mL), cooled on ice, was added methylmagnesium bromide (4.20 mL, 12.61 mmol, 3M in THF) dropwise.The mixture was allowed to warm to RT overnight. The mixture was cooled on ice and quenched by the slow addition of 1 M $NH_4Cl$ (25 mL). This was then extracted with EtOAc (2X), combined organics were dried over $Na_2SO_4$, filtered, and concentrated. The residue was purified by silica gel chromatography (0-45%, EtOAc-hexanes gradient) to afford the title compound as a clear oil (268 mg, 50% yield). $^1H$ NMR (400 MHz, $CDCl_3$) $\delta$ ppm 1.07 (s, 3 H), 1.13 (s, 3 H), 1.22 - 1.38 (m, 1H), 1.58 (dd, *J* = 12, 4 Hz, 1 H), 1.71 (d, *J* = 13 Hz, 1 H), 2.03 - 2.12 (m, 1 H), 2.39 (s, 1 H), 2.72 (tt, *J* = 11, 4 Hz, 1 H), 2.99 (dd, *J* = 11, 2 Hz, 1 H), 3.40 (t, *J* = 11 Hz, 1 H), 3.58 - 3.73 (m, 4H), 3.99 - 4.09 (m, 1 H), 7.06 - 7.50 (m, 10 H). MS (ESI) *m/z* 340 (M+1).

**J. Racemic 2-((2S,5R)-5-Aminotetrahydro-2H-pyran-2-yl)propan-2-ol hydrochloride**

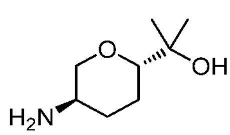

A Fisher-Porter bottle was flushed with $N_2$ and charged with racemic 2-((2S,5R)-5-(dibenzylamino)tetrahydro-2H-pyran-2-yl)propan-2-ol (265 mg, 0.781 mmol) and EtOH (7 mL). Under a $N_2$ atm was added Pearlman's catalyst (137 mg, 0.195 mmol), and the vessel was evacuated and flushed with $N_2$ and then stirred under 40 psi $H_2$ for 24 h. The vessel was evacuated and flushed with $N_2$ and the mixture filtered through a pad of Celite®. To the filtrate was added 4N HCl in dioxane (1 mL), and then the reaction mixture was concentrated to give the title compound as a white foam (147 mg, 96% yield). $^1H$ NMR (400 MHz, $CD_3SOCD_3$) $\delta$ ppm 0.98 (s, 3 H), 1.04 (s, 3 H), 1.24 - 1.38 (m, 1 H), 1.41 - 1.54 (m, 1 H), 1.75 (d, *J* = 13 Hz, 1 H), 2.06 (d, *J* = 12 Hz, 1 H), 2.87 - 3.04 (m, 2 H), 3.22 (t, *J* = 11 Hz, 1 H), 3.95 - 4.07 (m, 1 H). MS (ESI) *m/z* 160 (M+1).

**Intermediate 50: trans-4-((3-azidopropoxy)methyl)cyclohexanamine, hydrochloride**

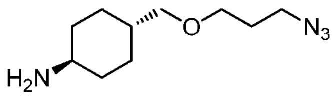

**A. tert-Butyl (trans-4-((allyloxy)methyl)cyclohexyl)carbamate**

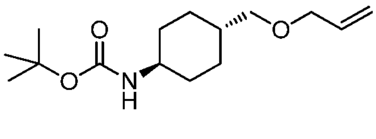

To tert-butyl (trans-4-(hydroxymethyl)cyclohexyl)carbamate (intermediate 43, step B) (250 mg, 1.090 mmol) was added 3-bromoprop-1-ene (3 g, 24.80 mmol), tetrabutylammonium hydrogen sulfate (130 mg, 0.382 mmol), and 50 % NaOH (4 mL). The mixture was stirred at RT for 3 days and then diluted with water and extracted with DCM (2X). The combined organics were dried over $Na_2SO_4$, filtered, and concentrated. The residue was purified by silica gel chromatography (0-35% EtOAc-hexanes gradient) to afford the title compound as a semi-solid (153 mg, 52% yield). $^1H$ NMR (400 MHz, $CDCl_3$) δ ppm 0.96 - 1.16 (m, 4 H), 1.39 - 1.44 (m, 11 H), 1.82 (d, *J* = 11 Hz, 2 H), 1.95 - 2.06 (m, 2 H), 3.22 (d, *J* = 7 Hz, 2 H), 3.93 (d, *J* = 6 Hz, 2 H), 5.15 (d, *J* = 10 Hz, 1 H), 5.25 (dd, *J* = 17, 2 Hz, 1 H), 5.81 - 5.99 (m, 1 H).

**B. tert-Butyl (trans-4-((3-hydroxypropoxy)methyl)cyclohexyl)carbamate**

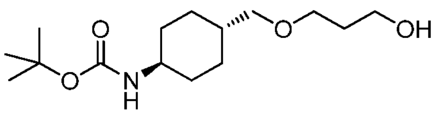

To a solution of tert-butyl (trans-4-((allyloxy)methyl)cyclohexyl)carbamate (150 mg, 0.557 mmol) in THF (5 mL) was added $BH_3$•THF (0.278 mL, 0.278 mmol) dropwise. After stirring at RT for 3 h, NaOH (0.928 mL, 2.78 mmol, 3 M) was added, followed by the slow addition of $H_2O_2$ (0.284 mL, 2.78 mmol). The mixture was stirred at RT for 3 h, diluted with EtOAc and wash with 5% $Na_2S_2O_3$ (aq.), water, and brine, then dried over $Na_2SO_4$, filtered, and concentrated. The residue was purified by silica gel chromatography (0-75% EtOAc-hexanes gradient) to afford the title compound as a semi-solid (110 mg, 69% yield). $^1H$ NMR (400 MHz, $CDCl_3$) δ ppm 0.94 - 1.11 (m, 4H), 1.42 (s, 9 H), 1.46-1.56 (m, 1 H), 1.77 - 1.85 (m, 4 H), 1.96 - 2.14 (m, 2 H), 2.41 (t, *J* = 5 Hz, 1 H), 3.22 (d, *J* = 7 Hz, 2 H), 3.59 (t, *J* = 6 Hz, 2 H), 3.76 (q, *J* = 6 Hz, 2H).

**C. 3-((trans-4-((tert-Butoxycarbonyl)amino)cyclohexyl)methoxy)propyl methanesulfonate**

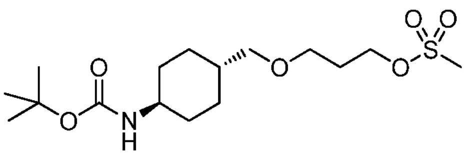

To a solution of tert-butyl (trans-4-((3-hydroxypropoxy)methyl)cyclohexyl)carbamate (108 mg, 0.376 mmol) in DCM (4 mL), and pyridine (0.122 mL, 1.503 mmol), cooled on ice, was added methanesulfonyl chloride (0.059 mL, 0.752 mmol) dropwise over ca. 10 mins. The reaction was stirred at RT overnight, and then concentrated. The residue was dissolved in EtOAc, washed with water and brine, dried over $Na_2SO_4$, filtered, and concentrated to give the title compound

as a white solid (119 mg, 87% yield). $^1H$ NMR (400 MHz, $CDCl_3$) δ ppm 0.85 - 1.16 (m, 4 H), 1.43 (s, 9 H), 1.44-1.56 (m, 1 H), 1.79 (d, *J* = 11 Hz, 2 H), 1.92 - 2.06 (m, 4 H), 2.99 (s, 3 H), 3.20 (d, *J* = 7 Hz, 2 H), 3.32 - 3.42 (m, 1 H), 3.48 (t, *J* = 6 Hz, 2 H), 4.32 (t, *J* = 6 Hz, 2 H).

**D. tert-Butyl (trans-4-((3-azidopropoxy)methyl)cyclohexyl)carbamate**

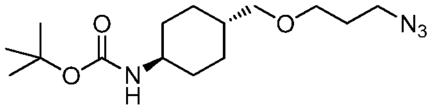

To a solution of 3-((trans-4-((tert-butoxycarbonyl)amino)cyclohexyl)methoxy)propyl methanesulfonate (110 mg, 0.301 mmol) in DMSO (3 mL) was added sodium azide (21.52 mg, 0.331 mmol) and the reaction was stirred at RT for 2 days. The mixture was diluted with water (ca. 15 mL) and extracted with EtOAc (3X). The combined organics were dried over $Na_2SO_4$, filtered, and concentrated to give the title compound as a clear oil (85 mg, 90% yield). $^1H$ NMR (400 MHz, $CDCl_3$) δ ppm 0.98 - 1.17 (m, 4 H), 1.44 (s, 9 H), 1.44 - 1.56 (m, 1 H), 1.76 - 1.90 (m, 4H), 1.96 - 2.09 (m, 2 H), 3.22 (d, *J* = 6 Hz, 2 H), 3.38 (t, *J* = 7 Hz, 2 H), 3.47 (t, *J* = 6 Hz, 2 H), 4.27 - 4.48 (m, 1 H).

**E. trans-4-((3-Azidopropoxy)methyl)cyclohexanamine hydrochloride**

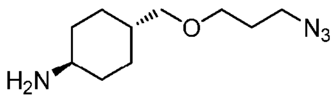

To a solution of tert-butyl (trans-4-((3-azidopropoxy)methyl)cyclohexyl)carbamate (97 mg, 0.310 mmol), in 1,4-dioxane (3 mL) was added HCl (0.310 mL, 1.242 mmol, 4M in dioxane), and the mixture was stirred at RT for 4 h. The reaction was concentrated to give the title compound as a white waxy solid (82 mg, 107% yield). MS (ESI) *m/z* 213 (M+1).

<u>**Intermediate 51: 3-Amino-1-methylcyclobutanol hydrochloride**</u>

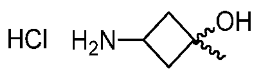

**A. 3-Methylenecyclobutanecarboxylic acid**

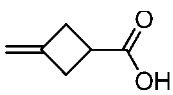

Potassium hydroxide (12.29 g, 219 mmol) was added to 3-methylenecyclobutanecarbonitrile (5.10 g, 54.8 mmol) in EtOH (27.4 mL) and water (27.4 mL) at RT and the reaction mixture was heated at reflux for 16 h. After cooling, the EtOH was removed under vacuum, ice was added, and the reaction mixture was acidified to pH = 1 with concentrated hydrochloric acid. The reaction mixture was extracted with EtOAc, dried over magnesium sulfate, filtered, and concentrated to give the title compound (6.37 g, 99 % yield). $^1H$ NMR (400 MHz, $CD_3SOCD_3$) δ 2.82 (dt, *J* = 9, 2 Hz, 4 H), 3.04 (p, *J* = 8 Hz, 1 H), 4.76 (p, *J* = 2 Hz, 2 H), 12.20 (br s, 1 H); LC-MS (LC-ES) M-H = 111.

**B. *tert*-Butyl (3-methylenecyclobutyl)carbamate**

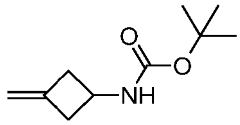

Triethylamine (11.88 mL, 85 mmol) was added to 3-methylenecyclobutanecarboxylic acid (6.37 g, 56.8 mmol) in *tert*-butanol (56.8 mL) at RT, followed by diphenyl phosphoryl azide (14.69 mL, 68.2 mmol) and the reaction mixture was heated at 85 °C under nitrogen for 17 h. The reaction mixture was quenched with water and concentrated. The reaction mixture was taken up in diethyl ether, washed with 10% citric acid, followed by saturated sodium bicarbonate, dried over magnesium sulfate, filtered, and concentrated. The residue was purified by silica gel chromatography, eluting with EtOAc:hexanes (1:9) to give the title compound (6.08 g, 55.5% yield). $^1H$ NMR (400 MHz, $CD_3SOCD_3$) δ 1.36 (s, 9 H), 2.54-2.64 (m, 2 H), 2.76-2.86 (m, 2 H), 3.92 (h, *J* = 8 Hz, 1 H), 4.76 (p, *J* = 2 Hz, 2 H), 7.23 (d, *J* = 7 Hz, 1 H).

**C. *tert*-Butyl 1-oxaspiro[2.3]hexan-5-ylcarbamate**

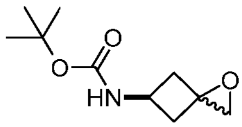

3-Chloroperbenzoic acid (6.30 g, 36.5 mmol) was added to *tert*-butyl (3-methylenecyclobutyl)carbamate (6.08 g, 33.2 mmol) in DCM (111 mL) at 0 °C and the reaction mixture was stirred for 4 h. The reaction mixture was washed with 10% sodium sulfite, followed by saturated sodium bicarbonate, and brine, dried over magnesium sulfate, filtered, and concentrated. The residue was purified by silica gel chromatography, eluting with EtOAc:hexanes (1:4 to 2:3) to give a 1:1 mixture of *tert*-butyl 1-oxaspiro[2.3]hexan-5-ylcarbamate (5.34 g, 25.5 mmol, 77 % yield) and recovered starting material (0.98 g, 16% yield). $^1H$ NMR (400 MHz, $CD_3SOCD_3$) δ 1.37 & 1.37 (s, 9 H), 2.30-2.40 (m, 2 H), 2.44-2.54 (m, 2 H), 2.62 (s, 1 H), 2.66 (s, 1 H), 3.83 (h, *J* = 8 Hz, 0.5 H), 3.94-4.06 (m, 0.5 H), 7.24 (d, *J* = 7 Hz, 0.5 H), 7.34 (d, *J* = 6 Hz, 0.5 H).

**D. *tert*-Butyl (3-hydroxy-3-methylcyclobutyl)carbamate**

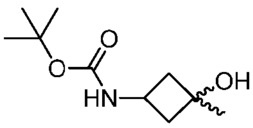

Lithium triethylborohydride (34.8 mL, 34.8 mmol) in THF (1.0 M) was added to *tert*-butyl 1-oxaspiro[2.3]hexan-5-ylcarbamate (5.34 g, 26.8 mmol) in THF (89 mL) at 0 °C under nitrogen and the reaction mixture was stirred for 3 h at 0 °C. The reaction mixture was quenched with water, solid potassium carbonate was added, then extracted with diethyl ether, dried over magnesium sulfate, filtered, and concentrated. The residue was purified by silica gel chromatography, eluting with EtOAc:hexanes (2:3 to 1:1) to give the title compound (5.36 g, 25.3 mmol, 94 % yield). $^1H$ NMR (400 MHz, $CD_3SOCD_3$) δ 1.17 (s, 1.5 H), 1.19 (s, 1.5 H), 1.35 (s, 9 H), 1.78-1.92 (m, 2 H), 2.08-2.18 (m, 2 H), 3.46 (h, *J* = 8 Hz, 0.5 H), 3.99 (h, *J* = 8 Hz, 0.5 H), 4.70 (br s, 0.5 H), 4.83 (br s, 0.5 H), 7.00 (d, *J* = 6 Hz, 1 H); LC-MS (ES-MS) M+H = 202.

**E. 3-Amino-1-methylcyclobutanol hydrochloride**

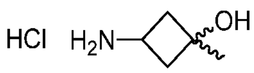

4.0 M Hydrochloric acid (33.3 mL, 133 mmol) in dioxane was added to *tert*-butyl (3-hydroxy-3-methylcyclobutyl)carbamate (5.36 g, 26.6 mmol) in MeOH (33.3 mL) at RT and the reaction mixture was stirred for 16 h. The reaction mixture was concentrated to give the title compound (3.83 g, 26.4 mmol, 99 % yield). $^1H$ NMR (400 MHz, $CD_3SOCD_3$) δ 1.22 (s, 1.5 H), 1.25 (s, 1.5 H), 2.04-2.14 (m, 2 H), 2.14-2.26 (m, 2 H), 3.15 (s, 0.5 H), 3.20-3.32 (m, 0.5 H), 3.56 (s, 0.5 H), 3.66-3.78 (m, 0.5 H), 8.09 (br s, 3 H); LC-MS (LC-ES) M+H = 102.

**Intermediate 52: tert-Butyl (cis-3-hydroxy-3-methylcyclobutyl)carbamate**

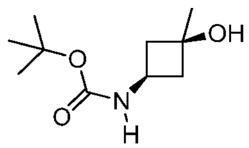

Cerium(III) chloride heptahydrate (80.165 g, 215 mmole) was dried at 140°C under high vacuum overnight. The powder was cooled to RT and added with stirring to a 1L Morton flask containing THF (400 mL). The slurry was stirred for 60 min and was cooled to -78 °C. Methyllithium (1.6M solution in diethyl ether, 135 mL, 216 mmole) was added and the mixture was stirred at -78 °C for 60 minutes. A solution of tert-butyl (3-oxocyclobutyl)carbamate (20.021 g, 108 mmole) in THF (200 mL) was added dropwise over 60 minutes, and the resulting mixture was stirred at -78°C for 2.5 h. The reaction was poured into a mixture of saturated aqueous ammonium chloride (400 mL) and water (400 mL). The two layers were separated and the aqueous layer was extracted with EtOAc (3 x 200 mL). The combined organics were washed with brine (1 x 100 mL), dried over magnesium sulfate and concentrated. The crude residue was purified by silica gel chromatography, eluting with 30-80% EtOAc/heptane gradient to provide the title compound (18.663 g, 86%) as a white solid. $^1H$ NMR ($CDCl_3$): δ 1.35 (s, 3H), 1.43 (s, 9H), 1.98 (t, *J* = 9 Hz, 2H), 2.41-2.54 (m, 3H), 3.72 (d, *J* = 7 Hz, 1H), 4.74 (br s, 1H).

**Intermediate 53: trans-N1-(2.2-Difluoroethyl)cyclohexane-1.4-diamine dihydrochloride**

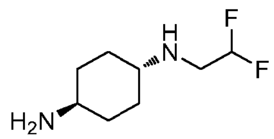

**A. tert-Butyl (trans-4-((2,2-difluoroethyl)amino)cyclohexyl)carbamate**

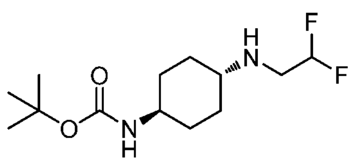

DIEA (1.63 mL, 9.33 mmol) was added to tert-butyl (trans-4-aminocyclohexyl)carbamate (1.00 g, 4.67 mmol) in 1,4-dioxane (7.70 mL) at RT followed by 2,2-difluoroethyl trifluoromethanesulfonate (1.20 g, 5.60 mmol) and the

reaction mixture was stirred at 70 °C for 16 h. The reaction mixture was concentrated and the residue was purified by silica gel chromatography, eluting with EtOAc:hexanes (3:2) to give the title compound (0.791 g, 2.70 mmol). $^1$H NMR ($CD_3SOCD_3$) δ 0.97 (dq, *J* = 13, 3 Hz, 2 H), 1.11 (dq, *J* = 14, 3 Hz, 2 H), 1.35 (s, 9 H), 1.73 (m, 3 H), 1.82 (d, *J* = 12 Hz, 2 H), 2.28 (m, 1 H), 2.85 (tt, J = 16, 5 Hz, 2 H), 3.13 (m, 1 H), 5.90 (dt, , *J* = 56, 5 Hz, 1 H), 6.68 (d, *J* = 8 Hz, 1 H); LCMS: ES+ve *m/z* 279 $[M+H]^+$.

**B. trans-N1-(2,2-Difluoroethyl)cyclohexane-1,4-diamine dihydrochloride**

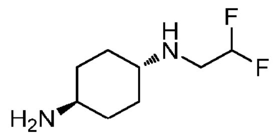

4.0 M Hydrochloric acid (7.10 mL, 28.4 mmol) in dioxane was added to tert-butyl (trans-4-((2,2-difluoroethyl)amino)cyclohexyl)carbamate (0.791 g, 2.84 mmol) in MeOH (7.10 mL) at RT and the reaction mixture was stirred for 20 h. The reaction mixture was concentrated to give the title compound (0.705 g, 2.67 mmol). $^1$H NMR (400 MHz, $CD_3SOCD_3$) (51.35 (q, *J* = 13 Hz, 2 H), 1.45 (q, *J* = 13 Hz, 2 H), 2.01 (d, *J* = 11 Hz, 2 H), 2.13 (d, *J* = 11 Hz, 2 H), 2.95 (m, 2 H), 3.32-3.58 (m, 2 H), 6.48 (tt, *J* = 54, 3 Hz, 1 H), 9.61 (br s, 2 H); LCMS: ES+ve *m/z* 179 $[M+H]^+$.

**Intermediate 54: *trans*-N1-(1,1,1-Trifluoropropan-2-yl)cyclohexane-1,4-diamine dihydrochloride**

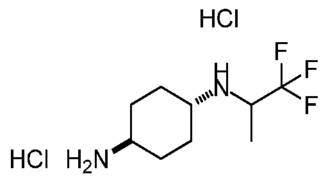

**A. *tert*-Butyl (*trans*-4-((1,1,1-trifluoropropan-2-yl)amino)cyclohexyl)carbamate**

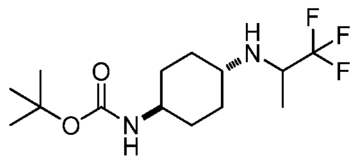

DIEA (1.646 mL, 9.43 mmol) was added to *tert*-butyl (*trans*-4-aminocyclohexyl)carbamate (1.01 g, 4.71 mmol) in 1,4-dioxane (7.78 mL) at RT, followed by 1,1,1-trifluoropropan-2-yl trifluoromethanesulfonate (1.392 g, 5.66 mmol) and the reaction mixture was stirred at 70 °C for 16 h. The reaction mixture was concentrated and the residue was purified by silica gel chromatography, eluting with EtOAc:hexanes (1:4) to give the title compound (0.9062 g, 58.9 % yield). $^1$H NMR (400 MHz, $CD_3SOCD_3$) δ 0.98 (dq, *J* = 11 Hz, 2 H), 1.11 (dq, *J* = 13, 3 Hz, 2 H), 1.11 (d, *J* = 7 Hz, 3 H), 1.35 (s, 9 H), 1.66-1.82 (m, 4 H), 1.82-1.90 (m, 1 H), 2.30-2.44 (m, 1 H), 3.06-3.18 (m, 1 H), 3.28 (h, *J* = 7 Hz, 1 H), 6.66 (d, *J* = 8 Hz, 1 H); LC-MS (LC-ES) M+H = 311.

**B. *trans*-N1-(1,1,1-Trifluoropropan-2-yl)cyclohexane-1,4-diamine dihydrochloride**

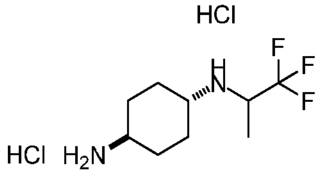

4.0 M Hydrochloric acid (7.30 mL, 29.2 mmol) in dioxane was added to *tert*-butyl (*trans*-4-((1,1,1-trifluoropropan-2-yl)amino)cyclohexyl)carbamate (0.9062 g, 2.92 mmol) in MeOH (7.30 mL) at RT and the reaction mixture was stirred for 15 h. The reaction mixture was concentrated to give the title compound (0.8173 g, 2.74 mmol, 94 % yield). $^1H$ NMR (400 MHz, $CD_3SOCD_3$) δ 1.26-1.70 (m, 6 H), 1.99 (br s, 3 H), 2.14 (br s, 2 H), 2.93 (br s, 1 H), 3.15 (br s, 1 H), 4.41 (br s, 1 H), 8.08 (br s, 3 H), 9.56 (br s, 1 H), 10.25 (br s, 1 H); LC-MS (LC-ES) M+H = 211.

**Intermediate 55: 1-(2,2,2-Trifluoroethyl)piperidin-4-amine dihydrochloride**

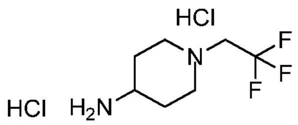

**A. *tert*-Butyl (1-(2,2,2-trifluoroethyl)piperidin-4-yl)carbamate**

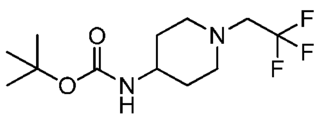

DIEA (0.640 mL, 3.67 mmol) was added to *tert*-butyl piperidin-4-ylcarbamate (0.3679 g, 1.837 mmol) in 1,4-dioxane (3.03 mL) at RT, followed by 2,2,2-trifluoroethyl trifluoromethanesulfonate (0.512 g, 2.204 mmol) and the reaction mixture was stirred at 75 °C for 7 days. The reaction mixture was concentrated and the residue was purified by silica gel chromatography, eluting with EtOAc:hexanes (1:4) to give the title compound (0.501 g, 92 % yield). $^1H$ NMR (400 MHz, $CD_3SOCD_3$) δ 1.36 (s, 9 H), 1.37 (dq, *J* = 12, 4 Hz, 2 H), 1.64 (br d, *J* = 11 Hz, 2 H), 2.30 (dt, *J* = 11, 2 Hz, 2 H), 2.83 (br d, *J* = 12 Hz, 2 H), 3.09 (q, *J* = 10 Hz, 2 H), 3.12-3.26 (m, 1 H), 6.76 (d, *J* = 8 Hz, 1 H); LC-MS (LC-ES) M+H = 283.

**B. 1-(2,2,2-Trifluoroethyl)piperidin-4-amine dihydrochloride**

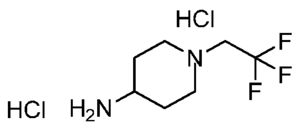

4.0 M Hydrochloric acid (4.44 mL, 17.75 mmol) in dioxane was added to *tert*-butyl (1-(2,2,2-trifluoroethyl)piperidin-4-yl)carbamate (0.501 g, 1.775 mmol) in MeOH (4.44 mL) at RT and the reaction mixture was stirred for 17 h. The reaction mixture was concentrated to give the title compound (0.4604 g, 97 % yield). $^1H$ NMR (400 MHz, $CD_3SOCD_3$) δ 1.56 (br q, *J* = 11 Hz, 2 H), 1.85 (br d, *J* = 11 Hz, 2 H), 2.38-2.56 (m, 2 H), 2.90-3.08 (m, 3 H), 3.28 (br s, 2 H), 4.95 (br s, 1 H), 8.00 (br s, 3 H); LC-MS (LC-ES) M+H = 183.

**Intermediate 56: *trans*-N1-(2,2,2-Trifluoroethyl)cyclobutane-1,3-diamine dihydrochloride**

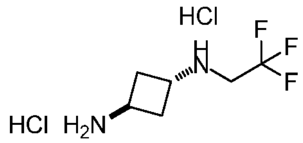

**A. *tert*-Butyl (*trans*-3-((2,2,2-trifluoroethyl)amino)cyclobutyl)carbamate**

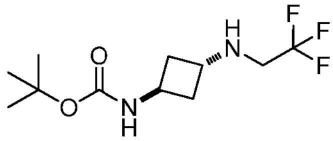

DIEA (0.935 mL, 5.37 mmol) was added to *tert*-butyl (*trans*-3-aminocyclobutyl)carbamate (0.50 g, 2.68 mmol) in 1,4-dioxane (4.43 mL) at RT, followed by 2,2,2-trifluoroethyl trifluoromethanesulfonate (0.748 g, 3.22 mmol) and the reaction mixture was stirred at 75 °C for 64 h. The reaction mixture was concentrated and the residue was purified by silica gel chromatography, eluting with EtOAc:hexanes (2:3) to give the title compound (0.649 g, 86 % yield). $^1$H NMR (400 MHz, $CD_3SOCD_3$) $\delta$ 1.35 (s, 9 H), 1.88-2.02 (m, 4 H), 2.54-2.68 (m, 1 H), 3.02-3.18 (m, 2 H), 3.20-3.36 (m, 1 H), 3.96 (h, *J* = 7 Hz, 1 H), 7.12 (d, *J* = 7 Hz, 1 H); LC-MS (LC-ES) M+H = 269.

**B. *trans*-N1-(2,2,2-Trifluoroethyl)cyclobutane-1,3-diamine dihydrochloride**

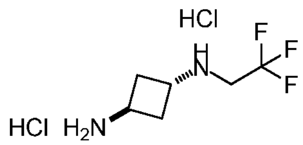

4.0 M Hydrochloric acid (6.05 mL, 24.20 mmol) in dioxane was added to *tert*-butyl (*trans*-3-((2,2,2-trifluoroethyl)amino)cyclobutyl)carbamate (0.6493 g, 2.420 mmol) in MeOH (6.05 mL) at RT and the reaction mixture was stirred for 15 h. The reaction mixture was concentrated to give the title compound (0.590 g, 96 % yield). $^1$H NMR (400 MHz, $CD_3SOCD_3$) $\delta$ 2.34-2.68 (m, 4 H), 3.88 (br s, 3 H), 4.38 (br s, 1 H), 8.34 (br s, 3 H), 10.50 (br s, 2 H); LC-MS (LC-ES) M+H = 169.

**Intermediate 57: N2-(2.2.2-Trifluoroethyl)spiro[3.3]heptane-2,6-diamine dihydrochloride**

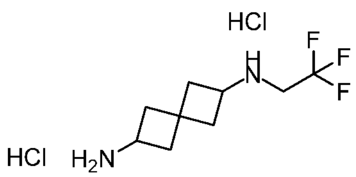

**A. *tert*-Butyl (6-((2,2,2-trifluoroethyl)amino)spiro[3.3]heptan-2-yl)carbamate**

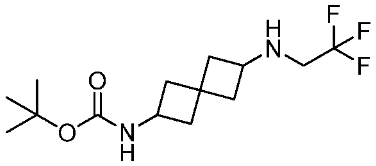

DIEA (0.773 mL, 4.44 mmol) was added to *tert*-butyl (6-aminospiro[3.3]heptan-2-yl)carbamate (0.502 g, 2.218 mmol) in 1,4-dioxane (3.66 mL) at room temperature, followed by 2,2,2-trifluoroethyl trifluoromethanesulfonate (0.618 g, 2.66 mmol) and the reaction mixture was stirred at 75 °C for 64 h. The reaction mixture was concentrated and the residue was purified by silica gel chromatography, eluting with EtOAc:hexanes (3:7) to give the title compound (0.5465 g, 76 % yield). $^1$H NMR (400 MHz, $CD_3SOCD_3$) δ 1.34 (s, 9 H), 1.60-1.70 (m, 2 H), 1.76-1.86 (m, 2 H), 1.96-2.08 (m, 2 H), 2.14-2.26 (m, 2 H), 2.45 (q, *J* = 6 Hz, 1 H), 3.00-3.14 (m, 3 H), 3.77 (h, *J* = 8 Hz, 1 H), 7.02 (d, *J* = 8 Hz, 1 H); LC-MS (LC-ES) M+H = 309.

**B. N2-(2,2,2-Trifluoroethyl)spiro[3.3]heptane-2,6-diamine dihydrochloride**

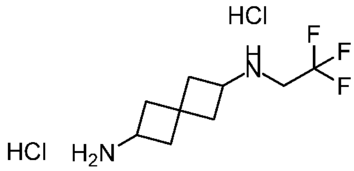

4.0 M Hydrochloric acid (4.43 mL, 17.72 mmol) in dioxane was added to *tert*-butyl (6-((2,2,2-trifluoroethyl)amino)spiro[3.3]heptan-2-yl)carbamate (0.5465 g, 1.772 mmol) in MeOH (4.43 mL) at RT and the reaction mixture was stirred for 64 h. The reaction mixture was concentrated to give the title compound (0.486 g, 93 % yield). $^1$H NMR (400 MHz, $CD_3SOCD_3$) δ 2.10-2.40 (m, 8 H), 3.50-4.00 (m, 4 H), 8.10 (br s, 3 H), 10.21 (br s, 2 H); LC-MS (LC-ES) M+H = 209.

**Intermediate 58: (1R.3R)-N1-(2,2,2-Trifluoroethyl)cyclopentane-1,3-diamine dihydrochloride**

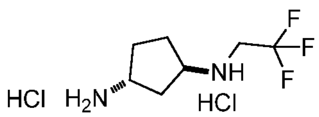

**A. *tert*-Butyl ((1R,3R)-3-((2,2,2-trifluoroethyl)amino)cyclopentyl)carbamate**

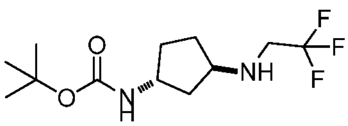

DIEA (1.57 mL, 8.99 mmol) was added to *tert*-butyl ((1R,3R)-3-aminocyclopentyl)carbamate (900 mg, 4.49 mmol) in 1,4-dioxane (9 mL) at RT, followed by 2,2,2-trifluoroethyl trifluoromethanesulfonate (1252 mg, 5.39 mmol) and the reaction mixture was stirred at 70 °C for 18 h. The reaction mixture was concentrated and the residue was purified by silica gel chromatography, eluting with EtOAc:hexanes (10:90-60:40) to give the title compound (1.2 g, 95 % yield) as a white solid. $^1$H NMR (400 MHz, $CDCl_3$) δ 1.32-1.46 (m, 3 H), 1.44 (s, 9 H), 1.64-1.84 (m, 2 H), 1.94-2.06 (m, 1 H), 2.10-2.22 (m, 1 H), 3.14 (q, *J* = 10 Hz, 2 H), 3.33 (p, *J* = 6 Hz, 1 H), 4.00-4.16 (m, 1 H), 4.46 (br s, 1 H).

**B. (1R,3R)-N1-(2,2,2-Trifluoroethyl)cyclopentane-1,3-diamine dihydrochloride**

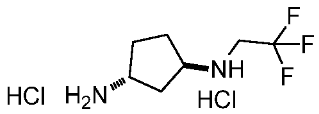

4 N Hydrochloric acid (8 mL, 32.0 mmol) in dioxane was added to *tert*-butyl ((1R,3R)-3-((2,2,2-trifluoroethyl)amino)cyclopentyl)carbamate (1.2 g, 4.25 mmol) in DCM (1.5 mL). A precipitate appeared immediately. More DCM was added to make it possible to stir and the mixture was stirred at RT for 3 h at which time the solvents were removed to afford the title compound (1.15 g, 106 % yield) as a white solid. $^1$H NMR (400 MHz, $CD_3OD$) δ 1.70-1.88 (m, 2 H), 2.22-2.48 (m, 4 H), 3.82-3.90 (m, 1 H), 3.92-4.04 (m, 1 H), 4.04-4.16 (m, 2 H).

**Intermediate 59: 1-(2,2-Difluoroethyl)piperidin-4-amine**

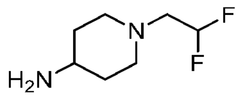

**A. Benzyl (1-(2,2-difluoroethyl)piperidin-4-yl)carbamate**

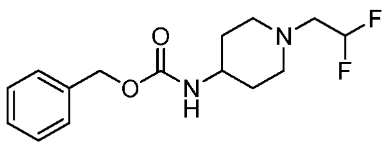

DIEA (2.230 mL, 12.80 mmol) was added to benzyl piperidin-4-ylcarbamate (1.00 g, 4.27 mmol) in 1,4-dioxane (6.31 mL) at room temperature, followed by 2,2-difluoroethyl trifluoromethanesulfonate (1.097 g, 5.12 mmol) and the reaction mixture was stirred at 85 °C for 44 h. The reaction mixture was concentrated and the residue was purified by silica gel chromatography, eluting with EtOAc:hexanes (1:1) to give the title compound (1.08 g, 81 % yield). $^1$H NMR (400 MHz, $CD_3SOCD_3$) δ 1.38 (dq, *J* = 12, 3 Hz, 2 H), 1.68 (d, *J* = 13 Hz, 2 H), 2.16 (t, *J* = 10 Hz, 2 H), 2.66 (dt, *J* = 16, 4 Hz, 2 H), 2.81 (br d, *J* = 12 Hz, 2 H), 3.18-3.32 (m, 1 H), 4.98 (s, 2 H), 6.08 (tt, *J* = 56, 4 Hz, 1 H), 7.24 (d, *J* = 8 Hz, 1 H), 7.26 (m, 5 H); LC-MS (LC-ES) M+H = 298.

**B. 1-(2,2-Difluoroethyl)piperidin-4-amine**

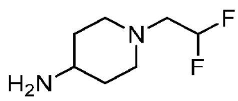

Palladium on carbon (0.039 g, 0.362 mmol) was added to benzyl (1-(2,2-difluoroethyl)piperidin-4-yl)carbamate (1.08 g, 3.62 mmol) in MeOH (18.10 mL) at 25 °C under nitrogen atmosphere. Then, the reaction vessel was fitted with a hydrogen balloon and the vessel was repeatedly evacuated and purged with hydrogen, then stirred for 62 h. Then, the vessel was repeatedly evacuated and purged with nitrogen, filtered through Celite®, and concentrated to give the title compound (0.4898 g, 78 % yield). $^1$H NMR (400 MHz, $CD_3SOCD_3$) δ 1.19 (dq, *J* = 10, 2 Hz, 2 H), 1.43 (br s, 2 H), 1.56-1.66 (m, 2 H), 2.11 (dt, *J* = 11, 2 Hz, 2 H), 2.42-2.52 (m, 1 H), 2.64 (dt, *J* = 16, 5 Hz, 2 H), 2.78 (dt, *J* = 12, 4 Hz, 2

H), 6.08 (tt, *J* = 56, 4 Hz, 1 H); LC-MS (LC-ES) M+H = 165.

**Intermediate 60: *trans*-N1-(1,1,1-Trifluoro-2-methylpropan-2-yl)cyclohexane-1.4-diamine**

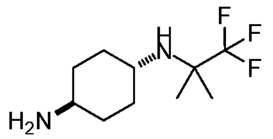

**A. Benzyl (*cis*-4-((1,1,1-trifluoro-2-methylpropan-2-yl)amino)cyclohexyl)carbamate and Benzyl (*trans*-4-((1,1,1-trifluoro-2-methylpropan-2-yl)amino)cyclohexyl)carbamate**

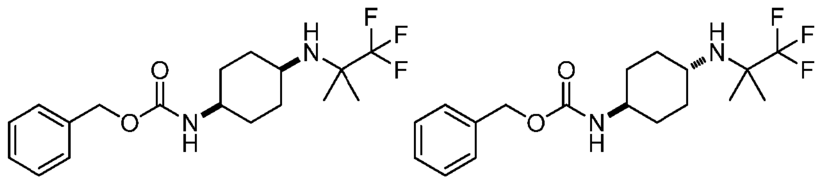

1,1,1-Trifluoro-2-methylpropan-2-amine (1.02 g, 8.02 mmol) was added to benzyl (4-oxocyclohexyl)carbamate (1.984 g, 8.02 mmol) in 1,2-dichloroethane (40 mL) at RT and stirred for 5 min, followed by acetic acid (0.024 g, 0.401 mmol) and 4A molecular sieves (8.0 g) and the reaction was stirred for 16 h at 50 °C. Then, sodium triacetoxyborohydride (1.701 g, 8.02 mmol) was added, and the reaction mixture was stirred for 7 days. The reaction mixture was filtered through Celite®, saturated sodium bicarbonate added, extracted with DCM, dried over magnesium sulfate, filtered, and concentrated. The residue was purified by silica gel chromatography, eluting with EtOAc:hexanes (1:3 to 1:1) to give benzyl (*cis*-4-((1,1,1-trifluoro-2-methylpropan-2-yl)amino)cyclohexyl)carbamate (0.0468 g, 1.6 % yield) and benzyl (*trans*-4-((1,1,1-trifluoro-2-methylpropan-2-yl)amino)cyclohexyl)carbamate (0.139 g, 4.6 % yield).

**Benzyl (*cis*-4-((1,1,1-trifluoro-2-methylpropan-2-yl)amino)cyclohexyl)carbamate:** $^1$H NMR (400 MHz, $CD_3SOCD_3$) δ 1.18 (s, 6 H), 1.38-1.62 (m, 8 H), 2.76-2.86 (m, 1 H), 3.32-3.44 (m, 1 H), 4.99 (s, 2 H), 7.14 (d, *J* = 7 Hz, 1 H), 7.26-7.38 (m, 5 H); LC-MS (LC-ES) M+H = 359.

**Benzyl (*trans*-4-((1,1,1-trifluoro-2-methylpropan-2-yl)amino)cyclohexyl)carbamate:** $^1$H NMR (400 MHz, $CD_3SOCD_3$) δ 1.04-1.20 (m, 4 H), 1.17 (s, 6 H), 1.66-1.78 (m, 4 H), 2.46-2.58 (m, 1 H), 3.10-3.24 (m, 1 H), 4.97 (s, 2 H), 7.15 (d, *J* = 8 Hz, 1 H), 7.26-7.38 (m, 5 H); LC-MS (LC-ES) M+H = 359.

**B. *trans*-N1-(1,1,1-Trifluoro-2-methylpropan-2-yl)cyclohexane-1,4-diamine**

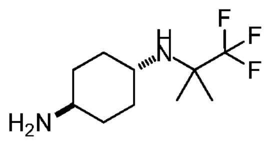

Palladium on carbon (5.61 mg, 0.053 mmol) was added to benzyl (*trans*-4-((1,1,1-trifluoro-2-methylpropan-2-yl)amino)cyclohexyl)carbamate (0.189 g, 0.527 mmol) in MeOH (2.64 mL) at 25 °C under nitrogen atmosphere. Then, the reaction vessel was fitted with an hydrogen balloon and the vessel was repeatedly evacuated and purged with hydrogen, then stirred for 16 h. The vessel was repeatedly evacuated and purged with nitrogen, filtered through Celite®, and concentrated to give the title compound (0.1017 g, 68.8 % yield). $^1$H NMR (400 MHz, $CD_3SOCD_3$) δ 0.96-1.08 (m, 2 H), 1.16 (s, 6 H), 1.24-1.40 (m, 2 H), 1.60-1.74 (m, 4 H), 2.34-2.46 (m, 1 H), 2.48-2.58 (m, 1 H).

**Intermediate 61: *trans*-N1-(1,1-Difluoropropan-2-yl)cyclohexane-1,4-diamine**

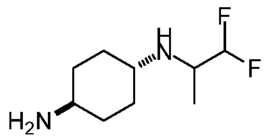

**A. Benzyl (*trans*-4-((1,1-difluoropropan-2-yl)amino)cyclohexyl)carbamate**

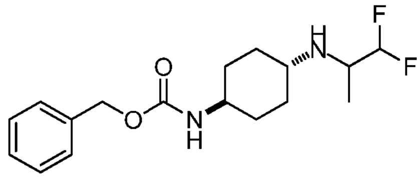

1,1-Difluoropropan-2-one (3.13 g, 33.3 mmol) was added to benzyl (*trans*-4-aminocyclohexyl)carbamate (7.52 g, 30.3 mmol) in 1,2-dichloroethane (151 mL) at RT and stirred for 5 min, followed by acetic acid (0.091 g, 1.514 mmol) and 4A molecular sieves (20.0 g) and the reaction was stirred for 2 h at RT. Then, sodium triacetoxyborohydride (6.42 g, 30.3 mmol) was added, and the reaction mixture was stirred for 20 h. The reaction mixture was filtered through Celite®, saturated sodium bicarbonate added, extracted with DCM, dried over magnesium sulfate, filtered, and concentrated. The residue was purified by silica gel chromatography, eluting with EtOAc:hexanes (1:1) to give the title compound (8.41 g, 81 % yield). $^1H$ NMR (400 MHz, $CD_3SOCD_3$) δ 0.99 (d, *J* = 7 Hz, 3 H), 0.94-1.06 (m, 2 H), 1.08-1.22 (m, 2 H), 1.45 (br s, 1 H), 1.70-1.88 (m, 4 H), 2.36-2.48 (m, 1 H), 2.86-3.00 (m, 1 H), 3.14-3.28 (m, 1H), 4.97 (s, 2 H), 5.74 (dt, *J* = 56, 4 Hz, 1 H), 7.16 (d, *J* = 8 Hz, 1 H), 7.26-7.38 (m, 5 H); LC-MS (LC-ES) M+H = 327.

**B. t*rans*-N1-(1,1-Difluoropropan-2-yl)cyclohexane-1,4-diamine**

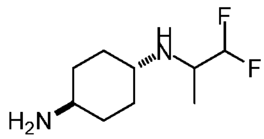

Palladium on carbon (0.137 g, 1.288 mmol) was added to benzyl (*trans*-4-((1,1-difluoropropan-2-yl)amino)cyclohexyl)carbamate (8.41 g, 25.8 mmol) in MeOH (51.5 mL) at 25 °C under nitrogen atmosphere. Then, the reaction vessel was fitted with a hydrogen balloon and the vessel was repeatedly evacuated and purged with hydrogen, then stirred for 6 h. Then, the vessel was repeatedly evacuated and purged with nitrogen, filtered through Celite®, and concentrated to give the title compound (5.05 g, 97 % yield). $^1H$ NMR (400 MHz, $CD_3SOCD_3$) δ 0.90-1.04 (m, 4 H), 0.99 (d, *J* = 7 Hz, 3 H), 1.30-1.60 (m, 3 H), 1.62-1.84 (m, 4 H), 2.34-2.48 (m, 2 H), 2.86-3.00 (m, 1 H), 5.73 (dt, *J* = 56, 4 Hz, 1 H); LC-MS (LC-ES) M+H = 193.

**Intermediate 62: *trans*-4-(3,3-Difluoroazetidin-1-yl)cyclohexanamine**

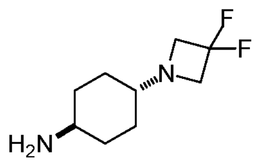

**A. Benzyl (*cis*-4-(3,3-difluoroazetidin-1-yl)cyclohexyl)carbamate and Benzyl (*trans*-4-(3,3-difluoroazetidin-1-yl)cyclohexyl)carbamate**

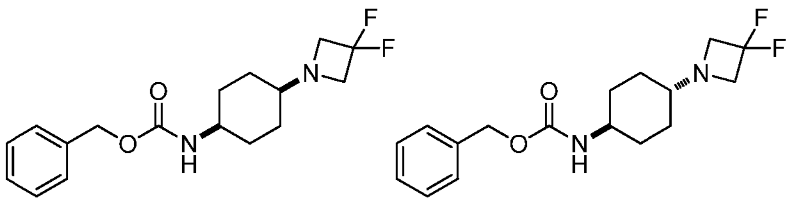

3,3-Difluoroazetidine hydrochloride (0.593 g, 4.58 mmol) was added to benzyl (4-oxocyclohexyl)carbamate (1.03 g, 4.17 mmol) in 1,2-dichloroethane (20.83 mL) at RT and stirred for 5 minutes, followed by acetic acid (0.013 g, 0.208 mmol) and 4A molecular sieves (4.0 g) and the reaction was stirred for 2 h at RT. Then, sodium triacetoxyborohydride (0.883 g, 4.17 mmol) was added, and the reaction mixture was stirred for 16 h. The reaction mixture was filtered through Celite®, saturated sodium bicarbonate added, extracted with DCM, dried over magnesium sulfate, filtered, and concentrated. The residue was purified by silica gel chromatography, eluting with EtOAc:hexanes (2:3) to give benzyl (*cis*-4-(3,3-difluoroazetidin-1-yl)cyclohexyl)carbamate (0.3787 g, 26.6 % yield) and benzyl (*trans*-4-(3,3-difluoroazetidin-1-yl)cyclohexyl)carbamate (0.5901 g, 41.5 % yield).

**Benzyl (cis-4-(3,3-difluoroazetidin-1-yl)cyclohexyl)carbamate:** $^1H$ NMR (400 MHz, $CD_3SOCD_3$) $\delta$ 1.32-1.58 (m, 8 H), 2.24-2.30 (m, 1 H), 3.28-3.40 (m, 1 H), 3.46 (t, *J* = 12 Hz, 4 H), 4.98 (s, 2 H), 7.20 (d, *J* = 8 Hz, 1 H), 7.26-7.38 (m, 5 H); LC-MS (LC-ES) M+H = 325.

**Benzyl (*trans*-4-(3,3-difluoroazetidin-1-yl)cyclohexyl)carbamate:** $^1H$ NMR (400 MHz, $CD_3SOCD_3$) $\delta$ 0.97 (q, *J* = 13 Hz, 2 H), 1.14 (dq, *J* = 13, 3 Hz, 2 H), 1.68 (br d, *J* = 12 Hz, 2 H), 1.76 (br d, *J* = 12 Hz, 2 H), 2.03 (t, *J* = 10 Hz, 1 H), 3.16-3.30 (m, 1 H), 3.96 (t, *J* = 12 Hz, 4 H), 4.98 (s, 2 H), 7.18 (d, *J* = 8 Hz, 1 H), 7.26-7.38 (m, 5 H); LC-MS (LC-ES) M+H = 325.

**B. *trans*-4-(3,3-Difluoroazetidin-1-yl)cyclohexanamine**

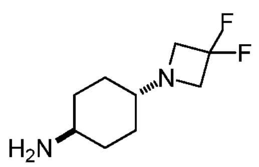

Palladium on carbon (0.019 g, 0.182 mmol) was added to benzyl (*trans*-4-(3,3-difluoroazetidin-1-yl)cyclohexyl)carbamate (0.5901 g, 1.819 mmol) in MeOH (9.10 mL) at 25 °C under nitrogen atmosphere. Then, the reaction vessel was fitted with a hydrogen balloon and the vessel was repeatedly evacuated and purged with hydrogen, then stirred for 3 h. Then, the vessel was repeatedly evacuated and purged with nitrogen, filtered through Celite®, and concentrated to give the title compound (0.3403 g, 93 % yield). $^1H$ NMR (400 MHz, $CD_3SOCD_3$) $\delta$ 0.88-1.02 (m, 4 H), 1.54 (br s, 2 H), 1.68-1.74 (m, 4 H), 1.96-2.06 (m, 1 H), 2.40-2.52 (m, 1 H), 3.48 (t, *J* = 12 Hz, 4 H); LC-MS (LC-ES) M+H = 191.

**Intermediate 63: *trans*-N1-(1,1-Difluoropropan-2-yl)cyclobutane-1,3-diamine dihydrochloride**

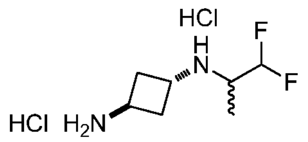

**A. *tert*-Butyl (*trans*-3-((1,1-difluoropropan-2-yl)amino)cyclobutyl)carbamate**

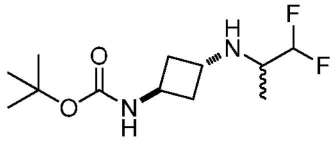

1,1-Difluoropropan-2-one (0.279 g, 2.96 mmol) was added to *tert*-butyl (*trans*-3-aminocyclobutyl)carbamate (0.5014 g, 2.69 mmol) in 1,2-dichloroethane (13.46 mL) at RT and stirred for 5 min, followed by acetic acid (8.08 mg, 0.135 mmol) and 4A molecular sieves (4.0 g) and the reaction was stirred for 2 h at RT. Then, sodium triacetoxyborohydride (0.571 g, 2.69 mmol) was added, and the reaction mixture was stirred for 20 h. The reaction mixture was filtered through Celite®, saturated sodium bicarbonate added, extracted with DCM, dried over magnesium sulfate, filtered, and concentrated. The residue was purified by silica gel chromatography, eluting with EtOAc:hexanes (1:1) to give the title compound (0.6269 g, 84 % yield). $^1$H NMR (400 MHz, $CD_3SOCD_3$) δ 0.98 (d, *J* = 7 Hz, 3 H), 1.35 (s, 9 H), 1.86-2.04 (m, 5 H), 2.66-2.82 (m, 1 H), 3.30-3.40 (m, 1 H), 3.86-3.98 (m, 1 H), 5.75 (dt, *J* = 57, 4 Hz, 1 H), 7.10 (d, *J* = 7 Hz, 1 H); LC-MS (LC-ES) M+H = 265.

**B. *trans*-N1-(1,1-Difluoropropan-2-yl)cyclobutane-1,3-diamine dihydrochloride**

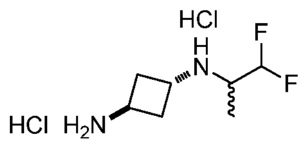

4.0 M Hydrochloric acid (5.93 mL, 23.72 mmol) in dioxane was added to *tert*-butyl (*trans*-3-((1,1-difluoropropan-2-yl)amino)cyclobutyl)carbamate (0.6269 g, 2.372 mmol) in MeOH (5.93 mL) at RT and the reaction mixture was stirred for 16 h. The reaction mixture was concentrated to give the title compound (0.5510 g, 93 % yield). $^1$H NMR (400 MHz, $CD_3SOCD_3$) δ 1.26 (d, *J* = 7 Hz, 3 H), 2.36-2.50 (m, 2 H), 2.54-2.76 (m, 2 H), 3.72 (m, 1 H), 3.88 (br s, 1 H), 4.10 (br s, 1 H), 6.39 (t, *J* = 54 Hz, 1 H), 8.30 (br s, 3 H), 10.02 (br s, 1 H), 10.11 (br s, 1 H); LC-MS (LC-ES) M+H = 165.

**Intermediate 64: *trans*-4-(((1,1-Difluoropropan-2-yl)amino)methyl)cyclohexanamine**

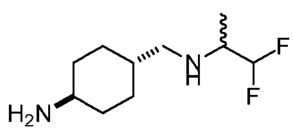

**A. Benzyl (*trans*-4-(((1,1-difluoropropan-2-yl)amino)methyl)cyclohexyl)carbamate**

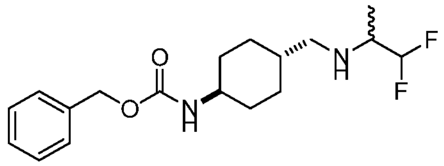

1,1-Difluoropropan-2-one (0.394 g, 4.19 mmol) was added to benzyl (*trans*-4-(aminomethyl)cyclohexyl)carbamate (1 g, 3.81 mmol) in 1,2-dichloroethane (19.06 mL) (N-methylpyrrolidinone was mistakenly also added) at RT and stirred for 5 min, followed by acetic acid (0.011 g, 0.191 mmol) and 4A molecular sieves (4.0 g) and the reaction mixture was stirred for 2 h at RT. Then, sodium triacetoxyborohydride (0.808 g, 3.81 mmol) was added, and the reaction mixture was stirred for 2 days. The reaction mixture was filtered through Celite®, saturated sodium bicarbonate added, extracted with EtOAc, and then washed with water and brine, dried over magnesium sulfate, filtered, and concentrated. The residue (containing N-methylpyrrolidinone) was purified by silica gel chromatography with EtOAc:hexanes (10:90-50:50) to give the title compound (510 mg, 39.3 % yield) as a white solid. The fractions that had the lower spot by TLC were also collected as benzyl (*tran*s-4-(((1,1-difluoropropan-2-yl)amino)methyl)cyclohexyl)carbamate (302 mg, 0.887 mmol, 23.3 % yield). LC-MS (LC-ES) M+H = 341.

**B. *trans*-4-(((1,1-Difluoropropan-2-yl)amino)methyl)cyclohexanamine**

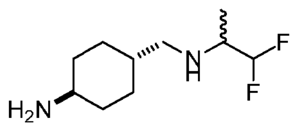

10% Palladium on carbon (237 mg, 0.223 mmol) was added to benzyl (*trans*-4-(((1,1-difluoropropan-2-yl)amino)methyl)cyclohexyl)carbamate (505 mg, 1.484 mmol) in MeOH (10 mL) and the mixture was degassed and then was stirred under a hydrogen balloon overnight at which time the mixture was filtered through Celite® and the resulting filtrate was concentrated under vacuum to afford the title compound (296 mg, 97 % yield) as an oil. $^1$H NMR (400 MHz, $CD_3OD$) δ 0.86-0.98 (m, 2 H), 1.06-1.20 (m, 5 H), 1.30-1.44 (m, 1 H), 1.76-1.92 (m, 4 H), 2.40-2.50 (m, 2 H), 2.52-2.64 (m, 1 H), 2.76-2.92 (m, 1 H), 5.66 (dt, 1 H); LC-MS (LC-ES) M+H = 207.

**Intermediate 65: (R)-2-((*trans*-4-Aminocyclohexyl)amino)-3,3,3-trifluoropropan-1-ol**

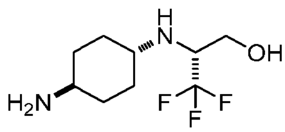

A. (R)-Benzyl **(3-(trifluoromethyl)-1-oxa-4-azaspiro[4.5]decan-8-yl)carbamate**

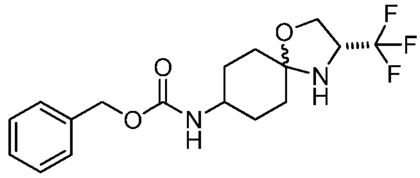

(R)-2-Amino-3,3,3-trifluoropropan-1-ol hydrochloride (0.676 g, 4.08 mmol) was added to benzyl (4-oxocyclohexyl)carbamate (1.01 g, 4.08 mmol) in benzene (40.8 mL) at RT and the reaction was heated with a Dean-Stark trap for 16 h. Then, the reaction mixture was cooled, saturated sodium bicarbonate added, extracted with diethyl ether,

dried over magnesium sulfate, filtered, and concentrated. The residue was purified by silica gel chromatography, eluting with EtOAc:hexanes (2:3) to give the title compound (1.21 g, 70.3 % yield) contaminated with -10% of starting ketone. $^1$H NMR (400 MHz, $CD_3SOCD_3$) δ 1.32-1.80 (m, 8 H), 3.62-4.04 (m, 4H), 4.98 & 4.99 (s, 2 H), 7.20 & 7.24 (d, *J* = 8 Hz, 1 H), 7.34-7.40 (m, 5 H); LC-MS (LC-ES) M+H = 359.

**B. Benzyl (*cis*-4-(((R)-1,1,1-trifluoro-3-hydroxypropan-2-yl)amino)cyclohexyl)carbamate and Benzyl (*trans*-4-(((R)-1,1,1-trifluoro-3-hydroxypropan-2-yl)amino)cyclohexyl)carbamate**

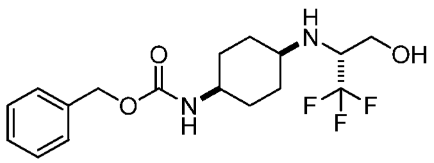

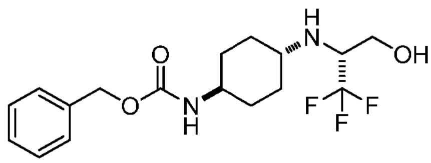

Sodium triacetoxyborohydride (0.481 g, 2.269 mmol) was added to benzyl ((3R)-3-(trifluoromethyl)-1-oxa-4-azaspiro[4.5]decan-8-yl)carbamate (0.8130 g, 2.269 mmol) in 1,2-dichloroethane (11.34 mL) at RT, followed by acetic acid (6.81 mg, 0.113 mmol) and the reaction was stirred for 64 h. The reaction mixture was diluted with saturated sodium bicarbonate, extracted with DCM, dried over magnesium sulfate, filtered, and concentrated. The residue was purified by silica gel chromatography, eluting with EtOAc:hexanes (1:1) to give benzyl (*cis*-4-(((R)-1,1,1-trifluoro-3-hydroxypropan-2-yl)amino)cyclohexyl)carbamate (0.3625 g, 26.6 % yield) and benzyl (*trans*-4-(((R)-1,1,1-trifluoro-3-hydroxypropan-2-yl)amino)cyclohexyl)carbamate (0.3917 g, 45.5 % yield).

**Benzyl (*cis*-4-(((R)-1,1,1-trifluoro-3-hydroxypropan-2-yl)amino)cyclohexyl)carbamate:** $^1$H NMR (400 MHz, $CD_3SOCD_3$) δ 1.40-1.70 (m, 8 H), 2.34-2.46 (m, 1 H), 3.12-3.26 (m, 1 H), 3.34-3.44 (m, 1 H), 3.44-3.52 (m, 1 H), 3.58-3.66 (m, 1 H), 4.97 (t, *J* = 6 Hz, 1 H), 4.99 (s, 2 H), 7.20 (d, *J* = 7 Hz, 1 H), 7.26-7.38 (m, 5 H); LC-MS (LC-ES) M+H = 361.

**Benzyl (*trans*-4-(((R)-1,1,1-trifluoro-3-hydroxypropan-2-yl)amino)cyclohexyl)carbamate:** $^1$H NMR (400 MHz, $CD_3SOCD_3$) δ 1.02 (q, *J* = 13 Hz, 2 H), 1.14 (q, *J* = 13 Hz, 2 H), 1.72-1.92 (m, 5 H), 2.36-2.48 (m, 1 H), 3.14-3.28 (m, 2 H), 3.40-3.50 (m, 1 H), 3.54-3.64 (m, 1 H), 4.96 (t, *J* = 6 Hz, 1 H), 4.98 (s, 2 H), 7.15 (d, *J* = 8 Hz, 1 H), 7.26-7.38 (m, 5 H); LC-MS (LC-ES) M+H = 361.

**C. (R)-2-((*trans*-4-Aminocyclohexyl)amino)-3,3,3-trifluoropropan-1-ol**

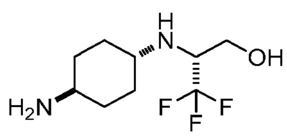

Palladium on carbon (0.012 g, 0.109 mmol) was added to benzyl (*trans*-4-(((R)-1,1,1-trifluoro-3-hydroxypropan-2-yl)amino)cyclohexyl)carbamate (0.3917 g, 1.087 mmol) in MeOH (5.43 mL) at 25 °C under nitrogen atmosphere. Then, the reaction vessel was fitted with a hydrogen balloon and the vessel was repeatedly evacuated and purged with hydrogen, then stirred for 16 h. Then, the vessel was repeatedly evacuated and purged with nitrogen, filtered through Celite®, and concentrated to give the title compound (0.2481 g, 96 % yield). $^1$H NMR (400 MHz, $CD_3SOCD_3$) δ 0.92-1.06 (m, 4 H), 1.62-1.88 (m, 7 H), 2.36-2.50 (m, 2 H), 3.16-3.28 (m, 1 H), 3.40-3.50 (m, 1 H), 3.54-3.64 (m, 1 H), 4.96 (t, *J* = 6 Hz, 1 H); LC-MS (LC-ES) M+H = 227.

<u>**Intermediate 66: 1-((trans-4-Aminocyclohexyl)oxy)-3-methylbutan-2-ol**</u>

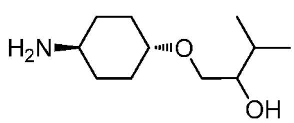

**A. 1-((trans-4-(Dibenzylamino)cyclohexyl)oxy)-3-methylbutan-2-one**

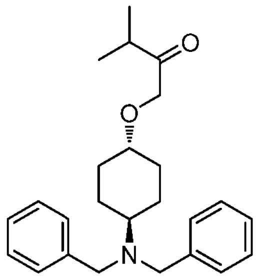

2-((trans-4-(Dibenzylamino)cyclohexyl)oxy)-N-methoxy-N-methylacetamide (Intermediate 47, step B) (1.2 g, 3.03 mmol) was stirred in THF (10 mL) at 0 °C under nitrogen, then a 1.3 M solution in THF of isopropylmagnesium chloride (3.5 mL, 4.55 mmol) was slowly added. The reaction was allowed to stir at 0 °C for ca. 10 min before warming to RT for 1 h. The reaction was re-cooled to 0°C and quenched with saturated aqueous $NH_4Cl$. The organics were extracted with EtOAc and washed with water then brine. The organics were dried over sodium sulfate, filtered, and concentrated under reduced pressure to afford the title compound as a yellow oil (1.18 g, 3.11 mmol). $^1H$ NMR ($CD_3OD$) δ 1.03 - 1.19 (m, 8 H) 1.44 (m, 2 H) 1.90 (d, *J* = 13 Hz, 2 H) 2.09 (d, *J* = 12 Hz, 2 H) 2.50 (tt, *J* = 12, 3 Hz, 1 H) 2.75 (m, 1 H) 3.23 (tt, *J* = 11, 4 Hz, 1 H) 3.59 (s, 4 H) 4.22 (s, 2 H) 7.18 (m, 2 H) 7.26 (t, *J* = 7 Hz, 4 H) 7.31 - 7.37 (m, 4 H). LCMS: ES+ve *m/z* 381 $[M+H]^+$.

**B. 1-((trans-4-(Dibenzylamino)cyclohexyl)oxy)-3-methylbutan-2-ol**

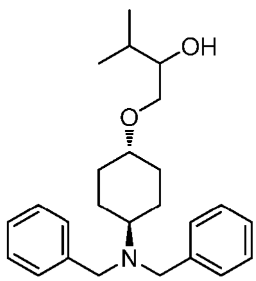

1-((trans-4-(Dibenzylamino)cyclohexyl)oxy)-3-methylbutan-2-one (0.558 g, 1.470 mmol) was stirred in THF (3.5 mL) at 0 °C under nitrogen, then a 1.0 M solution LAH in THF (1.5 mL, 1.5 mmol) was slowly added. The reaction was allowed to stir for 1 h before quenching with water (0.05 mL), then 15% aq. NaOH (0.05 mL) followed by water (0.15 mL). After quenching, the reaction was diluted with EtOAc and washed with water, followed by brine. The organics were dried over sodium sulfate, filtered, and concentrated under reduced pressure to afford the title compound (540 mg, 1.42 mmol). $^1H$ NMR ($CDCl_3$) δ 0.90 (d, *J* = 7 Hz, 3 H) 0.96 (d, *J* = 7 Hz, 3 H) 1.17 (m, 2 H) 1.39 (m, 2 H) 1.69 (dq, *J* = 13, 7 Hz, 1 H) 1.92 (d, *J* = 13 Hz, 2 H) 2.07 (m, 2 H) 2.54 (m, 1 H) 3.19 (tt, *J* = 11, 4 Hz, 1 H) 3.30 (m, 1 H) 3.43 (ddt, *J* = 8, 6, 3 Hz, 1 H) 3.53 (dd, *J* = 9, 3 Hz, 1 H) 3.62 (s, 4 H) 7.21 (m, 2 H) 7.30 (m, 4 H) 7.36 (m, 4 H). LCMS: ES+ve *m/z* 382 $[M+H]^+$.

**C. 1-((trans-4-Aminocyclohexyl)oxy)-3-methylbutan-2-ol**

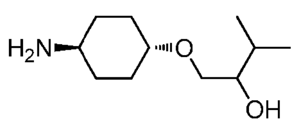

1-((trans-4-(Dibenzylamino)cyclohexyl)oxy)-3-methylbutan-2-ol (0.54 g, 1.415 mmol) and 20 wt% Pearlman's catalyst (0.199 g, 0.283 mmol) were stirred in EtOH (15 mL) under an atmosphere of $H_2$ (balloon) for 2 h. The reaction

was filtered through Celite® and the pad was rinsed with EtOAc. The filtrate was concentrated under reduced pressure to afford the title compound as a colorless oil. $^1$H NMR ($CD_3OD$) δ 0.93 (d, *J* = 7 Hz, 6 H), 1.11 - 1.34 (m, 4 H), 1.68 - 1.80 (m, 1 H), 1.90 (m, 2 H), 2.05 (m, 2 H), 2.64 (tt, *J* = 11, 4 Hz, 1 H), 3.25 (m, 1 H), 3.41 (m, 2 H), 3.52 (m, 1 H).

**Intermediate 67: (S)-2-((*trans*-4-aminocyclohexyl)amino)-3,3,3-trifluoropropan-1-ol**

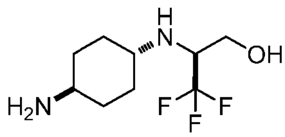

**A. (S)-Benzyl (3-(trifluoromethyl)-1-oxa-4-azaspiro[4.5]decan-8-yl)carbamate**

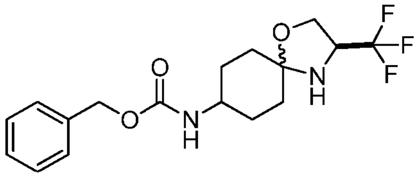

(S)-2-Amino-3,3,3-trifluoropropan-1-ol hydrochloride (0.676 g, 4.08 mmol) was added to benzyl (4-oxocyclohexyl)carbamate (1.01 g, 4.08 mmol) in benzene (40.8 mL) at RT and the reaction was heated with a Dean-Stark trap for 16 h. Then, the reaction mixture was cooled, saturated sodium bicarbonate added, extracted with diethyl ether, dried over magnesium sulfate, filtered, and concentrated to give the title compound (1.52 g, 99 % yield). $^1$H NMR (400 MHz, $CD_3SOCD_3$) δ 1.32-1.80 (m, 8 H), 3.62-4.04 (m, 4 H), 4.98 & 4.99 (s, 2 H), 7.20 & 7.24 (d, *J* = 8 Hz, 1 H), 7.26-7.40 (m, 5 H); LC-MS (LC-ES) M+H = 359.

**B. Benzyl (*cis*-4-(((S)-1,1,1-trif)uoro-3-hydroxypropan-2-yl)amino)cyclohexyl)carbamate and Benzyl (*trans*-4-(((S)-1,1,1-trifluoro-3-hydroxypropan-2-yl)amino)cyclohexyl)carbamate**

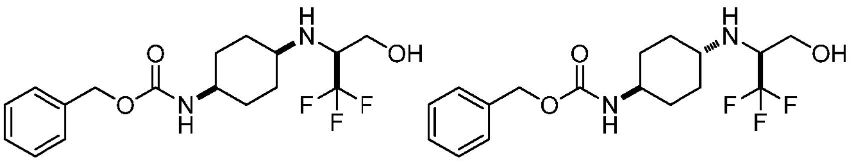

Sodium triacetoxyborohydride (0.899 g, 4.24 mmol) was added to benzyl ((3S)-3-(trifluoromethyl)-1-oxa-4-azaspiro[4.5]decan-8-yl)carbamate (1.52 g, 4.24 mmol) in 1,2-dichloroethane (21.21 mL) at RT, followed by acetic acid (0.013 g, 0.212 mmol) and the reaction was stirred for 4 h. The reaction mixture was diluted with saturated sodium bicarbonate, extracted with DCM, dried over magnesium sulfate, filtered, and concentrated. The residue was purified by silica gel chromatography, eluting with EtOAc:hexanes (1:1) to give benzyl (*cis*-4-(((S)-1,1,1-trifluoro-3-hydroxypropan-2-yl)amino)cyclohexyl)carbamate (0.5875 g, 36.5 % yield) and benzyl (trans-4-(((S)-1,1,1-trifluoro-3-hydroxypropan-2-yl)amino)cyclohexyl)carbamate (0.7712 g, 47.9 % yield).

**Benzyl (*cis*-4-(((S)-1,1,1-trifluoro-3-hydroxypropan-2-yl)amino)cyclohexyl)carbamate:** $^1$H NMR (400 MHz, $CD_3SOCD_3$) δ 1.38-1.70 (m, 8 H), 1.72-1.78 (m, 1 H), 2.66-2.74 (m, 1 H), 3.12-3.24 (m, 1 H), 3.34-3.44 (m, 1 H), 3.44-3.52 (m, 1 H), 3.56-3.66 (m, 1 H), 4.97 (t, *J* = 6 Hz, 1 H), 4.99 (s, 2 H), 7.20 (d, *J* = 7 Hz, 1 H), 7.24-7.38 (m, 5 H); LC-MS (LC-ES) M+H = 361.

**Benzyl (*trans*-4-(((S)-1,1,1-trifluoro-3-hydroxypropan-2-yl)amino)cyclohexyl)carbamate:** $^1$H NMR (400 MHz, $CD_3SOCD_3$) δ 1.02 (q, *J* = 13 Hz, 2 H), 1.14 (q, *J* = 13 Hz, 2 H), 1.72-1.92 (m, 5 H), 2.36-2.48 (m, 1 H), 3.14-3.28 (m, 2 H), 3.40-3.50 (m, 1 H), 3.54-3.64 (m, 1 H), 4.96 (t, *J* = 6 Hz, 1 H), 4.98 (s, 2 H), 7.15 (d, *J* = 8 Hz, 1 H), 7.26-7.38

(m, 5 H); LC-MS (LC-ES) M+H = 361.

**C. (S)-2-((*trans*-4-aminocyclohexyl)amino)-3,3,3-trifluoropropan-1-ol**

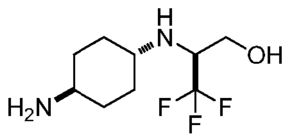

Palladium on carbon (0.023 g, 0.214 mmol) was added to benzyl (*trans*-4-(((S)-*1,1,1*-trifluoro-3-hydroxypropan-2-yl)amino)cyclohexyl)carbamate (0.7712 g, 2.140 mmol) in MeOH (7.13 mL) at 25 °C under nitrogen atmosphere. Then, the reaction vessel was fitted with a hydrogen balloon and the vessel was repeatedly evacuated and purged with hydrogen, then stirred for 16 h. Then, the vessel was repeatedly evacuated and purged with nitrogen, filtered through Celite®, and concentrated to give the title compound (0.5036 g, 99 % yield). $^1$H NMR (400 MHz, $CD_3SOCD_3$) δ 0.92-1.06 (m, 4 H), 1.46-1.88 (m, 7 H), 2.36-2.50 (m, 2 H), 3.16-3.28 (m, 1H), 3.40-3.50 (m, 1 H), 3.54-3.64 (m, 1 H), 4.97 (br s, 1 H); LC-MS (LC-ES) M+H = 227.

**Intermediate 68: *cis*-4-(3-Fluoroazetidin-1-yl)cyclohexanamine**

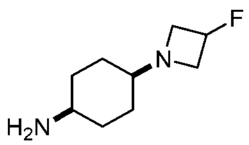

**A. Benzyl (*cis*-4-(3-fluoroazetidin-1-yl)cyclohexyl)carbamate and Benzyl (*trans*-4-(3-fluoroazetidin-1-yl)cyclohexyl)carbamate**

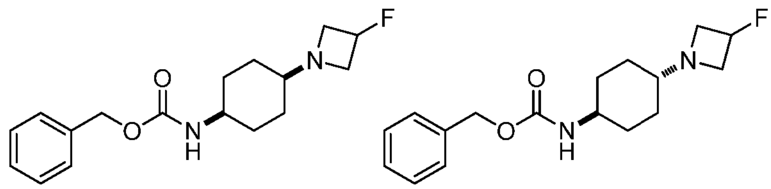

3-Fluoroazetidine hydrochloride (0.506 g, 4.54 mmol) was added to benzyl (4-oxocyclohexyl)carbamate (1.02 g, 4.12 mmol) in 1,2-dichloroethane (20.62 mL) at RT and stirred for 5 min, followed by acetic acid (0.012 g, 0.206 mmol) and 4A molecular sieves (4.0 g) and the reaction was stirred for 2 h at RT. Then, sodium triacetoxyborohydride (0.874 g, 4.12 mmol) was added, and the reaction mixture was stirred for 66 h. The reaction mixture was filtered through Celite®, saturated sodium bicarbonate added, extracted with DCM, dried over magnesium sulfate, filtered, and concentrated. The residue was purified by silica gel chromatography, eluting with MeOH:EtOAc (0:1 to 1:9) to give benzyl (*cis*-4-(3-fluoroazetidin-1-yl)cyclohexyl)carbamate (0.5051 g, 30.0 % yield) and benzyl (*trans*-4-(3-fluoroazetidin-1-yl)cyclohexyl)carbamate (0.6475 g, 46.1 % yield).

**Benzyl (*cis*-4-(3-fluoroazetidin-1-yl)cyclohexyl)carbamate:** $^1$H NMR (400 MHz, $CD_3SOCD_3$) δ 1.28-1.54 (m, 8 H), 2.12-2.20 (m, 1 H), 2.88-3.00 (m, 2 H), 3.26-3.38 (m, 1 H), 3.42-3.52 (m, 2 H), 4.97 (s, 2 H), 5.09 (dp, *J* = 58, 5 Hz, 1 H), 7.15 (d, *J* = 8 Hz, 1 H), 7.26-7.38 (m, 5 H); LC-MS (LC-ES) M+H = 307.

**Benzyl (*trans*-4-(3-fluoroazetidin-1-yl)cyclohexyl)carbamate:** $^1$H NMR (400 MHz, $CD_3SOCD_3$) δ 0.91 (q, *J* = 13 Hz, 2 H), 1.13 (q, *J* = 13 Hz, 2 H), 1.68 (br d, *J* = 12 Hz, 2 H), 1.75 (br d, *J* = 12 Hz, 2 H), 1.92 (tt, *J* = 11, 3 Hz, 1 H), 2.92-3.04 (m, 2 H), 3.14-3.26 (m, 1 H), 3.42-3.52 (m, 2 H), 4.98 (s, 2 H), 5.07 (dp, *J* = 58, 5 Hz, 1 H), 7.14 (d, *J* = 8 Hz, 1 H), 7.26-7.38 (m, 5 H); LC-MS (LC-ES) M+H = 307.

**B. *cis*-4-(3-Fluoroazetidin-1-yl)cyclohexanamine**

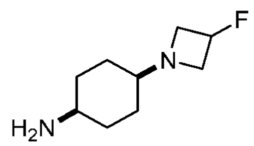

Palladium on carbon (0.018 g, 0.165 mmol) was added to benzyl (*cis*-4-(3-fluoroazetidin-1-yl)cyclohexyl)carbamate (0.5051 g, 1.649 mmol) in MeOH (5.50 mL) at 25 °C under nitrogen atmosphere. Then, the reaction vessel was fitted with a hydrogen balloon and the vessel was repeatedly evacuated and purged with hydrogen, then stirred for 16 h. Then, the vessel was repeatedly evacuated and purged with nitrogen, filtered through Celite®, and concentrated to give the title compound (0.2627 g, 79 % yield). $^1H$ NMR (400 MHz, $CD_3SOCD_3$) δ 1.24-1.48 (m, 8 H), 2.10-2.16 (m, 1 H), 2.21 (br s, 2 H), 2.58-2.68 (m, 1 H), 2.86-2.98 (m, 2 H), 3.44-3.54 (m, 2 H), 5.09 (dp, *J* = 58, 5 Hz, 1 H); LC-MS (LC-ES) M+H = 173.

**Intermediate 69: *trans*-4-(3-Fluoroazetidin-1-yl)cyclohexanamine**

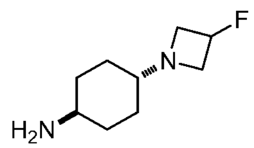

Palladium on carbon (0.022 g, 0.211 mmol) was added to benzyl (*trans*-4-(3-fluoroazetidin-1-yl)cyclohexyl)carbamate (0.6475 g, 2.113 mmol, Intermediate 68, step A) in MeOH (7.04 mL) at 25 °C under nitrogen atmosphere. Then, the reaction vessel was fitted with a hydrogen balloon and the vessel was repeatedly evacuated and purged with hydrogen, then stirred for 17 h. Then, the vessel was repeatedly evacuated and purged with nitrogen, filtered through Celite®, and concentrated to give the title compound (0.3991 g, 99 % yield). $^1H$ NMR (400 MHz, $CD_3SOCD_3$) δ 0.82-1.04 (m, 4 H), 1.58-1.74 (m, 6 H), 1.84-1.94 (m, 1 H), 2.38-2.50 (m, 1 H), 2.90-3.02 (m, 2 H), 3.42-3.52 (m, 2 H), 5.06 (dp, *J* = 58, 5 Hz, 1 H); LC-MS (LC-ES) M+H = 173.

**Intermediate 70: 1-(*cis*-4-Aminocyclohexyl)-3-(trifluoromethyl)azetidin-3-ol**

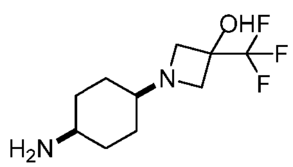

**A. Benzyl (*cis*-4-(3-hydroxy-3-(trifluoromethyl)azetidin-1-yl)cyclohexyl)carbamate and Benzyl (*trans*-4-(3-hydroxy-3-(trifluoromethyl)azetidin-1-yl)cyclohexyl)carbamate**

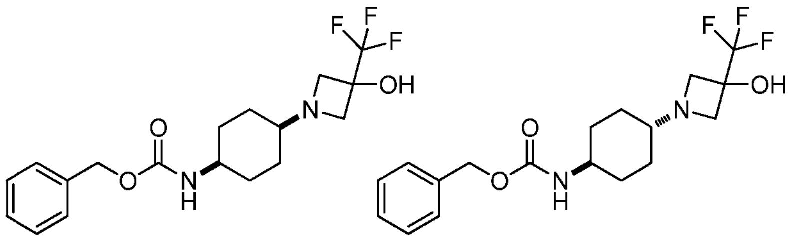

3-(Trifluoromethyl)azetidin-3-ol hydrochloride (0.806 g, 4.54 mmol) was added to benzyl (4-oxocyclohexyl)carbamate (1.02 g, 4.12 mmol) in 1,2-dichloroethane (20.62 mL) at RT and stirred for 5 min, followed by acetic acid (0.012 g, 0.206 mmol) and 4A molecular sieves (4.0 g) and the reaction was stirred for 2 h at RT. Then, sodium triacetoxyborohydride (0.874 g, 4.12 mmol) was added, and the reaction mixture was stirred for 66 h. The reaction mixture was filtered through Celite®, saturated sodium bicarbonate added, extracted with DCM, dried over magnesium sulfate, filtered, and concentrated. The residue was purified by silica gel chromatography, eluting with EtOAc:hexanes (3:2 to 1:0) to give benzyl (*cis*-4-(3-hydroxy-3-(trifluoromethyl)azetidin-1-yl)cyclohexyl)carbamate (0.6099 g, 37.7 % yield) and benzyl (*trans*-4-(3-hydroxy-3-(trifluoromethyl)azetidin-1-yl)cyclohexyl)carbamate (0.6234 g, 38.6 % yield). **Benzyl (*cis*-4-(3-hydroxy-3-(trifluoromethyl)azetidin-1-yl)cyclohexyl)carbamate:** $^1H$ NMR (400 MHz, $CD_3SOCD_3$) δ 1.28-1.58 (m, 8 H), 2.14-2.22 (m, 1 H), 3.03 (d, *J* = 9 Hz, 2 H), 3.26-3.40 (m, 1 H), 3.41 (d, *J* = 9 Hz, 2 H), 4.98 (s, 2 H), 6.77 (s, 1 H), 7.19 (d, *J* = 7 Hz, 1 H), 7.26-7.38 (m, 5 H); LC-MS (LC-ES) M+H = 373.

**Benzyl (*trans*-4-(3-hydroxy-3-(trifluoromethyl)azetidin-1-yl)cyclohexyl)carbamate:** $^1H$ NMR (400 MHz, $CD_3SOCD_3$) δ 0.91 (dq, *J* = 13, 3 Hz, 2 H), 1.13 (dq, *J* = 13, 3 Hz, 2 H), 1.68 (br d, *J* = 12 Hz, 2 H), 1.75 (dd, *J* = 13, 3 Hz, 2 H), 1.94 (tt, *J* = 11, 3 Hz, 1 H), 3.06 (d, *J* = 9 Hz, 2 H), 3.21 (qt, *J* = 8, 4 Hz, 1 H), 3.43 (d, *J* = 9 Hz, 2 H), 4.98 (s, 2 H), 6.78 (s, 1 H), 7.15 (d, *J* = 8 Hz, 1 H), 7.26-7.38 (m, 5 H); LC-MS (LC-ES) M+H = 373.

**B.1-(*cis*-4-Aminocyclohexyl)-3-(trifluoromethyl)azetidin-3-ol**

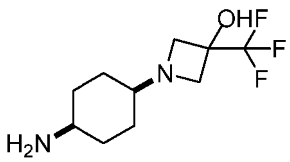

Palladium on carbon (0.017 g, 0.164 mmol) was added to benzyl (*cis*-4-(3-hydroxy-3-(trifluoromethyl)azetidin-1-yl)cyclohexyl)carbamate (0.6099 g, 1.638 mmol) in MeOH (5.46 mL) at 25 °C under nitrogen atmosphere. Then, the reaction vessel was fitted with a hydrogen balloon and the vessel was repeatedly evacuated and purged with hydrogen, then stirred for 16 h. Then, the vessel was repeatedly evacuated and purged with nitrogen, filtered through Celite®, and concentrated to give the title compound (0.3807 g, 93 % yield). $^1H$ NMR (400 MHz, $CD_3SOCD_3$) δ 1.24-1.48 (m, 8 H), 2.10-2.18 (m, 1 H), 2.64-2.72 (m, 1 H), 3.03 (d, *J* = 9 Hz, 2 H), 3.41 (d, *J* = 9 Hz, 2 H), 4.08 (br s, 2 H), 6.79 (br s, 1 H); LC-MS (LC-ES) M+H = 239.

**Intermediate 71: 1-(*trans*-4-Aminocyclohexyl)-3-(trifluoromethyl)azetidin-3-ol**

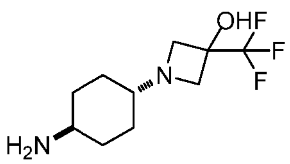

Palladium on carbon (0.018 g, 0.167 mmol) was added to benzyl (*trans*-4-(3-hydroxy-3-(trifluoromethyl)azetidin-1-yl)cyclohexyl)carbamate (0.6234 g, 1.674 mmol, Intermediate 70, Step A) in MeOH (5.58 mL) at 25 °C under nitrogen atmosphere. Then, the reaction vessel was fitted with a hydrogen balloon and the vessel was repeatedly evacuated and

purged with hydrogen, then stirred for 16 h. Then, the vessel was repeatedly evacuated and purged with nitrogen, filtered through Celite®, and concentrated to give the title compound (0.4144 g, 99 % yield). $^1H$ NMR (400 MHz, $CD_3SOCD_3$) δ 0.87 (q, *J* = 13 Hz, 2 H), 0.97 (q, *J* = 13 Hz, 2 H), 1.60-1.74 (m, 4 H), 1.93 (tt, *J* = 11, 4 Hz, 1 H), 2.10 (br s, 2 H), 2.46 (tt, *J* = 11, 4 Hz, 1 H), 3.05 (d, *J* = 9 Hz, 2 H), 3.43 (d, *J* = 9 Hz, 2 H), 6.80 (br s, 1 H); LC-MS (LC-ES) M+H = 239.

**Intermediate 72: trans-4-(2-(methylsulfonyl)ethoxy)cylohexanamine**

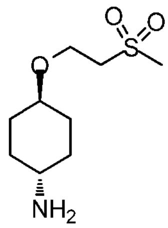

**A. 2-((trans-4-(dibenzylamino)cyclohexyl)oxy)ethanol**

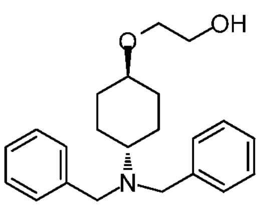

To a solution of tert-butyl 2-((trans-4-(dibenzylamino)cyclohexyl)oxy)acetate (Intermediate 42, step B) (500 mg, 1.221 mmol) in THF (50 mL) was added LAH (1.0 M in THF) (4.88 mL, 4.88 mmol) by a dropwise addition and the reaction was stirred at RT overnight. The reaction was cooled to 0 °C in an ice bath and quenched by the slow dropwise addition of water (0.5 mL) in THF (25 mL), then 1 M NaOH (0.5 mL). The salts were removed by filtering through Celite® and rinsing the pad with EtOAc. The THF was removed under reduced pressure and the aqueous layer was extracted with EtOAc (3 X 50 mL). The combined organic layers were filtered through cotton and evaporated to afford the title compound (400 mg, 97%). MS (ESI) *m/z* 340(M+1).

**B. 2-((trans-4-(dibenzylamino)cyclohexyl)oxy)ethyl 4-methylbenzenesulfonate**

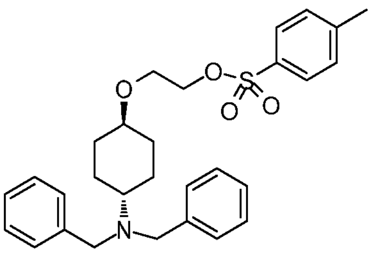

To a solution of 2-((trans-4-(dibenzylamino)cyclohexyl)oxy)ethanol (400 mg, 1.178 mmol) in DCM (60 mL) was added triethylamine (1.642 mL, 11.78 mmol), p-toluenesulfonyl chloride (247 mg, 1.296 mmol) and DMAP (14.40 mg, 0.118 mmol). The reaction was stirred at RT for 4 h, then was diluted with DCM and washed with water followed by brine. The organics were combined, and absorbed onto silica gel. The crude material was purified by silica gel chromatography (5-25% EtOAc/hexanes) to afford the title compound (400 mg, 69%). MS (ESI) *m/z* 494(M+1).

**C. trans-N,N-dibenzyl-4-(2-(methylthio)ethoxy)cyclohexanamine**

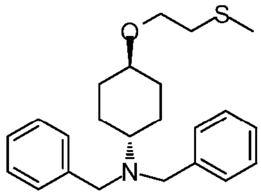

To a solution of 2-((trans-4-(dibenzylamino)cyclohexyl)oxy)ethyl 4-methylbenzenesulfonate (400 mg, 0.810 mmol) in DMF was added sodium thiomethoxide (142 mg, 2.026 mmol) and the reaction was stirred at RT overnight. The reaction was diluted with with EtOAc (50 mL) and washed with water (2X50 mL). The organic layer was then absorbed onto silica gel and purified by silica gel chromatography (5-30% EtOAc/hexanes) to afford the title compound (250 mg, 83%). MS (ESI) *m*/*z* 370(M+1).

**D. trans-N,N-dibenzyl-4-(2-(methylsulfonyl)ethoxy)cyclohexanamine**

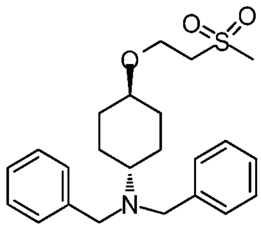

To a solution oftrans-N,N-dibenzyl-4-(2-(methylthio)ethoxy)cyclohexanamine (300 mg, 0.812 mmol) in MeOH (50 mL) was added Oxone® (1248 mg, 2.029 mmol) as a solution in water (5 mL). The reaction was then allowed to stir at RT overnight. The reaction was added to a 10% solution of sodium thiosulfate (100 mL) and allowed to to stir 30 min at RT. The MeOH was removed under reduced pressure and the aqueous layer was extracted with DCM (3X50 mL). The organic layers were then adborbed onto silica gel and purified by silica gel chromatography (5-30% EtOAc/hexanes) to afford the title compound (250 mg, 77%). MS (ESI) *m*/*z* 402(M+1).

**E. trans-4-(2-(methylsulfonyl)ethoxy)cyclohexanamine**

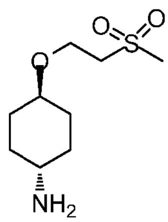

To a $N_2$ degassed solution of trans-N,N-dibenzyl-4-(2-(methylsulfonyl)ethoxy)cyclohexanamine (250 mg, 0.623 mmol) in EtOH (50 mL) was added palladium hydroxide on carbon (43.7 mg, 0.311 mmol). The reaction was evacuated and then hydrogenated overnight under an $H_2$ balloon. The hydrogen was removed and the reaction mixture was purged with $N_2$. The catalyst was filtered off using a Celite® pad and the residue was rinsed with EtOH. The filtrate was then concentrated to afford the title compound (100 mg, 73%). MS (ESI) *m*/*z* 222(M+1).

**Intermediate 73: trans-4-((Prop-2-yn-1-yloxy)methyl)cyclohexanamine, hydrochloride**

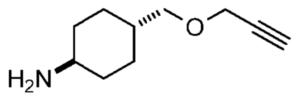

**A. tert-Butyl (trans-4-((prop-2-yn-1-yloxy)methyl)cyclohexyl)carbamate**

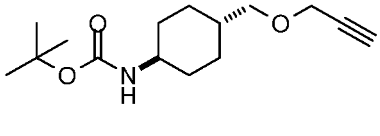

To a solution of tert-butyl (trans-4-(hydroxymethyl)cyclohexyl)carbamate (Intermediate 43, step B) (200 mg, 0.872 mmol) in DMF (8 mL) was added NaH (77 mg, 1.92 mmol, 60%) and the mixture was stirred for 1 h. To this was added 3-bromoprop-1-yne (104 mg, 0.872 mmol) and the reaction was stirred at RT overnight.The reaction was quenched into water and extracted with EtOAc (3X). The combined organics were washed with brine, dried over $Na_2SO_4$, filtered, and concentrated. The residue was purified by silica gel chromatography (EtOAc-hexanes gradient) to afford the title compound as a white solid (35 mg, 15% yield). $^1H$ NMR (400 MHz, $CDCl_3$) δ ppm 0.95 - 1.16 (m, 4 H), 1.44 (s, 9 H), 1.81 - 1.87 (m, 2 H), 2.02 (d, *J* = 9 Hz, 2 H), 2.41 (t, *J* = 2 Hz, 1 H), 3.32 (d, *J* = 7 Hz, 2 H), 4.12 (d, *J* = 2 Hz, 2 H).

**B. trans-4-((Prop-2-yn-1-yloxy)methyl)cyclohexanamine hydrochloride**

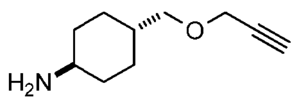

To a solution of tert-butyl (trans-4-((prop-2-yn-1-yloxy)methyl)cyclohexyl)carbamate (32 mg, 0.120 mmol), in 1,4-dioxane (1 mL), cooled on ice, was added HCl (0.120 mL, 0.479 mmol, 4M in dioxane). The ice was removed and the reaction stirred at RT for 4 h. The reaction was concentrated to give a white solid. NMR shows small amount of starting material. To this was added dioxane (1 mL) and HCl (0.2 mL, 4M in dioxane) and the mixture was stirred for 5 h. The reaction was concentrated to give the title compound as a white solid (24 mg, 98% yield). $^1H$ NMR (400 MHz, $CD_3SOCD_3$) δ ppm 0.86 - 1.16 (m, 3 H), 1.20 - 1.33 (m, 1 H), 1.73 (d, *J* =5 Hz, 3 H), 1.93 (d, *J* = 10 Hz, 1 H), 2.91 (d, *J* =4 Hz, 1 H), 3.19 - 3.27 (m, 2 H), 4.06 - 4.14 (m, 2 H).

**Intermediate 74: 1 -(((trans-4-Aminocyclohexyl)oxy)methyl)cyclopropanol hydrochloride**

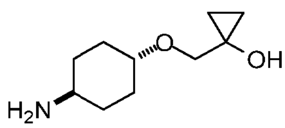

**A. 1-(((trans-4-(Dibenzylamino)cyclohexyl)oxy)methyl)cyclopropanol**

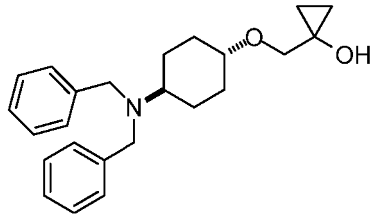

To a solution of tert-butyl 2-((trans-4-(dibenzylamino)cyclohexyl)oxy)acetate (Intermediate 42, step B) (1000 mg, 2.442 mmol) in THF (28 mL) was added titanium(IV) isopropoxide (1.00 mL, 3.42 mmol). To this mixture was added ethylmagnesium chloride (3.42 mL, 6.84 mmol) dropwise over 30 min and the reaction was stirred at RT overnight. The reaction was quench by the addition of water (25 mL) with stirring, filtered to remove a ppt and the filtrate was extracted with EtOAc (3X). The combined organics were washed with water and brine, dried over $Na_2SO_4$, filtered, and concentrated. The residue was purified by silica gel chromatography (0-60% EtOAc-hexanes gradient) to afford the title compound as a white solid (496 mg, 56% yield). $^1H$ NMR (400 MHz, $CDCl_3$) $\delta$ ppm 0.45 - 0.54 (m, 2 H), 0.75 - 0.83 (m, 2 H), 1.10 -1.22 (m, 2 H), 1.32 - 1.46 (m, 2 H), 1.91 (d, *J* = 12 Hz, 2 H), 2.03 - 2.10 (m, 2 H), 2.52 (tt, *J* = 12, 3 Hz, 1 H), 3.22 (tt, *J* = 11, 4 Hz, 1 H), 3.45 (s, 2 H), 3.59 (s, 4 H), 7.15 - 7.21 (m, 2 H), 7.25 - 7.29 (m, 4 H), 7.32 - 7.37 (m, 4 H). MS (ESI) *m/z* 366 (M+1).

B. **1-(((trans-4-Aminocyclohexyl)oxy)methyl)cyclopropanol, hydrochloride**

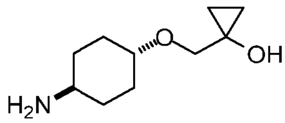

A Fisher-porter bottle was flushed with $N_2$ and charged with 1-((((trans)-4-(dibenzylamino)cyclohexyl)oxy)methyl)cyclopropanol (550 mg, 1.505 mmol) and EtOH (10 mL). Under a $N_2$ atm Pearlman's catalyst (106 mg, 0.150 mmol) was added, and the vessel was evacuated and flushed with $N_2$ and then stirred under 30 psi $H_2$ for 20 h. The vessel was evacuated and flushed with $N_2$, filtered through a pad of Celite®, washing with MeOH, and concentrated. The residue was disolved in THF (5 mL) and to this was added 4N HCl in dioxane (1 mL), after stirring for 3 min, the mixture was concentrated to give the title compound (331 mg, 99% yield). MS (ESI) *m/z* 186 (M+1).

**Intermediate 75: Racemic (2S,5R)-5-Amino-N,N-dimethyltetrahydro-2H-pyran-2-carboxamide, hydrochloride**

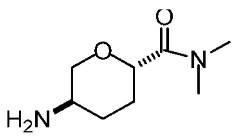

**A. Racemic tert-Butyl (6-(dimethylcarbamoyl)tetrahydro-2Hpyran-3-yl)carbamate**

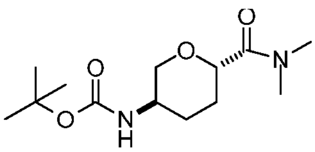

To a solution of 5-((tertbutoxycarbonyl)amino)tetrahydro-2H-pyran-2-carboxylic acid (Intermediate 49, step E) (100 mg, 0.408 mmol) in DMF (4 mL), was added DIEA (0.071 mL, 0.408 mmol) and HATU (155 mg, 0.408 mmol). After stirring for 5 min, dimethylamine (0.204 mL, 0.408 mmol, 2M in THF) was added and the reaction was stirred at RT for

2.5 h. The reaction was quenched into 10 mL water and extracted with EtOAc (3X). The combined organics were dried over $Na_2SO_4$, filtered, and concentrated. The residue was purified by silica gel chromatography (0-80%(3:1 EtOAc:EtOH)-hexanes gradient) to afford the title compound as a thick oil (71 mg, 64% yield). MS (ESI) *m*/*z* 273 (M+1).

**B. Racemic (2S,5R)-5-Amino-N,N-dimethyltetrahydro-2H-pyran-2-carboxamide hydrochloride**

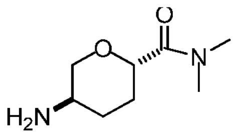

To tert-butyl (6-(dimethylcarbamoyl)tetrahydro-2H-pyran-3-yl)carbamate (70 mg, 0.257 mmol) was added 1,4-dioxane (3 mL) followed by HCl (0.643 mL, 2.57 mmol, 4M in dioxane). The mixture was stirred at RT overnight, and concentrated to give the title compound as a white solid (55 mg, 103% yield). MS (ESI) *m*/*z* 173 (M+1).

**Intermediate 76: (3S)-3-Amino-5-methylpyrrolidin-2-one hydrochloride**

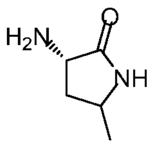

**A. (S)-tert-Butyl (5-methylene-2-oxotetrahydrofuran-3-yl)carbamate**

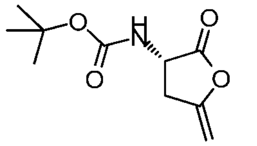

To a solution of (S)-2-((tert-butoxycarbonyl)amino)pent-4-ynoic acid (0.5 g, 2.345 mmol) in tert-butanol (10 mL) and water (10 mL) was added copper(I) bromide (0.067 g, 0.469 mmol). After stirring at RT for 24 h, the crude reaction was concentrated to afford the title compound (0.5 g, 100%), which was used for the next reaction without further purification. MS (ESI) *m*/*z* 158(M-t-butyl +1).

**B. (S)-Methyl 2-((tert-butoxycarbonyl)amino)-4-oxopentanoate**

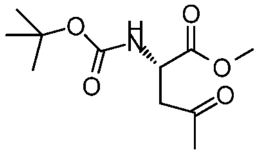

A solution of (S)-tert-butyl (5-methylene-2-oxotetrahydrofuran-3-yl)carbamate (0.5 g, 2.345 mmol) in MeOH (30 mL) was stirred at RT for 24 h. The reaction mixture was absorbed to silica gel and purified by silica gel chromatography (5-25% EtOAc/hexanes) (using $KMnO_4$ to determine which fractions contained the product) to afford the title compound (0.4 g, 70%). $^1H$ NMR (400 MHz, $CD_3SOCD_3$) δ ppm 1.29 - 1.45 (m, 9 H) 2.10 (s, 3 H) 2.71 - 2.95 (m, 2 H) 3.32 (s, 3 H) 4.31-4.37 (m, 1 H) 7.16 (d, *J* = 8 Hz, 1 H) MS (ESI, basic method) *m*/*z* 246(M+1).

**C. (2S)-Methyl 4-amino-2-((tert-butoxycarbonyl)amino)pentanoate**

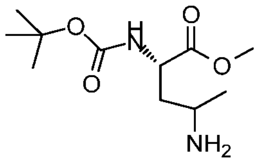

To a solution of (S)-methyl 2-((tert-butoxycarbonyl)amino)-4-oxopentanoate (0.4 g, 1.631 mmol) in MeOH (30 mL) was added ammonium acetate (1.006 g, 13.05 mmol) and sodium cyanoborohydride (1.025 g, 16.31 mmol). After stirring at RT for 24 h, the reaction was concentrated under reduced pressure and the residue was quenched with $NH_4Cl$ (25 mL). The aqueous layer was extracted with 10% MeOH/DCM (3X25 mL). The combined organic layers were then filtered through a cotton plug, evaporated, and carried forward to the cyclization step without further characterization.

**D: tert-Butyl ((3S)-5-methyl-2-oxopyrrolidin-3-yl)carbamate**

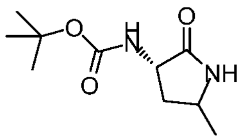

A solution of (2S)-methyl 4-amino-2-((tert-butoxycarbonyl)amino)pentanoate (200 mg, 0.812 mmol) in 1,4-dioxane (15 mL) was heated at 90 °C for 24 h. The reaction was absorbed on to silica gel and purified by silica gel chromatography (DCM to 5% MeOH/DCM) (using $KMnO_4$ to visualize product) to afford the title compound (90 mg, 52%). MS (ESI, basic method) *m/z* 215(M+1).

**E: (3S)-3-Amino-5-methylpyrrolidin-2-one hydrochloride**

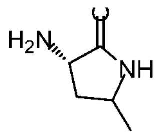

To a solution of tert-butyl ((3S)-5-methyl-2-oxopyrrolidin-3-yl)carbamate (90 mg, 0.420 mmol) in 1,4-dioxane (10 mL) was added 4.0 M HCl in dioxane (2.63 mL, 10.50 mmol) and the reaction was allowed to stir overnight at RT. The crude reaction was concentrated under reduced pressure and used immediately.

**Intermediate 77: trans-4-Aminocvclohexvl sulfamate**

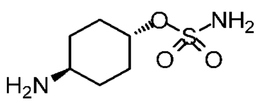

**A. trans-4-(Dibenzylamino)cyclohexyl sulfamate**

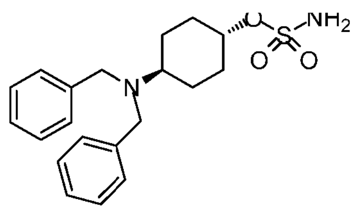

To a suspension of sodium hydride (122 mg, 3.05 mmol) in DMF (15 mL) was added trans-4-(dibenzylamino)cyclohexanol (300 mg, 1.016 mmol) at 0 °C and the reaction was allowed to then stir at RT for 1 h. Next, the reaction was then placed back into the ice bath and sulfamoyl chloride (176 mg, 1.523 mmol) was added dropwise as a solution in acetonitrile (2 mL). The reaction was allowed to stir at RT for 3 days. At this time, the reaction was quenched with saturated $NH_4Cl$ (25 mL), and the suspension was extracted with EtOAc (3X25 mL). The organic layer was then absorbed to silica gel and purified by silica gel chromatography (5-30% EtOAc/hexanes) to afford the title compound (250 mg, 66%). MS (ESI) *m/z* 375(M+1).

**B. trans-4-Aminocyclohexyl sulfamate**

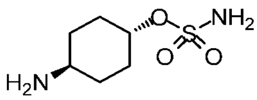

To a degassed solution oftrans-4-(dibenzylamino)cyclohexyl sulfamate (250 mg, 0.668 mmol) in EtOH (50 mL) was added palladium hydroxide on carbon (18.75 mg, 0.134 mmol) and the reaction was hydrogenated at RT under an $H_2$ atmosphere using a balloon. The reaction was filtered through Celite®, rinsed with MeOH, and concentrated to afford the title compound (90 mg, 69%). MS (ESI) *m/z* 195(M+1).

**Intermediate 78: (3S,4R)-3-Amino-4-methylpyrrolidin-2-one**

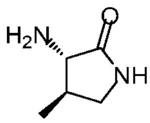

**A. (S)-Dimethyl 2-((9-phenyl-9H-fluoren-9-yl)amino)succinate**

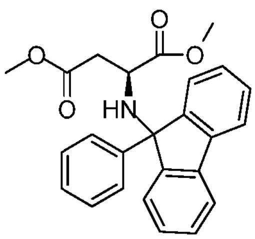

To a suspension of (S)-dimethyl 2-aminosuccinate hydrochloride (2.6g, 13.16 mmol) in acetonitrile (100 mL) was added nitrate lead(II) salt (4.36 g, 13.16 mmol), potassuim phosphate, tribasic (5.59 g, 26.3 mmol), and 9-bromo-9-phenyl-9H-fluorene (5.07 g, 15.79 mmol) . The reaction was allowed to stir at RT for 72 h. The solids were filtered and the filtrate was washed with EtOAc (200 mL). The crude material was then absorbed onto silica gel and purified by silica gel chromatography (5-30% EtOAc/hexanes) to afford the title compound (4.5g, 85%). MS (ESI) *m/z* 402(M+1).

**B. (3S)-Dimethyl 2-methyl-3-((9-phenyl-9H-fluoren-9-yl)amino)succinate**

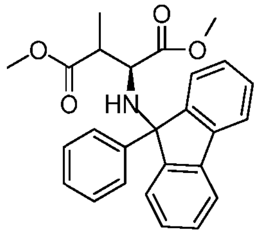

To an oven-dried flask under $N_2$ was added THF (60 mL), followed by potassium hexamethyldisilazide (1M in THF, 2.242 mL, 2.242 mmol). The resulting solution was then cooled to -78 °C. Next, a solution of (S)-dimethyl 2-((9-phenyl-9H-fluoren-9-yl)amino)succinate (500 mg, 1.245 mmol) in THF (5 mL) was added dropwise and the resulting reaction mixture was stirred at -78 °C for 1 h. At this time, iodomethane (0.234 mL, 3.74 mmol) was added and the reaction was stirred at -78 °C until judged complete by TLC (25% EtOAc/hexane). The reaction was quenched with saturated $NH_4Cl$ (50 mL), the organic layer removed, the aqueous layer extracted with EtOAc (2 X 50 mL), and the combined organic extracts were absorbed onto silica gel. The product was then purified by silica gel chromatography (2-20% EtOAc/hexanes) to afford the title compound (4.88g, 76%). MS (ESI) *m*/*z* 416(M+1).

**C. (3S,4S)-4-Methyl-3-((9-phenyl-9H-fluoren-9-yl)amino)dihydrofuran-2(3H)-one**

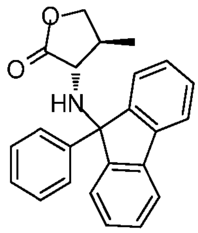

To an oven-dried flask under $N_2$ was added a solution of (3S)-dimethyl 2-methyl-3-((9-phenyl-9H-fluoren-9-yl)amino)succinate (4.88 g, 11.75 mmol) in THF (100 mL). The reaction was cooled to -35°C in a dry ice/acetone bath and allowed to equilibrate at this temperature for 30 min. Next, DIBAL-H (1.0 M in hexanes) (35.2 mL, 35.2 mmol) was added dropwise and the resulting reaction was stirred at -35 °C for 1 h. The reaction was then quenched with saturated $NH_4Cl$ (75 mL), organic layer separated, and aqueous layer re-extracted with EtOAc (2 X 50 mL). The combined organic layers were absorbed to silica gel and purified by silica gel chromatography (5-50% EtOAc/hexanes). The uncyclized alcohol product was isolated and added to DCM (100 mL). Next, tosic acid (2.234 g, 11.75 mmol) was added and the reaction was stirred at RT overnight. The reaction was washed with saturated $NaHCO_3$ (25 mL), organic layer separated, and absorbed onto silica gel. The product was purified by silica gel chromatography (5-40% EtOAc/hexanes) to afford the title compound (2.2 g, 53%). $^1H$ NMR (400 MHz, $CD_3SOCD_3$) $\delta$ ppm 0.27 (d, *J* = 6 Hz, 3 H) 2.10 - 2.24 (m, 1 H) 2.45 - 2.49 (m, 1 H) 3.45 (dd, *J* = 10, 9 Hz, 1 H) 3.59 (d, *J* = 6 Hz, 1 H) 4.10 - 4.21 (m, 1 H) 7.14 - 7.56 (m, 11 H) 7.80 - 7.91 (m, 2 H) MS (ESI) *m*/*z* 378(M+Na).

**D. (2S,3S)-4-Hydroxy-3-methyl-2-((9-phenyl-9H-fluoren-9-yl)amino)butanamide**

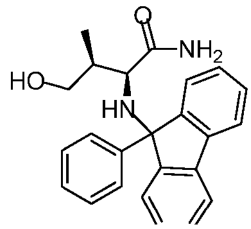

To a solution of ammonium hydroxide (5.48 mL, 141 mmol) in trifluoroethanol (20 mL) and THF (20 mL) was added (3S,4S)-4-methyl-3-((9-phenyl-9H-fluoren-9-yl)amino)dihydrofuran-2(3H)-one (2.5 g, 7.03 mmol). The reaction was allowed to stir at RT for 24 h. The reaction was diluted with EtOAc, and the aqueous layer was removed. The aqueous layer was extracted with EtOAc (2 X 25 mL). The combined organic layers were absorbed onto silica gel and purified by silica gel chromatography (DCM to 10% MeOH/DCM) to afford the title compound (2.0 g, 76%). MS (ESI) *m/z* 373(M+1).

**E. (3S,4R)-4-Methyl-3-((9-phenyl-9H-fluoren-9-yl)amino)pyrrolidin-2-one**

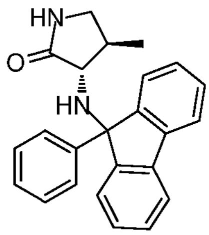

To a solution of (E)-di-tert-butyl diazene-1,2-dicarboxylate (2.38 mL, 10.74 mmol) in THF (50 mL) was added tributylphosphine (5.43 g, 26.8 mmol). The reaction was allowed to stir at RT for 15 min. Next, a solution of (2S, 3S)-4-hydroxy-3-methyl-2-((9-phenyl-9H-fluoren-9-yl)amino)butanamide (2 g, 5.37 mmol) in THF (2 mL) was added at 0 °C and the resulting reaction was stirred at RT overnight. The reaction was absorbed on to silica gel and purified by silica gel chromatography (DCM to 10% MeOH/DCM) to afford the title compound (1.3 g, 68%). MS (ESI) *m/z* 355(M+1).

**F. (3S,4R)-3-Amino-4-methylpyrrolidin-2-one**

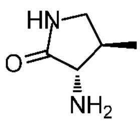

To a $N_2$ degassed solution of (3S,4R)-4-methyl-3-((9-phenyl-9H-fluoren-9-yl)amino)pyrrolidin-2-one (1.3 g, 3.67 mmols) in EtOAc (100 mL) was added palladium on carbon (0.390 g, 3.67 mmol) and the reaction was stirred under a $H_2$ balloon overnight. The reaction was filtered through Celite®, rinsed with EtOAc, and concentrated. The crude material (413 mg, 99% yield) was concentrated ca. 1 h prior to coupling the amine to an appropriate carboxylic acid core.

**Intermediate 79: ((trans)-4-Aminocyclohexyl)methanol, hydrochloride**

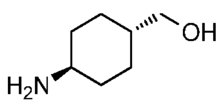

To (trans)-methyl 4-((tert-butoxycarbonyl)amino)cyclohexanecarboxylate (intermediate 43, step B) (315 mg, 1.374 mmol) was added HCl (20 mL, 80 mmol) (4N in dioxane), and the reaction was stirred at RT for 6 h. The mixture was concentrated to give the title compound as a white solid (240 mg, 105% yield), which was used without purification.

**Intermediate 80: tert-Butyl 2-((trans-4-aminocyclohexyl)oxy)acetate**

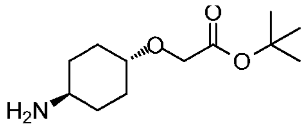

To tert-butyl 2-((trans-4-(dibenzylamino)cyclohexyl)oxy)acetate (Interemediate 42, step B) (1000 mg, 2.442 mmol) was added EtOH (30 mL) and Pearlman's catalyst (343 mg, 0.488 mmol). The flask was flushed well with $N_2$, then stirred under $H_2$ (balloon) for 72 h.The reaction was flushed with $N_2$, filtered through a pad of Celite®, washing with EtOH, and concentrated to give the title compound as a thick oil (582 mg, 104% yield). MS (ESI) *m/z* 230 (M+1).

**Intermediate 81: (S)-6-Chloro-7-(2-methylazetidin-1-yl)quinoline-3-carboxylic acid ammonia salt**

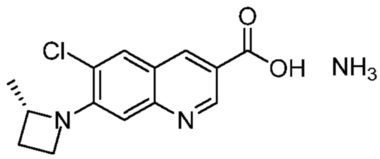

**A. (R)-1-(tert-Butoxycarbonyl)azetidine-2-carboxylic acid**

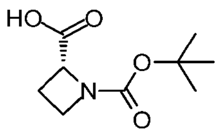

Di-tert-butyl dicarbonate (11.33 g, 51.9 mmol) in 1,4-dioxane (49.5 mL) was added to (R)-azetidine-2-carboxylic acid (5.00 g, 49.5 mmol) in 1,4-dioxane (49.5 mL) and water (49.5 mL) at 0 °C and the reaction mixture was stirred for 2 h at RT. The reaction mixture was concentrated, diethyl ether added, 10% citric acid added, extracted with EtOAc, dried over magnesium sulfate, filtered, and concentrated to give the title compound (7.87 g, 75 % yield). $^1H$ NMR (400 MHz, $CD_3SOCD_3$) δ 1.34 (s, 9 H), 1.96-2.06 (m, 1 H), 2.42-2.52 (m, 1 H), 2.72 (br s, 1 H), 3.66-3.80 (m, 1 H), 3.83 (q, *J* = 8 Hz, 1 H), 4.42 (dd, *J* = 9, 5 Hz, 1 H); LC-MS (LC-ES) M-H = 200.

**B. (R)-tert-Butyl 2-(hydroxymethyl)azetidine-1-carboxylate**

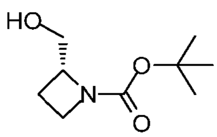

Trimethylsilyl chloride (13.52 mL, 156 mmol) was added slowly to 2.0 M lithium borohydride (39.1 mL, 78 mmol)

in THF at 0 °C and the reaction mixture was stirred for 30 min at RT. After cooling to 0 °C, (R)-1-(tert-butoxycarbonyl)azetidine-2-carboxylic acid (7.87 g, 39.1 mmol) in THF (78 mL) was added dropwise and the reaction was stirred for 2 h at RT. The reaction mixture was quenched with MeOH, followed by water, then concentrated. The reaction mixture was extracted with EtOAc, washed with saturated sodium chloride, dried over magnesium sulfate, filtered, and concentrated. The resulting residue was purified by silica gel chromatography, eluting with ethyl acetate:hexanes (1:1) to give the title compound (1.44 g, 18.7 % yield). $^1$H NMR (400 MHz, $CD_3SOCD_3$) δ 1.35 (s, 9 H), 1.96-2.06 (m, 1 H), 2.06-2.18 (m, 1 H), 3.44-3.52 (m, 1 H), 3.54-3.74 (m, 3 H), 4.06-4.16 (m, 1 H), 4.72 (t, *J* = 6 Hz, 1 H).

**C. (R)-tert-Butyl 2-(((methylsulfonyl)oxy)methyl)azetidine-1-carboxylate**

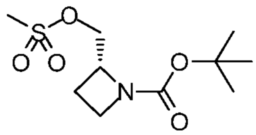

Triethylamine (1.286 mL, 9.23 mmol) was added to (R)-tert-butyl 2-(hydroxymethyl)azetidine-1-carboxylate (1.44 g, 7.69 mmol) in DCM (15.38 mL) at 0 °C, then methanesulfonyl chloride (0.595 mL, 7.69 mmol) was added dropwise and the reaction was stirred for 16 h at RT. The reaction mixture was treated with saturated sodium bicarbonate, extracted with DCM, dried over magnesium sulfate, filtered, and concentrated. The resulting residue was purified by silica gel chromatography, eluting with ethyl acetate:hexanes (2:3) to give the title compound (1.95 g, 91 % yield). $^1$H NMR (400 MHz, $CD_3SOCD_3$) δ 1.37 (s, 9 H), 2.00-2.10 (m, 1 H), 2.20-2.32 (m, 1 H), 3.20 (s, 3 H), 3.60-3.76 (m, 2 H), 4.22-4.30 (m, 1 H), 4.36-4.44 (m, 2 H); LC-MS (LC-ES) M-H = 266.

**D. (S)-tert-Butyl 2-methylazetidine-1-carboxylate**

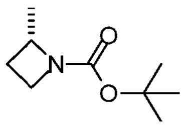

Lithium triethylborohydride (29.4 mL, 29.4 mmol) in THF (1.0 M) was added to (R)-tert-butyl 2-(((methylsulfonyl)oxy)methyl)azetidine-1-carboxylate (1.95 g, 7.35 mmol) in THF (7.35 mL) at 0 °C under nitrogen and the reaction mixture was stirred for 3 h at RT. Then the reaction mixture was quenched with water at 0 °C, extracted with EtOAc, washed with 10% citric acid, washed with saturated sodium bicarbonate, dried over magnesium sulfate, filtered, and concentrated. The residue was purified by silica gel chromatography, eluting with EtOAc:hexanes (1:4) to give the title compound (0.9826 g, 74.2 % yield). Care should be taken as the product is volatile. $^1$H NMR (400 MHz, $CD_3SOCD_3$) δ 1.27 (d, *J* = 6 Hz, 3 H), 1.35 (s, 9 H), 1.66-1.80 (m, 1 H), 2.18-2.34 (m, 1 H), 3.64-3.80 (m, 2 H), 4.14-4.26 (m, 1 H).

**E. (S)-2-Methylazetidine hydrochloride**

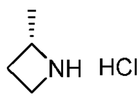

2.0 M HCl (11.48 mL, 22.95 mmol) in diethyl ether was added to (S)-tert-butyl 2-methylazetidine-1-carboxylate (0.9826 g, 5.74 mmol) at RT and the reaction mixture was stirred for 16 h. The reaction mixture was concentrated to give (S)-2-methylazetidine hydrochloride (0.6389 g, 46.6 % yield), contaminated with decomposition products. This product is low molecular weight and might be volatile and was carried forward without further purification. $^1$H NMR (400 MHz, $CD_3SOCD_3$) δ 1.41 (d, *J* = 7 Hz, 3 H), 2.04-2.16 (m, 1 H), 2.36-2.48 (m, 1 H), 3.71 (dt, *J* = 10, 6 Hz, 1 H), 3.81 (q, *J* = 9 Hz, 1 H), 4.41 (h, *J* = 7 Hz, 1 H), 8.70 (br s, 2 H).

**F. (S)-Ethyl 6-chloro-7-(2-methylazetidin-1-yl)quinoline-3-carboxylate**

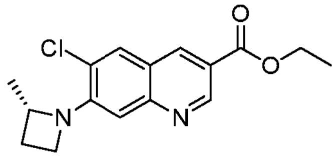

DIEA (0.847 mL, 4.85 mmol) was added to ethyl 6-chloro-7-fluoroquinoline-3-carboxylate (Intermediate 28, step E) (0.3075 g, 1.212 mmol) in EtOH (6.06 mL) at RT. Then, (S)-2-methylazetidine hydrochloride (0.222 g, 2.061 mmol) was added and the reaction mixture was heated at 100 °C in the microwave for 2 h and concentrated. The reaction mixture was dissolved in DCM, washed with saturated sodium bicarbonate, dried over magnesium sulfate, filtered, and concentrated. The residue was purified by silica gel chromatography, eluting with ethyl acetate:hexanes (1:3) to give the title compound (0.1579 g, 40.6 % yield). $^1H$ NMR (400 MHz, $CD_3SOCD_3$) $\delta$ 1.35 (t, *J* = 7 Hz, 3 H), 1.38 (d, *J* = 6 Hz, 3 H), 1.92-2.02 (m, 1 H), 2.46-2.52 (m, 1 H), 3.86-3.94 (m, 1 H), 4.36 (q, *J* = 7 Hz, 2 H), 4.36-4.46 (m, 1 H), 4.52-4.64 (m, 1 H), 7.04 (s, 1 H), 8.19 (s, 1 H), 8.74 (d, *J* = 2 Hz, 1 H), 9.13 (d, *J* = 2 Hz, 1 H); LC-MS (LC-ES) M+H = 305.

**G. (S)-6-Chloro-7-(2-methylazetidin-1-yl)quinoline-3-carboxylic acid ammonia salt**

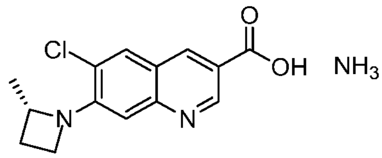

Lithium hydroxide (0.036 g, 1.512 mmol) was added to (S)-ethyl 6-chloro-7-(2-methylazetidin-1-yl)quinoline-3-carboxylate (0.1536 g, 0.504 mmol) in MeOH (4.03 mL) and water (1.008 mL) at RT and the reaction mixture was stirred 4 h at 60 °C. The reaction mixture was concentrated. The reaction mixture was purified by RP HPLC eluting with acetonitrile:water with 0.1 % ammonium hydroxide (5:95:100:0) to give the title compound (0.1289 g, 83 % yield). $^1H$ NMR (400 MHz, $CD_3SOCD_3$) $\delta$ 1.36 (d, *J* = 6 Hz, 3 H), 1.92-2.02 (m, 1 H), 2.42-2.50 (m, 1 H), 3.80 (q, *J* = 8 Hz, 1 H), 4.32-4.40 (m, 1 H), 4.52 (h, *J* = 7 Hz, 1 H), 7.03 (s, 1 H), 8.06 (s, 1 H), 8.55 (d, *J* = 2 Hz, 1 H), 9.14 (d, *J* = 2 Hz, 1 H); LC-MS (LC-ES) M-H = 275.

**Intermediate 82**

**Racemic 2-(6-Aminospiro[3.3]heptan-2-yl)propan-2-ol**

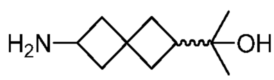

**A. Methyl 3-methylenecyclobutanecarboxylate**

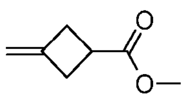

To a N,N-dimethylformamide (350 mL) mixture of 3-methylenecyclobutanecarboxylic acid (11.6 g, 103 mmol) and cesium carbonate (70.8 g, 217 mmol) was added iodomethane (17.6 g, 124 mmol). After stirring overnight, the reaction was partitioned between diethyl ether and water, the organic layer separated and the aqueous layer extracted with diethyl ether (3X). The combined organic layers were washed with water, dried over magnesium sulfate, filtered and concentrated to give the title compound (10.9 g, 84%) as a yellow oil. $^1$H NMR (400 MHz, $CDCl_3$) δ 2.85-2.92 (m, 2 H), 2.92-3.04 (m, 2 H), 3.09-3.17 (m, 1 H), 3.70 (s, 3 H), 4.77-4.82 (m, 2 H).

**B. Methyl 6-oxospiro[3.3]heptane-2-carboxylate**

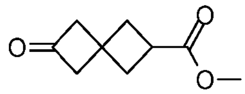

To a methyl acetate (45 mL) solution of methyl 3-methylenecyclobutanecarboxylate (5.0 g, 39.6 mmol) was added copper powder (2.77 g, 43.6 mmol) and zinc powder (5.70 g, 87 mmol). To this mixture was added a methyl acetate (45 mL) solution of 2,2,2-trichloroacetyl chloride (4.86 mL, 43.6 mmol) and phosphorus oxychloride (0.37 mL, 4.0 mmol) dropwise over 2 h. After 3 h, the reaction was cooled to 0°C, and additional zinc powder (5.70 g, 87 mmol) was added followed by acetic acid (22.7 mL, 400 mmol) dropwise at a rate keeping the temperature below 7°C. The reaction was allowed to slowly warm to room temperature and after stirring overnight was filtered through Celite®, rinsing with ethyl acetate. The filtrate was carefully washed (warning: gas evolution) with saturated aqueous sodium bicarbonate (2 X 200 mL), and the aqueous layers extracted with 1:1 ethyl acetate:diethyl ether (2 X 100 mL). The combined organic layers were dried over magnesium sulfate, filtered and concentrated, and the residue purified on silica gel eluting with a 0%-50% ethyl acetate:hexanes gradient. The appropriate fractions were combined, evaporated under reduced pressure and placed *in vacuo* to give the title compound (4.10 g, 61%) as a yellow oil. $^1$H NMR (400 MHz, $CDCl_3$) δ 2.38-2.48 (m, 2 H), 2.50-2.61 (m, 2 H), 3.00-3.09 (m, 2 H), 3.09-3.22 (m, 3 H), 3.68 (s, 3 H).

**C. Racemic Methyl 6-(dibenzylamino)spiro[3.3]heptane-2-carboxylate**

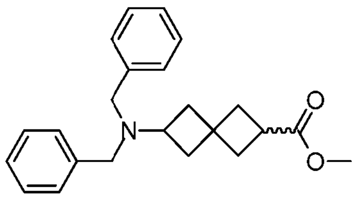

To a tetrahydrofuran (200 mL) solution of methyl 6-oxospiro[3.3]heptane-2-carboxylate (3.89 g, 23.1 mmol) was added dibenzylamine (4.67 mL, 24.29 mmol). After 10 minutes, the reaction was cooled to 0°C, and sodium triacetoxyborohydride (7.35 g, 34.7 mmol) was added as a solid, portionwise, over 10 minutes followed by 4-5 drops of glacial acetic acid. The reaction was allowed to warm to room temperature. After 4 h, the reaction mixture was diluted with water (20 mL), extracted with diethyl ether (200 mL) and washed with saturated sodium bicarbonate (100 mL). The aqueous layer was extracted with diethyl ether (1 X 100 mL) and the organic layers were combined, dried over magnesium sulfate, filtered and concentrated, and the residue was purified on silica gel eluting with a 0%-50% ethyl acetate:hexanes gradient. The appropriate fractions were combined, evaporated under reduced pressure and placed *in vacuo* to give the title compound (5.00 g, 62%) as a white solid. $^1$H NMR (400 MHz, $CDCl_3$) δ 1.74-1.92 (m, 2 H), 1.97-2.34 (m, 6 H), 2.90-3.09 (m, 2 H), 3.35-3.51 (m, 4 H), 3.64 (s, 3 H), 7.09-7.44 (m, 10 H); LC-MS (LC-ES) M+H = 350.

**D. Racemic 2-(6-(Dibenzylamino)spiro[3.3]heptan-2-yl)propan-2-ol**

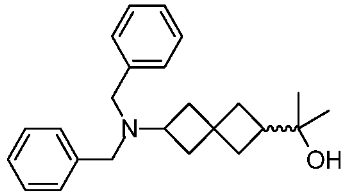

To methyl 6-(dibenzylamino)spiro[3.3]heptane-2-carboxylate (5.00 g, 14.3 mmol) in diethyl ether (200 mL) at 0°C was added a 3.0 M solution of methylmagnesium chloride in diethyl ether (15.7 mL, 47.2 mmol). After 30 minutes, the mixture was warmed to room temperature for 70 minutes, cooled to 0°C, quenched with 3 N hydrochloric acid and partitioned between saturated aqueous sodium bicarbonate (150 mL) and diethyl ether (100 mL). The aqueous layer was separated and extracted with ethyl acetate 100 mL). The organic layers were combined, dried over magnesium sulfate, filtered and concentrated, and the residue was purified on silica gel eluting with a 0%-100% ethyl acetate:hexanes gradient. The appropriate fractions were combined, evaporated under reduced pressure, and placed *in vacuo* to give the title compound (4.65 g, 93%) as a white solid. $^1$H NMR ($CDCl_3$) δ 1.06 (s, 6 H), 1.66-1.97 (m, 7 H), 2.05-2.31 (m, 2 H), 2.91-3.06 (m, 1 H), 3.45 (s, 4 H), 7.09-7.41 (m, 10 H); LC-MS (LC-ES) M+H = 350.

**E. Racemic 2-(6-Aminospiro[3.3]heptan-2-yl)propan-2-ol**

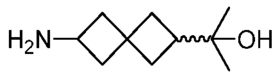

To 2-(6-(dibenzylamino)spiro[3.3]heptan-2-yl)propan-2-ol (4.10 g, 11.7 mmol) and ethanol (100 mL) under an nitrogen atmosphere was added 20% palladium hydroxide (329 mg, 2.35 mmol), and the vessel was evacuated and flushed with nitrogen and then stirred under 35 psi hydrogen overnight. The vessel was evacuated and flushed with nitrogen and the mixture filtered through a pad of Celite® and rinsed with methanol. The filtrate was concentrated to give the title compound as a white solid (2.18 g, quantitative). $^1$H NMR (400 MHz, $CDCl_3$) δ 1.07 (d, *J* = 1 Hz, 6 H), 1.39 (s, 3 H), 1.52 -1.61 (m, 1 H), 1.63-1.69 (m, 1 H), 1.74-1.97 (m, 4 H), 2.11 - 2.27 (m, 2 H), 2.35-2.48 (m, 1 H), 3.30 (p, *J* = 8 Hz, 1 H).

**Intermediate 83**

***trans*-N1-(2,2,2-Trifluoroethyl)cyclohexane-1,4-diamine dihydrochloride**

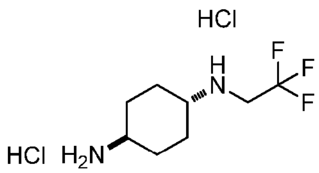

**A. tert-Butyl (*trans*-4-((2,2,2-trifluoroethyl)amino)cyclohexyl)carbamate**

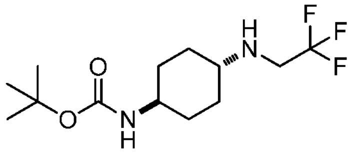

N,N-Di*i*sopropylethylamine (0.759 mL, 4.35 mmol) was added to *tert*-butyl *trans*-(4-aminocyclohexyl)carbamate

(0.4658 g, 2.174 mmol) in N,N-dimethylformamide (4.67 mL) at room temperature, followed by 2,2,2-trifluoroethyl trifluoromethanesulfonate (0.605 g, 2.61 mmol) and the reaction mixture was stirred at 65 °C for sixty-four hours. The reaction mixture was concentrated and the residue was purified by RP HPLC, eluting with acetonitrile:water with 0.1% ammonium hydroxide (5:95 to 100:0). After concentration, the residue was purified by silica gel chromatography, eluting with ethyl acetate:hexanes (2:3) to give *tert*-butyl *(trans*-4-((2,2,2-trifluoroethyl)amino)cyclohexyl)carbamate (0.4926 g, 1.579 mmol, 72.7 % yield). $^1$H NMR (400 MHz, $CD_3SOCD_3$) δ 0.99 (q, *J* = 13 Hz, 2 H), 1.11 (q, *J* = 13 Hz, 2 H), 1.36 (s, 9 H), 1.72 (br d, *J* = 12 Hz, 2 H), 1.83 (br d, *J* = 12 Hz, 2 H), 2.15 (q, *J* = 7 Hz, 1 H), 2.24-2.36 (m, 1 H), 3.08-3.26 (m, 3 H), 6.69 (d, *J* = 8 Hz, 1 H); LC-MS (LC-ES) M+H = 297.

**B. *trans*-N1-(2,2,2-Trifluoroethyl)cyclohexane-1,4-diamine dihydrochloride**

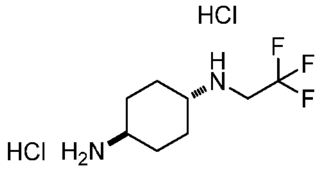

4.0 M Hydrochloric acid (4.16 mL, 16.62 mmol) in dioxane was added to *tert*-butyl (*trans*-4-((2,2,2-trifluoroethyl)amino)cyclohexyl)carbamate (0.4926 g, 1.662 mmol) in ethanol (4.16 mL) at room temperature and the reaction mixture was stirred for twenty hours. The reaction mixture was concentrated to give *trans*-N1-(2,2,2-trifluoroethyl)cyclohexane-1,4-diamine dihydrochloride (0.4435 g, 1.565 mmol, 94 % yield). $^1$H NMR (400 MHz, $CD_3SOCD_3$) δ 1.36 (p, *J* = 12 Hz, 2 H), 1.47 (m, 2 H), 2.01 (d, *J* = 11 Hz, 2 H), 2.13 (br s, 2 H), 2.95 (br s, 1 H), 3.07 (br s, 1 H), 4.05 (br s, 2 H), 8.07 (br s, 3 H), 9.97 (br s, 2 H); LC-MS (LC-ES) M+H = 197.

**Intermediate 84**

**trans-4-Amino-N,N-dimethylcyclohexanamine oxide hydrochloride**

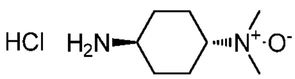

**A. trans-4-((tert-Butoxycarbonyl)amino)-N,N-dimethylcyclohexanamine oxide**

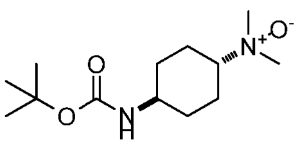

Ammonium bicarbonate (0.412 g, 5.21 mmol) was added to hydrogen peroxide (1.012 g, 10.42 mmol) in water (5.21 mL) at room temperature and stirred for fifteen minutes. Then, tert-butyl (trans-4-(dimethylamino)cyclohexyl)carbamate (0.5049 g, 2.083 mmol) was added and the reaction mixture was stirred for sixteen hours, then concentrated to give trans-4-((tert-butoxycarbonyl)amino)-N,N-dimethylcyclohexanamine oxide (0.5291 g, 1.946 mmol, 93 % yield). $^1$H NMR (400 MHz, $CD_3SOCD_3$) δ 1.19 (q, *J* = 13 Hz, 2 H), 1.38 (s, 9 H), 1.49 (q, *J* = 13 Hz, 2 H), 1.87 (br d, *J* = 12 Hz, 2 H), 2.17 (br d, *J* = 12 Hz, 2 H), 3.06 (s, 6 H), 3.08-3.26 (m, 2 H), 6.75 (br d, *J* = 7 Hz, 1 H); LC-MS (LC-ES) M+H = 259.

**B. trans-4-Amino-N,N-dimethylcyclohexanamine oxide hydrochloride**

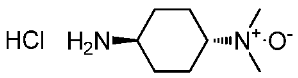

4.0 M Hydrochloric acid (2.048 mL, 8.19 mmol) in dioxane was added to trans-4-((tert-butoxycarbonyl)amino)-N,N-dimethylcyclohexanamine oxide (0.5291 g, 2.048 mmol) at room temperature and the reaction mixture was stirred for sixteen hours. The reaction mixture was concentrated to give crude trans-4-amino-N,N-dimethylcyclohexanamine oxide hydrochloride (0.3963 g, 1.934 mmol, 94 % yield). $^1H$ NMR (400 MHz, $CD_3SOCD_3$) δ 1.43 (q, *J* = 12 Hz, 2 H), 1.63 (q, *J* = 12 Hz, 2 H), 2.10 (br d, *J* = 13 Hz, 2 H), 2.22 (br d, *J* = 12 Hz, 2 H), 2.96-3.06 (m, 1 H), 3.39 (s, 6 H), 3.62-3.74 (m, 1 H), 8.20 (br s, 3 H); LC-MS (LC-ES) M+H = 159.

**Intermediate 85**

**6-Chloro-7-fluoro-N-((trans)-4-(2-hydroxypropan-2-yl)cyclohexyl)quinoline-3-carboxamide**

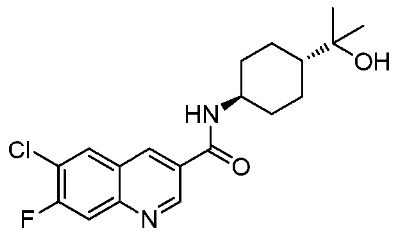

**A. 6-Chloro-7-fluoroquinoline-3-carboxylic acid**

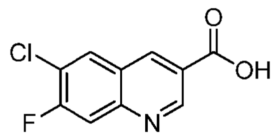

To a solution of ethyl 6-chloro-7-fluoroquinoline-3-carboxylate (Intermediate 28E) (200 mg, 0.788 mmol) in tetrahydrofuran (4.393 mL) and methanol (4.393 mL) were added a solution of sodium hydroxide (134 mg, 3.34 mmol) in water (1 mL), upon which a precipitate crashed out in solution, and the reaction was stirred at room temperature and periodically monitored by LC-MS. The reaction mixture was concentrated down to the aqueous phase and acidified with 1 M hydrochloric acid to pH ~3-4, which caused precipitation of a solid. The mixture was stirred for a few minutes and then the solids were collected by vacuum filtration and washed sequentially with hexanes and dried under high vacuum overnight to give 6-chloro-7-fluoroquinoline-3-carboxylic acid (115 mg, 0.510 mmol, 65% yield). $^1H$ NMR (400 MHz, $CD_3OD$) δ 7.93 (d, *J* = 10 Hz, 1 H), 8.36 (d, *J* = 8 Hz, 1 H), 8.99 (s, 1 H), 9.40 (s, 1 H); LC-MS (LC-ES) M+H = 225.

**B. 6-Chloro-7-fluoro-N-((trans)-4-(2-hydroxypropan-2-yl)cyclohexyl)quinoline-3-carboxamide**

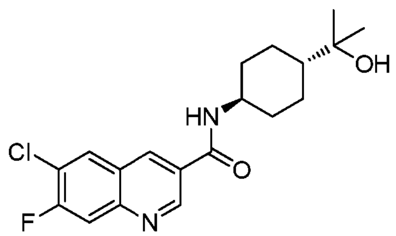

6-Chloro-7-fluoroquinoline-3-carboxylic acid (87 mg, 0.386 mmol) was dissolved in N,N-dimethylformamide (3.472 mL), after which N,N-diisopropylethylamine (0.101 mL, 0.578 mmol) and O-(7-azabenzotriazol-1-yl)-N,N,N',N'-tetramethyluronium hexafluorophosphate (183 mg, 0.482 mmol) were added. After several minutes of stirring, 2-((trans)-4-aminocyclohexyl)propan-2-ol (91 mg, 0.578 mmol) and N,N-diisopropylethylamine (0.101 mL, 0.578 mmol) were added. The resulting solution was stirred at room temperature. Upon consumption of the starting material, water was added to crash out the product as a precipitate, which was then vacuum filtered to give 6-chloro-7-fluoro-N-((trans)-4-(2-hydroxypropan-2-yl)cyclohexyl)quinoline-3-carboxamide (91 mg, 0.225 mmol, 58% yield). $^1$H NMR (400 MHz, $CD_3OD$) δ 1.20 (s, 7 H), 1.23-1.54 (m, 6 H), 1.90-2.22 (m, 4 H), 3.90 (br s, 1 H), 7.90 (d, *J* = 10 Hz, 1 H), 8.28 (d, *J* = 8 Hz, 1 H), 8.75 (s, 1 H), 9.26 (s, 1 H); LC-MS (LC-ES) M+H = 365.

**Intermediate 86**

**(trans)-4-(6-Chloro-7-(difluoromethoxy)quinoline-3-carboxamido)cyclohexanecarboxylic acid**

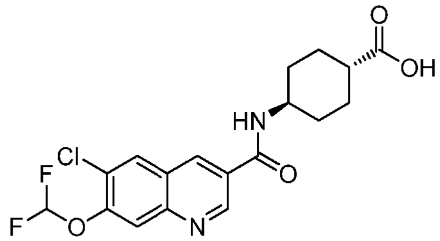

**A. (trans)-Methyl 4-(6-chloro-7-(difluoromethoxy)quinoline-3-carboxamido)cyclohexanecarboxylate**

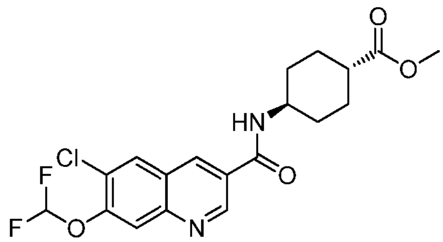

6-Chloro-7-(difluoromethoxy)quinoline-3-carboxylic acid (Intermediate 20) (219 mg, 0.800 mmol), (trans)-methyl 4-aminocyclohexanecarboxylate hydrochloride (233 mg, 1.201 mmol), and N,N-diisopropylethylamine (0.559 mL, 3.20 mmol) were suspended in N,N-dimethylformamide (2.80 mL) followed by the addition of propylphosphonic anhydride solution (50 wt % in ethyl acetate) (0.953 mL, 1.601 mmol). Upon consumption of the starting material saturated sodium bicarbonate (2 mL) was used to quench the reaction, which was then stirred for an additional hour. An ethyl acetate (3X 10 mL) extraction was performed and the organic layers were washed with brine, dried over magnesium sulfate, filtered, and concentrated *in vacuo.* The residue was purified using silica gel chromatography, eluting with 0-30% ethyl acetate:hexanes to give (trans)-methyl 4-(6-chloro-7-(difluoromethoxy)quinoline-3-carboxamido)cyclohexanecarboxylate (100 mg, 0.242 mmol, 30% yield). $^1$H NMR (400 MHz, $CD_3OD$) δ 1.40-1.55 (m, 2 H), 1.55-1.70 (m, 2 H), 2.12 (t, *J* = 11 Hz, 4 H), 2.38 (t, *J* = 12 Hz, 1 H), 3.70 (s, 3 H), 3.94 (t, *J* = 12 Hz, 1 H), 6.94-7.47 (m, 1 H), 7.89 (s, 1 H), 8.28 (s, 1 H), 8.73 (s, 1 H), 9.26 (s, 1 H); LC-MS (LC-ES) M+H = 413.

**B. (trans)-4-(6-Chloro-7-(difluoromethoxy)quinoline-3-carboxamido)cyclohexanecarboxylic acid**

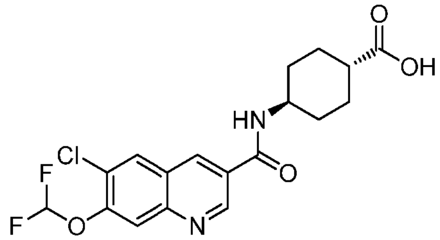

(trans)-Methyl 4-(6-chloro-7-(difluoromethoxy)quinoline-3-carboxamido)cyclohexanecarboxylate (100 mg, 0.242 mmol) was suspended in a tetrahydrofuran (2.95 mL):methanol (2.95 mL) solution. To this solution was added sodium hydroxide (48.4 mg, 1.211 mmol) in water (2 mL). The resulting solution was stirred at room temperature and subsequently monitored by LC-MS. The reaction mixture was stripped down *in vacuo* and acidified to give a white precipitate in the reaction mixture. The precipitate was vacuum filtered and dried to give (trans)-4-(6-chloro-7-(difluoromethoxy)quinoline-3-carboxamido)cyclohexanecarboxylic acid (89 mg, 0.212 mmol, 88% yield). $^1$H NMR (400 MHz, $CD_3OD$) δ 1.31 (brs, 1 H), 1.41-1.54 (m, 2 H), 1.62 (q, *J* = 12 Hz, 2 H), 2.13 (d, *J* = 10 Hz, 4 H), 2.33 (t, *J* = 12 Hz, 1 H), 3.95 (t, *J* = 12 Hz, 1 H), 6.94-7.43 (m, 1 H), 7.90 (s, 1 H), 8.28 (s, 1 H), 8.74 (s, 1 H), 9.26 (s, 1 H); LC-MS (LC-ES) M+H = 399.

**Intermediate 87**

**Ethyl 6-chloro-7-(isopropylamino)quinoline-3-carboxylate**

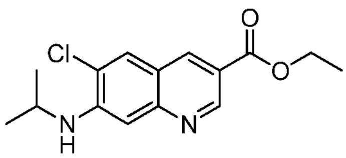

Ethyl 6-chloro-7-fluoroquinoline-3-carboxylate (Intermediate 28E) (100 mg, 0.394 mmol), propan-2-amine (0.067 mL, 0.788 mmol), and N,N-diisopropylethylamine (0.344 mL, 1.971 mmol) were suspended in acetonitrile (0.920 mL), heated to 95 °C overnight, and subsequently monitored by LC-MS. The solution was concentrated under reduced pressure and taken up in ethyl acetate and saturated sodium bicarbonate solution. The solution was poured into a separatory funnel and an ethyl acetate extraction was performed. The organic layers were combined, filtered, and concentrated *in vacuo* to give a brown solid. The residue was purified using silica gel chromatography, eluting with 0-25% ethyl acetate:hexanes to give ethyl 6-chloro-7-(isopropylamino)quinoline-3-carboxylate (88 mg, 0.301 mmol, 76% yield). $^1$H NMR (400 MHz, $CD_3OD$) δ 1.34 (d, *J* = 6 Hz, 6 H), 1.42 (t, *J* = 7 Hz, 3 H), 3.76-3.84 (m, 1 H), 4.39 (q, *J* = 7 Hz, 2 H), 6.89 (s, 1 H), 7.72 (s, 1 H), 8.39 (s, 1 H), 8.96 (s, 1 H); LC-MS (LC-ES) M+H = 293.

**Intermediate 88**

**6-Chloro-7-(cyclopropylamino)quinoline-3-carboxylic acid**

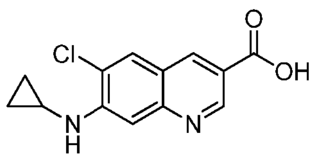

**A. Ethyl 6-chloro-7-(cyclopropylamino)quinoline-3-carboxylate**

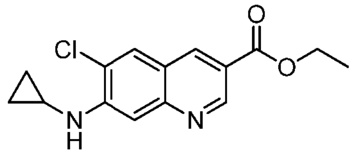

Ethyl 6-chloro-7-fluoroquinoline-3-carboxylate (Intermediate 28E) (100 mg, 0.394 mmol), cyclopropanamine (0.082 mL, 1.183 mmol), and N,N-diisopropylethylamine (0.344 mL, 1.971 mmol) were suspended in acetonitrile (0.920 mL), and heated to 85 °C. Later, 0.4 mL of cyclopropylamine was added to the solution which was then heated to 100 °C and subsequently monitored by LC-MS. The solution was concentrated under reduced pressure, and then taken up in ethyl acetate and saturated sodium bicarbonate solution. The resulting solution was poured into a separatory funnel and an ethyl acetate extraction was performed. The organic layers were combined, filtered, and concentrated *in vacuo* to give the title compound (122 mg, 0.420 mmol, 106% crude yield). $^1$H NMR (400 MHz, $CD_3OD$) δ 1.11-1.24 (m, 4 H), 1.48 (t, *J* = 7 Hz, 3 H), 2.27-2.41 (m, 1 H), 4.50 (q, *J* = 7 Hz, 2 H), 7.82 (s, 1 H), 9.12 (d, *J* = 3 Hz, 1 H), 9.34 (s, 1 H), 9.50 (d, *J* = 2 Hz, 1 H); LC-MS (LC-ES) M+H = 290.

**B. 6-Chloro-7-(cyclopropylamino)quinoline-3-carboxylic acid**

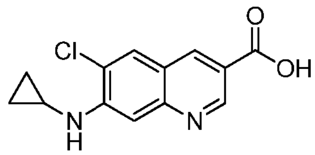

Ethyl 6-chloro-7-(cyclopropylamino)quinoline-3-carboxylate (122 mg, 0.420 mmol) was dissolved in tetrahydrofuran (2.208 mL) and methanol (2.208 mL) after which sodium hydroxide (84 mg, 2.098 mmol) in water was slowly added. The reaction was stirred at room temperature and subsequently monitored by LC-MS. The mixture was concentrated *in vacuo* to the aqueous phase and subsequently acidified with 1 M hydrochloric acid solution upon which the product crashed out as a solid. The solid was filtered and dried *via* vacuum filtration to yield the 6-chloro-7-(cyclopropylamino)quinoline-3-carboxylic acid (54 mg, 0.185 mmol, 44% yield). $^1$H NMR (400 MHz, $CD_3OD$) δ 0.65-0.75 (m, 2 H), 0.90-0.98 (m, 2 H), 2.64 (br s, 1 H), 7.53 (s, 1 H), 8.04 (s, 1 H), 8.77 (s, 1 H), 9.17 (s, 1 H); LC-MS (LC-ES) M+H = 263.

## Intermediate 89

**6-Chloro-7-(difluoromethoxy)-N-(3-oxocyclobutyl)quinoline-3-carboxamide**

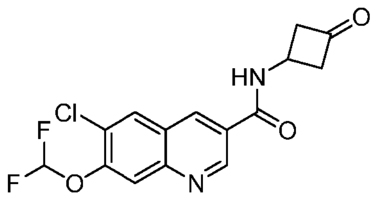

To a solution of 6-chloro-7-(difluoromethoxy)quinoline-3-carboxylic acid (Intermediate 20) (125 mg, 0.457 mmol) in N,N-dimethylformamide (4.116 mL) was added N,N-diisopropylethylamine (0.120 mL, 0.685 mmol) and O-(7-azabenzotriazol-1-yl)-N,N,N',N'-tetramethyluronium hexafluorophosphate (217 mg, 0.571 mmol), which was stirred for 3-5 minutes prior to the addition of 3-aminocyclobutanone hydrochloride (61.1 mg, 0.503 mmol) and N,N-diisopropylethylamine (0.239 mL, 1.371 mmol). The reaction was stirred and subsequently monitored by LC-MS. Water was added and a precipitate crashed out in solution which was vacuum filtered to give 6-chloro-7-(difluoromethoxy)-N-(3-oxocyclobutyl)quinoline-3-carboxamide (87 mg, 0.230 mmol, 50% yield). $^1$H NMR (400 MHz, $CD_3OD$) δ 3.26-3.36 (m, 2 H), 3.48-3.59 (m, 2 H), 4.59-4.77 (m, 1 H), 6.97-7.45 (m, 1 H), 7.91 (s, 1 H), 8.29 (s, 1 H), 8.79 (s, 1 H), 9.31 (s, 1 H); LC-MS (LC-ES) M+H = 341.

**Intermediate 90**

**6-Chloro-7-(trifluoromethoxy)quinoline-3-carboxylic acid and 8-chloro-7-(trifluoromethoxy)quinoline-3-carboxylic acid**

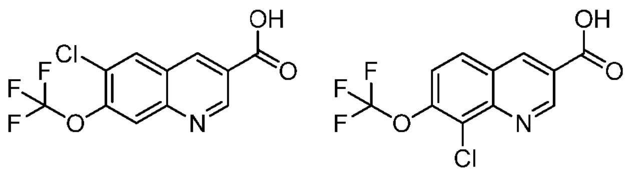

**A. Methyl 2-((3-chloro-4-(trifluoromethoxy)phenyl)(hydroxy)methyl)acrylate**

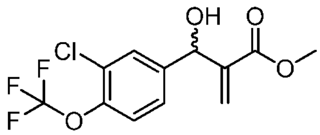

To 3-chloro-4-(trifluoromethoxy)benzaldehyde (1.90 g, 8.46 mmol) was added 1,4-dioxane:water (1:1) (0.500 mL), methyl acrylate (2.33 mL, 25.4 mmol), and 1,4-diazabicyclo[2.2.2]octane (0.949 g, 8.46 mmol). The reaction was stirred at room temperature and monitored by TLC (8:2 hexanes:ethyl acetate). Upon completion (18 h), water (10 mL) was added and the reaction was transferred to a separatory funnel and extracted with dichloromethane (3X 30 mL). The organic fractions were combined, dried over anhydrous sodium sulfate, filtered, and concentrated under reduced pressure. The crude oil was purified via normal phase chromatography (silica-gel, 40 g ISCO, hexanes:ethyl acetate (95:5 to 8:2)) to afford the title compound as a tan oil (2.19 g, 7.03 mmol, 83% yield). $^1$H NMR (400 MHz, $CD_3SOCD_3$) $\delta$ 3.63 (s, 3 H), 5.48 (d, *J* = 4 Hz, 1 H), 6.00-6.08 (m, 2 H), 6.27 (d, *J* = 1 Hz, 1 H), 7.40 (dd, *J* = 8, 2 Hz, 1 H), 7.53 (dd, *J* = 8, 1 Hz, 1 H), 7.58 (d, *J* = 2 Hz, 1 H); LC-MS (LC-ES) M+H-$H_2$O]=293.

**B. Methyl 2-(acetoxy(3-chloro-4-(trifluoromethoxy)phenyl)methyl)acrylate**

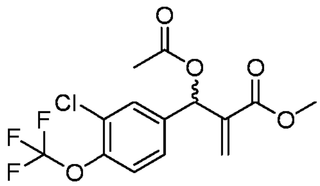

To a stirring solution of methyl 2-((3-chloro-4-(trifluoromethoxy)phenyl)(hydroxy)methyl)acrylate (2.10 g, 6.76 mmol) in dichloromethane (10.0 mL) was added acetic anhydride (0.765 mL, 8.11 mmol), followed by 4-dimethylaminopyridine (0.206 g, 1.69 mmol). The reaction was monitored by TLC (9:1 hexanes:ethyl acetate) and upon completion (2 h), the reaction was quenched with water (10 mL). The mixture was transferred to a separatory funnel and extracted with dichloromethane (25 mL, 3X). This extract was dried over sodium sulfate, concentrated to a minimal volume, and purified by chromatography (ISCO system, 24 g silica gel cartridge, (9:1 to 8:2) hexanes:ethyl acetate). The pure fractions were collected and concentrated to dryness to give the title compound as a clear oil (1.86 g, 5.27 mmol, 78% yield). $^1$H NMR (400 MHz, $CD_3SOCD_3$) $\delta$ 2.13 (s, 3 H), 3.69 (s, 3 H), 6.00 (d, *J* = 1 Hz, 1 H), 6.39 (s, 1 H), 6.52 (s, 1 H), 7.48 (dd, *J* = 9, 2 Hz, 1 H), 7.60 (dd, *J* = 9, 1 Hz, 1 H), 7.70 (d, *J* = 2 Hz, 1 H); LC-MS (LC-ES) M-OAc = 293.

**C. (E)-Methyl 2-(azidomethyl)-3-(3-chloro-4-(trifluoromethoxy)phenyl)acrylate**

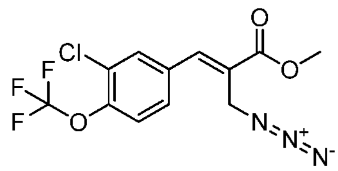

To a stirring colorless solution of methyl 2-(acetoxy(3-chloro-4-(trifluoromethoxy)phenyl)methyl)acrylate (1.60 g, 4.54 mmol) and N,N-dimethylformamide (4 mL) at room temperature was added sodium azide (0.590 g, 9.07 mmol), which formed a suspension. The reaction was monitored by TLC (9:1 hexanes:ethyl acetate) and upon completion (20 min), the reaction was transferred to a separatory funnel with water (25 mL) and extracted with diethyl ether (25 mL, 4X). The organic fractions were collected, dried over sodium sulfate, and filtered. The filtrate was concentrated to a tan oil, dissolved into minimal dichloromethane and purified by chromatography (ISCO, silica gel, 9:1 hexanes:ethyl acetate). The pure fractions were collected and concentrated to dryness *in vacuo* to give the title compound as a tan oil (1.52 g, 4.53 mmol, 100% yield) as a tan oil. $^1H$ NMR (400 MHz, $CD_3SOCD_3$) $\delta$ 3.83 (s, 3 H), 4.22 (s, 2 H), 7.58 (dd, *J* = 9, 2 Hz, 1 H), 7.70 (dd, *J* = 9, 1 Hz, 1 H), 7.83 (d, *J* = 2 Hz, 1 H), 7.90 (s, 1 H); LC-MS (LC-ES) M+H-$N_2$ = 308.

**D. (E)-Methyl 2-(aminomethyl)-3-(3-chloro-4-(trifluoromethoxy)phenyl)acrylate**

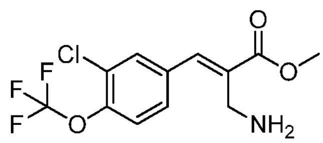

To a stirring solution of (E)-methyl 2-(azidomethyl)-3-(3-chloro-4-(trifluoromethoxy)phenyl)acrylate (1.52 g, 4.53 mmol) in tetrahydrofuran (5 mL) at room temperature was added polymer supported triphenylphosphine (2.28 g, 6.79 mmol) which formed a brown suspension. The reaction was monitored by LC-MS and upon completion (24 h), water (15 mL) was added to the suspension, and the reaction was sonicated (5 minutes, with swirling) followed by filtration. The filtrate was dried *in vacuo,* subsequently dissolved in methyl alcohol and purified by chromatography (C18 ISCO, gradient elution, water:acetonitrile). The pure fractions were collected and concentrated to dryness to give the title compound (339 mg, 1.095 mmol, 24.2% yield) as a pure clear oil. $^1H$ NMR (400 MHz, $CD_3SOCD_3$) $\delta$ 3.83 (m, 5 H), 7.62 (dd, *J* = 9, 2 Hz, 1 H), 7.67-7.73 (m, 1 H), 7.90 (d, *J* = 2 Hz, 1 H), 7.95 (s, 1 H), 8.02 (br s, 2 H); M+H = 310.

**E. Methyl 6-chloro-7-(trifluoromethoxy)quinoline-3-carboxylate and methyl 8-chloro-7-(trifluoromethoxy)quinoline-3-carboxylate**

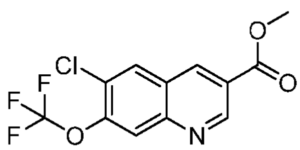

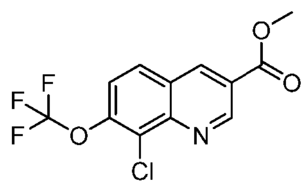

(E)-Methyl 2-(aminomethyl)-3-(3-chloro-4-(trifluoromethoxy)phenyl)acrylate (0.250 g, 0.807 mmol) was dissolved into acetonitrile (3 mL) and stirred at room temperature. Iodine (0.820 g, 3.23 mmol) was added to this solution and the reaction mixture was stirred for 5 minutes, before adding potassium carbonate (446 mg, 3.23 mmol). The reaction was monitored by TLC (9:1 hexanes:ethyl acetate) and LC-MS. Within 40 minutes, starting material had been converted to the respective quinolines in an isomeric excess of 16%, as determined by relative peak area from LC-MS, favoring the methyl 8-chloro-7-(trifluoromethoxy)quinoline-3-carboxylate. The reaction was then transferred to a separatory funnel, diluted with ethyl acetate (50 mL), and washed with saturated sodium thiosulfate (3X, 25 mL) to remove iodine. The organic layer was dried over sodium sulfate, filtered, and concentrated to dryness via evaporation. An analytical portion of the tan concentrate was purified by HPLC (C18, water:acetonitrile, 1% TFA) and fractions containing regioisomers of desired compounds were separated and concentrated to dryness. The two title compounds methyl 6-chloro-7-(trifluoromethoxy)quinoline-3-carboxylate (40 mg, 0.131 mmol, 16.2% yield) and methyl 8-chloro-7-(trifluoromethoxy)quinoline-3-carboxylate (159 mg, 0.520 mmol, 64.4% yield) were obtained as white powders.

**Methyl 6-chloro-7-(trifluoromethoxy)quinoline-3-carboxylate**

$^1$H NMR (400 MHz, $CD_3SOCD_3$) δ 3.99 (s, 3 H), 7.97 (dd, *J* = 9, 1 Hz, 1 H), 8.43 (d, *J* = 9 Hz, 1 H), 9.24 (d, *J* = 2 Hz, 1 H), 9.50 (d, *J* = 2 Hz, 1 H); LC-MS (LC-ES) M+H = 306.

**Methyl 8-chloro-7-(trifluoromethoxy)quinoline-3-carboxylate**

$^1$H NMR (400 MHz, $CD_3SOCD_3$) δ 3.98 (s, 3 H), 8.26 (s, 1 H), 8.71 (s, 1 H), 9.11 (d, *J* = 2 Hz, 1 H), 9.41 (d, *J* = 2 Hz, 1 H); LC-MS (LC-ES) M+H = 306.

**F. 6-Chloro-7-(trifluoromethoxy)quinoline-3-carboxylic acid and 8-chloro-7-(trifluoromethoxy)quinoline-3-carboxylic acid**

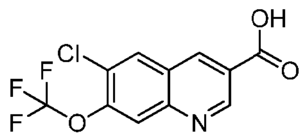

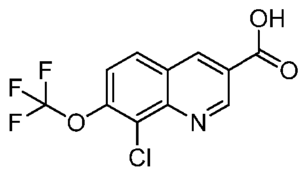

A powder consisting of 6:4 methyl 8-chloro-7-(trifluoromethoxy)quinoline-3-carboxylate (48.0 mg, 0.157 mmol) and methyl 6-chloro-7-(trifluoromethoxy)quinoline-3-carboxylate (32.0 mg, 0.105 mmol) was dissolved into acetone (5 mL) and stirred at room temperature. To the stirring solution was added 1 N sodium hydroxide (5 mL). The pale yellow solution was monitored by TLC and upon completion (40 min), the majority of acetone was removed by evaporation. The resulting aqueous solution was cooled to 0 °C and acidified with 5 N hydrochloric acid (2 mL), which precipitated a white solid. The cooled solution was filtered and the solid was rinsed with water. The white powder was collected and received further purification via reverse phase HPLC (water:acetonitrile, 1% TFA). Subsequently, fractions containing desired compounds were collected, and dried *in* vacuo to give the title compounds 6-chloro-7-(trifluoromethoxy)quinoline-3-carboxylic acid (27.0 mg, 0.093 mmol, 88% yield) and 8-chloro-7-(trifluoromethoxy)quinoline-3-carboxylic acid (40.0 mg, 0.137 mmol, 100% yield) as pure white powders.

**8-Chloro-7-(trifluoromethoxy)quinoline-3-carboxylic acid**

$^1$H NMR (400 MHz, $CD_3SOCD_3$) δ 7.94 (d, *J* = 8 Hz, 1 H), 8.40 (d, *J* = 9 Hz, 1 H), 9.17 (s, 1 H), 9.49 (d, J = 2 Hz, 1 H), 13.83 (br s, 1 H); LC-MS (LC-ES) M+H = 292.

**6-Chloro-7-(trifluoromethoxy)quinoline-3-carboxylic acid**

$^1$H NMR (400 MHz, $CD_3SOCD_3$) δ 8.25 (s, 1 H), 8.69 (s, 1 H), 9.06 (d, *J* = 2 Hz, 1 H), 9.39 (d, *J* = 2 Hz, 1 H); LC-MS (LC-ES) M+H = 292.

**<u>Intermediate 91</u>**

**8-Chloro-7-(difluoromethoxy)quinoline-3-carboxylic acid**

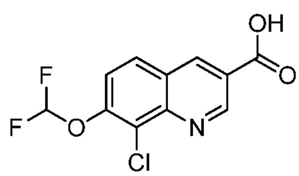

**A. 3-Chloro-4-(difluoromethoxy)benzaldehyde**

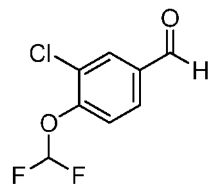

To a suspension of cesium carbonate (6.35 g, 19.5 mmol) in N,N-dimethylformamide (7 mL), heated at 100 °C, was added slowly, via a dropping funnel, a solution of 3-chloro-4-hydroxybenzaldehyde (1.02 g, 6.50 mmol) and sodium 2-chloro-2,2-difluoroacetate (1.98 g, 13.0 mmol) in N,N-dimethylformamide (7 mL) over ~ 2 h. After addition was complete, heating was continued for another ~7 h. With careful monitoring via LC-MS, the reaction showed 34% product and 45% starting material after the time allotment. Upon cooling, water (~75 mL) was added slowly to the reaction mixture and precipitation occurred. The reaction mixture was extracted with ethyl acetate (50 mL, 3X). The organic phase was washed once with 1 N hydrochloric acid, then with water, followed by brine. The combined aqueous phases were back-extracted with ethyl acetate (100 mL). This ethyl acetate phase was washed with water and with brine. The organic phases were combined, dried over sodium sulfate, filtered and concentrated to an oil via evaporation. The tan oil was purified by silica gel chromatography (12 g ISCO column) eluting with hexanes, to give the title compound as a clear oil (740. mg, 3.58 mmol, 55.1% yield). $^1$H NMR (400 MHz, $CD_3SOCD_3$) δ 7.49 (t, *J* = 72 Hz, 1 H), 7.58 (d, *J* = 8 Hz, 1 H), 7.90-8.02 (d, *J* = 8 Hz, 1 H), 8.12 (d, *J* = 2 Hz, 1 H), 9.97 (s, 1 H); LC-MS (LC-ES) M+H = 207.

**B. 2-((3-Chloro-4-(difluoromethoxy)phenyl)(hydroxy)methyl)acrylate**

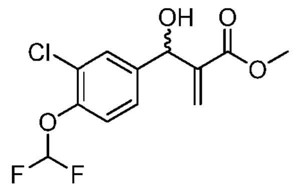

To a solution of 3-chloro-4-(difluoromethoxy)benzaldehyde (0.700 g, 3.39 mmol) in 1,4-dioxane:water (1:1) (0.100 mL) was added methyl acrylate (0.931 mL, 10.2 mmol) and 1,4-diazabicyclo[2.2.2]octane (0.380 g, 3.39 mmol). The reaction mixture was stirred at room temperature and monitored by TLC (8:2 hexanes:ethyl acetate). Upon completion, water (10 mL) was added and the reaction was transferred to a separatory funnel where it was extracted with dichloromethane (3X 30 mL). Organic fractions were collected, dried over anhydrous sodium sulfate, filtered, and concentrated via evaporation. The crude was purified by chromatography (silica-gel, 12 g ISCO, hexanes:ethyl acetate (8:2 to 6:4)) to afford the title compound as a clear oil (893 mg, 3.05 mmol, 90% yield). $^1$H NMR (400 MHz, $CD_3SOCD_3$) δ 3.63 (s, 3 H), 5.44 (d, *J* = 5 Hz, 1 H), 5.96 (d, *J* = 5 Hz, 1 H), 6.01-6.08 (m, 1 H), 6.25 (s, 1 H), 7.27 (t, *J* = 72 Hz, 1 H), 7.32 (m, 2 H), 7.48 (s, 1 H); LC-MS (LC-ES) M-$H_2O$ = 275

**C. Methyl 2-(acetoxy(3-chloro-4-(difluoromethoxy)phenyl)methyl)acrylate**

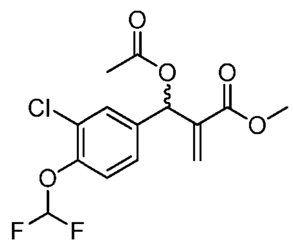

To a solution of methyl 2-((3-chloro-4-(difluoromethoxy)phenyl)(hydroxy)methyl)acrylate (0.120 g, 0.410 mmol) in dichloromethane (8 mL) was added acetic anhydride (0.580 mL, 0.615 mmol) and 4-dimethylaminopyridine (5.01 mg, 0.0410 mmol). The reaction mixture was stirred at room temperature until complete conversion was apparent by TLC (8:2 hexanes:ethyl acetate, 2.5 h). At that time, water (10 mL) was added to the reaction mixture and the opaque reaction was extracted with dichloromethane (3X, 10 mL). Organic layers were combined, dried over sodium sulfate, and filtered. The filtrate was concentrated to minimal volume via evaporation and subsequently loaded onto a column for silica gel chromatography (12 g, ISCO, hexanes:ethyl acetate, (9:1 to 7:3)). Pure fractions were collected, concentrated to an oil via evaporation, and dried further under high vacuum, to give the title compound as a clear oil (116 mg, 0.347 mmol, 85% yield). $^1H$ NMR (400 MHz, $CD_3SOCD_3$) $\delta$ 2.11 (s, 3 H), 3.64-3.72 (s, 3 H), 5.98 (s, 1 H), 6.37 (s, 1 H), 6.48 (s, 1 H), 7.31 (t, *J* = 72 Hz 1 H), 7.34-7.43 (m, 2 H), 7.59 (d, *J* = 2 Hz, 1 H); LC-MS (LC-ES) M-OAc = 275.

**D. (E)-Methyl 2-(azidomethyl)-3-(3-chloro-4-(difluoromethoxy)phenyl)acrylate N39271-39-1**

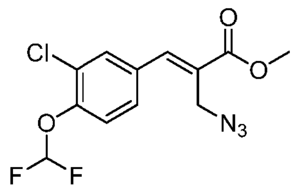

To a stirring solution of methyl 2-(acetoxy(3-chloro-4-(difluoromethoxy)phenyl)methyl)acrylate (0.150 g, 0.448 mmol) and N,N-dimethylformamide (4 mL) was added sodium azide (29.1 mg, 0.448 mmol). The reaction was monitored by TLC (9:1 hexanes:ethyl acetate) and upon completion (20 min), the reaction was transferred to a separatory funnel with water (25 mL) and extracted with diethyl ether (25 mL, 4X). The organic fractions were collected, dried over sodium sulfate, and filtered. The filtrate was concentrated to a tan oil, dissolved in minimal dichloromethane and purified by chromatography (12 g, ISCO, silica gel, 9:1 hexanes:ethyl acetate). The pure fractions were collected and concentrated to dryness to give the title compound as a clear oil (130. mg, 0.409 mmol, 91% yield). $^1H$ NMR (400 MHz, $CD_3SOCD_3$) $\delta$ 3.83 (s, 3 H), 4.22 (s, 2 H), 7.39 (t, *J* = 72 Hz, 1 H), 7.48 (d, *J* = 8 Hz, 1 H), 7.55 (dd, *J* = 8 Hz, 4 Hz, 1 H), 7.76 (d, *J* = 2 Hz, 1 H), 7.89 (s, 1 H); LC-MS (LC-ES) M+H-$N_2$ = 290.

**E. (E)-Methyl 2-(aminomethyl)-3-(3-chloro-4-(difluoromethoxy)phenyl)acrylate**

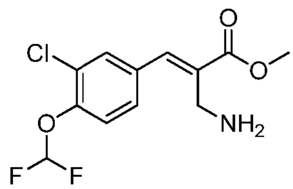

To a stirring solution of (E)-methyl 2-(azidomethyl)-3-(3-chloro-4-(difluoromethoxy)phenyl)acrylate (0.175 g, 0.551 mmol) in tetrahydrofuran (5 mL) at room temperature was added polymer supported triphenylphosphine (3 mmol/g) (0.370 g, 1.10 mmol, 3 mmol/g) which formed a brown suspension. The reaction was monitored by LC-MS and upon completion (28 h) water (15 mL) was added to the suspension, and the reaction was sonicated (5 minutes, with swirling), followed by filtration. The filtrate was dried *in vacuo,* subsequently dissolved in methyl alcohol and purified by chromatography (C18 ISCO, 80 g, gradient elution, water:acetonitrile, 0.1% TFA). The pure fractions were collected and concentrated to dryness to give the title compound as a clear oil (118 mg, 0.405 mmol, 73.4% yield). $^1H$ NMR (400 MHz, $CD_3SOCD_3$) $\delta$ 3.83 (s, 3 H), 3.84 (s, 2 H), 7.41 (t, *J* = 72 Hz, 1 H), 7.45-7.51 (d, *J* = 4 Hz, 1 H), 7.57 (dd, *J* = 9, 2 Hz, 1 H), 7.83 (d, *J* = 2 Hz, 1 H), 7.92 (s, 1 H), 8.02 (br s, 2 H); LC-MS (LC-ES) M+H = 292.

**F. Methyl 8-chloro-7-(difluoromethoxy)quinoline-3-carboxylate**

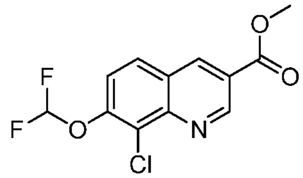

(E)-Methyl 2-(aminomethyl)-3-(3-chloro-4-(difluoromethoxy)phenyl)acrylate (80.0 mg, 0.274 mmol) was dissolved into acetonitrile (5 mL) and stirred at room temperature. Iodine (278 mg, 1.10 mmol) was added to this stirring solution and allowed to stir for 5 minutes, before adding potassium carbonate (152 mg, 1.10 mmol). The reaction was monitored by TLC (9:1 hexanes:ethyl acetate) and LC-MS, and upon completion (40 minutes.), the reaction mixture was transferred to a separatory funnel, diluted with ethyl acetate (50 mL), and washed with saturated sodium thiosulfate (3X, 25 mL). The organic layer was dried over sodium sulfate, filtered, and concentrated to a minimal volume via evaporation. The tan concentrate was purified by HPLC chromatography (C18, 80 g ISCO, water:acetonitrile, 0.1%TFA) and fractions containing regioisomers of desired compounds were separated and concentrated to dryness to give the title compound as a white powder (28.0 mg, 97.0 μmol, 35.5% yield). $^1$H NMR (400 MHz, $CD_3SOCD_3$) δ 3.98 (s, 3 H), 7.54 (t, *J* = 72 Hz, 1 H), 7.82 (d, *J* = 9 Hz, 1 H), 8.36 (d, *J* = 9 Hz, 1 H), 9.17 (d, *J* = 2 Hz, 1 H), 9.46 (d, *J* = 2 Hz, 1 H); LC-MS (LC-ES) M + H = 288.

**G. 8-Chloro-7-(difluoromethoxy)quinoline-3-carboxylic acid**

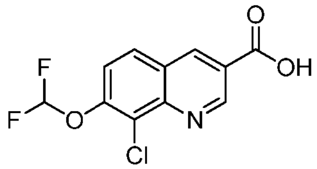

Methyl 8-chloro-7-(difluoromethoxy)quinoline-3-carboxylate (48.0 mg, 0.167 mmol) was dissolved into acetone (5 mL) and stirred at room temperature. To the stirring solution was added 1 N sodium hydroxide (10.0 mL). The pale yellow solution was monitored by TLC and upon completion (30 min), the majority of acetone was removed by evaporation. The resulting aqueous solution was cooled to 0 °C and acidified to pH = 0 with 5 N hydrochloric acid (2 mL) which precipitated a white solid. The cool solution was filtered and the solid was rinsed with water. The white powder was collected to give the title compound (41.0 mg, 0.150 mmol, 90% yield). $^1$H NMR (400 MHz, $CD_3SOCD_3$) δ 7.54 (t, *J* = 72 Hz, 1 H), 7.80 (d, *J* = 9 Hz, 1 H), 8.33 (d, *J* = 9 Hz, 1 H), 9.11 (d, *J* = 2 Hz, 1 H), 9.44 (d, *J* = 2 Hz, 1 H), 13.72 (br s, 1 H); LC-MS (LC-ES) M + H = 274.

**<u>Intermediate 92</u>**

**6,8-Dichloro-7-(difluoromethoxy)quinoline-3-carboxylic acid**

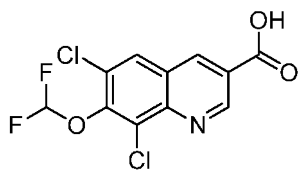

**A. 3,5-Dichloro-4-(difluoromethoxy)benzaldehyde**

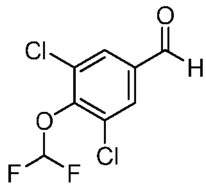

To a suspension of cesium carbonate (13.0 g, 40.0 mmol) in N,N-dimethylformamide (14.0 mL), heated at 90 °C, was added slowly, via a dropping funnel, a solution of 3,5-dichloro-4-hydroxybenzaldehyde (2.50 g, 13.1 mmol) and sodium 2-chloro-2,2-difluoroacetate (3.96 g, 26.0 mmol) in N,N-dimethylformamide (7 mL) over ~1 h. After addition was complete, the reaction was allowed to stir for an additional 2 h, while monitoring via LC-MS. LC-MS shows 31% product and 65% starting material after the time allotment. After another 1 h, the product decreased in relative peak area along with the emergence of side products. At this point, the reaction was cooled to room temperature, water (~75 mL) was added slowly to the reaction mixture and precipitation occurred. The reaction mixture was extracted with ethyl acetate (50 mL, 3X). The organic phase was washed with 1 N hydrochloric acid, water and brine. The combined aqueous phases were back-extracted with ethyl acetate. This ethyl acetate phase was washed with water and brine. The organic phases were combined, dried over sodium sulfate, filtered and concentrated via evaporation. The tan oil was processed without further purification, to give the title compound (1.51 g, 6.28 mmol, 48% yield). $^1H$ NMR (400 MHz, $CD_3SOCD_3$) δ 7.28 (t, *J* = 72 Hz, 1 H), 8.15 (s, 2 H), 9.97 (s, 1 H); LC-MS (LC-ES) T = 0.92 min.

**B. Methyl 2-((3,5-dichloro-4-(difluoromethoxy)phenyl)(hydroxy)methyl)acrylate**

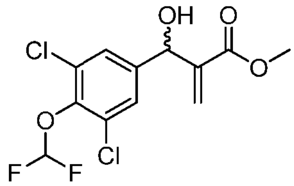

To a solution of 3,5-dichloro-4-(difluoromethoxy)benzaldehyde (1.00 g, 4.15 mmol) in 1,4-dioxane:water (1:1) (0.100 mL) was added methyl acrylate (2.00 mL, 21.84 mmol) and 1,4-diazabicyclo[2.2.2]octane (0.465 g, 4.15 mmol). The reaction was monitored by TLC (8:2 hexanes:ethyl acetate) and LC-MS. Upon completion (24 h), water (7 mL) was added and the mixture was transferred to a separatory funnel and extracted with dichloromethane (3X 30 mL), dried over anhydrous sodium sulfate, filtered, and concentrated to a minimal volume under reduced pressure. The concentrate was purified by chromatography (ISCO, 24.0 g silica, hexanes:ethyl acetate (9:1 to 8:2)) and pure fractions were collected and dried *in vacuo* to give the title compound as an opaque solid (1.34 g, 3.36 mmol, 81% yield). $^1H$ NMR (400 MHz, $CD_3SOCD_3$) δ 3.65 (s, 3 H), 5.46 (d, *J* = 5 Hz, 1 H), 6.07 (s, 1 H), 6.11 (d, *J* = 5 Hz, 1 H), 6.28 (s, 1 H), 7.17 (t, 1 H), 7.50 (s, 2 H); LC-MS (LC-ES) M-$H_2O$ = 309.

**C. Methyl 2-(acetoxy(3,5-dichloro-4-(difluoromethoxy)phenyl)methyl)acrylate**

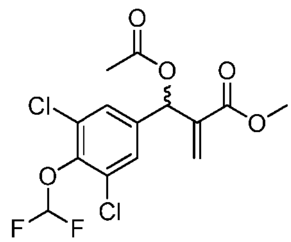

To a stirring solution of methyl 2-((3,5-dichloro-4-(difluoromethoxy)phenyl)(hydroxy)methyl)acrylate (1.30 g, 3.97 mmol) in dichloromethane (15.0 mL) was added acetic anhydride (0.450 mL, 4.77 mmol), followed by 4-dimethylaminopyridine (24.0 mg, 0.199 mmol). The reaction was monitored by TLC (9:1 hexanes:ethyl acetate) and upon completion (2 h), water (10 mL) was added. The mixture was then transferred to a separatory funnel and extracted with dichloromethane (30 mL, 3X). This extract was dried over sodium sulfate, concentrated to a minimal volume *in vacuo,* and purified by chromatography (ISCO system, 24 g silica gel cartridge, (9:1 to 8:2) hexanes:ethyl acetate). The pure fractions were collected and concentrated to dryness *in vacuo* to give the title compound as a clear oil (1.40 g, 3.19 mmol, 80% yield). $^1$H NMR (400 MHz, $CD_3SOCD_3$) $\delta$ 2.13 (s, 3 H), 3.70 (s, 3 H), 6.02 (d, *J* = 1 Hz, 1 H), 6.40 (s, 1 H), 6.49 (s, 1 H), 7.18 (t, *J* = 72 Hz, 1 H), 7.62 (s, 2 H); LC-MS (LC-ES) M-OAc = 309.

**D. (E)-Methyl 2-(azidomethyl)-3-(3,5-dichloro-4-(difluoromethoxy)phenyl)acrylate**

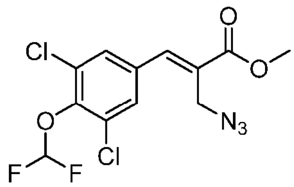

To a stirring colorless solution of methyl 2-(acetoxy(3,5-dichloro-4-(difluoromethoxy)phenyl)methyl)acrylate (900 mg, 2.44 mmol) in tetrahydrofuran (10.0 mL) at room temperature was added sodium azide (396 mg, 6.10 mmol) which formed a suspension. The reaction was followed by TLC (9:1 hexanes:ethyl acetate) and upon completion (25 min) the yellow solution was transferred to a separatory funnel with water (25 mL) and extracted with diethyl ether (25 mL, 4X). The organic fractions were collected, dried over sodium sulfate, and filtered. The filtrate was concentrated to a tan oil, dissolved into minimal dichloromethane and purified by chromatography (ISCO, silica gel, 0-15% hexanes:ethyl acetate). The pure fractions were collected and concentrated to dryness to give the title compound as a white solid (0.883 g, 2.41 mmol, 99% yield$^1$H NMR (400 MHz, $CD_3SOCD_3$) $\delta$ 3.83 (s, 3 H), 4.22 (s, 2 H), 7.21 (t, *J* = 72 Hz, 1 H), 7.74 (s, 2 H), 7.86 (s, 1 H); LC-MS (LC-ES) M-$N_2$ = 324.

**E. (E)-Methyl 2-(aminomethyl)-3-(3,5-dichloro-4-(difluoromethoxy)phenyl)acrylate**

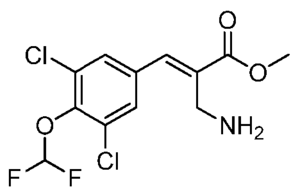

To a stirring solution of (E)-methyl 2-(azidomethyl)-3-(3,5-dichloro-4-(difluoromethoxy)phenyl)acrylate (500 mg, 1.42 mmol) in tetrahydrofuran (5 mL) at room temperature was added polymer supported triphenylphosphine (372 mg, 1.42 mmol), which formed a brown suspension. The reaction was monitored by LC-MS and after 24 h ample starting material remained so the mixture was heated to reflux. Upon full conversion to product (3 h), water (15 mL) was added to the suspension, and the reaction was sonicated (5 minutes, with swirling) followed by filtration. The filtrate was dried *in vacuo,* subsequently dissolved in methanol and purified by chromatography (C18 ISCO, gradient elution, water:acetonitrile). The pure fractions were collected and concentrated to dryness to give the title compound (80.0 mg, 0.245 mmol, 17.3% yield) as a pure clear oil. $^1$H NMR (400 MHz, $CD_3SOCD_3$) $\delta$ 3.43 (s, 2 H), 3.78 (s, 3 H), 7.20 (t, *J* = 72 Hz, 1 H), 7.55 (s, 1 H), 7.94 (s, 2 H); LC-MS (LC-ES) M+H = 326.

**F. Methyl 6,8-dichloro-7-(difluoromethoxy)quinoline-3-carboxylate**

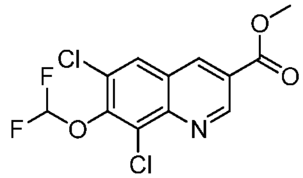

(E)-Methyl 2-(aminomethyl)-3-(3,5-dichloro-4-(difluoromethoxy)phenyl)acrylate (40.0 mg, 0.123 mmol) was dissolved into acetonitrile (3 mL) and stirred at room temperature. Iodine (125 mg, 0.491 mmol) was added to this stirring solution and allowed to stir for5 minutes, before adding potassium carbonate (67.8 mg, 0.491 mmol). The reaction was monitored by TLC (9:1 hexanes:ethyl acetate) and LC-MS. Within 40 minutes, starting material had been converted to the respective quinoline. The reaction was then transferred to a separatory funnel, diluted with ethyl acetate (75 mL), and washed with saturated sodium thiosulfate (3X, 25 mL) to remove iodine. The organic layer was dried over sodium sulfate, filtered, and concentrated to dryness. The tan concentrate was purified by chromatography (silica, ISCO, 0-50% hexanes:ethyl acetate) and fractions containing compound were separated and concentrated to dryness to give the title compound (40 mg, 0.118 mmol, 96 % yield) as a white powder. $^1$H NMR (400 MHz, $CD_3SOCD_3$) δ 3.99 (s, 3 H), 7.35 (t, *J* = 72 Hz, 1 H), 8.65 (s, 1 H), 9.15 (d, *J* = 2 Hz, 1 H), 9.47 (d, *J* = 2 Hz, 1 H); LC-MS (LC-ES) M+H = 322.

**G. 6,8-Dichloro-7-(difluoromethoxy)quinoline-3-carboxylic acid**

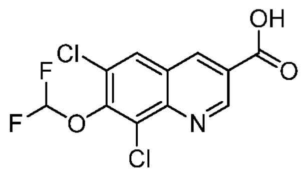

Methyl 6,8-dichloro-7-(difluoromethoxy)quinoline-3-carboxylate (48.0 mg, 0.149 mmol) was dissolved into acetone (5 mL) and stirred at room temperature. To the stirring solution was added 1 N sodium hydroxide (5 mL). The pale yellow solution was monitored by TLC (70:30 hexanes:ethyl acetate) and upon completion (40 min), the majority of acetone was removed by evaporation. The resulting aqueous solution was cooled to 0 °C and acidified with 5 N hydrochloric acid (2 mL, pH = 0) which formed a white precipitate. The cool solution was filtered and the solid was rinsed with water. The white powder was collected and dried further *in vacuo* to give the title compound as a white powder (43.0 mg, 0.140 mmol, 94% yield). $^1$H NMR (400 MHz, $CD_3SOCD_3$) δ 7.35 (t, *J* = 72, 1 H), 8.63 (s, 1 H), 9.10 (s, 1 H), 9.46 (s, 1 H); LC-MS (LC-ES) M+H = 308.

**Intermediate 93**

**8-Chloro-7-ethoxyquinoline-3-carboxylic acid**

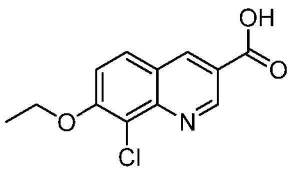

**A. Methyl 2-((3-chloro-4-ethoxy-2-fluorophenyl)(hydroxy)methyl)acrylate**

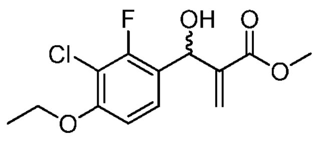

To a solution of 3-chloro-4-ethoxy-2-fluorobenzaldehyde (0.940 g, 4.64 mmol) in 1,4-dioxane:water (1:1) (0.1 mL) was added methyl acrylate (1.28 mL, 13.9 mmol) and 1,4-diazabicyclo[2.2.2]octane (0.520 g, 4.64 mmol). The reaction was monitored by TLC (8:2 hexanes:ethyl acetate) and LC-MS. Upon completion (96 h), water was added and poured into a separatory funnel and extracted with dichloromethane (3X, 30 mL), dried over anhydrous sodium sulfate, filtered, and concentrated under reduced pressure. Through reverse phase chromatography (Waters Sunfire 30X150 mm acetonitrile:water w/0.1% TFA 30-70%) the title compound was obtained as a pink oil (276 mg, 0.956 mmol, 20.6% yield). $^1$H NMR (400 MHz, $CD_3SOCD_3$) $\delta$ 1.36 (t, *J* = 7 Hz, 3 H), 3.61 (s, 3 H), 4.13 (q, *J* = 7 Hz, 2 H), 5.65 (d, *J* = 5 Hz, 1 H), 5.89 (d, *J* = 5 Hz, 1 H), 6.00 (s, 1 H), 6.29 (s, 1 H), 6.97 (d, *J* = 9 Hz, 1 H), 7.16 (t, *J* = 9 Hz, 1 H); LC-MS (LC-ES) M+H-$H_2O$ = 271.

**B. Methyl 2-(acetoxy(3-chloro-4-ethoxy-2-fluorophenyl)methyl)acrylate**

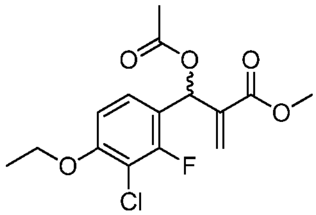

To a solution of methyl 2-((3-chloro-4-ethoxy-2-fluorophenyl)(hydroxy)methyl)acrylate (272 mg, 0.942 mmol) in dichloromethane (5 mL) was added acetic anhydride (0.116 mL, 1.23 mmol) and N,N-dimethylpyridin-4-amine (5.76 mg, 0.0470 mmol). The reaction mixture was stirred at room temperature until complete conversion, monitoring by TLC (8:2 hexanes:ethyl acetate, 2 h) and LC-MS. At that time, the reaction mixture was quenched with water (15 mL), transferred to a separatory funnel and extracted with dichloromethane (15 mL, 3X). Organic fractions were collected, dried over sodium sulfate, concentrated *in vacuo* and subsequently loaded onto an ISCO (12 g) column for silica gel chromatography (12 g column, hexanes:ethyl acetate (9:1 to 7:3)). Pure fractions were collected, concentrated to dryness under reduced pressure, and dried further on high vacuum, to give the title compound as a clear oil (263 mg, 0.795 mmol, 84% yield). $^1$H NMR (400 MHz, $CD_3SOCD_3$) $\delta$ 1.37 (t, *J* = 7 Hz, 3 H), 2.10 (s, 3 H), 3.67 (s, 3 H), 4.16 (q, *J* = 7 Hz, 2 H), 5.97 (d, *J* = 1 Hz, 1 H), 6.41 (s, 1 H), 6.68 (s, 1 H), 7.02 (dd, *J* = 9, 1 Hz, 1 H), 7.24 (t, *J* = 9 Hz, 1 H); LC-MS (LC-ES) M-OAc = 271.

**C. (E)-Methyl 2-(azidomethyl)-3-(3-chloro-4-ethoxy-2-fluorophenyl)acrylate**

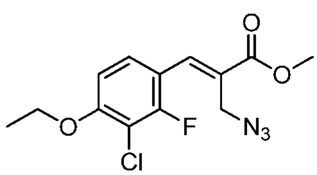

To a stirring tan solution of methyl 2-(acetoxy(3-chloro-4-ethoxy-2-fluorophenyl)methyl)acrylate (272 mg, 0.822 mmol) and N,N-dimethylformamide (8 mL) at room temperature was added sodium azide (214 mg, 3.29 mmol) which formed a yellow suspension. The reaction was monitored by TLC (9:1 hexanes:ethyl acetate) and upon completion (20 min), the reaction was transferred to a separatory funnel with water (25 mL) and extracted with diethyl ether (25 mL, 4X). The organic fractions were collected, dried over sodium sulfate, and filtered. The filtrate was concentrated to a tan oil via evaporation, dissolved into minimal dichloromethane, and purified by chromatography (ISCO, silica gel, 9:1 hexanes:ethyl acetate). The pure fractions were collected and concentrated to dryness *in vacuo,* to give the title compound

as a tan oil (257 mg, 0.819 mmol, 100% yield). $^1H$ NMR (400 MHz, $CD_3SOCD_3$) δ 1.39 (t, *J* = 7 Hz, 3 H), 3.83 (s, 3 H), 4.17-4.28 (m, 4 H), 7.16 (d, *J* = 9 Hz, 1 H), 7.42 (t, *J* = 9 Hz, 1 H), 7.82 (s, 1 H); LC-MS (LC-ES) M+H-$N_2$ = 286.

**D. 8-Chloro-7-ethoxyquinoline-3-carboxylic acid**

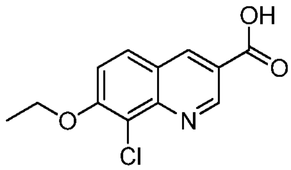

To a stirring solution of (E)-methyl 2-(azidomethyl)-3-(3-chloro-4-ethoxy-2-fluorophenyl)acrylate (0.250 g, 0.797 mmol) in tetrahydrofuran (5 mL) at room temperature was added polymer supported triphenylphosphine (0.402 g, 1.20 mmol) forming a brown suspension. The reaction was monitored by LC-MS and within 24 h, a trace amount of the desired amine was observed by LC-MS, but the corresponding methyl-8-chloro-7-ethoxy-3-carboxylate-quinoline and the methyl-8-chloro-7-ethoxy-3,4-dihydro-3-carboxylate-quinoline were present predominantly as seen by LC-MS. The reaction mixture was filtered at this point to remove phosphines, purified by chromatography (silica ISCO, gradient elution, hexanes:ethyl acetate), and dried to give the dihydro-quinoline as a fluorescent green solid, and the fully aromatized quinoline as a lightly fluorescent green/white powder. The white powder was contaminated with dihydroquinolines by $^1H$-NMR. The two compounds were combined and in an effort to push methyl-8-chloro-7-ethoxy-3,4-dihydro-3-carboxylate-quinoline to the methyl-8-chloro-7-ethoxy-3-carboxylate-quinoline, to the reaction was added iodine (125 mg, 0.491 mmol), potassium carbonate (67.8 mg, 0.491 mmol) and acetonitrile (3 mL). As the reaction converted the dihydroquinoline species to the quinoline, hydrolysis of the fully aromatized quinoline ester was observed as a side reaction. Also, the fluorescent solution faded to a pale yellow. Saponification continued slowly until the full conversion of dihydroquinoline to quinoline was witnessed by LC-MS, at which time, complete saponification was induced by adding 1 N sodium hydroxide (15.0 mL). Upon completion (45 min), the reaction mixture was concentrated to minimal volume via evaporation, cooled in an ice bath, and the pH was reduced to 0 with 5 N hydrochloric acid to give a white precipitation. The precipitate was filtered and processed without further purification, to give the title compound (190 mg, 0.755 mmol, 95% yield) as a white solid. $^1H$ NMR (400 MHz, $CD_3SOCD_3$) δ 1.44 (t, *J* = 7 Hz, 3 H), 4.39 (q, *J* = 7 Hz, 2 H), 7.76 (d, *J* = 9 Hz, 1 H), 8.22 (d, *J* = 9 Hz, 1 H), 8.98 (d, *J* = 2 Hz, 1 H), 9.34 (d, *J* = 2 Hz, 1 H), 13.49 (br s, 1 H); LC-MS (LC-ES) M +H = 252.

## Intermediate 94

**1-(4-Aminopiperidin-1-yl)-2-hydroxy-2-methylpropan-1-one**

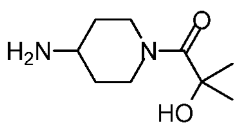

**A. Benzyl (1-(2-hydroxy-2-methylpropanoyl)piperidin-4-yl)carbamate**

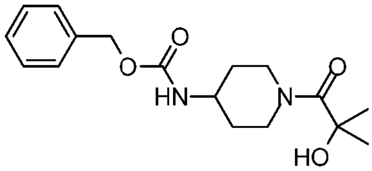

Benzyl piperidin-4-ylcarbamate (0.512 g, 2.183 mmol) was added to 2-hydroxy-2-methylpropanoic acid (0.2273 g, 2.183 mmol) in 1,4-dioxane (10.92 mL) at room temperature. Then, N,N-diisopropylethylamine (1.144 mL, 6.55 mmol)

was added and the reaction mixture was stirred for five minutes. Then, O-(7-azabenzotriazol-1-yl)-N,N,N',N'-tetramethyluronium hexafluorophosphate (0.830 g, 2.183 mmol) was added and the reaction mixture was stirred for sixteen hours. The reaction mixture was poured into saturated sodium bicarbonate, extracted with ethyl acetate (3X), dried over magnesium sulfate, filtered, and concentrated. The reaction mixture was purified by silica gel chromatography, eluting with ethyl acetate:hexanes (0:1 to 1:1) to give benzyl (1-(2-hydroxy-2-methylpropanoyl)piperidin-4-yl)carbamate (0.3151 g, 0.934 mmol, 42.8 % yield). $^1$H NMR (400 MHz, $CD_3SOCD_3$) δ 1.28 (s, 6 H), 1.28-1.36 (m, 2 H), 1.74 (d, *J* = 12 Hz, 2 H), 2.60-3.24 (m, 2 H), 3.48-3.62 (m, 1 H), 4.10-4.70 (m, 2 H), 5.00 (s, 2 H), 5.33 (s, 1 H), 7.26-7.40 (m, 6 H); LC-MS (LC-ES) M+H = 321.

**B. 1-(4-Aminopiperidin-1-yl)-2-hydroxy-2-methylpropan-1-one**

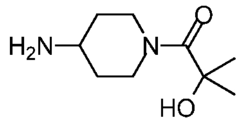

Palladium on carbon (0.105 g, 0.098 mmol) was added to benzyl (1-(2-hydroxy-2-methylpropanoyl)piperidin-4-yl)carbamate (0.3151 g, 0.984 mmol) in methanol (3.28 mL) at 25 °C under nitrogen atmosphere. Then, the reaction vessel was fitted with a hydrogen balloon and the vessel was repeatedly evacuated and purged with hydrogen, then stirred for sixteen hours. Then, the vessel was repeatedly evacuated and purged with nitrogen, filtered through Celite®, and concentrated to give 1-(4-aminopiperidin-1-yl)-2-hydroxy-2-methylpropan-1-one (0.1752g, 0.894 mmol, 91 % yield). $^1$H NMR (400 MHz, $CD_3SOCD_3$) δ 1.00-1.18 (m, 2 H), 1.29 (s, 6 H), 1.54 (br s, 2 H), 1.67 (d, *J* = 12 Hz, 2 H), 2.76 (p, *J* = 5 Hz, 1 H), 2.54-3.18 (m, 2 H), 4.02-4.72 (m, 2 H), 5.28 (s, 1 H); LC-MS (LC-ES) M+H = 187.

**Intermediate 95**

**1-(3-Aminoazetidin-1-yl)-2-hydroxy-2-methylpropan-1-one**

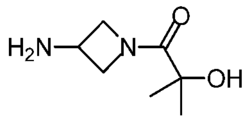

**A. Benzyl (1-(2-hydroxy-2-methylpropanoyl)azetidin-3-yl)carbamate**

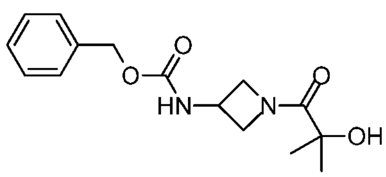

Benzyl azetidin-3-ylcarbamate (1.000 g, 4.85 mmol) was added to 2-hydroxy-2-methylpropanoic acid (0.505 g, 4.85 mmol) in 1,4-dioxane (24.25 mL) at room temperature. Then, N,N-diisopropylethylamine (2.54 mL, 14.55 mmol) was added and the reaction mixture was stirred for five minutes. Then, O-(7-azabenzotriazol-1-yl)-N,N,N',N'-tetramethyluronium hexafluorophosphate (1.844 g, 4.85 mmol) was added and the reaction mixture was stirred for six hours. The reaction mixture was poured into saturated sodium bicarbonate, extracted with ethyl acetate (3X), dried over magnesium sulfate, filtered, and concentrated. The reaction mixture was purified by silica gel chromatography, eluting with hexanes:ethyl acetate (1:1 to 0:1), then further purified by RP HPLC, eluting with acetonitrile:water with 0.1% ammonium hydroxide (5:95:100:0) to give benzyl (1-(2-hydroxy-2-methylpropanoyl)azetidin-3-yl)carbamate (0.6440 g, 2.093 mmol, 43.1 % yield). $^1$H NMR (400 MHz, $CD_3SOCD_3$) δ 1.22 (s, 6 H), 3.62-3.72 (m, 1 H), 4.03 (t, *J* = 8 Hz, 1 H), 4.12-4.22 (m, 1 H), 4.25 (q, *J* = 6 Hz, 1 H), 4.56 (t, *J* = 6 Hz, 1 H), 5.01 (s, 1 H), 5.02 (s, 2 H), 7.26-7.40 (m, 5 H), 7.92 (d, *J* = 6 Hz, 1

H); LC-MS (LC-ES) M+H = 293.

**B. 1-(3-Aminoazetidin-1-yl)-2-hydroxy-2-methylpropan-1-one**

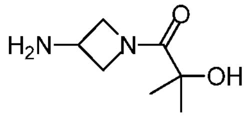

Palladium on carbon (0.234 g, 0.220 mmol) was added to benzyl (1-(2-hydroxy-2-methylpropanoyl)azetidin-3-yl)carbamate (0.6440 g, 2.203 mmol) in methanol (7.34 mL) at 25 °C under nitrogen atmosphere. Then, the reaction vessel was fitted with a hydrogen balloon and the vessel was repeatedly evacuated and purged with hydrogen, then stirred for sixteen hours. Then, the vessel was repeatedly evacuated and purged with nitrogen, filtered through Celite®, and concentrated to give 1-(3-aminoazetidin-1-yl)-2-hydroxy-2-methylpropan-1-one (0.3647 g, 2.190 mmol, 99 % yield). $^1$H NMR (400 MHz, $CD_3SOCD_3$) δ 1.22 (s, 6 H), 3.16 (d, *J* = 3 Hz, 2 H), 3.36-3.46 (m, 1 H), 3.61 (p, *J* = 7 Hz, 1 H), 3.86-3.94 (m, 1 H), 3.95 (t, *J* = 8 Hz, 1 H), 4.48 (t, *J* = 8 Hz, 1 H), 4.92 (s, 1 H); LC-MS (LC-ES) M+H = 159.

**Intermediate 96**

**6-Chloro-7-(2,2-difluorocyclopropyl)quinoline-3-carboxylic acid**

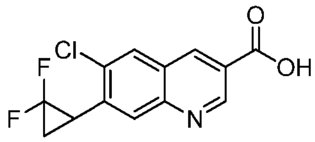

**A. Ethyl 6-chloro-7-(2,2-difluorocyclopropyl)quinoline-3-carboxylate**

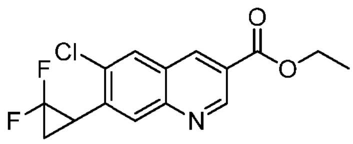

An oven-dried 20 mL microwave vial was charged with ethyl 6-chloro-7-vinylquinoline-3-carboxylate (94 mg, 0.359 mmol, intermediate 98A) and sodium iodide (10.77 mg, 0.072 mmol), sealed with a rubber septum and flushed with nitrogen. Tetrahydrofuran (1.437 mL) and trimethyl(trifluoromethyl)silane (2 M in tetrahydrofuran) (0.449 mL, 0.898 mmol) were added via syringe and the septum was replaced with a crimp top. The mixture was stirred in an oil bath at 65 °C for 2 h. Upon cooling the mixture was poured into water and extracted with ethyl acetate (3X). The combined organics were washed with water and brine, dried over sodium sulfate, and concentrated in vacuo. The residue was purified by flash chromatography (silica gel, ethyl acetate:hexanes (0:1 to 1:0)) to give ethyl 6-chloro-7-(2,2-difluorocyclopropyl)quinoline-3-carboxylate (26 mg, 0.083 mmol, 23.22 % yield) as an orange solid. $^1$H NMR (400 MHz, $CDCl_3$) δ 1.47 (t, *J* = 7 Hz, 3 H), 1.83-1.94 (m, 1 H), 1.98-2.10 (m, 1 H), 2.97-3.10 (m, 1 H), 4.49 (q, *J* = 7 Hz, 2 H), 7.58 (d, *J* = 10 Hz, 1 H), 7.97 (d, *J* = 7 Hz, 1 H), 8.75-8.80 (m, 1 H), 9.40 (d, *J* = 2 Hz, 1 H); LC-MS (LC-ES) M+H = 296.

**B. 6-Chloro-7-(2,2-difluorocyclopropyl)quinoline-3-carboxylic acid**

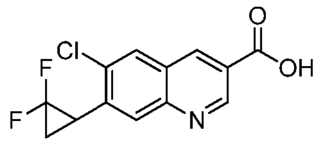

To a solution of ethyl 6-chloro-7-(2,2-difluorocyclopropyl)quinoline-3-carboxylate (26 mg, 0.083 mmol) in methanol (0.5 mL) and tetrahydrofuran (0.5 mL) was added a solution of sodium hydroxide (4 M in water) (0.042 mL, 0.167 mmol) slowly. The reaction was allowed to stir at room temperature and subsequently monitored by LC-MS. The solution was stripped down to the aqueous layer, acidified with 1 M hydrochloric acid, and extracted with ethyl acetate (3X, 5 mL). The organic layers were combined, dried over magnesium sulfate, filtered, and concentrated in vacuo to yield 24 mg of 6-chloro-7-(2,2-difluorocyclopropyl)quinoline-3-carboxylic acid as a light brown oil. LC-MS (LC-ES) M+H = 284.

**Intermediate 97**

**1-(4-Aminopiperazin-1-yl)-2-hydroxy-2-methylpropan-1-one hydrochloride**

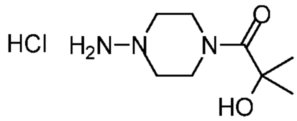

**A. tert-Butyl (4-(2-hydroxy-2-methylpropanoyl)piperazin-1-yl)carbamate**

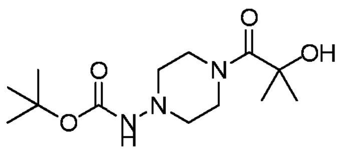

tert-Butyl piperazin-1-ylcarbamate (0.999 g, 4.96 mmol) was added to 2-hydroxy-2-methylpropanoic acid (0.5165 g, 4.96 mmol) in 1,4-dioxane (24.81 mL) at room temperature. Then, N,N-diisopropylethylamine (2.60 mL, 14.88 mmol) was added and the reaction mixture was stirred for five minutes. Then, O-(7-azabenzotriazol-1-yl)-N,N,N',N'-tetramethyluronium hexafluorophosphate (1.886 g, 4.96 mmol) was added and the reaction mixture was stirred for sixteen hours. The reaction mixture was poured into saturated sodium bicarbonate, extracted with ethyl acetate (2X), dried over magnesium sulfate, filtered, and concentrated. The reaction mixture was purified by RP HPLC, eluting with acetonitrile:water with 0.1% ammonium hydroxide (5:95:100:0) to give tert-butyl (4-(2-hydroxy-2-methylpropanoyl)piperazin-1-yl)carbamate (0.7090 g, 2.344 mmol, 47.2 % yield). $^1H$ NMR (400 MHz, $CD_3SOCD_3$) $\delta$ 1.29 (s, 6 H), 1.37 (s, 9 H), 2.67 (s, 4 H), 3.34-4.06 (m, 4 H), 5.38 (s, 1 H), 8.04 (br s, 1 H); LC-MS (LC-ES) M+H = 288.

**B. 1-(4-Aminopiperazin-1-yl)-2-hydroxy-2-methylpropan-1-one hydrochloride**

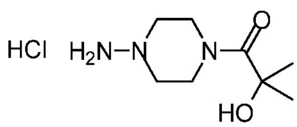

4.0 M Hydrochloric acid (0.431 mL, 1.723 mmol) in dioxane was added to tert-butyl (4-(2-hydroxy-2-methylpropanoyl)piperazin-1-yl)carbamate (0.1238 g, 0.431 mmol) in methanol (0.431 mL) at room temperature and the reaction mixture was stirred for sixteen hours. The reaction mixture was concentrated to give 1-(4-aminopiperazin-1-yl)-2-hydroxy-2-methylpropan-1-one hydrochloride (0.0963 g, 0.409 mmol, 95 % yield). $^1H$ NMR (400 MHz, $CD_3SOCD_3$) $\delta$ 1.30 (s, 6 H), 3.16 (t, $J$ = 13 Hz, 4 H), 3.46-4.24 (m, 4 H), 5.47 (s, 1 H), 9.58 (br s, 3 H); LC-MS (LC-ES) M+H = 188.

**Intermediate 98**

**6-Chloro-7-vinylquinoline-3-carboxylic acid**

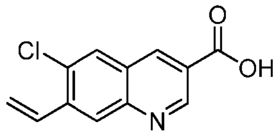

**A. Ethyl 6-Chloro-7-vinylquinoline-3-carboxylate**

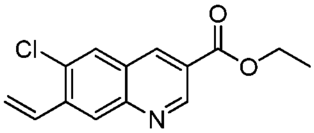

To a high pressure tube was added ethyl 7-bromo-6-chloroquinoline-3-carboxylate (Intermediate 4B) (500 mg, 1.590 mmol), potassium trifluoro(vinyl)borate (234 mg, 1.748 mmol), palladium (II) chloride (5.64 mg, 0.032 mmol), triphenylphosphine (25.01 mg, 0.095 mmol), and cesium carbonate (1554 mg, 4.77 mmol) in tetrahydrofuran (2.119 mL) and water (0.235 mL). The reaction was stirred and heated to 90 °C. Upon consumption of the starting material the reaction mixture was filtered through Celite® and rinsed with dichloromethane. The filtrate was concentrated to dryness *in vacuo* and purified via silica gel chromatography, eluting with ethyl acetate:hexanes (0:1 to 3:7) to give ethyl 6-chloro-7-vinylquinoline-3-carboxylate as a white solid (67 mg, 0.256 mmol, 16% yield). $^1$H NMR (400 MHz, $CD_3SOCD_3$) $\delta$ 1.40 (t, *J* = 7 Hz, 3 H), 4.43 (q, *J* = 7 Hz, 2 H), 5.69 (d, *J* = 12 Hz, 1 H), 6.23 (d, *J* = 17 Hz, 1 H), 7.19(dd, *J* = 17, 11 Hz, 1 H), 8.43 (d, *J* = 10 Hz, 2 H), 8.99 (d, *J* = 2 Hz, 1 H), 9.33 (d, *J* = 2 Hz, 1 H); LC-MS (LC-ES) M+H = 262.

**B. 6-Chloro-7-vinylquinoline-3-carboxylic acid**

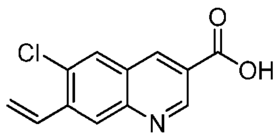

To a solution of ethyl 6-chloro-7-vinylquinoline-3-carboxylate (66 mg, 0.252 mmol) in methanol (1.261 mL) and tetrahydrofuran (1.261 mL) was added a solution of sodium hydroxide (4 M in water) (0.126 mL, 0.504 mmol) slowly. The reaction was stirred at room temperature and upon consumption of the starting material the reaction mixture was stripped down to the aqueous phase and acidified with 1 M hydrochloric acid. The precipitate was filtered via vacuum filtration to give 6-chloro-7-vinylquinoline-3-carboxylicacid as a white solid (40 mg, 0.149 mmol, 59% yield). $^1$H NMR (400 MHz, $CD_3SOCD_3$) $\delta$ 5.68 (d, *J* = 12 Hz, 1 H), 6.21 (d, *J* = 17 Hz, 1 H), 7.18 (dd, *J* = 17, 11 Hz, 1 H), 8.40 (d, *J* = 5 Hz, 2 H), 8.95 (d, *J* = 2 Hz, 1 H), 9.31 (d, *J* = 2 Hz, 1 H), 13.63 (br s, 1 H); LC-MS (LC-ES) M+H = 234.

**Intermediate 99**

**N-(trans-3-Aminocyclobutyl)-2-hydroxy-2-methylpropanamide hydrochloride**

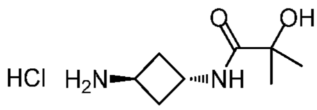

**A. tert-Butyl (trans-3-(2-hydroxy-2-methylpropanamido)cyclobutyl)carbamate**

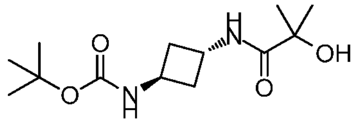

tert-Butyl (trans-3-aminocyclobutyl)carbamate (0.461 g, 2.474 mmol) was added to 2-hydroxy-2-methylpropanoic acid (0.2576 g, 2.474 mmol) in 1,4-dioxane (24.74 mL) at room temperature. Then, N,N-diisopropylethylamine (1.297 mL, 7.42 mmol) was added and the reaction mixture was stirred for five minutes. Then, O-(7-azabenzotriazol-1-yl)-N,N,N',N'-tetramethyluronium hexafluorophosphate (0.941 g, 2.474 mmol) was added and the reaction mixture was stirred for sixteen hours. The reaction mixture was poured into saturated sodium bicarbonate, extracted with ethyl acetate (2X), dried over magnesium sulfate, filtered, and concentrated. The reaction mixture was purified by RP HPLC, eluting with acetonitrile:water with 0.1% ammonium hydroxide (5:95:100:0) to give tert-butyl (trans-3-(2-hydroxy-2-methylpropanamido)cyclobutyl)carbamate (0.4201 g, 1.465 mmol, 59.2 % yield). $^1H$ NMR (400 MHz, $CD_3SOCD_3$) δ 1.20 (s, 6 H), 1.36 (s, 9 H), 2.06-2.14 (m, 2 H), 2.16-2.24 (m, 2 H), 3.94 (h, *J* = 7 Hz, 1 H), 4.18 (h, *J* = 8 Hz, 1 H), 5.29 (s, 1 H), 7.23 (d, *J* = 7 Hz, 1 H), 7.83 (d, *J* = 8 Hz, 1 H); LC-MS (LC-ES) M+H = 273.

**B. N-(trans-3-Aminocyclobutyl)-2-hydroxy-2-methylpropanamide hydrochloride**

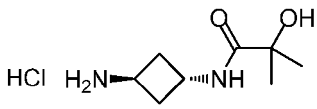

4.0 M Hydrochloric acid (1.543 mL, 6.17 mmol) in dioxane was added to tert-butyl (trans-3-(2-hydroxy-2-methylpropanamido)cyclobutyl)carbamate (0.4201 g, 1.543 mmol) in methanol (1.543 mL) at room temperature and the reaction mixture was stirred for sixteen hours. The reaction mixture was concentrated to give N-(trans-3-aminocyclobutyl)-2-hydroxy-2-methylpropanamide hydrochloride (0.3288 g, 1.497 mmol, 97 % yield). $^1H$ NMR (400 MHz, $CD_3SOCD_3$) δ 1.22 (s, 6 H), 2.24-2.42 (m, 4 H), 3.62-3.74 (m, 1 H), 4.49 (h, *J* = 8 Hz, 1 H), 5.29 (br s, 1 H), 8.04 (d, *J* = 8 Hz, 1 H), 8.13 (br s, 3 H); LC-MS (LC-ES) M+H = 173.

**Intermediate 100**

**Methyl 6-chloro-7-(difluoromethoxy)-8-methylquinoline-3-carboxylate trifluoroacetate and Methyl 8-chloro-7-(difluoromethoxy)-6-methylquinoline-3-carboxylate trifluoroacetate**

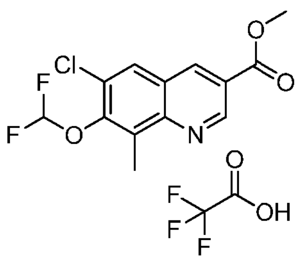

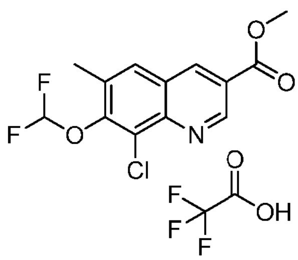

**A. 3-Chloro-4-(difluoromethoxy)-5-methylbenzaldehyde**

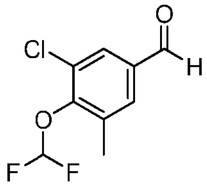

To a suspension of cesium carbonate (13.0 g, 40.0 mmol) in N,N-dimethylformamide (14.0 mL), heated at 90 °C, was added slowly, via a dropping funnel, a solution of 3-chloro-4-hydroxy-5-methylbenzaldehyde (1.00 g, 5.86 mmol) and sodium 2-chloro-2,2-difluoroacetate (1.79 g, 11.7 mmol) in N,N-dimethylformamide (7 mL) over ~1 h. After addition was complete, the reaction was allowed to stir for an additional 2 h, while monitoring via LC-MS. When the product peak began to decrease in relative peak area (<3 h) the reaction was cooled to room temperature, water (75 mL) was added slowly to the reaction mixture and some precipitation occurred. The reaction mixture was transferred to a separatory funnel and extracted with ethyl acetate (50 mL, 3X). The organic phase was washed with 1 N sodium hydroxide (2X), water, and brine. The combined aqueous phases were back-extracted with ethyl acetate. This ethyl acetate phase was washed with water and with brine. The organic phases were combined, dried over sodium sulfate, filtered and concentrated to a minimal volume via evaporation. The tan oil was purified further via silica gel chromatography (24 g ISCO, silica, ethyl acetate:hexanes (0-30%)), pure fractions were collected and concentrated to dryness *in vacuo,* to give the title compound as a white powder (1.00 g, 4.53 mmol, 77% yield). $^1$H NMR (400 MHz, $CD_3SOCD_3$) $\delta$ 2.39 (s, 3 H), 7.18 (t, *J* = 72 Hz, 1 H), 7.88 (d, *J* = 1 Hz, 1 H), 7.99 (d, *J* = 2 Hz, 1 H), 9.97 (s, 1 H); LC-MS (LC-ES) M +H = *no ion,* 100% peak area, T = 0.92 min.

**B. Methyl 2-((3-chloro-4-(difluoromethoxy)-5-methylphenyl)(hydroxy)methyl)acrylate**

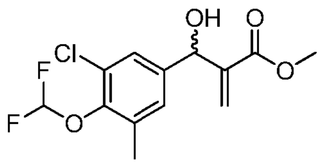

To a solution of 3-chloro-4-(difluoromethoxy)-5-methylbenzaldehyde (0.950 g, 4.31 mmol) in 1,4-dioxane:water (1:1) (0.100 mL) was added 1,4-diazabicyclo[2.2.2]octane (0.483 g, 4.31 mmol) and methyl acrylate (1.18 mL, 12.9 mmol). The reaction was monitored by TLC (8:2 hexanes:ethyl acetate). Upon completion (16 h), water (10 mL) was added, the reaction was transferred to a separatory funnel and extracted with dichloromethane (3X, 30 mL). The organic fractions were dried over anhydrous sodium sulfate, and filtered. The filtrate was concentrated to minimal volume and purified by chromatography (silica-gel, 40 g ISCO, hexanes:ethyl acetate (8:2 to 6:4)) to afford the desired compound as a clear oil (1.42 g, 3.57 mmol, 83% yield). $^1$H NMR (400 MHz, $CD_3SOCD_3$) $\delta$ 2.29 (s, 3 H), 3.64 (s, 3 H), 5.41 (d, *J* = 5 Hz, 1 H), 5.93 (d, *J* = 5 Hz, 1 H), 6.04 (s, 1 H), 6.25 (s, 1 H), 7.05 (t, *J* = 72 Hz, 1 H), 7.23 (d, *J* = 2 Hz, 1 H), 7.31 (d, *J* = 2 Hz, 1 H); LC-MS (LC-ES) M-$H_2O$ = 289.

**C. Methyl 2-(acetoxy(3-chloro-4-(difluoromethoxy)-5-methylphenyl)methyl)acrylate**

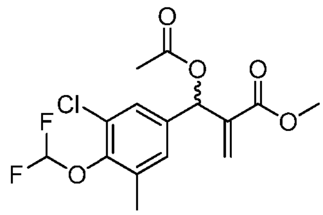

To a stirring solution of methyl 2-((3-chloro-4-(difluoromethoxy)-5-methylphenyl)(hydroxy)methyl)acrylate (1.00 g, 3.26 mmol) in dichloromethane (10.0 mL) was added acetic anhydride (0.369 mL, 3.91 mmol) followed by 4-dimethylaminopyridine (60.0 mg, 0.489 mmol). The reaction was monitored by TLC (9:1 hexanes:ethyl acetate) and upon completion (2 h), was quenched with water (10 mL). The mixture was then transferred to a separatory funnel and extracted with dichloromethane (25 mL, 3X). This extract was dried over sodium sulfate, concentrated to a minimal volume via evaporation, and purified by chromatography (ISCO system, 24 g silica gel cartridge, (9:1 to 8:2) hexanes:ethyl acetate). The pure fractions were collected and concentrated to dryness *in vacuo* to give the title compound as a clear oil (1.02 g, 2.92 mmol, 90% yield). $^1H$ NMR (400 MHz, $CD_3SOCD_3$) $\delta$ 2.11 (s, 3 H), 2.30 (s, 3 H), 3.69 (s, 3 H), 5.98 (d, *J* = 1 Hz, 1 H), 6.38 (s, 1 H), 6.46 (s, 1 H), 7.08 (t, *J* = 72 Hz, 1 H), 7.32 (d, *J* = 2 Hz, 1 H), 7.42 (d, *J* = 2 Hz, 1 H); LC-MS (LC-ES) M-OAc = 289.

**D. (E)-Methyl 2-(azidomethyl)-3-(3-chloro-4-(difluoromethoxy)-5-methylphenyl)acrylate**

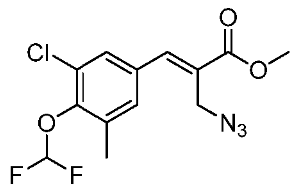

To a stirring solution of methyl 2-(acetoxy(3-chloro-4-(difluoromethoxy)-5-methylphenyl)methyl)acrylate (1.00 g, 2.87 mmol) in N,N-dimethylformamide (3 mL) was added sodium azide (0.196 g, 3.01 mmol). The reaction was monitored by TLC (9:1 hexanes:ethyl acetate) and upon completion (2 h), it was quenched with water (10 mL). The mixture was then transferred to a separatory funnel and extracted with dichloromethane (25 mL, 3X). This extract was dried over sodium sulfate, and concentrated to dryness with the aid of a heptane azeotrope. The residue was dissolved in dichloromethane and purified by chromatography (ISCO system, 24 g silica gel cartridge, (9:1 to 8:2) hexanes:ethyl acetate). The pure fractions were collected and concentrated to dryness *in vacuo* to give the title compound as a clear oil (943 mg, 2.76 mmol, 96% yield). $^1H$ NMR (400 MHz, $CD_3SOCD_3$) $\delta$ 2.34 (s, 3 H), 3.83 (s, 3 H), 4.23 (s, 2 H), 7.12 (t, *J* = 72 Hz, 1 H), 7.44 (s, 1 H), 7.58 (d, *J* = 2 Hz, 1 H), 7.85 (s, 1 H); LC-MS (LC-ES) M-$N_2$ = 304.

**E. (E)-Methyl 2-(aminomethyl)-3-(3-chloro-4-(difluoromethoxy)-5-methylphenyl)acrylate trifluoroacetate**

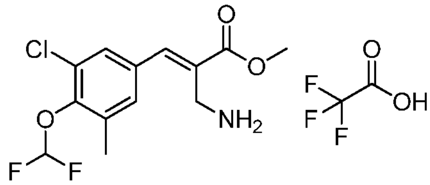

To a stirring solution of (E)-methyl 2-(azidomethyl)-3-(3-chloro-4-(difluoromethoxy)-5-methylphenyl)acrylate (0.78 g, 2.35 mmol) in tetrahydrofuran (15.0 mL) at reflux was added triphenylphosphine (0.678 g, 2.59 mmol) which formed a yellow suspension. The reaction was monitored by LC-MS and within 1 h the iminophosphorane intermediate was formed. With this, water (3 mL) was added which hydrolyzed the iminophosphorane to triphenylphosphine oxide and the desired free amine product (10 min). The reaction flask was concentrated to an oil. The oil was redissolved in minimal methanol and purified by chromatography under reverse phase conditions (C18 ISCO, gradient elution, water

(0.1%TFA:acetonitrile)). The pure fractions were collected and concentrated to dryness *in vacuo* to give the title compound as a clear oil (710 mg, 2.323 mmol, 99% yield). $^1$H NMR (400 MHz, $CD_3SOCD_3$) δ 2.35 (s, 3 H), 3.83 (m, 5 H), 7.12 (t, *J* = 72 Hz, 1 H), 7.47 (d, *J* = 2 Hz, 1 H), 7.66 (d, *J* = 2 Hz, 1 H), 7.89 (s, 1 H), 8.06 (br s, 3 H); LC-MS (LC-ES) M+H = 306.

**F. Methyl 6-chloro-7-(difluoromethoxy)-8-methylquinoline-3-carboxylate trifluoroacetate and Methyl 8-chloro-7-(difluoromethoxy)-6-methylquinoline-3-carboxylate trifluoroacetate**

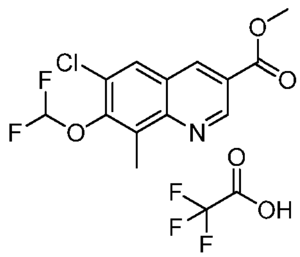

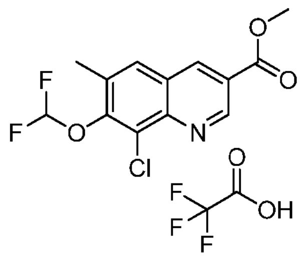

(E)-Methyl 2-(aminomethyl)-3-(3-chloro-4-(difluoromethoxy)-5-methylphenyl)acrylate trifluoroacetate (685 mg, 2.24 mmol) was dissolved into acetonitrile (3 mL) and stirred at room temperature. Iodine (2.28 mg, 8.96 mmol) was added to this stirring solution and allowed to stir for 5 minutes, before adding potassium carbonate (1.24 mg, 8.96 mmol). The reaction was monitored by TLC (9:1 hexanes:ethyl acetate) and LC-MS. Starting material had been converted to the respective quinoline in a roughly 1:1 regioisomeric ratio (overnight). The reaction was then transferred to a separatory funnel, diluted with ethyl acetate (50 mL), and washed with saturated sodium thiosulfate (3X, 25 mL) to remove iodine. The organic layer was dried over sodium sulfate, filtered, and concentrated to dryness via evaporation. The tan concentrate was purified by chromatography via HPLC (C18, water:acetonitrile, 1%TFA) and fractions containing regioisomers of desired compounds were separated and solvent was removed *in vacuo* to give the title compounds methyl 6-chloro-7-(difluoromethoxy)-8-methylquinoline-3-carboxylate (179 mg, 0.563 mmol, 26.5% yield) and methyl 8-chloro-7-(difluoromethoxy)-6-methylquinoline-3-carboxylate (170. mg, 0.564 mmol, 25.1% yield) as white powders.

**Methyl 6-chloro-7-(difluoromethoxy)-8-methylquinoline-3-carboxylate trifluoroacetate**

$^1$H NMR (400 MHz, $CD_3SOCD_3$) δ 2.75 (s, 3 H), 3.98 (s, 3 H), 7.23 (t, *J* = 72 Hz, 1 H), 8.47 (s, 1 H), 9.06 (d, *J* = 2 Hz, 1 H), 9.40 (d, *J* = 2 Hz, 1 H); LC-MS (LC-ES) M+H = 302.

**Methyl 8-chloro-7-(difluoromethoxy)-6-methylquinoline-3-carboxylate trifluoroacetate**

$^1$H NMR (400 MHz, $CD_3SOCD_3$) δ 2.50 (s, 3 H), 3.98 (s, 3 H), 7.29 (t, *J* = 72 Hz, 1 H), 8.20 (s, 1 H), 9.04 (d, *J* = 2 Hz, 1 H), 9.40 (d, *J* = 2 Hz, 1 H); LC-MS (LC-ES) M+H = 302.

**Intermediate 101**

**6-Chloro-7-(difluoromethoxy)-8-methylquinoline-3-carboxylic acid**

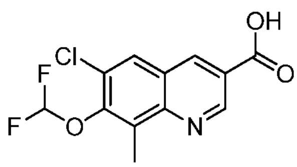

To methyl 6-chloro-7-(difluoromethoxy)-8-methylquinoline-3-carboxylate trifluoroacetate (178 mg, 0.590 mmol, Intermediate 100) was added acetone (5 mL) at room temperature. To the stirring solution was added 1 N sodium hydroxide (10 mL). The pale yellow solution was monitored by TLC (7:3 hexanes:ethyl acetate) for the disappearance of starting material, and upon completion (45 min), the majority of acetone was removed by evaporation. The resulting

aqueous solution was cooled to 0 °C and acidified to pH = 0 with 5 N hydrochloric acid (2 mL) which formed a white precipitate and clear supernatant. The cooled mixture was filtered and rinsed with water. The solid was collected and processed without further purification to give the title compound as a white powder (172 mg, 0.598 mmol, 100% yield). $^1$H NMR (400 MHz, $CD_3SOCD_3$) $\delta$ 2.75 (s, 3 H), 7.23 (t, *J* = 72 Hz, 1 H), 8.44 (s, 1 H), 9.00 (d, *J* = 2 Hz, 1 H), 9.39 (d, *J* = 2 Hz, 1 H), 13.72 (br s, 1 H); LC-MS (LC-ES) M+H = 288.

**Intermediate 102**

**8-Chloro-7-(difluoromethoxy)-6-methylquinoline-3-carboxylate**

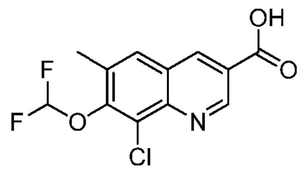

To methyl 8-chloro-7-(difluoromethoxy)-6-methylquinoline-3-carboxylate trifluoroacetate (171 mg, 0.567 mmol, Intermediate 100) was added acetone (5 mL) at room temperature. To the stirring solution was added 1 N sodium hydroxide (10.0 mL). The pale yellow solution was monitored by TLC (7:3 hexanes:ethyl acetate) for the disappearance of starting material, and upon completion (45 min), the majority of acetone was removed by evaporation. The resulting aqueous solution was cooled to 0 °C and acidified to pH = 0 with 5 N hydrochloric acid (2 mL) which formed a white precipitate with clear supernatant. The cooled mixture was filtered and rinsed with water. The solid was collected, dried further *in vacuo,* and processed without further purification to give the title compound as a white powder (164 mg, 0.570 mmol, 100% yield). $^1$H NMR (400 MHz, $CD_3SOCD_3$) $\delta$ 2.50 (s, 3 H), 7.28 (t, *J* = 72 Hz, 1 H), 8.18 (s, 1 H), 8.99 (d, *J* = 2 Hz, 1 H), 9.39 (d, *J* = 2 Hz, 1 H), 13.75 (br s, 1 H); LC-MS (LC-ES) M+H = 288.

**Intermediate 103**

**6-Chloro-7-(2-fluoropropan-2-yl)quinoline-3-carboxylic acid**

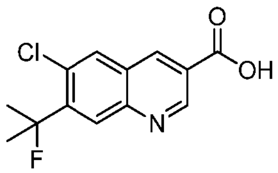

**A. Ethyl 7-acetyl-6-chloroquinoline-3-carboxylate**

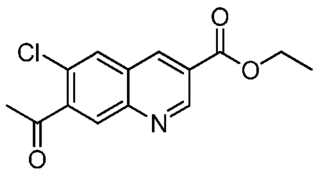

A mixture of ethyl 7-bromo-6-chloroquinoline-3-carboxylate (Intermediate 4B) (1.06 g, 3.37 mmol), tributyl(1-ethoxyvinyl)stannane (1.366 mL, 4.04 mmol), and $PdCl_2(PPh_3)_2$ (0.177 g, 0.253 mmol) in 1,4-dioxane (9.63 mL) was purged with nitrogen for 5 minutes. The mixture was then heated in a sealed vial for 90 minutes. Upon cooling, -20 mL of 1 M hydrochloric acid solution was added to the reaction vial and the resulting solution was stirred vigorously for 60 minutes. A saturated solution of potassium fluoride was then added to the mixture, which was further stirred for 45

minutes. Then, the reaction mixture was extracted with ethyl acetate (3X 20 mL) washed with water, potassium fluoride, and brine. The organics were combined, dried over sodium sulfate, filtered, and concentrated *in vacuo* to yield a yellow solid. The crude material was purified via silica gel chromatography, eluting with ethyl acetate:hexanes (0:1 to 3:2) to give ethyl 7-acetyl-6-chloroquinoline-3-carboxylate as a white solid (425 mg, 1.53 mmol, 45% yield). $^1$H NMR (400 MHz, $CD_3SOCD_3$) δ 1.40 (t, *J* = 7 Hz, 3 H), 2.73 (s, 3 H), 4.38-4.49 (m, 2 H), 8.46 (s, 1 H), 8.51 (s, 1 H), 9.06 (d, *J* = 2 Hz, 1 H), 9.40 (d, *J* = 2 Hz, 1 H); LC-MS (LC-ES) M+H = 279.

**B. Ethyl 6-chloro-7-(2-hydroxypropan-2-yl)quinoline-3-carboxylate**

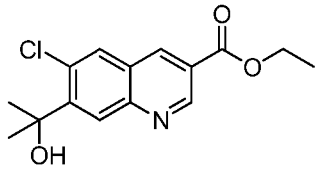

To a cooled solution at -41 °C of ethyl 7-acetyl-6-chloroquinoline-3-carboxylate (150 mg, 0.540 mmol) in tetrahydrofuran (27.00 mL) was slowly added methylmagnesium bromide (0.189 mL, 0.567 mmol). The solution was stirred and maintained between -41 and -50 °C using dry ice and acetonitrile. After 60 minutes, the solution was quenched with water, stripped down to the aqueous phase and an ethyl acetate (3X 20 mL) extraction was performed. The organics were combined, dried over magnesium sulfate, filtered and concentrated *in vacuo* to yield a crude light yellow solid. The crude mixture was purified via silica gel chromatography, eluting with ethyl acetate:hexanes (0:1 to 3:2) to give ethyl 6-chloro-7-(2-hydroxypropan-2-yl)quinoline-3-carboxylate as a white solid (69 mg, 0.235 mmol, 44% yield). $^1$H NMR (400 MHz, $CD_3SOCD_3$) δ 1.39 (t, *J* = 7 Hz, 3 H), 1.70 (s, 6 H), 4.42 (q, *J* = 7 Hz, 2 H), 5.65 (s, 1 H), 8.36 (s, 1 H), 8.52 (s, 1 H), 8.97(d, *J* = 2 Hz, 1 H), 9.32 (d, *J* = 2 Hz, 1 H); LC-MS (LC-ES) M+H = 294.

**C. 6-Chloro-7-(2-fluoropropan-2-yl)quinoline-3-carboxylic acid**

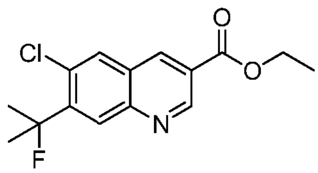

To a solution of ethyl 6-chloro-7-(2-hydroxypropan-2-yl)quinoline-3-carboxylate (29 mg, 0.099 mmol) at 0 °C in dichloromethane (3.000 mL) was slowly added (diethylamino)sulfur trifluoride (0.026 mL, 0.197 mmol). The solution was stirred for 15 minutes, slowly warmed to room temperature, and stirred overnight. The organics were washed with water and concentrated *in vacuo* to give ethyl 6-chloro-7-(2-fluoropropan-2-yl)quinoline-3-carboxylate as a light yellow solid. LC-MS (LC-ES) M+H = 296.

**D. 6-Chloro-7-(2-fluoropropan-2-yl)quinoline-3-carboxylic acid**

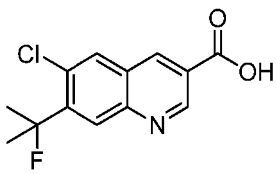

To a solution of ethyl 6-chloro-7-(2-fluoropropan-2-yl)quinoline-3-carboxylate (29 mg, 0.098 mmol) in methanol (1.000 mL) and tetrahydrofuran (1.000 mL) was slowly added sodium hydroxide (4 M solution) (0.074 mL, 0.294 mmol).

The solution was stripped down to the aqueous phase and acidified using 1 M hydrochloric acid. An ethyl acetate (3X, 3 mL) extraction was performed and the organics were washed with water, combined, dried over magnesium sulfate, filtered, and concentrated *in vacuo* to give 6-chloro-7-(2-fluoropropan-2-yl)quinoline-3-carboxylic acid as a white solid (25 mg, 0.093 mmol, 95% yield). LC-MS (LC-ES) M+H = 268.

**Intermediate 104**

**6-Chloro-7-(1,1-difluoroethyl)quinoline-3-carboxylic acid**

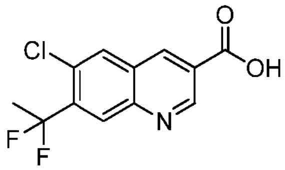

**A. Ethyl 6-chloro-7-(1,1-difluoroethyl)quinoline-3-carboxylate**

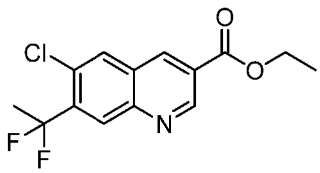

To ethyl 7-acetyl-6-chloroquinoline-3-carboxylate (Intermediate 103A) (100 mg, 0.360 mmol) in a small vial was added deoxofluor (0.133 mL, 0.720 mmol). The vial was purged quickly with nitrogen, capped, and heated to 95 °C overnight. The crude mixture was purified via silica gel chromatography, eluting with ethyl acetate:hexanes (0:1 to 1:4) to give ethyl 6-chloro-7-(1,1-difluoroethyl)quinoline-3-carboxylate as a white solid (26 mg, 0.087 mmol, 24% yield). While transferring the product to a vial, some was spilled on the bench top, accounting for the lower yield. $^1$H NMR (400 MHz, $CD_3SOCD_3$) $\delta$ 1.40 (t, *J* = 7 Hz, 3 H), 2.19 (t, *J* = 19 Hz, 3 H), 4.45 (q, *J* = 7 Hz, 2 H), 8.34 (s, 1 H), 8.59 (s, 1 H), 9.09 (d, *J* = 2 Hz, 1 H), 9.41 (d, *J* = 2 Hz, 1 H); LC-MS (LC-ES) M+H = 300.

**B. 6-Chloro-7-(1,1-difluoroethyl)quinoline-3-carboxylic acid**

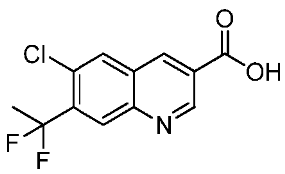

To a solution of ethyl 6-chloro-7-(1,1-difluoroethyl)quinoline-3-carboxylate (26 mg, 0.087 mmol) in methanol (1.000 mL) and tetrahydrofuran (1.000 mL) was slowly added sodium hydroxide (0.108 mL, 0.434 mmol). After 1 hour, the solution was acidified to a pH of ~5 using a 1 M hydrochloric acid solution. An ethyl acetate (3X, 5 mL) extraction was performed and the organics were washed once with water, combined, dried over magnesium sulfate, filtered, and concentrated *in vacuo* to give 6-chloro-7-(1,1-difluoroethyl)quinoline-3-carboxylic acid as a white solid (25 mg, 0.091 mmol, quant. yield). $^1$H NMR (400 MHz, $CD_3SOCD_3$) $\delta$ 2.19 (t, *J* = 19 Hz, 3 H), 8.32 (s, 1 H), 8.54 (s, 1 H), 9.01 (d, *J* = 2 Hz, 1 H), 9.40 (d, *J* = 2 Hz, 1 H); LC-MS (LC-ES) M+H = 272.

**Intermediate 105**

**(1s,4s)-4-Amino-1-(difluoromethyl)cyclohexan-1-ol**

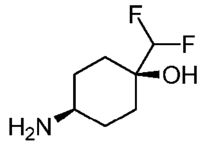

**A. (1s,4s)-4-(Dibenzylamino)-1-(difluoromethyl)cyclohexan-1-ol and (1r,4r)-4-(Dibenzylamino)-1-(difluoromethyl)cyclohexan-1-ol**

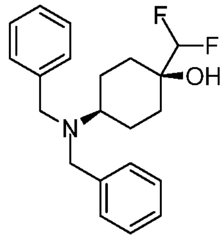

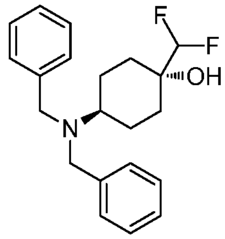

Cesium fluoride (0.155 g, 1.022 mmol) was added to 4-(dibenzylamino)cyclohexan-1-one (1 g, 3.41 mmol) and hexamethylphosphoramide (2.96 mL, 17.04 mmol) in tetrahydrofuran (8 mL). Then, (difluoromethyl)trimethylsilane (0.847 g, 6.82 mmol) was added. The resulting mixture was heated to reflux for 24 h at which time LC-MS showed a clean reaction with two peaks, the small peak pointed to HMPA while the major peak had the mw of 417, the intermediate of trimethylsilyl ether of the desired product. The mixture was cooled down a little bit (not quite room temperature yet) and tetrabutylammonium fluoride (3.41 mL, 3.41 mmol) was added and the mixture was stirred at room temperature for 1 h, then the mixture was poured into water (20 mL). The reaction mixture was extracted with ethyl acetate. The combined extracts were washed with water (2X) and brine, dried over sodium sulfate, filtered, and concentrated in vacuo. The residue was purified on an ISCO (80 g gold), eluting with 0 to 30% ethyl acetate:hexanes to afford (1s,4s)-4-(dibenzylamino)-1-(difluoromethyl)cyclohexan-1-ol (352 mg, 1.019 mmol, 29.9 % yield), which eluted first, and (1r,4r)-4-(dibenzylamino)-1-(difluoromethyl)cyclohexan-1-ol (560 mg, 1.621 mmol, 47.6 % yield), both white solids. The stereochemistry was confirmed with 2D NMR and NOE.

**(1s,4s)-4-(Dibenzylamino)-1-(difluoromethyl)cyclohexan-1-ol**

$^1$H NMR (400 MHz, $CDCl_3$) $\delta$ 1.38-1.48 (m, 2 H), 1.72-1.82 (m, 2 H), 1.82-1.90 (m, 4 H), 2.50-2.60 (m, 1 H), 3.69 (s, 4 H), 5.44 (t, *J* = 56 Hz, 1 H), 7.23 (t, *J* = 7 Hz, 2 H), 7.31 (t, *J* = 7 Hz, 4 H), 7.39 (d, *J* = 7 Hz, 4 H); LC-MS (LC-ES) M+H = 346.

**(1r,4r)-4-(Dibenzylamino)-1-(difluoromethyl)cyclohexan-1-ol**

$^1$H NMR (400 MHz, $CDCl_3$) $\delta$ 1.42-1.48 (m, 2 H), 1.62-1.74 (m, 2 H), 1.82-1.92 (m, 2 H), 2.04-2.12 (m, 2 H), 2.68-2.76 (m, 1 H), 3.66 (s, 4 H), 5.77 (t, *J* = 56 Hz, 1 H), 7.24 (t, *J* = 7 Hz, 2 H), 7.31 (t, *J* = 7 Hz, 4 H), 7.35 (d, *J* = 7 Hz, 4 H); LC-MS (LC-ES) M+H = 346.

**B. (1s,4s)-4-Amino-1-(difluoromethyl)cyclohexan-1-ol**

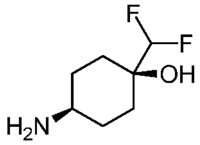

(1s,4s)-4-(Dibenzylamino)-1-(difluoromethyl)cyclohexan-1-ol (330 mg, 0.955 mmol) was dissolved in ethanol (15 mL), palladium hydroxide on carbon (20%, 168 mg, 0.239 mmol) was added and the mixture was degassed under hydrogen balloon (3X) and then stirred under the hydrogen balloon overnight (16 h) at which time LC-MS showed the disappearance of the starting material. The mixture was filtered through Celite® and washed with plenty of methanol. The filtrate was concentrated in vacuo to afford (1s,4s)-4-amino-1-(difluoromethyl)cyclohexan-1-ol (137 mg, 0.829 mmol, 87 % yield) as a white solid. $^1$H NMR (400 MHz, $CD_3OD$) δ 1.44-1.64 (m, 4 H), 1.66-1.84 (m, 4 H), 2.56-2.74 (m, 1 H), 5.52 (t, *J* = 56 Hz, 1 H); LC-MS (LC-ES) M+H = 166.

**Intermediate 106**

**(1r,4r)-4-Amino-1-(difluoromethyl)cyclohexan-1-ol**

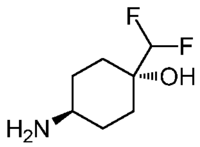

(1r,4r)-4-(Dibenzylamino)-1-(difluoromethyl)cyclohexan-1-ol (425 mg, 1.230 mmol) was dissolved in ethanol (7 mL), palladium hydroxide on carbon (20%, 216 mg, 0.308 mmol) was added and the mixture was degassed under hydrogen balloon (3X) and then stirred under the hydrogen balloon overnight (18 h) at which time LC-MS showed the disappearance of the starting material. The mixture was filtered through Celite® and washed with plenty of ethanol. The filtrate was concentrated in vacuo to afford (1r,4r)-4-amino-1-(difluoromethyl)cyclohexan-1-ol (185 mg, 1.120 mmol, 91 % yield) as a greenish solid. $^1$H NMR (400 MHz, $CD_3OD$) δ 1.44-1.54 (m, 4 H), 1.82-1.96 (m, 4 H), 2.96-3.04 (m, 1 H), 5.67 (t, *J* = 56 Hz, 1 H); LC-MS (LC-ES) M+H = 166.

**Intermediate 107**

**Lithium 6-chloro-7-(difluoromethoxy)-8-fluoroquinoline-3-carboxylate**

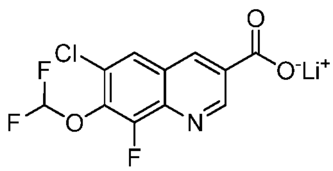

**A. 3-Chloro-4-(difluoromethoxy)-5-fluorobenzaldehyde**

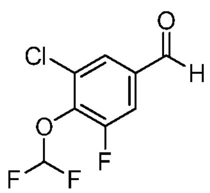

To a suspension of cesium carbonate (5.70 g, 17.5 mmol) in N,N-dimethylformamide (7 mL), heated at 100 °C, was added slowly, via a dropping funnel, a solution of 3-chloro-5-fluoro-4-hydroxybenzaldehyde (1.02 g, 5.83 mmol) and sodium 2-chloro-2,2-difluoroacetate (1.78 g, 11.7 mmol) in N,N-dimethylformamide (7 mL) over ~2 h. After addition was complete, heating was continued for another ~7 h. Monitoring by LC-MS showed 36% product and 38% starting material and the emergence of side products after the time allotment. The reaction was cooled and water (75 mL) was added slowly to the reaction mixture. The reaction mixture was then transferred to a separatory funnel and extracted with ethyl acetate (50 mL, 3X). The organic phase was collected and washed (75 mL) with 1 N hydrochloric acid, water and brine. The combined aqueous phases were back-extracted with ethyl acetate (100 mL). This organic phase was washed with water and brine. The organic phases were combined, dried over sodium sulfate, filtered and concentrated via evaporation. The tan oil was purified by silica gel chromatography (12 g ISCO column) eluting with 9:1 hexanes:ethyl acetate, to give the title compound as a tan oil (520 mg, 2.32 mmol, 39.7% yield). $^1$H NMR (400 MHz, $CD_3SOCD_3$) δ 7.34 (t, *J* = 72 Hz, 1 H), 7.96 (dd, *J* = 10, 2 Hz, 1 H), 8.06 (t, *J* = 2 Hz, 1 H), 9.97 (d, *J* = 2 Hz, 1 H).

**B. Methyl 2-((3-chloro-4-(difluoromethoxy)-5-fluorophenyl)(hydroxy)methyl)acrylate**

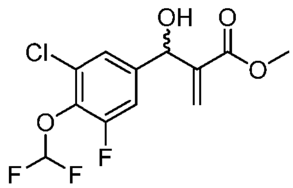

To a solution of 3-chloro-4-(difluoromethoxy)-5-fluorobenzaldehyde (0.510 g, 2.27 mmol) in 1,4-dioxane:water (1:1) (1 mL) was added methyl acrylate (0.624 mL, 6.81 mmol) and 1,4-diazabicyclo[2.2.2]octane (0.255 g, 2.27 mmol). The reaction was monitored by TLC (8:2 hexanes:ethyl acetate). Upon completion (24 h), water (7 mL) was added and poured into a separatory funnel and extracted with dichloromethane (3X, 30 mL), dried over anhydrous sodium sulfate, filtered, and concentrated under reduced pressure. The crude material was purified through chromatography (silica-gel, 12 g ISCO, hexanes:ethyl acetate (8:2 to 6:4)) to afford the title compound as a clear oil (558 mg, 1.796 mmol, 79% yield). $^1$H NMR (400 MHz, $CD_3SOCD_3$) δ 3.65 (s, 3 H), 5.46 (d, *J* = 5 Hz, 1 H), 6.05 (s, 1 H), 6.11 (d, *J* = 5 Hz, 1 H), 6.27 (s, 1 H), 7.32 (t, *J* = 72 Hz, 1 H), 7.33 (dd, *J* = 11, 2, 1 H), 7.37 (t, *J* = 2 Hz, 1 H), LC-MS (LC-ES) M+H-$H_2O$ = 293.

**C. Methyl 2-(acetoxy(3-chloro-4-(difluoromethoxy)-5-fluorophenyl)methyl)acrylate**

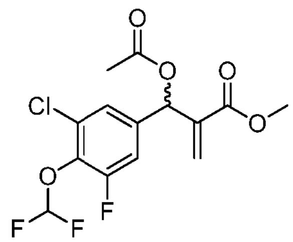

To a stirring solution of methyl 2-((3-chloro-4-(difluoromethoxy)-5-fluorophenyl)(hydroxy)methyl)acrylate (467 mg, 1.50 mmol) in dichloromethane (8 mL) was added acetic anhydride (0.213 mL, 2.26 mmol), followed by 4-dimethylaminopyridine (18.4 mg, 0.150 mmol). The reaction was monitored by TLC (9:1 hexanes:ethyl acetate) and upon completion (2 h), water (10 mL) was added. The mixture was then transferred to a separatory funnel and extracted with dichloromethane (25 mL, 3X). The extract was dried over sodium sulfate, filtered, and concentrated to a minimal volume via evaporation. The concentrate was then purified by chromatography (ISCO system, 24 g silica gel, (9:1 to 8:2) hexanes:ethyl acetate) and the pure fractions were collected and concentrated to dryness *in vacuo* to give the title compound as a clear oil (425 mg, 1.21 mmol, 80% yield). $^1$H-NMR (400 MHz, $CD_3SOCD_3$) δ 2.13 (s, 3 H), 3.70 (s, 3 H), 5.99 (d, *J* = 1 Hz, 1 H), 6.39 (s, 1 H), 6.49 (s, 1 H), 7.23 (t, *J* = 72 Hz, 1 H), 7.45-7.52 (m, 2 H); LC-MS (LC-ES) M-OAc = 293.

**D. (E)-Methyl 2-(azidomethyl)-3-(3-chloro-4-(difluoromethoxy)-5-fluorophenyl)acrylate**

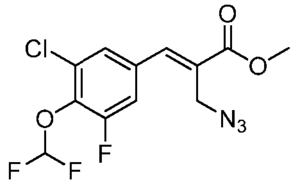

To a stirring light yellow solution of methyl 2-(acetoxy(3-chloro-4-(difluoromethoxy)-5-fluorophenyl)methyl)acrylate (425 mg, 1.21 mmol) in N,N-dimethylformamide (4 mL) at room temperature, was added sodium azide (157 mg, 2.41 mmol) which formed a suspension that quickly took on a bright yellow color. The reaction was followed by TLC (9:1 hexanes:ethyl acetate) and upon completion (28 min) the yellow solution was transferred to a separatory funnel with water (25 mL) and extracted with diethyl ether (25 mL, 4X). The organic fractions were collected, dried over sodium sulfate, and filtered. The filtrate was concentrated to an oil via evaporation, dissolved into minimal dichloromethane and purified by chromatography (ISCO, silica gel, (9:1 hexanes:ethyl acetate)). The pure fractions were collected and concentrated to dryness *in vacuo* to give the title compound as a clear oil (410. mg, 1.22 mmol, 101 % yield). $^1H$ NMR (400 MHz, $CD_3SOCD_3$) δ 3.83 (s, 3 H), 4.24 (s, 2 H), 7.27 (t, *J* = 72 Hz, 1 H), 7.58 (dd, *J* = 10, 2 Hz, 1 H), 7.61 (s, 1 H), 7.85 (s, 1 H).

**E. (E)-Methyl 2-(aminomethyl)-3-(3-chloro-4-(difluoromethoxy)-5-fluorophenyl)acrylate trifluoroacetate**

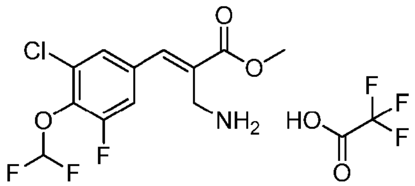

To a stirring solution of (E)-methyl 2-(azidomethyl)-3-(3-chloro-4-(difluoromethoxy)-5-fluorophenyl)acrylate (394 mg, 1.17 mmol) in tetrahydrofuran (15.0 mL) at reflux was added triphenylphosphine (314 mg, 1.20 mmol) which formed a clear solution. The reaction was monitored by LC-MS and within 1 h the full conversion of the parent azide to the iminophosphorane intermediate was observed. With this, water (3 mL) was added to the refluxing solution to hydrolyze the iminophosphorane to triphenylphosphine oxide and the desired free amine product (15 min). The reaction mixture was then concentrated to minimal volume. The resulting concentrate was purified by chromatography under reverse phase conditions (C18 ISCO, 100 g, gradient elution, water (0.1%TFA:acetonitrile)). Pure fractions were collected and concentrated to dryness to give the title compound as a clear oil (294 mg, 0.949 mmol, 81% yield). $^1H$ NMR (400 MHz, $CD_3SOCD_3$) δ 3.83 (m, 5 H), 7.29 (t, *J* = 72 Hz, 1 H), 7.69 (m, 2 H), 7.90 (s, 1 H), 8.04 (br s, 3 H); LC-MS (LC-ES) M+H = 310.

**F. Methyl 8-chloro-7-(difluoromethoxy)-6-fluoro-quinoline-3-carboxylate and Methyl 6-chloro-7-(difluoromethoxy)-8-fluoro-quinoline-3-carboxylate**

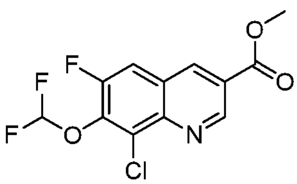

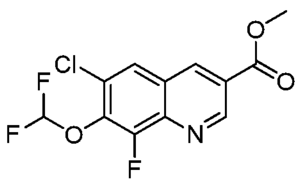

(E)-Methyl-2-(aminomethyl)-3-(3-chloro-4-(difluoromethoxy)-5-fluorophenyl)acrylate (294 mg, 0.949 mmol) was dissolved into acetonitrile (3 mL) and stirred at room temperature. Iodine (2.28 g, 8.96 mmol) was added to this stirring solution and allowed to stir for 5 minutes, before adding potassium carbonate (1.24 mg, 8.96 mmol). The reaction was monitored by TLC (9:1 hexanes:ethyl acetate) and LC-MS. Within 40 minutes, starting material had been converted

to the respective quinoline in a roughly 1:1 regioisomeric ratio. The reaction was then transferred to a separatory funnel, diluted with ethyl acetate (50 mL), and washed with saturated sodium thiosulfate (3X, 25 mL) to remove iodine. The organic layer was dried over sodium sulfate, filtered, and concentrated to dryness via evaporation. The tan concentrate was purified by HPLC chromatography (C18, Sunfire, water:acetonitrile, 1% TFA) and fractions containing regioisomers of desired compounds were separated and concentrated to dryness to give methyl 8-chloro-7-(difluoromethoxy)-6-fluoroquinoline-3-carboxylate (41 mg, 0.134 mmol, 14.1% yield) and methyl 6-chloro-7-(difluoromethoxy)-8-fluoroquinoline-3-carboxylate (45 mg, 0.147 mmol, 15.5% yield) as pure white powders.

**Methyl 8-chloro-7-(difluoromethoxy)-6-fluoro-quinoline-3-carboxylate**

$^1$H NMR (400 MHz, $CD_3SOCD_3$) δ 3.97 (s, 3 H), 7.39 (t, *J* = 72 Hz, 1 H), 8.34 (d, *J* = 11 Hz, 1 H), 9.13 (d, *J* = 2 Hz, 1 H), 9.42 (d, *J* = 2 Hz, 1 H); LC-MS (LC-ES) M+H = 306.

**Methyl 6-chloro-7-(difluoromethoxy)-8-fluoro-quinoline-3-carboxylate**

$^1$H NMR (400 MHz, $CD_3SOCD_3$) δ 3.97 (s, 3 H), 7.40 (t, *J* = 72 Hz, 1 H), 8.47 (d, *J* = 2 Hz, 1 H), 9.11 (t, *J* = 2 Hz, 1 H), 9.40 (d, *J* = 2 Hz, 1 H); LC-MS (LC-ES) M+H = 306.

**G. Lithium 6-chloro-7-(difluoromethoxy)-8-fluoroquinoline-3-carboxylate**

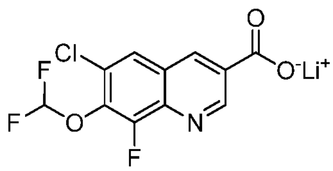

Lithium hydroxide (10.58 mg, 0.442 mmol) was added to methyl 6-chloro-7-(difluoromethoxy)-8-fluoroquinoline-3-carboxylate (0.0450 g, 0.147 mmol) in methanol (2.356 mL) and water (0.589 mL) at room temperature and the reaction mixture was stirred two hours at 60 °C. The reaction mixture was concentrated to give lithium 6-chloro-7-(difluoromethoxy)-8-fluoroquinoline-3-carboxylate (0.0437 g, 0.140 mmol, 95 % yield). $^1$H NMR (400 MHz, $CD_3SOCD_3$) δ 7.33 (t, *J* = 72 Hz, 1 H), 8.25 (d, *J* = 2 Hz, 1 H), 8.68 (t, *J* = 2 Hz, 1 H), 9.37 (d, *J* = 2 Hz, 1 H); LC-MS (LC-ES) M+H = 292.

**Intermediate 108**

**Lithium 8-chloro-7-(difluoromethoxy)-6-fluoroquinoline-3-carboxylate**

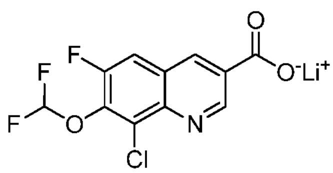

Lithium hydroxide (9.64 mg, 0.402 mmol) was added to methyl 8-chloro-7-(difluoromethoxy)-6-fluoroquinoline-3-carboxylate (0.0410 g, 0.134 mmol) in methanol (2.146 mL) and water (0.537 mL) at room temperature and the reaction mixture was stirred three hours at 60 °C. The reaction mixture was concentrated to give lithium 8-chloro-7-(difluoromethoxy)-6-fluoroquinoline-3-carboxylate (0.0409 g, 0.131 mmol, 97 % yield). $^1$H NMR (400 MHz, $CD_3SOCD_3$) δ 7.32 (t, *J* = 72 Hz, 1 H), 8.13 (d, *J* = 11 Hz, 1 H), 8.70 (d, *J* = 2 Hz, 1 H), 9.36 (d, *J* = 2 Hz, 1 H); LC-MS (LC-ES) M+H = 292.

## EXAMPLES

### Example 1

**7-Methoxy-N-(1-(1-methyl-1*H*-tetrazo)-5-yl)piperidin-4-yl)quinoline-3-carboxamide**

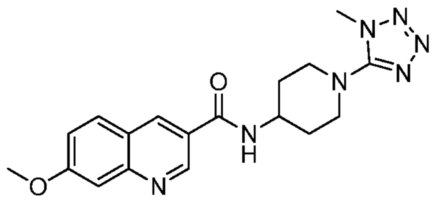

A solution of 7-methoxyquinoline-3-carboxylic acid (Intermediate 1) (250 mg, 1.230 mmol) and HATU (561 mg, 1.476 mmol) in DMF (4 mL) was treated with DIEA (0.643 mL, 3.69 mmol) and the reaction mixture was stirred at 21 °C for 3 min. 1-(1-Methyl-1*H*-tetrazol-5-yl)piperidin-4-amine hydrochloride (Intermediate 39) (269 mg, 1.476 mmol) was added and the reaction mixture was stirred at 21 °C for 12 h. The reaction mixture was concentrated *in vacuo* and the residue was dissolved in DCM, loaded onto an aminopropyl ($NH_2$) ion-exchange SPE cartridge (10 g) and eluted with 1:1 DCM/MeOH. The eluent was concentrated *in vacuo* and the residue was purified by column chromatography on silica gel using a 0-10 % MeOH in DCM gradient. Appropriate fractions were combined and evaporated to give the title compound (248 mg). $^1H$ NMR (400 MHz, $CDCl_3$) $\delta$ 1.76 - 1.95 (m, 2 H) 2.10 - 2.26 (m, 2 H) 3.17 - 3.37 (m, 2 H) 3.68 (d, *J* = 13 Hz, 2 H) 3.90 (s, 3 H) 3.97 (s, 3 H) 4.19 - 4.42 (m, 1 H) 6.74 (d, *J* = 8 Hz, 1 H) 7.25 (dd, *J* = 9, 2 Hz, 1 H) 7.42 (d, *J* = 2 Hz, 1 H) 7.81 (d, *J* = 9 Hz, 1 H) 8.57 (d, *J* = 2 Hz, 1 H) 9.22 (d, *J* = 2 Hz, 1 H). LCMS ES+ve *m/z* 368 $[M+H]^+$.

### Example 2

**(S)-7-Methoxy-N-(2-oxopyrrolidin-3-yl)quinoline-3-carboxamide**

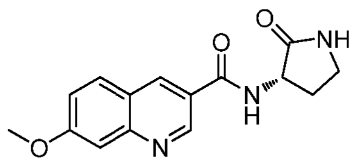

A solution of 7-methoxyquinoline-3-carboxylic acid (Intermediate 1) (100 mg, 0.492 mmol) in anhydrous DMF (2 mL) was treated with HATU (168 mg, 0.443 mmol) and stirred at 21 °C for 3 min prior to the addition of DIEA (0.193 mL, 1.107 mmol). The reaction mixture was then stirred at 21 °C for 3 min prior to the addition of 3-aminopyrrolidin-2-one (40.6 mg, 0.406 mmol). The reaction mixture was stirred at 21 °C for 2 h. The solvent was evaporated under a stream of nitrogen and the residue was purified by Mass-Directed Auto-Preparative HPLC (Waters SunFire C18 column, $CH_3CN$ / Water + 0.1% formic acid) to give 7-Methoxy-N-(2-oxopyrrolidin-3-yl)quinoline-3-carboxamide (56 mg). LCMS ES+ve m/z 286 $[M+H]^+$. 7-Methoxy-N-(2-oxopyrrolidin-3-yl)quinoline-3-carboxamide (45 mg, 0.157mmol) was resolved by preparative chiral HPLC on a Daicel CHIRALPAK IB column (250 mm x 4.6 mm, 5 micron) eluting with 25 % EtOH in heptane at a flow rate of 20 mL/min and with UV detection at 300 nm (bandwidth 180 nm; reference 550 nm, bandwidth 100nm). Evaporation of the solvent from fractions containing the later eluting enantiomer afforded the title compound (20 mg). $^1H$ NMR (400 MHz, $CD_3OD$) $\delta$ 2.23 (m, 1 H), 2.62 (m, 1 H), 3.47 (m, 3 H), 4.02 (s, 3 H), 7.36 (dd, *J* = 9, 2 Hz, 1 H), 7.46 (d, *J* = 2 Hz, 1 H), 7.96 (d, *J* = 9 Hz, 1 H), 8.76 (d, *J* = 2 Hz, 1 H), 9.22 (d, *J* = 2 Hz, 1 H); LCMS ES+ve m/z 286 $[M+H]^+$.

## Example 3

**7-(Difluoromethoxy)-N-(1-(1-methyl-1H-tetrazol-5-yl)piperidin-4-yl)quinoline-3-carboxamide**

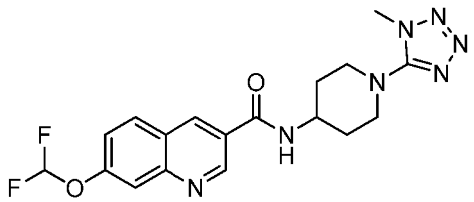

A mixture of 7-(difluoromethoxy)quinoline-3-carboxylic acid (Intermediate 2) (150 mg, 0.627 mmol) and 1-(1-methyl-1H-tetrazol-5-yl)piperidin-4-amine hydrochloride (Intermediate 39) (151 mg, 0.690 mmol) in DMF (5 mL) was treated with DIEA (0.329 mL, 1.881 mmol) and HATU (262 mg, 0.690 mmol) and the mixture was stirred at RT for 90 min. The mixture was diluted with DCM (50 mL) and treated with saturated aqueous sodium bicarbonate (20 mL). The mixture was separated and the organic phase was evaporated to dryness. The product was subjected to purification by Mass-Directed Automated Preparative HPLC (Waters SunFire C18 column, CH3CN / Water + 0.1 % formic acid) to afford the title compound (86 mg, 34.0 % yield). $^1$H NMR (400 MHz, $CD_3SOCD_3$) $\delta$ 1.78 (m, 2 H), 1.97 (m, 2 H), 3.17 (m, 2 H), 3.69 (m, 2 H), 3.91 (s, 3 H), 4.15 (m, 1 H), 7.35 - 7.75 (m, 2 H), 7.78 (s, 1 H), 8.20 (d, *J* = 9 Hz, 1 H), 8.72 (d, *J* = 8 Hz, 1 H), 8.86 (s, 1 H), 9.31 (d, *J* = 2 Hz, 1 H); LCMS ES+ve m/z 404 [M+H]+.

## Example 4

**(S)-7-(Difluoromethoxy)-N-(2-oxopyrrolidin-3-yl)quinoline-3-carboxamide**

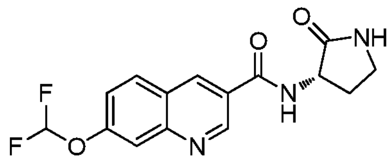

A mixture of 7-(difluoromethoxy)quinoline-3-carboxylic acid (Intermediate 2) (100 mg, 0.418 mmol) and (S)-3-aminopyrrolidin-2-one hydrochloride (68 mg, 0.500 mmol) in DMF (3 mL) was treated with DIEA (0.219 mL, 1.254 mmol) and HATU (167 mg, 0.439 mmol) and the mixture was stirred for 15 min. The mixture was subjected to purification by Mass-Directed Auto-Preparative HPLC (Waters SunFire C18 column, $CH_3CN$ /Water + 0.1% formic acid) to afford the title compound (118 mg). $^1$H NMR (400 MHz, $CD_3SOCD_3$) $\delta$ 2.06 (m, 1 H), 2.43 (m, 1 H), 3.28 (m, 2 H), 4.64 (m, 1 H), 7.36 - 7.75 (m, 2 H), 7.79 (d, *J* = 2 Hz, 1 H), 7.90 (s, 1 H), 8.21 (d, *J* = 9 Hz, 1 H), 8.89 (d, *J* = 2 Hz, 1 H), 9.05 (d, *J* = 8 Hz, 1 H), 9.33 (d, *J* = 2 Hz, 1 H); LCMS ES+ve *m/z* 322 $[M+H]^+$.

## Example 5

**7-(Difluoromethoxy)-N-((trans)-4-(dimethylcarbamoyl)cyclohexyl)quinoline-3-carboxamide**

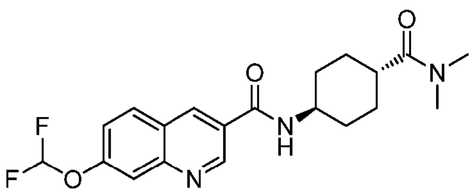

A mixture of 7-(difluoromethoxy)quinoline-3-carboxylic acid (Intermediate 2) (75 mg, 0.314 mmol), (trans)-4-amino-N,N-dimethylcyclohexanecarboxamide (Intermediate 40) (58.7 mg, 0.345 mmol) and HATU (143 mg, 0.376 mmol) was dissolved in DCM (1.6 mL). DIEA (0.164 mL, 0.941 mmol) was added and the reaction mixture was stirred at RT for 18 h. The reaction mixture was evaporated to give a residue. The residue was purified by Mass-Directed Auto-Preparative HPLC (Waters SunFire C18 column, $CH_3CN$ / Water + 0.1% formic acid) to give the title compound (64.2 mg). $^1H$ NMR (400 MHz, $CD_3SOCD_3$) $\delta$ 1.46 (m, 4 H), 1.76 (m, 2 H), 1.98 (m, 2 H), 2.58 (d, *J* = 3 Hz, 1 H), 2.83 (s, 3 H), 3.04 (s, 3 H), 3.80 (m, 1 H), 7.37 - 7.75 (m, 2 H), 7.78 (s, 1 H), 8.20 (d, *J* = 9 Hz, 1 H), 8.60 (d, *J* = 8 Hz, 1 H), 8.83 (d, *J* = 2 Hz, 1 H), 9.29 (d, *J* = 2 Hz, 1 H); LCMS ES+ve *m/z* 392 $[M+H]^+$.

## Example 6

**7-(Difluoromethoxy)-N-((trans)-4-(2-hydroxypropan-2-yl)cyclohexyl)quinoline-3-carboxamide**

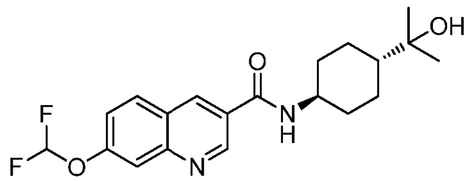

A mixture of 7-(difluoromethoxy)quinoline-3-carboxylic acid (Intermediate 2) (75 mg, 0.314 mmol), 2-((trans)-4-aminocyclohexyl)propan-2-ol (54.2 mg, 0.345 mmol) and HATU (143 mg, 0.376 mmol) was dissolved in DCM (1.6 mL). DIEA (0.164 mL, 0.941 mmol) was added and the reaction mixture was stirred at RT for 18 h. The reaction mixture was evaporated giving a residue that was purified by Mass-Directed Auto-Preparative HPLC (Waters SunFire C18 column, $CH_3CN$ / Water + 0.1 % formic acid) to give the title compound (80.6 mg). $^1H$ NMR (400 MHz, $CD_3SOCD_3$) $\delta$ 1.07 (s, 6 H), 1.09 - 1.41 (m, 6 H), 1.87 (d, *J* = 12 Hz, 2 H), 1.97 (d, *J* = 10 Hz, 2 H), 3.77 (m, 1 H), 7.36 - 7.75 (m, 2 H), 7.78 (d, *J* = 2 Hz, 1 H), 8.19 (d, *J* = 9 Hz, 1 H), 8.55 (d, *J* = 8 Hz, 1 H), 8.83 (d, *J* = 2 Hz, 1 H), 9.29 (d, *J* = 2 Hz, 1 H); LCMS ES+ve *m/z* 379 $[M+H]^+$.

## Example 7

**7-(Difluoromethoxy)-N-(1-(methylsulfonyl)piperidin-4-yl)quinoline-3-carboxamide**

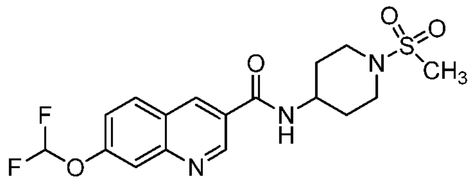

7-(Difluoromethoxy)quinoline-3-carboxylic acid (Intermediate 2) (0.075 g, 0.314 mmol), methanesulfonylpiperidin-4-amine trifluoroacetic acid (0.095 g, 0.345 mmol), and HATU (0.143 g, 0.377 mmol) were combined in a 20 mL scintillation vial with stir bar and teflon cap then dissolved in DCM (1.6 mL) and DMF (1 mL). DIEA (0.219 mL, 1.256 mmol) was added, then the reaction vessel was capped and stirred at RT overnight. The volatiles were then evaporated in vacuo and the residue dissolved in a 1:1 MeOH: DMSO and purified by preparative HPLC (Agilent, Sunfire C18 column, 50X30 mm 5 micron, 10-90% acetonitrile in water + 0.1 % formic acid over 15 min). The appropriate fractions were combined and concentrated in vacuo to afford the title compound (97 mg). $^1H$ NMR (400 MHz, $CD_3SOCD_3$) $\delta$ 1.66 (m, 2 H), 1.98 (m, 2 H), 2.90 (m, 5 H), 3.59 (m, 2 H), 3.99 (m, 1 H), 7.37 - 7.76 (m, 2 H), 7.78 (d, *J* = 2 Hz, 1 H), 8.21 (d, *J* = 9 Hz, 1 H), 8.73 (d, *J* = 7 Hz, 1 H), 8.85 (d, *J* = 2 Hz, 1 H), 9.29 (d, *J* = 2 Hz, 1 H); LCMS ES+ve *m/z* 400 $[M+H]^+$.

**Example 8**

**7-(Difluoromethoxy)-N-(3-methyl-1H-pyrazol-5-yl)quinoline-3-carboxamide**

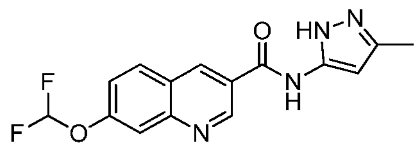

7-(Difluoromethoxy)quinoline-3-carboxylic acid (Intermediate 2) (0.075 g, 0.314 mmol), 3-methyl-1H-pyrazol-5-amine (0.034 g, 0.345 mmol), and HATU (0.143 g, 0.377 mmol) were combined in a 20 mL scintillation vial with stir bar and teflon cap then dissolved in DCM (1.6 mL) and DMF (1 mL). DIEA (0.219 mL, 1.256 mmol) was added, then the reaction vessel was capped and stirred at RT overnight. The volatiles were then evaporated in vacuo and the residue dissolved in a 1:1 MeOH: DMSO and purified by preparative HPLC (Agilent, Sunfire C18 column, 50X30 mm 5 micron, 10-90% acetonitrile in water + 0.1 % formic acid over 15 min). The appropriate fractions were combined and concentrated in vacuo to afford the title compound (26 mg).). $^1$H NMR (400 MHz, $CD_3SOCD_3$) δ ppm 2.26 (s, 3 H), 6.47 (br s, 1 H), 7.58 (t, *J* = 73 Hz, 1 H), 7.57 (d, *J* = 11 Hz, 1 H), 7.79 (s, 1 H), 8.20 (d, *J* = 9 Hz, 1 H), 9.02 (s, 1 H), 9.39 (s, 1 H), 11.08 (s, 1 H), 12.18 (br s, 1 H); LCMS ES+ve *m*/*z* 319 $[M+H]^+$.

**Example 9**

**7-(Difluoromethoxy)-N-(2-oxo-1,2,3,4-tetrahvdroquinolin-4-yl)quinoline-3-carboxamide**

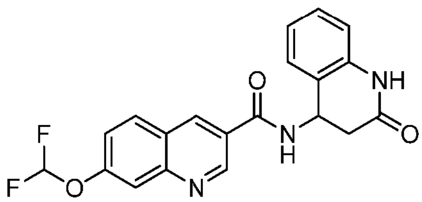

7-(Difluoromethoxy)quinoline-3-carboxylic acid (Intermediate 2) (0.075 g, 0.314 mmol), 4-amino-3,4-dihydroquinolin-2(1*H*)-one hydrochloride (0.070 g, 0.345 mmol), and HATU (0.143 g, 0.377 mmol) were combined in a 20 mL scintillation vial with stir bar and teflon cap then dissolved in DCM (1.6 mL) and DMF (1 mL). DIEA (0.219 mL, 1.256 mmol) was added then the reaction vessel was capped and stirred at RT overnight. The volatiles were then evaporated in vacuo and the residue dissolved in a 1:1 MeOH: DMSO and purified by preparative HPLC (Agilent, Sunfire C18 column, 50X30 mm 5 micron, 10-90% acetonitrile in water + 0.1 % formic acid over 15 min). The appropriate fractions were combined and concentrated in vacuo to afford the title compound (69 mg). $^1$H NMR (400 MHz, $CD_3SOCD_3$) δ 2.69 - 2.87 (m, 2 H), 5.43 (q, *J* = 7 Hz, 1 H), 6.98 (m, 2 H), 7.25 (m, 1 H), 7.32 (d, *J* = 7 Hz, 1 H), 7.38 - 7.76 (m, 2 H), 8 (d, *J* = 2 Hz, 1 H), 8.19 (d, *J* = 9 Hz, 1 H), 8.91 (d, *J* = 2 Hz, 1 H), 9.29 (d, *J* = 8 Hz, 1 H), 9.33 (d, *J* = 2 Hz, 1 H), 10.31 (s, 1 H); LCMS ES+ve *m*/*z* 384 $[M+H]^+$.

**Example 10**

**7-(Difluoromethoxy)-N-(thiazo)-2-yl)quinoline-3-carboxamide**

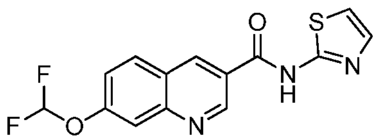

7-(Difluoromethoxy)quinoline-3-carboxylic acid (Intermediate 2) (0.075 g, 0.314 mmol), 2-aminothiazole (0.035 g, 0.345 mmol), and HATU (0.143 g, 0.377 mmol) were combined in a 20 mL scintillation vial with stir bar and teflon cap then dissolved in DCM (1.6 mL) and DMF (1 mL). DIEA (0.219 mL, 1.256 mmol) was added then the reaction vessel was capped and stirred at RT overnight. The volatiles were then evaporated in vacuo and the residue dissolved in a 1:1 MeOH: DMSO and purified by preparative HPLC (Agilent, Sunfire C18 column, 50X30 mm 5 micron, 10-90% acetonitrile in water + 0.1 % formic acid over 15 min). The appropriate fractions were combined and concentrated in vacuo to afford the title compound (43 mg). $^1$H NMR (400 MHz, $CD_3SOCD_3$) δ 7.33 (d, *J* = 3 Hz, 1 H), 7.40 - 7.79 (m, 3 H), 7.81 (d, *J* = 2 Hz, 1 H), 8.24 (d, *J* = 9 Hz, 1 H), 9.14 (d, *J* = 2 Hz, 1 H), 9.46 (d, *J* = 2 Hz, 1 H), 13.00 (br s, 1 H); LCMS ES+ve *m/z* 322 $[M+H]^+$.

**Example 11**

**7-(Difluoromethoxy)-N-(trans-3-(2-hydroxypropan-2-yl)cyclobutyl)quinoline-3-carboxamide**

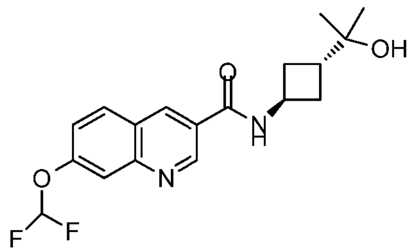

To a suspension of 7-(difluoromethoxy)quinoline-3-carboxylic acid (Intermediate 2) (100 mg, 0.418 mmol) in DMF (10 mL) and THF (10 mL) at RT, was added EDC (240 mg, 1.254 mmol), HOBt (169 mg, 1.254 mmol), and 2-(-3-aminocyclobutyl)propan-2-ol (Intermediate 41) (81 mg, 0.627 mmol). After stirring at RT for 24 h, the solvent was removed under reduced pressure, the residue dissolved in 10% MeOH/DCM (50 mL),absorbed onto silica gel, and purified by silica gel chromatography (DCM to 5% MeOH/DCM) to provide the title compound (30 mg, 20% yield). $^1$H NMR (400 MHz, $CD_3SOCD_3$) δ ppm 1.06 (s, 6 H), 2.01 - 2.15 (m, 2 H), 2.22 - 2.42 (m, 3 H), 4.26 (br s, 1 H), 4.35-4.41 (m, 1 H), 7.54 (dd, *J* = 9, 2 Hz, 1 H), 7.57 (t, *J* = 73 Hz, 1 H), 7.77 (d, *J* = 2 Hz, 1 H), 8.19 (d, *J* = 9 Hz, 1 H), 8.86 (d, *J* = 2 Hz, 1 H), 8.97 (d, *J* = 7 Hz, 1 H), 9.30 (d, *J* = 2 Hz, 1 H). MS (ESI) *m/z* 351 (M+1).

**Example 12**

**7-(Difluoromethoxy)-N-(trans-4-(2-hydroxy-2-methylpropoxy)cyclohexyl)quinoline-3-carboxamide**

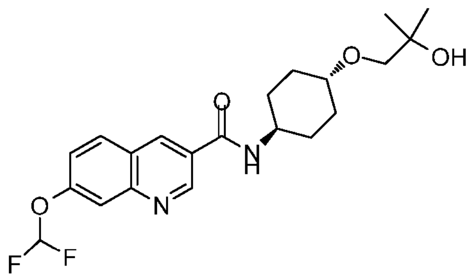

To a suspension of 7-(difluoromethoxy)quinoline-3-carboxylic acid (Intermediate 2) (100 mg, 0.418 mmol) in DMF (10 mL) and THF (10 mL) at RT, was added EDC (240 mg, 1.254 mmol), HOBT (192 mg, 1.254 mmol), and 1-((trans-4-aminocyclohexyl)oxy)-2-methylpropan-2-ol (Intermediate 42) (117 mg, 0.627 mmol). After stirring at RT for 24 h, the solvent was removed under reduced pressure, the residue dissolved in 10% MeOH/DCM (50 mL), and absorbed onto Celite®. The crude material was then purified by reverse phase chromatography (10-50% acetonitrile(with 0.1 % TFA)/ water (with 0.1 % TFA)) to provide the title compound (60 mg, 28% yield). $^1$H NMR (400 MHz, $CD_3SOCD_3$) δ ppm 1.05 (s, 6 H), 1.18 - 1.49 (m, 4 H), 1.90-1.92 (m, 2 H), 1.97 - 2.08 (m, 2 H), 3.15 (s, 1H), 3.17 (s, 2 H), 3.22-3.24 (m, 1 H), 3.75 - 3.89 (m, 1 H), 7.51 - 7.54 (m, 1 H), 7.55 (t, *J* = 73 Hz, 1 H), 7.76 (d, *J* = 2 Hz, 1 H), 8.18 (d, *J* = 9 Hz, 1 H),

8.56 (d, $J$ = 8 Hz, 1 H), 8.83 (d, $J$ = 2 Hz, 1 H), 9.27 (d, $J$ = 2 Hz, 1 H). MS (ESI) $m/z$ 409(M+1).

**Example 13**

**7-(Difluoromethoxy)-N-(trans-4-(-1-hydroxyethyl)cyclohexyl)quinoline-3-carboxamide**

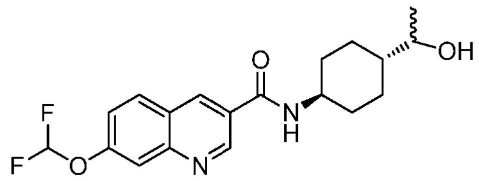

To a solution of 7-(difluoromethoxy)quinoline-3-carboxylic acid (40 mg, 0.167 mmol) (intermediate 2) in DMF (5 mL) was added DIEA (0.09 mL, 0.50 mmol) and HATU (76 mg, 0.20 mmol). After stirring for 5 min, (R)-1-(trans-4-aminocyclohexyl)ethanol hydrochloride (intermediate 43) (30 mg, 0.17 mmol) was added as a solution in DMF (1 mL) and DIEA (0.75 mL). The reaction was stirred at RT for 2 h, quenched into water and extracted with EtOAc. The combined organics were dried over sodium sulfate, filtered, and concentrated. The residue was purified by silica gel chromatography (0-80% (3:1 EtOAc:EtOH)-hexanes gradient) to afford the title compound as a white solid (45 mg, 61% yield). $^1$H NMR (400 MHz, $CD_3SOCD_3$) $\delta$ ppm 1.02 (d, $J$ = 6 Hz, 3 H), 1.05 - 1.19 (m, 3 H), 1.25 - 1.41 (m, 2 H), 1.69 (br s, 1 H), 1.85 - 2.00 (m, 3 H), 3.38 (br s, 1 H), 3.70 - 3.86 (m, 1 H), 7.35 - 7.74 (m, 2 H), 7.75 (s, 1 H), 8.17 (d, $J$ = 9 Hz, 1 H), 8.56 (d, $J$ = 8 Hz, 1 H), 8.81 (s, 1 H), 9.26 (s, 1 H). MS (ESI) $m/z$ 365 (M+1).

**Example 14**

**N-(trans-4-(-Cyclopropyl(hydroxy)methyl)cyclohexyl)-7-(difluoromethoxy)quinoline-3-carboxamide**

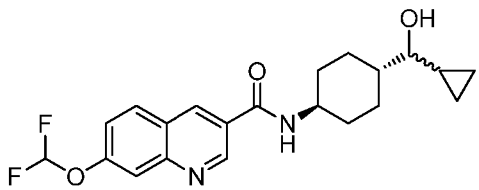

**A. 7-(Difluoromethoxy)-N-(trans-4-(hydroxymethyl)cyclohexyl)quinoline-3-carboxamide**

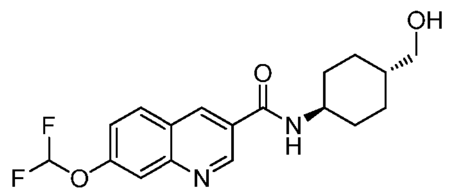

To a solution of 7-(difluoromethoxy)quinoline-3-carboxylic acid (328 mg, 1.37 mmol) (intermediate 2) in DMF (15 mL) was added DIEA (0.72 mL, 4.11 mmol) and HATU (625 mg, 1.64 mmol). After stirring for 5 minutes (trans-4-aminocyclohexyl)methanol hydrochloride (intermediate 79) (227 mg, 1.37 mmol) was added as a solution in DMF (3 mL) and DIEA (0.24 mL). The reaction was stirred at RT for 2 hr, quenched into water and extracted with EtOAc. The combined organics were dried over sodium sulfate, filtered, and concentrated to give a brown solid. This was triturated with DCM/EtOAc to afford the title compound as a white solid (345 mg). The mother liquors were concentrated and purified by silica gel chromatography (0-80% (3:1 EtOAc:EtOH)-hexanes gradient) to afford additional title compound as a white solid (50 mg). These material were combined to give the title compound as a white solid (395 mg, 82%). $^1$H

NMR (400 MHz, $CD_3SOCD_3$) δ ppm 0.90 - 1.08 (m, 2 H), 1.26 - 1.44 (m, 3 H), 1.79 (d, *J* = 12 Hz, 2 H), 1.91 (d, *J* = 10 Hz, 2 H), 3.22 (t, *J* = 6 Hz, 2 H), 3.69 - 3.86 (m, 1 H), 4.40 (t, *J* = 5 Hz, 1 H), 7.33 -7.74 (m, 2 H), 7.75 (s, 1 H), 8.17 (d, *J* = 9 Hz, 1 H), 8.56 (d, *J* = 8 Hz, 1 H), 8.81 (s, 1 H), 9.26 (d, *J* = 2 Hz, 1 H). MS (ESI) *m/z* 351 (M+1).

**B. 7-(Difluoromethoxy)-N-(trans-4-formylcyclohexyl)quinoline-3-carboxamide**

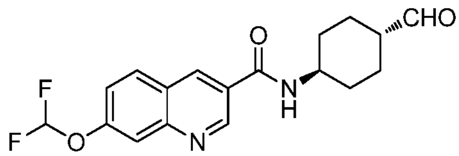

To a ice cold solution of 7-(difluoromethoxy)-N-(trans-4-(hydroxymethyl)cyclohexyl)quinoline-3-carboxamide (375 mg, 1.07 mmol) in DCM (10 mL) and DMSO (2.5 mL) was added DIEA (0.75 mL, 4.28 mmol) followed by pyridine sulfur trioxide (681 mg, 4.28 mmol) dissolved in DMSO (2.5 mL). The bath was removed and the reaction stirred at RT for 15 min. The mixture was quenched into water and extracted with EtOAc. The organic phase was washed with brine, dried over magnesium sulfate, filtered, and concentrated to afford the title compound as a white solid (371 mg, 100%). $^1H$ NMR (400 MHz, $CD_3SOCD_3$) δ ppm 1.24 - 1.49 (m, 4 H), 1.99 (d, *J* = 11 Hz, 4 H), 2.26 (t, *J* = 12 Hz, 1 H), 3.72 - 3.86 (m, 1 H), 7.35 - 7.74 (m, 2 H), 7.76 (s, 1 H), 8.17 (d, *J* = 9 Hz, 1 H), 8.62 (d, *J* = 8 Hz, 1 H), 8.82 (s, 1 H), 9.27 (d, *J* = 2 Hz, 1 H), 9.58 (s, 1 H). MS (ESI) *m/z* 349 (M+1).

**C. N-(trans-4-(Cyclopropyl(hydroxy)methyl)cyclohexyl)-7-(difluoromethoxy)quinoline-3-carboxamide**

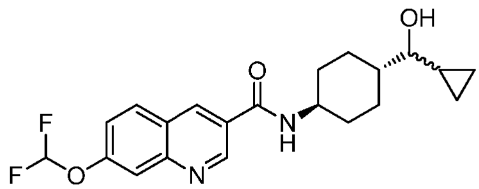

To a -78 °C solution of 7-(difluoromethoxy)-N-(trans-4-formylcyclohexyl)quinoline-3-carboxamide (75 mg, 0.22 mmol) in THF (5 mL) was added cyclopropylmagnesium bromide (0.5 M in THF) (1.29 mL, 0.65 mmol) dropwise. After stirring at -78 °C for 30 min the reaction was quenched by the addition of 1 M ammonium chloride (3 mL). After warming to RT the mixture was extracted with EtOAc. The combined organics were washed with brine, dried over sodium sulfate, and concentrated. The residue was purified by silica gel chromatography (0-80% (3:1 EtOAc:EtOH)-hexanes gradient) to afford the title compound. This material was subsequently purified by RP HPLC (acetonitrile-water /TFA gradient) and freebased to afford the title compound as a white solid (29 mg, 35%). $^1H$ NMR (400 MHz, $CD_3SOCD_3$) δ ppm 0.18 (d, *J* = 3 Hz, 2 H), 0.27 - 0.44 (m, 2 H), 0.79 (d, *J* = 7 Hz, 1 H), 1.08 - 1.42 (m, 6 H), 1.84 (d, *J* = 11 Hz, 1 H), 1.92 (br s, 3 H), 3.75 (d, *J* = 7 Hz, 1 H), 4.30 (d, *J* = 4 Hz, 1 H), 7.33 - 7.79 (m, 3 H), 8.17 (d, *J* = 9 Hz, 1 H), 8.56 (d, *J* = 8 Hz, 1 H), 8.81 (br s, 1 H), 9.26 (br s, 1 H). MS (ESI) *m/z* 391 (M+1).

**Example 15**

**7-(Difluoromethoxy)-N-(trans-4-(2,2,2-trifluoro-1-hydroxyethyl)cyclohexyl)quinoline-3-carboxamide**

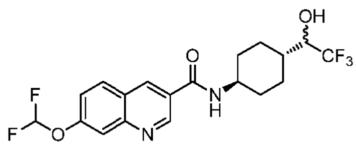

To a ice cold solution of 7-(difluoromethoxy)-N-(trans-4-formylcyclohexyl)quinoline-3-carboxamide (example

14, step B) (100 mg, 0.29 mmol) in THF (4 mL) was added CsF (131 mg, 0.86 mmol) followed by trimethyl(trifluoromethyl)silane (0.25 mL, 1.72 mmol). After stirring for 30 min the reaction was quenched into water and extracted with EtOAc. The combined organics were washed with brine, dried over sodium sulfate and concentrated. The residue was dissolved in THF (5 mL) and TBAF (1M in THF) (0.57 mL, 0.57 mmol) was added dropwise. After stirring for 30 min the reaction was quenched with sat. ammonium chloride and extracted with EtOAc. The combined organics were washed with brine, dried over sodium sulfate, filtered, and concentrated. The residue was purified by silica gel chromatography (0-80% (3:1 EtOAc:EtOH)-hexanes gradient) to afford the title compound, which was triturated with DCM to yield the title compound as a white solid (82 mg, 68%). $^1$H NMR (400 MHz, $CD_3SOCD_3$) $\delta$ ppm 1.24 - 1.41 (m, 4 H), 1.56 (d, $J$ = 10 Hz, 1 H), 1.72 (d, $J$ =6 Hz, 1 H), 1.88 (br s, 1 H), 1.95 (d, $J$ = 7 Hz, 2 H), 3.70 - 3.82 (m, 2 H), 6.10 (d, $J$ = 7 Hz, 1 H), 7.35 - 7.73 (m, 2 H), 7.75 (d, $J$ = 2 Hz, 1 H), 8.17 (d, $J$ = 9 Hz, 1 H), 8.58 (d, $J$ = 8 Hz, 1 H), 8.81 (d, $J$ = 2 Hz, 1 H), 9.26 (d, $J$ = 2 Hz, 1 H). MS (ESI) *m/z* 419 (M+1).

**Example 16**

**7-(Difluoromethoxy)-N-(trans-4-(2-hydroxypropoxyl)cyclohexyl)quinoline-3-carboxamide**

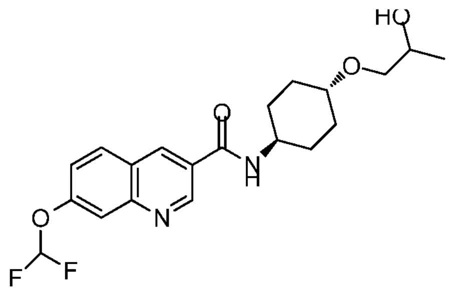

To a suspension of 7-(difluoromethoxy)quinoline-3-carboxylic acid (Intermediate 2) (100 mg, 0.418 mmol) in DMF (10 mL) at RT, was added EDC (240 mg, 1.254 mmol), HOBT (192 mg, 1.254 mmol), and 1-((trans-4-aminocyclohexyl)oxy)propan-2-ol (intermediate 44) (109 mg, 0.627 mmol). After stirring at RT for 2 h, the solvent was removed under reduced pressure, the residue dissolved in 10% MeOH/DCM (50 mL) and absorbed onto Celite®. The crude material was then purified by silica gel chromatography (DCM to 10% MeOH/DCM). The combined fractions that contained product were washed with 1 M NaOH (25 mL) to remove any residual HOBt and this resulting organic layer was concentrated to provide the title compound (80 mg, 49% yield). $^1$H NMR (400 MHz, $CD_3SOCD_3$) $\delta$ ppm 1.04 (d, $J$ = 6 Hz, 3 H), 1.21 - 1.33 (m, 2 H), 1.35 - 1.49 (m, 2 H), 1.91-1.94 (m, 2 H), 2.02-2.05 (m, 2 H), 3.18 - 3.37 (m, 3H), 3.63 - 3.74 (m, 1 H), 3.77 - 3.91 (m, 1 H), 4.51 (d, $J$ = 5 Hz, 1 H), 7.54 (dd, $J$ = 9, 2 Hz, 1 H), 7.57 (t, $J$ = 73 Hz, 1 H), 7.77 (d, $J$ = 2 Hz, 1 H), 8.19 (d, $J$ = 9 Hz, 1 H), 8.58 (d, $J$ = 8 Hz, 1 H), 8.83 (d, $J$ = 2 Hz, 1 H), 9.28 (d, $J$ = 2 Hz, 1 H). MS (ESI) *m/z* 395(M+1).

**Example 17**

**Racemic 7-(Difluoromethoxy)-N-((3*R*,6*S*)-6-(2,2,2-trifluoro-1-hydroxyethyl)tetrahydro-2H-pyran-3-yl)quinoline-3-carboxamide**

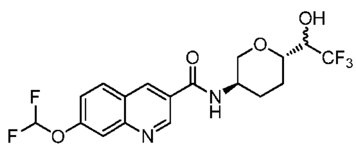

A. **Racemic 7-(Difluoromethoxy)-N-((3*R*,6*S*)-6-(hydroxymethyl)tetrahydro-2H-pyran-3-yl)quinoline-3-carboxamide**

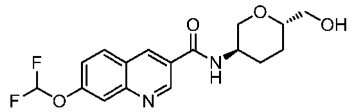

To a solution of 7-(difluoromethoxy)quinoline-3-carboxylic acid (100 mg, 0.42 mmol) (intermediate 2) in DMF (2 mL) was added DIEA (0.22 mL, 1.25 mmol) and HATU (191 mg, 0.50 mmol). After stirring for 5 min, (5-aminotetrahydro-2H-pyran-2-yl)methanol hydrochloride (intermediate 45) (77 mg, 0.46 mmol) (cis/trans mixture) was added as a solution in DMF (2 mL) and DIEA (0.24 mL). The reaction was stirred at RT for 2 h, quenched into water and extracted with EtOAc. The combined organics were dried over sodium sulfate, filtered, and concentrated. The residue was purified by silica gel chromatography (0-100% (3:1 EtOAc:EtOH)-hexanes gradient) to afford a mixture of cis and trans isomers (133 mg). The cis and trans isomers were separated by C18 reverse phase HPLC to afford the desired trans isomer (52 mg). Relative stereochemistry was assigned by NMR. $^1$H NMR (400 MHz, $CD_3SOCD_3$) $\delta$ ppm 1.25 - 1.40 (m, 1 H), 1.61 (dq, *J* = 12, 4 Hz, 1 H), 1.73 (d, *J* = 14 Hz, 1 H), 2.00 (d, *J* = 12 Hz, 1 H), 3.11 - 3.18 (m, 1 H), 3.21 - 3.29 (m, 2 H), 3.35 - 3.42 (m, 1 H), 3.85 - 4.00 (m, 2 H), 4.64 (br s, 1 H), 7.35 - 7.74 (m, 2 H), 7.75 (d, *J* = 2 Hz, 1 H), 8.17 (d, *J* = 9 Hz, 1 H), 8.57 (d, *J* = 7 Hz, 1 H), 8.82 (d, *J* = 2 Hz, 1 H), 9.26 (d, *J* = 2 Hz, 1 H). MS (ESI) *m/z* 353 (M+1).

B. **Racemic 7-(Difluoromethoxy)-N-((3R,6S)-6-formyltetrahydro-2H-pyran-3-yl)quinoline-3-carboxamide**

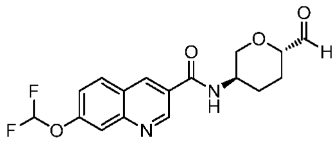

To a ice cold solution of racemic 7-(difluoromethoxy)-N-((3*R*,6*S*)-6-(hydroxymethyl)tetrahydro-2H-pyran-3-yl)quinoline-3-carboxamide (40 mg, 0.11 mmol) in DCM (2 mL) and DMSO (0.5 mL) was added DIEA (0.08 mL, 0.45 mmol) followed by pyridine sulfur trioxide (72.3 mg, 0.45 mmol) dissolved in DMSO (0.5 mL). The bath was removed and the reaction stirred at RT for 15 min. The mixture was quenched into water and extracted with EtOAc. The organic phase was washed with brine, dried over magnesium sulfate, filtered, and concentrated to afford 35 mg of a white solid. By NMR this was a mixture of product and starting material. Return material to the above reaction conditions for 1 h. The reaction was worked up as above to afford 33 mg of a white solid, which was taken on to the next step without purification.

C. **Racemic 7-(Difluoromethoxy)-N-((3R,6S)-6-(2,2,2-trifluoro-1-hydroxyethyl)tetrahydro-2H-pyran-3-yl)quinoline-3-carboxamide**

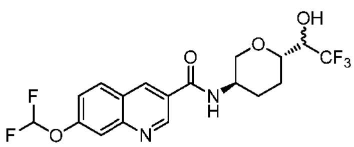

To an ice cold solution of racemic 7-(difluoromethoxy)-N-((3*R*,6*S*)-6-formyltetrahydro-2H-pyran-3-yl)quinoline-3-carboxamide (30 mg, 0.09 mmol) in THF (2 mL) was added CsF (39 mg, 0.26 mmol), followed by trimethyl(trifluoromethyl)silane (73 mg, 0.51 mmol). After stirring for 30 min the reaction was poured into water and extracted with EtOAc. The combined organics were washed with brine, dried over sodium sulfate, filtered, and concentrated. The residue was dissolved in THF (2 mL) and TBAF (1 M in THF) (0.17 mL, 0.17 mmol) was added dropwise. After stirring for 30 min the reaction was quenched with sat. ammonium chloride and extracted with EtOAc. The combined organics were washed with brine, dried over sodium sulfate, filtered, and concentrated. The residue was purified by silica gel chromatography (0-100% (3:1 EtOAc:EtOH)-hexanes gradient) to afford the title compound as an off white solid (16 mg, 44%) (mixture of diastereomers). $^1$H NMR (400 MHz, $CD_3SOCD_3$) $\delta$ ppm 1.48 - 1.94 (m, 3 H), 2.07 (br s, 1 H), 3.12 - 3.24 (m, 1 H), 3.41 - 3.58 (m, 1 H), 3.82 - 4.05 (m, 3 H), 6.19 (d, *J* = 8 Hz, 0.5 H), 6.47 (d, *J* = 7 Hz, 0.5 H), 7.35 - 7.74 (m, 2 H), 7.76 (br s, 1 H), 8.17 (d, *J* = 9 Hz, 1 H), 8.59 (br s, 1 H), 8.82 (br s, 1 H), 9.26 (br s, 1 H). MS (ESI) *m/z* 421 (M+1).

**Example 18**

**7-(Difluoromethoxy)-N-(trans-4-(1-hydroxycyclopropyl)cyclohexyl)quinoline-3-carboxamide**

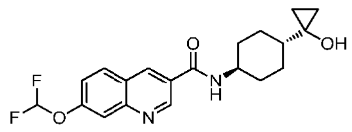

To a solution of 7-(difluoromethoxy)quinoline-3-carboxylic acid (intermediate 2) (85 mg, 0.36 mmol) in DMF (3 mL) was added DIEA (0.19 mL, 1.07 mmol) and HATU (162 mg, 0.43 mmol). After stirring for 5 minutes, 1-(trans-4-aminocyclohexyl)cyclopropanol (intermediate 46) (60.7 mg, 0.391 mmol) was added as a solution in 1 mL DMF. The reaction was stirred at RT for 2.5 h, quenched into water and extracted with EtOAc. The combined organics were dried over sodium sulfate, filtered, and concentrated. The residue was purified by silica gel chromatography (0-80% (3:1 EtOAc:EtOH)-hexanes gradient) to afford the title compound as a white solid (88 mg, 66% yield). $^{1}H$ NMR (400 MHz, $CD_3SOCD_3$) δ ppm 0.30 - 0.39 (m, 2 H), 0.48 (s, 2 H), 0.91 (d, *J* = 8 Hz, 1 H), 1.20 - 1.46 (m, 4 H), 1.72 (br s, 2 H), 1.94 (br s, 2 H), 3.74 (br s, 1 H), 4.87 (s, 1 H), 7.34 - 7.73 (m, 2 H), 7.75 (s, 1 H), 8.17 (d, *J* = 9 Hz, 1 H), 8.56 (d, *J* = 8 Hz, 1 H), 8.81 (s, 1 H), 9.26 (d, *J* = 2 Hz, 1 H). MS (ESI) *m/z* 377 (M+1).

**Example 19**

**7-(Difluoromethoxy)-N-(trans-4-(3,3,3-trifluoro-2-hydroxy-2-methylpropoxy)cyclohexyl)quinoline-3-carboxamide**

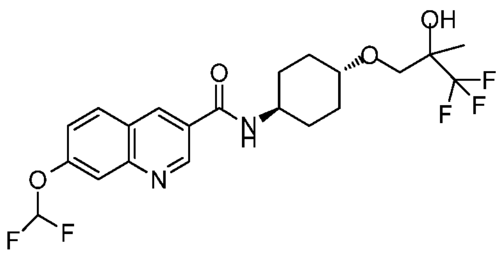

To a solution of 3-((trans-4-aminocyclohexyl)oxy)-1,1,1-trifluoro-2-methylpropan-2-ol hydrochloride (Intermediate 47) (50 mg, 0.180 mmol) in DMF (5 mL) was added 7-(difluoromethoxy)quinoline-3-carboxylic acid (Intermediate 2) (40 mg, 0.167 mmol), followed by HATU (95 mg, 0.251 mmol) and DIEA (0.175 mL, 1.003 mmol). The reaction was allowed to stir at RT for 24 h. The solvent was removed under reduced pressure, and the residue was absorbed onto Celite®. The crude product was then purified by reverse phase chromotography (C18 column, 10-100% acetonitrile (with 0.1% TFA)/ water (with 0.1% TFA)) to afford the title compound (45 mg, 47% yield). $^{1}H$ NMR (400 MHz, $CD_3SOCD_3$) δ ppm 1.20 - 1.49 (m, 7 H), 1.92-1.94 (m, 2 H), 1.99 - 2.09 (m, 2 H), 3.17 (s, 1 H), 3.27 - 3.37 (m, 1 H), 3.41 - 3.58 (m, 2 H), 3.77 - 3.90 (m, 1 H), 7.53-7.56 (m, 1H), 7.57 (t, *J* = 73 Hz, 1 H), 7.76- 7.78 (m, 1 H), 8.20 (d, *J* = 9 Hz, 1 H), 8.59 (d, *J* = 8 Hz, 1 H), 8.83 (d, *J* = 2 Hz, 1 H), 9.28 (d, *J* = 2 Hz, 1 H). MS (ESI) *m/z* 463 (M+1).

**Examples 20 and 21**

**7-(Difluoromethoxy)-N-(trans-4-((*R*)-2,2,2-trifluoro-1-hydroxyethyl)cyclohexyl)quinoline-3-carboxamide and 7-(Difluoromethoxy)-N-(trans-4-((S)-2,2,2-trifluoro-1-hydroxyethyl)cyclohexyl)quinoline-3-carboxamide**

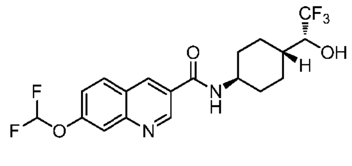

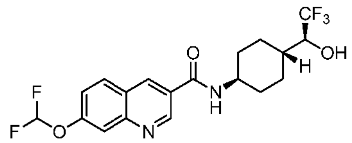

To a solution of 7-(difluoromethoxy)quinoline-3-carboxylic acid (intermediate 2) (225 mg, 0.94 mmol) in DMF (8 mL) was added DIEA (0.49 mL, 2.82 mmol) and HATU (429 mg, 1.13 mmol). After stirring for 5 minutes, racemic 1-(trans-4-aminocyclohexyl)-2,2,2-trifluoroethanol hydrochloride (intermediate 48) (231 mg, 0.988 mmol) was added as a solution in DMF (2 mL). The reaction was stirred at RT for 2 h, quenched into water and extracted with EtOAc. The combined organics were dried over sodium sulfate, filtered, and concentrated. The residue was purified by silica gel chromatography (0-80% (3:1 EtOAc:EtOH)-hexanes gradient) to afford a white solid. The enantiomers were separated by chiral chromatography (SFC, OD column, 10% MeOH/$CO_2$) to give 48 mg of each of the title compounds. Absolute stereochemistry was assigned by Ab Initio VCD analysis. First peak assigned as 7-(difluoromethoxy)-N-(trans-4-((*R*)-2,2,2-trifluoro-1-hydroxyethyl)cyclohexyl)quinoline-3-carboxamide, second peak assigned as 7-(difluoromethoxy)-N-(trans-4-((*S*)-2,2,2-trifluoro-1-hydroxyethyl)cyclohexyl)quinoline-3-carboxamide. NMR and MS matched example 15.

## Example 22

**Racemic 7-(Difluoromethoxy)-N-((3*s*,6*r*)-6-(2-hydroxypropan-2-yl)tetrahydro-2H-pyran-3-yl)quinoline-3-carboxamide**

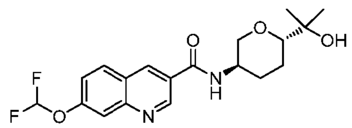

To a solution of 7-(difluoromethoxy)quinoline-3-carboxylic acid (intermediate 2) (75 mg, 0.31 mmol) in DMF (4 mL) was added DIEA (0.16 mL, 0.94 mmol) and HATU (143 mg, 0.38 mmol). After stirring for 5 min, 2-((2s,5r)-5-aminotetrahydro-2H-pyran-2-yl)propan-2-ol hydrochloride (Intermediate 49) (64 mg, 0.33 mmol) was added as a solution in DMF (1 mL) and DIEA (0.05 mL). The reaction was stirred at RT for 2.5 h, quenched into water and extracted with EtOAc. The combined organics were dried over sodium sulfate, filtered, and concentrated. The residue was purified by silica gel chromatography (0-70% (3:1 EtOAc:EtOH)-hexanes gradient) to afford the title compound as a white solid (97 mg, 81% yield). $^1H$ NMR (400 MHz, $CD_3SOCD_3$) $\delta$ ppm 0.99 - 1.12 (m, 6 H), 1.30 - 1.46 (m, 1 H), 1.58 (qd, *J* = 12, 3 Hz, 1 H), 1.80 (d, *J* = 14 Hz, 1 H), 2.01 (d, *J* = 12 Hz, 1 H), 2.97 (d, *J* = 10 Hz, 1 H), 3.14 (t, *J* = 10 Hz, 1 H), 3.80 - 4.03 (m, 2 H), 4.24 (s, 1 H), 7.34 - 7.73 (m, 2 H), 7.75 (d, *J* = 2 Hz, 1 H), 8.17 (d, *J* = 9 Hz, 1 H), 8.55 (d, *J* = 7 Hz, 1 H), 8.81 (d, *J* = 2 Hz, 1 H), 9.26 (d, *J* = 2 Hz, 1 H). MS (ESI) *m/z* 381 (M+1).

## Example 23

**N-(trans-4-((3-Aminopropoxy)methyl)cyclohexyl)-7-(difluoromethoxy)quinoline-3-carboxamide**

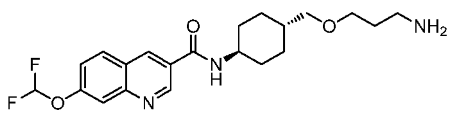

**A. N-(trans-4-((3-Azidopropoxy)methyl)cyclohexyl)-7-(difluoromethoxy)quinoline-3-carboxamide**

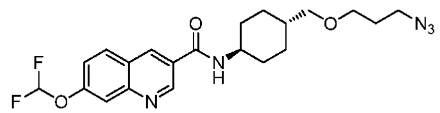

To a solution of 7-(difluoromethoxy)quinoline-3-carboxylic acid (intermediate 2) (70 mg, 0.29 mmol) in DMF (3 mL) was added DIEA (0.15 mL, 0.88 mmol) and HATU (134 mg, 0.35 mmol). After stirring for 10 min trans-4-((3-azidopropoxy)methyl)cyclohexanamine hydrochloride (intermediate 50) (73 mg, 0.29 mmol) was added as a solution in DMF (1 mL) and DIEA (0.05 mL). The reaction was stirred at RT overnight, poured into water and extracted with EtOAc. The combined organics were dried over sodium sulfate, filtered, and concentrated. The residue was purified by silica gel chromatography (0-70% (3:1 EtOAc:EtOH)-hexanes gradient) to afford the title compound as a white solid (78 mg, 61% yield). $^1$H NMR (400 MHz, $CD_3SOCD_3$) δ ppm 0.96 - 1.12 (m, 2 H), 1.29 - 1.42 (m, 2 H), 1.45 - 1.58 (m, 1 H), 1.67 - 1.84 (m, 4 H), 1.91 (d, *J* = 10 Hz, 2 H), 3.20 (d, *J* =6 Hz, 2 H), 3.39 (dt, *J* = 15, 6 Hz, 4 H), 3.78 (ddt, *J* = 12, 8, 4 Hz, 1 H), 7.35 - 7.73 (m, 2 H), 7.75 (d, *J* = 2 Hz, 1 H), 8.17 (d, *J* = 9 Hz, 1 H), 8.58 (d, *J* = 8 Hz, 1 H), 8.81 (d, *J* = 2 Hz, 1 H), 9.26 (d, *J* = 2 Hz, 1 H). MS (ESI) *m/z* 434 (M+1).

**B. N-(trans-4-(3-Aminopropoxy)cyclohexyl)-7-(difluoromethoxy)quinoline-3-carboxamide**

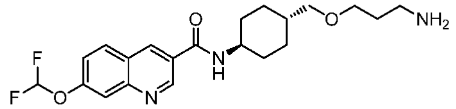

To a solution of N-(trans-4-((3-azidopropoxy)methyl)cyclohexyl)-7-(difluoromethoxy)quinoline-3-carboxamide (75 mg, 0.17 mmol) in THF (4 mL) and water (0.2 mL) was added triphenylphosphine (91 mg, 0.35 mmol). The mixture was heated at 40 °C overnight, diluted with EtOAc, washed with brine, dried over sodium sulfate, filtered, and concentrated. The residue was purified by C18 RP HPLC (acetonitrile-water/TFA gradient) to give a tacky solid. This material was freebased to afford the title compound as a white solid (47 mg, 67% yield). $^1$H NMR (400 MHz, $CD_3SOCD_3$) δ ppm 0.97 - 1.10 (m, 2 H), 1.28 - 1.42 (m, 2 H), 1.46 - 1.58 (m, 1 H), 1.72-1.84 (m, 4 H), 1.88 - 1.98 (m, 2 H), 2.78 - 2.92 (m, 2 H), 3.18 (d, *J* = 6 Hz, 2 H), 3.39 (t, *J* = 6 Hz, 2 H), 3.72 - 3.87 (m, 1 H), 7.35 - 7.73 (m, 2 H), 7.62 (br s, 2H), 7.75 (d, *J* = 2 Hz, 1 H), 8.17 (d, *J* = 9 Hz, 1 H), 8.58 (d, *J* = 8 Hz, 1 H), 8.81 (d, *J* = 2 Hz, 1 H), 9.26 (d, *J* = 2 Hz, 1 H). MS (ESI) *m/z* 408 (M+1).

**Example 24**

**6-Cyano-7-cyclopropyl-N-(1-(1-methyl-1*H*-tetrazol-5-yl)piperidin-4-yl)quinoline-3-carboxamide**

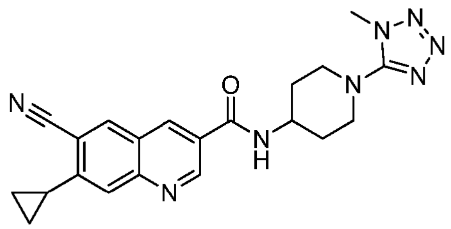

A mixture of 6-cyano-7-cyclopropylquinoline-3-carboxylic acid (Intermediate 3) (50 mg, 0.21 mmol), 1-(1-methyl-1*H*-tetrazol-5-yl)piperidin-4-amine hydrochloride (Intermediate 39) (42.1 mg, 0.231 mmol) and DIEA (0.110 mL, 0.63 mmol) in DMF (5 mL) was treated with HATU (96 mg, 0.252 mmol) and the mixture was stirred at RT for 3 h. The mixture was evaporated to dryness and the residue was purified by Mass-Directed Auto-Preparative HPLC (Waters SunFire C18 column, $CH_3CN$ / Water + 0.1 % formic acid) to afford the title compound (46 mg). $^1$H NMR (400 MHz, $CD_3SOCD_3$) δ

1.02 (m, 2 H), 1.23 (m, 2 H), 1.78 (m, 2 H), 1.97 (d, *J* = 10 Hz, 2 H), 2.32 (m, 1 H), 3.17 (m, 2 H), 3.68 (d, *J* = 13 Hz, 2 H), 3.90 (s, 3 H), 4.14 (m, 1 H), 7.76 (s, 1 H), 8.72 (s, 1 H), 8.82 (m, 1 H), 8.89 (d, *J* = 2 Hz, 1 H), 9.38 (d, *J* = 2 Hz, 1 H). LCMS ES+ve *m/z* 403 $[M+H]^+$.

**Example 25**

**(S)-6-Cyano-7-cyclopropyl-N-(2-oxopyrrolidin-3-yl)quinoline-3-carboxamide hydrochloride**

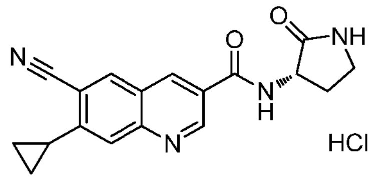

A solution of 6-cyano-7-cyclopropylquinoline-3-carboxylic acid (Intermediate 3) (40 mg, 0.168 mmol) in DMF (2 mL) was treated with DIEA (0.059 mL, 0.336 mmol) and HATU (70 mg, 0.185 mmol), stirred at RT for 10 min and then treated with (S)-3-aminopyrrolidin-2-one (20 mg, 0.201 mmol). After stirring for 1 h the mixture was evaporated to dryness and the residue was subjected to purification by Mass-Directed Auto-Preparative HPLC (Waters SunFire C18 column, $CH_3CN$ / Water + 0.1% formic acid). The recovered product was dissolved in MeOH (3 mL), treated with 4M HCl in 1,4-dioxane (0.5 mL) and evaporated to dryness to afford the title compound (36 mg). $^1H$ NMR (400 MHz, $CD_3OD$) $\delta$ 1.12 (m, 2 H), 1.46 (m, 2 H), 2.25 (m, 1 H), 2.49 - 2.70 (m, 2 H), 3.47 (m, 2 H), 3.57 - 3.79 (m, 1 H), 7.81 (s, 1 H), 8.81 (s, 1 H), 9.40 (br s, 1 H), 9.59 (s, 1 H); LCMS ES+ve *m/z* 320 $[M+H]^+$.

**Example 26**

**6-Cyano-7-cyclopropyl-N-(2-oxo-1,2,3,4-tetrahydroquinolin-4-yl)quinoline-3-carboxamide**

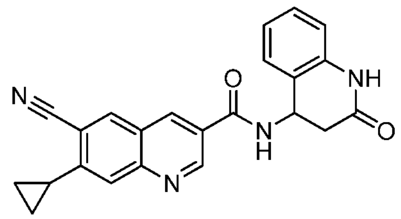

A solution of 6-cyano-7-cyclopropylquinoline-3-carboxylic acid (Intermediate 3) (42 mg, 0.176 mmol) in DMF (5 mL) was treated with DIEA (0.092 mL, 0.529 mmol) and HATU (74 mg, 0.194 mmol) and the mixture was stirred at RT for 10 min. The mixture was then treated with 4-amino-3,4-dihydroquinolin-2(1*H*)-one hydrochloride (42 mg, 0.212 mmol). After stirring for 1 h the mixture was evaporated to dryness and the product was subjected to purification by Mass-Directed Auto-Preparative HPLC (Waters SunFire C18 column, $CH_3CN$ / Water + 0.1% formic acid) to afford the title compound (41 mg). $^1H$ NMR (400 MHz, $CD_3SOCD_3$) $\delta$ 1.02 (m, 2 H), 1.22 (m, 2 H), 2.34 (m, 1 H), 2.79 (m, 2 H), 5.43 (q, *J* = 7 Hz, 1 H), 6.98 (m, 2 H), 7.26 (t, *J* = 7 Hz, 1 H), 7.33 (d, *J* = 7 Hz, 1 H), 7.76 (s, 1H), 8.71 (s, 1 H), 8.91 (d, *J* = 2 Hz, 1 H), 9.33 (d, *J* = 8 Hz, 1 H), 9.39 (d, *J* = 2 Hz, 1 H), 10.28 (s, 1 H); LCMS ES+ve *m/z* 383 $[M+H]^+$.

**Example 27**

**7-Bromo-6-chloro-N-(1-(1-methyl-1*H*-tetrazol-5-yl)piperidin-4-yl)quinoline-3-carboxamide**

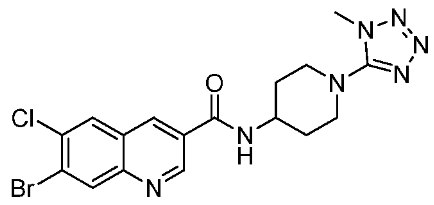

A mixture of 7-bromo-6-chloroquinoline-3-carboxylic acid (Intermediate 4) (150 mg, 0.524 mmol) and 1-(1-methyl-1*H*-tetrazol-5-yl)piperidin-4-amine hydrochloride (Intermediate 39) (105 mg, 0.576 mmol) in DMF (5 mL) was treated with DIEA (0.274 mL, 1.57 mmol) and HATU (219 mg, 0.576 mmol) and the mixture was stirred at RT for 90 min. The mixture was diluted with DCM (50 mL) and treated with saturated aqueous sodium bicarbonate (20 mL). The layers were then separated and the organic phase was evaporated to dryness. The product was purified by Mass-Directed Auto-Preparative HPLC (Waters SunFire C18 column, $CH_3CN$ / Water + 0.1% formic acid) to afford the title compound (210 mg). $^1H$ NMR (400 MHz, $CD_3SOCD_3$) δ 1.67 - 1.88 (m, 2 H), 1.98 (m, 2 H), 3.16 (m, 2 H), 3.68 (d, *J* = 13 Hz, 2 H), 3.90 (s, 3 H), 4.13 (m, 1H), 8.48 (s, 1 H), 8.54 (s, 1 H), 8.79 (d, *J* = 8 Hz, 1 H), 8.82 (d, *J* = 2 Hz, 1 H), 9.31 (d, *J* = 2 Hz, 1 H); LCMS ES+ve *m*/*z* 450/452 $[M+H]^+$.

**Example 28**

**8-Methoxy-N-(thiazol-2-yl)quinoline-3-carboxamide**

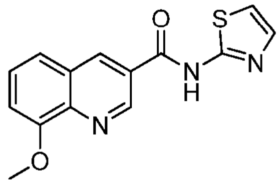

To a solution of 8-methoxy-3-quinolinecarboxylic acid (Intermediate 5) (100 mg, 0.492 mmol) and HATU (206 mg, 0.541 mmol) in DMF (2 mL) was added DIEA (0.258 mL, 1.476 mmol) and the reaction mixture was stirred at 21 °C for 3 min. Thiazol-2-amine (59.1 mg, 0.591 mmol) was added and the reaction mixture was stirred at 21 °C for 3 h. The reaction mixture was concentrated *in vacuo* and the residue was dissolved in DCM, loaded onto an aminopropyl (NH2) ion-exchange SPE cartridge (5 g) and eluted with 1:1 MeOH/DCM. Eluent fractions were combined and evaporated *in vacuo.* The residue was purified by Mass-Directed Auto-Preparative HPLC (Waters SunFire C18 column, CH3CN / Water + 0.1 % formic acid) to give the title compound (35.2 mg). $^1H$ NMR (400 MHz, $CDCl_3$) δ 4.17 (s, 3 H), 7.06 (d, *J* = 3 Hz, 1 H), 7.23 - 7.27 (m, 1 H), 7.32 (d, *J* = 3 Hz, 1 H), 7.51 - 7.55 (m, 1 H), 7.60 - 7.66 (m, 1 H), 8.83 (d, *J* = 2 Hz, 1 H), 9.52 (d, *J* = 2 Hz, 1 H); LCMS ES+ve m/z 286 $[M+H]^+$.

**Example 29**

**7-Methoxy-6-methyl-N-(1-(1-methyl-1*H*-tetrazol-5-yl)piperidin-4-yl)quinoline-3-carboxamide**

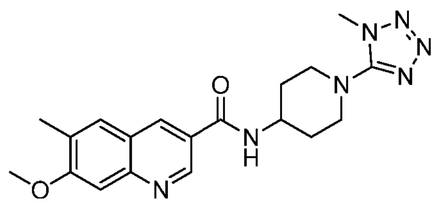

Triethylammonium 7-methoxy-6-methylquinoline-3-carboxylate (Intermediate 6) (20 mg, 0.092 mmol), HATU (38.5 mg, 0.101 mmol) and DIEA (0.048 mL, 0.276 mmol) were stirred in DMF (2 mL) at RT for 5 min. 1-(1-Methyl-1*H*-tetrazol-5-yl)piperidin-4-amine hydrochloride (Intermediate 39) (16.78 mg, 0.092 mmol) was added and the reaction

mixture was stirred at RT for 15 h. The crude reaction mixture was purified by Mass-Directed Auto-Preparative HPLC (Waters SunFire C18 column, $CH_3CN$ / Water + 0.1% formic acid) to yield the title compound (34 mg). $^1H$ NMR (400 MHz, $CD_3SOCD_3$) δ 1.78 (m, 2 H), 1.96 (m, 2 H), 2.36 (s, 3 H), 3.16 (m, 2 H), 3.68 (d, *J* = 13 Hz, 2 H), 3.90 (s, 3 H), 3.99 (s, 3 H), 4.13 (m, 1 H), 7.43 (s, 1 H), 7.82 (s, 1 H), 8.59 (d, *J* = 8 Hz, 1 H), 8.64 (d, *J* = 2 Hz, 1 H), 9.17 (d, *J* = 2 Hz, 1 H); LCMS ES+ve *m/z* 382 $[M+H]^+$.

**Example 30**

**5-Fluoro-7-methoxy-N-(1-(1-methyl-1*H*-tetrazol-5-yl)piperidin-4-yl)quinoline-3-carboxamide**

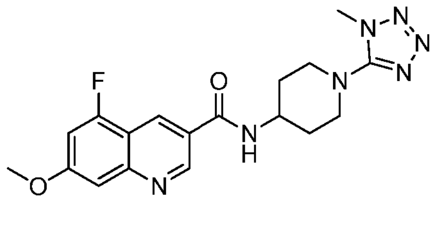

Triethylammonium 5-fluoro-7-methoxyquinoline-3-carboxylate (Intermediate 7) (20 mg, 0.090 mmol), HATU (37.8 mg, 0.099 mmol) and DIEA (0.047 mL, 0.271 mmol) were stirred in DMF (2 mL) at RT for 5 min. 1-(1-Methyl-1*H*-tetrazol-5-yl)piperidin-4-amine (Intermediate 39) (16.48 mg, 0.090 mmol) was added and the reaction mixture was stirred at RT for 15 h. The crude reaction mixture was purified by Mass-Directed Auto-Preparative HPLC (Waters SunFire C18 column, $CH_3CN$ / Water + 0.1 % formic acid) to yield the title compound (20.9 mg). $^1H$ NMR (400 MHz, $CD_3SOCD_3$) δ 1.79 (m, 2 H), 1.96 (m, 2 H), 3.68 (m, 2 H), 3.90 (s, 3 H), 3.98 (s, 3 H), 4.08 (m, 2 H), 6.50 (s, 1 H), 7.28 (dd, *J* = 11, 2 Hz, 1 H), 7.37 (d, *J* = 2 Hz, 1 H), 8.75 (d, *J* = 8 Hz, 1 H), 8.88 (d, *J* = 2 Hz, 1 H), 9.30 (d, *J* = 2 Hz, 1 H); LCMS ES+ve *m/z* 386 $[M+H]^+$.

**Example 31**

**7-Chloro-8-methoxy-N-(1-(1-methyl-1*H*-tetrazol-5-yl)piperidin-4-yl)quinoline-3-carboxamide**

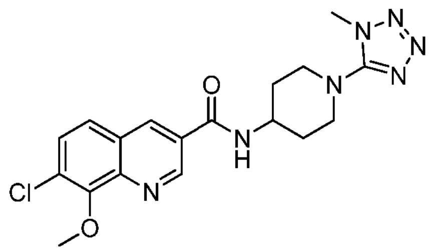

Triethylammonium 7-chloro-8-methoxyquinoline-3-carboxylate (Intermediate 8) (20 mg, 0.084 mmol), HATU (35.2 mg, 0.093 mmol) and DIEA (44.0 μL, 0.252 mmol) were stirred in DMF (2 mL) at RT for 5 min. 1-(1-Methyl-1*H*-tetrazol-5-yl)piperidin-4-amine hydrochloride (Intermediate 39) (15.34 mg, 0.084 mmol) was added and the reaction mixture was stirred at RT for 15 h. The crude reaction mixture was purified by Mass-Directed Auto-Preparative HPLC (Waters SunFire C18 column, $CH_3CN$ / Water + 0.1% formic acid) to yield the title compound (6.7 mg). $^1H$ NMR (400 MHz, $CD_3SOCD_3$) δ ppm 01.80 (m, 2 H), 1.96 (m 2 H), 3.16 (m, 2H), 3.68 (m, 2 H), 3.90 (s, 3 H), 4.08 (m, 1H), 4.12 (s, 3 H), 7.75 (d, *J* = 9 Hz, 1 H), 7.89 (d, *J* = 9 Hz, 1 H), 8.76 (d, *J* = 7 Hz, 1 H), 8.87 (d, *J* = 2 Hz, 1 H), 9.31 (d, *J* = 2 Hz, 1 H); LCMS ES+ve *m/z* 402 $[M+H]^+$.

**Example 32**

**6-Chloro-7-methoxy-N-(1-(1-methyl-1*H*-tetrazol-5-yl)piperidin-4-yl)quinoline-3-carboxamide**

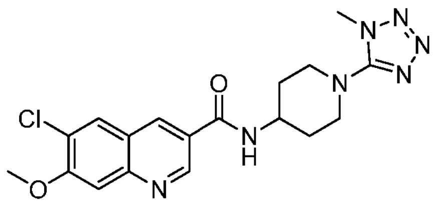

6-Chloro-7-methoxyquinoline-3-carboxylate (Intermediate 9) (50 mg, 0.210 mmol), HATU (88 mg, 0.231 mmol), and DIEA (0.110 mL, 0.631 mmol) were stirred in DMF (2 mL) at RT for 5 min. 1-(1-Methyl-1*H*-tetrazol-5-yl)piperidin-4-amine hydrochloride (Intermediate 39) (38.3 mg, 0.210 mmol) was added and the reaction mixture was stirred at room temperature for 15 h. The crude reaction mixture was purified by Mass-Directed Auto-Preparative HPLC (Waters SunFire C18 column, $CH_3CN$ / Water + 0.1% formic acid) to yield the title compound (25.6 mg). $^1H$ NMR (400 MHz, $CD_3SOCD_3$) $\delta$ 1.79 (m, 2 H), 1.96 (m, 2 H), 3.16 (m, 2 H), 3.68 (m, 2 H), 3.90 (s, 3 H), 4.06 (s, 3 H), 4.14 (m, 1 H), 7.64 (s, 1 H), 8.26 (s, 1 H), 8.67 (d, *J* = 7 Hz, 1 H), 8.73 (d, *J* = 2 Hz, 1 H), 9.25 (d, *J* = 2 Hz, 1 H); LCMS ES+ve m/z 402 $[M+H]^+$.

**Example 33**

**7,8-Dimethoxy-N-(1-(1-methyl-1*H*-tetrazo)-5-yl)piperidin-4-yl)quinoline-3-carboxamide**

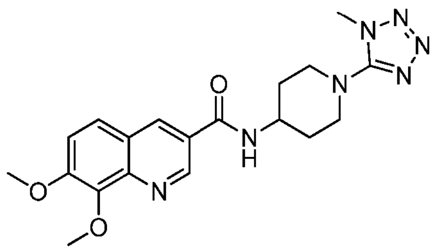

Lithium 7,8-dimethoxyquinoline-3-carboxylate (Intermediate 10) (150 mg, 0.643 mmol), HATU (245 mg, 0.643 mmol), and DIEA (112 μL, 0.643 mmol) was stirred in DMF (2 mL) at RT for 5 min. 1-(1-Methyl-1*H*-tetrazol-5-yl)piperidin-4-amine hydrochloride (Intermediate 39) (117 mg, 0.643 mmol) was added and the reaction mixture was stirred at RT for 15 h. The crude reaction mixture was purified by Mass-Directed Auto-Preparative HPLC (Waters SunFire C18 column, $CH_3CN$ / Water + 0.1 % formic acid) to yield the title compound (126 mg). $^1H$ NMR (400 MHz, $CD_3SOCD_3$) $\delta$ 1.65 - 1.87 (m, 2 H), 1.97 (m, 2 H), 3.16 (t, *J* = 11 Hz, 2 H), 3.68 (d, *J* = 13 Hz, 2 H), 3.90 (s, 3 H), 3.97 (s, 3 H), 4.00 (s, 3 H), 4.14 (m, 1 H), 7.63 (d, *J* = 9 Hz, 1 H), 7.86 (d, *J* = 9 Hz, 1 H), 8.63 (d, *J* = 7 Hz, 1 H), 8.76 (d, *J* = 2 Hz, 1 H), 9.23 (d, *J* = 2 Hz, 1 H); LCMS ES+ve *m/z* 398 $[M+H]^+$.

**Example 34**

**6-Chloro-7-cyclopropyl-N-(1-(1-methyl-1*H*-tetrazol-5-yl)piperidin-4-yl)quinoline-3-carboxamide**

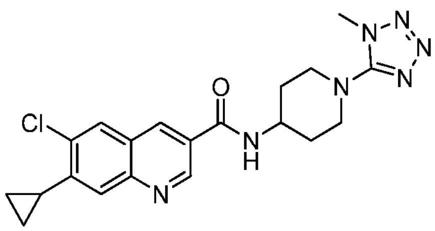

A mixture of 7-bromo-6-chloro-N-(1-(1-methyl-1*H*-tetrazol-5-yl)piperidin-4-yl)quinoline-3-carboxamide (Example 27) (90 mg, 0.20 mmol), cyclopropylboronic acid (39 mg, 0.454 mmol), 1,1'-bis(diphenylphosphino)ferrocenepalladium(II) chloride (13.3 mg, 0.018 mmol), and potassium phosphate (116 mg, 0.545 mmol) in water (1 mL) was treated

with DMF (3 mL) and the mixture was heated in a sealed vial by microwave at 120 °C for 40 min. The crude mixture was evaporated to dryness and the residue was subjected to purification by Mass-Directed Auto-Preparative HPLC (Waters SunFire C18 column, $CH_3CN$ / Water + 0.1 % formic acid) to afford the title compound (37 mg). $^1H$ NMR (400 MHz, $CD_3SOCD_3$) δ 0.92 (m, 2 H), 1.14 (m, 2 H), 1.68 - 1.87 (m, 2 H), 1.99 (m, 2 H), 2.31 (m, 1 H), 3.16 (m, 2 H), 3.68 (d, *J* = 13 Hz, 2 H), 3.90 (s, 3 H), 4.13 (m, 1 H), 7.72 (s, 1 H), 8.24 (s, 1 H), 8.72 (d, *J* = 8 Hz, 1 H), 8.76 (d, *J* = 2 Hz, 1 H), 9.25 (d, *J* = 2 Hz, 1 H); LCMS ES+ve *m*/*z* 412 $[M+H]^+$.

**Example 35**

**(S)-6-Chloro-7-cyclopropyl-N-(2-oxopyrrolidin-3-yl)quinoline-3-carboxamide hydrochloride**

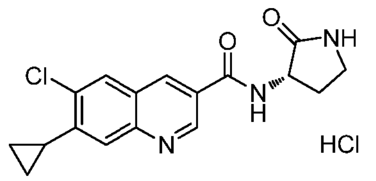

A mixture of 6-chloro-7-cyclopropylquinoline-3-carboxylic acid (Intermediate 11) (100 mg, 0.404 mmol) in DMF (5 mL) was treated with DIEA (0.212 mL, 1.21 mmol) and HATU (169 mg, 0.444 mmol) and the mixture was stirred at RT for 10 min, then treated with (S)-3-aminopyrrolidin-2-one (53 mg, 0.525 mmol) and stirred for 2 h. The mixture was subjected to purification by Mass-Directed Auto-Preparative HPLC (Waters SunFire C18 column, $CH_3CN$ / Water + 0.1 % formic acid). The recovered product was dissolved in MeOH (3 mL), treated with 4N HCl in 1,4-dioxane (0.5 mL) and then evaporated to dryness to afford the title compound (88 mg). $^1H$ NMR (400 MHz, $CD_3SOCD_3$) δ 0.93 (m, 2 H), 1.15 (m, 2 H), 2.06 (m, 1 H), 2.28 - 2.47 (m, 2 H), 3.27 (m, 2 H), 4.62 (m, 1 H), 7.75 (s, 1 H), 7.90 (s, 1 H), 8.28 (s, 1 H), 8.84 (d, *J* = 2 Hz, 1 H), 9.07 (d, *J* = 8 Hz, 1 H), 9.29 (d, *J* = 2 Hz, 1 H); LCMS ES+ve *m*/*z* 330 $[M+H]^+$.

**Example 36**

**(S)-6-Chloro-7-cyclopropyl-N-(2-oxopiperidin-3-yl)quinoline-3-carboxamide hydrochloride**

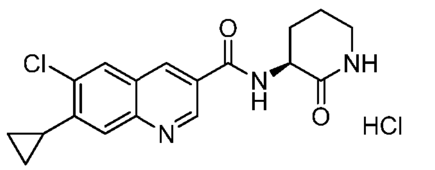

A mixture of 6-chloro-7-cyclopropylquinoline-3-carboxylic acid (Intermediate 11) (52 mg, 0.210 mmol) in DMF (5 mL) was treated with DIEA (0.073 mL, 0.420 mmol) and HATU (84 mg, 0.220 mmol). The mixture was stirred at RT for 10 min and then treated with (S)-3-aminopiperidin-2-one (29 mg, 0.252 mmol). After 6 h, the mixture was evaporated to dryness and the product was subjected to purification by Mass-Directed Auto-Preparative HPLC (Waters SunFire C18 column, $CH_3CN$ /Water + 0.1 % formic acid). The recovered product was dissolved in MeOH (3 mL), treated with 4N HCl in 1,4-dioxane (0.5 mL) and evaporated to dryness to afford the title compound (63 mg). $^1H$ NMR (400 MHz, $CD_3SOCD_3$) δ 0.92 (m, 2 H), 1.17 (m, 2 H), 1.77 (dt, *J* = 7, 3 Hz, 1 H), 1.86 (m, 3 H), 2.08 (m, 1 H), 2.35 (m, 1 H), 3.59 (m, 1 H), 4.45 (m, 2 H), 7.68 (br s, 1 H), 7.77 (s, 1 H), 8.31 (s, 1 H), 8.89 (d, *J* = 2 Hz, 1 H), 9.04 (d, *J* = 8 Hz, 1 H), 9.32 (d, *J* = 2 Hz, 1 H); LCMS ES+ve *m*/*z* 344 $[M+H]^+$.

**Example 37**

**6-Chloro-7-cyclopropyl-N-(2-oxo-1,2,3,4-tetrahydroquinolin-4-yl)quinoline-3-carboxamide**

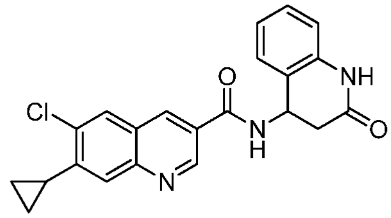

A solution of 6-chloro-7-cyclopropylquinoline-3-carboxylic acid (Intermediate 11) (35 mg, 0.141 mmol) in DMF (5 mL) was treated with DIEA (0.074 mL, 0.424 mmol) and HATU (59 mg, 0.155 mmol) and the mixture was stirred at RT for 10 min. The mixture was then treated with 4-amino-3,4-dihydroquinolin-2(1*H*)-one hydrochloride (34 mg, 0.170 mmol) and stirred for 1 h. The mixture was evaporated to dryness and the product was subjected to purification by Mass-Directed Auto-Preparative HPLC (Waters SunFire C18 column, $CH_3CN$ / Water + 0.1% formic acid) to afford the title compound (43 mg). $^1H$ NMR (400 MHz, $CD_3SOCD_3$) $\delta$ 0.92 (m, 2 H), 1.14 (m, 2 H), 2.31 (m, 1 H), 2.78 (m, 2 H), 5.43 (q, *J* = 7 Hz, 1 H), 6.98 (m, 2 H), 7.25 (m, 1 H), 7.32 (d, *J* = 7 Hz, 1 H), 7.72 (s, 1 H), 8.24 (s, 1 H), 8.80 (d, *J* = 2 Hz, 1 H), 9.27 (m, 2 H), 10.27 (s, 1 H); LCMS ES+ve *m/z* 392 $[M+H]^+$.

**Example 38**

**(*S*)-6-Chloro-7-cyclopropyl-N-(3-oxoisoxazolidin-4-yl)quinoline-3-carboxamide**

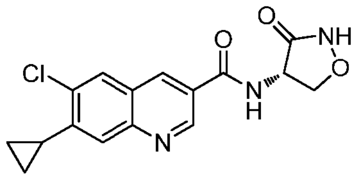

6-Chloro-7-cyclopropylquinoline-3-carboxylic acid (Intermediate 11) (50 mg, 0.202 mmol) and HATU (84 mg, 0.222 mmol) were dissolved in DMF (2 mL) and DIEA (78 mg, 0.606 mmol) and stirred at RT for 10 min. (S)-4-Aminoisoxazolidin-3-one (20.61 mg, 0.202 mmol) was added and the reaction mixture was stirred for 1 h. Purification by Mass-Directed Auto-Preparative HPLC (Waters SunFire C18 column, $CH_3CN$ / Water + 0.1% formic acid) yielded the title compound (7.8 mg). $^1H$ NMR (400 MHz, $CD_3SOCD_3$) $\delta$ 0.93 (dd, *J* = 5, 2 Hz, 2 H), 1.16 (m, 2 H), 2.33 (tt, *J* = 8, 5 Hz, 1 H), 2.78 (d, *J* = 16 Hz, 1 H), 4.64 (t, *J* = 8 Hz, 1 H), 5.10 (m, 1 H), 7.73 (s, 1 H), 8.16 (s, 1 H), 8.26 (s, 1 H), 8.79 (d, *J* = 2 Hz, 1 H), 9.26 (d, *J* = 2 Hz, 1 H), 9.33 (d, *J* = 8 Hz, 1 H); LCMS ES+ve *m/z* 332 $[M+H]^+$.

**Example 39**

**6-Chloro-7-cyclopropyl-N-(transl-4-(2-hydroxypropan-2-yl)cyclohexyl)quinoline-3-carboxamide trifluoroacetic acid salt**

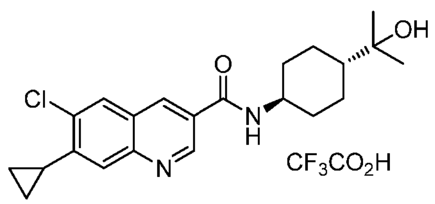

6-chloro-7-cyclopropylquinoline-3-carboxylic acid (Intermediate 11) (78 mg, 0.315 mmol), 2-((trans)-4-aminocyclohexyl)propan-2-ol (63 mg, 0.401 mmol), and HATU (144 mg, 0.378 mmol) were combined, then dissolved in DCM (1.6 mL) and DMF (1 mL). DIEA (0.220 mL, 1.260 mmol) was added and the reaction was capped and stirred at RT overnight. The volatiles were evaporated *in vacuo* then the remaining material was purified by preparative HPLC (Agilent, Phenomonex Luna 5u C18, 100A Axia, 50X30 mm 5 micron, 20-95% acetonitrile in water (0.1 % TFA) over 15 min with

a 2 min organic rinse, 254 nm). The appropriate fractions were concentrated *in vacuo* to afford the title compound as an off white glass (91 mg). $^1$H NMR (400 MHz, $CD_3SOCD_3$) δ 0.91 (m, 2 H), 1.05 (s, 6 H), 1.16 (m, 6 H), 1.33 (m, 2 H), 1.85 (m, 2 H), 1.95 (m, 2 H), 2.31 (m, 1 H), 3.75 (m, 1 H), 7.71 (s, 1 H), 8.24 (s, 1 H), 8.57 (d, *J* = 8 Hz, 1 H), 8.73 (d, *J* = 2 Hz, 1 H), 9.23 (d, *J* = 2 Hz, 1 H); LCMS ES+ve *m*/*z* 387 $[M+H]^+$.

**Example 40**

**6-Chloro-7-cyclopropyl-N-((1s,3s)-3-hydroxy-3-methylcyclobutyl)quinoline-3-carboxamide**

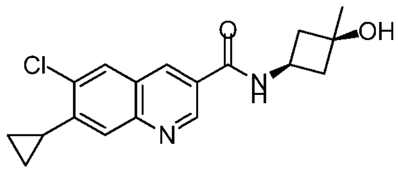

To a solution of 6-chloro-7-cyclopropylquinoline-3-carboxylic acid (Intermediate 11) (100 mg, 0.404 mmol) in DMF (10 mL) at RT, was added (1s,3s)-3-amino-1-methylcyclobutanol hydrochloride (Intermediate 51) (55.6 mg, 0.404 mmol) and DIEA (0.564 mL, 3.23 mmol). Next, n-propylphosphonicacid anhydride ($T_3P$) (50% in EtOAc) (514 mg, 0.808 mmol) was added by a slow dropwise addition and the reaction was allowed to stir at RT overnight. Saturated $NaHCO_3$ solution was added to adjust the pH to 8. The mixture was repeatedly extracted with EtOAc until no product was in the organic layer as observed by TLC (10% MeOH/DCM). The combined extracts were concentrated, absorbed to silica gel, and purified by silica gel chromatography (DCM to 5% MeOH/DCM) to afford the title compound (80 mg, 60% yield). $^1$H NMR (400 MHz, $CD_3SOCD_3$) δ ppm 0.82 - 0.97 (m, 2 H), 1.05 - 1.20 (m, 2 H), 1.27 (s, 3 H), 2.06 - 2.18 (m, 2 H), 2.24 - 2.38 (m, 3 H), 3.94 - 4.08 (m, 1 H), 4.96 - 5.04 (m, 1 H), 7.69 (s, 1 H), 8.20 (s, 1 H), 8.72 (d, *J* = 2 Hz, 1 H), 8.92 (d, *J* = 7 Hz, 1 H), 9.21 (d, *J* = 2 Hz, 1 H). MS (ESI) *m*/*z* 331 (M+1).

**Example 41:**

**(S)-7-Cyclopropyl-6-fluoro-N-(2-oxopyrrolidin-3-yl)quinoline-3-carboxamide hydrochloride**

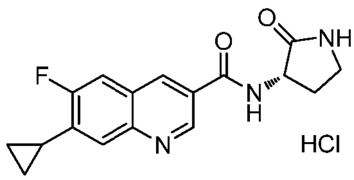

A mixture of 7-cyclopropyl-6-fluoroquinoline-3-carboxylic acid (Intermediate 12) (100 mg, 0.432 mmol) in DMF (5 mL) was treated with DIEA (0.227 mL, 1.30 mmol) and HATU (181 mg, 0.476 mmol) and the mixture was stirred at RT for 10 min, then treated with (S)-3-aminopyrrolidin-2-one (52.0 mg, 0.519 mmol) and left for 2 h. The mixture was subjected to purification by Mass-Directed Auto-Preparative HPLC (Waters SunFire C18 column, $CH_3CN$ / Water + 0.1 % formic acid). The recovered product was dissolved in MeOH (3 mL), treated with 4M HCl in 1,4-dioxane (0.5 mL) and then evaporated to dryness to afford the title compound (73 mg). $^1$H NMR (400 MHz, $CD_3SOCD_3$) δ 0.98 (m, 2 H), 1.15 (m, 2 H), 2.06 (m, 1 H), 2.25 (dd, *J* = 9, 4 Hz, 1 H), 2.41 (m, 1 H), 3.27 (m, 2 H), 4.63 (m, 1 H), 7.73 (d, *J* = 7 Hz, 1 H), 7.90 (m, 2 H), 8.84 (s, 1 H), 9.07 (d, *J* = 8 Hz, 1 H), 9.26 (s, 1 H); LCMS ES+ve *m*/*z* 314 $[M+H]^+$.

**Example 42**

**7-Cyclopropyl-6-fluoro-N-(trans-4-(2-hydroxypropan-2-yl)cyclohexyl)quinoline-3-carboxamide**

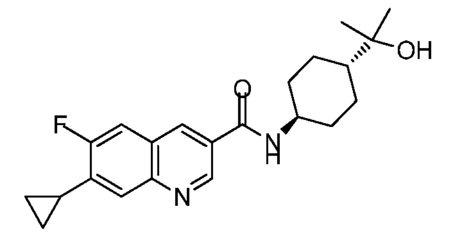

To a suspension of 7-cyclopropyl-6-fluoroquinoline-3-carboxylic acid (Intermediate 12) (200 mg, 0.865 mmol) in DMF (20 mL) at RT, was added EDC (497 mg, 2.59 mmol), HOBT (397 mg, 2.59 mmol), and 2-(trans-4-aminocyclohexyl)propan-2-ol (204 mg, 1.297 mmol). After stirring at RT for 24 h, the solvent was removed under reduced pressure, the residue dissolved in 10% MeOH/DCM (50 mL), and absorbed onto Celite®. The crude material was then purified by reverse phase chromotography (10-50% acetonitrile (with 0.1% TFA)/ water (with 0.1% TFA)) to provide the title compound (100 mg, 24% yield). $^1$H NMR (400 MHz, $CD_3SOCD_3$) δ ppm 0.83 - 1.48 (m, 15 H) 1.83-1.86 (m, 2 H) 1.93-1.96 (m, 2 H) 2.19 - 2.32 (m, 1 H) 3.72-3.75 (m, 1 H) 7.69 (d, *J* = 7 Hz, 1 H) 7.85 (d, *J* = 11 Hz, 1 H) 8.57 (d, *J* = 8 Hz, 1 H) 8.73 (d, *J* = 2 Hz, 1 H) 9.19 (d, *J* = 2 Hz, 1 H). MS (ESI) *m/z* 371 (M+1).

**Example 43**

**7-Cyclopropyl-N-(*trans*-4-((2,2-difluoroethyl)amino)cyclohexyl)-6-fluoroquinoline-3-carboxamide**

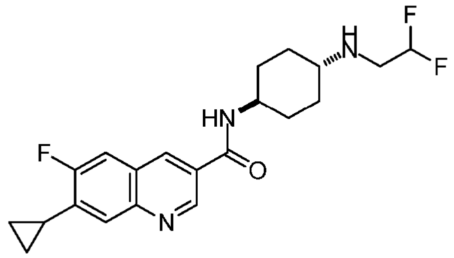

DIEA (0.302 mL, 1.732 mmol) was added to 7-cyclopropyl-6-fluoroquinoline-3-carboxylic acid (intermediate 12) (0.1001 g, 0.433 mmol) in DMF (1.14 mL) at RT. Then, HATU (0.214 g, 0.563 mmol) was added and the reaction mixture was stirred for 5 min. Then, *trans*-N1-(2,2-difluoroethyl)cyclohexane-1,4-diamine dihydrochloride (Intermediate 53) (0.130 g, 0.519 mmol) was added and the reaction mixture was stirred for 66 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc (1:19) to give the title compound (0.0578 g, 31.0 % yield). $^1$H NMR (400 MHz, $CD_3SOCD_3$) δ 0.92-0.98 (m, 2 H), 1.04-1.18 (m, 4 H), 1.36 (br q, *J* = 12 Hz, 2 H), 1.91 (br t, *J* = 14 Hz, 4 H), 2.16-2.26 (m, 1 H), 2.32-2.46 (m, 1 H), 2.90 (br t, *J* = 14 Hz, 2 H), 3.70-3.84 (m, 1 H), 5.95 (tt, *J* = 57, 5 Hz, 1 H), 7.67 (d, *J* = 8 Hz, 1 H), 7.84 (d, *J* = 11 Hz, 1 H), 8.56 (d, *J* = 8 Hz, 1 H), 8.69 (d, *J* = 2 Hz, 1 H), 9.16 (d, *J* = 2 Hz, 1 H); LC-MS (LC-ES) M+H = 392.

**Example 44**

**7-Cyclopropyl-N-(1-(1-methyl-1*H*-tetrazol-5-yl)piperidin-4-yl)quinoline-3-carboxamide**

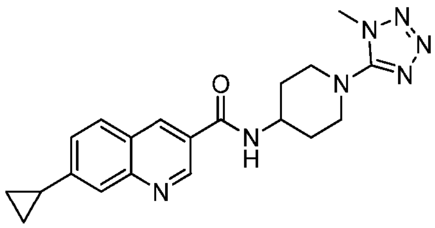

A mixture of 7-bromo-N-(1-(1-methyl-1*H*-tetrazol-5-yl)piperidin-4-yl)quinoline-3-carboxamide (Example 108) (500 mg, 1.20 mmol), cyclopropylboronic acid (234 mg, 2.73 mmol), tricyclohexylphosphine (61 mg, 0.218 mmol) and potassium phosphate (811 mg, 3.82 mmol) was treated with toluene (25 mL) and water (0.7 mL) and the mixture was degassed by the sequential application of vacuum and nitrogen pressure. The mixture was treated with palladium(II) acetate (24.5 mg, 0.109 mmol) and then heated at 100 °C for 7 h. The cooled mixture was evaporated to dryness and then treated with a mixture of water (80 mL) and MeOH (20 mL) and stirred for 30 min. The mixture was filtered and the collected solid was washed with water and dried *in vacuo.* The solid was purified by Mass-Directed Auto-Preparative HPLC (Waters SunFire C18 column, $CH_3CN$ / Water + 0.1% formic acid) to afford the title compound (258 mg). LCMS ES+ve *m*/*z* 378 $[M+H]^+$.

## Example 45

**(S)-7-Cyclopropyl-N-(2-oxopyrrolidin-3-yl)quinoline-3-carboxamide hydrochloride**

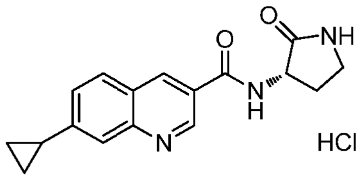

**A. (S)-7-Bromo-N-(2-oxopyrrolidin-3-yl)quinoline-3-carboxamide**

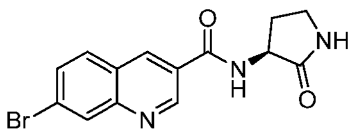

A mixture of 7-bromoquinoline-3-carboxylic acid (200 mg, 0.793 mmol) and (S)-3-aminopyrrolidin-2-one (83 mg, 0.833 mmol) in DMF (5 mL) was treated with DIEA (0.416 mL, 2.380 mmol) and HATU (332 mg, 0.873 mmol) and then stirred at RT for 90 min. The mixture was diluted with DCM (50 mL), treated with saturated aqueous sodium bicarbonate (20 mL) and the mixture was separated and the organic phase was evaporated to dryness. The residue was subjected to purification by Mass-Directed Auto-Preparative HPLC (Waters SunFire C18 column, $CH_3CN$ / Water + 0.1% formic acid) to afford the title compound (178 mg). $^1H$ NMR (400 MHz, $CD_3SOCD_3$) $\delta$ 2.06 (m, 1 H), 2.42 (dddd, *J* = 12, 9, 6, 3 Hz, 1 H), 3.27 (m, 2 H), 4.63 (m, 1 H), 7.85 (dd, *J* = 9, 2 Hz, 1 H), 7.89 (s, 1 H), 8.09 (d, *J* = 9 Hz, 1 H), 8.33 (d, *J* = 2 Hz, 1 H), 8.88 (d, *J* = 2 Hz, 1 H), 9.06 (d, *J* = 8 Hz, 1 H), 9.31 (d, *J* = 2 Hz, 1 H); LCMS ES+ve *m*/*z* 334,336 $[M+H]^+$.

**B. (S)-7-Cyclopropyl-N-(2-oxopyrrolidin-3-yl)quinoline-3-carboxamide hydrochloride**

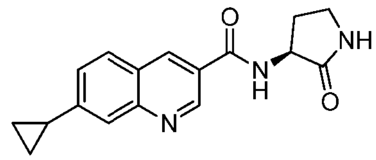

HCl

A mixture of (S)-7-bromo-N-(2-oxopyrrolidin-3-yl)quinoline-3-carboxamide (30 mg, 0.090 mmol), cyclopropylboronic acid (18 mg, 0.204 mmol) and potassium phosphate (61 mg, 0.286 mmol) was treated with toluene (2.5 mL), DMF (0.5 mL), and water (70 μL) and the mixture was degassed by the repeated sequential application of vacuum and nitrogen pressure. The mixture was treated with palladium(II) acetate (1.8 mg, 8.16 μmol) and then heated at 100 °C for 3 h. The cooled mixture was evaporated to dryness and the residue was subjected to purification by Mass-Directed Auto-Preparative HPLC (Waters SunFire C18 column, $CH_3CN$ / Water + 0.1 % formic acid). The recovered product was dissolved in MeOH (3 mL), treated with 4N HCl in 1,4-dioxane (0.5 mL) and re-evaporated to dryness to afford the title compound (17 mg). $^1H$ NMR (400 MHz, $CD_3OD$) δ 1.09 (m, 2 H), 1.38 (m, 2 H), 2.26 (m, 1 H), 2.38 (m, 1 H), 2.65 (dtd, *J* = 15, 6, 2 Hz, 1 H), 3.48 (m, 2 H), 4.72 - 4.88 (m, 1 H), 7.78 (dd, *J* = 9, 1 Hz, 1 H), 7.93 (s, 1 H), 8.33 (d, *J* = 9 Hz, 1 H), 9.48 - 9.56 (m, 2 H); LCMS ES+ve *m*/*z* 296 $[M+H]^+$.

**Example 46**

**6-Fluoro-7-methoxy-N-(1-(1-methyl-1*H*-tetrazol-5-yl)piperidin-4-yl)quinoline-3-carboxamide**

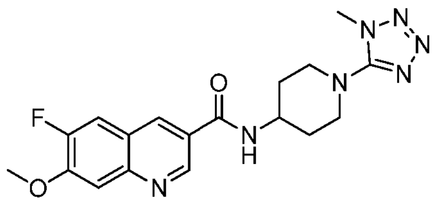

**A. 6-Fluoro-7-methoxy-N-(piperidin-4-yl)quinoline-3-carboxamide, hydrochloride**

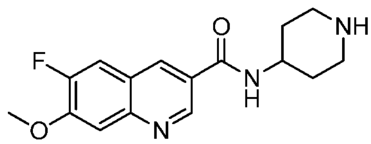

To a solution of triethylammonium 6-fluoro-7-methoxyquinoline-3-carboxylate (Intermediate 13) (146 mg, 0.453 mmol) and HATU (207 mg, 0.543 mmol) in DMF (3 mL) was added DIEA (0.237 mL, 1.359 mmol) and the reaction mixture was stirred at 21 °C for 3 min. tert-Butyl 4-aminopiperidine-1-carboxylate (109 mg, 0.543 mmol) was added and the reaction mixture was stirred at 21 °C for 12 h. The reaction mixture was concentrated in vacuo. The sample was loaded in DCM and purified by SPE on aminopropyl ($NH_2$) 10 g eluting with 1:1 DCM : MeOH. The eluent was concentrated in vacuo. Next, the material obtained was dissolved in 4N HCl in dioxane (0.566 mL, 2.264 mmol) and stirred at RT overnight. The volatiles were evaporated in vacuo to give the title compound (95 mg) as a brown solid. $^1H$ NMR (400 MHz, $CD_3OD$) δ 1.77 (m, 2 H), 2.13 (m, 2 H), 2.94 (m, 2 H), 3.25 - 3.39 (m, 2 H), 4.07 (s, 3 H), 4.15 (m, 1 H), 7.53 (d, *J* = 8 Hz, 1 H), 7.70 (d, *J* = 11 Hz, 1 H), 8.66 (d, *J* = 2 Hz, 1 H), 9.13 (d, *J* = 2 Hz, 1 H);); LCMS ES+ve *m*/*z* 304 $[M+H]^+$.

**B. 6-Fluoro-7-methoxy-N-(1-(1-methyl-1*H*-tetrazol-5-yl)piperidin-4-yl)quinoline-3-carboxamide**

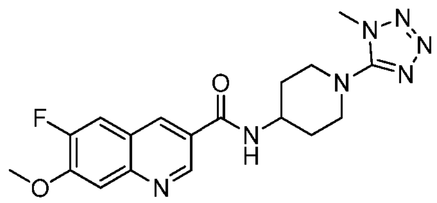

A suspension of 6-fluoro-7-methoxy-N-(piperidin-4-yl)quinoline-3-carboxamide hydrochloride (0.093 g, 0.274 mmol), 5-bromo-1-methyl-1*H*-tetrazole (0.045 g, 0.274 mmol), cesium fluoride (0.249 g, 1.642 mmol) and $Et_3N$ (0.114 mL, 0.821 mmol) in dry, degassed DMF (2 mL) was heated at 90 °C under nitrogen for 16 h. The resulting mixture was cooled, filtered, and concentrated to give a residue that was purified by Mass-Directed Auto-Preparative HPLC (Waters SunFire C18 column, CH3CN / Water + 0.1% formic acid). The material obtained was triturated with diethyl ether (4 mL), and dried *in vacuo* to afford the title compound (60 mg). $^1H$ NMR (400 MHz, $CD_3SOCD_3$) δ 1.78 (m, 2 H), 1.96 (m, 2 H), 3.16 (m, 2 H), 3.68 (d, *J* = 13 Hz, 2 H), 3.90 (s, 3 H), 4.05 (s, 3 H), 4.13 (br s, 1 H), 7.66 (d, *J* = 8 Hz, 1 H), 7.93 (d, *J* = 11 Hz, 1 H), 8.66 (d, *J* = 7 Hz, 1 H), 8.73 (d, *J* = 2 Hz, 1 H), 9.22 (d, *J* = 2 Hz, 1 H); LCMS ES+ve *m/z* 386 $[M+H]^+$.

**Example 47**

**8-Fluoro-7-methoxy-N-(thiazol-2-yl)quinoline-3-carboxamide hydrochloride**

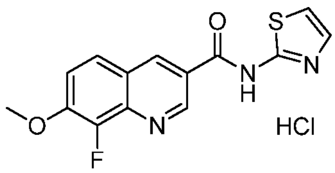

To a solution of triethylammonium 8-fluoro-7-methoxyquinoline-3-carboxylate (Intermediate 14) (75 mg, 0.233 mmol) and HATU (97 mg, 0.256 mmol) in DMF (2 mL) was added DIEA (0.122 mL, 0.698 mmol) and the reaction mixture was stirred at 21 °C for 3 min. 1,3-thiazol-2-amine (28.0 mg, 0.279 mmol) was added and the reaction mixture was stirred at 21 °C for 3 h. Additional HATU (97 mg, 0.256 mmol) and DIEA (0.122 mL, 0.698 mmol) were added and the mixture was stirred for 3 min at 21 °C before 1,3-thiazol-2-amine (28.0 mg, 0.279 mmol) was added. The reaction mixture was stirred for a further 12 h at 21 °C. The reaction mixture was concentrated *in vacuo* and the residue was purified by Mass-Directed Auto-Preparative HPLC (Waters SunFire C18 column, CH3CN / Water + 0.1% formic acid) to give the title compound (22 mg). $^1H$ NMR (400 MHz, $CD_3SOCD_3$) δ 4.07 (s, 3 H), 7.33 (d, *J* = 4 Hz, 1 H), 7.61 (d, *J* = 3 Hz, 1 H), 7.79 (dd, *J* = 9, 8 Hz, 1 H), 8.00 (dd, *J* = 9, 2 Hz, 1 H), 9.12 (s, 1 H), 9.43 (d, *J* = 2 Hz, 1 H); LCMS ES+ve *m/z* 304 $[M+H]^+$.

**Example 48**

**(S)-7-(Difluoromethoxy)-6-fluoro-N-(2-oxopyrrolidin-3-yl)quinoline-3-carboxamide**

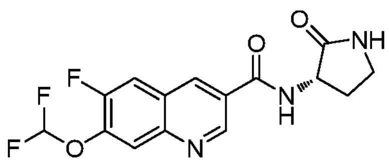

A mixture of 7-(difluoromethoxy)-6-fluoroquinoline-3-carboxylic acid hydrochloride (Intermediate 15) (900 mg, 3.07 mmol) and (S)-3-aminopyrrolidin-2-one hydrochloride (544 mg, 3.98 mmol) in DMF (20 mL) was treated with DIEA (2.141 mL, 12.26 mmol) and then HATU (1515 mg, 3.98 mmol) and the mixture was stirred for 30 min. The mixture was then treated with DCM (200 mL), water (100 mL), and saturated aqueous sodium bicarbonate (100 mL). The mixture was separated and the aqueous phase was extracted with further DCM (3 x 150 mL). The combined organics were evaporated to dryness, absorbed on to flash silica using MeOH as a solvent and then purified by column chromatography

on silica using a 0-15 % MeOH in DCM gradient. Product-containing fractions were combined and evaporated to dryness. The residue was recrystallised from acetonitrile to afford the title compound (774 mg). $^1$H NMR (400 MHz, $CD_3SOCD_3$) δ 2.06 (m, 1 H), 2.42 (m, 1 H), 3.27 (m, 2 H), 4.64 (m, 1 H), 7.59 (t, *J* = 73 Hz, 1 H), 7.90 (s, 1 H), 8.00 (d, *J* = 8 Hz, 1 H), 8.17 (d, *J* = 11 Hz, 1 H), 8.85 (d, *J* = 2 Hz, 1 H), 9.08 (d, *J* = 8 Hz, 1 H), 9.30 (d, *J* = 2 Hz, 1 H); LCMS ES+ve *m/z* 340 $[M+H]^+$.

**Example 49**

**N-(trans-4-((2,2-Dif)loroethyl)amino)cyclohexyl)-7-(difluoromethoxy)-6-fluoroquinoline-3-carboxamide**

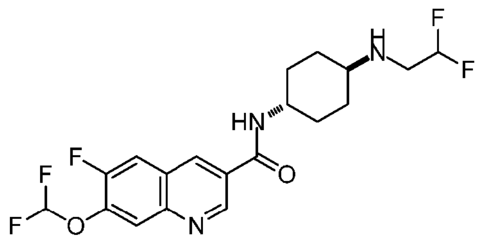

DIEA (0.306 mL, 1.752 mmol) was added to 7-(difluoromethoxy)-6-fluoroquinoline-3-carboxylic acid hydrochloride (Intermediate 15) (0.0901 g, 0.350 mmol) in DMF (3.20 mL) at RT. Then, HATU (0.200 g, 0.526 mmol) was added and the reaction mixture was stirred for 5 min. Then, *trans*-N1-(2,2-difluoroethyl)cyclohexane-1,4-diamine dihydrochloride (intermediate 53) (0.088 g, 0.350 mmol) was added and the reaction mixture was stirred for 16 h. The reaction mixture was purified by RP HPLC eluting with acetonitrile:water with 0.1 % ammonium hydroxide (5:95-100:0), then further purified by silica gel chromatography, eluting with MeOH:EtOAc (1:19) to give the title compound (0.0341 g, 22.1 % yield). $^1$H NMR (400 MHz, $CD_3SOCD_3$) δ 1.10 (q, *J* = 13 Hz, 2 H), 1.36 (q, *J* = 12 Hz, 2 H), 1.76-1.88 (m, 1 H), 1.91 (br t, *J* = 12 Hz, 4 H), 2.34-2.46 (m, 1 H), 2.90 (br t, *J* = 16 Hz, 2 H), 3.74-3.86 (m, 1 H), 5.94 (tt, *J* = 57, 4 Hz, 1 H), 7.58 (t, *J* = 73 Hz, 1 H), 7.97 (d, *J* = 8 Hz, 1 H), 8.14 (d, *J* = 11 Hz, 1 H), 8.62 (d, *J* = 8 Hz, 1 H), 8.77 (d, *J* = 2 Hz, 1 H), 9.25 (d, *J* = 2 Hz, 1 H); LC-MS (LC-ES) M+H = 418.

**Example 50**

**(S)-7-(Difluoromethoxy)-6-fluoro-N-(1-isobutyl-2-oxopyrrolidin-3-yl)quinoline-3-carboxamide**

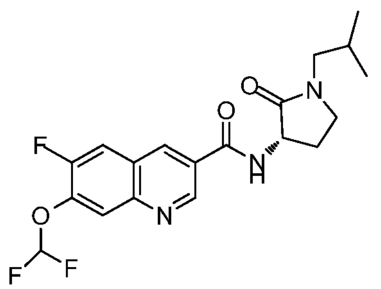

To a suspension of (S)-7-(difluoromethoxy)-6-fluoro-N-(2-oxopyrrolidin-3-yl)quinoline-3-carboxamide (example 48) (100 mg, 0.295 mmol) in DMF (50 mL) at 0 °C was added sodium hydride (25.9 mg, 0.648 mmol). After stirring at RT for 20 min, 1-bromo-2-methylpropane (0.265 mL, 0.265 mmol) was added and the resulting reaction was stirred at RT for 24 h. The solution was diluted with EtOAc and quenched with saturated $NH_4Cl$ (25 mL). The aqueous layer was extracted with EtOAc (3x20 mL), the organic layers combined, and absorbed onto Celite®. The crude material was then purified by reverse phase chromatography (10-50% acetonitrile (with 0.1% TFA)/ water (with 0.1% TFA)) to provide the title compound (25 mg, 17% yield). $^1$H NMR (400 MHz, $CD_3SOCD_3$) δ ppm 0.85 (dd, *J* = 11, 7 Hz, 6 H), 1.83 - 2.09 (m, 2 H), 2.32 - 2.43 (m, 1 H), 2.98 - 3.10 (m, 2 H), 3.30 - 3.41 (m, 2 H), 4.62 - 4.81 (m, 1 H), 7.58 (t, *J* = 73 Hz, 1 H), 7.98 (d, *J* = 8 Hz, 1 H), 8.16 (d, *J* = 11 Hz, 1 H), 8.83 (d, *J* = 2 Hz, 1 H), 9.14 (d, *J* = 8 Hz, 1 H), 9.28 (d, *J* = 2 Hz, 1 H). MS

(ESI) *m/z* 396(M+1).

**Example 51**

**7-(Difluoromethoxy)-6-fluoro-N-(trans-4-(2-hydroxypropan-2-yl)cyclohexyl)quinoline-3-carboxamide**

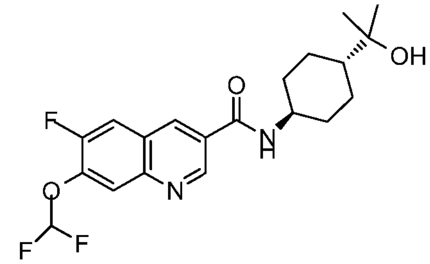

To a suspension of 7-(difluoromethoxy)-6-fluoroquinoline-3-carboxylic acid hydrochloride (Intermediate 15) (100 mg, 0.389 mmol) in DMF (20 mL) at RT, was added EDC (224 mg, 1.167 mmol), HOBT (179 mg, 1.167 mmol), and 2-(trans-4-aminocyclohexyl)propan-2-ol (92 mg, 0.583 mmol). After stirring at RT for 24 h, the solvent was removed under reduced pressure, the residue dissolved in 10% MeOH/DCM (50 mL), and absorbed onto Celite®. The crude material was then purified by reverse phase chromatography, 10-50% acetonitrile (with 0.1% TFA)/ water (with 0.1% TFA) to provide the title compound (55 mg, 28% yield). $^1$H NMR (400 MHz, $CD_3SOCD_3$) δ ppm 0.99 - 1.24 (m, 9 H), 1.26 -1.41 (m, 2 H), 1.84-1.87 (m, 2 H), 1.94-1.97 (m, 2 H), 3.72-3.80 (m, 1 H), 7.59 (t, *J* = 72 Hz, 1 H), 7.98 (d, *J* = 8 Hz, 1 H), 8.15 (d, *J* = 11 Hz, 1 H), 8.63 (d, *J* = 8 Hz, 1 H), 8.79 (d, *J* = 2 Hz, 1 H), 9.27 (d, *J* = 2 Hz, 1 H). MS (ESI) *m/z* 397(M+1).

**Example 52**

**7-(Difluoromethoxy)-6-fluoro-N-((1S,2R)-2-hydroxycyclopentyl)quinoline-3-carboxamide**

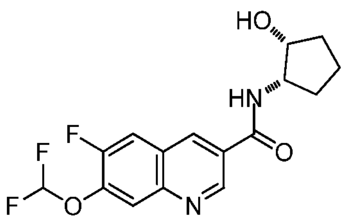

DIEA (1.703 mL, 9.75 mmol) was added to 7-(difluoromethoxy)-6-fluoroquinoline-3-carboxylic acid hydrochloride (Intermediate 15) (0.5015 g, 1.950 mmol) in DMF (8.05 mL) at RT. Then, HATU (0.964 g, 2.54 mmol) was added and the reaction mixture was stirred for 5 min. Then, (1R,2S)-2-aminocyclopentanol hydrochloride (0.322 g, 2.340 mmol) was added and the reaction mixture was stirred for 17 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (4:1) to give the title compound (0.3008 g, 43.1 % yield). $^1$H NMR (400 MHz, $CD_3SOCD_3$) δ 1.46-1.68 (m, 2 H), 1.70-1.90 (m, 4 H), 4.06-4.16 (m, 2 H), 4.74 (d, *J* = 4 Hz, 1 H), 7.58 (t, *J* = 73 Hz, 1 H), 7.97 (d, *J* = 8 Hz, 1 H), 8.13 (d, *J* = 11 Hz, 1 H), 8.39 (d, *J* = 7 Hz, 1 H), 8.84 (d, *J* = 2 Hz, 1 H), 9.30 (d, *J* = 2 Hz, 1 H); LC-MS (LC-ES) M+H = 341.

**Example 53**

**(S)-N-(1-(Cyclopropylmethyl)-2-oxopyrrolidin-3-yl)-7-(difluoromethoxy)-6-fluoroquinoline-3-carboxamide**

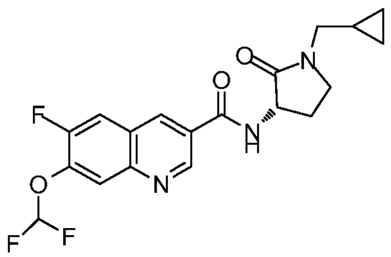

To a suspension of (S)-7-(difluoromethoxy)-6-fluoro-N-(2-oxopyrrolidin-3-yl)quinoline-3-carboxamide (Example 48) (200 mg, 0.590 mmol) in DMF (50 mL) at 0 °C was added sodium hydride (51.9 mg, 1.297 mmol). After stirring at RT for 20 min, (bromomethyl)cyclopropane (0.531 mL, 0.531 mmol) was added and the resulting reaction was stirred at RT for 24 h. The solution was diluted with EtOAc and quenched with saturated $NH_4Cl$ (25 mL). The aqueous layer was extracted with EtOAc (3x20 mL), the organic layers combined, and absorbed onto Celite®. The crude material was then purified by reverse phase chromatography (10-50% acetonitrile (with 0.1% TFA)/ water (with 0.1% TFA)) to provide the title compound (100 mg, 33% yield). $^1H$ NMR (400 MHz, $CD_3SOCD_3$) δ ppm 0.18 - 0.30 (m, 2 H), 0.43 - 0.54 (m, 2 H), 0.86 - 1.00 (m, 1 H), 1.93 - 2.07 (m, 1 H), 2.37 - 2.47 (m, 1 H), 2.97 - 3.26 (m, 2 H), 3.41 - 3.56 (m, 2 H), 4.67 - 4.83 (m, 1 H), 7.60 (t, *J* = 73 Hz, 1 H), 7.99 (d, *J* = 8 Hz, 1 H), 8.18 (d, *J* = 11 Hz, 1 H), 8.85 (d, *J* = 2 Hz, 1 H), 9.18 (d, *J* = 8 Hz, 1 H), 9.30 (d, *J* = 2 Hz, 1 H). MS (ESI) *m/z* 394(M+1).

**Example 54**

**7-(Difluoromethoxy)-6-fluoro-N-(2-oxo-1,2,3,4-tetrahydroquinolin-4-yl)quinoline-3-carboxamide**

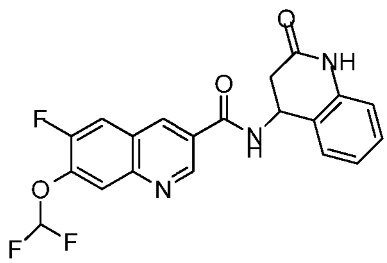

To a suspension of 7-(difluoromethoxy)-6-fluoroquinoline-3-carboxylic acid hydrochloride (Intermediate 15) (100 mg, 0.389 mmol) in DMF (20 mL) at RT, was added EDC (224 mg, 1.167 mmol), HOBt (179 mg, 1.167 mmol), and 4-amino-3,4-dihydroquinolin-2(1H)-one (95 mg, 0.583 mmol). After stirring at RT for 24 h, the solvent was removed under reduced pressure, the residue dissolved in 10% MeOH/DCM (50 mL), and absorbed onto Celite®. The crude material was then purified by reverse phase chromatography (10-50% acetonitrile (with 0.1% TFA)/ water (with 0.1% TFA)) to provide the title compound (72 mg, 36% yield). $^1H$ NMR (400 MHz, $CD_3SOCD_3$) δ ppm 2.68 - 2.88 (m, 2 H), 5.38 - 5.47 (m, 1 H), 6.91 - 7.06 (m, 2 H), 7.23 - 7.28 (m, 1 H), 7.32 (d, *J* = 8 Hz, 1 H), 7.60 (t, *J* = 72 Hz, 1 H), 7.99 (d, *J* = 8 Hz, 1 H), 8.16 (d, *J* = 11 Hz, 1 H), 8.86 (d, *J* = 2 Hz, 1 H), 9.27 - 9.38 (m, 2 H), 10.31 (s, 1 H). MS (ESI) *m/z* 402(M+1).

**Example 55**

**7-(Difluoromethoxy)-6-fluoro-N-(trans-4-methoxycyclohexyl)quinoline-3-carboxamide**

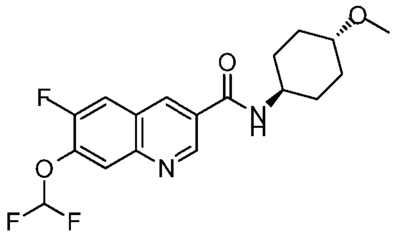

To a suspension of 7-(difluoromethoxy)-6-fluoroquinoline-3-carboxylic acid hydrochloride (Intermediate 15) (100 mg, 0.389 mmol) in DMF (20 mL) at RT, was added EDC (224 mg, 1.167 mmol), HOBt (179 mg, 1.167 mmol), and trans-4-methoxycyclohexanamine (75 mg, 0.583 mmol). After stirring at RT for 24 h, the solvent was removed under reduced pressure, the residue dissolved in 10% MeOH/DCM (50 mL), and absorbed onto Celite®. The crude material was then purified by reverse phase chromatography (10-50% acetonitrile (with 0.1% TFA)/ water (with 0.1% TFA)) to provide the title compound (50 mg, 27% yield). $^1H$ NMR (400 MHz, $CD_3SOCD_3$) δ ppm 1.18 - 1.32 (m, 2 H), 1.35 - 1.51 (m, 2 H), 1.89 - 1.98 (m, 2 H), 2.01 - 2.13 (m, 2 H), 3.10-3.15 (m, 1 H), 3.25 (s, 3 H), 3.79-3.86 (m, 1 H), 7.59 (t, *J* = 73 Hz, 1 H), 7.98 (d, *J* = 8 Hz, 1 H), 8.15 (d, *J* = 11 Hz, 1 H), 8.64 (d, *J* = 8 Hz, 1 H), 8.79 (d, *J* = 2 Hz, 1 H), 9.26 (d, *J* = 2 Hz, 1 H). MS (ESI) *m/z* 369(M+1).

## Example 56

### 7-(Difluoromethoxy)-6-fluoro-N-(trans-4-(methylsulfonamido)cyclohexyl)quinoline-3-carboxamide

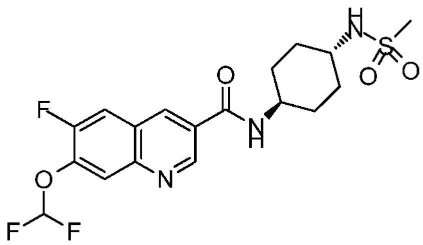

To a suspension of 7-(difluoromethoxy)-6-fluoroquinoline-3-carboxylic acid hydrochloride (Intermediate 15) (100 mg, 0.389 mmol) in DMF (20 mL) at RT, was added EDC (224 mg, 1.167 mmol), HOBt (179 mg, 1.167 mmol), and N-(trans-4-aminocyclohexyl)methanesulfonamide (112 mg, 0.583 mmol). After stirring at RT for 24 h, the solvent was removed under reduced pressure, the residue dissolved in 10% MeOH/DCM, and absorbed onto Celite®. The crude material was then purified by reverse phase chromatography (10-50% acetonitrile (with 0.1% TFA)/ water (with 0.1% TFA)) to provide the title compound (55 mg, 26% yield). $^1H$ NMR (400 MHz, $CD_3SOCD_3$) δ ppm 1.27 - 1.59 (m, 4 H), 1.83 - 2.05 (m, 4 H), 2.93 (s, 3H), 3.11 - 3.21 (m, 1 H), 3.71 - 3.86 (m, 1 H), 7.05 (d, *J* = 7 Hz, 1 H), 7.59 (t, *J* = 73 Hz, 1 H), 7.98 (d, *J* = 8 Hz, 1 H), 8.16 (d, *J* = 11 Hz, 1 H), 8.67 (d, *J* = 8 Hz, 1 H), 8.79 (d, *J* = 2 Hz, 1 H), 9.26 (d, *J* = 2 Hz, 1 H). MS (ESI) *m/z* 432(M+1).

## Example 57

### 7-(Difluoromethoxy)-6-fluoro-N-(*trans*-4-((1,1,1-trifluoropropan-2-yl)amino)cyclohexyl)quinoline-3-carboxamide

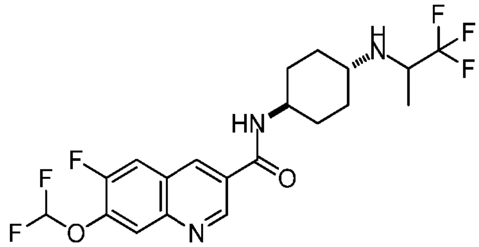

DIEA (0.504 mL, 2.88 mmol) was added to 7-(difluoromethoxy)-6-fluoroquinoline-3-carboxylic acid hydrochloride (Intermediate 15) (0.1483 g, 0.577 mmol) in DMF (2.380 mL) at RT. Then, HATU (0.285 g, 0.750 mmol) was added and the reaction mixture was stirred for 5 min. Then, *trans*-N1-(1,1,1-trifluoropropan-2-yl)cyclohexane-1,4-diamine dihydrochloride (Intermediate 54) (0.180 g, 0.634 mmol) was added and the reaction mixture was stirred for 64 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (7:3) to give the title compound (0.1587 g, 58.2 % yield). $^1$H NMR (400 MHz, $CD_3SOCD_3$) δ 1.11 (q, *J* = 11 Hz, 2 H), 1.15 (d, *J* = 7 Hz, 3 H), 1.36 (dq, *J* = 13, 3 Hz, 2 H), 1.78 (t, *J* = 6 Hz, 1 H), 1.82-2.02 (m, 4 H), 2.42-2.54 (m, 1 H), 3.26-3.42 (m, 1 H), 3.70-3.84 (m, 1 H), 7.58 (t, *J* = 73 Hz, 1 H), 7.97 (d, *J* = 8 Hz, 1 H), 8.14 (d, *J* = 11 Hz, 1 H), 8.61 (d, *J* = 8 Hz, 1 H), 8.77 (d, *J* = 2 Hz, 1 H), 9.24 (d, *J* = 2 Hz, 1 H); LC-MS (LC-ES) M+H = 450.

**Example 58**

**7-(Difluoromethoxy)-6-fluoro-N-(1-(2,2,2-trifluoroethyl)piperidin-4-yl)quinoline-3-carboxamide**

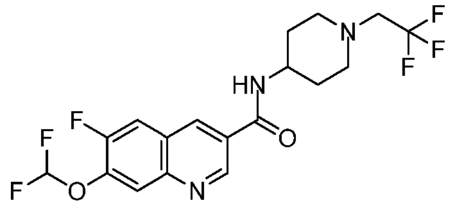

DIEA (0.369 mL, 2.115 mmol) was added to 7-(difluoromethoxy)-6-fluoroquinoline-3-carboxylic acid hydrochloride (Intermediate 15) (0.1088 g, 0.423 mmol) in DMF (1.746 mL) at RT. Then, HATU (0.209 g, 0.550 mmol) was added and the reaction mixture was stirred for 5 minutes. Then, 1-(2,2,2-trifluoroethyl)piperidin-4-amine dihydrochloride (Intermediate 55) (0.119 g, 0.465 mmol) was added and the reaction mixture was stirred for 17 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (7:3) to give the title compound (0.1140 g, 60.8 % yield). $^1$H NMR (400 MHz, $CD_3SOCD_3$) δ 1.60 (dq, *J* = 12, 3 Hz, 2 H), 1.82 (br d, *J* = 10 Hz, 2 H), 2.45 (t, *J* = 11 Hz, 2 H), 2.94 (br d, *J* = 12 Hz, 2 H), 3.17 (q, *J* = 10 Hz, 2 H), 3.76-3.90 (m, 1 H), 7.59 (t, *J* = 73 Hz, 1 H), 7.97 (d, *J* = 8 Hz, 1 H), 8.15 (d, *J* = 11 Hz, 1 H), 8.67 (d, *J* = 8 Hz, 1 H), 8.78 (d, *J* = 2 Hz, 1 H), 9.25 (d, *J* = 2 Hz, 1 H); LC-MS (LC-ES) M+H = 422.

**Example 59**

**7-(Difluoromethoxy)-6-fluoro-N-(*trans*-3-((2,2,2-trifluoroethyl)amino)cyclobutyl)quinoline-3-carboxamide**

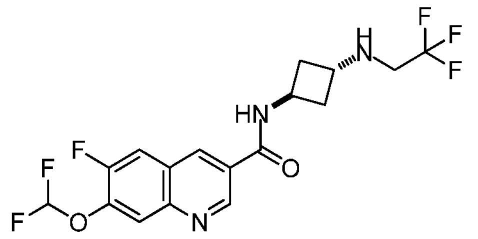

DIEA (0.381 mL, 2.183 mmol) was added to 7-(difluoromethoxy)-6-fluoroquinoline-3-carboxylic acid hydrochloride (Intermediate 15) (0.1123 g, 0.437 mmol) in DMF (1.802 mL) at RT. Then, HATU (0.216 g, 0.568 mmol) was added and the reaction mixture was stirred for 5 min. Then, *trans*-N1-(2,2,2-trifluoroethyl)cyclobutane-1,3-diamine dihydrochloride (Intermediate 56) (0.116 g, 0.480 mmol) was added and the reaction mixture was stirred for 1 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc to give the title compound (0.0938 g, 50.1 % yield). $^1H$ NMR (400 MHz, $CD_3SOCD_3$) $\delta$ 2.08-2.18 (m, 2 H), 2.18-2.28 (m, 2 H), 2.70-2.84 (m, 1 H), 3.10-3.26 (m, 2 H), 3.43 (br s, 1 H), 4.50 (h, *J* = 7 Hz, 1 H), 7.59 (t, *J* = 73 Hz, 1 H), 7.97 (d, *J* = 8 Hz, 1 H), 8.14 (d, *J* = 11 Hz, 1 H), 8.80 (d, *J* = 2 Hz, 1 H), 9.04 (d, *J* = 7 Hz, 1 H), 9.27 (d, *J* = 2 Hz, 1 H); LC-MS (LC-ES) M+H = 408.

**Example 60**

**7-(Difluoromethoxy)-6-fluoro-N-(trans-4-(2-hydroxy-2-methylpropoxy)cyclohexyl)quinoline-3-carboxamide**

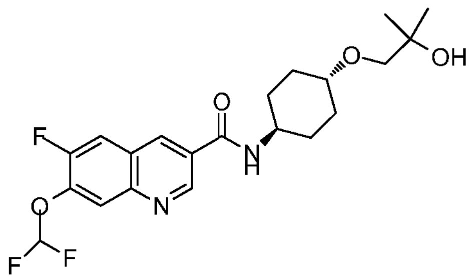

To a solution of 7-(difluoromethoxy)-6-fluoroquinoline-3-carboxylic acid hydrochloride (Intermediate 15) (100 mg, 0.389 mmol) in DMF (10 mL) at RT, was added 1-((trans-4-aminocyclohexyl)oxy)-2-methylpropan-2-ol (Intermediate 42) (87 mg, 0.467 mmol), followed by HATU (222 mg, 0.583 mmol) and DIEA (0.204 mL, 1.167 mmol). After stirring at RT for 24 h, the solvent was removed under reduced pressure, the residue dissolved in 10% MeOH/DCM (50 mL), and absorbed onto Celite®. The crude material was then purified by reverse phase chromatography (10-50% acetonitrile (with 0.1% TFA)/ water (with 0.1% TFA)) to provide the title compound (120 mg, 57% yield). $^1H$ NMR (400 MHz, $CD_3OD$) $\delta$ ppm 1.16 (s, 6 H), 1.32 - 1.58 (m, 4 H), 2.00 - 2.24 (m, 4 H), 3.24 - 3.32 (m, 1 H), 3.85 - 4.03 (m, 1 H), 4.84 (s, 2 H), 7.15 (t, *J* = 73 Hz, 1 H), 7.81 - 7.98 (m, 2 H), 8.59 (d, *J* = 8 Hz, 1 H), 8.74 (d, *J* = 2 Hz, 1 H), 9.20 (d, *J* = 2 Hz, 1 H). MS (ESI) *m/z* 427(M+1).

**Example 61**

**7-(Difluoromethoxy)-6-fluoro-N-(6-((2,2,2-trifluoroethyl)amino)spiro[3.3]heptan-2-yl)quinoline-3-carboxamide**

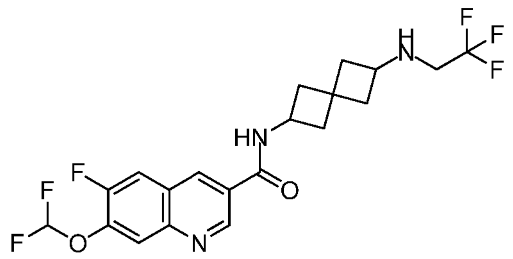

DIEA (0.536 mL, 3.07 mmol) was added to 7-(difluoromethoxy)-6-fluoroquinoline-3-carboxylic acid hydrochloride (Intermediate 15) (0.1577 g, 0.613 mmol) in DMF (2.5 mL) at RT. Then, HATU (0.303 g, 0.797 mmol) was added and the reaction mixture was stirred for 5 min. Then, racemic N2-(2,2,2-trifluoroethyl)spiro[3.3]heptane-2,6-diamine dihydrochloride (Intermediate 57) (0.190 g, 0.675 mmol) was added and the reaction mixture was stirred for 3 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (4:1) to give racemic 7-(difluoromethoxy)-6-fluoro-N-(6-((2,2,2-trifluoroethyl)amino)spiro[3.3]heptan-2-yl)quinoline-3-carboxamide (0.1510 g, 52.3 % yield). $^1$H NMR (400 MHz, $CD_3SOCD_3$) δ 1.68-1.82 (m, 2 H), 2.04-2.18 (m, 3 H), 2.18-2.28 (m, 1 H), 2.28-2.42 (m, 2 H), 2.46-2.54 (m, 1 H), 3.04-3.18 (m, 3 H), 4.35 (h, *J* = 8 Hz, 1 H), 7.58 (t, *J* = 73 Hz, 1 H), 7.97 (d, *J* = 8 Hz, 1 H), 8.13 (d, *J* = 11 Hz, 1 H), 8.78 (d, *J* = 2 Hz, 1 H), 8.94 (d, *J* = 8 Hz, 1 H), 9.24 (d, *J* = 2 Hz, 1 H); LC-MS (LC-ES) M+H = 448.

**Examples 62 and 63**

**7-(Difluoromethoxy)-6-fluoro-N-(trans-4-(((R)-1,1,1-trifluoropropan-2-yl)amino)cyclohexyl)quinoline-3-carboxamide and 7-(Difluoromethoxy)-6-fluoro-N-(trans-4-(((S)-1,1,1-trifluoropropan-2-yl)amino)cyclohexyl)quinoline-3-carboxamide**

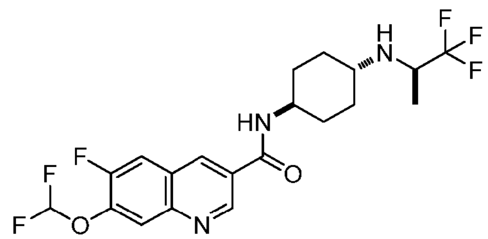

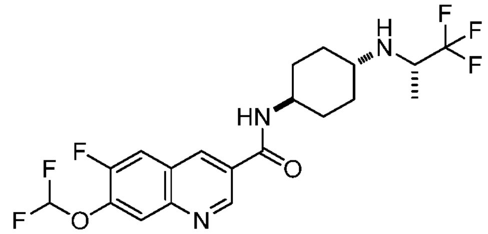

Racemic 7-(difluoromethoxy)-6-fluoro-N-(*trans*-4-((1,1,1-trifluoropropan-2-yl)amino)cyclohexyl)quinoline-3-carboxamide (Example 57) (0.1269 g, 0.282 mmol) was separated into its enantiomers via super critical fluid chromatography on a chiral ES Industries CC4 column eluting with MeOH:carbon dioxide (1:9) to give 7-(difluoromethoxy)-6-fluoro-N-(trans-4-(((R)-1,1,1-trifluoropropan-2-yl)amino)cyclohexyl)quinoline-3-carboxamide (0.0358 g, 28.2 % yield) as the first enantiomer to elute and 7-(difluoromethoxy)-6-fluoro-N-(trans-4-(((S)-1,1,1-trifluoropropan-2-yl)amino)cyclohexyl)quinoline-3-carboxamide (0.0281 g, 22.1 % yield) as the last enantiomer to elute. The structures were assigned by vibrational circular dichroism.

**7-(Difluoromethoxy)-6-fluoro-N-(trans-4-(((R)-1,1,1-trifluoropropan-2-yl)amino)cyclohexyl)quinoline-3-carboxamide:** $^1$H NMR (400 MHz, $CD_3SOCD_3$) δ 1.11 (q, *J* = 11 Hz, 2 H), 1.15 (d, *J* = 7 Hz, 3 H), 1.36 (dq, *J* = 13, 3 Hz, 2 H), 1.78 (t, *J* = 6 Hz, 1 H), 1.82-2.02 (m, 4 H), 2.42-2.54 (m, 1 H), 3.26-3.42 (m, 1 H), 3.70-3.84 (m, 1 H), 7.58 (t,

$J$ = 73 Hz, 1 H), 7.97 (d, $J$ = 8 Hz, 1 H), 8.14 (d, $J$ = 11 Hz, 1 H), 8.61 (d, $J$ = 8 Hz, 1 H), 8.77 (d, $J$ = 2 Hz, 1 H), 9.24 (d, $J$ = 2 Hz, 1 H); LC-MS (LC-ES) M+H = 450.

**7-(Difluoromethoxy)-6-fluoro-N-(trans-4-(((S)-1,1,1 -trifluoropropan-2-yl)amino)cyclohexyl)quinoline-3-carboxamide:** $^1H$ NMR (400 MHz, $CD_3SOCD_3$) δ 1.11 (q, $J$ = 11 Hz, 2 H), 1.15 (d, $J$ = 7 Hz, 3 H), 1.36 (dq, $J$ = 13, 3 Hz, 2 H), 1.78 (t, $J$ = 6 Hz, 1 H), 1.82-2.02 (m, 4 H), 2.42-2.54 (m, 1 H), 3.26-3.42 (m, 1 H), 3.70-3.84 (m, 1 H), 7.58 (t, $J$ = 73 Hz, 1 H), 7.97 (d, $J$ = 8 Hz, 1 H), 8.14 (d, $J$ = 11 Hz, 1 H), 8.61 (d, $J$ = 8 Hz, 1 H), 8.77 (d, $J$ = 2 Hz, 1 H), 9.24 (d, $J$ = 2 Hz, 1 H); LC-MS (LC-ES) M+H = 450.

## Example 64

### 7-(Difluoromethoxy)-6-fluoro-N-((1R,3R)-3-((2,2,2-trifluoroethyl)amino)cyclopentyl)quinoline-3-carboxamide

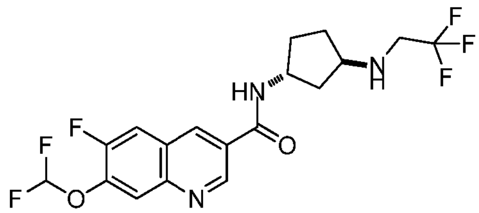

DIEA (0.282 mL, 1.616 mmol) was added to 7-(difluoromethoxy)-6-fluoroquinoline-3-carboxylic acid hydrochloride (Intermediate 15) (0.1385 g, 0.539 mmol) in DMF (2.411 mL) at RT. Then, HATU (0.266 g, 0.700 mmol) was added and the reaction mixture was stirred for 5 min. Then, (1R,3R)-N1-(2,2,2-trifluoroethyl)cyclopentane-1,3-diamine dihydrochloride (Intermediate 58) (0.151 g, 0.592 mmol) was added and the reaction mixture was stirred for 3 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (4:1) to give the title compound (0.1283 g, 53.7 % yield). $^1H$ NMR (400 MHz, $CD_3SOCD_3$) δ 1.32-1.44 (m, 1 H), 1.48-1.60 (m, 1 H), 1.78 (t, $J$ = 7 Hz, 2 H), 1.90-2.02 (m, 1 H), 2.02-2.12 (m, 1 H), 2.39 (q, $J$ = 7 Hz, 1 H), 3.14-3.32 (m, 3 H), 4.42 (h, $J$ = 7 Hz, 1 H), 7.58 (t, $J$ = 73 Hz, 1 H), 7.97 (d, $J$ = 8 Hz, 1 H), 8.13 (d, $J$ = 11 Hz, 1 H), 8.71 (d, $J$ = 7 Hz, 1 H), 8.77 (d, $J$ = 2 Hz, 1 H), 9.25 (d, $J$ = 2 Hz, 1 H); LC-MS (LC-ES) M+H = 422.

## Example 65

### N-(1-(2,2-Difluoroethyl)piperidin-4-yl)-7-(difluoromethoxy)-6-fluoroquinoline-3-carboxamide

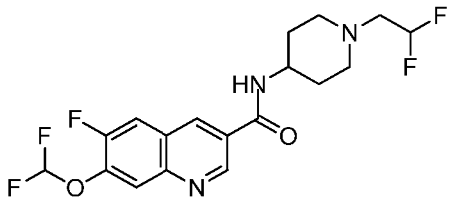

DIEA (0.208 mL, 1.189 mmol) was added to 7-(difluoromethoxy)-6-fluoroquinoline-3-carboxylic acid hydrochloride (Intermediate 15) (0.1019 g, 0.396 mmol) in DMF (1.774 mL) at RT. Then, HATU (0.196 g, 0.515 mmol) was added and the reaction mixture was stirred for 5 min. Then, 1-(2,2-difluoroethyl)piperidin-4-amine (Intermediate 59) (0.072 g, 0.436 mmol) was added and the reaction mixture was stirred for 17 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc to give the title compound (0.0742 g, 44.1 % yield). $^1H$ NMR (400 MHz, $CD_3SOCD_3$) δ 1.59 (dq, $J$ = 11, 3 Hz, 2 H), 1.82 (br d, $J$ = 10 Hz, 2 H), 2.26 (dd, $J$ = 12, 10 Hz, 2 H), 2.72 (dt, $J$ = 16, 4 Hz, 2 H), 2.92 (br d, $J$ = 12 Hz, 2 H), 3.74-3.88 (m, 1 H), 6.14 (tt, $J$ = 56, 4 Hz, 1 H), 7.58 (t, $J$ = 73 Hz, 1 H), 7.97 (d, $J$ = 8 Hz, 1 H), 8.15 (d, $J$ = 11 Hz, 1 H), 8.67 (d, $J$ = 8 Hz, 1 H), 8.78 (d, J = 2 Hz, 1 H), 9.25 (d, J = 2 Hz, 1 H); LC-MS (LC-ES) M+H = 404.

**Example 66**

**7-(Difluoromethoxy)-6-fluoro-N-(*trans*-4-((1,1,1-trifluoro-2-methylpropan-2-yl)amino)cvclohexyl)quinoline-3-carboxamide**

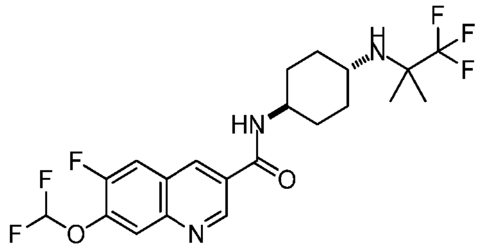

DIEA (0.237 mL, 1.354 mmol) was added to 7-(difluoromethoxy)-6-fluoroquinoline-3-carboxylic acid hydrochloride (Intermediate 15) (0.1161 g, 0.451 mmol) in DMF (2.021 mL) at RT. Then, HATU (0.223 g, 0.587 mmol) was added and the reaction mixture was stirred for 5 min. Then, *trans*-N1-(1,1,1-trifluoro-2-methylpropan-2-yl)cyclohexane-1,4-diamine (Intermediate 60) (0.101 g, 0.451 mmol) was added and the reaction mixture was stirred for 17 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (7:3) to give the title compound (0.0563 g, 25.6 % yield). $^1$H NMR (400 MHz, $CD_3SOCD_3$) δ 1.20 (s, 6 H), 1.21 (q, *J* = 10 Hz, 2 H), 1.39 (q, *J* = 14 Hz, 2 H), 1.76-1.92 (m, 5 H), 2.56-2.68 (m, 1 H), 3.66-3.80 (m, 1 H), 7.58 (t, *J* = 73 Hz, 1 H), 7.97 (d, *J* = 8 Hz, 1 H), 8.14 (d, *J* = 11 Hz, 1 H), 8.60 (d, *J* = 8 Hz, 1 H), 8.76 (d, *J* = 2 Hz, 1 H), 9.24 (d, *J* = 2 Hz, 1 H); LC-MS (LC-ES) M+H = 464.

**Example 67**

**7-(Difluoromethoxy)-N-(*trans*-4-((1,1-difluoropropan-2-yl)amino)cyclohexyl)-6-fluoroquinoline-3-carboxamide**

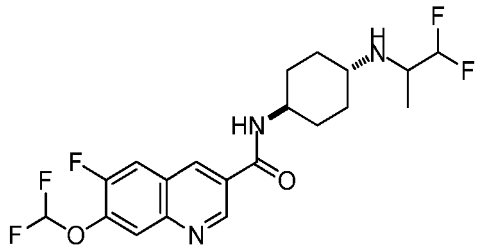

DIEA (11.55 mL, 66.1 mmol) was added to 7-(difluoromethoxy)-6-fluoroquinoline-3-carboxylic acid hydrochloride (Intermediate 15) (3.40 g, 13.22 mmol) in DMF (21.5 mL) at RT. Then, HATU (6.54 g, 17.19 mmol) was added and the reaction mixture was stirred for 5 min. Then, *trans*-N1-(1,1-difluoropropan-2-yl)cyclohexane-1,4-diamine (Intermediate 61) (2.67 g, 13.88 mmol) was added and the reaction mixture was stirred for 2 h. The reaction mixture was concentrated. The reaction mixture was purified by silica gel chromatography, eluting with MeOH:EtOAc (1:49), then further purified by RP HPLC eluting with acetonitrile:water with 0.1% ammonium hydroxide (5:95-100:0) to give the title compound (4.68 g, 78 % yield). $^1$H NMR (400 MHz, $CD_3SOCD_3$) δ 1.02 (d, *J* = 7 Hz, 3 H), 1.11 (q, *J* = 12 Hz, 2 H), 1.37 (dq, *J* = 12, 4 Hz, 2 H), 1.44-1.52 (m, 1 H), 1.84-1.98 (m, 4 H), 2.44-2.56 (m, 1 H), 2.90-3.06 (m, 1 H), 3.70-3.84 (m, 1 H), 5.77 (dt, *J* = 56, 4 Hz, 1 H), 7.58 (t, *J* = 73 Hz, 1 H), 7.97 (d, *J* = 8 Hz, 1 H), 8.14 (d, *J* = 11 Hz, 1 H), 8.62 (d, *J* = 8 Hz, 1 H), 8.77 (d, *J* = 2 Hz, 1 H), 9.24 (d, *J* = 2 Hz, 1 H); LC-MS (LC-ES) M+H = 432.

**Example 68**

**7-(Difluoromethoxy)-6-fluoro-N-(4-morpholinocyclohexyl)quinoline-3-carboxamide trifluoroacetic acid salt**

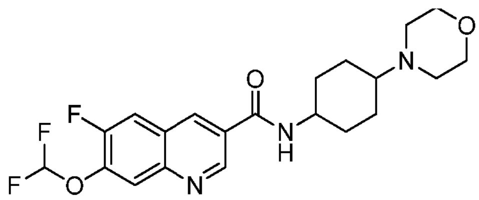

**A. 7-(Difluoromethoxy)-6-fluoro-N-(1,4-dioxaspiro[4.5]decan-8-yl)quinoline-3-carboxamide**

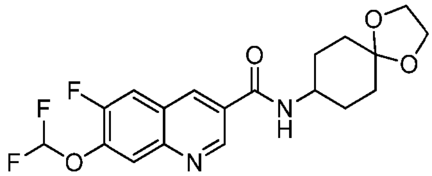

To a solution of 7-(difluoromethoxy)-6-fluoroquinoline-3-carboxylic acid hydrochloride (1.404 g, 5.46 mmol, Intermediate 15) and HATU (2.180 g, 5.73 mmol) in DMF (15 mL) was added DIEA (1.049 mL, 6.01 mmol) and the mixture was stirred 1 h at RT. A solution of 1,4-dioxaspiro[4.5]decan-8-amine (1.030 g, 6.55 mmol) in DMF (2 mL) was added *via* syringe and stirring continued. After 1 h the mixture was poured into sat. $NaHCO_3$ and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound (0.9981 g, 46 % yield) as a faintly yellow solid. $^1$H NMR (400 MHz, $CDCl_3$) $\delta$ ppm 1.58 - 1.91 (m, 6 H), 2.06 - 2.15 (m, 2 H), 3.94 - 4.03 (m, 4 H), 4.08 - 4.22 (m, 1 H), 6.17 (d, *J* = 8 Hz, 1 H), 6.76 (t, *J* = 72 Hz, 1 H), 7.63 (d, *J* = 10 Hz, 1 H), 7.95 (d, *J* = 8 Hz, 1 H), 8.50 (d, *J* = 2 Hz, 1 H), 9.18 (d, *J* = 2 Hz, 1 H). MS (ESI) *m/z* 397 (M+H).

**B. 7-(Difluoromethoxy)-6-fluoro-N-(4-oxocyclohexyl)quinoline-3-carboxamide**

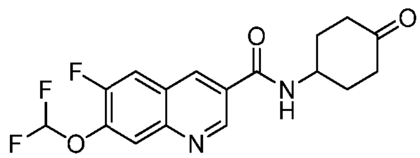

To a slurry of 7-(difluoromethoxy)-6-fluoro-N-(1,4-dioxaspiro[4.5]decan-8-yl)quinoline-3-carboxamide (1.216 g, 3.07 mmol) in acetone (25 mL) was added 1 N HCl (9.2 mL, 9.2 mmol) and the mixture was heated under reflux. After 10 h the mixture was cooled, poured into sat. $NaHCO_3$ and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound (0.893 g, 83 % yield) as a colorless solid. $^1$H NMR (400 MHz, $CDCl_3$) $\delta$ ppm 1.85 (qd, *J* = 12, 5 Hz, 2 H), 2.38 - 2.66 (m, 6 H), 4.54 (tdt, *J* = 11, 7, 4 Hz, 1 H), 6.19 (d, *J* = 7 Hz, 1 H), 6.76 (t, *J* = 72 Hz, 1 H), 7.65 (d, *J* = 10 Hz, 1 H), 7.97 (d, *J* = 8 Hz, 1 H), 8.54 (d, *J* = 2 Hz, 1 H), 9.21 (s, 1 H). MS (ESI) *m/z* 353 (M+H).

**C. 7-(Difluoromethoxy)-6-fluoro-N-(4-morpholinocyclohexyl)quinoline-3-carboxamide trifluoroacetic acid salt**

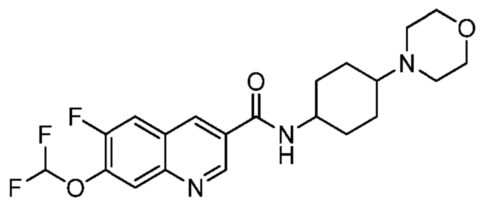

To a solution of 7-(difluoromethoxy)-6-fluoro-N-(4-oxocyclohexyl)quinoline-3-carboxamide (0.050 g, 0.142 mmol) and morpholine (0.015 mL, 0.170 mmol) in 1:1 MeOH / $CH_2Cl_2$ + 5% HOAc (3 mL) was added MP-$BH_3CN$ (0.182 g, 0.426 mmol) and the mixture was agitated at RT (flatbed shaker). After 20 h the resin was removed by filtration and washed with MeOH. The filtrate and washings were concentrated *in vacuo* and the residue was purified by preparative HPLC (C18 column, MeCN / water gradient with 0.1% TFA modifier) affording the title compound (0.0509 g, 67 % yield) as a colorless solid. $^1$H NMR indicated a mixture of cis/trans isomers (ca. 10:1, major/minor not assigned). $^1$H NMR (400 MHz, $CD_3SOCD_3$) δ ppm 1.34 - 1.52 (m, 2 H), 1.52 - 1.68 (m, 2 H), 2.06 (d, *J* = 11 Hz, 2 H), 2.16 (d, *J* = 11 Hz, 2 H), 3.05 - 3.19 (m, 2 H), 3.20 - 3.33 (m, 1 H), 3.38 - 3.52 (m, 2 H), 3.69 (t, *J* = 12 Hz, 2 H), 3.85 (tdt, *J* = 12, 8, 4 Hz, 1 H), 4.02 (d, *J* = 11 Hz, 2 H), 7.60 (t, *J* = 72 Hz, 1 H), 7.99 (d, *J* = 8 Hz, 1 H), 8.16 (d, *J* = 11 Hz, 1 H), 8.75 (d, *J* = 8 Hz, 1 H), 8.80 (d, *J* = 2 Hz, 1 H), 9.27 (d, *J* = 2 Hz, 1 H), 9.79 (br s, 1 H). MS (ESI) *m/z* 424 (M+H, parent).

**Examples 69 and 70**

**7-(Difluoromethoxy)-N-(trans-4-(((R)-1,1-difluoropropan-2-yl)amino)cyclohexyl)-6-fluoroquinoline-3-carboxamide and 7-(Difluoromethoxy)-N-(trans-4-(((S)-1,1-difluoropropan-2-yl)amino)cyclohexyl)-6-fluoroquinoline-3-carboxamide**

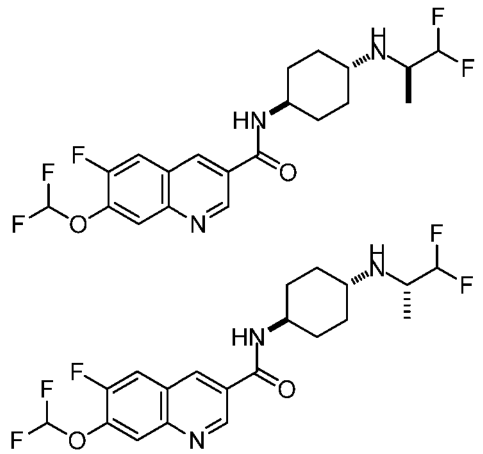

Racemic 7-(difluoromethoxy)-N-(trans-4-((1,1-difluoropropan-2-yl)amino)cyclohexyl)-6-fluoroquinoline-3-carboxamide (Example 67) (4.68 g, 10.85 mmol) was separated into its enantiomers via super critical fluid chromatography on a chiral AD column eluting with MeOH:carbon dioxide (1:4) to give 7-(difluoromethoxy)-N-(trans-4-(((R)-1,1-difluoropropan-2-yl)amino)cyclohexyl)-6-fluoroquinoline-3-carboxamide (1.66 g, 33.7 % yield) as the first enantiomer to elute and 7-(difluoromethoxy)-N-(trans-4-(((S)-1,1-difluoropropan-2-yl)amino)cyclohexyl)-6-fluoroquinoline-3-carboxamide (1.79 g, 36.3 % yield) as the last enantiomer to elute. The structures were assigned by vibrational circular dichroism.

**7-(Difluoromethoxy)-N-(trans-4-(((R)-1,1-difluoropropan-2-yl)amino)cyclohexyl)-6-fluoroquinoline-3-carboxamide:** $^1$H NMR (400 MHz, $CD_3SOCD_3$) δ 1.02 (d, *J* = 7 Hz, 3 H), 1.11 (q, *J* = 12 Hz, 2 H), 1.37 (dq, *J* = 12, 4 Hz, 2 H), 1.44-1.52 (m, 1 H), 1.84-1.98 (m, 4 H), 2.44-2.56 (m, 1 H), 2.90-3.06 (m, 1 H), 3.70-3.84 (m, 1 H), 5.77 (dt, *J* = 56, 4 Hz, 1 H), 7.58 (t, *J* = 73 Hz, 1 H), 7.97 (d, *J* = 8 Hz, 1 H), 8.14 (d, *J* = 11 Hz, 1 H), 8.62 (d, *J* = 8 Hz, 1 H), 8.77 (d, *J* = 2 Hz, 1 H), 9.24 (d, *J* = 2 Hz, 1 H); LC-MS (LC-ES) M+H = 432.

**7-(Difluoromethoxy)-N-(trans-4-(((S)-1,1-difluoropropan-2-yl)amino)cyclohexyl)-6-fluoroquinoline-3-carboxamide:** $^1$H NMR (400 MHz, $CD_3SOCD_3$) δ 1.02 (d, *J* = 7 Hz, 3 H), 1.11 (q, *J* = 12 Hz, 2 H), 1.37 (dq, *J* = 12, 4 Hz, 2 H), 1.44-1.52 (m, 1 H), 1.84-1.98 (m, 4 H), 2.44-2.56 (m, 1 H), 2.90-3.06 (m, 1 H), 3.70-3.84 (m, 1 H), 5.77 (dt, *J* = 56, 4 Hz, 1 H), 7.58 (t, *J* = 73 Hz, 1 H), 7.97 (d, *J* = 8 Hz, 1 H), 8.14 (d, *J* = 11 Hz, 1 H), 8.62 (d, J = 8 Hz, 1 H), 8.77

(d, *J* = 2 Hz, 1 H), 9.24 (d, *J* = 2 Hz, 1 H); LC-MS (LC-ES) M+H = 432.

**Example 71**

**N-(*trans*-4-(3,3-Difluoroazetidin-1-yl)cyclohexyl)-7-(difluoromethoxy)-6-fluoroquinoline-3-carboxamide**

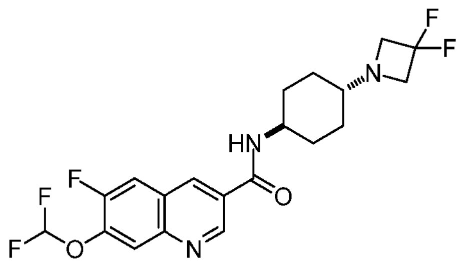

DIEA (0.367 mL, 2.102 mmol) was added to 7-(difluoromethoxy)-6-fluoroquinoline-3-carboxylic acid hydrochloride (Intermediate 15) (0.1081 g, 0.420 mmol) in DMF (1.735 mL) at RT. Then, HATU (0.208 g, 0.546 mmol) was added and the reaction mixture was stirred for 5 min. Then, *trans*-4-(3,3-difluoroazetidin-1-yl)cyclohexanamine (Intermediate 62) (0.084 g, 0.441 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc to give the title compound (0.1139 g, 59.9 % yield). $^1$H NMR (400 MHz, $CD_3SOCD_3$) $\delta$ 1.08 (dq, *J* = 12, 3 Hz, 2 H), 1.36 (dq, *J* = 13, 3 Hz, 2 H), 1.78 (br d, *J* = 12 Hz, 2 H), 1.89 (dd, *J* = 13, 3 Hz, 2 H), 2.13 (br t, *J* = 11 Hz, 1 H), 3.54 (t, *J* = 12 Hz, 4 H), 3.72-3.86 (m, 1 H), 7.58 (t, *J* = 73 Hz, 1 H), 7.97 (d, *J* = 8 Hz, 1 H), 8.14 (d, *J* = 11 Hz, 1 H), 8.63 (d, *J* = 8 Hz, 1 H), 8.77 (d, *J* = 2 Hz, 1 H), 9.24 (d, *J* = 2 Hz, 1 H); LC-MS (LC-ES) M+H = 430.

**Example 72**

**Ethyl 2-(4-(7-(difluoromethoxy)-6-fluoroquinoline-3-carboxamido)piperidin-1-yl)oxazole-5-carboxylate**

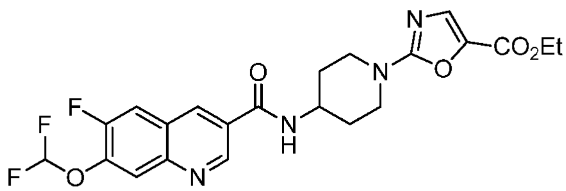

*A.* ***tert*-Butyl 4-(7-(difluoromethoxy)-6-fluoroquinoline-3-carboxamido)piperidine-1-carboxylate**

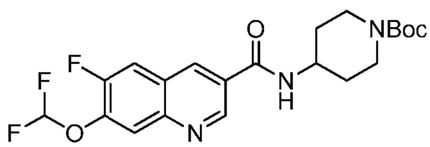

To a solution of 7-(difluoromethoxy)-6-fluoroquinoline-3-carboxylic acid hydrochloride (Intermediate 15) (0.262 g; 1.02 mmol) and HATU (0.245 g; 0.122 mmol) in DMF (5 mL) was added DIEA (0.20 mL; 1.1 mmol), and the mixture was stirred 1 h at RT. A slurry of *tert*-butyl 4-aminopiperidine-1-carboxylate in DMF (1 mL) was added *via* syringe and the mixture was stirred overnight at RT. After 18 h the mixture was poured into half-saturated $NaHCO_3$ and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound

(0.175 g, 39 % yield) as a colorless solid. $^1$H NMR (400 MHz, $CDCl_3$) δ ppm 1.42 - 1.55 (m, 11 H), 2.05 - 2.13 (m, 2 H), 2.93 (t, *J* = 12 Hz, 2 H), 4.05 - 4.31 (m, 3 H), 6.22 (d, *J* = 8 Hz, 1 H), 6.75 (t, *J* = 72 Hz, 1 H), 7.64 (d, *J* = 10 Hz, 1 H), 7.96 (d, *J* = 8 Hz, 1 H), 8.52 (d, *J* = 2 Hz, 1 H), 9.20 (d, *J* = 2 Hz, 1 H). MS (ESI) *m*/*z* 440 (M+H).

**B. 7-(Difluoromethoxy)-6-fluoro-N-(piperidin-4-yl)quinoline-3-carboxamide**

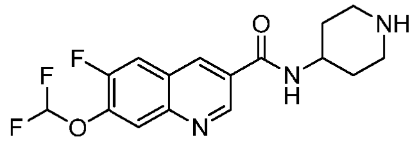

To a solution of *tert*-butyl 4-(7-(difluoromethoxy)-6-fluoroquinoline-3-carboxamido)piperidine-1-carboxylate (0.175 g, 0.398 mmol) in DCM (8 mL) was added TFA (1 mL) and the mixture was stirred at RT for 30 min. Voltiles were removed *in vacuo.* The residue was partitioned between DCM / 1 N NaOH and the layers were separated. The aqueous layer was extracted with DCM (2X). Combined organics were washed with brine, dried over $Na_2SO_4$, and concentrated *in vacuo* affording the title compound (0.1165 g, 86 % yield) as a colorless solid. MS (ESI) *m*/*z* 330 (M+H).

**C. Ethyl 2-(4-(7-(difluoromethoxy)-6-fluoroquinoline-3-carboxamido)piperidin-1-yl)oxazole-5-carboxylate**

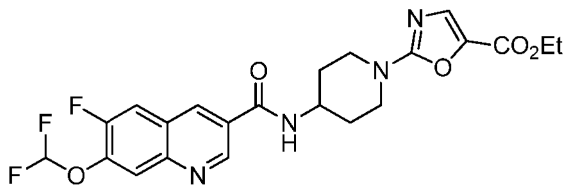

A mixture of 7-(difluoromethoxy)-6-fluoro-N-(piperidin-4-yl)quinoline-3-carboxamide (0.0542 g, 0.160 mmol), ethyl 2-chlorooxazole-5-carboxylate (0.028 g, 0.160 mmol), and $K_2CO_3$ (0.044 g, 0.319 mmol) in MeCN (4 mL) was stirred in a heating block at 80 °C. After 15 h the mixture was cooled, poured into water and extracted with EtOAc (3X). Combined organics were washed (water, brine), dried over $Na_2SO_4$, and concentrated *in vacuo.* The residue was purified by preparative HPLC (C18 column, MeCN / water gradient with 0.1% TFA modifier) affording the title compound (0.0657 g, 86 % yield) as a colorless solid. $^1$H NMR (400 MHz, $CDCl_3$) δ ppm 1.35 (t, *J* = 7 Hz, 3 H), 1.69 (qd, *J* = 12, 4 Hz, 2 H), 2.22 (dd, *J* = 13, 3 Hz, 2 H), 3.22 - 3.34 (m, 2 H), 4.23 - 4.41 (m, 5 H), 6.60 - 7.08 (m, 2 H), 7.59 (s, 1 H), 7.74 (d, *J* = 10 Hz, 1 H), 8.08 (d, *J* = 7 Hz, 1 H), 8.80 (d, *J* = 1 Hz, 1 H), 9.35 (s, 1 H). MS (ESI) *m*/*z* 479 (M+H).

**Example 73 and 74**

**7-(Difluoromethoxy)-N-(trans-3-(((S)-1,1-difluoropropan-2-yl)amino)cyclobutyl)-6-fluoroquinoline-3-carboxamide and 7-(Difluoromethoxy)-N-(*trans*-3-(((R)-1,1-difluoropropan-2-yl)amino)cyclobutyl)-6-fluoroquinoline-3-carboxamide**

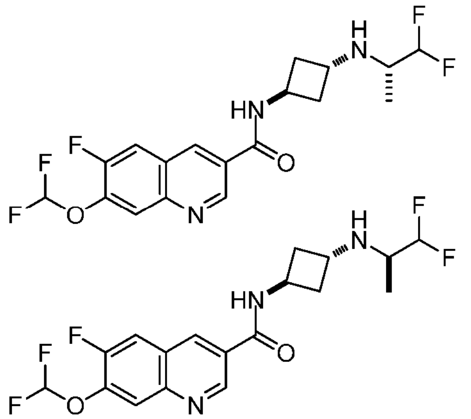

A. **7-(Difluoromethoxy)-N-(*trans*-3-((1,1-difluoropropan-2-yl)amino)cyclobutyl)-6-fluoroquinoline-3-carboxamide**

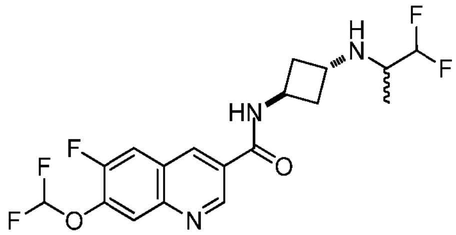

DIEA (0.524 mL, 3.00 mmol) was added to 7-(difluoromethoxy)-6-fluoroquinoline-3-carboxylic acid hydrochloride (Intermediate 15) (0.1543 g, 0.600 mmol) in DMF (2.47 mL) at RT. Then, HATU (0.297 g, 0.780 mmol) was added and the reaction mixture was stirred for 5 min. Then, *trans*-N1-(1,1-difluoropropan-2-yl)cyclobutane-1,3-diamine dihydrochloride (Intermediate 63) (0.149 g, 0.630 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc (1:99) to give the title compound (0.1638 g, 64.3 % yield). $^1$H NMR (400 MHz, $CD_3SOCD_3$) $\delta$ 1.03 (d, *J* = 7 Hz, 3 H), 2.04-2.16 (m, 3 H), 2.18-2.30 (m, 2 H), 2.74-2.90 (m, 1 H), 3.48-3.58 (m, 1 H), 4.46 (h, *J* = 7 Hz, 1 H), 5.80 (dt, *J* = 54, 4 Hz, 1 H), 7.58 (t, *J* = 73 Hz, 1 H), 7.97 (d, *J* = 8 Hz, 1 H), 8.14 (d, *J* = 11 Hz, 1 H), 8.80 (d, *J* = 2 Hz, 1 H), 9.02 (d, *J* = 7 Hz, 1 H), 9.27 (d, *J* = 2 Hz, 1 H); LC-MS (LC-ES) M+H = 404.

**B. Chiral separation**

Racemic 7-(difluoromethoxy)-N-(*trans*-3-((1,1-difluoropropan-2-yl)amino)cyclobutyl)-6-fluoroquinoline-3-carboxamide (0.1527 g, 0.379 mmol) was separated into its enantiomers via chiral chromatography on a chiral IC column eluting with ethanol:hexanes (1:9) to give 7-(difluoromethoxy)-N-(trans-3-(((S)-1,1-difluoropropan-2-yl)amino)cyclobutyl)-6-fluoroquinoline-3-carboxamide (0.0646 g, 40.2 % yield) as the first enantiomerto elute and 7-(difluoromethoxy)-N-(trans-3-(((R)-1,1-difluoropropan-2-yl)amino)cyclobutyl)-6-fluoroquinoline-3-carboxamide (0.0641 g, 39.9 % yield) as the last enantiomer to elute. The structures were assigned by vibrational circular dichroism.

**7-(Difluoromethoxy)-N-(trans-3-(((S)-1,1-difluoropropan-2-yl)amino)cyclobutyl)-6-fluoroquinoline-3-carboxamide:** $^1$H NMR (400 MHz, $CD_3SOCD_3$) $\delta$ 1.03 (d, *J* = 7 Hz, 3 H), 2.04-2.16 (m, 3 H), 2.18-2.30 (m, 2 H), 2.74-2.90 (m, 1 H), 3.48-3.58 (m, 1 H), 4.46 (h, *J* = 7 Hz, 1 H), 5.80 (dt, *J* = 54, 4 Hz, 1 H), 7.58 (t, *J* = 73 Hz, 1 H), 7.97 (d, *J* = 8 Hz, 1 H), 8.14 (d, *J* = 11 Hz, 1 H), 8.80 (d, *J* = 2 Hz, 1 H), 9.02 (d, *J* = 7 Hz, 1 H), 9.27 (d, *J* = 2 Hz, 1 H); LC-MS (LC-ES) M+H = 404.

**7-(Difluoromethoxy)-N-(trans-3-(((R)-1,1-difluoropropan-2-yl)amino)cyclobutyl)-6-fluoroquinoline-3-carboxamide:** $^1$H NMR (400 MHz, $CD_3SOCD_3$) $\delta$ 1.03 (d, *J* = 7 Hz, 3 H), 2.04-2.16 (m, 3 H), 2.18-2.30 (m, 2 H), 2.74-2.90 (m, 1 H), 3.48-3.58 (m, 1 H), 4.46 (h, *J* = 7 Hz, 1 H), 5.80 (dt, *J* = 54, 4 Hz, 1 H), 7.58 (t, *J* = 73 Hz, 1 H), 7.97 (d, *J* = 8 Hz, 1 H), 8.14 (d, *J* = 11 Hz, 1 H), 8.80 (d, *J* = 2 Hz, 1 H), 9.02 (d, *J* = 7 Hz, 1 H), 9.27 (d, *J* = 2 Hz, 1 H); LC-MS (LC-ES) M+H = 404.

**Example 75**

**7-(Difluoromethoxy)-N-(*trans*-4-(((1,1-difluoropropan-2-yl)amino)methyl)cyclohexyl)-6-fluoroquinoline-3-carboxamide**

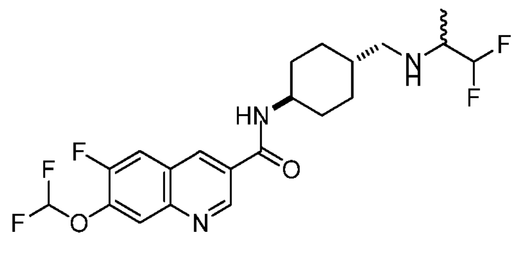

DIEA (0.518 mL, 2.97 mmol) was added to 7-(difluoromethoxy)-6-fluoroquinoline-3-carboxylic acid hydrochloride (Intermediate 15) (0.1525 g, 0.593 mmol) in DMF (2.447 mL) at RT. Then, HATU (0.293 g, 0.771 mmol) was added and the reaction mixture was stirred for 5 min. Then, *trans-4-(((1,1-difluoropropan-2-yl)amino)methyl)cyclohexanamine* (Intermediate 64) (0.128 g, 0.623 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc(1 :499) to give the title compound (0.177 g, 63.9 % yield). $^1$H NMR (400 MHz, $CD_3SOCD_3$) δ 0.98 (q, *J* = 13 Hz, 2 H), 1.02 (d, *J* = 7 Hz, 3 H), 1.24-1.40 (m, 3 H), 1.62 (br s, 1 H), 1.78-1.96 (m, 4 H), 2.36-2.52 (m, 2 H), 2.74-2.88 (m, 1 H), 3.72-3.84 (m, 1 H), 5.81 (dt, *J* = 57, 4 Hz, 1 H), 7.58 (t, *J* = 73 Hz, 1 H), 7.97 (d, *J* = 8 Hz, 1 H), 8.14 (d, *J* = 11 Hz, 1 H), 8.63 (d, *J* = 8 Hz, 1 H), 8.78 (d, *J* = 2 Hz, 1 H), 9.25 (d, *J* = 2 Hz, 1 H); LC-MS (LC-ES) M+H = 446.

**Example 76**

**7-(Difluoromethoxy)-6-fluoro-N-(trans-4-(3,3,3-trifluoro-2-hydroxy-2-methylpropoxy)cyclohexyl)quinoline-3-carboxamide**

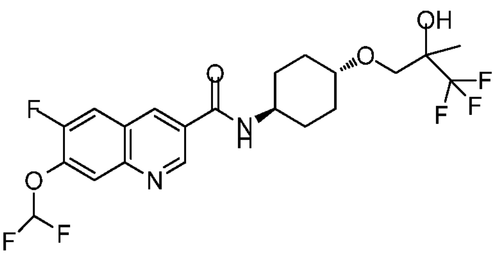

To a solution of 3-((trans-4-aminocyclohexyl)oxy)-1,1,1-trifluoro-2-methylpropan-2-ol (Intermediate 47) (50 mg, 0.180 mmol) in DMF (5 mL) was added 7-(difluoromethoxy)-6-fluoroquinoline-3-carboxylic acid hydrochloride (Intermediate 15) (40 mg, 0.156 mmol), followed by HATU (89 mg, 0.233 mmol) and DIEA (0.163 mL, 0.933 mmol). The reaction was stirred at RT for 24 h. The solvent was removed under reduced pressure and the residue was absorbed to Celite®. The crude product was purified by reverse phase chromatography (10-100% acetonitrile (with 0.1% TFA)/ water (with 0.1% TFA)) to afford the title compound (30 mg, 32% yield). $^1$H NMR (400 MHz, $CD_3SOCD_3$) δ ppm 1.18 - 1.50 (m, 7 H), 1.87 - 1.97 (m, 2 H), 1.99 - 2.13 (m, 2 H), 3.23 - 3.38 (m, 1 H), 3.42 - 3.63 (m, 3 H), 3.78 - 3.98 (m, 1 H), 7.59 (t, *J* = 73 Hz, 1 H), 7.98 (d, *J* = 8 Hz, 1 H), 8.15 (d, *J* = 11 Hz, 1 H), 8.63 (d, *J* = 8 Hz, 1 H), 8.79 (d, *J* = 3 Hz, 1 H), 9.26 (d, *J* = 3 Hz, 1 H). MS (ESI) *m/z* 481 (M+1).

**Example 77**

**7-(Difluoromethoxy)-6-fluoro-N-(*trans*-4-(((R)-1,1,1-trifluoro-3-hydroxypropan-2-yl)amino)cyclohexyl)quinoline-3-carboxamide**

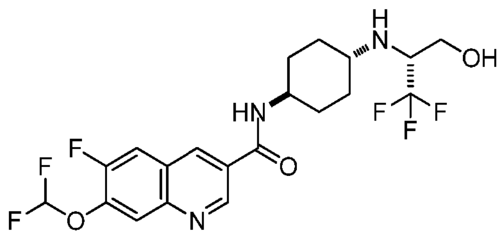

DIEA (0.348 mL, 1.993 mmol) was added to 7-(difluoromethoxy)-6-fluoroquinoline-3-carboxylic acid hydrochloride (Intermediate 15) (0.1025 g, 0.399 mmol) in DMF (1.645 mL) at RT. Then, HATU (0.197 g, 0.518 mmol) was added and the reaction mixture was stirred for 5 min. Then, (R)-2-((*trans*-4-aminocyclohexyl)amino)-3,3,3-trifluoropropan-1-ol (Intermediate 65) (0.095 g, 0.419 mmol) was added and the reaction mixture was stirred for 2 h. The reaction mixture was concentrated. The reaction mixture was purified by RP HPLC eluting with acetonitrile:water with 0.1% ammonium hydroxide *(5:95-100:0),* then further purified by silica gel chromatography, eluting with EtOAc:hexanes (9:1) to give the title compound (0.1196 g, 61.3 % yield). $^1$H NMR (400 MHz, $CD_3SOCD_3$) δ 1.13 (q, *J* = 12 Hz, 2 H), 1.37 (dq, *J* = 13, 3 Hz, 2 H), 1.84-2.02 (m, 5 H), 2.48-2.58 (m, 1 H), 3.22-3.34 (m, 1 H), 3.46-3.54 (m, 1 H), 3.58-3.68 (m, 1 H), 3.72-3.86 (m, 1 H), 4.99 (t, *J* = 6 Hz, 1 H), 7.58 (t, *J* = 73 Hz, 1 H), 7.97 (d, *J* = 8 Hz, 1 H), 8.14 (d, *J* = 11 Hz, 1 H), 8.61 (d, *J* = 8 Hz, 1 H), 8.77 (d, *J* = 2 Hz, 1 H), 9.24 (d, *J* = 2 Hz, 1 H); LC-MS (LC-ES) M+H = 466.

**Example 78**

**(S)-N-(1-(Cyclopentylmethyl)-2-oxopyrrolidin-3-yl)-7-(difluoromethoxy)-6-fluoroquinoline-3-carboxamide**

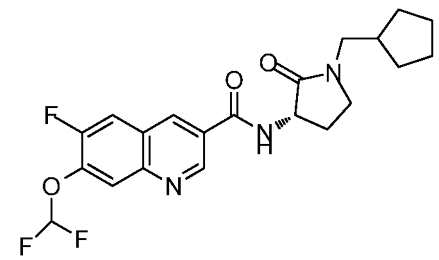

To a suspension of (S)-7-(difluoromethoxy)-6-fluoro-N-(2-oxopyrrolidin-3-yl)quinoline-3-carboxamide (Example 48) (100 mg, 0.295 mmol) in DMF (15 mL) at 0 °C was added sodium hydride (17.68 mg, 0.737 mmol). After stirring at RT for 20 min, (bromomethyl)cyclopentane (72.1 mg, 0.442 mmol) was added and the resulting reaction was stirred at RT for 24 h. The solution was diluted with EtOAc and quenched with saturated $NH_4Cl$ (25 mL). The aqueous layer was extracted with EtOAc (3x20 mL), the organic layers combined, and absorbed onto Celite®. The crude material was then purified by reverse phase chromatography (10-50% acetonitrile (with 0.1% TFA)/ water (with 0.1% TFA)) to afford the title compound (60 mg, 38% yield). $^1$H NMR (400 MHz, $CD_3SOCD_3$) δ ppm 1.09 - 1.31 (m, 2 H), 1.44 - 1.81 (m, 6 H), 1.93 - 2.08 (m, 1 H), 2.16-2.21 (m, 1 H), 2.37-2.40 (m, 1 H), 3.10-3.22 (m, 2 H), 3.33 - 3.50 (m, 2 H), 4.66 - 4.80 (m, 1 H), 7.60 (t, *J* = 72 Hz, 1 H), 8.00 (d, *J* = 8 Hz, 1 H), 8.18 (d, *J* = 11 Hz, 1 H), 8.85 (d, *J* = 2 Hz, 1 H), 9.15 (d, *J* = 8 Hz, 1 H), 9.29 (d, *J* = 2 Hz, 1 H). MS (ESI) *m*/*z* 422 (M+1).

Example 79

**7-(Difluoromethoxy)-6-fluoro-N-(trans-4-(2-hydroxy-3-methylbutoxy)cyclohexyl)quinoline-3-carboxamide**

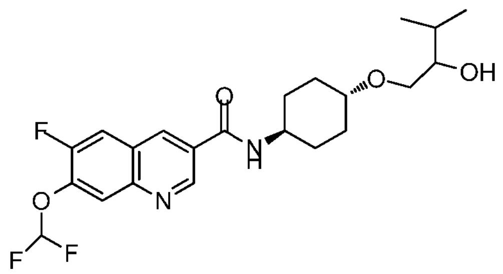

To a solution of 1-((trans-4-aminocyclohexyl)oxy)-3-methylbutan-2-ol (Intermediate 66) (50 mg, 0.248 mmol) in DMF (5 mL) was added 7-(difluoromethoxy)-6-fluoroquinoline-3-carboxylic acid hydrochloride (Intermediate 15) (40 mg, 0.156 mmol), followed by HATU (89 mg, 0.233 mmol) and DIEA (0.081 mL, 0.467 mmol). The reaction was stirred at RT for 24 h. The solvent was removed under reduced pressure, the residue was absorbed onto Celite® and purified by reverse phase chromatography (10-50% acetonitrile (with 0.1% TFA)/ water (with 0.1% TFA)) to afford the title compound (70 mg, 81% yield). $^1H$ NMR (400 MHz, $CD_3SOCD_3$) $\delta$ ppm 0.77 - 0.99 (m, 6 H), 1.17 - 1.52 (m, 4 H), 1.64-1.69 (m, 1 H), 1.92-1.93 (m, 2 H), 2.00 - 2.13 (m, 2 H), 3.18 - 3.48 (m, 4 H), 3.76 - 3.90 (m, 1 H), 7.59 (t, *J* = 72 Hz, 1 H), 7.98 (d, *J* = 8 Hz, 1 H), 8.16 (d, *J* = 11 Hz, 1 H), 8.63 (d, *J* = 8 Hz, 1 H), 8.79 (d, *J* = 2 Hz, 1 H), 9.26 (d, *J* = 2 Hz, 1 H). MS (ESI) *m/z* 441 (M+1).

**Example 80**

**(*S*)-Ethyl 2-(3-(7-(difluoromethoxy)-6-fluoroquinoline-3-carboxamido)pyrrolidin-1-yl)oxazole-5-carboxylate trifluoroacetic acid salt**

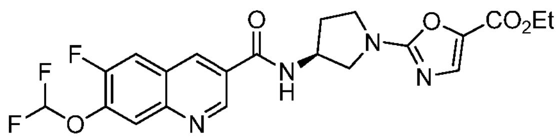

**A. (*S*)-*tert*-Butyl 3-(7-(difluoromethoxy)-6-fluoroquinoline-3-carboxamido)pyrrolidine-1-carboxylate**

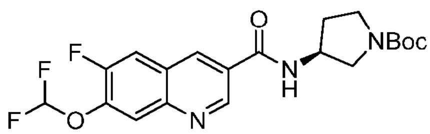

To a solution of 7-(difluoromethoxy)-6-fluoroquinoline-3-carboxylic acid hydrochloride (Intermediate 15) (0.203 g, 0.789 mmol) and HATU (0.315 g, 0.829 mmol) in anhydrous DMF (3 mL) was added DIEA (0.145 mL, 0.829 mmol) and the mixture was stirred 1 h. A solution of (*S*)-*tert*-butyl 3-aminopyrrolidine-1-carboxylate (0.176 g, 0.947 mmol) in DMF (1 mL) was added and stirring continued. After 20 h the mixture was poured into sat. $NaHCO_3$ and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound (0.289 g, 86 % yield) as a yellow gum. $^1H$ NMR (400 MHz, $CD_3SOCD_3$) $\delta$ ppm 1.41 (s, 9 H), 1.88 - 2.01 (m, 1 H), 2.08 - 2.20 (m, 1 H), 3.26 (m, 1 H), 3.33 (s, 1 H), 3.44 (dt, *J* = 11, 7 Hz, 1 H), 3.59 (ddd, *J* = 15, 11 6 Hz, 1 H), 4.49 (dt, *J* = 11, 6 Hz, 1 H), 7.59 (t, *J* = 73 Hz, 1 H), 7.98 (d, *J* = 8 Hz, 1 H), 8.15 (d, *J* = 11 Hz, 1 H), 8.81 (d, *J* = 2 Hz, 1 H), 8.96 (d, *J* = 6

Hz, 1 H), 9.27 (d, *J* = 2 Hz, 1 H). MS (ESI) *m/z* 426 (M+H), 370 ([M+H]-isobutylene).

**B. (*S*)-7-(Difluoromethoxy)-6-fluoro-N-(pyrrolidin-3-yl)quinoline-3-carboxamide**

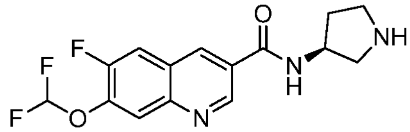

A mixture of (*S*)-*tert*-butyl 3-(7-(difluoromethoxy)-6-fluoroquinoline-3-carboxamido)pyrrolidine-1-carboxylate (0.277 g, 0.651 mmol) in DCM (5 mL) and TFA (1 mL) was stirred at RT for 2 h. Volatiles were removed *in vacuo* and the residue was partitioned between DCM / 1 N NaOH. The aqueous layer was extracted with DCM (2X). Combined organics were washed (water, brine), dried over $Na_2SO_4$, and concentrated *in vacuo* to give the title compound (0.179 g, 85 % yield) as a pale yellow solid, used without further purification. $^1H$ NMR (400 MHz, $CD_3SOCD_3$) δ ppm 1.66 - 1.76 (m, 1 H), 2.01 (m, 1 H), 2.69 - 2.81 (m, 2 H), 2.89 - 3.01 (m, 2 H), 4.31 - 4.41 (m, 1 H), 7.59 (t, *J* = 73 Hz, 1 H), 7.98 (d, *J* = 8 Hz, 1 H), 8.13 (d, *J* = 11 Hz, 1 H), 8.78 (d, *J* = 7 Hz, 1 H), 8.82 (d, *J* = 2 Hz, 1 H), 9.28 (d, *J* = 2 Hz, 1 H). MS (ESI) *m/z* 326 (M+H).

**C. (*S*)-Ethyl 2-(3-(7-(difluoromethoxy)-6-fluoroquinoline-3-carboxamido)pyrrolidin-1-yl)oxazole-5-carboxylate trifluoroacetic acid salt**

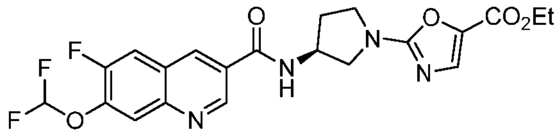

A mixture of (S)-7-(difluoromethoxy)-6-fluoro-N-(pyrrolidin-3-yl)quinoline-3-carboxamide (0.0858 g, 0.264 mmol), ethyl 2-chlorooxazole-5-carboxylate (0.038 mL, 0.290 mmol), and $K_2CO_3$ (0.073 g, 0.528 mmol) in MeCN (5 mL) was subjected to microwave heating for 30 min at 140 °C. Upon cooling the mixture was filtered, poured into water and extracted with EtOAc (3X). Combined organics were washed (water, brine), dried over $Na_2SO_4$, and concentrated *in vacuo*. The residue was purified by preparative HPLC (C18 column, MeCN / water gradient with 0.1% TFA modifier) affording the title compound (0.1175 g, 77 % yield) as a pale yellow solid. $^1H$ NMR (400 MHz, $CD_3SOCD_3$) δ ppm 1.25 (t, *J* = 7 Hz, 3 H), 2.12 (ddt, *J* = 12, 7, 5 Hz, 1 H), 2.24 - 2.36 (m, 1 H), 3.56 - 3.75 (m, 3 H), 3.84 (dd, *J* = 11, 6 Hz, 1 H), 4.23 (q, *J* = 7 Hz, 2 H), 4.61 - 4.70 (m, 1 H), 7.59 (t, *J* = 73 Hz, 1 H), 7.72 (s, 1 H), 7.98 (d, *J* = 8 Hz, 1 H), 8.15 (d, *J* = 11 Hz, 1 H), 8.82 (d, *J* = 2 Hz, 1 H), 9.06 (d, *J* = 6 Hz, 1 H), 9.28 (d, *J* = 2 Hz, 1 H). MS (ESI) *m/z* 465 (M+H, parent).

**Example 81**

**7-(Difluoromethoxy)-N-((1r,4r)-4-ethyl-4-hydroxycyclohexyl)-6-fluoroquinoline-3-carboxamide**

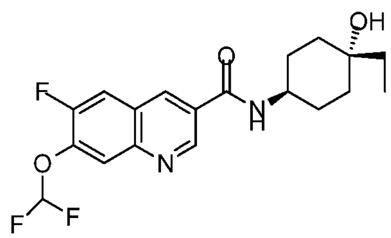

To a solution of 7-(difluoromethoxy)-6-fluoroquinoline-3-carboxylic acid hydrochloride (Intermediate 15) (50

mg, 0.194 mmol) in DMF (10 mL) at RT, was added (1r,4r)-4-amino-1-ethylcyclohexanol (see WO 2010 027097) (30.6 mg, 0.214 mmol), followed by HATU (111 mg, 0.292 mmol) and DIEA (0.102 mL, 0.583 mmol). After stirring at RT for 24 h, the solvent was removed under reduced pressure, the residue dissolved in 10% MeOH/DCM (50 mL), and absorbed onto Celite®. The crude material was then purified by reverse phase chromatography (10-50% acetonitrile/ water (with 0.2% $NH_4OH$) to afford the title compound (50 mg, 67% yield). $^1H$ NMR (400 MHz, $CD_3SOCD_3$) δ ppm 0.85 (t, *J* = 7 Hz, 3 H) 1.36 - 1.57 (m, 6 H) 1.62 - 1.73 (m, 2 H) 1.78-1.83 (m, 2 H) 3.87 - 4.00 (m, 1 H) 4.06 (s, 1 H) 7.59 (t, *J* = 73 Hz, 1 H) 7.98 (d, *J* = 8 Hz, 1 H) 8.15 (d, *J* = 11 Hz, 1 H) 8.55 (d, *J* = 8 Hz, 1 H) 8.78 (d, *J* = 2 Hz, 1 H) 9.25 (d, *J* = 2 Hz, 1 H). MS (ESI) *m/z* 383(M+1).

**Example 82**

**Ethyl 2-(3-(7-(difluoromethoxv)-6-fluoroquinoline-3-carboxamido)azetidin-1-yl)oxazole-4-carboxylate**

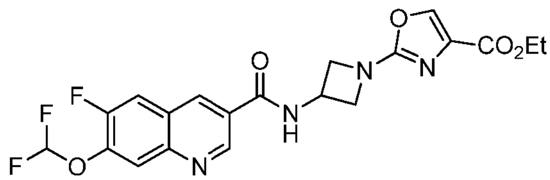

*A.* ***tert*-Butyl 3-(7-(difluoromethoxy)-6-fluoroquinoline-3-carboxamido)azetidine-1-carboxylate**

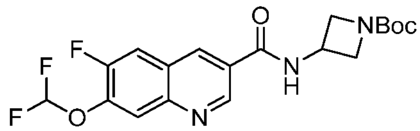

To a solution of 7-(difluoromethoxy)-6-fluoroquinoline-3-carboxylic acid hydrochloride (Intermediate 15) (0.205 g, 0.797 mmol) and HATU (0.318 g, 0.837 mmol) in DMF (3 mL) was added DIEA (0.146 mL, 0.837 mmol) and the mixture was stirred 1 h. A solution of *tert*-butyl 3-aminoazetidine-1-carboxylate (0.137 g, 0.797 mmol) in DMF (1 mL) was added and stirring continued. After 1 h the mixture was poured into sat. $NaHCO_3$ and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound (0.257 g, 78 % yield) as a yellow foam. $^1H$ NMR (400 MHz, $CD_3SOCD_3$) δ ppm 1.40 (s, 9 H), 3.86 - 3.95 (m, 2 H), 4.17 (t, *J* = 8 Hz, 2 H), 4.66 - 4.78 (m, 1 H), 7.60 (t, *J* = 73 Hz, 1 H), 7.99 (d, *J* = 8 Hz, 1 H), 8.16 (d, *J* = 11 Hz, 1 H), 8.84 (d, *J* = 2 Hz, 1 H), 9.30 (d, *J* = 2 Hz, 1 H), 9.38 (d, *J* = 7 Hz, 1 H). MS (ESI) *m/z* 412 (M+H).

**B. N-(Azetidin-3-yl)-7-(difluoromethoxy)-6-fluoroquinoline-3-carboxamide**

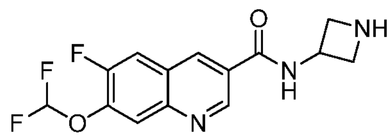

A solution of *tert*-butyl 3-(7-(difluoromethoxy)-6-fluoroquinoline-3-carboxamido)azetidine-1-carboxylate (0.246 g, 0.598 mmol) in DCM (5 mL) and TFA (1 mL) was stirred at RT. After 90 min volatiles were removed *in vacuo.* The residue was partitioned between DCM / 1N NaOH. The aqueous layer was extracted with DCM (2X). Combined organics were washed (water, brine), dried over $Na_2SO_4$, and concentrated *in vacuo* affording the title compound (0.156 g, 84 % yield) as an off-white powder. $^1H$ NMR (400 MHz, $CD_3SOCD_3$) δ ppm 3.53 - 3.67 (m, 4 H), 4.76 (sxt, *J* = 7 Hz, 1 H), 7.59 (t, *J* = 73 Hz, 1 H), 7.98 (d, *J* = 8 Hz, 1 H), 8.15 (d, *J* = 11 Hz, 1 H), 8.83 (d, *J* = 2 Hz, 1 H), 9.26 (d, *J* = 7 Hz, 1 H),

9.29 (d, *J* = 2 Hz, 1 H). MS (ESI) *m/z* 312 (M+H).

**C. Ethyl 2-(3-(7-(difluoromethoxy)-6-fluoroquinoline-3-carboxamido)azetidin-1-yl)oxazole-4-carboxylate**

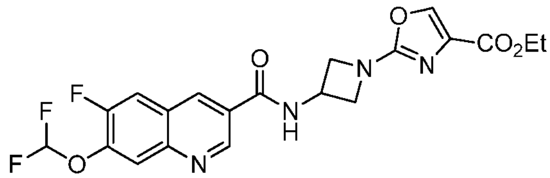

A mixture of N-(azetidin-3-yl)-7-(difluoromethoxy)-6-fluoroquinoline-3-carboxamide (0.0759 g, 0.244 mmol), ethyl 2-chlorooxazole-4-carboxylate (0.047 g, 0.268 mmol), and $K_2CO_3$ (0.067 g, 0.488 mmol) in anhydrous MeCN (7 mL) was subjected to microwave heating at 140 °C for 30 min. Upon cooling the mixture was filtered, the filtrate was poured into water and extracted with EtOAc (3X). Combined organics were washed (water, brine), dried over $Na_2SO_4$, and concentrated *in vacuo.* The residue was purified by preparative HPLC (C18 column, MeCN / water gradient with 0.1% TFA modifier) affording the title compound (0.0778 g, 71 % yield) as an off-white solid. $^1H$ NMR (400 MHz, $CD_3SOCD_3$) δ ppm 1.26 (t, *J* = 7 Hz, 3 H), 4.16 (dd, *J* = 8, 6 Hz, 2 H), 4.23 (q, *J* = 7 Hz, 2 H), 4.44 (t, *J* = 8 Hz, 2 H), 4.95 (qt, *J* = 7, 6 Hz, 1 H), 7.60 (t, *J* = 72 Hz, 1 H), 8.00 (d, *J* = 8 Hz, 1 H), 8.17 (d, *J* = 11 Hz, 1 H), 8.35 (s, 1 H), 8.85 (d, *J* = 2 Hz, 1 H), 9.31 (d, *J* = 2 Hz, 1 H), 9.49 (d, *J* = 7 Hz, 1 H). MS (ESI) *m/z* 451 (M+H).

**Example 83**

**Ethyl 2-((trans-3-(7-(difluoromethoxy)-6-fluoroquinoline-3-carboxamido)cyclobutyl)amino)oxazole-5-carboxvlate**

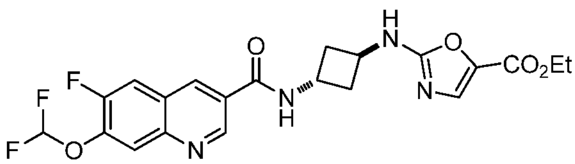

***A. tert*-Butyl (trans-3-(7-(difluoromethoxy)-6-fluoroquinoline-3-carboxamido)cyclobutyl)carbamate**

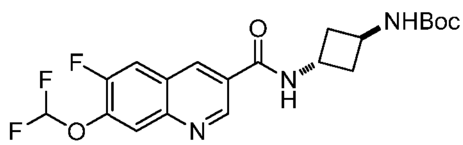

To a solution of 7-(difluoromethoxy)-6-fluoroquinoline-3-carboxylic acid hydrochloride (Intermediate 15) (0.210 g, 0.817 mmol) and HATU (0.326 g, 0.857 mmol) in DMF (3 mL) was added DIEA (0.150 mL, 0.857 mmol) and the mixture was stirred 1 h. A solution of *tert*-butyl (trans-3-aminocyclobutyl)carbamate (0.152 g, 0.817 mmol) in DMF (1 mL) was added and stirring continued. After 1 h the mixture was poured into sat. $NaHCO_3$ and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound (0.357 g, 103 % yield) as a tan solid. $^1H$ NMR (400 MHz, $CD_3SOCD_3$) δ ppm 1.39 (s, 9 H), 2.22 - 2.39 (m, 4 H), 4.14 (sxt, *J* = 7 Hz, 1 H), 4.40 - 4.51 (m, 1 H), 7.32 (d, *J* = 7 Hz, 1 H), 7.59 (t, *J* = 72 Hz, 1 H), 7.99 (d, *J* = 8 Hz, 1 H), 8.15 (d, *J* = 11 Hz, 1 H), 8.81 (d, *J* = 2 Hz, 1 H), 9.10 (d, *J* = 7 Hz, 1 H), 9.28 (d, *J* = 2 Hz, 1 H). MS (ESI) *m/z* 426 (M+H).

**B. N-(trans-3-Aminocyclobutyl)-7-(difluoromethoxy)-6-fluoroquinoline-3-carboxamide**

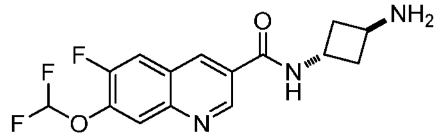

A solution of *tert*-butyl (trans-3-(7-(difluoromethoxy)-6-fluoroquinoline-3-carboxamido)cyclobutyl)carbamate (0.357 g, 0.839 mmol) in DCM (5 mL) and TFA (1 mL) was stirred at RT. After 90 min volatiles were removed *in vacuo.* The residue was partitioned between DCM / 1N NaOH. The aqueous layer was extracted with DCM (2X). Combined organics were washed (water, brine), dried over $Na_2SO_4$, and concentrated *in vacuo* affording the title compound (0.168 g, 62 % yield) as a yellow powder, used without further purification. $^1H$ NMR (400 MHz, $CD_3SOCD_3$) δ ppm 1.83 (br s, 2 H), 1.96 - 2.08 (m, 2 H), 2.21 - 2.33 (m, 2 H), 3.47 - 3.61 (m, 1 H), 4.44 - 4.58 (m, 1 H), 7.59 (t, *J* = 73 Hz, 1 H), 7.98 (d, *J* = 8 Hz, 1 H), 8.14 (d, *J* = 11 Hz, 1 H), 8.80 (d, *J* = 2 Hz, 1 H), 8.98 (d, *J* = 7 Hz, 1 H), 9.27 (d, *J* = 2 Hz, 1 H). MS (ESI) *m/z* 326 (M+H).

**C. Ethyl 2-((trans-3-(7-(difluoromethoxy)-6-fluoroquinoline-3-carboxamido)cyclobutyl)amino)oxazole-5-carboxylate**

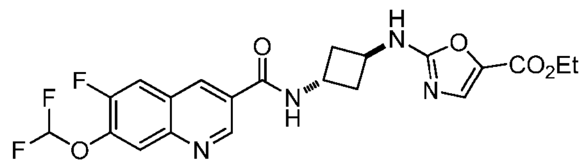

A mixture of N-(trans-3-aminocyclobutyl)-7-(difluoromethoxy)-6-fluoroquinoline-3-carboxamide (0.0820 g, 0.252 mmol), ethyl 2-chlorooxazole-5-carboxylate (0.037 mL, 0.28 mmol), and $K_2CO_3$ (0.070 g, 0.50 mmol) in anhydrous MeCN (7 mL) was subjected to microwave heating at 140 °C for 30 min. Upon cooling the mixture was filtered, the filtrate was poured into water and extracted with EtOAc (3X). Combined organics were washed (water, brine), dried over $Na_2SO_4$, and concentrated *in vacuo.* The residue was purified by preparative HPLC (C18 column, MeCN / water gradient with 0.1% TFA modifier) affording the title compound (0.0991 g, 85 % yield) as an off-white solid. $^1H$ NMR (400 MHz, $CD_3SOCD_3$) δ ppm 1.25 (t, *J* = 7 Hz, 3 H), 2.35 - 2.44 (m, 2 H), 2.45 - 2.49 (m, 2 H), 4.22 (q, *J* = 7 Hz, 2 H), 4.26 - 4.37 (m, 1 H), 4.51 - 4.62 (m, 1 H), 7.37 - 7.81 (m, 2 H), 7.99 (d, *J* = 8 Hz, 1 H), 8.16 (d, *J* = 11 Hz, 1 H), 8.63 (d, *J* = 7 Hz, 1 H), 8.83 (d, *J* = 2 Hz, 1 H), 9.18 (d, *J* = 7 Hz, 1 H), 9.30 (d, *J* = 2 Hz, 1 H). MS (ESI) *m/z* 465 (M+H).

**Example 84: Ethyl 2-(3-(7-(difluoromethoxy)-6-fluoroquinoline-3-carboxamido)azetidin-1-yl)oxazole-5-carboxylate**

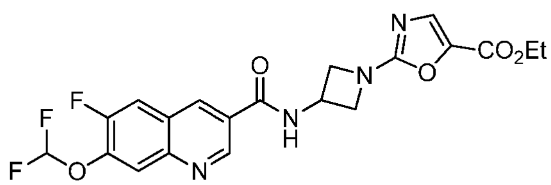

**A. Ethyl 2-(3-((*tert*-butoxycarbonyl)amino)azetidin-1-yl)oxazole-5-carboxylate**

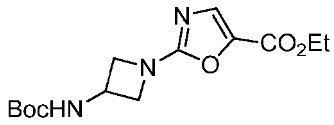

A mixture of *tert*-butyl azetidin-3-ylcarbamate hydrochloride (1.00 g, 4.79 mmol), ethyl 2-chlorooxazole-5-carboxylate (0.633 mL, 4.79 mmol), and $K_2CO_3$ (1.987 g, 14.38 mmol) in anhydrous MeCN (15 mL) was stirred in a heating block at 80 °C. After 8 h the mixture was cooled, poured into water and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound (0.860 g, 58 % yield) as a colorless solid. $^1H$ NMR (400 MHz, $CDCl_3$) δ ppm 1.34 (t, *J* = 7 Hz, 3 H), 1.45 (s, 9 H), 4.06 (dd, *J* = 9, 5 Hz, 2 H), 4.31 (q, *J* = 7 Hz, 2 H), 4.50 (t, *J* = 8 Hz, 2 H), 4.67 (br s, 1 H), 5.06 (br s, 1 H), 7.50 (s, 1 H). MS (ESI) *m/z* 312 (M+H).

**B. Ethyl 2-(3-aminoazetidin-1-yl)oxazole-5-carboxylate**

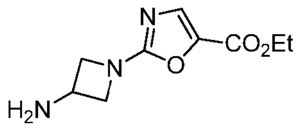

To a solution of ethyl 2-(3-((*tert*-butoxycarbonyl)amino)azetidin-1-yl)oxazole-5-carboxylate (0.850 g, 2.73 mmol) in DCM (12 mL) was added TFA (3 mL) and the mixture was stirred at RT. After 10 h volatiles were removed *in vacuo* and the residue was partitioned between sat. $Na_2CO_3$ / DCM. The aqueous layer was extracted with DCM (2X). Combined organics were washed (water, brine), dried over $Na_2SO_4$, and concentrated *in vacuo* affording the title compound (0.532 g, 92 % yield) as a colorless solid. $^1H$ NMR (400 MHz, $CDCl_3$) δ ppm 1.34 (t, *J* = 7 Hz, 3 H), 3.86 - 3.91 (m, 2 H), 3.99 - 4.07 (m, 1 H), 4.31 (q, *J* = 7 Hz, 2 H), 4.42 - 4.48 (m, 2 H), 7.51 (s, 1 H). MS (ESI) *m/z* 212 (M+H).

**C. Ethyl 2-(3-(7-(difluoromethoxy)-6-fluoroquinoline-3-carboxamido)azetidin-1-yl)oxazole-5-carboxylate**

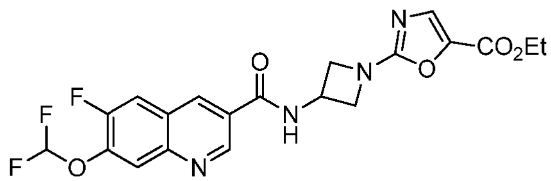

To a solution of 7-(difluoromethoxy)-6-fluoroquinoline-3-carboxylic acid hydrochloride (Intermediate 15) (0.641 g, 2.493 mmol) and HATU (0.995 g, 2.62 mmol) in DMF (6 mL) was added DIEA (0.457 mL, 2.62 mmol), dropwise, and the mixture was stirred 1 h at RT. A solution of ethyl 2-(3-aminoazetidin-1-yl)oxazole-5-carboxylate (0.526 g, 2.493 mmol) in DMF (2 mL) was added and the mixture was stirred at RT. After 15 h the mixture was transferred into EtOAc/water and stirred until dissolution was complete. Layers were separated and the aqueous layer was extracted with EtOAc (2X). Combined organics were washed (water, brine), dried over $Na_2SO_4$, and concentrated *in vacuo.* The residue was purified by flash chromatography (silica gel, EtOAc / hexanes, gradient elution) affording the title compound (0.916 g, 82 % yield) as a pale yellow solid. $^1H$ NMR (400 MHz, $CD_3SOCD_3$) δ ppm 1.26 (t, J = 7 Hz, 3 H), 4.19 - 4.28 (m, 4 H), 4.52 (t, *J* = 8 Hz, 2 H), 4.98 (qt, *J* = 8, 5 Hz, 1 H), 7.41 - 7.79 (m, 2 H), 8.00 (d, *J* = 8 Hz, 1 H), 8.17 (d, *J* = 11 Hz, 1 H), 8.85 (d, *J* = 2 Hz, 1 H), 9.31 (d, *J* = 2 Hz, 1 H), 9.51 (d, *J* = 7 Hz, 1 H). MS (ESI) *m/z* 451 (M+H).

**Example 85**

**7-(Difluoromethoxy)-6-fluoro-N-(*trans*-4-(((S)-1,1,1-trifluoro-3-hydroxypropan-2-yl)amino)cyclohexyl)quinoline-3-carboxamide**

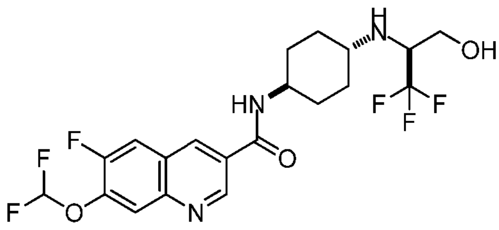

DIEA (0.356 mL, 2.038 mmol) was added to 7-(difluoromethoxy)-6-fluoroquinoline-3-carboxylic acid hydrochloride (Intermediate 15) (0.1048 g, 0.408 mmol) in DMF (1.003 mL) at RT. Then, HATU (0.201 g, 0.530 mmol) was added and the reaction mixture was stirred for 5 min. Then, (S)-2-((*trans*-4-aminocyclohexyl)amino)-3,3,3-trifluoropropan-1-ol (Intermediate 67) (0.097 g, 0.428 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (9:1) to give the title compound (0.1243 g, 62.3 % yield). $^1$H NMR (400 MHz, $CD_3SOCD_3$) δ 1.13 (q, *J* = 12 Hz, 2 H), 1.37 (dq, *J* = 13, 3 Hz, 2 H), 1.84-2.02 (m, 5 H), 2.48-2.58 (m, 1 H), 3.22-3.34 (m, 1 H), 3.44-3.52 (m, 1 H), 3.58-3.68 (m, 1 H), 3.72-3.86 (m, 1 H), 4.98 (t, *J* = 6 Hz, 1 H), 7.58 (t, *J* = 73 Hz, 1 H), 7.97 (d, *J* = 8 Hz, 1 H), 8.14 (d, *J* = 11 Hz, 1 H), 8.61 (d, *J* = 8 Hz, 1 H), 8.77 (d, *J* = 2 Hz, 1 H), 9.24 (d, *J* = 2 Hz, 1 H); LC-MS (LC-ES) M+H = 466.

**Example 86**

***trans*-Methyl 4-(7-(difluoromethoxy)-6-fluoroquinoline-3-carboxamido)cyclohexanecarboxylate**

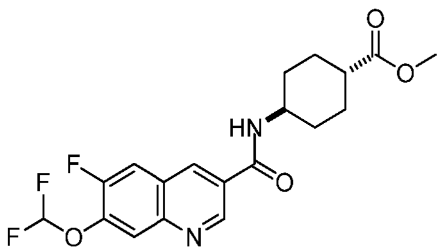

DIEA (0.364 mL, 2.086 mmol) was added to 7-(difluoromethoxy)-6-fluoroquinoline-3-carboxylic acid hydrochloride (Intermediate 15) (0.1073 g, 0.417 mmol) in DMF (1.026 mL) at RT. Then HATU (0.206 g, 0.542 mmol) was added and the reaction mixture was stirred for 5 min. Then, *trans*-methyl 4-aminocyclohexanecarboxylate (0.069 g, 0.438 mmol) was added and the reaction mixture was stirred for 16 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (7:3) to give the title compound (0.1122 g, 64.5 % yield). $^1$H NMR (400 MHz, $CD_3SOCD_3$) δ 1.39 (dq, *J* = 13, 3 Hz, 2 H), 1.45 (dq, *J* = 13, 3 Hz, 2 H), 1.96 (br t, *J* = 10 Hz, 4 H), 2.26-2.36 (m, 1 H), 3.60 (s, 3 H), 3.74-3.86 (m, 1 H), 7.58 (t, *J* = 73 Hz, 1 H), 7.97 (d, *J* = 8 Hz, 1 H), 8.14 (d, *J* = 11 Hz, 1 H), 8.66 (d, *J* = 8 Hz, 1 H), 8.78 (d, *J* = 2 Hz, 1 H), 9.25 (d, *J* = 2 Hz, 1 H); LC-MS (LC-ES) M+H = 397.

**Example 87**

**N-(*trans*-4-Cyanocyclohexyl)-7-(difluoromethoxy)-6-fluoroquinoline-3-carboxamide**

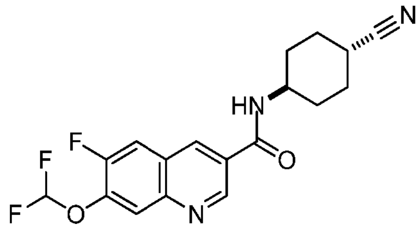

DIEA (0.678 mL, 3.88 mmol) was added to 7-(difluoromethoxy)-6-fluoroquinoline-3-carboxylic acid hydrochloride (Intermediate 15) (0.1998 g, 0.777 mmol) in DMF (1.91 mL) at RT. Then, HATU (0.384 g, 1.010 mmol) was added and the reaction mixture was stirred for 5 min. Then, *trans*-4-aminocyclohexanecarbonitrile hydrochloride (0.131 g, 0.816 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (7:3) to give the title compound (0.2076 g, 69.9 % yield). $^1$H NMR (400 MHz, $CD_3SOCD_3$) $\delta$ 1.40 (dq, *J* = 12, 3 Hz, 2 H), 1.64 (dq, *J* = 13, 3 Hz, 2 H), 1.92 (dd, *J* = 13, 3 Hz, 2 H), 2.06 (dd, *J* = 10, 3 Hz, 2 H), 2.71 (tt, *J* = 12, 4 Hz, 1 H), 3.80-3.92 (m, 1 H), 7.58 (t, *J* = 73 Hz, 1 H), 7.97 (d, *J* = 8 Hz, 1 H), 8.14 (d, *J* = 11 Hz, 1 H), 8.66 (d, *J* = 8 Hz, 1 H), 8.78 (d, *J* = 2 Hz, 1 H), 9.24 (d, *J* = 2 Hz, 1 H); LC-MS (LC-ES) M+H = 364.

**Example 88**

**Ethyl 2-(*trans*-4-(7-(difluoromethoxy)-6-fluoroquinoline-3-carboxamido)cyclohexyl)acetate**

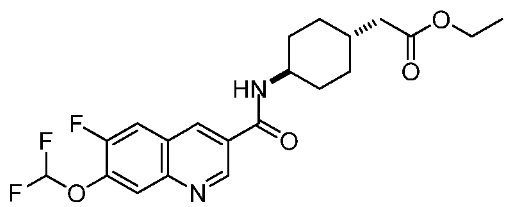

DIEA (0.533 mL, 3.05 mmol) was added to 7-(difluoromethoxy)-6-fluoroquinoline-3-carboxylic acid hydrochloride (Intermediate 15) (0.1570 g, 0.611 mmol) in DMF (1.50 mL) at RT. Then, HATU (0.302 g, 0.794 mmol) was added and the reaction mixture was stirred for 5 min. Then, ethyl 2-(*trans*-4-aminocyclohexyl)acetate hydrochloride (0.142 g, 0.641 mmol) was added and the reaction mixture was stirred for 16 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (3:2) to give the title compound (0.1449 g, 53.1 % yield). $^1$H NMR (400 MHz, $CD_3SOCD_3$) $\delta$ 1.10 (q, *J* = 12 Hz, 2 H), 1.18 (t, *J* = 7 Hz, 3 H), 1.37 (dq, *J* = 13, 3 Hz, 2 H), 1.60-1.74 (m, 1 H), 1.74 (br d, *J* = 13 Hz, 2 H), 1.90 (br d, *J* = 13 Hz, 2 H), 2.21 (d, *J* = 7 Hz, 2 H), 3.70-3.84 (m, 1 H), 4.05 (q, *J* = 7 Hz, 2 H), 7.58 (t, *J* = 73 Hz, 1 H), 7.97 (d, *J* = 8 Hz, 1 H), 8.14 (d, *J* = 11 Hz, 1 H), 8.62 (d, *J* = 8 Hz, 1 H), 8.78 (d, *J* = 2 Hz, 1 H), 9.25 (d, *J* = 2 Hz, 1 H); LC-MS (LC-ES) M+H = 425.

**Example 89**

**7-(Difluoromethoxy)-6-fluoro-N-(*cis*-4-(3-fluoroazetidin-1-yl)cyclohexyl)quinoline-3-carboxamide**

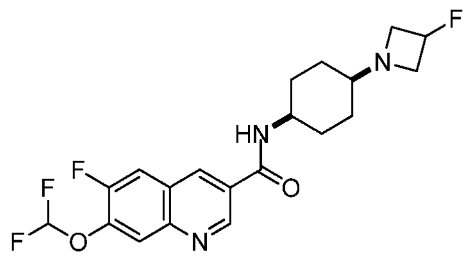

DIEA (0.286 mL, 1.635 mmol) was added to 7-(difluoromethoxy)-6-fluoroquinoline-3-carboxylic acid hydrochloride (Intermediate 15) (0.1051 g, 0.409 mmol) in DMF (1.077 mL) at RT. Then, HATU (0.233 g, 0.613 mmol) was added and the reaction mixture was stirred for 5 min. Then, *cis*-4-(3-fluoroazetidin-1-yl)cyclohexanamine (Intermediate 68) (0.084 g, 0.490 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc (1:9) to give the title compound (0.0867 g, 49.0 % yield). $^1$H NMR (400 MHz, $CD_3SOCD_3$) δ 1.38-1.48 (m, 2 H), 1.50-1.64 (m, 4 H), 1.71 (q, *J* = 12 Hz, 2 H), 2.24-2.30 (m, 1 H), 2.96-3.08 (m, 2 H), 3.46-3.58 (m, 2 H), 3.82-3.94 (m, 1 H), 5.12 (dp, *J* = 58, 5 Hz, 1 H), 7.57 (t, *J* = 73 Hz, 1 H), 7.96 (d, *J* = 8 Hz, 1 H), 8.11 (d, *J* = 11 Hz, 1 H), 8.60 (d, *J* = 8 Hz, 1 H), 8.80 (d, *J* = 2 Hz, 1 H), 9.25 (d, *J* = 2 Hz, 1 H); LC-MS (LC-ES) M+H = 412.

**Example 90**

**7-(Difluoromethoxy)-6-fluoro-N-(*trans*-4-(3-fluoroazetidin-1-yl)cyclohexyl)quinoline-3-carboxamide**

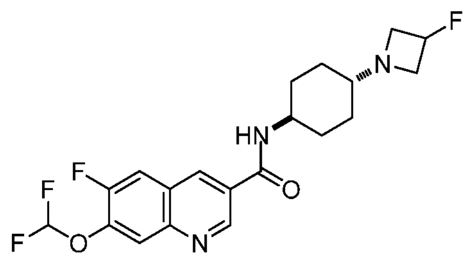

DIEA (0.289 mL, 1.653 mmol) was added to 7-(difluoromethoxy)-6-fluoroquinoline-3-carboxylic acid hydrochloride (Intermediate 15) (0.1063 g, 0.413 mmol) in DMF (1.089 mL) at RT. Then, HATU (0.236 g, 0.620 mmol) was added and the reaction mixture was stirred for 5 min. Then, *trans*-4-(3-fluoroazetidin-1-yl)cyclohexanamine (Intermediate 69) (0.085 g, 0.496 mmol) was added and the reaction mixture was stirred for 3 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc (1:4) to give the title compound (0.1078 g, 60.2 % yield). $^1$H NMR (400 MHz, $CD_3SOCD_3$) δ 1.01 (dq, *J* = 13, 3 Hz, 2 H), 1.35 (dq, *J* = 12, 3 Hz, 2 H), 1.77 (br d, *J* = 12 Hz, 2 H), 1.88 (dd, *J* = 13, 3 Hz, 2 H), 2.02 (tt, *J* = 11, 4 Hz, 1 H), 2.98-3.10 (m, 2 H), 3.46-3.58 (m, 2 H), 3.77 (qt, *J* = 7, 4 Hz, 1 H), 5.10 (dp, *J* = 58, 5 Hz, 1 H), 7.58 (t, *J* = 73 Hz, 1 H), 7.97 (d, *J* = 8 Hz, 1 H), 8.14 (d, *J* = 11 Hz, 1 H), 8.62 (d, *J* = 8 Hz, 1 H), 8.77 (d, *J* = 2 Hz, 1 H), 9.24 (d, *J* = 2 Hz, 1 H); LC-MS (LC-ES) M+H = 412.

**Example 91**

**7-(Difluoromethoxy)-6-fluoro-N-(*cis*-4-(3-hydroxy-3-(trifluoromethyl)azetidin-1-yl)cyclohexyl)quinoline-3-carboxamide**

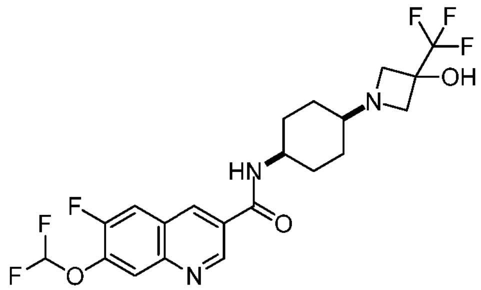

DIEA (0.295 mL, 1.688 mmol) was added to 7-(difluoromethoxy)-6-fluoroquinoline-3-carboxylic acid hydrochloride (Intermediate 15) (0.1085 g, 0.422 mmol) in DMF (1.112 mL) at RT. Then, HATU (0.241 g, 0.633 mmol) was added and the reaction mixture was stirred for 5 min. Then, 1-(*cis*-4-aminocyclohexyl)-3-(trifluoromethyl)azetidin-3-ol (Intermediate 70) (0.121 g, 0.506 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (4:1) to give the title compound (0.1589 g, 74.9 % yield). $^1$H NMR (400 MHz, $CD_3SOCD_3$) δ 1.45 (dq, *J* = 13, 3 Hz, 2 H), 1.48-1.64 (m, 4 H), 1.70 (q, *J* = 11 Hz, 2 H), 2.24-2.34 (m, 1 H), 3.07 (d, *J* = 9 Hz, 2 H), 3.48 (d, *J* = 9 Hz, 2 H), 3.82-3.94 (m, 1 H), 6.79 (s, 1 H), 7.57 (t, *J* = 73 Hz, 1 H), 7.96 (d, *J* = 8 Hz, 1 H), 8.12 (d, *J* = 11 Hz, 1 H), 8.62 (d, *J* = 8 Hz, 1 H), 8.79 (d, *J* = 2 Hz, 1 H), 9.24 (d, *J* = 2 Hz, 1 H); LC-MS (LC-ES) M+H = 478.

**Example 92**

**7-(Difluoromethoxy)-6-fluoro-N-(*trans*-4-(3-hydroxy-3-(trifluoromethyl)azetidin-1-yl)cyclohexyl)quinoline-3-carboxamide**

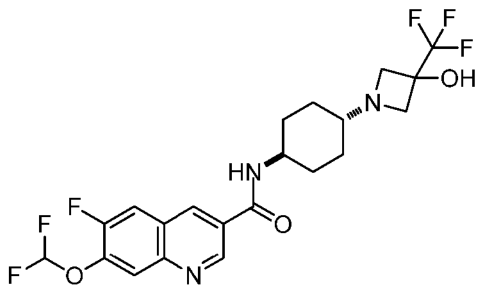

DIEA (0.294 mL, 1.685 mmol) was added to 7-(difluoromethoxy)-6-fluoroquinoline-3-carboxylic acid hydrochloride (Intermediate 15) (0.1083 g, 0.421 mmol) in DMF (1.11 mL) at RT. Then, HATU (0.240 g, 0.632 mmol) was added and the reaction mixture was stirred for 5 min. Then, 1-(*trans*-4-aminocyclohexyl)-3-(trifluoromethyl)azetidin-3-ol (Intermediate 71) (0.120 g, 0.505 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc (1:12) to give the title compound (0.1501 g, 70.9 % yield). $^1$H NMR (400 MHz, $CD_3SOCD_3$) δ 1.01 (dq, *J* = 13 Hz, 2 H), 1.35 (q, *J* = 14 Hz, 2 H), 1.77 (br d, *J* = 11 Hz, 2 H), 1.88 (dd, *J* = 13, 3 Hz, 2 H), 2.04 (tt, *J* = 11, 4 Hz, 1 H), 3.12 (d, *J* = 9 Hz, 2 H), 3.48 (d, *J* = 9 Hz, 2 H), 3.77 (qt, *J* = 7, 4 Hz, 1 H), 6.80 (s, 1 H), 7.58 (t, *J* = 73 Hz, 1 H), 7.97 (d, *J* = 8 Hz, 1 H), 8.14 (d, *J* = 11 Hz, 1 H), 8.61 (d, *J* = 8 Hz, 1 H), 8.77 (d, *J* = 2 Hz, 1 H), 9.24 (d, *J* = 2 Hz, 1 H); LC-MS (LC-ES) M+H = 478.

**Example 93**

**7-(Difluoromethoxy)-6-fluoro-N-((1s,3s)-3-hydroxy-3-methylcyclobutyl)quinoline-3-carboxamide**

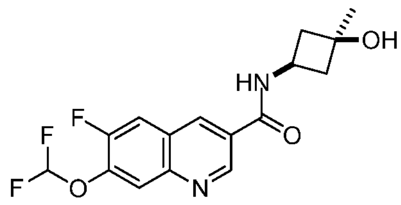

DIEA (0.304 mL, 1.741 mmol) was added to 7-(difluoromethoxy)-6-fluoroquinoline-3-carboxylic acid hydrochloride (Intermediate 15) (0.1119 g, 0.435 mmol) in DMF (1.146 mL) at RT. Then, HATU (0.248 g, 0.653 mmol) was added and the reaction mixture was stirred for 5 min. Then, (1s,3s)-3-amino-1-methylcyclobutanol hydrochloride (Intermediate 51) (0.090 g, 0.653 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc to give the title compound (0.0189 g, 11.4 % yield). $^1$H NMR (400 MHz, $CD_3SOCD_3$) δ 1.28 (s, 3 H), 2.13 (dt, *J* = 9, 2 Hz, 2 H), 2.32 (dt, *J* = 8, 3 Hz, 2 H), 4.02 (h, *J* = 8 Hz, 1 H), 5.02 (s, 1 H), 7.58 (t, *J* = 73 Hz, 1 H), 7.97 (d, *J* = 8 Hz, 1 H), 8.13 (d, *J* = 11 Hz, 1 H), 8.80 (d, *J* = 2 Hz, 1 H), 8.99 (d, *J* = 7 Hz, 1 H), 9.27 (d, *J* = 2 Hz, 1 H); LC-MS (LC-ES) M+H = 341.

**Example 94**

**7-(Difluoromethoxy)-6-fluoro-N-(trans-4-(2-(methylsulfonyl)ethoxy)cyclohexyl)quinoline-3-carboxamide**

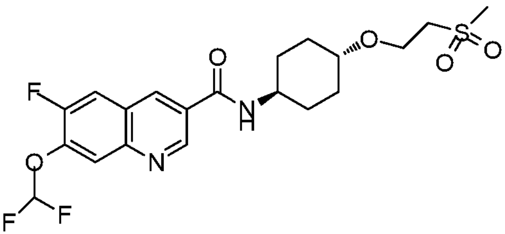

To a solution of 7-(difluoromethoxy)-6-fluoroquinoline-3-carboxylic acid hydrochloride (Intermediate 15) (48 mg, 0.187 mmol) in DMF (5 mL) at RT, was added DIEA (0.098 mL, 0.560 mmol), HATU (106 mg, 0.280 mmol), and trans-4-(2-(methylsulfonyl)ethoxy)cyclohexanamine (Intermediate 72) (49.6 mg, 0.224 mmol). After stirring at RT for 24 h, the solvent was removed under reduced pressure, the residue dissolved in 10% MeOH/DCM (50 mL), absorbed onto Celite® and purified by reverse phase chromatography (10-50% acetonitrile/ water, with 0.2% $NH_4OH$) to afford the title compound (30 mg, 35% yield). $^1$H NMR (400 MHz, $CD_3SOCD_3$) δ ppm 1.19 - 1.49 (m, 4 H), 1.92-1.94 (m, 2 H), 2.04-2.06 (m, 2 H), 2.99 (s, 3 H), 3.27 - 3.38 (m, 3 H), 3.70 - 3.90 (m, 3 H), 7.59 (t, *J* = 73 Hz, 1 H), 7.98 (d, *J* = 8 Hz, 1 H), 8.15 (d, *J* = 11 Hz, 1 H), 8.64 (d, *J* = 8 Hz, 1 H), 8.79 (d, *J* = 2 Hz, 1 H), 9.26 (d, *J* = 2 Hz, 1 H). MS (ESI) *m/z* 461 (M+1).

**Example 95**

**7-(Difluoromethoxy)-6-fluoro-N-(1-(pyridin-2-yl)piperidin-4-yl)quinoline-3-carboxamide**

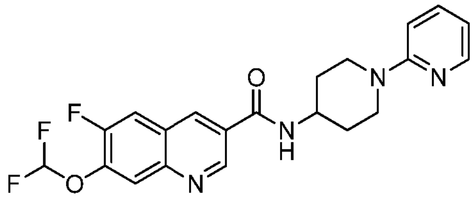

To a solution of 7-(difluoromethoxy)-6-fluoroquinoline-3-carboxylic acid hydrochloride (Intermediate 15) (0.1071 g, 0.416 mmol) and HATU (0.166 g, 0.437 mmol) in anhydrous DMF (3 mL) was added DIEA (0.076 mL, 0.437 mmol),

and the mixture was stirred at RT. After 1 h 1-(pyridin-2-yl)piperidin-4-amine (0.081 g, 0.458 mmol) was added in one portion and stirring continued. After 18 h the mixture was poured into sat. $NaHCO_3$ and extracted with EtOAc (x3). Combined organics were washed (water, brine), dried over $Na_2SO_4$, and concentrated *in vacuo.* The residue was purified by reverse phase flash chromatography (MeCN / water gradient) affording the title compound (0.0936 g, 54 % yield) as a pale yellow solid. $^1H$ NMR (400 MHz, $CD_3SOCD_3$) δ ppm 1.57 (qd, *J* = 12, 4 Hz, 2 H), 1.91 (dd, *J* = 12, 3 Hz, 2 H), 2.93 - 3.03 (m, 2 H), 4.09 - 4.21 (m, 1 H), 4.32 (d, *J* = 13 Hz, 2 H), 6.61 (ddd, *J* = 7, 5, 1 Hz, 1 H), 6.88 (d, *J* = 9 Hz, 1 H), 7.39 - 7.79 (m, 2 H), 7.98 (d, *J* = 8 Hz, 1 H), 8.09 - 8.17 (m, 2 H), 8.69 (d, *J* = 8 Hz, 1 H), 8.81 (d, *J* = 2 Hz, 1 H), 9.27 (d, *J* = 2 Hz, 1 H). MS (ESI) *m/z* 417 (M+H).

**Example 96**

**7-(Difluoromethoxy)-6-fluoro-N-(1-(pyridin-3-yl)piperidin-4-yl)quinoline-3-carboxamide**

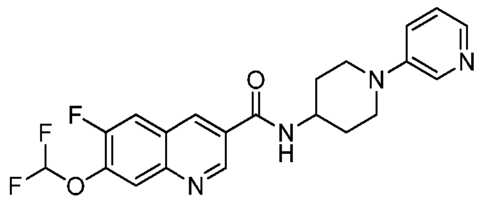

To a solution of 7-(difluoromethoxy)-6-fluoroquinoline-3-carboxylic acid hydrochloride (Intermediate 15) (0.1012 g, 0.394 mmol) and HATU (0.157 g, 0.413 mmol) in anhydrous DMF (3 mL) was added DIEA (0.072 mL, 0.413 mmol), and the mixture was stirred at RT. After 1 h 1-(pyridin-3-yl)piperidin-4-amine (0.077 g, 0.433 mmol) was added in one portion and stirring continued. After 18 h the mixture was poured into sat. $NaHCO_3$ and extracted with EtOAc (3X). Combined organics were washed (water, brine), dried over $Na_2SO_4$, and concentrated *in vacuo.* The residue was purified by reverse phase flash chromatography (MeCN / water gradient) affording the title compound (0.0947 g, 58 % yield) as a colorless solid. $^1H$ NMR (400 MHz, $CD_3SOCD_3$) δ ppm 1.63 - 1.76 (m, 2 H), 1.95 (dd, *J* = 12, 2 Hz, 2 H), 2.92 (dt, *J* = 11, 2 Hz, 2 H), 3.83 (d, *J* = 13 Hz, 2 H), 4.01 - 4.15 (m, 1 H), 7.21 (dd, *J* = 8, 5 Hz, 1 H), 7.32-7.38 (m, 1 H), 7.59 (t, *J* = 73 Hz, 1 H), 7.94 - 8.02 (m, 2 H), 8.15 (d, *J* = 11 Hz, 1 H), 8.34 (d, *J* = 3 Hz, 1 H), 8.73 (d, *J* = 8 Hz, 1 H), 8.82 (d, *J* = 2 Hz, 1 H), 9.28 (d, *J* = 2 Hz, 1 H). MS (ESI) *m/z* 417 (M+H).

**Example 97**

**N-(1-(5-Cyanooxazol-2-yl)piperidin-4-yl)-7-(difluoromethoxy)-6-fluoroquinoline-3-carboxamide**

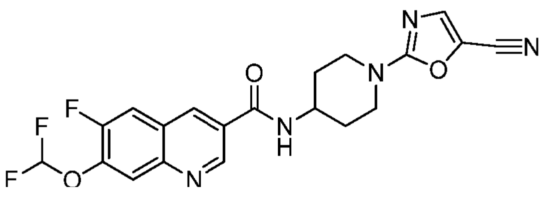

**A. Ethyl 2-(4-((*tert*-butoxycarbonyl)amino)piperidin-1-yl)oxazole-5-carboxylate**

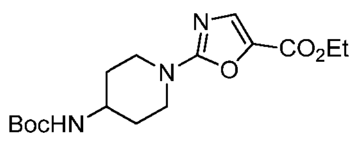

A mixture of *tert*-butyl piperidin-4-ylcarbamate (1.00 g, 4.99 mmol), ethyl 2-chlorooxazole-5-carboxylate (0.659

mL, 4.99 mmol), and $K_2CO_3$ (1.380 g, 9.99 mmol) in anhydrous MeCN (12 mL) was stirred in a heating block at 80 °C. After 15 h the mixture was cooled, poured into water and extracted with EtOAc (3X). Combined organics were washed (water, brine), dried over $Na_2SO_4$, and concentrated *in vacuo.* The residue was purified by flash chromatography (silica gel, EtOAc / hexanes, gradient elution) affording the title compound (1.685 g, 99 % yield) as a colorless solid. $^1H$ NMR (400 MHz, $CDCl_3$) δ ppm 1.34 (t, *J* = 7 Hz, 3 H), 1.41 - 1.53 (m, 11 H), 2.04 (dd, *J* = 13, 3 Hz, 2 H), 3.16 (ddd, *J* = 14, 12, 3 Hz, 2 H), 3.68 (br s, 1 H), 4.17 (dt, *J* = 14, 3 Hz, 2 H), 4.31 (q, *J* = 7 Hz, 2 H), 4.48 (br s, 1 H), 7.52 (s, 1 H). MS (ESI) *m/z* 340 (M+H).

**B. *tert*-Butyl (1-(5-carbamoyloxazol-2-yl)piperidin-4-yl)carbamate**

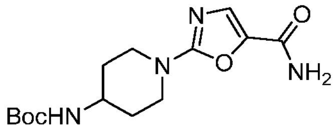

A thick-walled glass pressure vessel was charged with ethyl 2-(4-((*tert*-butoxycarbonyl)amino)piperidin-1-yl)oxazole-5-carboxylate (1.10 g, 3.24 mmol), a small crystal of NaCN (ca. 0.010 g), and a solution of $NH_3$ in MeOH (30 mL, 210 mmol) and sealed with a teflon bushing. The mixture was stirred in an oil bath at 80 °C for 30 h. Volatiles were removed *in vacuo* and the residue was partitioned between sat. $NaHCO_3$ / EtOAc. The aqueous layer was extracted with EtOAc (2X). Combined organics were washed (water, brine), dried over $Na_2SO_4$, and concentrated *in vacuo* to give the title compound (0.959 g, 3.09 mmol, 95 % yield) as a yellow solid, used without further purification. $^1H$ NMR (400 MHz, $CD_3SOCD_3$) δ ppm 1.30 - 1.43 (m, 11 H), 1.78 (dd, *J* = 13, 3 Hz, 2 H), 3.09 (ddd, *J* = 13, 12, 3 Hz, 2 H), 3.42 - 3.57 (m, 1 H), 3.93 - 4.02 (m, 2 H), 6.88 (d, *J* = 8 Hz, 1 H), 7.19 (br s, 1 H), 7.40 (s, 1 H), 7.53 (br s, 1 H). MS (ESI) *m/z* 311 (M+H).

**C. *tert*-Butyl (1-(5-cyanooxazol-2-yl)piperidin-4-yl)carbamate**

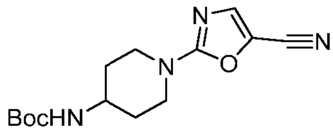

An oven dried vial was charged with *tert*-butyl (1-(5-carbamoyloxazol-2-yl)piperidin-4-yl)carbamate (0.317 g, 1.021 mmol), PhMe (3 mL), phenylsilane (0.126 mL, 1.021 mmol), and a solution of TBAF in THF (0.051 mL, 0.051 mmol) and sealed with a crimp top. *N.B.* gas evolution was observed during addition of TBAF. The mixture was stirred in an oil bath at 80 °C for 45 min. Volatiles were removed *in vacuo* and the residue was purified by flash chromatography (silica gel, EtOAc / hexanes, gradient elution) affording the title compound (0.058 g, 19 % yield) as a yellow solid. $^1H$ NMR (400 MHz, $CDCl_3$) δ ppm 1.37 - 1.51 (m, 11 H), 2.00 - 2.11 (m, 2 H), 3.18 (ddd, *J* = 14, 12, 3 Hz, 2 H), 3.68 (br s, 1 H), 4.12 (dt, *J* = 14, 3 Hz, 2 H), 4.47 (br s, 1 H), 7.45 (s, 1 H). MS (ESI) *m/z* 293 (M+H).

**D. 2-(4-Aminopiperidin-1-yl)oxazole-5-carbonitrile**

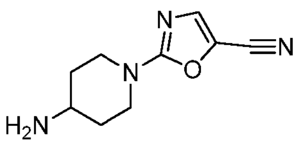

To a solution of *tert*-butyl (1-(5-cyanooxazol-2-yl)piperidin-4-yl)carbamate (0.058 g, 0.198 mmol) in DCM (4 mL) was added TFA (1 mL) and the mixture was stirred at RT for 1 h. Volatiles were removed *in vacuo* and the residue

was partitioned between DCM / 1N NaOH. The aqueous layer was extracted with DCM (2X). Combined organics were washed (water, brine), dried over $Na_2SO_4$, and concentrated *in vacuo* affording 2-(4-aminopiperidin-1-yl)oxazole-5-carbonitrile (0.0312 g, 82 % yield) as a yellow semi-solid which was used without further purification. MS (ESI) *m/z* 193 (M+H).

**E. N-(1-(5-Cyanooxazol-2-yl)piperidin-4-yl)-7-(difluoromethoxy)-6-fluoroquinoline-3-carboxam**ide

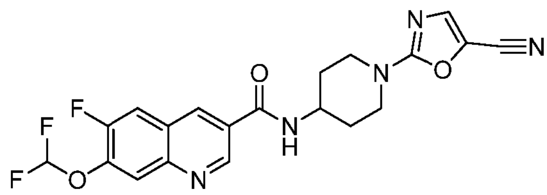

To a solution of 7-(difluoromethoxy)-6-fluoroquinoline-3-carboxylic acid hydrochloride (Intermediate 15) (0.046 g; 0.18 mmol) and HATU (0.071 g; 0.19 mmol) in anhydrous DMF (2 mL) was added DIEA (0.033 mL; 0.19 mmol) and the mixture was stirred 90 min at RT. A solution of 2-(4-aminopiperidin-1-yl)oxazole-5-carbonitrile (0.031 g; 0.16 mmol) in DMF (1 mL) was added *via* syringe and the mixture was stirred 15 h at RT. The mixture was poured into sat. $NaHCO_3$ and extracted with EtOAc (3X). Combined organics were washed (water, brine), dried over $Na_2SO_4$, and concentrated *in vacuo*. The residue was purified by preparative HPLC (C18 column, MeCN / water gradient with 0.1% TFA modifier) affording the title compound (0.0479 g, 68 % yield) as a yellow film. $^1H$ NMR (400 MHz, $CD_3SOCD_3$) $\delta$ ppm 1.55 - 1.70 (m, 2 H), 1.98 (dd, *J* = 13, 3 Hz, 2 H), 3.24 - 3.37 (m, 2 H), 4.01 - 4.21 (m, 3 H), 7.59 (t, *J* = 73 Hz, 2 H), 7.96 - 8.02 (m, 2 H), 8.16 (d, *J* = 11 Hz, 1 H), 8.76 (d, *J* = 7 Hz, 1 H), 8.82 (d, *J* = 2 Hz, 1 H), 9.28 (d, *J* = 2 Hz, 1 H). MS (ESI) *m/z* 432 (M+H).

**Example 98**

**N-(1-(5-Cyanopyridin-2-yl)azetidin-3-yl)-7-(difluoromethoxy)-6-fluoroquinoline-3-carboxamide**

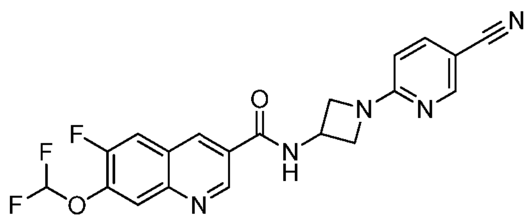

*A.* ***tert*-Butyl 3-(7-(difluoromethoxy)-6-fluoroquinoline-3-carboxamido)azetidine-1-carboxylate**

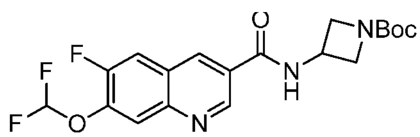

To a slurry of 7-(difluoromethoxy)-6-fluoroquinoline-3-carboxylic acid hydrochloride (Intermediate 15) (0.257 g, 0.999 mmol) and HATU (0.399 g, 1.049 mmol) in DMF (3 mL) was added DIEA (0.183 mL, 1.049 mmol), and the solution was stirred 1 h under nitrogen. *Tert*-butyl 3-aminoazetidine-1-carboxylate (0.181 g, 1.049 mmol) was added in one portion and the mixture was stirred overnight. After 18 h the mixture was poured into sat. $NaHCO_3$ and extracted with EtOAc (3X). Combined organics were washed (water, brine), dried over $Na_2SO_4$, and concentrated *in vacuo*. The residue was purified by flash chromatography (EtOAc/hexanes gradient) affording the title compound (0.317 g, 77 % yield) as a colorless solid. $^1H$ NMR (400 MHz, $CD_3SOCD_3$) $\delta$ ppm 1.40 (s, 9 H), 3.91 (dd, *J* = 8, 6 Hz, 2 H), 4.17 (t, *J* =

8 Hz, 2 H), 4.66 - 4.78 (m, 1 H), 7.59 (t, *J* = 73 Hz, 1 H), 7.99 (d, *J* = 8 Hz, 1 H), 8.16 (d, *J* = 11 Hz, 1 H), 8.84 (d, *J* = 2 Hz, 1 H), 9.30 (d, *J* = 2 Hz, 1 H), 9.38 (d, *J* = 7 Hz, 1 H). MS (ESI) *m/z* 412 (M+H).

**B. N-(Azetidin-3-yl)-7-(difluoromethoxy)-6-fluoroquinoline-3-carboxamide**

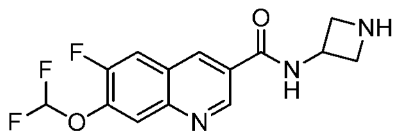

To a solution of *tert*-butyl 3-(7-(difluoromethoxy)-6-fluoroquinoline-3-carboxamido)azetidine-1-carboxylate (0.297 g, 0.722 mmol) in DCM (5 mL) was added TFA (1 mL) and the mixture was stirred overnight. After 18 h volatiles were removed *in vacuo.* The residue was partitioned between DCM / sat. $NaHCO_3$ and the layers were separated. The aqueous layer was extracted with DCM (2X). Combined organics were washed (water, brine), dried over $Na_2SO_4$, and concentrated *in vacuo* affording N-(azetidin-3-yl)-7-(difluoromethoxy)-6-fluoroquinoline-3-carboxamide (0.197 g, 88 % yield) as a tan solid, used without further purification. MS (ESI) *m/z* 312 (M+H).

**C. N-(1-(5-Cyanopyridin-2-yl)azetidin-3-yl)-7-(difluoromethoxy)-6-fluoroquinoline-3-carboxamide**

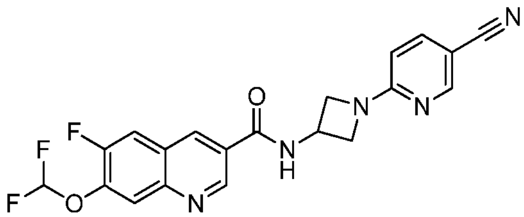

An oven dried vial was charged with N-(azetidin-3-yl)-7-(difluoromethoxy)-6-fluoroquinoline-3-carboxamide (0.0566 g, 0.182 mmol), 6-bromonicotinitrile (0.033 g; 0.182 mmol), $K_2CO_3$ (0.050 g; 0.36 mmol) and DMF (3 mL). The mixture was stirred in a heating block at 80 °C for 3 h. Upon cooling the mixture was poured into sat. $NaHCO_3$, mixed with EtOAc and allowed to partition. The resulting solids were collected by filtration and dried on the Buchner funnel affording the title compound (0.0318 g, 42 % yield) as a colorless solid. $^1$H NMR (400 MHz, $CD_3SOCD_3$) $\delta$ ppm 4.13 (dd, *J* = 9, 5 Hz, 2 H), 4.45 (t, *J* = 9 Hz, 2 H), 4.94 (m, *J* = 5 Hz, 1 H), 6.52 (d, *J* = 9 Hz, 1 H), 7.60 (t, *J* = 73 Hz, 1 H), 7.86 (dd, *J* = 9, 2 Hz, 1 H), 7.99 (d, *J* = 8 Hz, 1 H), 8.16 (d, *J* = 11 Hz, 1 H), 8.49 (dd, *J* = 2, 1 Hz, 1 H), 8.88 (d, *J* = 2 Hz, 1 H), 9.32 (d, *J* = 2 Hz, 1 H), 9.56 (br s, 1 H). MS (ESI) *m/z* 414 (M+H).

**Example 99 and 100**

**7-(Difluoromethoxy)-N-(*trans*-4-((((R)-1,1-difluoropropan-2-yl)amino)methyl)cyclohexyl)-6-fluoroquinoline-3-carboxamide and 7-(Difluoromethoxy)-N-(*trans*-4-((((S)-1,1-difluoropropan-2-yl)amino)methyl)cyclohexyl)-6-fluoroquinoline-3-carboxamide**

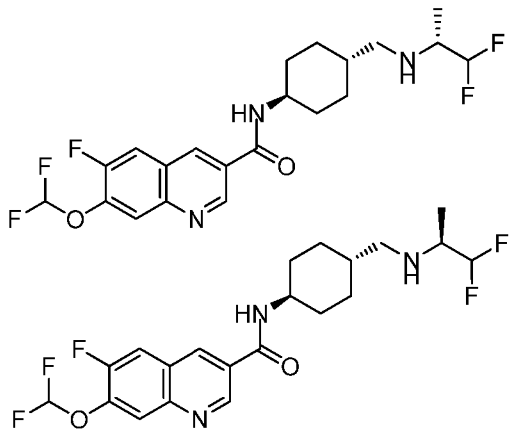

Racemic 7-(difluoromethoxy)-N-(*trans*-4-(((1,1-difluoropropan-2-yl)amino)methyl)cyclohexyl)-6-fluoroquinoline-3-carboxamide (Example 75) (0.1674 g, 0.376 mmol) was separated into its enantiomers via super critical fluid chromatography on a chiral ADH column eluting with MeOH:carbon dioxide (1:4 with 0.1% ammonium hydroxide) to give 7-(difluoromethoxy)-N-(trans-4-((((R)-1,1-difluoropropan-2-yl)amino)methyl)cyclohexyl)-6-fluoroquinoline-3-carboxamide (0.0558 g, 31.7 % yield) as the first enantiomer to elute and 7-(difluoromethoxy)-N-(*trans*-4-((((S)-1,1-difluoropropan-2-yl)amino)methyl)cyclohexyl)-6-fluoroquinoline-3-carboxamide (0.0598 g, 33.9 % yield) as the last enantiomer to elute. The structures were assigned by vibrational circular dichroism.

**7-(Difluoromethoxy)-N-(*trans*-4-((((R)-1,1-difluoropropan-2-yl)amino)methyl)cyclohexyl)-6-fluoroquinoline-3-carboxamide:** $^1$H NMR (400 MHz, $CD_3SOCD_3$) δ 0.98 (q, *J* = 13 Hz, 2 H), 1.02 (d, *J* = 7 Hz, 3 H), 1.24-1.40 (m, 3 H), 1.59 (br s, 1 H), 1.78-1.90 (m, 2 H), 1.91 (br d, *J* = 10 Hz, 2 H), 2.36-2.52 (m, 2 H), 2.74-2.88 (m, 1 H), 3.78 (qt, *J* = 8, 4 Hz, 1 H), 5.80 (dt, *J* = 57, 4 Hz, 1 H), 7.58 (t, *J* = 73 Hz, 1 H), 7.97 (d, *J* = 8 Hz, 1 H), 8.13 (d, *J* = 11 Hz, 1 H), 8.62 (d, *J* = 8 Hz, 1 H), 8.78 (d, *J* = 2 Hz, 1 H), 9.25 (d, *J* = 2 Hz, 1 H); LC-MS (LC-ES) M+H = 446.

**7-(Difluoromethoxy)-N-(*trans*-4-((((S)-1,1-difluoropropan-2-yl)amino)methyl)cyclohexyl)-6-fluoroquinoline-3-carboxamide:** $^1$H NMR (400 MHz, $CD_3SOCD_3$) δ 0.98 (q, *J* = 13 Hz, 2 H), 1.02 (d, *J* = 7 Hz, 3 H), 1.24-1.40 (m, 3 H), 1.59 (br s, 1 H), 1.78-1.90 (m, 2 H), 1.91 (br d, *J* = 10 Hz, 2 H), 2.36-2.52 (m, 2 H), 2.74-2.88 (m, 1 H), 3.78 (qt, *J* = 8, 4 Hz, 1 H), 5.80 (dt, *J* = 57, 4 Hz, 1 H), 7.58 (t, *J* = 73 Hz, 1 H), 7.97 (d, *J* = 8 Hz, 1 H), 8.13 (d, *J* = 11 Hz, 1 H), 8.62 (d, *J* = 8 Hz, 1 H), 8.78 (d, *J* = 2 Hz, 1 H), 9.25 (d, *J* = 2 Hz, 1 H); LC-MS (LC-ES) M+H = 446.

**Example 101**

**7-(Difluoromethoxy)-6-fluoro-N-(trans-4-((prop-2-yn-1-yloxy)methyl)cyclohexyl)quinoline-3-carboxamide**

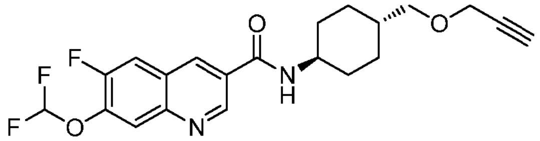

To a solution of 7-(difluoromethoxy)-6-fluoroquinoline-3-carboxylic acid hydrochloride (Intermediate 15) (30 mg, 0.12 mmol) in DMF (1 mL) was added DIEA (0.06 mL, 0.35 mmol) and HATU (53 mg, 0.12 mmol). After stirring for 5 min trans-4-((prop-2-yn-1-yloxy)methyl)cyclohexanamine hydrochloride (Intermediate 73) (24 mg, 0.12 mmol) was added. The reaction was stirred at RT overnight, quenched into water and extracted with EtOAc. The combined organics were dried over sodium sulfate, filtered, and concentrated. The residue was purified by silica gel chromatography (0-50% (3:1 EtOAc:EtOH)-hexanes gradient) to afford the title compound as a white solid (32 mg, 67% yield). $^1$H NMR (400 MHz, $CD_3SOCD_3$) δ ppm 0.99 - 1.17 (m, 2 H), 1.29 - 1.45 (m, 2 H), 1.54 (d, *J* = 3 Hz, 1 H), 1.80 (d, *J* = 12 Hz, 2 H), 1.93 (d, *J* = 10 Hz, 2 H), 3.29 (d, *J* = 6 Hz, 2 H), 3.42 (s, 1 H), 3.71 - 3.89 (m, 1 H), 4.12 (d, *J* = 2 Hz, 2 H), 7.59 (t, *J* = 73 Hz, 1 H), 7.98 (d, *J* = 8 Hz, 1 H), 8.15 (d, *J* = 11 Hz, 1 H), 8.64 (d, *J* = 8 Hz, 1 H), 8.80 (s, 1 H), 9.26 (d, *J* = 2 Hz, 1 H). MS (ESI) *m/z* 407 (M+1).

**Example 102**

**7-(Difluoromethoxy)-6-fluoro-N-(trans-4-(2-oxo-2-(prop-2-yn-1-ylamino)ethoxy)cyclohexyl)quinoline-3-carboxamide**

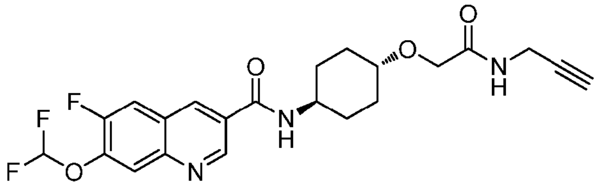

A. **tert-Butyl 2-((trans-4-(7-(difluoromethoxy)-6-fluoroquinoline-3-carboxamido)cyclohexyl)oxy)acetate**

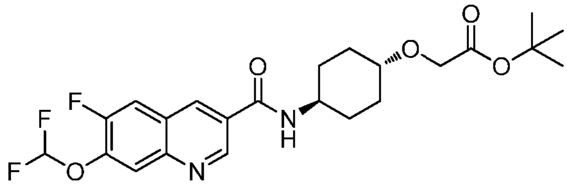

To a solution of 7-(difluoromethoxy)-6-fluoroquinoline-3-carboxylic acid hydrochloride (Intermediate 15) (150 mg, 0.58 mmol) in DMF (5 mL) was added DIEA (0.31 mL, 1.75 mmol) and HATU (266 mg, 0.70 mmol). After stirring for 5 min tert-butyl 2-((trans-4-aminocyclohexyl)oxy)acetate (intermediate 80) (134 mg, 0.583 mmol) was added as a solution in DMF (1 mL). The reaction was stirred at RT overnight, quenched into water and extracted with EtOAc. The combined organics were dried over sodium sulfate, filtered, and concentrated. The residue was purified by silica gel chromatography (0-50% (3:1 EtOAc:EtOH)-hexanes gradient) to afford the title compound as a pale yellow solid (195 mg, 71% yield). $^1H$ NMR (400 MHz, $CD_3SOCD_3$) δ ppm 1.22 - 1.40 (m, 4 H), 1.44 (s, 9 H), 1.93 (d, *J* = 11 Hz, 2 H), 2.05 (d, *J* = 11 Hz, 2 H), 3.34 - 3.38 (m, 1 H), 3.77 - 3.88 (m, 1 H), 4.01 (s, 2 H), 7.59 (t, *J* = 73 Hz, 1 H), 7.98 (d, *J* = 8 Hz, 1 H), 8.15 (d, *J* = 11 Hz, 1 H), 8.61 (d, *J* = 8 Hz, 1 H), 8.79 (d, *J* = 2 Hz, 1 H), 9.26 (d, *J* = 2 Hz, 1 H). MS (ESI) *m/z* 469 (M+1).

B. **2-((trans-4-(7-(Difluoromethoxy)-6-fluoroquinoline-3-carboxamido)cyclohexyl)oxy)acetic acid trifluoroacetic acid salt**

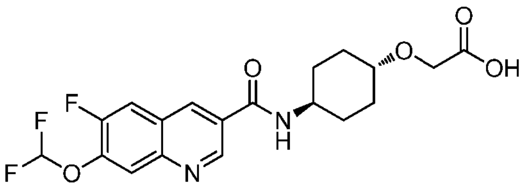

To a solution of tert-butyl 2-((trans-4-(7-(difluoromethoxy)-6-fluoroquinoline-3-carboxamido)cyclohexyl)oxy)acetate (185 mg, 0.38 mmol) in DCM (5 mL) was added TFA (5 mL) and the reaction was stirred at RT overnight. The mixture was concentrated to afford the title compound as a yellow solid (220 mg, 109%). $^1H$ NMR (400 MHz, $CD_3SOCD_3$) δ ppm 1.16 - 1.45 (m, 4 H), 1.90 (d, *J* = 11 Hz, 2 H), 2.03 (d, *J* = 11 Hz, 2 H), 3.25 - 3.38 (m, 1 H), 3.71 - 3.88 (m, 1 H), 4.02 (s, 2 H), 7.58 (t, *J* = 74 Hz, 1 H), 7.96 (d, *J* = 8 Hz, 1 H), 8.14 (d, *J* = 11 Hz, 1 H), 8.62 (d, *J* = 8 Hz, 1 H), 8.77 (d, *J* = 2 Hz, 1 H), 9.24 (d, *J* = 2 Hz, 1 H).MS (ESI) *m/z* 413 (M+1).

C. **7-(Difluoromethoxy)-6-fluoro-N-(trans-4-(2-oxo-2-(prop-2-yn-1-ylamino)ethoxy)cyclohexyl)quinoline-3-carboxamide**

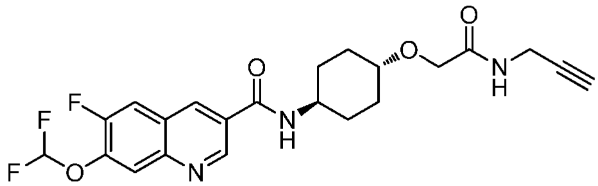

To a solution of 2-((trans-4-(7-(difluoromethoxy)-6-fluoroquinoline-3-carboxamido)cyclohexyl)oxy)acetic acid trifluoroacetic acid salt (100 mg, 0.243 mmol) in DMF (2 mL) was added DIEA (0.17 mL, 0.97 mmol) and HATU (184 mg, 0.49 mmol). After stirring for 5 min, prop-2-yn-1-amine (26.7 mg, 0.485 mmol) was added. The reaction was stirred at RT overnight, quenched into water and extracted with EtOAc. The combined organics were dried over sodium sulfate, filtered, and concentrated. The residue was purified by silica gel chromatography (0-60% (3:1 EtOAc:EtOH)-hexanes gradient) to afford the title compound as a white solid (54 mg, 50% yield). $^1$H NMR (400 MHz, $CD_3SOCD_3$) $\delta$ ppm 1.25 - 1.48 (m, 4 H), 1.87 - 1.97 (m, 2 H), 2.06 (d, *J* = 9 Hz, 2 H), 3.06 (t, *J* = 2 Hz, 1 H), 3.31 - 3.36 (m, 1 H), 3.81 (d, *J* = 7 Hz, 1 H), 3.87 (dd, *J* = 6, 2 Hz, 2 H), 3.90 (s, 2 H), 7.57 (t, *J* = 73 Hz, 1 H), 7.96 (d, *J* = 8 Hz, 1 H), 8.05 (t, *J* = 6 Hz, 1 H), 8.13 (d, *J* = 11 Hz, 1 H), 8.62 (d, *J* = 8 Hz, 1 H), 8.77 (d, *J* = 2 Hz, 1 H), 9.24 (d, *J* = 2 Hz, 1 H). MS (ESI) *m/z* 450 (M+1).

## Example 103

**N-(trans-4-(2-(but-3-yn-1-ylamino)-2-oxoethoxy)cyclohexyl)-7-(difluoromethoxy)-6-fluoroquinoline-3-carboxamide**

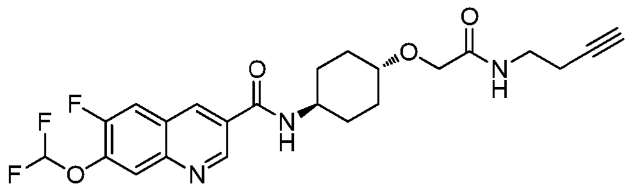

To a solution of 2-((trans-4-(7-(difluoromethoxy)-6-fluoroquinoline-3-carboxamido)cyclohexyl)oxy)acetic acid trifluoroacetic acid salt (Example 102, step B) (155 mg, 0.38 mmol) in DMF (2 mL) was added DIEA (0.26 mL, 1.50 mmol) and HATU (286 mg, 0.75 mmol). After stirring for 5 min, but-3-yn-1-amine (52.0 mg, 0.752 mmol) was added. The reaction was stirred at RT overnight, quenched into water and extracted with EtOAc. The combined organics were dried over sodium sulfate, filtered, and concentrated. The residue was purified by silica gel chromatography (0-65% (3:1 EtOAc:EtOH)-hexanes gradient) to afford the title compound as a white solid (129 mg, 74% yield). $^1$H NMR (400 MHz, $CD_3SOCD_3$) $\delta$ ppm 1.24 - 1.47 (m, 4 H), 1.88 - 1.96 (m, 2 H), 2.06 (d, *J* = 10 Hz, 2 H), 2.32 (td, *J* = 7, 3 Hz, 2 H), 2.82 (t, *J* = 3 Hz, 1 H), 3.22 (q, *J* = 7 Hz, 2 H), 3.32 - 3.38 (m, 1 H), 3.81 (dd, *J* = 7, 3 Hz, 1 H), 3.88 (s, 2 H), 7.37 - 7.78 (m, 2 H), 7.96 (d, *J* = 8 Hz, 1 H), 8.13 (d, *J* = 11 Hz, 1 H), 8.61 (d, *J* = 8 Hz, 1 H), 8.77 (d, *J* = 2 Hz, 1 H), 9.24 (d, *J* = 2 Hz, 1 H). MS (ESI) *m/z* 464 (M+1).

## Example 104

**(S)-N-(2-Oxopyrro)idin-3-yl)-7-(trifluoromethoxy)quinoline-3-carboxamide**

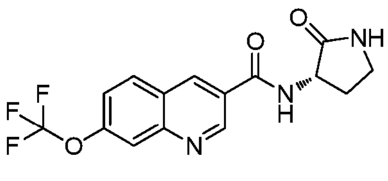

A solution of 7-(trifluoromethoxy)quinoline-3-carboxylic acid (Intermediate 16) (33 mg, 0.128 mmol) in DMF (2 mL) was treated with HATU (53.7 mg, 0.141 mmol) and then DIEA (0.067 mL, 0.385 mmol). The reaction mixture was stirred for 3 min prior to the addition of (S)-3-aminopyrrolidin-2-one (14.13 mg, 0.141 mmol). The reaction mixture was stirred at 21 °C for a further 3 hr prior to evaporation under a stream of nitrogen. The residue was purified by Mass-Directed Auto-Preparative HPLC (Waters SunFire C18 column, $CH_3CN$ / Water + 0.1% formic acid) to give the title compound (3.3 mg). $^1H$ NMR (400 MHz, $CD_3OD$) δ 2.24 (m, 1 H), 2.63 (m, 1 H), 3.47 (m, 3 H), 7.65 (dd, *J* = 9, 2 Hz, 1 H), 7.97 (s, 1 H), 8.22 (d, *J* = 9 Hz, 1 H), 8.46 (br s, 1 H), 8.89 (d, *J* = 2 Hz, 1 H), 9.36 (d, *J* = 2 Hz, 1 H); LCMS ES+ve *m/z* 340 $[M+H]^+$.

## Example 105

### 8-Ethyl-N-(thiazol-2-yl)quinoline-3-carboxamide

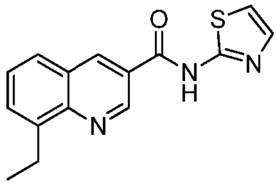

To a solution of 8-ethylquinoline-3-carboxylic acid (100 mg, 0.497 mmol) and HATU (189 mg, 0.497 mmol) in DMF (2 mL) was added DIEA (0.260 mL, 1.491 mmol) and the reaction mixture was stirred at 21 °C for 3 min. Thiazol-2-amine (49.8 mg, 0.497 mmol) was added and the reaction mixture stirred at 21 °C for 3 h. The reaction mixture was concentrated *in vacuo* and the residue was dissolved in DCM, loaded onto an aminopropyl ($NH_2$) ion-exchange SPE cartridge (5 g) and eluted with 1:1 MeOH/DCM. Eluent fractions were combined and evaporated *in vacuo.* The residue was purified by Mass-Directed Auto-Preparative HPLC (Waters SunFire C18 column, CH3CN / Water + 0.1% formic acid) to give the title compound (85 mg). $^1H$ NMR (400 MHz, $CDCl_3$) δ 1.45 (t, *J* = 7 Hz, 3 H), 3.39 (q, *J* = 7 Hz, 2 H), 7.03 (d, *J* = 4 Hz, 1 H), 7.19 (d, *J* = 4 Hz, 1 H), 7.62 (m, 1 H), 7.76 (dd, *J* = 7, 3 Hz, 2 H), 8.79 (d, *J* = 2 Hz, 1 H), 9.53 (d, *J* = 2 Hz, 1 H), 12.11 (br s, 1 H); LCMS ES+ve *m/z* 284 $[M+H]^+$.

## Example 106

### 6-Chloro-N-(1-(1-methyl-1H-tetrazol-5-yl)piperidin-4-yl)quinoline-3-carboxamide

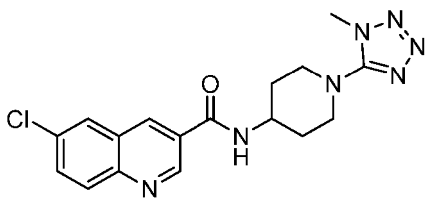

6-Chloroquinoline-3-carboxylic acid (100 mg, 0.482 mmol), HATU (201 mg, 0.530 mmol) and DIEA (0.252 mL, 1.445 mmol) were stirred in DMF (5 mL) at RT for 5 min. 1-(1-Methyl-1H-tetrazol-5-yl)piperidin-4-amine (Intermediate 39) (88 mg, 0.482 mmol) was added and the reaction mixture was stirred at RT for 15 h. The crude reaction mixture was purified by Mass-Directed Auto Preparative HPLC (Waters XBridge C18 column, $CH_3CN$ / Water + 0.1% formic acid) to yield the title compound as a white solid (84.7 mg, 47.3 % yield). $^1H$ NMR (400 MHz, $CD_3SOCD_3$) δ 1.79 (m, 2 H), 1.98 (dd, *J* = 13, 3 Hz, 2 H), 3.17 (m, 2 H), 3.68 (d, *J* = 13 Hz, 2 H), 3.90 (s, 3 H), 4.15 (m, 1 H), 7.87 (dd, *J* = 9, 2 Hz, 1 H), 8.11 (d, *J* = 9 Hz, 1 H), 8.24 (d, *J* = 2 Hz, 1 H), 8.76 (d, *J* = 7 Hz, 1 H), 8.81 (d, *J* = 2 Hz, 1 H), 9.30 (d, *J* = 2 Hz, 1 H); LCMS ES+ve *m/z* 372 $[M+H]^+$.

**Example 107**

**N-(1-(1-Methyl-1*H*-tetrazol-5-yl)piperidin-4-yl)-7-(trifluoromethyl)quinoline-3-carboxamide**

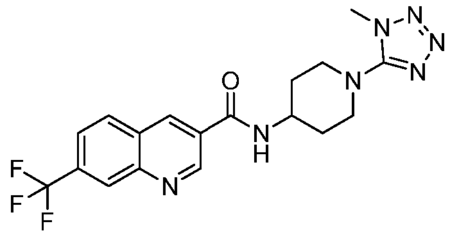

7-(Trifluoromethyl)quinoline-3-carboxylic acid (100 mg, 0.415 mmol, Intermediate 16), HATU (173 mg, 0.456 mmol), and DIEA (0.217 mL, 1.244 mmol) were stirred in DMF (5 mL) at RT for 5 min. 1-(1-Methyl-1*H*-tetrazol-5-yl)piperidin-4-amine hydrochloride (Intermediate 39) (76 mg, 0.415 mmol) was added and the reaction mixture was stirred at room temperature for 15 h. The crude reaction mixture was purified by Mass-Directed Auto-Preparative HPLC (Waters SunFire C18 column, $CH_3CN$ / Water + 0.1% formic acid) to yield the title compound (116.8 mg). $^1H$ NMR (400 MHz, $CD_3SOCD_3$) $\delta$ 1.80 (m, 2 H), 1.99 (dd, *J* = 13, 3 Hz, 2 H), 3.17 (m, 2 H), 3.69 (d, *J* = 13 Hz, 2 H), 3.91 (s, 3 H), 4.17 (m, 1 H), 7.97 (dd, *J* = 8, 2 Hz, 1 H), 8.37 (d, *J* = 8 Hz, 1 H), 8.44 (s, 1 H), 8.84 (d, *J* = 8 Hz, 1 H), 8.97 (d, *J* = 2 Hz, 1 H), 9.41 (d, *J* = 2 Hz, 1 H); LCMS ES+ve *m*/*z* 406 $[M+H]^+$.

**Example 108**

**7-Bromo-N-(1-(1-methyl-1*H*-tetrazol-5-yl)piperidin-4-yl)quinoline-3-carboxamide**

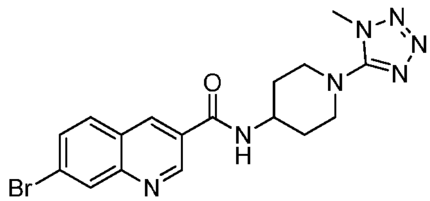

A mixture of 7-bromoquinoline-3-carboxylic acid (1.3 g, 5.16 mmol), 1-(1-methyl-1*H*-tetrazol-5-yl)piperidin-4-amine hydrochloride (Intermediate 39) (1.03 g, 5.67 mmol), and DIEA (2.7 mL, 15.47 mmol) in DMF (5 mL) was treated with HATU (2.16 g, 5.67 mmol) and the mixture was stirred at RT for 3 h. The mixture was evaporated to dryness and the residue was purified by column chromatography on silica using a 0-15 % MeOH in DCM gradient to afford the title compound (1.6 g). $^1H$ NMR (400 MHz, DMSO-d6) d ppm 1.78 (q, *J* = 12 Hz, 2 H), 1.97 (d, *J* = 11 Hz, 2 H), 3.17 (t, *J* = 12 Hz, 2 H), 3.68 (d, *J* = 13 Hz, 2 H), 3.91 (s, 3 H), 4.15 (brs, 1 H), 7.86 (d, *J* = 9 Hz, 1 H), 8.10 (d, *J* = 9 Hz, 1 H), 8.33 (s, 1 H), 8.78 (d, *J* = 8 Hz, 1 H), 8.87 (s, 1 H), 9.30 (s, 1 H); ES+ve *m*/*z* 416,418 $[M+H]^+$.

**Example 109**

**7-Chloro-N-(1-(1-methyl-1*H*-tetrazol-5-yl)piperidin-4-yl)quinoline-3-carboxamide**

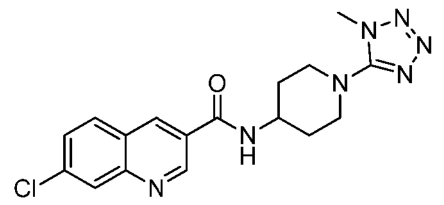

7-Chloroquinoline-3-carboxylic acid (100 mg, 0.482 mmol), HATU (201 mg, 0.530 mmol), and DIEA (0.252 mL, 1.445 mmol) were stirred in DMF (2 mL) at RT for 5 min. 1-(1-Methyl-1*H*-tetrazol-5-yl)piperidin-4-amine hydrochloride (Intermediate 39) (88 mg, 0.482 mmol) was added and the reaction mixture was stirred at RT for 15 h. The crude reaction mixture was purified by Mass-Directed Auto-Preparative HPLC (Waters SunFire C18 column, $CH_3CN$ / Water + 0.1 % formic acid) to yield the title compound (110.8 mg). $^1H$ NMR (400 MHz, $CD_3SOCD_3$) δ 1.79 (m, 2 H), 1.98 (d, *J* = 10 Hz, 2 H), 3.17 (t, *J* = 11 Hz, 2 H), 3.68 (d, *J* = 13 Hz, 2 H), 3.90 (s, 3 H), 4.15 (m, 1 H), 7.74 (dd, *J* = 9, 2 Hz, 1 H), 8.17 (m, 2 H), 8.75 (d, *J* = 8 Hz, 1 H), 8.88 (d, *J* = 2 Hz, 1 H), 9.31 (d, *J* = 2 Hz, 1 H); LCMS ES+ve *m/z* 372 $[M+H]^+$.

**Example 110**

**7-Ethyl-N-(1-(1-methyl-1*H*-tetrazol-5-yl)piperidin-4-yl)quinoline-3-carboxamide**

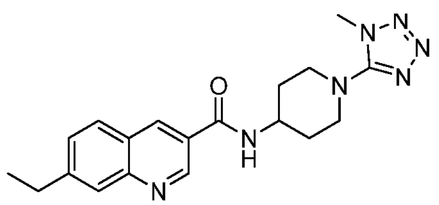

A mixture of 7-bromo-N-(1-(1-methyl-1*H*-tetrazol-5-yl)piperidin-4-yl)quinoline-3-carboxamide (Example 108) (90 mg, 0.216 mmol), ethylboronic acid (70 mg, 0.947 mmol), 1,1'-bis(diphenylphosphino)ferrocenepalladium(II) chloride (14 mg, 0.020 mmol), and potassium phosphate (125 mg, 0.59 mmol) in water (1 mL) was treated with DMF (3 mL) and the mixture was heated in a sealed vial by microwave at 120 °C for 40 min. The crude mixture was evaporated to dryness and the residue was purified by Mass-Directed Auto-Preparative HPLC (Waters SunFire C18 column, $CH_3CN$ / Water + 0.1 % formic acid) to afford the title compound (15 mg). $^1H$ NMR (400 MHz, $CD_3SOCD_3$) δ 1.31 (t, *J* = 8 Hz, 3 H), 1.66 - 1.87 (m, 2 H), 1.97 (m, 2 H), 2.87 (q, *J* = 8 Hz, 2 H), 3.16 (m, 2 H), 3.68 (d, *J* = 13 Hz, 2 H), 3.90 (s, 3 H), 4.15 (m, 1 H), 7.58 (dd, J = 8, 2 Hz, 1 H), 7.89 (s, 1 H), 8.01 (d, *J* = 8 Hz, 1 H), 8.68 (d, *J* = 8 Hz, 1 H), 8.78 (d, *J* = 2 Hz, 1 H), 9.25 (d, *J* = 2 Hz, 1 H). LCMS ES+ve *m/z* 366 $[M+H]^+$.

**Example 111**

**N-(1-(1-Methyl-1H-tetrazol-5-yl)piperidin-4-yl)-2,3-dihydro-[1,4]dioxino[2,3-g]quinoline-8-carboxamide**

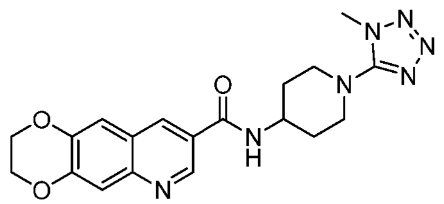

Lithium 2,3-dihydro-[1,4]dioxino[2,3-g]quinoline-8-carboxylate (Intermediate 17) (50 mg, 0.216 mmol), HATU (90 mg, 0.238 mmol), and DIEA (0.113 mL, 0.649 mmol) was stirred in DMF (2 mL) at RT for 5 min. 1-(1-methyl-1H-tetrazol-5- yl)piperidin-4-amine hydrochloride (Intermediate 39) (39.4 mg, 0.216 mmol) was added and the reaction mixture was stirred at RT for 2 h. The crude reaction mixture was purified by Mass-Directed Auto Preparative HPLC

(Waters XBridge C18 column, $CH_3CN$ / Water + 0.1% formic acid) to yield the title compound as a white solid (37.7 mg, 44.1 % yield). $^1H$ NMR (400 MHz, $CD_3SOCD_3$) δ 1.79 (m, 2 H), 1.97 (m, 2 H), 3.16 (m, 2 H), 3.68 (d, *J* = 13 Hz, 2 H), 3.90 (s, 3 H), 4.13 (m, 1 H), 4.46 (m, 4 H), 7.34 (d, *J* = 9 Hz, 1 H), 7.61 (d, *J* = 9 Hz, 1 H), 8.66 (d, *J* = 7 Hz, 1 H), 8.79 (d, *J* = 2 Hz, 1 H), 9.20 (d, *J* = 2 Hz, 1 H); LCMS ES+ve *m/z* 396 $[M+H]^+$.

**Example 112**

**N-(1-(1-)Methyl-1H-tetrazo)-5-yl)piperidin-4-yl)-2,3-dihydrofuro[3,2-h]quinoline-7-carboxamide**

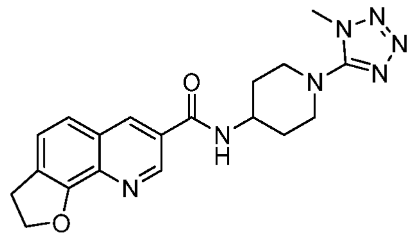

A solution of a mixture of 2,3-dihydrofuro[3,2-h]quinoline-7-carboxylic acid (Intermediate 18) (60 mg, 0.279mmol), 1-(1-methyl-1H-tetrazol-5-yl)piperidin-4-amine hydrochloride (Intermediate 39) (67.1 mg, 0.307 mmol), and DIEA (0.146 mL, 0.836 mmol) in DMF (5 mL) was treated with HATU (127 mg, 0.335 mmol) and the mixture was stirred at RT for 3 h. The mixture was evaporated to dryness and then the product was subjected to purification by Mass-Directed Auto-Preparative HPLC (Waters SunFire C18 column, $CH_3CN$ / Water + 0.1 % formic acid) to afford the title compound (44 mg, 41.6 % yield). $^1H$ NMR (400 MHz, $CD_3SOCD_3$) δ 1.79 (m, 2 H), 1.97 (m, 2 H), 3.17 (m, 2 H), 3.45 (m, 2 H), 3.68 (m, 2 H), 3.90 (s, 3 H), 4.13 (m, 1 H), 4.79 (t, *J* = 9 Hz, 2 H), 7.58 (dd, *J* = 16, 8 Hz, 2 H), 8.68 (d, *J* = 8 Hz, 1 H), 8.77 (d, *J* = 2 Hz, 1 H), 9.17 (d, *J* = 2 Hz, 1 H); LCMS ES+ve *m/z* 380 $[M+H]^+$.

**Example 113**

**N-(trans-4-(2-Hydroxypropan-2-yl)cyclohexyl)-2,3-dihydrofuro[3,2-h]quinoline-7-carboxamide**

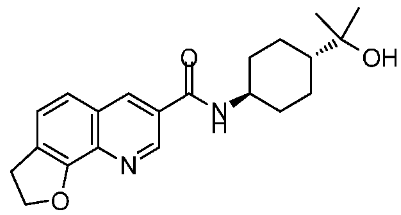

To a solution of 8,9-dihydrofuro[2,3-h]quinoline-3-carboxylic acid (Intermediate 18) (45 mg, 0.209 mmol) in DMF (5 mL) was added DIEA (0.110 mL, 0.627 mmol), HATU (119 mg, 0.314 mmol), and 2-(trans-4-aminocyclohexyl)propan-2-ol (36.2 mg, 0.230 mmol). After stirring at RT for 24 h, the solvent was removed under reduced pressure, the residue dissolved in 10% MeOH/DCM (50 mL) and absorbed onto Celite®. The crude material was then purified by reverse phase chromatography (10-50% acetonitrile/ water, with 0.2% $NH_4OH$) to afford the title compound (35 mg, 47% yield). $^1H$ NMR (400 MHz, $CD_3SOCD_3$) δ ppm 0.94 - 1.46 (m, 11 H), 1.72 - 1.90 (m, 2 H), 1.93 - 2.01 (m, 2 H), 3.44 (t, *J* = 9 Hz, 2 H), 3.72 - 3.83 (m, 1 H), 4.05 (s, 1 H), 4.78 (t, *J* = 9 Hz, 2 H), 7.57 (q, *J* = 8 Hz, 2 H), 8.52 (d, *J* = 8 Hz, 1 H), 8.73 (d, *J* = 2 Hz, 1 H), 9.14 (d, *J* = 2 Hz, 1 H). MS (ESI) *m/z* 355 (M+1).

**Example 114**

**6-Cyano-7-methoxy-N-(1-(1-methyl-1*H*-tetrazol-5-yl)piperidin-4-yl)quinoline-3-carboxamide**

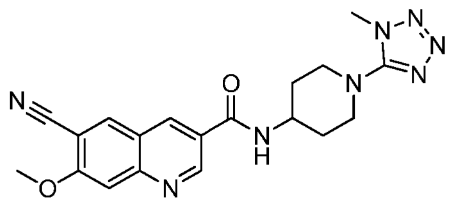

A mixture of 6-chloro-7-methoxy-N-(1-(1-methyl-1*H*-tetrazol-5-yl)piperidin-4-yl)quinoline-3-carboxamide (Example 32) (35.4 mg, 0.088 mmol), zinc (0.691 mg, 10.57 μmol), zinc cyanide (6.21 mg, 0.053 mmol), $Pd_2(dba)_3$ (1.013 mg, 1.762 μmol) and 1,1'-bis(diphenylphosphino)ferrocene (1.953 mg, 3.52 μmol) was heated by microwave in a sealed vial at 150 °C for 2 h. Additional quantities of zinc (0.691 mg, 10.57 μmol), zinc cyanide (6.21 mg, 0.053 mmol), $Pd_2(dba)_3$ (1.013 mg, 1.762 μmol), and 1,1'-bis(diphenylphosphino)ferrocene (1.953 mg, 3.52 μmol) were added and the reaction mixture was heated by microwave at 180 °C for a further 2 h. The reaction mixture was filtered and the filtrate was purified by Mass-Directed Auto-Preparative HPLC (Waters SunFire C18 column, $CH_3CN$ / Water + 0.1 % formic acid) to yield the title compound (6.4 mg). $^1H$ NMR (400 MHz, $CD_3SOCD_3$) δ 1.78 (m, 2 H), 1.97 (m, 2 H), 3.16 (m, 2 H), 3.68 (d, *J* = 13 Hz, 2 H), 3.90 (s, 3 H), 4.09 (s, 3 H), 4.08 - 4.18 (m, 1H), 7.69 (s, 1 H), 8.72 (s, 1 H), 8.75 (d, *J* = 7 Hz, 1 H), 8.84 (d, *J* = 2 Hz, 1 H), 9.36 (d, *J* = 2 Hz, 1 H); LCMS ES+ve *m/z* 393 $[M+H]^+$.

## Example 115

### 7-Isopropoxy-N-(1-(1-methyl-1*H*-tetrazol-5-yl)piperidin-4-yl)quinoline-3-carboxamide

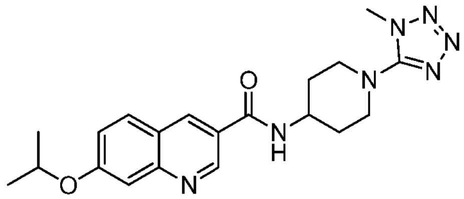

### A. 7-Hydroxy-N-(1-(1-methyl-1*H*-tetrazol-5-yl)piperidin-4-yl)quinoline-3-carboxamide

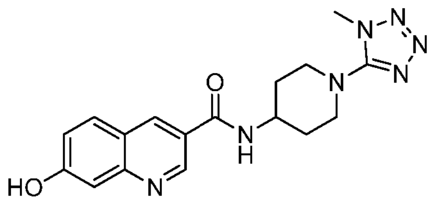

To a solution of 7-hydroxyquinoline-3-carboxylic acid (Intermediate 19) (40 mg, 0.211 mmol) in DMF (2 mL) was added HATU (88 mg, 0.233 mmol) and DIEA (82 mg, 0.634 mmol). The reaction mixture was stirred at RT for 5 min, then 1-(1-methyl-1*H*-tetrazol-5-yl)piperidin-4-amine hydrochloride (Intermediate 39) (38.5 mg, 0.211 mmol) was added. The reaction mixture was stirred at RT for 1 h. The reaction mixture was purified by Mass-Directed Auto-Preparative HPLC (Waters SunFire C18 column, $CH_3CN$ / Water + 0.1% formic acid) to yield the title compound (7.6 mg). $^1H$ NMR (400 MHz, $CD_3SOCD_3$) δ 1.78 (m, 2 H), 1.96 (m, 2 H), 3.15 (m, 2 H), 3.68 (d, *J* = 13 Hz, 2 H), 3.90 (s, 3 H), 4.12 (m, 1 H), 7.24 (dd, *J* = 9, 2 Hz, 1 H), 7.29 (d, *J* = 2 Hz, 1 H), 7.92 (d, *J* = 9 Hz, 1 H), 8.30 (br s, 1H), 8.56 (d, *J* = 7 Hz, 1 H), 8.68 (d, *J* = 2 Hz, 1 H), 9.15 (d, *J* = 2 Hz, 1 H); LCMS ES+ve *m/z* 354 $[M+H]^+$.

### B. 7-Isopropoxy-N-(1-(1-methyl-1*H*-tetrazol-5-yl)piperidin-4-yl)quinoline-3-carboxamide

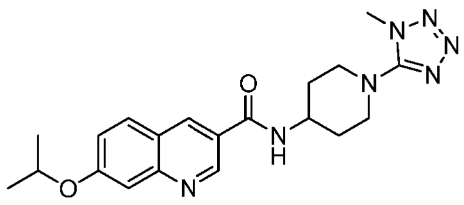

7-Hydroxy-N-(1-(1-methyl-1H-tetrazol-5-yl)piperidin-4-yl)quinoline-3-carboxamide (20 mg, 0.057 mmol), sodium hydride (2.264 mg, 0.057 mmol), and 2-iodopropane (19.24 mg, 0.113 mmol) were stirred in DMF (2 mL) at RT for 2 h. The crude reaction mixture was purified by Mass-Directed Auto-Preparative HPLC (Waters SunFire C18 column, $CH_3CN$ / Water + 0.1% formic acid) to yield the title compound (4.0 mg). $^1H$ NMR (400 MHz, $CD_3SOCD_3$) δ 1.38 (d, *J* = 6 Hz, 6 H), 1.78 (m, 2 H), 1.96 (m, 2 H), 3.15 (m, 2 H), 3.68 (m, 2 H), 3.90 (s, 3 H), 4.13 (m, br, 1 H), 4.89 (quin, *J* = 6 Hz, 1 H), 7.29 (dd, *J* = 9, 2 Hz, 1 H), 7.44 (s, 1 H), 7.98 (d, *J* = 9 Hz, 1 H), 8.61 (d, *J* = 7 Hz, 1 H), 8.73 (s, 1 H), 9.21 (d, *J* = 2 Hz, 1 H); LCMS ES+ve *m/z* 396 $[M+H]^+$.

**Example 116**

**(6-Chloro-7-(difluoromethoxy)-N-(trans-4-(2-hydroxypropan-2-yl)cyclohexyl)quinoline-3-carboxamide, trifluoroacetate**

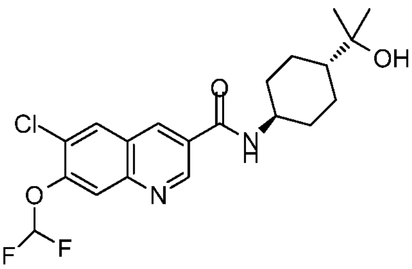

To a suspension of 6-chloro-7-(difluoromethoxy)quinoline-3-carboxylic acid (Intermediate 20) (50 mg, 0.183 mmol) in DMF (20 mL) and THF (20 mL) at RT, was added EDC (105 mg, 0.548 mmol), HOBT (84 mg, 0.548 mmol), and 2-(trans-4-aminocyclohexyl)propan-2-ol (34.5 mg, 0.219 mmol). After stirring at RT for 2 h, the solvent was removed under reduced pressure, the residue dissolved in 10% MeOH/DCM (50 mL), and absorbed onto Celite®. The crude material was then purified by reverse phase chromatography (10-50% acetonitrile, (with 0.1 % TFA)/ water, with (0.1% TFA)) to provide the title compound (60 mg, 80% yield). $^1H$ NMR (400 MHz, $CD_3SOCD_3$) δ ppm 0.99 - 1.23 (m, 9 H), 1.27-1.36 (m, 2 H), 1.84 (d, *J* = 12 Hz, 2 H), 1.94 (d, *J* = 10 Hz, 2 H), 3.62 - 3.85 (m, 1 H), 7.60 (t, *J* = 72 Hz, 1 H), 7.93 (s, 1 H), 8.43 (s, 1 H), 8.60 (d, *J* = 8 Hz, 1 H), 8.77 (d, *J* = 2 Hz, 1 H), 9.28 (d, *J* = 2 Hz, 1 H). MS (ESI) *m/z* 413(M+1).

**Example 117**

**(S)-6-Chloro-7-(difluoromethoxy)-N-(2-oxopyrrolidin-3-y)quinoline-3-carboxamide, trifluoroacetate**

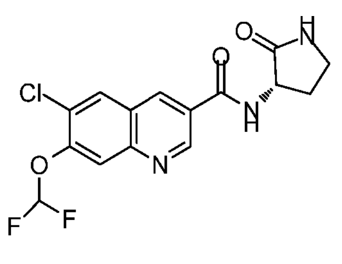

To a solution of 6-chloro-7-(difluoromethoxy)quinoline-3-carboxylic acid (Intermediate 20) (0.5 g, 1.827 mmol)

in DMF (50 mL) was added HATU (1.042 g, 2.74 mmol), DIEA (0.957 mL, 5.48 mmol), and (S)-3-aminopyrrolidin-2-one (0.220 g, 2.193 mmol). After stirring at RT for 24 h, the solvent was removed under reduced pressure, the residue dissolved in 10 % MeOH/DCM and absorbed onto Celite®. The crude reaction was then purified by reverse phase chromatography (10-50% acetonitrile( with 0.1% TFA)/ water (with 0.1% TFA)) to provide the title compound (550 mg, 64% yield). $^1$H NMR (400 MHz, $CD_3SOCD_3$) δ ppm 1.93 - 2.18 (m, 1 H), 2.38-2.43 (m, 1 H), 3.17 - 3.34 (m, 2 H), 4.60-4.64 (m, 1 H), 7.63 (t, *J* = 72 Hz, 1 H), 7.92 - 8.03 (m, 2 H), 8.48 (s, 1 H), 8.84 (d, *J* = 2 Hz, 1 H), 9.12 (d, *J* = 8 Hz, 1 H), 9.32 (d, *J* = 2 Hz, 1 H). MS (ESI) *m/z* 356(M+1).

**Example 118**

**6-Chloro-7-(difluoromethoxy)-N-(trans-3-(2-hydroxypropan-2-yl)cyclobutyl)quinoline-3-carboxamide**

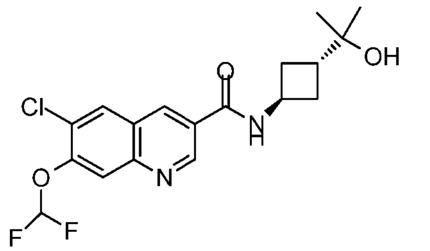

To a solution of 6-chloro-7-(difluoromethoxy)quinoline-3-carboxylic acid (Intermediate 20) (250 mg, 0.914 mmol) in DMF (20 mL) and THF (20 mL) at RT was added EDC (525 mg, 2.74 mmol), HOBt (370 mg, 2.74 mmol), and 2-(-3-aminocyclobutyl)propan-2-ol (Intermediate 41) (177 mg, 1.371 mmol). After stirring at RT for 24 h, the solvent was removed under reduced pressure, the residue was dissolved in 10% MeOH/DCM, absorbed onto silica gel, and purified by silica gel chromatography (10-75% EtOAc/hexanes) to provide the title compound (215 mg, 61 % yield). $^1$H NMR (400 MHz, $CD_3SOCD_3$) δ ppm 0.96 - 1.09 (m, 6 H), 1.98 - 2.15 (m, 2 H), 2.20 - 2.38 (m, 3 H), 4.24 (s, 1 H), 4.32 - 4.46 (m, 1 H), 7.60 (t, *J* = 72 Hz, 1 H), 7.93 (s, 1 H), 8.43 (s, 1 H), 8.79 (d, *J* = 2 Hz, 1 H), 8.99 (d, *J* = 7 Hz, 1 H), 9.30 (d, *J* = 2 Hz, 1 H). MS (ESI) *m/z* 385(M+1).

**Example 119**

**(S)-6-Chloro-7-(difluoromethoxy)-N-(1-isopropyl-2-oxopyrrolidin-3-yl)quinoline-3-carboxamide**

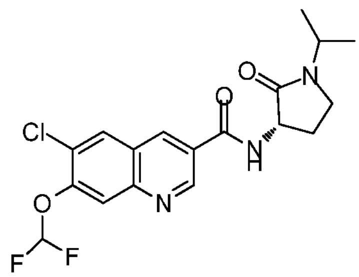

To a suspension of (S)-6-chloro-7-(difluoromethoxy)-N-(2-oxopyrrolidin-3-yl)quinoline-3-carboxamide trifluoroacetate (Example 117) (60 mg, 0.169 mmol) in DMF (50 mL) at 0 °C was added sodium hydride (27.0 mg, 0.675 mmol). After stirring at RT for 20 min, 2-iodopropane (0.337 mL, 0.337 mmol) was added and the resulting reaction was stirred at RT for 72 h. The solution was diluted with EtOAc and quenched with saturated $NH_4Cl$ (25 mL). The aqueous layer was extracted with EtOAc (3x20 mL), the organic layers combined, and absorbed onto silica gel. The crude material was then purified by reverse phase chromatography (10-50% acetonitrile (with 0.1% TFA)/ water (with 0.1% TFA)) to provide the title compound (40 mg, 46% yield). $^1$H NMR (400 MHz, $CD_3SOCD_3$) δ ppm 1.08 (d, *J* = 7 Hz, 3 H), 1.12 (d, *J* = 7 Hz, 3 H), 1.84 - 2.00 (m, 1 H), 2.30 - 2.42 (m, 1 H), 3.23-3.26 (m, 1 H), 3.36-3.39 (m, 1 H), 4.13-4.19 (m, 1 H), 4.65-4.72 (m, 1 H), 7.61 (t, *J* = 72 Hz, 1 H), 7.94 (s, 1 H), 8.45 (s, 1 H), 8.83 (d, *J* = 2 Hz, 1 H), 9.13 (d, *J* = 8 Hz, 1 H), 9.31 (d, *J* = 2 Hz, 1 H). MS (ESI) *m/z* 398(M+1).

**Example 120**

**(S)-6-Chloro-N-(1-(cyclopropylmethyl)-2-oxopyrrolidin-3-yl)-7-(difluoromethoxy)quinoline-3-carboxamide**

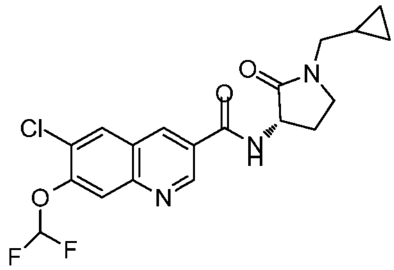

To a suspension of (S)-6-chloro-7-(difluoromethoxy)-N-(2-oxopyrrolidin-3-yl)quinoline-3-carboxamide trifluoroacetate (Example 117) (50 mg, 0.141 mmol) in DMF (50 mL) at 0 °C was added sodium hydride (16.87 mg, 0.422 mmol). After stirring at RT for 20 min, (bromomethyl)cyclopropane (0.169 mL, 0.169 mmol) was added and the resulting reaction was stirred at RT for 24 h. The solution was diluted with EtOAc and quenched with saturated $NH_4Cl$ (25 mL). The aqueous layer was extracted with EtOAc (3x20 mL), the organic layers combined, and absorbed onto silica gel. The crude material was then purified by silica gel chromatography (DCM to 10% MeOH/DCM) to provide the title compound (16 mg, 28% yield). $^1H$ NMR (400 MHz, $CD_3SOCD_3$) δ ppm 0.19-0.22 (m, 2 H) 0.43 - 0.57 (m, 2 H) 0.84 - 1.01 (m, 1 H) 1.87 - 2.09 (m, 1 H) 2.33 - 2.46 (m, 1 H) 2.99 - 3.08 (m, 1 H) 3.11 - 3.20 (m, 1 H) 3.40 - 3.53 (m, 2 H) 4.65 - 4.79 (m, 1 H) 7.61 (t, *J* = 72 Hz, 1 H) 7.94 (s, 1 H) 8.45 (s, 1 H) 8.83 (d, *J* = 2 Hz, 1 H) 9.15 (d, *J* = 8 Hz, 1 H) 9.31 (d, *J* = 2 Hz, 1 H) MS (ESI) *m/z* 410(M+1).

**Example 121**

**6-Chloro-7-(difluoromethoxy)-N-(trans-4-(1-hydroxycyclopropyl)cyclohexyl)quinoline-3-carboxamide**

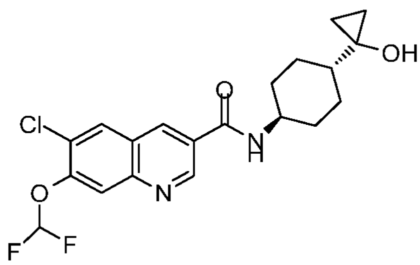

To a solution of 6-chloro-7-(difluoromethoxy)quinoline-3-carboxylic acid (Intermediate 20) (60 mg, 0.268 mmol) in DMF (20 mL) and THF (20 mL) at RT was added HOBt (123 mg, 0.805 mmol), EDC (154 mg, 0.805 mmol), and 1-(trans-4-aminocyclohexyl)cyclopropanol (Intermediate 46) (48.0 mg, 0.322 mmol). After stirring at RT for 24 h, the solvent was removed under reduced pressure, the residue was dissolved in 10% MeOH/DCM, washed with saturated $NaHCO_3$ (20 mL), absorbed to silica gel, and purified by silica gel chromatography (10-75% EtOAc/hexanes) to provide the title compound (40 mg, 37% yield). $^1H$ NMR (400 MHz, $CD_3SOCD_3$) δ ppm 0.29 - 0.41 (m, 2 H), 0.45 - 0.54 (m, 2 H), 0.85 - 1.00 (m, 1 H), 1.24 - 1.48 (m, 4 H), 1.62 - 1.82 (m, 2 H), 1.92-1.94 (m, 2 H), 3.3-3.76 (m, 1 H), 4.87 (s, 1 H), 7.60 (t, *J* = 72 Hz, 1 H), 7.92 (s, 1 H), 8.43 (s, 1 H), 8.61 (d, *J* = 8 Hz, 1 H), 8.77 (d, *J* = 2 Hz, 1 H), 9.27 (d, *J* = 2 Hz, 1 H). MS (ESI) *m/z* 411 (M+1).

**Example 122**

**6-Chloro-7-(difluoromethoxy)-N-(trans-4-(2-hydroxy-2-methylpropoxy)cyclohexyl)quinoline-3-carboxamide**

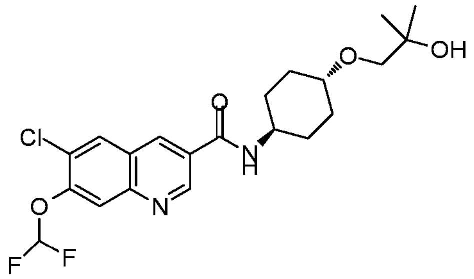

To a solution of 6-chloro-7-(difluoromethoxy)quinoline-3-carboxylic acid (Intermediate 20) (50 mg, 0.183 mmol) in DMF (10 mL) and THF (10 mL) at RT, was added EDC (105 mg, 0.548 mmol), HOBt (84 mg, 0.548 mmol), and 1-((trans-4-aminocyclohexyl)oxy)-2-methylpropan-2-ol (Intermediate 42) (51.3 mg, 0.274 mmol). After stirring at RT for 24 h, the solvent was removed under reduced pressure, the residue was dissolved in 10% MeOH/DCM (50 mL), and absorbed onto Celite®. The crude material was then purified by reverse phase chromatography (10-50% acetonitrile (with 0.1 % TFA)/ water (with 0.1 % TFA)) to provide the title compound (65 mg, 64% yield). $^1H$ NMR (400 MHz, $CD_3SOCD_3$) δ ppm 1.05 (s, 6 H), 1.20 - 1.46 (m, 4 H), 1.85 - 1.95 (m, 2 H), 2.01 (d, *J* = 9 Hz, 2 H), 3.16 (s, 2 H), 3.23 - 3.34 (m, 1 H), 3.74 - 3.91 (m, 1 H), 7.60 (t, *J* = 73 Hz, 1 H), 7.93 (s, 1 H), 8.43 (s, 1 H), 8.61 (s, 1 H), 8.77 (d, *J* = 2 Hz, 1 H), 9.27 (d, *J* = 2 Hz, 1 H). MS (ESI) *m*/*z* 443 (M+1).

**Example 123**

**6-Chloro-7-(difluoromethoxy)-N-(trans-4-((1-hydroxycyclopropyl)methoxy)cyclohexyl)quinoline-3-carboxamide**

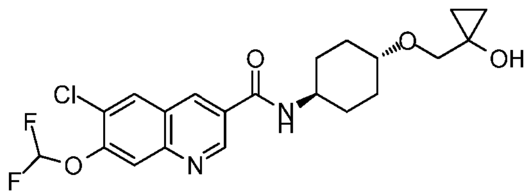

To a solution of 6-chloro-7-(difluoromethoxy)quinoline-3-carboxylic acid (Intermediate 20) (75 mg, 0.27 mmol) in DMF (4 mL) was added DIEA (0.14 mL, 0.82 mmol) and HATU (125 mg, 0.33 mmol). After stirring for 5 min 1-(((trans-4-aminocyclohexyl)oxy)methyl)cyclopropanol hydrochloride (intermediate 74) (66.9 mg, 0.302 mmol) was added. The reaction was stirred at RT for 2.5 h, quenched into water and extracted with EtOAc. The combined organics were dried over sodium sulfate, filtered, and concentrated. The residue was purified by silica gel chromatography (0-65% (3:1 EtOAc:EtOH)-hexanes gradient) to afford the title compound as a white solid (68 mg, 56% yield). $^1H$ NMR (400 MHz, $CD_3SOCD_3$) δ ppm 0.37 - 0.58 (m, 4 H), 1.19 - 1.30 (m, 2 H), 1.31 - 1.43 (m, 2 H), 1.89 (d, *J* = 11 Hz, 2 H), 2.02 (d, *J* = 10 Hz, 2 H), 3.40 (s, 2 H), 3.73 - 3.89 (m, 1 H), 5.22 (s, 1 H), 7.59 (t, *J* = 72, 1 H), 7.91 (s, 1 H), 8.42 (s, 1 H), 8.59 (d, *J* = 8 Hz, 1 H), 8.75 (s, 1 H), 9.25 (s, 1 H). MS (ESI) *m*/*z* 441 (M+1).

**Example 124**

**Racemic 6-chloro-7-(difluoromethoxy)-N-((3*r*,6*s*)-6-(2-hydroxypropan-2-yl)tetrahydro-2H-pyran-3-yl)quinoline-3-carboxamide**

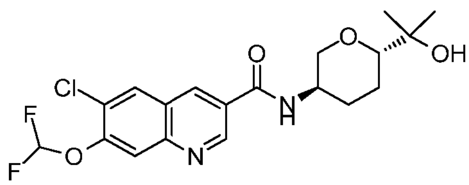

To a solution of 6-chloro-7-(difluoromethoxy)quinoline-3-carboxylic acid (intermediate 20) (90 mg, 0.33 mmol) in DMF (4 mL) was added DIEA (0.17 mL, 0.94 mmol) and HATU (150 mg, 0.4 mmol). After stirring for 5 min racemic 2-((2S,5R)-5-aminotetrahydro-2H-pyran-2-yl)propan-2-ol hydrochloride (intermediate 49) (55 mg, 0.35 mmol) was added as a solution in DMF (1 mL). The reaction was stirred at RT for 2.5 h, quenched into water and extracted with EtOAc. The combined organics were dried over sodium sulfate, filtered, and concentrated. The residue was purified by silica gel chromatography (0-80% (3:1 EtOAc:EtOH)-hexanes gradient) to afford the title compound as a white solid (94 mg, 69% yield). $^1$H NMR (400 MHz, $CD_3SOCD_3$) δ ppm 1.03 (s, 3 H), 1.08 (s, 3 H), 1.31 - 1.46 (m, 1 H), 1.58 (qd, *J* = 12, 4 Hz, 1 H), 1.80 (d, *J* = 13 Hz, 1 H), 2.01 (d, *J* = 12 Hz, 1 H), 2.94 - 3.02 (m, 1 H), 3.14 (t, *J* = 10 Hz, 1 H), 3.83 - 4.02 (m, 2 H), 4.24 (s, 1 H), 7.60 (t, *J* = 73 Hz, 1 H), 7.92 (s, 1 H), 8.43 (s, 1 H), 8.60 (d, *J* = 8 Hz, 1 H), 8.78 (d, *J* = 2 Hz, 1 H), 9.27 (d, *J* = 2 Hz, 1 H). MS (ESI) *m/z* 415, 417 (M+1).

## Example 125

**6-Chloro-7-(difluoromethoxy)-N-(*trans*-4-((1,1-difluoropropan-2-yl)amino)cyclohexyl)quinoline-3-carboxamide**

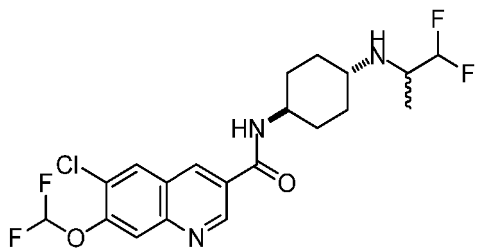

DIEA (0.524 mL, 3.00 mmol) was added to 6-chloro-7-(difluoromethoxy)quinoline-3-carboxylic acid (Intermediate 20) (0.1641 g, 0.600 mmol) in DMF (1.475 mL) at RT. Then, HATU (0.296 g, 0.780 mmol) was added and the reaction mixture was stirred for 5 min. Then, *trans*-N1-(1,1-difluoropropan-2-yl)cyclohexane-1,4-diamine (Intermediate 61) (0.121 g, 0.630 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc (1:49) to give the title compound (0.1719 g, 60.8 % yield). $^1$H NMR (400 MHz, $CD_3SOCD_3$) δ 1.02 (d, *J* = 7 Hz, 3 H), 1.11 (dq, *J* = 13, 3 Hz, 2 H), 1.34 (dq, *J* = 11, 4 Hz, 2 H), 1.48 (br t, *J* = 6 Hz, 1 H), 1.84-1.98 (m, 4 H), 2.42-2.56 (m, 1 H), 2.92-3.06 (m, 1 H), 3.72-3.82 (m, 1 H), 5.77 (dt, *J* = 57, 4 Hz, 1 H), 7.62 (t, *J* = 73 Hz, 1 H), 7.93 (s, 1 H), 8.44 (s, 1 H), 8.62 (d, *J* = 8 Hz, 1 H), 8.77 (d, *J* = 2 Hz, 1 H), 9.27 (d, *J* = 2 Hz, 1 H); LC-MS (LC-ES) M+H = 448.

## Example 126

**Racemic 6-Chloro-7-(difluoromethoxy)-N-(3*r*,6*s*)-6-(dimethylcarbamoyl)tetrahydro-2H-pyran-3-yl)quinoline-3-carboxamide**

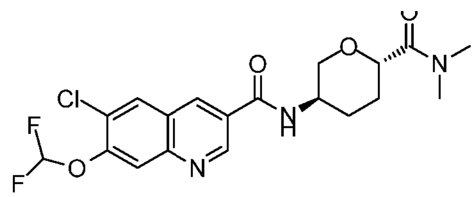

To a solution of 6-chloro-7-(difluoromethoxy)quinoline-3-carboxylic acid (intermediate 20) (70 mg, 0.27 mmol) in DMF (4 mL) was added DIEA (0.13 mL, 0.77 mmol) and HATU (117 mg, 0.31 mmol). After stirring for 5 min, 5-amino-N,N-dimethyltetrahydro-2H-pyran-2-carboxamide hydrochloride (intermediate 75) (53.4 mg, 0.256 mmol) (racemic, mixture of cis and trans isomers) was added as a solution in DMF (1 mL) and DIEA (0.03 mL). The reaction was stirred at RT overnight, quenched into water and extracted with EtOAc. The combined organics were dried over sodium sulfate, filtered, and concentrated. The residue was purified by silica gel chromatography (0-10% (3:1 MeOH-DCM gradient) to

afford the title compound as a white solid (56 mg, 51 % yield). $^1$H NMR (400 MHz, $CD_3SOCD_3$) δ ppm 1.19 - 1.26 (m, 1 H), 1.59 - 1.80 (m, 3 H), 2.04 (d, *J* = 11 Hz, 1 H), 2.80 (s, 3 H), 3.01 (s, 3 H), 3.88 - 4.02 (m, 2 H), 4.11 (t, *J* = 6 Hz, 1 H), 7.60 (t, *J* = 73 Hz, 1 H), 7.92 (s, 1 H), 8.43 (s, 1 H), 8.65 (d, *J* = 7 Hz, 1 H), 8.77 (d, *J* = 2 Hz, 1 H), 9.27 (d, *J* = 2 Hz, 1 H). MS (ESI) *m*/*z* 428 (M+1).

**Example 127**

**6-Chloro-7-(difluoromethoxy)-N-(trans-4-(2-hydroxy-3-methylbutoxy)cyclohexyl)quinoline-3-carboxamide**

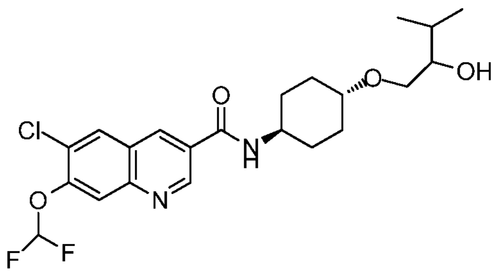

To a solution of 1-((trans-4-aminocyclohexyl)oxy)-3-methylbutan-2-ol (Intermediate 66) (50 mg, 0.248 mmol) in DMF (5 mL) was added 6-chloro-7-(difluoromethoxy)quinoline-3-carboxylic acid (Intermediate 20) (40 mg, 0.146 mmol), followed by HATU (83 mg, 0.219 mmol) and DIEA (0.077 mL, 0.439 mmol). The reaction was allowed to stir at RT for 24 h. The solvent was removed under reduced pressure, the residue was absorbed onto Celite® and purified by reverse phase chromatography (10-50% acetonitrile( with 0.1 % TFA)/ water (with 0.1 % TFA)) to afford the title compound (55 mg, 66% yield). $^1$H NMR (400 MHz, $CD_3SOCD_3$) δ ppm 0.85 (dd, *J* = 13, 7 Hz, 6 H), 1.14 - 1.51 (m, 5 H), 1.65-1.69 (m, 1 H), 1.91-1.94 (m, 2 H), 2.03-2.04 (m, 2 H), 3.14 - 3.51 (m, 4 H), 3.72 - 3.95 (m, 1 H), 7.63 (t, *J* = 73 Hz, 1 H), 7.95 (s, 1 H), 8.46 (s, 1 H), 8.63 (d, *J* = 8 Hz, 1 H), 8.79 (d, *J* = 2 Hz, 1 H), 9.29 (d, *J* = 2 Hz, 1 H). MS (ESI) *m*/*z* 457 (M+1).

**Example 128**

**6-Chloro-N-(3-cyclopropyl-1-methyl-1H-pyrazol-5-yl)-7-(difluoromethoxy)quinoline-3-carboxamide**

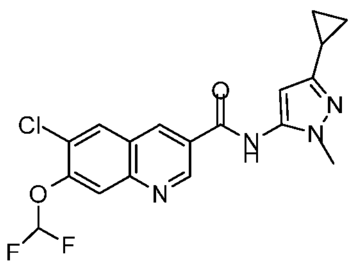

To a solution of 6-chloro-7-(difluoromethoxy)quinoline-3-carboxylic acid (Intermediate 20) (50 mg, 0.183 mmol) in DMF (10 mL) at RT, was added 3-cyclopropyl-1-methyl-1H-pyrazol-5-amine (27.6 mg, 0.201 mmol), followed by HATU (104 mg, 0.274 mmol) and DIEA (0.096 mL, 0.548 mmol). After stirring at RT for 24 h, the solvent was removed under reduced pressure, the residue was dissolved in 10% MeOH/DCM (50 mL), and absorbed onto Celite®. The crude material was then purified by reverse phase chromatography (10-50% acetonitrile/ water (with 0.2% $NH_4OH$)) to afford the title compound (40 mg, 56% yield). $^1$H NMR (400 MHz, $CD_3SOCD_3$) δ ppm 0.59 - 0.73 (m, 2 H), 0.80 - 0.92 (m, 2 H), 1.75 - 1.89 (m, 1 H), 3.33 (s, 3 H), 6.03 (s, 1 H), 7.66 (t, *J* = 73 Hz, 1 H), 7.99 (s, 1 H), 8.52 (s, 1 H), 8.95 (d, *J* = 2 Hz, 1 H), 9.39 (d, *J* = 2 Hz, 1 H), 10.67 (br s, 1 H). MS (ESI) *m*/*z* 393(M+1).

**Example 129 and 130**

**6-Chloro-7-(difluoromethoxy)-N-(*trans*-4-(((R)-1,1-difluoropropan-2 yl)amino)cyclohexyl)quinoline-3-carboxamide and 6-Chloro-7-(difluoromethoxy)-N-(*trans*-4-(((S)-1,1-difluoropropan-2-yl)amino)cyclohexyl)quinoline-3-carboxamide**

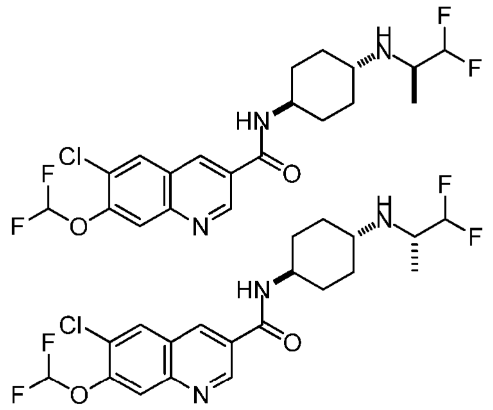

Racemic 6-chloro-7-(difluoromethoxy)-N-(trans-4-((1,1-difluoropropan-2-yl)amino)cyclohexyl)quinoline-3-carboxamide (Example 125) (0.1591 g, 0.355 mmol) was separated into its enantiomers via super critical fluid chromatography on a chiral Phenomenex Cell2 column eluting with MeOH:carbon dioxide (2:9) to give 6-chloro-7-(difluoromethoxy)-N-(*trans*-4-(((R)-1,1-difluoropropan-2-yl)amino)cyclohexyl)quinoline-3-carboxamide (0.0416 g, 24.8 % yield) as the first enantiomer to elute and 6-chloro-7-(difluoromethoxy)-N-(*trans*-4-(((S)-1,1-difluoropropan-2-yl)amino)cyclohexyl)quinoline-3-carboxamide (0.0501 g, 29.9 % yield) as the last enantiomer to elute. The structures were assigned by vibrational circular dichroism.

**6-Chloro-7-(difluoromethoxy)-N-(*trans*-4-(((R)-1, 1-difluoropropan-2-yl)amino)cyclohexyl)quinoline-3-carboxamide:** $^1$H NMR (400 MHz, $CD_3SOCD_3$) δ 1.03 (d, J = 7 Hz, 3 H), 1.14 (dq, *J* = 13, 3 Hz, 2 H), 1.37 (dq, *J* = 11, 4 Hz, 2 H), 1.48 (br t, *J* =6 Hz, 1 H), 1.84-1.98 (m, 4 H), 2.42-2.56 (m, 1 H), 2.92-3.06 (m, 1 H), 3.70-3.84 (m, 1 H), 5.77 (dt, *J* = 57, 4 Hz, 1 H), 7.61 (t, *J* = 73 Hz, 1 H), 7.93 (s, 1 H), 8.44 (s, 1 H), 8.61 (d, *J* = 8 Hz, 1 H), 8.77 (d, *J* = 2 Hz, 1 H), 9.27 (d, *J* = 2 Hz, 1 H); LC-MS (LC-ES) M+H = 448.

**6-Chloro-7-(difluoromethoxy)-N-(*trans*-4-(((S)-1, 1-difluoropropan-2-yl)amino)cyclohexyl)quinoline-3-carboxamide:** $^1$H NMR (400 MHz, $CD_3SOCD_3$) δ 1.03 (d, J = 7 Hz, 3 H), 1.14 (dq, *J* = 13, 3 Hz, 2 H), 1.37 (dq, *J* = 11, 4 Hz, 2 H), 1.48 (br t, *J* = 6 Hz, 1 H), 1.84-1.98 (m, 4 H), 2.42-2.56 (m, 1 H), 2.92-3.06 (m, 1 H), 3.70-3.84 (m, 1 H), 5.77 (dt, *J* = 57, 4 Hz, 1 H), 7.61 (t, *J* = 73 Hz, 1 H), 7.93 (s, 1 H), 8.44 (s, 1 H), 8.61 (d, *J* = 8 Hz, 1 H), 8.77 (d, *J* = 2 Hz, 1 H), 9.27 (d, *J* = 2 Hz, 1 H); LC-MS (LC-ES) M+H = 448.

**Example 131**

**6-Chloro-7-(difluoromethoxy)-N-(*trans*-4-(((S)-1,1,1-trifluoro-3-hydroxypropan-2-yl)amino)cyclohexyl)quinoline-3-carboxamide**

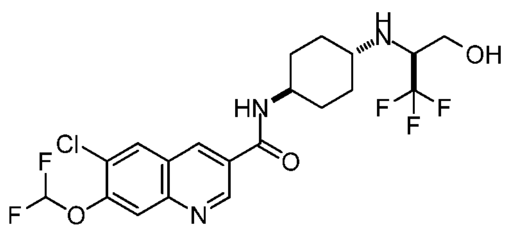

DIEA (0.499 mL, 2.86 mmol) was added to 6-chloro-7-(difluoromethoxy)quinoline-3-carboxylic acid (Intermediate 20) (0.1564 g, 0.572 mmol) in DMF (1.406 mL) at RT. Then, HATU (0.283 g, 0.743 mmol) was added and the

reaction mixture was stirred for 5 min. Then, (S)-2-((*trans*-4-aminocyclohexyl)amino)-3,3,3-trifluoropropan-1-ol (Intermediate 67) (0.136 g, 0.600 mmol) was added and the reaction mixture was stirred for 2 h. The reaction mixture was concentrated and the residue was purified by RP HPLC eluting with acetonitrile:water with 0.1% ammonium hydroxide (5:95-100:0), then further purified by silica gel chromatography, eluting with EtOAc:hexanes (9:1) to give the title compound (0.1125 g, 38.8 % yield). $^1$H NMR (400 MHz, $CD_3SOCD_3$) δ 1.13 (q, *J* = 11 Hz, 2 H), 1.37 (dq, *J* = 13, 3 Hz, 2 H), 1.82-2.00 (m, 5 H), 2.46-2.58 (m, 1 H), 3.22-3.34 (m, 1 H), 3.44-3.54 (m, 1 H), 3.58-3.68 (m, 1 H), 3.72-3.86 (m, 1 H), 4.98 (t, *J* = 6 Hz, 1 H), 7.61 (t, *J* = 73 Hz, 1 H), 7.93 (s, 1 H), 8.44 (s, 1 H), 8.60 (d, *J* = 8 Hz, 1 H), 8.77 (d, *J* = 2 Hz, 1 H), 9.27 (d, *J* = 2 Hz, 1 H); LC-MS (LC-ES) M+H = 482.

## Example 132

### 6-Chloro-7-(difluoromethoxy)-N-(*trans*-4-13-fluoroazetidin-1-yl)cyclohexyl)quinoline-3-carboxamide

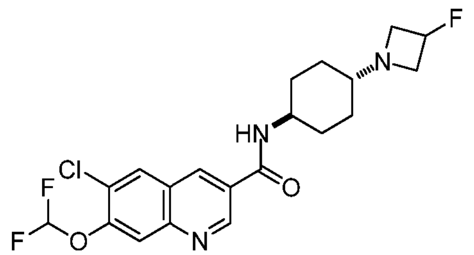

DIEA (0.330 mL, 1.891 mmol) was added to 6-chloro-7-(difluoromethoxy)quinoline-3-carboxylic acid (Intermediate 20) (0.1035 g, 0.378 mmol) in DMF (0.931 mL) at RT. Then, HATU (0.187 g, 0.492 mmol) was added and the reaction mixture was stirred for 5 min. Then, *trans*-4-(3-fluoroazetidin-1-yl)cyclohexanamine (Intermediate 69) (0.068 g, 0.397 mmol) was added and the reaction mixture was stirred for 3 h. The reaction mixture was concentrated. The reaction mixture was purified by RP HPLC eluting with acetonitrile:water with 0.1 % ammonium hydroxide (5:95-100:0), then further purified by silica gel chromatography, eluting with MeOH:EtOAc (1:4) to give the title compound (0.0759 g, 43.6 % yield). $^1$H NMR (400 MHz, $CD_3SOCD_3$) δ 1.02 (dq, *J* = 13, 3 Hz, 2 H), 1.35 (dq, *J* = 12, 3 Hz, 2 H),, 1.77 (br d, *J* = 12 Hz, 2 H), 1.88 (dd, *J* = 13, 3 Hz, 2 H), 1.96-2.08 (m, 1 H), 2.96-3.10 (m, 2 H), 3.46-3.58 (m, 2 H), 3.77 (qt, *J* = 7, 4 Hz, 1 H), 5.10 (dp, *J* = 58, 5 Hz, 1 H), 7.61 (t, *J* = 73 Hz, 1 H), 7.93 (s, 1 H), 8.44 (s, 1 H), 8.62 (d, *J* = 8 Hz, 1 H), 8.77 (d, *J* = 2 Hz, 1 H), 9.27 (d, *J* = 2 Hz, 1 H); LC-MS (LC-ES) M+H = 428.

## Example 133

### 6-Chloro-7-(difluoromethoxy)-N-((3S)-5-methyl-2-oxopyrrolidin-3-vl)quinoline-3-carboxamide

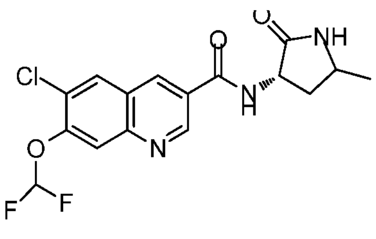

To a solution of 6-chloro-7-(difluoromethoxy)quinoline-3-carboxylic acid (Intermediate 20) (115 mg, 0.420 mmol) in DMF (10 mL) at RT, was added (3S)-3-amino-5-methylpyrrolidin-2-one hydrochloride (Intermediate 76) (63.3 mg, 0.420 mmol), followed by HATU (240 mg, 0.630 mmol) and DIEA (0.440 mL, 2.52 mmol). After stirring at RT for 24 h, the solvent was removed under reduced pressure, the residue was dissolved in 10% MeOH/DCM and absorbed onto silica gel. The crude material was then purified by silica gel chromatography (DCM to 10% MeOH/DCM) to afford the title compound (40 mg, 48% yield) as a mixture of diastereomers. $^1$H NMR (400 MHz, $CD_3SOCD_3$) δ ppm 1.18 (dd, *J* = 6, 2 Hz, 6 H), 1.58-1.64 (m, 1 H), 2.01 - 2.12 (m, 1 H), 2.18-2.25 (m, 1 H), 2.51 - 2.63 (m, 1 H), 3.60-3.65 (m, 1 H),

3.69-3.76 (m, 1H), 4.63 - 4.78 (m, 2 H), 7.63(t, *J* = 73 Hz, 2 H), (m, 2 H), 7.96 (s, 2 H), 8.05 (d, *J* = 15 Hz, 2 H), 8.47 (d, *J* = 2 Hz, 2 H), 8.80 - 8.88 (m, 2 H), 9.10 (dd, *J* = 16, 8 Hz, 2 H), 9.32 (dd, *J* = 3, 2 Hz, 2 H). MS (ESI) *m/z* 370(M+1).

**Example 134**

**6-Chloro-7-(difluoromethoxy)-N-(*trans*-4-(3-hydroxy-3-(trifluoromethyl)azetidin-1-yl)cyclohexyl)quinoline-3-carboxamide**

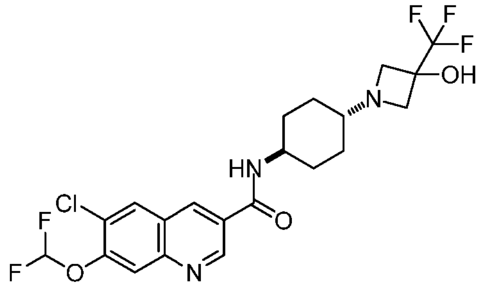

DIEA (0.347 mL, 1.986 mmol) was added to 6-chloro-7-(difluoromethoxy)quinoline-3-carboxylic acid (Intermediate 20) (0.1087 g, 0.397 mmol) in DMF (0.977 mL) at RT. Then, HATU (0.196 g, 0.516 mmol) was added and the reaction mixture was stirred for 5 min. Then, 1-(*trans*-4-aminocyclohexyl)-3-(trifluoromethyl)azetidin-3-ol (Intermediate 71) (0.099 g, 0.417 mmol) was added and the reaction mixture was stirred for 2 h. The reaction mixture was concentrated. The reaction mixture was purified by RP HPLC eluting with acetonitrile:water with 0.1 % ammonium hydroxide (5:95-100:0), then further purified by silica gel chromatography, eluting with MeOH:EtOAc (1:15) to give the title compound (0.1020 g, 48.4 % yield). $^1$H NMR (400 MHz, $CD_3SOCD_3$) δ 1.01 (q, *J* = 13 Hz, 2 H), 1.35 (q, *J* = 13 Hz, 2 H), 1.77 (br d, *J* = 12 Hz, 2 H), 1.88 (dd, *J* = 13, 3 Hz, 2 H), 2.04 (tt, *J* = 11, 4 Hz, 1 H), 3.11 (d, *J* = 9 Hz, 2 H), 3.48 (d, *J* = 9 Hz, 2 H), 3.77 (qt, *J* = 8, 4 Hz, 1 H), 6.82 (s, 1 H), 7.62 (t, *J* = 73 Hz, 1 H), 7.93 (s, 1 H), 8.45 (s, 1 H), 8.63 (d, *J* = 8 Hz, 1 H), 8.77 (d, *J* = 2 Hz, 1 H), 9.27 (d, *J* = 2 Hz, 1 H); LC-MS (LC-ES) M+H = 494.

**Example 135**

**6-Chloro-7-(difluoromethoxy)-N-(*trans*-4-(((R)-1,1,1-trifluoro-3-hydroxypropan-2-yl)amino)cyclohexyl)quinoline-3-carboxamide**

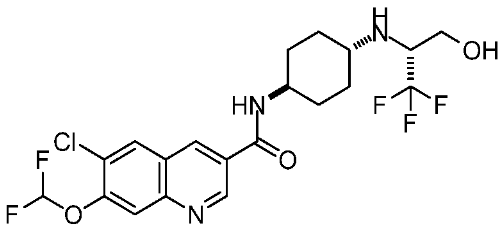

DIEA (0.328 mL, 1.880 mmol) was added to 6-chloro-7-(difluoromethoxy)quinoline-3-carboxylic acid (Intermediate 20) (0.1029 g, 0.376 mmol) in DMF (0.925 mL) at RT. Then, HATU (0.186 g, 0.489 mmol) was added and the reaction mixture was stirred for 5 min. Then, (R)-2-((*trans*-4-aminocyclohexyl)amino)-3,3,3-trifluoropropan-1-ol (Intermediate 65) (0.089 g, 0.395 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (9:1) to give the title compound (0.0835 g, 43.8 % yield). $^1$H NMR (400 MHz, $CD_3SOCD_3$) δ 1.12 (q, *J* = 12 Hz, 2 H), 1.37 (dq, *J* = 13, 3 Hz, 2 H), 1.82-2.02 (m, 5 H), 2.46-2.58 (m, 1 H), 3.20-3.36 (m, 1 H), 3.42-3.54 (m, 1 H), 3.56-3.68 (m, 1 H), 3.78 (qt, *J* = 8, 4 Hz, 1 H), 5.00 (t, *J* = 6 Hz, 1 H), 7.62 (t, J = 73 Hz, 1 H), 8.45 (s, 1 H), 7.93 (s, 1 H), 8.62 (d, *J* = 8 Hz, 1 H), 8.77 (d, *J* = 2 Hz, 1 H), 9.28 (d, *J* = 2 Hz, 1 H); LC-MS (LC-ES) M+H = 482.

**Example 136**

**N-(trans-4-(2-Amino-2-oxoethoxy)cyclohexyl)-6-chloro-7-(difluoromethoxy)quinoline-3-carboxamide**

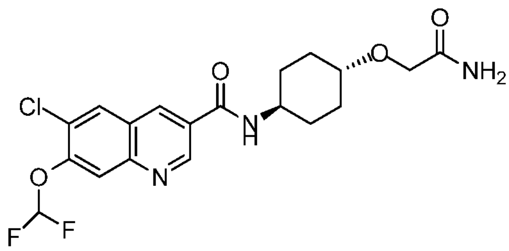

To a solution of 6-chloro-7-(difluoromethoxy)quinoline-3-carboxylic acid (Intermediate 20) (60 mg, 0.219 mmol) in DMF (50 mL) was added HATU (125 mg, 0.329 mmol), DIEA (0.115 mL, 0.658 mmol), and 2-((trans-4-aminocyclohexyl)oxy)acetamide (39.7 mg, 0.230 mmol). After stirring at RT for 24 h, the solvent was removed under reduced pressure, the residue was dissolved in 10 % MeOH/DCM (50 mL) and absorbed onto Celite®. The crude reaction was then purified by reverse phase chromatography (10-50% acetonitrile/ water (with 0.1% $NH_4OH$)) to afford the title compound (35 mg, 37% yield). $^1H$ NMR (400 MHz, $CD_3SOCD_3$) $\delta$ ppm 1.20 - 1.49 (m, 4 H) 1.90-1.93 (m, 2 H) 2.03 - 2.09 (m, 2 H) 3.23 - 3.31 (m, 1 H) 3.77-3.84 (m, 3 H) 7.05 (br s, 1 H) 7.23 (br s, 1 H) 7.60 (t, *J* = 73 Hz, 1 H) 7.92 (s, 1 H) 8.43 (s, 1 H) 8.61 (d, *J* = 7 Hz, 1 H) 8.77 (d, *J* = 2 Hz, 1 H) 9.27 (d, *J* = 2 Hz, 1 H). MS (ESI) *m/z* 428(M+1).

**Example 137**

**6-Chloro-7-(difluoromethoxy)-N-((1r,3r)-3-hydroxy-3-methylcyclobutyl)quinoline-3-carboxamide**

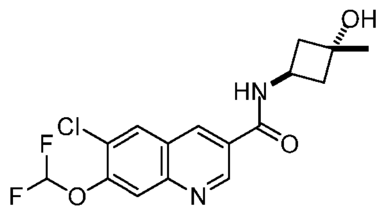

DIEA (7.22 mL, 41.3 mmol) was added to 6-chloro-7-(difluoromethoxy)quinoline-3-carboxylic acid (Intermediate 20) (3.23 g, 11.80 mmol) in DMF (11.80 mL) at RT. Then, HATU (5.84 g, 15.35 mmol) was added and the reaction mixture was stirred for 5 min. Then, 3-amino-1-methylcyclobutanol hydrochloride (Intermediate 51) (1.949 g, 14.17 mmol) was added and the reaction mixture was stirred for 16 h. The reaction mixture was concentrated. EtOAc was added and the solution was washed with sodium bicarbonate and water, dried over magnesium sulfate, filtered, and concentrated. The residue was purified by silica gel chromatography, eluting with EtOAc, then further purified by silica gel chromatography, eluting with MeOH:DCM 1:12, then further purified by RP HPLC eluting with acetonitrile:water with 0.1 % ammonium hydroxide (5:95-100:0) to give the title compound (0.9141 g, 20.6 % yield). $^1H$ NMR (400 MHz, $CD_3SOCD_3$) $\delta$ 1.29 (s, 3 H), 2.06-2.14 (m, 2 H), 2.26-2.36 (m, 2 H), 4.54 (h, *J* = 7 Hz, 1 H), 4.88 (s, 1 H), 7.61 (t, *J* = 73 Hz, 1 H), 7.94 (s, 1 H), 8.44 (s, 1 H), 8.78 (d, *J* = 2 Hz, 1 H), 8.94 (d, *J* = 7 Hz, 1 H), 9.28 (d, *J* = 2 Hz, 1 H); LC-MS (LC-ES) M+H = 357.

**Example 138**

**6-Chloro-7-(difluoromethoxy)-N-((1s,3s)-3-hydroxy-3-methylcyclobutyl)quinoline-3-carboxamide**

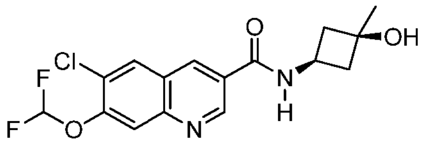

Tert-butyl (cis-3-hydroxy-3-methylcyclobutyl)carbamate (Intermediate 52) (14.644 g, 72.8 mmole) was dissolved in MeOH (225 mL) and HCl (4M solution in 1,4-dioxane, 79.0 mL, 316 mmole) was added. The solution was stirred at RT overnight and was concentrated. 6-Chloro-7-(difluoromethoxy)quinoline-3-carboxylic acid (Intermediate 20) (17.320 g, 63.3 mmole) was added and the mixture was dissolved in DMF (225 mL). DIEA (33.0 mL, 189 mmole) was added followed by dropwise addition of propylphosphonic anhydride ($T_3P$, 50% solution in ethyl acetate, 43.5 mL, 73.1 mmole). The resulting mixture was stirred at RT overnight. Saturated aqueous sodium bicarbonate (400 mL) and water (400 mL) were added (caution, foaming) and the mixture was extracted with EtOAc (8 x 150 mL). The combined organics were dried over magnesium sulfate and concentrated. The residue was split into 4 equal portions and purified by silica gel chromatography eluting with 50% EtOAc/heptane. The cis (desired) isomer eluted first. When the trans isomers was observed to elute the solvent was changed to 100% ethyl acetate. Pure fractions were pooled and concentrated. Mixed fractions were pooled, concentrated and re-subjected to silica gel chromatography using the above conditions. The pure product was recrystallized from isopropanol to afford the title compound (15.086 g, 67% yield) as a white solid. $^1$H NMR ($CD_3SOCD_3$): δ 1.27 (s, 3H), 2.06-2.20 (m, 2H), 2.26-2.39 (m, 2H), 4.02 (s, *J* = 8 Hz, 1H), 5.00 (s, 1H), 7.60 (t, *J* = 72 Hz, 1H), 7.92 (s, 1H), 8.41 (s, 1H), 8.79 (d, *J* = 2 Hz, 1H), 8.97 (d, *J* = 7 Hz, 1H), 9.29 (d, *J* = 2 Hz, 1H). MS (ESI) *m/z* 357 (M+1).

**Example 139**

**6-Chloro-7-(difluoromethoxy)-N-(trans-4-(2-(methylsulfonyl)ethoxy)cyclohexyl)quinoline-3-carboxamide**

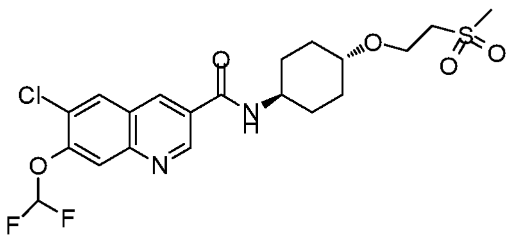

To a solution of 6-chloro-7-(difluoromethoxy)quinoline-3-carboxylic acid (Intermediate 20) (48 mg, 0.175 mmol) in DMF (5 mL) at RT, was added DIEA (0.092 mL, 0.526 mmol), HATU (100 mg, 0.263 mmol), and trans-4-(2-(methylsulfonyl)ethoxy)cyclohexanamine (Intermediate 72) (46.6 mg, 0.211 mmol). After stirring at RT for 24 h, the solvent was removed under reduced pressure, the residue was dissolved in MeOH/DCM, absorbed onto Celite® and purified by reverse phase chromatography (10-50% acetonitrile/ water (with 0.2% $NH_4OH$)) to afford the title compound (40 mg, 48% yield). $^1$H NMR (400 MHz, $CD_3SOCD_3$) δ ppm 1.18 - 1.55 (m, 4 H), 1.92-1.93 (m, 2 H), 2.04-2.07 (m, 2 H), 2.99 (s, 3 H), 3.27 - 3.37 (m, 3 H), 3.77 - 3.97 (m, 3 H), 7.62 (t, *J* = 72 Hz, 1 H), 7.95 (s, 1 H), 8.45 (s, 1 H), 8.64 (d, *J* = 8 Hz, 1 H), 8.79 (d, *J* = 2 Hz, 1 H), 9.29 (d, *J* = 2 Hz, 1 H). MS (ESI) *m/z* 477(M+1).

**Example 140**

**6-Chloro-7-(difluoromethoxy)-N-(*trans*-3-hydroxycyclobutyl)quinoline-3-carboxamide**

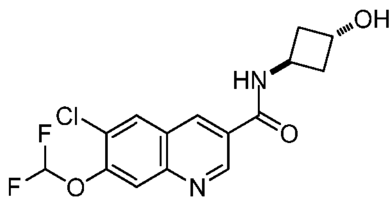

DIEA (0.174 mL, 0.994 mmol) was added to 6-chloro-7-(difluoromethoxy)quinoline-3-carboxylic acid (Intermediate 20) (0.0544 g, 0.199 mmol) in DMF (0.489 mL) at RT. Then, HATU (0.098 g, 0.258 mmol) was added and the reaction mixture was stirred for 5 min. Then, *trans*-3-aminocyclobutanol hydrochloride (0.026 g, 0.209 mmol) was added and the reaction mixture was stirred for 2 h. The reaction mixture was concentrated. The reaction mixture was purified by RP HPLC eluting with acetonitrile:water with 0.1 % ammonium hydroxide (5:95-100:0), then further purified by silica gel chromatography, eluting with MeOH:EtOAc (1:49) to give the title compound (0.0373 g, 52.0 % yield). $^1$H NMR (400 MHz, $CD_3SOCD_3$) δ 2.14-2.24 (m, 2 H), 2.26-2.36 (m, 2 H), 4.32-4.38 (m, 1 H), 4.40-4.52 (m, 1 H), 5.05 (br s, 1 H), 7.61 (t, *J* = 73 Hz, 1 H), 7.94 (s, 1 H), 8.44 (s, 1 H), 8.79 (d, *J* = 2 Hz, 1 H), 8.98 (d, *J* = 7 Hz, 1 H), 9.29 (d, *J* = 2 Hz, 1 H); LC-MS (LC-ES) M+H = 343.

**Example 141**

**trans-4-(6-Chloro-7-(difluoromethoxy)quinoline-3-carboxamido)cyclohexyl sulfamate**

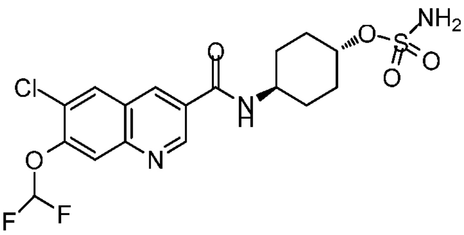

To a solution of 6-chloro-7-(difluoromethoxy)quinoline-3-carboxylic acid (Intermediate 20) (48 mg, 0.175 mmol) in DMF (5 mL) at RT, was added DIEA (0.092 mL, 0.526 mmol), HATU (100 mg, 0.263 mmol), and trans-4-aminocyclohexyl sulfamate (Intermediate 77) (40.9 mg, 0.211 mmol). After stirring at RT for 24 h, the solvent was removed under reduced pressure, the residue dissolved in 10% MeOH/DCM, absorbed onto Celite® and purified by reverse phase chromatography (10-50% acetonitrile/ water (with 0.2% $NH_4OH$)) to afford the title compound (40 mg, 51 % yield). $^1$H NMR (400 MHz, $CD_3SOCD_3$) δ ppm 1.40 - 1.71 (m, 4 H), 1.97-1.99 (m, 2 H), 2.11-2.13 (m, 2 H), 3.81 - 3.96 (m, 1 H), 4.35-4.40 (m, 1 H), 7.43 (s, 2 H), 7.63 (t, *J* = 73 Hz, 1 H), 7.95 (s, 1 H), 8.46 (s, 1 H), 8.66 (d, *J* = 8 Hz, 1 H), 8.79 (d, *J* = 2 Hz, 1 H), 9.30 (d, *J* = 2 Hz, 1 H). MS (ESI) *m/z* 450(M+1).

**Example 142**

**6-Chloro-7-(difluoromethoxy)-N-(3-oxocyclobutyl)quinoline-3-carboxamide**

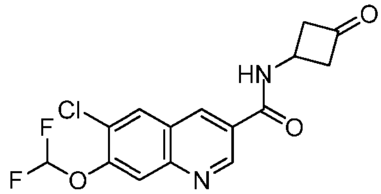

DIEA (0.510 mL, 2.92 mmol) was added to 6-chloro-7-(difluoromethoxy)quinoline-3-carboxylic acid (Intermediate 20) (0.1599 g, 0.584 mmol) in DMF (1.438 mL) at RT. Then, HATU (0.289 g, 0.760 mmol) was added and the

reaction mixture was stirred for 5 min. Then, 3-aminocyclobutanone hydrochloride (0.075 g, 0.614 mmol) was added and the reaction mixture was stirred for 2 h. The reaction mixture was concentrated. The reaction mixture was purified by RP HPLC eluting with acetonitrile:water with 0.1 % ammonium hydroxide (5:95-100:0), then further purified by silica gel chromatography, eluting with EtOAc:hexanes (4:1) to give the title compound (0.1097 g, 49.6 % yield). $^1$H NMR (400 MHz, $CD_3SOCD_3$) δ 3.20-3.30 (m, 2 H), 3.40-3.50 (m, 2 H), 4.54-4.66 (m, 1 H), 7.62 (t, *J* = 73 Hz, 1 H), 7.95 (s, 1 H), 8.46 (s, 1 H), 8.82 (d, *J* = 2 Hz, 1 H), 9.30 (d, *J* = 6 Hz, 1 H), 9.33 (d, *J* = 2 Hz, 1 H); LC-MS (LC-ES) M+H = 341.

**Example 143**

**6-Chloro-7-(difluoromethoxy)-N-((1r,4r)-4-hydroxy-4-methylcyclohexy)quinoline-3-carboxamide**

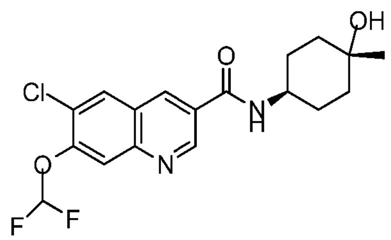

To a solution of 6-chloro-7-(difluoromethoxy)quinoline-3-carboxylic acid (Intermediate 20) (100 mg, 0.365 mmol) in DMF (50 mL) was added HATU (208 mg, 0.548 mmol), DIEA (0.191 mL, 1.096 mmol), and (1r,4r)-4-amino-1-methylcyclohexanol (see WO 2010 027097) (51.9 mg, 0.402 mmol). After stirring at RT for 24 h, the solvent was removed under reduced pressure, the residue was dissolved in 10 % MeOH/DCM and absorbed onto silica gel. The crude reaction was then purified by silica gel chromatography (DCM to 10% MeOH/DCM) to afford the title compound (125 mg, 44% yield). $^1$H NMR (400 MHz, $CD_3SOCD_3$) δ ppm 1.17 (s, 3 H), 1.39 - 1.72 (m, 6 H), 1.79 - 1.91 (m, 2 H), 3.82 - 3.99 (m, 1 H), 4.30 (s, 1 H), 7.63 (t, *J* = 73 Hz, 1 H), 7.95 (s, 1 H), 8.46 (s, 1 H), 8.57 (d, *J* = 8 Hz, 1 H), 8.79 (d, *J* = 2 Hz, 1 H), 9.28 (d, *J* = 2 Hz, 1 H). MS (ESI) *m/z* 385 (M+1).

**Example 144**

**6-Chloro-7-(difluoromethoxy)-N-((3S,4R)-4-methyl-2-oxopyrrolidin-3-y)quino)ine-3-carboxamide**

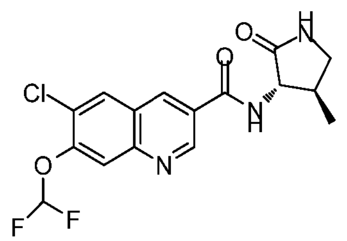

To a solution of 6-chloro-7-(difluoromethoxy)quinoline-3-carboxylic acid (Intermediate 20) (900 mg, 3.29 mmol) in DMF (30 mL) at RT, was added (3S,4R)-3-amino-4-methylpyrrolidin-2-one (Intermediate 78) (413 mg, 3.62 mmol), followed by HATU (1876 mg, 4.93 mmol) and DIEA (1.723 mL, 9.87 mmol). After stirring at RT for 24 h, the solvent was removed under reduced pressure, the residue was dissolved in 10% MeOH/DCM and absorbed onto silica gel. The crude reaction was then purified by silica gel chromatography (DCM to 10% MeOH/DCM) to afford the title compound (550 mg, 45% yield). $^1$H NMR (400 MHz, $CD_3SOCD_3$) δ ppm 1.12 (d, J = 7 Hz, 3 H), 2.39 - 2.49 (m, 1 H), 2.91 (t, *J* = 9 Hz, 1 H), 3.33 - 3.42 (m, 1 H), 4.33 (dd, *J* = 11, 9 Hz, 1 H), 7.63 (t, *J* = 72 Hz, 1 H), 7.87 (s, 1 H), 7.96 (s, 1 H), 8.48 (s, 1 H), 8.85 (d, *J* = 2 Hz, 1 H), 9.03 (d, *J* = 9 Hz, 1 H), 9.34 (d, *J* = 2 Hz, 1 H). MS (ESI) *m/z* 370 (M+1).

**Example 145**

***6*-Chloro-7-(difluoromethoxy)-N-(3-hydroxy-3-methylcyclopentyl)quinoline-3-carboxamide**

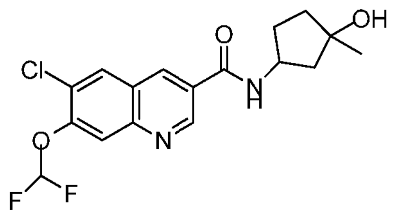

To a solution of 6-chloro-7-(difluoromethoxy)quinoline-3-carboxylic acid (Intermediate 20) (100 mg, 0.365 mmol) in DMF (50 mL) was added HATU (208 mg, 0.548 mmol), DIEA (0.191 mL, 1.096 mmol), and 3-amino-1-methylcyclopentanol (44 mg, 0.382 mmol). After stirring at RT for 24 h, the solvent was removed under reduced pressure, the residue was dissolved in 10 % MeOH/DCM (50 mL) and absorbed onto Celite®. The crude reaction was then purified by reverse phase chromatography (10-50% acetonitrile (with 0.1 % TFA)/ water (with 0.1 % TFA)) to afford the title compound (30 mg, 17% yield). $^1H$ NMR (400 MHz, $CD_3SOCD_3$) δ ppm 1.21 - 1.32 (m, 3 H), 1.44 - 1.61 (m, 1 H), 1.66 - 1.91 (m, 3 H), 1.95 - 2.12 (m, 2 H), 4.33 (dd, *J* = 15, 8 Hz, 1 H), 7.62 (t, *J* = 73 Hz, 1 H), 7.95 (s, 1 H) 8.44 (s, 1 H), 8.73 (d, *J* = 7 Hz, 1 H), 8.80 (d, *J* = 2 Hz, 1 H), 9.29 (d, *J* = 2 Hz, 1 H). MS (ESI) *m/z* 371 (M+1).

**Example 146**

**Racemic 6-Chloro-7-(difluoromethoxy)-N-((1S,3S)-3-hydroxy-3-methylcyclopentyl)quinoline-3-carboxamide**

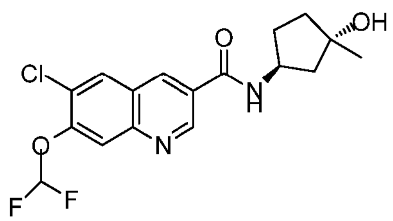

To a flask was added a solution of racemic tert-butyl ((1R,3R)-3-hydroxy-3-methylcyclopentyl)carbamate (see; WO 2011 156610) (42 mg, 0.195 mmol) in 1,4-dioxane (10 mL), followed by HCl (4.0 M in 1,4-dioxane, 2.439 mL, 9.75 mmol). After stirring overnight at RT, the reaction was concentrated. To the crude amine hydrochloride was added a solution of 6-chloro-7-(difluoromethoxy)quinoline-3-carboxylic acid (Intermediate 20) (53.4 mg, 0.195 mmol) in DMF (5 mL) followed by the addition of HATU (111 mg, 0.293 mmol), and DIEA (0.204 mL, 1.171 mmol). After stirring overnight at RT, the solvent was removed under reduced pressure, and the crude material was absorbed onto Celite®. This was then purified by reverse phase chromatography (10-50% acetonitrile/ water (with 0.2% $NH_4OH$)) to afford the title compound (30 mg, 42% yield). $^1H$ NMR (400 MHz, $CD_3SOCD_3$) δ ppm 1.29 (s, 3 H), 1.53 - 1.68 (m, 3 H), 1.69 - 1.79 (m, 1 H), 1.99-2.05 (m, 1 H), 2.13 - 2.26 (m, 1 H), 4.37 (s, 1 H), 4.49 - 4.65 (m, 1 H), 7.62 (t, *J* = 72 Hz, 1 H), 7.95 (s, 1 H), 8.44 (s, 1 H), 8.72 (d, *J* = 7 Hz, 1 H), 8.78 (d, *J* = 2 Hz, 1 H), 9.29 (d, *J* = 2 Hz, 1 H). MS (ESI) *m/z* 371 (M+1).

**Example 147**

**6-Chloro-7-(difluoromethoxy)-N-((1s,4s)-4-hydroxy-4-methylcyclohexyl)quinoline-3-carboxamide**

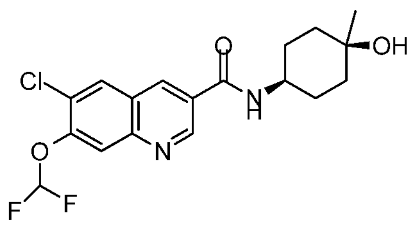

To a solution of 6-chloro-7-(difluoromethoxy)quinoline-3-carboxylic acid (Intermediate 20) (50 mg, 0.183 mmol) in DMF (50 mL) was added HATU (104 mg, 0.274 mmol), DIEA (0.096 mL, 0.548 mmol), and (1s,4s)-4-amino-1-methylcyclohexanol (see WO 2010 027097) (26.0 mg, 0.201 mmol). After stirring at RT for 24 h, the solvent was removed under reduced pressure, the residue was dissolved in 10 % MeOH/DCM (50 mL) and absorbed onto Celite®. The crude reaction was then purified by reverse phase chromatography (10-50% acetonitrile/water (with 0.2% $NH_4OH$)) to afford the title compound (20 mg, 28% yield). $^1H$ NMR (400 MHz, $CD_3SOCD_3$) δ ppm 1.13 (s, 3 H), 1.35-1.43 (m, 2 H), 1.53 - 1.68 (m, 4 H), 1.71 - 1.89 (m, 2 H), 3.76-3.82 (m, 1 H), 4.08 (s, 1 H), 7.63 (t, *J* = 72 Hz, 1 H), 7.94 (s, 1 H), 8.43 (s, 1 H), 8.63 (d, *J* = 8 Hz, 1 H), 8.81 (d, *J* = 2 Hz, 1 H), 9.31 (d, *J* = 2 Hz, 1 H). MS (ESI) *m/z* 385 (M+1).

**Example 148**

**6-Chloro-7-(difluoromethoxy)-N-(tetrahydro-2H-pyran-4-yl)quinoline-3-carboxamide**

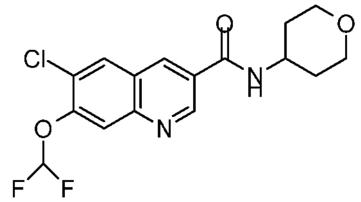

To a solution of 6-chloro-7-(difluoromethoxy)quinoline-3-carboxylic acid (Intermediate 20) (100 mg, 0.365 mmol) in DMF (10 mL) at RT, was added tetrahydro-2H-pyran-4-amine (37.0 mg, 0.365 mmol), followed by DIEA (0.255 mL, 1.462 mmol). Next, n-propylphosphonic acid anhydride ($T_3P$, 50% in EtOAc) (465 mg, 0.731 mmol) was added by a slow dropwise addition and the reaction was allowed to stir at RT overnight. Saturated $NaHCO_3$ solution was added to adjust the pH to 8. The mixture was repeatedly extracted with EtOAc until no product was in the organic layer as observed by TLC (10% MeOH/DCM). The combined extracts were concentrated, absorbed onto silica gel, and purified by silica gel chromatography (DCM to 10% MeOH/DCM) to afford the title compound (40 mg, 31 % yield). $^1H$ NMR (400 MHz, $CD_3SOCD_3$) δ ppm 1.54-1.64 (m, 2 H), 1.79-1.83 (m, 2 H), 3.37-3.43 (m, 2 H), 3.87-3.90 (m, 2 H), 3.99 - 4.12 (m, 1 H), 7.61 (t, *J* = 72 Hz, 1 H), 7.93 (s, 1 H), 8.44 (s, 1 H), 8.71 (d, *J* = 8 Hz, 1 H), 8.79 (d, *J* = 2 Hz, 1 H), 9.29 (d, *J* = 2 Hz, 1 H). MS (ESI) *m/z* 357 (M+1).

**Example 149**

**6-Chloro-7-(difluoromethoxy)-N-(tetrahydro-2H-pyran-3-yl)quinoline-3-carboxamide**

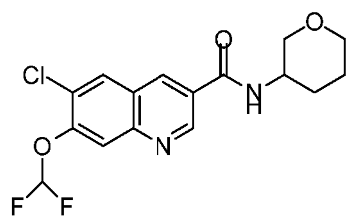

To a solution of 6-chloro-7-(difluoromethoxy)quinoline-3-carboxylic acid (Intermediate 20) (100 mg, 0.365 mmol)

in DMF (10 mL) at RT, was added tetrahydro-2H-pyran-3-amine (37.0 mg, 0.365 mmol) and DIEA (0.255 mL, 1.462 mmol). Next, n-propylphosphonic acid anhydride ($T_3P$, 50% in EtOAc) (465 mg, 0.731 mmol) was added by a slow dropwise addition and the reaction was allowed to stir at RT overnight. Saturated $NaHCO_3$ solution was added to adjust the pH to 8. The mixture was repeatedly extracted with EtOAc until no product was in the organic layer as observed by TLC (10% MeOH/DCM). The combined extracts were concentrated, absorbed onto silica gel, and purified by silica gel chromatography (DCM to 10% MeOH/DCM) to afford the title compound (90 mg, 64% yield). $^1H$ NMR (400 MHz, $CD_3SOCD_3$) $\delta$ ppm 1.53 - 1.78 (m, 3 H), 1.95-1.97 (m, 1 H), 3.19-3.24 (m, 1 H), 3.32 - 3.37 (m, 1 H), 3.70 - 3.79 (m, 1 H), 3.82 - 3.87 (m, 1 H), 3.91 - 4.01 (m, 1 H), 7.61 (t, *J* = 73 Hz, 1 H), 7.93 (s, 1 H), 8.43 (s, 1 H), 8.64 (d, *J* = 8 Hz, 1 H), 8.79 (d, *J* = 2 Hz, 1 H), 9.27 (d, *J* = 2 Hz, 1 H). MS (ESI) *m*/*z* 357 (M+1).

**Example 150**

**6-Chloro-7-(difluoromethoxy)-N-(2,2-dimethyltetrahydro-2H-pyran-4-yl)quinoline-3-carboxamide**

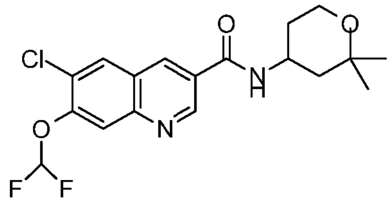

To a solution of 6-chloro-7-(difluoromethoxy)quinoline-3-carboxylic acid (Intermediate 20) (100 mg, 0.365 mmol) in DMF (10 mL) at RT, was added DIEA (0.255 mL, 1.462 mmol) and 2,2-dimethyltetrahydro-2H-pyran-4-amine (47.2 mg, 0.365 mmol). Next, n-propylphosphonic acid anhydride ($T_3P$, 50% in EtOAc) (465 mg, 0.731 mmol) was added by a slow dropwise addition and the reaction was allowed to stir at RT overnight. Saturated $NaHCO_3$ solution was added to adjust the pH to 8. The mixture was repeatedly extracted with EtOAc until no product was in the organic layer as observed by TLC (10% MeOH/DCM). The combined extracts were concentrated, absorbed onto silica gel, and purified by silica gel chromatography (DCM to 5% MeOH/DCM) to afford the title compound (90 mg, 64% yield). $^1H$ NMR (400 MHz, $CD_3SOCD_3$) $\delta$ ppm 1.16 - 1.29 (m, 6 H), 1.36 - 1.58 (m, 2 H), 1.76 - 1.85 (m, 2 H), 3.60 - 3.76 (m, 2 H), 4.20-4.28 (m, 1 H), 7.63 (t, *J* = 72 Hz, 1 H), 7.95 (s, 1 H), 8.46 (s, 1 H), 8.65 (d, *J* = 8 Hz, 1 H), 8.79 (d, *J* = 2 Hz, 1 H), 9.30 (d, *J* = 2 Hz, 1 H). MS (ESI) *m*/*z* 385 (M+1).

**Example 151**

**7-(2,2-Difluorocyclopropyl)-6-fluoro-N-(trans-4-(2-hydroxypropan-2-vl)cyclohexyl)quinoline-3-carboxamide**

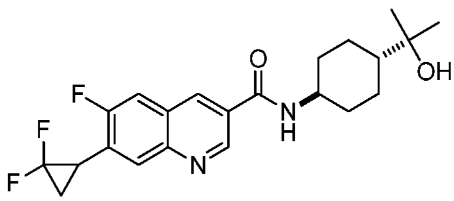

To a solution of 7-(2,2-difluorocyclopropyl)-6-fluoroquinoline-3-carboxylic acid (Intermediate 21) (0.059 g, 0.221 mmol) and HATU (0.088 g, 0.232 mmol) in DMF (3 mL) was added DIEA (0.042 mL, 0.243 mmol). The mixture was stirred 1 h at RT and 2-(trans-4-aminocyclohexyl)propan-2-ol (0.042 g, 0.265 mmol) was added in one portion. After 1 h the mixture was poured into water and extracted with EtOAc (3X). Combined organics were washed (water, brine), dried over $Na_2SO_4$, and concentrated *in vacuo*. The residue was purified by preparative HPLC (C18 column, MeCN / water gradient with 0.1 % TFA modifier) affording the title compound (0.0518 g, 58 % yield) as an orange gum. $^1H$ NMR (400 MHz, $CD_3OD$) $\delta$ ppm 1.18 (s, 6 H), 1.22 - 1.50 (m, 6 H), 1.91 - 2.19 (m, 6 H), 3.09 - 3.22 (m, 1 H), 3.88 (tt, *J* = 12, 4 Hz, 1 H), 7.82 (d, *J* = 10 Hz, 1 H), 7.97 (d, *J* = 7 Hz, 1 H), 8.81 (d, *J* = 2 Hz, 1 H), 9.23 (d, *J* = 2 Hz, 1 H). MS (ESI) *m*/*z* 407 (M+H).

**Example 152**

**6-Bromo-7-(difluoromethoxy)-N-(*trans*-4-( 2-hydroxypropan-2-vl)cyclohexyl)quinoline-3-carboxamide**

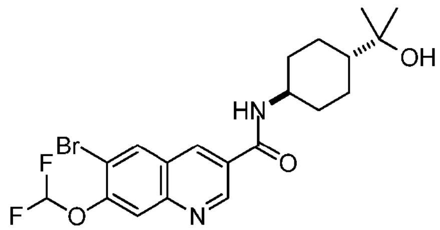

DIEA (0.212 mL, 1.215 mmol) was added to 6-bromo-7-(difluoromethoxy)quinoline-3-carboxylic acid (Intermediate 22) (0.0773 g, 0.243 mmol) in DMF (0.598 mL) at RT. Then, HATU (0.120 g, 0.316 mmol) was added and the reaction mixture was stirred for 5 min. Then, 2-(*trans*-4-aminocyclohexyl)propan-2-ol hydrochloride (0.049 g, 0.255 mmol) was added and the reaction mixture was stirred for 2 h. The reaction mixture was concentrated. The reaction mixture was purified by RP HPLC eluting with acetonitrile:water with 0.1 % ammonium hydroxide (5:95-100:0), then further purified by silica gel chromatography, eluting with EtOAc:hexanes (9:1) to give the title compound (0.0330 g, 28.2 % yield). $^1$H NMR (400 MHz, $CD_3SOCD_3$) $\delta$ 1.04 (s, 6 H), 1.04-1.24 (m, 3 H), 1.32 (br q, *J* = 12 Hz, 2 H), 1.84 (br d, *J* = 11 Hz, 2 H), 1.94 (br d, *J* = 10 Hz, 2 H), 3.68-3.80 (m, 1 H), 4.04 (s, 1 H), 7.61 (t, *J* = 73 Hz, 1 H), 7.89 (s, 1 H), 8.59 (d, *J* = 8 Hz, 1 H), 8.60 (s, 1 H), 8.77 (d, *J* = 2 Hz, 1 H), 9.29 (d, *J* = 2 Hz, 1 H); LC-MS (LC-ES) M+H = 457.

**Example 153**

**6-Bromo-7-(difluoromethoxy)-N-(*trans*-4-(((S)-1,1,1-trifluoro-3-hydroxypropan-2-yl)amino)cyclohexyl)quinoline-3-carboxamide**

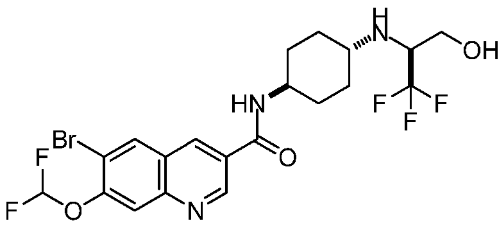

DIEA (0.303 mL, 1.732 mmol) was added to 6-bromo-7-(difluoromethoxy)quinoline-3-carboxylic acid (Intermediate 22) (0.1102 g, 0.346 mmol) in DMF (0.852 mL) at RT. Then, HATU (0.171 g, 0.450 mmol) was added and the reaction mixture was stirred for 5 min. Then, (S)-2-((*trans*-4-aminocyclohexyl)amino)-3,3,3-trifluoropropan-1-ol (Intermediate 67) (0.082 g, 0.364 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (9:1) to give the title compound (0.0454 g, 23.6 % yield). $^1$H NMR (400 MHz, $CD_3SOCD_3$) $\delta$ 1.13 (q, *J* = 12 Hz, 2 H), 1.37 (dq, *J* = 12, 3 Hz, 2 H), 1.84-2.02 (m, 4 H), 2.46-2.58 (m, 1 H), 3.22-3.34 (m, 1 H), 3.44-3.52 (m, 1 H), 3.58-3.66 (m, 1 H), 3.78 (qt, *J* = 8, 4 Hz, 1 H), 4.98 (t, *J* = 6 Hz, 1 H), 7.61 (t, *J* = 73 Hz, 1 H), 7.89 (s, 1 H), 8.59 (d, *J* = 8 Hz, 1 H), 8.60 (s, 1 H), 8.77 (d, *J* = 2 Hz, 1 H), 9.28 (d, *J* = 2 Hz, 1 H); LC-MS (LC-ES) M+H = 526.

**Example 154**

**(S)-6-Bromo-7-(difluoromethoxy)-N-(2-oxopyrrolidin-3-yl)quinoline-3-carboxamide**

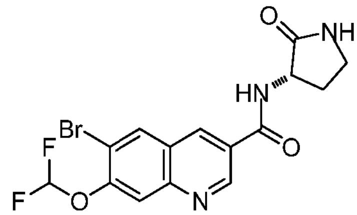

DIEA (0.267 mL, 1.530 mmol) was added to 6-bromo-7-(difluoromethoxy)quinoline-3-carboxylic acid (Intermediate 22) (0.0973 g, 0.306 mmol) in DMF (0.753 mL) at RT. Then, HATU (0.151 g, 0.398 mmol) was added and the reaction mixture was stirred for 5 min. Then, (S)-3-aminopyrrolidin-2-one (0.032 g, 0.321 mmol) was added and the reaction mixture was stirred for 2 h. The reaction mixture was concentrated. The reaction mixture was purified by RP HPLC eluting with acetonitrile:water with 0.1 % ammonium hydroxide (5:95-100:0), then further purified by silica gel chromatography, eluting with MeOH:EtOAc (1:6)to give the title compound (0.0387 g, 30.0 % yield). $^1$H NMR (400 MHz, $CD_3SOCD_3$) $\delta$ 1.98-2.10 (m, 1 H), 2.38-2.46 (m, 1 H), 3.22-3.32 (m, 2 H), 4.62 (q, *J* = 10 Hz, 1 H), 7.62 (t, *J* = 73 Hz, 1 H), 7.90 (s, 1 H), 7.91 (br s, 1 H), 8.62 (s, 1 H), 8.82 (d, *J* = 2 Hz, 1 H), 9.08 (d, *J* = 8 Hz, 1 H), 9.32 (d, *J* = 2 Hz, 1 H); LC-MS (LC-ES) M+H = 400.

## Example 155

### 6-Bromo-7-(difluoromethoxy)-N-(*trans*-4-(((R)-1,1,1-trifluoro-3-hydroxypropan-2-yl)amino)cyclohexyl)quinoline-3-carboxamide

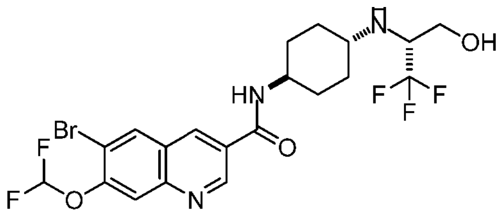

DIEA (0.239 mL, 1.368 mmol) was added to 6-bromo-7-(difluoromethoxy)quinoline-3-carboxylic acid (Intermediate 22) (0.0870 g, 0.274 mmol) in DMF (0.673 mL) at RT. Then, HATU (0.135 g, 0.356 mmol) was added and the reaction mixture was stirred for 5 min. Then, (R)-2-((*trans*-4-aminocyclohexyl)amino)-3,3,3-trifluoropropan-1-ol (Intermediate 65) (0.062 g, 0.274 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (9:1) to give the title compound (0.0365 g, 24.1 % yield). $^1$H NMR (400 MHz, $CD_3SOCD_3$) $\delta$ 1.13 (q, *J* = 12 Hz, 2 H), 1.37 (dq, *J* = 12, 3 Hz, 2 H), 1.84-2.02 (m, 4 H), 2.46-2.58 (m, 1 H), 3.22-3.34 (m, 1 H), 3.44-3.52 (m, 1 H), 3.58-3.66 (m, 1 H), 3.78 (qt, *J* = 8, 4 Hz, 1 H), 4.98 (t, *J* = 6 Hz, 1 H), 7.61 (t, *J* = 73 Hz, 1 H), 7.89 (s, 1 H), 8.59 (d, *J* = 8 Hz, 1 H), 8.60 (s, 1 H), 8.77 (d, *J* = 2 Hz, 1 H), 9.28 (d, *J* = 2 Hz, 1 H); LC-MS (LC-ES) M+H = 526.

## Example 156

### 6-Bromo-7-(difluoromethoxy)-N-(*trans*-4-((1,1-difluoropropan-2-yl)amino)cyclohexyl)quinoline-3-carboxamide

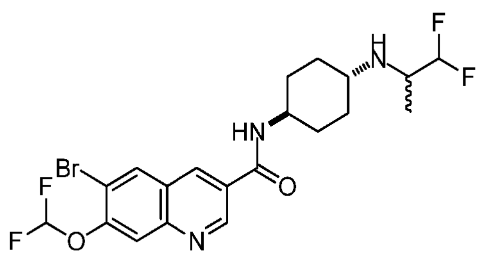

DIEA (0.586 mL, 3.35 mmol) was added to 6-bromo-7-(difluoromethoxy)quinoline-3-carboxylic acid (Intermediate 22) (0.2133 g, 0.671 mmol) in DMF (1.650 mL) at RT. Then, HATU (0.331 g, 0.872 mmol) was added and the reaction mixture was stirred for 5 min. Then, *trans*-N1-(1,1-difluoropropan-2-yl)cyclohexane-1,4-diamine (Intermediate 61) (0.129 g, 0.671 mmol) was added and the reaction mixture was stirred for 4 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc (1:49) to give the title compound (0.1238 g, 35.6 % yield). $^1$H NMR (400 MHz, $CD_3SOCD_3$) δ 1.03 (d, J = 7 Hz, 3 H), 1.11 (q, *J* = 12 Hz, 2 H), 1.37 (dq, *J* = 12, 3 Hz, 2 H), 1.48 (br s, 1 H), 1.84-1.98 (m, 4 H), 2.46-2.56 (m, 1 H), 2.92-3.06 (m, 1 H), 3.77 (qt, *J* = 8, 4 Hz, 1 H), 5.77 (dt, *J* = 57, 4 Hz, 1 H), 7.61 (t, *J* = 73 Hz, 1 H), 7.89 (s, 1 H), 8.60 (d, *J* = 8 Hz, 1 H), 8.60 (s, 1 H), 8.77 (d, *J* = 2 Hz, 1 H), 9.28 (d, *J* = 2 Hz, 1 H); LC-MS (LC-ES) M+H = 492.

**Example 157**

**6-Bromo-7-(difluoromethoxy)-N-((1s,3s)-3-hydroxy-3-methylcyclobutyl)quinoline-3-carboxamide**

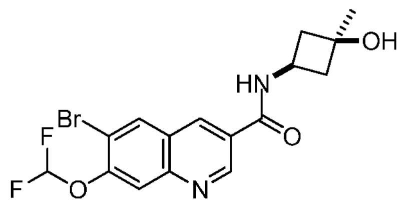

DIEA (0.288 mL, 1.649 mmol) was added to 6-bromo-7-(difluoromethoxy)quinoline-3-carboxylic acid (Intermediate 22) (0.1049 g, 0.330 mmol) in DMF (0.811 mL) at RT. Then, HATU (0.163 g, 0.429 mmol) was added and the reaction mixture was stirred for 5 min. Then, 3-amino-1-methylcyclobutanol hydrochloride (intermediate 51) (0.045 g, 0.330 mmol) was added and the reaction mixture was stirred for 2 h. The reaction mixture was concentrated. The reaction mixture was purified by RP HPLC eluting with acetonitrile:water with 0.1 % ammonium hydroxide (5:95-100:0), then further purified by silica gel chromatography, eluting with EtOAc to give the title compound (0.0176 g, 12.6 % yield). $^1$H NMR (400 MHz, $CD_3SOCD_3$) δ 1.28 (s, 3 H), 2.13 (dt, *J* = 9, 2 Hz, 2 H), 2.33 (dt, *J* = 8, 2 Hz, 2 H), 4.02 (h, *J* = 8 Hz, 1 H), 5.00 (s, 1 H), 7.61 (t, *J* = 73 Hz, 1 H), 7.89 (s, 1 H), 8.59 (s, 1 H), 8.79 (d, *J* = 2 Hz, 1 H), 8.97 (d, *J* = 7 Hz, 1 H), 9.31 (d, *J* = 2 Hz, 1 H); LC-MS (LC-ES) M+H = 401.

**Example 158 and 159**

**6-Bromo-7-(difluoromethoxy)-N-(*trans*-4-(((R)-1,1-difluoropropan-2-yl)amino)cyclohexyl)quinoline-3-carboxamide and 6-Bromo-7-(difluoromethoxy)-N-(*trans*-4-(((S)-1,1-difluoropropan-2-yl)amino)cyclohexyl)quinoline-3-carboxamide**

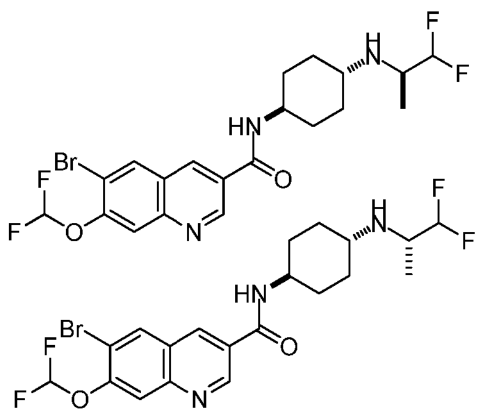

Racemic 6-bromo-7-(difluoromethoxy)-N-(*trans*-4-((1,1-difluoropropan-2-yl)amino)cyclohexyl)quinoline-3-carboxamide (Example 156) (0.1147 g, 0.233 mmol) was separated into its enantiomers via super critical fluid chromatography on a chiral ADH column eluting with MeOH:carbon dioxide (2:3) to give 6-bromo-7-(difluoromethoxy)-N-(*trans*-4-(((R)-1,1-difluoropropan-2-yl)amino)cyclohexyl)quinoline-3-carboxamide (0.0451 g, 37.4 % yield) as the first enantiomer to elute and 6-bromo-7-(difluoromethoxy)-N-(*trans*-4-(((S)-1,1-difluoropropan-2-yl)amino)cyclohexyl)quinoline-3-carboxamide (0.0456 g, 37.8 % yield) as the last enantiomer to elute. The structures were assigned by vibrational circular dichroism.

**6-Bromo-7-(difluoromethoxy)-N-(*trans*-4-(((R)-1,1-difluoropropan-2-yl)amino)cyclohexyl)quinoline-3-carboxamide:** $^1H$ NMR (400 MHz, $CD_3SOCD_3$) δ 1.03 (d, *J* = 7 Hz, 3 H), 1.11 (q, *J* = 12 Hz, 2 H), 1.37 (dq, *J* = 12, 3 Hz, 2 H), 1.50 (br s, 1 H), 1.84-1.98 (m, 4 H), 2.46-2.56 (m, 1 H), 2.92-3.06 (m, 1 H), 3.77 (qt, *J* = 8, 4 Hz, 1 H), 5.78 (dt, *J* = 57, 4 Hz, 1 H), 7.62 (t, *J* = 73 Hz, 1 H), 7.89 (s, 1 H), 8.60 (d, *J* = 8 Hz, 1 H), 8.61 (s, 1 H), 8.77 (d, *J* = 2 Hz, 1 H), 9.28 (d, *J* = 2 Hz, 1 H); LC-MS (LC-ES) M+H = 492.

**6-Bromo-7-(difluoromethoxy)-N-(*trans*-4-(((S)-1,1-difluoropropan-2-yl)amino)cyclohexyl)quinoline-3-carboxamide:** $^1H$ NMR (400 MHz, $CD_3SOCD_3$) δ 1.03 (d, *J* = 7 Hz, 3 H), 1.11 (q, *J* = 12 Hz, 2 H), 1.37 (dq, *J* = 12, 3 Hz, 2 H), 1.47 (br s, 1 H), 1.84-1.98 (m, 4 H), 2.46-2.56 (m, 1 H), 2.92-3.06 (m, 1 H), 3.77 (qt, *J* = 8, 4 Hz, 1 H), 5.77 (dt, *J* = 57, 4 Hz, 1 H), 7.61 (t, *J* = 73 Hz, 1 H), 7.89 (s, 1 H), 8.60 (d, *J* = 8 Hz, 1 H), 8.60 (s, 1 H), 8.77 (d, *J* = 2 Hz, 1 H), 9.28 (d, *J* = 2 Hz, 1 H); LC-MS (LC-ES) M+H = 492.

## Example 160

### 7-(Dimethylamino)-6-fluoro-N-(*trans*-4-(2-hydroxypropan-2-yl)cyclohexyl)quinoline-3-carboxamide

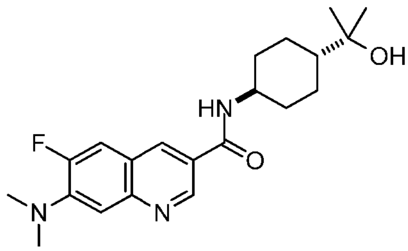

DIEA (0.172 mL, 0.986 mmol) was added to 7-(dimethylamino)-6-fluoroquinoline-3-carboxylic acid ammonia salt (Intermediate 23) (0.0462 g, 0.197 mmol) in DMF (0.485 mL) at RT. Then, HATU (0.097 g, 0.256 mmol) was added and the reaction mixture was stirred for 5 min. Then, 2-(*trans*-4-aminocyclohexyl)propan-2-ol (0.034 g, 0.217 mmol) was added and the reaction mixture was stirred for 16 h. The reaction mixture was concentrated. The reaction mixture was purified by RP HPLC eluting with acetonitrile:water with 0.1 % ammonium hydroxide (5:95-100:0), then further purified by silica gel chromatography, eluting with MeOH:EtOAc (1:16) to give the title compound (0.0444 g, 57.3 % yield). $^1H$ NMR (400 MHz, $CD_3SOCD_3$) δ 1.04 (s, 6 H), 1.09 (q, *J* = 13 Hz, 2 H), 1.12-1.24 (m, 1 H), 1.31 (q, *J* = 12 Hz, 2 H), 1.83 (br d, *J* = 12 Hz, 2 H), 1.92 (br d, *J* = 10 Hz, 2 H), 2.98 (s, 3 H), 2.98 (s, 3 H), 3.66-3.80 (m, 1 H), 4.03 (s, 1 H), 7.31 (d, *J* = 9 Hz, 1 H), 7.76 (d, *J* = 14 Hz, 1 H), 8.43 (d, *J* = 8 Hz, 1 H), 8.58 (d, *J* = 2 Hz, 1 H), 9.10 (d, *J* = 2 Hz, 1 H); LC-MS (LC-ES) M+H = 374.

## Example 161

### 6-Fluoro-N-(*trans*-4-(2-hydroxypropan-2-yl)cyclohexyl)-7-(methylthio)quinoline-3-carboxamide

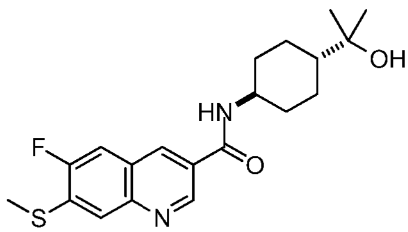

DIEA (0.063 mL, 0.362 mmol) was added to 6-fluoro-7-(methylthio)quinoline-3-carboxylic acid ammonia salt (Intermediate 24) (0.0172 g, 0.072 mmol) in DMF (0.242 mL) at RT. Then, HATU (0.036 g, 0.094 mmol) was added and the reaction mixture was stirred for 5 min. Then, 2-(*trans*-4-aminocyclohexyl)propan-2-ol (0.013 g, 0.080 mmol) was added and the reaction mixture was stirred for 16 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc (1:32) to give the title compound (0.0231 g, 80 % yield). $^1$H NMR (400 MHz, $CD_3SOCD_3$) δ 1.04 (s, 6 H), 1.06-1.24 (m, 3 H), 1.32 (q, *J* = 12 Hz, 2 H), 1.84 (br d, J = 12 Hz, 2 H), 1.94 (br d, *J* = 10 Hz, 2 H), 2.66 (s, 3 H), 3.75 (qt, *J* = 8, 4 Hz, 1 H), 4.04 (s, 1 H), 7.87 (d, *J* = 7 Hz, 1 H), 7.89 (d, *J* = 11 Hz, 1 H), 8.55 (d, *J* = 8 Hz, 1 H), 8.71 (d, *J* = 2 Hz, 1 H), 9.19 (d, *J* = 2 Hz, 1 H); LC-MS (LC-ES) M+H = 377.

**Example 162**

**7-(Azetidin-1-yl)-6-fluoro-N-(*trans*-4-(2-hydroxypropan-2-yl)cyclohexyl)quinoline-3-carboxamide**

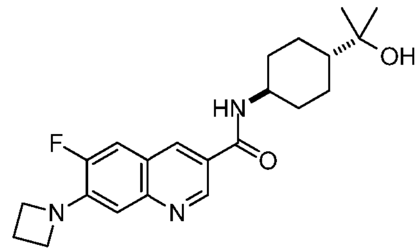

**A. Ethyl 7-(azetidin-1-yl)-6-fluoro-4-oxo-1-((2-(trimethylsilyl)ethoxy)methyl)-1,4-dihydroquinoline-3-carboxylate**

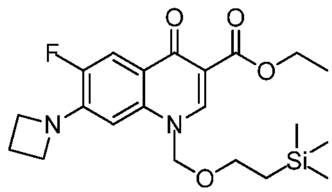

Azetidine (0.307 g, 5.37 mmol) was added to ethyl 6,7-difluoro-4-oxo-1-((2-(trimethylsilyl)ethoxy)methyl)-1,4-dihydroquinoline-3-carboxylate (Intermediate 23, step A) (1.03 g, 2.69 mmol) in 1,4-dioxane (7.55 mL) at RT, then DIEA (1.407 mL, 8.06 mmol) was added and the reaction mixture was heated at 100 °C for 42 h. The reaction mixture was poured into saturated sodium bicarbonate, extracted with DCM, dried over magnesium sulfate, filtered, and concentrated. The residue was purified by silica gel chromatography, eluting with EtOAc:hexanes (7:3) to give the title compound (0.7151 g, 60.1 % yield). $^1$H NMR (400 MHz, $CD_3SOCD_3$) δ -0.08 (s, 9 H), 0.85 (t, *J* = 8 Hz, 2 H), 1.26 (t, *J* = 7 Hz, 3 H), 2.36 (p, *J* = 7 Hz, 2 H), 3.57 (t, *J* = 8 Hz, 2 H), 4.09 (dt, *J* = 7, 2 Hz, 4 H), 4.20 (q, *J* = 7 Hz, 2 H), 5.63 (s, 2 H), 6.54 (d, *J* = 8 Hz, 1 H), 7.65 (d, *J* = 13 Hz, 1 H), 8.62 (s, 1 H); LC-MS (LC-ES) M+H = 421.

**B. Ethyl 4-chloro-7-((3-chloropropyl)amino)-6-fluoroquinoline-3-carboxylate**

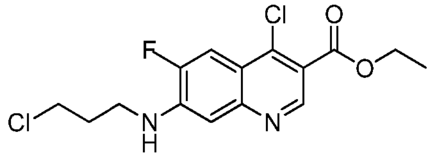

Phosphorus oxychloride (6.32 mL, 68.0 mmol) was added to ethyl 7-(azetidin-1-yl)-6-fluoro-4-oxo-1-((2-(trimethylsilyl)ethoxy)methyl)-1,4-dihydroquinoline-3-carboxylate (0.7151 g, 1.700 mmol) at RT, then the reaction mixture was heated at 105 °C for 2 h. The reaction mixture was poured into ice, quenched with 5 N sodium hydroxide, extracted with DCM, dried over magnesium sulfate, filtered, and concentrated. The residue was purified by silica gel chromatography, eluting with EtOAc:hexanes (3:7) to give the title compound (0.1816 g, 26.0 % yield). $^1H$ NMR (400 MHz, $CD_3SOCD_3$) δ 1.35 (t, *J* = 7 Hz, 3 H), 2.09 (p, *J* = 7 Hz, 2 H), 3.41 (q, *J* = 6 Hz, 2 H), 3.77 *(t, J* = 6 Hz, 2 H), 4.36 *(q, J* = 7 Hz, 2 H), 7.01 (br t, *J* = 4 Hz, 1 H), 7.11 (d, *J* = 8 Hz, 1 H), 7.88 (d, *J* = 13 Hz, 1 H), 9.26 (s, 1 H); LC-MS (LC-ES) M+H = 345.

**C. Ethyl 7-((3-chloropropyl)amino)-6-fluoroquinoline-3-carboxylate**

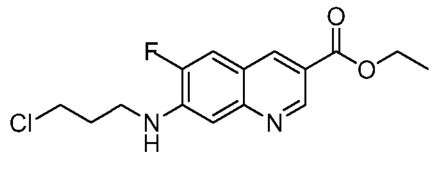

Bis(triphenylphosphine)palladium(II) chloride (0.018 g, 0.026 mmol) was added to ethyl 4-chloro-7-((3-chloropropyl)amino)-6-fluoroquinoline-3-carboxylate (0.1816 g, 0.526 mmol) in acetonitrile (5.26 mL) at RT, then triethylsilane (0.118 mL, 0.737 mmol) was added and the reaction mixture was heated at 70 °C for 16 h. The reaction mixture was poured into saturated sodium bicarbonate, extracted with DCM, dried over magnesium sulfate, filtered, and concentrated. The residue was purified by silica gel chromatography, eluting with EtOAc:hexanes (3:7) to give the title compound (0.0986 g, 57.3 % yield). $^1H$ NMR (400 MHz, $CD_3SOCD_3$) δ 1.35 (t, *J* = 7 Hz, 3 H), 2.09 (p, *J* = 7 Hz, 2 H), 3.39 (q, *J* = 6 Hz, 2 H), 3.77 (t, *J* = 6 Hz, 2 H), 4.35 (q, *J* = 7 Hz, 2 H), 6.84 (br t, *J* = 4 Hz, 1 H), 7.07 (d, *J* = 8 Hz, 1 H), 7.81 (d, *J* = 12 Hz, 1 H), 8.67 (d, *J* = 2 Hz, 1 H), 9.06 (d, *J* = 2 Hz, 1 H); LC-MS (LC-ES) M+H = 311.

**D. 7-((3-Chloropropyl)amino)-6-fluoroquinoline-3-carboxylic acid ammonia salt**

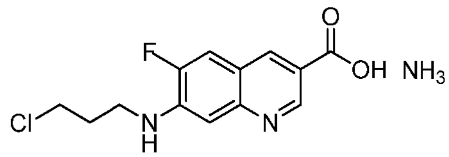

Lithium hydroxide (0.021 g, 0.892 mmol) was added to ethyl 7-((3-chloropropyl)amino)-6-fluoroquinoline-3-carboxylate (0.0924 g, 0.297 mmol) in MeOH (5.29 mL) and water (0.661 mL) at RT and the reaction mixture was stirred 2 h at 60 °C. The reaction mixture was concentrated. The reaction mixture was purified by RP HPLC eluting with acetonitrile:water with 0.1 % ammonium hydroxide (5:95-100:0) to give the title compound (0.0538 g, 57.3 % yield). $^1H$ NMR (400 MHz, $CD_3SOCD_3$) δ 2.09 (p, *J* = 7 Hz, 2 H), 3.39 (q, *J* = 6 Hz, 2 H), 3.77 (t, *J* = 6 Hz, 2 H), 6.78 (br t, *J* = 4 Hz, 1 H), 7.06 (d, *J* = 8 Hz, 1 H), 7.77 (d, *J* = 12 Hz, 1 H), 8.64 (d, *J* = 2 Hz, 1 H), 9.05 (d, *J* = 2 Hz, 1 H), 13.07 (br s, 1 H); LC-MS (LC-ES) M-H = 281.

**E. 7-((3-Chloropropyl)amino)-6-fluoro-N-(*trans*-4-(2-hydroxypropan-2-yl)cyclohexyl)quinoline-3-carboxamide**

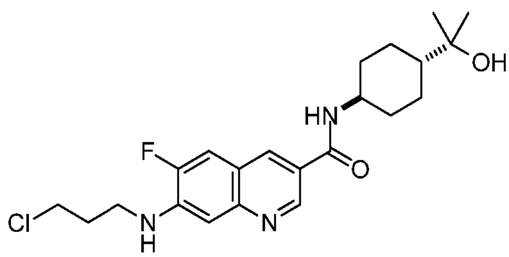

DIEA (0.153 mL, 0.875 mmol) was added to 7-((3-chloropropyl)amino)-6-fluoroquinoline-3-carboxylicacid (0.0495 g, 0.175 mmol) in DMF (0.584 mL) at RT. Then, HATU (0.087 g, 0.228 mmol) was added and the reaction mixture was stirred for 5 min. Then, 2-(*trans*-4-aminocyclohexyl)propan-2-ol (0.030 g, 0.193 mmol) was added and the reaction mixture was stirred for 16 h. The reaction mixture was concentrated. The reaction mixture was purified by RP HPLC eluting with acetonitrile:water with 0.1 % ammonium hydroxide (5:95-100:0), then further purified by silica gel chromatography, eluting with EtOAc to give the title compound (0.0431 g, 55.4 % yield). $^1$H NMR (400 MHz, $CD_3SOCD_3$) δ 1.04 (s, 6 H), 1.06-1.26 (m, 3 H), 1.30 (q, *J* = 12 Hz, 2 H), 1.83 (br d, *J* = 11 Hz, 2 H), 1.92 (br d, *J* = 10 Hz, 2 H), 2.09 (p, *J* = 7 Hz, 2 H), 3.37 (q, *J* = 7 Hz, 2 H), 3.73 (qt, J = 8, 4 Hz, 1 H), 3.77 (t, *J* = 7 Hz, 2 H), 4.03 (s, 1 H), 6.62 (br t, *J* = 4 Hz, 1 H), 7.05 (d, *J* = 8 Hz, 1 H), 7.66 (d, *J* = 12 Hz, 1 H), 8.33 (d, *J* = 8 Hz, 1 H), 8.50 (d, *J* = 2 Hz, 1 H), 9.03 (d, *J* = 2 Hz, 1 H); LC-MS (LC-ES) M+H = 422.

**F. 7-(Azetidin-1-yl)-6-fluoro-N-(*trans*-4-(2-hydroxypropan-2-vl)cyclohexyl)quinoline-3-carboxamide**

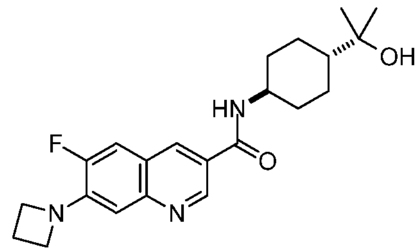

Sodium hydride (10.44 mg, 0.261 mmol) was added to 7-((3-chloropropyl)amino)-6-fluoro-N-(*trans*-4-(2-hydroxypropan-2-yl)cyclohexyl)quinoline-3-carboxamide (0.0367 g, 0.087 mmol) in DMF (1.740 mL) at RT, then the reaction mixture was heated at 80 °C for 4 h. The reaction mixture was quenched with MeOH and concentrated. The residue was purified by RP HPLC eluting with acetonitrile:water with 0.1 % ammonium hydroxide (5:95-100:0) to give the title compound (0.0186 g, 52.7 % yield). $^1$H NMR (400 MHz, $CD_3SOCD_3$) δ 1.04 (s, 6 H), 1.06-1.26 (m, 3 H), 1.30 (q, *J* = 12 Hz, 2 H), 1.83 (br d, *J* = 12 Hz, 2 H), 1.91 (br d, *J* = 10 Hz, 2 H), 2.37 (p, *J* = 7 Hz, 2 H), 3.72 (tq, *J* = 8, 4 Hz, 1 H), 4.02 (s, 1 H), 4.12 (dt, *J* = 7, 2 Hz, 4 H), 6.85 (d, *J* = 9 Hz, 1 H), 7.68 (d, *J* = 12 Hz, 1 H), 8.36 (d, *J* = 8 Hz, 1 H), 8.53 (d, *J* = 2 Hz, 1 H), 9.05 (d, *J* = 2 Hz, 1 H); LC-MS (LC-ES) M+H = 386.

**Example 163**

**6-Fluoro-N-(*trans*-4-(2-hydroxypropan-2-yl)cyclohexyl)-7-(pyrrolidin-1-yl)quinoline-3-carboxamide**

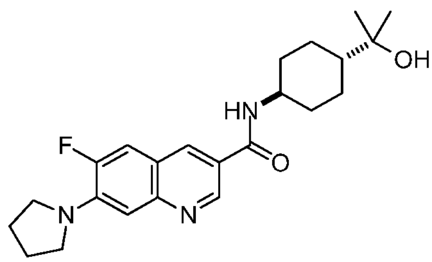

DIEA (0.164 mL, 0.941 mmol) was added to 6-fluoro-7-(pyrrolidin-1-yl)quinoline-3-carboxylic acid ammonia salt (Intermediate 25) (0.0522 g, 0.188 mmol) in DMF (0.627 mL) at RT. Then, HATU (0.093 g, 0.245 mmol) was added and the reaction mixture was stirred for 5 min. Then, 2-(*trans*-4-aminocyclohexyl)propan-2-ol (0.033 g, 0.207 mmol) was added and the reaction mixture was stirred for 16 h. The reaction mixture was concentrated. The reaction mixture was purified by RP HPLC eluting with acetonitrile:water with 0.1 % ammonium hydroxide (5:95-100:0), then further purified by silica gel chromatography, eluting with MeOH:EtOAc (1:12) to give the title compound (0.0444 g, 56.1 % yield). $^1$H NMR (400 MHz, $CD_3SOCD_3$) δ 1.04 (s, 6 H), 1.04-1.24 (m, 3 H), 1.30 (q, *J* = 12 Hz, 2 H), 1.83 (br d, *J* = 11 Hz, 2 H), 1.91 (br d, *J* = 10 Hz, 2 H), 1.96 (dt, *J* = 5, 3 Hz, 4 H), 3.52 (dt, *J* = 7, 3 Hz, 4 H), 3.73 (qt, *J* = 8, 4 Hz, 1 H), 4.03 (s, 1 H), 7.03 (d, *J* = 9 Hz, 1 H), 7.69 (d, *J* = 14 Hz, 1 H), 8.34 (d, *J* = 8 Hz, 1 H), 8.51 (d, *J* = 2 Hz, 1 H), 9.04 (d, *J* = 2 Hz, 1

H); LC-MS (LC-ES) M+H = 400.

**Example 164**

**6-Fluoro-7-(3-fluoroazetidin-1-yl)-N-(*trans*-4-(2-hydroxypropan-2-yl)cyclohexyl)quinoline-3-carboxamide**

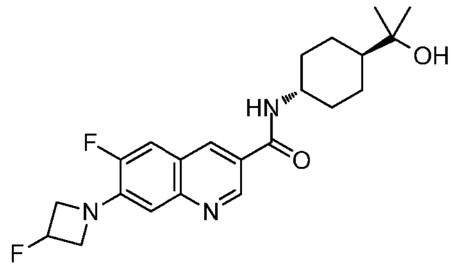

DIEA (0.153 mL, 0.877 mmol) was added to 6-fluoro-7-(3-fluoroazetidin-1-yl)quinoline-3-carboxylic acid ammonia salt (Intermediate 26) (0.0411 g, 0.146 mmol) in DMF (1.461 mL) at RT. Then, 2-(*trans*-4-aminocyclohexyl)propan-2-ol (0.034 g, 0.219 mmol) was added and the reaction mixture was stirred for 5 min. Then, n-propylphosphonic acid anhydride ($T_3P$) (0.174 mL, 0.292 mmol) was added and the reaction mixture was stirred for 16 h. The reaction mixture was poured into saturated sodium bicarbonate, extracted with EtOAc (3X), dried over magnesium sulfate, filtered, and concentrated. The reaction mixture was purified by silica gel chromatography, eluting with MeOH:EtOAc (1:12) to give the title compound (0.0125 g, 20.1 % yield). $^1$H NMR (400 MHz, $CD_3SOCD_3$) $\delta$ 1.04 (s, 6 H), 1.04-1.14 (m, 3 H), 1.30 (br q, *J* = 8 Hz, 2 H), 1.83 (br d, *J* = 12 Hz, 2 H), 1.92 (br d, *J* = 10 Hz, 2 H), 3.36-3.80 (m, 1 H), 4.03 (s, 1 H), 4.12-4.26 (m, 2 H), 4.38-4.52 (m, 2 H), 5.53 (dtt, *J* = 58, 6, 3 Hz, 1 H), 6.98 (d, *J* = 9 Hz, 1 H), 7.74 (d, *J* = 13 Hz, 1 H), 8.40 (d, *J* = 8 Hz, 1 H), 8.56 (d, *J* = 2 Hz, 1 H), 9.08 (d, *J* = 2 Hz, 1 H); LC-MS (LC-ES) M+H = 404.

**Example 165**

**6-Fluoro-7-(3-fluoro-3-methylazetidin-1-yl)-N-(trans-4-(2-hydroxypropan-2-yl)cyclohexyl)quinoline-3-carboxamide**

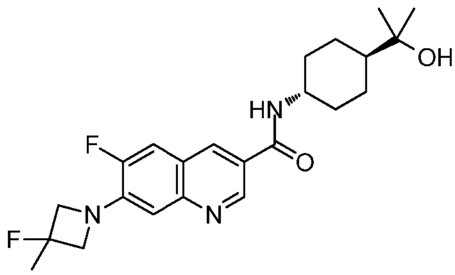

DIEA (0.132 mL, 0.754 mmol) was added to 6-fluoro-7-(3-fluoro-3-methylazetidin-1-yl)quinoline-3-carboxylic acid ammonia salt (Intermediate 27) (0.0371 g, 0.126 mmol) in DMF (1.256 mL) at RT. Then, 2-(*trans*-4-aminocyclohexyl)propan-2-ol (0.030 g, 0.188 mmol) was added and the reaction mixture was stirred for 5 min. Then, n-propylphosphonic acid anhydride ($T_3P$) (0.150 mL, 0.251 mmol) was added and the reaction mixture was stirred for 16 h. The reaction mixture was poured into saturated sodium bicarbonate, extracted with EtOAc (3X), dried over magnesium sulfate, filtered, and concentrated. The reaction mixture was purified by silica gel chromatography, eluting with MeOH:EtOAc to give the title compound (0.0225 g, 40.7 % yield). $^1$H NMR (400 MHz, $CD_3SOCD_3$) $\delta$ 1.04 (s, 6 H), 1.06-1.24 (m, 3 H), 1.30 (br q, *J* = 12 Hz, 2 H), 1.65 (d, *J* = 22 Hz, 3 H), 1.83 (br d, *J* = 12 Hz, 2 H), 1.92 (br d, *J* = 10 Hz, 2 H), 3.66-3.78 (m, 1 H), 4.03 (s, 1 H), 4.16-4.30 (m, 4 H), 6.98 (d, *J* = 9 Hz, 1 H), 7.74 (d, *J* = 13 Hz, 1 H), 8.40 (d, *J* = 8 Hz, 1 H), 8.56 (d, *J* = 2 Hz, 1 H), 9.08 (d, *J* = 2 Hz, 1 H); LC-MS (LC-ES) M+H = 418.

**Example 166**

**(S)-7-(Azetidin-1-yl)-6-chloro-N-(2-oxopyrrolidin-3-yl)quinoline-3-carboxamide trifluoroacetic acid salt**

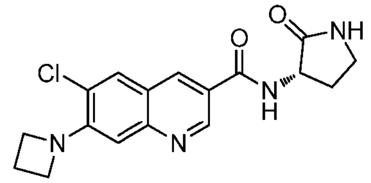

An oven dried 10 mL vial was charged with 7-(azetidin-1-yl)-6-chloroquinoline-3-carboxylic acid (Intermediate 28) (0.051 g, 0.194 mmol) and anhydrous DMF (2 mL). DIEA (0.036 mL, 0.204 mmol) was added *via* syringe. HATU (0.078 g, 0.204 mmol) was added and the mixture was stirred under nitrogen. After 1 h, (S)-3-aminopyrrolidin-2-one (0.021 g, 0.214 mmol) was added in one portion. After 1 h, the solution was poured into sat. $NaHCO_3$ and extracted with EtOAc (3X). Combined organics were washed (water, brine), dried over $Na_2SO_4$, and concentrated *in vacuo.* The residue was purified by preparative HPLC (C18 column, MeCN / water gradient with 0.1 % TFA modifier) affording the title compound (0.046 g, 52 % yield) as a bright yellow solid. $^1H$ NMR (400 MHz, $CD_3SOCD_3$) $\delta$ ppm 1.93 - 2.10 (m, 1 H), 2.26 - 2.46 (m, 3 H), 3.18 - 3.34 (m, 2 H), 4.30 (t, *J* = 7 Hz, 4 H), 4.52 - 4.70 (m, 1 H), 6.87 (s, 1 H), 7.92 (s, 1 H), 8.15 (s, 1 H), 8.80 (d, *J* = 2 Hz, 1 H), 8.98 (d, *J* = 8 Hz, 1 H), 9.19 (d, J = 2 Hz, 1 H). MS (ESI) *m/z* 345 (M+H, parent).

**Example 167**

**7-(Azetidin-1-yl)-6-chloro-N-(3-hydroxy-3-methylcyclobutyl)quinoline-3-carboxamide trifluoroacetic acid salt**

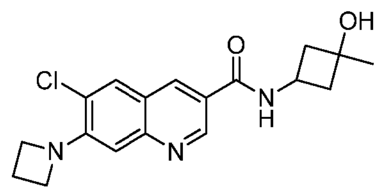

An oven dried 10 mL vial was charged with 7-(azetidin-1-yl)-6-chloroquinoline-3-carboxylic acid (Intermediate 28) (0.100 g, 0.381 mmol), HATU (0.152 g, 0.400 mmol) and anhydrous DMF (4 mL). DIEA (0.070 mL, 0.400 mmol) was added *via* syringe. After 1 h, a solution of 3-amino-1-methylcyclobutanol hydrochloride (Intermediate 51) (0.052 g, 0.381 mmol) and DIEA (0.070 mL, 0.400 mmol) in DMF (1 mL) was added. After stirring overnight, the solution was poured into sat. $NaHCO_3$ and extracted with EtOAc (3X). Combined organics were washed (water, brine), dried over $Na_2SO_4$, and concentrated *in vacuo.* The residue was purified by preparative HPLC (C18 column, MeCN / water gradient with 0.1 % TFA modifier) affording the title compound (0.149 g, 85 % yield) as an orange gum. $^1H$ NMR (400 MHz, $CD_3SOCD_3$) $\delta$ ppm 1.29 (d, *J* = 4 Hz, 3 H), 2.02 - 2.17 (m, 2 H), 2.25 - 2.44 (m, 4 H), 4.02 (dq, *J* = 15, 8 Hz, 1 H), 4.33 (t, *J* = 7 Hz, 4 H), 4.53 (dq, *J* = 15, 8 Hz, 1 H), 6.84 (d, *J* = 2 Hz, 1 H), 8.17 (d, *J* = 2 Hz, 1 H), 8.82 - 8.95 (m, 2 H), 9.20 (dd, *J* = 5, 2 Hz, 1 H). MS (ESI) *m/z* 346 (M+H, parent).

**Example 168**

**7-Azido-6-chloro-N-(trans-4-(2-hydroxypropan-2-yl)cyclohexyl)quinoline-3-carboxamide**

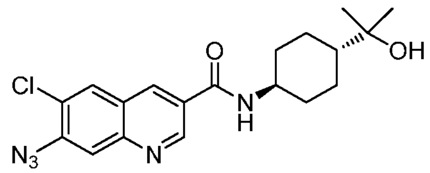

To a solution of 7-azido-6-chloroquinoline-3-carboxylic acid (intermediate 29) (100 mg, 0.40 mmol) in DMF (4 mL) was added DIEA (0.28 mL, 1.61 mmol) and 2-(trans-4-aminocyclohexyl)propan-2-ol (95 mg, 0.60 mmol). After 5 min, n-propylphosphonic acid anhydride ($T_3P$) (0.48 mL, 0.80 mmol) (50% by wt. in EtOAc) was added and the reaction was stirred at RT overnight. The reaction was quenched into sat. sodium bicarbonate and extracted with EtOAc. The combined organics were washed with water and brine, dried over sodium sulfate, and concentrated. The residue was purified by silica gel chromatography (0-60% (3:1 EtOAc:EtOH)-hexanes gradient) to afford the title compound as a white solid (52 mg, 33% yield). $^1$H NMR (400 MHz, $CD_3SOCD_3$) δ ppm 1.01 - 1.05 (m, 6 H), 1.07 - 1.23 (m, 3 H), 1.25 - 1.37 (m, 2 H), 1.83 (d, *J* = 11 Hz, 2 H), 1.90 - 1.99 (m, 2 H), 3.66 - 3.81 (m, 1 H), 4.03 (s, 1 H), 7.97 (s, 1 H), 8.32 (s, 1 H), 8.57 (d, *J* = 8 Hz, 1 H), 8.72 (d, *J* = 2 Hz, 1 H), 9.25 (d, *J* = 2 Hz, 1 H). MS (ESI) *m/z* 388 (M+1).

**Example 169**

**(S)-6-Chloro-7-(3-fluoroazetidin-1-yl)-N-(2-oxopyrrolidin-3-yl)quinoline-3-carboxamide**

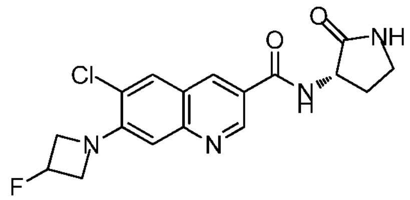

To a solution of 6-chloro-7-(3-fluoroazetidin-1-yl)quinoline-3-carboxylic acid (Intermediate 30) (40 mg, 0.143 mmol) in DMF (10 mL) at RT, was added DIEA (0.100 mL, 0.570 mmol) and (S)-3-aminopyrrolidin-2-one (15.69 mg, 0.157 mmol). Next, n-propylphosphonic acid anhydride ($T_3P$) (50% in EtOAc) (181 mg, 0.285 mmol) was added by a slow dropwise addition and the reaction was allowed to stir at RT for 48 h. Saturated $NaHCO_3$ solution was added to adjust the pH to 8. The mixture was repeatedly extracted with 10% THF/EtOAc until no product was in the organic layer as observed by TLC (10% MeOH/DCM). The combined extracts were absorbed onto silica gel and purified by silica gel chromatography (DCM to 10% MeOH/DCM) to afford the title compound (40 mg, 77% yield). $^1$H NMR (400 MHz, $CD_3SOCD_3$) δ ppm 1.94 - 2.10 (m, 1 H), 2.30 - 2.44 (m, 1 H), 3.21 - 3.29 (m, 2 H), 4.19 - 4.34 (m, 2 H), 4.42 - 4.68 (m, 3 H), 5.35 - 5.61 (m, 1 H), 7.05 (s, 1 H), 7.88 (s, 1 H), 8.08 (s, 1 H), 8.61 (d, *J* =2 Hz, 1 H), 8.89 (d, *J* = 8 Hz, 1 H), 9.14 (d, *J* =2 Hz, 1 H). MS (ESI) *m/z* 363 (M+1).

**Example 170**

**6-Fluoro-N-(*trans*-4-(2-hydroxypropan-2-yl)cyclohexyl)-7-(2-methylazetidin-1-yl)quinoline-3-carboxamide**

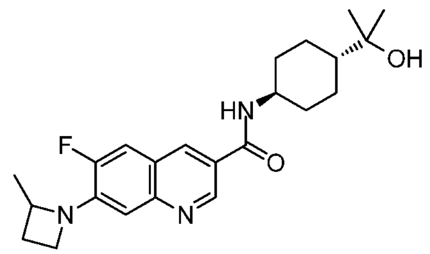

DIEA (0.388 mL, 2.224 mmol) was added to 6-fluoro-7-(2-methylazetidin-1-yl)quinoline-3-carboxylic acid ammonia salt (Intermediate 31) (0.1028 g, 0.371 mmol) in DMF (3.71 mL) at RT. Then, 2-(*trans*-4-aminocyclohexyl)propan-2-ol (0.070 g, 0.445 mmol) was added and the reaction mixture was stirred for 5 min. Then, n-propylphosphonic acid anhydride ($T_3P$) (0.441 mL, 0.741 mmol) was added and the reaction mixture was stirred for 16 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc (1:13) to give the title compound (0.0813 g, 52.1 % yield). $^1$H NMR (400 MHz, $CD_3SOCD_3$) δ 1.04 (s, 6 H), 1.04-1.24 (m, 3 H), 1.30 (br q, *J* = 12 Hz, 2 H), 1.43 (d, *J* = 6 Hz, 3 H), 1.83 (br d, *J* = 11 Hz, 2 H), 1.92 (br d, *J* = 10 Hz, 2 H), 1.96-2.08 (m, 1 H), 2.44-2.56 (m, 1 H), 3.66-3.78 (m, 1 H), 3.90 (q, *J* = 8 Hz, 1 H), 4.03 (s, 1 H), 4.10-4.20 (m, 1 H), 4.46 (h, *J* = 7 Hz, 1 H), 6.93 (d, *J* = 9 Hz, 1 H), 7.70 (d, *J* = 13 Hz, 1 H), 8.38 (d, *J* = 8 Hz, 1 H), 8.54 (d, *J* = 2 Hz, 1 H), 9.06 (d, *J* = 2 Hz, 1 H); LC-MS (LC-ES) M+H = 400.

**Example 171**

**6-Fluoro-7-(2-methylazetidin-1-yl)-N-((S)-2-oxopyrrolidin-3-yl)quinoline-3-carboxamide**

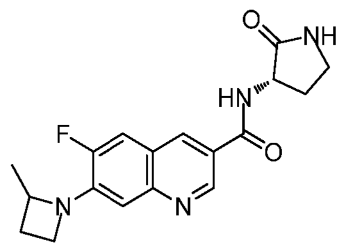

DIEA (0.381 mL, 2.181 mmol) was added to 6-fluoro-7-(2-methylazetidin-1-yl)quinoline-3-carboxylic acid ammonia salt (Intermediate 31) (0.1008 g, 0.364 mmol) in DMF (3.64 mL) at RT. Then, (S)-3-aminopyrrolidin-2-one (0.044 g, 0.436 mmol) was added and the reaction mixture was stirred for 5 min. Then, n-propylphosphonic acid anhydride ($T_3P$) (0.433 mL, 0.727 mmol) was added and the reaction mixture was stirred for 16 h. The reaction mixture was concentrated. The reaction mixture was purified by RP HPLC eluting with acetonitrile:water with 0.1% ammonium hydroxide (5:95-100:0), then further purified by silica gel chromatography, eluting with MeOH:EtOAc (1:3) to give the title compound (0.1170 g, 89 % yield). $^1$H NMR (400 MHz, $CD_3SOCD_3$) δ 1.43 (d, J = 6 Hz, 3 H), 1.96-2.08 (m, 2 H), 2.32-2.44 (m, 1 H), 2.48-2.58 (m, 1 H), 3.20-3.28 (m, 2 H), 3.92 (q, *J* = 8 Hz, 1 H), 4.12-4.20 (m, 1 H), 4.47 (h, *J* = 6 Hz, 1 H), 4.60 (q, *J* = 10 Hz, 1 H), 6.94 (d, *J* = 9 Hz, 1 H), 7.72 (d, *J* = 13 Hz, 1 H), 7.87 (br s, 1 H), 8.58 (d, *J* = 2 Hz, 1 H), 8.85 (d, *J* = 8 Hz, 1 H), 9.09 (d, *J* = 2 Hz, 1 H); LC-MS (LC-ES) M+H = 343.

**Example 172**

**7-Acetyl-6-chloro-N-(trans-4-(2-hydroxvpropan-2-yl)cyclohexyl)quinoline-3-carboxamide**

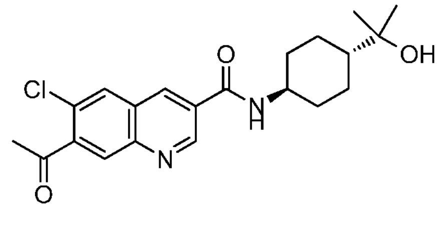

To a solution of 7-acetyl-6-chloroquinoline-3-carboxylic acid (Intermediate 32) (120 mg, 0.48 mmol) in DMF (4 mL) was added DIEA (0.25 mL, 1.44 mmol) and HATU (219 mg, 0.58 mmol). After stirring for 10 min 2-(*trans*-4-aminocyclohexyl)propan-2-ol (113 mg, 0.721 mmol) was added. The reaction was stirred at RT overnight, quenched into water and extracted with EtOAc. The combined organics were dried over sodium sulfate, filtered, and concentrated. The residue was purified by silica gel chromatography (0-65% (3:1 EtOAc:EtOH)-hexanes gradient) to give a yellow solid which was triturated with DCM to afford the title compound as a white solid (112 mg, 60% yield). $^1$H NMR (400 MHz, $CD_3SOCD_3$)

δ ppm 1.04 (s, 6 H), 1.07 - 1.22 (m, 3 H), 1.26 - 1.38 (m, 2 H), 1.84 (d, *J* = 11 Hz, 2 H), 1.95 (d, *J* = 10 Hz, 2 H), 2.70 (s, 3 H), 3.65 - 3.84 (m, 1 H), 4.04 (s, 1 H), 8.32 (s, 1 H), 8.41 (s, 1 H), 8.65 (d, *J* = 8 Hz, 1 H), 8.78 (d, *J* = 2 Hz, 1 H), 9.31 (d, *J* = 2 Hz, 1 H). MS (ESI) *m/z* 389 (M+1).

## Example 173

### 7-Amino-6-fluoro-N-(*trans*-4-(2-hydroxypropan-2-yl)cyclohexyl)quinoline-3-carboxamide

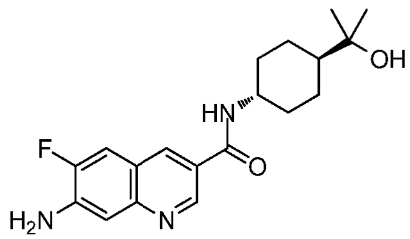

### A. Ethyl 7-azido-6-fluoroquinoline-3-carboxylate

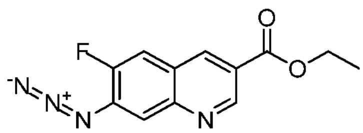

Sodium azide (0.275 g, 4.23 mmol) was added to ethyl 6,7-difluoroquinoline-3-carboxylate (Intermediate 26, step B) (0.5021 g, 2.117 mmol) in dimethyl sulfoxide (5.29 mL) at RT and the reaction mixture was heated at 100 °C in the microwave for 1 h. The reaction mixture was dissolved in DCM, washed with water, dried over magnesium sulfate, filtered, and concentrated. The residue was purified by silica gel chromatography, eluting with EtOAc:hexanes (1:4) to give the title compound (0.4914 g, 85 % yield). $^1$H NMR (400 MHz, $CD_3SOCD_3$) δ 1.37 (t, *J* = 7 Hz, 3 H), 4.40 (q, *J* = 7 Hz, 2 H), 7.95 (d, *J* = 8 Hz, 1 H), 8.16 (d, *J* = 11 Hz, 1 H), 8.96 (d, *J* = 2 Hz, 1 H), 9.27 (d, *J* = 2 Hz, 1 H); LC-MS (LC-ES) M+H = 261.

### B. 7-Azido-6-fluoroquinoline-3-carboxylic acid ammonia salt

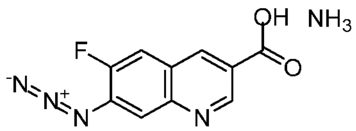

Lithium hydroxide (0.029 g, 1.200 mmol) was added to ethyl 7-azido-6-fluoroquinoline-3-carboxylate (0.1041 g, 0.400 mmol) in MeOH (3.56 mL) and water (0.444 mL) at RT and the reaction mixture was stirred for 1 h at 60 °C. The reaction mixture was concentrated. The reaction mixture was purified by RP HPLC eluting with acetonitrile:water with 0.1 % ammonium hydroxide (5:95-100:0) to give the title compound (0.0935 g, 89 % yield). $^1$H NMR (400 MHz, $CD_3SOCD_3$) δ 7.81 (d, *J* = 8Hz, 1H), 7.97 (d, *J* = 12 Hz, 1 H), 8.61 (br s, 1 H), 9.25 (d, *J* = 2 Hz, 1 H); LC-MS (LC-ES) M-H = 231.

### C. 7-Azido-6-fluoro-N-(*trans*-4-(2-hydroxypropan-2-yl)cyclohexyl)quinoline-3-carboxamide

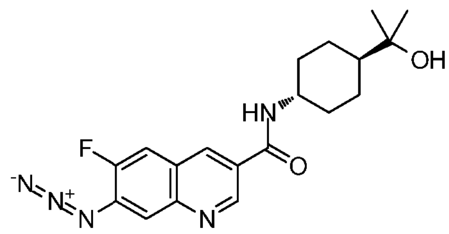

2-(trans-4-Aminocyclohexyl)propan-2-ol (0.068 g, 0.431 mmol) was added to 7-azido-6-fluoroquinoline-3-carboxylic acid ammonia salt (0.0895 g, 0.359 mmol) in DMF (3.59 mL) at RT. Then, DIEA (0.376 mL, 2.155 mmol) was added and the reaction mixture was stirred for 5 min. Then, n-propylphosphonic acid anhydride ($T_3P$) (0.428 mL, 0.718 mmol) was added and the reaction mixture was stirred for 16 h. The reaction mixture was concentrated. The reaction mixture was purified by RP HPLC, eluting with acetonitrile:water with 0.1% ammonium hydroxide (5:95-100:0), then further purified by silica gel chromatography, eluting with EtOAc to give the title compound (0.0675 g, 48.1 % yield). $^1H$ NMR (400 MHz, $CD_3SOCD_3$) δ 1.04 (s, 6 H), 1.04-1.24 (m, 3 H), 1.31 (br q, *J* = 12 Hz, 2 H), 1.84 (br d, *J* = 12 Hz, 2 H), 1.94 (br d, *J* = 10 Hz, 2 H), 3.66-3.82 (m, 1 H), 4.04 (s, 1 H), 7.92 (d, *J* = 8 Hz, 1 H), 8.03 (d, *J* = 12 Hz, 1 H), 8.57 (d, *J* = 8 Hz, 1 H), 8.73 (d, *J* = 2 Hz, 1 H), 9.22 (d, *J* = 2 Hz, 1 H); LC-MS (LC-ES) M+H = 372.

**D. 7-Amino-6-fluoro-N-(*trans*-4-(2-hydroxypropan-2-yl)cyclohexyl)quinoline-3-carboxamide**

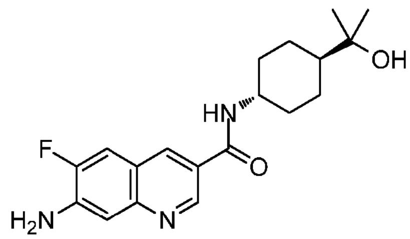

Triphenylphosphine (0.120 g, 0.457 mmol) was added to 7-azido-6-fluoro-N-(*trans*-4-(2-hydroxypropan-2-yl)cyclohexyl)quinoline-3-carboxamide (0.0566 g, 0.152 mmol) in THF (1.451 mL) and water (0.073 mL) at RT and the reaction mixture was stirred for 16 h at RT, then 24 h at 65 °C. The reaction mixture was concentrated. The reaction mixture was purified by RP HPLC eluting with acetonitrile:water with 0.1% ammonium hydroxide (5:95-100:0) to give the title compound (0.0213 g, 38.4 % yield). $^1H$ NMR (400 MHz, $CD_3SOCD_3$) δ 1.04 (s, 6 H), 1.04-1.24 (m, 3 H), 1.30 (br q, *J* = 12 Hz, 2 H), 1.83 (br d, *J* = 11 Hz, 2 H), 1.91 (br d, *J* = 10 Hz, 2 H), 3.66-3.78 (m, 1 H), 4.03 (s, 1 H), 6.16 (br s, 2 H), 7.16 (d, *J* = 9 Hz, 1 H), 7.63 (d, *J* = 12 Hz, 1 H), 8.31 (d, *J* = 8 Hz, 1 H), 8.48 (d, *J* = 2 Hz, 1 H), 8.99 (d, *J* = 2 Hz, 1 H); LC-MS (LC-ES) M+H = 346.

**Example 174 and 175**

**6-Fluoro-N-(trans-4-(2-hydroxypropan-2-yl)cyclohexyl)-7-((S)-2-methylazetidin-1-yl)quinoline-3-carboxamide and 6-Fluoro-N-(trans-4-(2-hydroxypropan-2-yl)cyclohexyl)-7-((R)-2-methylazetidin-1-yl)quinoline-3-carboxamide**

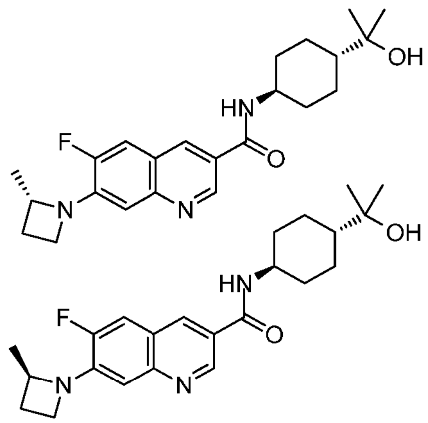

Racemic 6-fluoro-N-(trans-4-(2-hydroxypropan-2-yl)cyclohexyl)-7-(2-methylazetidin-1-yl)quinoline-3-carboxamide (Example 170) (0.0763 g, 0.191 mmol) was separated into its enantiomers on a chiral OZ column eluting with ethanol:hexanes (7:3) to give 6-fluoro-N-(trans-4-(2-hydroxypropan-2-yl)cyclohexyl)-7-((S)-2-methylazetidin-1-yl)quinoline-3-carboxamide (0.0383 g, 47.7 % yield) as the first enantiomer to elute and 6-fluoro-N-(trans-4-(2-hydroxypropan-2-yl)cyclohexyl)-7-((R)-2-methylazetidin-1-yl)quinoline-3-carboxamide (0.0375 g, 46.7 % yield) as the last enantiomer to elute. The structures were assigned by vibrational circular dichroism.

**6-Fluoro-N-(trans-4-(2-hydroxypropan-2-yl)cyclohexyl)-7-((S)-2-methylazetidin-1-yl)quinoline-3-carboxamide:** $^1$H NMR (400 MHz, $CD_3SOCD_3$) δ 1.04 (s, 6 H), 1.04-1.24 (m, 3 H), 1.30 (br q, *J* = 12 Hz, 2 H), 1.43 (d, *J* = 6 Hz, 3 H), 1.83 (br d, *J* = 11 Hz, 2 H), 1.92 (br d, *J* = 10 Hz, 2 H), 1.96-2.08 (m, 1 H), 2.44-2.56 (m, 1 H), 3.66-3.78 (m, 1 H), 3.90 (q, *J* = 8 Hz, 1 H), 4.03 (s, 1 H), 4.10-4.20 (m, 1 H), 4.46 (h, *J* = 7 Hz, 1 H), 6.93 (d, *J* = 9 Hz, 1 H), 7.70 (d, *J* = 13 Hz, 1 H), 8.38 (d, *J* = 8 Hz, 1 H), 8.54 (d, *J* = 2 Hz, 1 H), 9.06 (d, *J* = 2 Hz, 1 H); LC-MS (LC-ES) M+H = 400.

**6-Fluoro-N-(trans-4-(2-hydroxypropan-2-yl)cyclohexyl)-7-((R)-2-methylazetidin-1-yl)quinoline-3-carboxamide:** $^1$H NMR (400 MHz, $CD_3SOCD_3$) δ 1.04 (s, 6 H), 1.04-1.24 (m, 3 H), 1.30 (br q, J = 12 Hz, 2 H), 1.43 (d, *J* = 6 Hz, 3 H), 1.83 (br d, J = 11 Hz, 2 H), 1.92 (br d, J = 10 Hz, 2 H), 1.96-2.08 (m, 1 H), 2.44-2.56 (m, 1 H), 3.66-3.78 (m, 1 H), 3.90 (q, J = 8 Hz, 1 H), 4.03 (s, 1 H), 4.10-4.20 (m, 1 H), 4.46 (h, J = 7 Hz, 1 H), 6.93 (d, J = 9 Hz, 1 H), 7.70 (d, J = 13 Hz, 1 H), 8.38 (d, J = 8 Hz, 1 H), 8.54 (d, J = 2 Hz, 1 H), 9.06 (d, J = 2 Hz, 1 H); LC-MS (LC-ES) M+H = 400.

## Example 176

### 6-Fluoro-7-((S)-2-methylazetidin-1-yl)-N-((S)-2-oxopyrrolidin-3-yl)quinoline-3-carboxamide

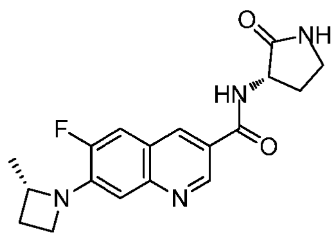

Diastereomeric 6-fluoro-7-(2-methylazetidin-1-yl)-N-((S)-2-oxopyrrolidin-3-yl)quinoline-3-carboxamide (Example 171) (0.1072 g, 0.313 mmol) was separated into its diastereomers on a chiral Cell2/OZ column eluting with ethanol:hexanes (2:1) to give 6-fluoro-7-((S)-2-methylazetidin-1-yl)-N-((S)-2-oxopyrrolidin-3-yl)quinoline-3-carboxamide (0.0464 g, 41.1 % yield) as the first diastereomer to elute. The structure were assigned by vibrational circular dichroism. $^1$H NMR (400 MHz, $CD_3SOCD_3$) δ 1.43 (d, *J* = 6 Hz, 3 H), 1.96-2.08 (m, 2 H), 2.32-2.44 (m, 1 H), 2.48-2.58 (m, 1 H), 3.20-3.28 (m, 2 H), 3.92 (q, *J* = 8 Hz, 1 H), 4.12-4.20 (m, 1 H), 4.47 (h, *J* = 6 Hz, 1 H), 4.60 *(q, J* = 10 Hz, 1 H), 6.94 (d, *J* = 9 Hz, 1 H), 7.72 *(d, J* = 13 Hz, 1 H), 7.87 (br s, 1 H), 8.58 (d, *J* = 2 Hz, 1 H), 8.85 (d, *J* = 8 Hz, 1 H), 9.09 (d, *J* = 2 Hz, 1 H); LC-MS (LC-ES) M+H = 343.

**Example 177**

**7-(3,3-Difluoroazetidin-1-yl)-6-fluoro-N-(*trans*-4-(2-hydroxypropan-2-yl)cyclohexyl)quinoline-3-carboxamide**

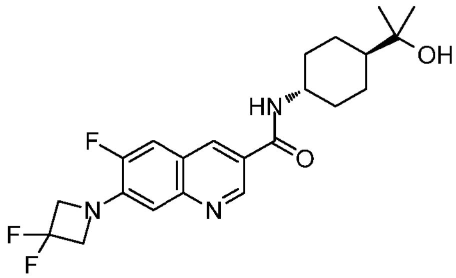

2-(trans-4-Aminocyclohexyl)propan-2-ol (0.020 g, 0.128 mmol) was added to 7-(3,3-difluoroazetidin-1-yl)-6-fluoroquinoline-3-carboxylic acid trifluoroacetate (Intermediate 33) (0.0422 g, 0.107 mmol) in DMF (1.068 mL) at RT. Then, DIEA (0.112 mL, 0.641 mmol) was added and the reaction mixture was stirred for 5 min. Then, n-propylphosphonic acid anhydride ($T_3P$) (0.127 mL, 0.214 mmol) was added and the reaction mixture was stirred for 16 h. The reaction mixture was concentrated. The reaction mixture was purified by RP HPLC eluting with acetonitrile:water with 0.1% ammonium hydroxide (5:95-100:0), then furthered purified by silica gel chromatography, eluting with MeOH:EtOAc (1:99) to give the title compound (0.0143 g, 30.2 % yield). $^1$H NMR (400 MHz, $CD_3SOCD_3$) $\delta$ 1.04 (s, 6 H), 1.04-1.26 (m, 3 H), 1.31 (br q, *J* = 12 Hz, 2 H), 1.83 (br d, *J* = 11 Hz, 2 H), 1.92 (br d, *J* = 10 Hz, 2 H), 3.66-3.78 (m, 1 H), 4.03 (s, 1 H), 4.57 (dt, *J* = 13, 2 Hz, 4 H), 7.12 (d, *J* = 9 Hz, 1 H), 7.81 (d, *J* = 13 Hz, 1 H), 8.43 (d, *J* = 8 Hz, 1 H), 8.60 (d, *J* = 2 Hz, 1 H), 9.11 (d, *J* = 2 Hz, 1 H); LC-MS (LC-ES) M+H = 422.

**Example 178**

**7-Ethoxy-6-fluoro-N-(*trans*-4-(2-hydroxypropan-2-yl)cyclohexyl)quinoline-3-carboxamide**

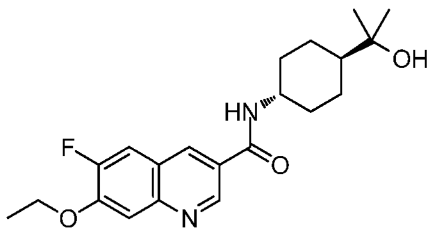

2-(trans-4-Aminocyclohexyl)propan-2-ol (0.051 g, 0.324 mmol) was added to 7-ethoxy-6-fluoroquinoline-3-carboxylic acid ammonia salt (Intermediate 34) (0.0682 g, 0.270 mmol) in DMF (2.70 mL) at RT. Then, DIEA (0.283 mL, 1.622 mmol) was added and the reaction mixture was stirred for 5 min. Then, n-propylphosphonic acid anhydride ($T_3P$) (0.322 mL, 0.541 mmol) was added and the reaction mixture was stirred for 2 h. The reaction mixture was concentrated. The reaction mixture was purified by RP HPLC eluting with acetonitrile:water with 0.1% ammonium hydroxide (5:95-100:0), then furthered purified by silica gel chromatography, eluting with MeOH:EtOAc (1:24) to give the title compound (0.0497 g, 46.6 % yield). $^1$H NMR (400 MHz, $CD_3SOCD_3$) $\delta$ 1.04 (s, 6 H), 1.04-1.24 (m, 3 H), 1.31 (br q, *J* = 12 Hz, 2 H), 1.43 (t, *J* = 7 Hz, 3 H), 1.83 (br d, *J* = 12 Hz, 2 H), 1.93 (br d, *J* = 10 Hz, 2 H), 3.66-3.80 (m, 1 H), 4.04 (s, 1 H), 4.30 (q, *J* = 7 Hz, 2 H), 7.61 (d, *J* = 8 Hz, 1 H), 7.90 (d, *J* = 12 Hz, 1 H), 8.49 (d, *J* = 8 Hz, 1 H), 8.67 (d, *J* = 2 Hz, 1 H), 9.16 (d, *J* = 2 Hz, 1 H); LC-MS (LC-ES) M+H = 375.

**Example 179**

**6-Chloro-7-cyclobutyl-N-(trans-4-(2-hydroxvpropan-yl)cyclohexyl)quinoline-3-carboxamide**

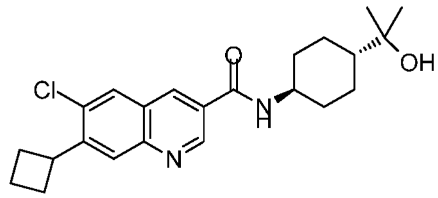

To a solution of 6-chloro-7-cyclobutylquinoline-3-carboxylic acid (Intermediate 35) (20 mg, 0.076 mmol) in DMF (10 mL) at RT, was added DIEA (0.053 mL, 0.306 mmol) and 2-(trans-4-aminocyclohexyl)propan-2-ol (14.42 mg, 0.092 mmol). Next, n-propylphosphonic acid anhydride ($T_3P$) (50% in EtOAc) (97 mg, 0.153 mmol) was added by a slow dropwise addition and the reaction was allowed to stir at RT overnight. The solvent was removed and saturated $NaHCO_3$ solution was added (10 mL). The mixture was repeatedly extracted with 10% THF/EtOAc until no product was in the organic layer as observed by TLC (10% MeOH/DCM). The combined extracts were absorbed onto silica gel and purified by silica gel chromatography (DCM to 10% MeOH/DCM) to afford the title compound (15 mg, 49% yield). $^1H$ NMR (400 MHz, $CD_3SOCD_3$) $\delta$ ppm 1.03 (s, 6 H), 1.07 - 1.22 (m, 4 H), 1.26 - 1.38 (m, 2 H), 1.79 - 1.88 (m, 2 H), 1.92- 1.94 (m, 2 H), 2.01 - 2.12 (m, 1 H), 2.18- 2.28 (m, 2 H), 2.40 - 2.47 (m, 2 H), 3.70-3.78 (m, 1 H), 3.81 - 3.90 (m, 1 H), 4.03 (s, 1 H), 7.97 (s, 1 H), 8.18 (s, 1 H), 8.56 (d, $J$ = 8 Hz, 1 H), 8.71 (d, $J$ = 2 Hz, 1 H), 9.24 (d, $J$ = 2 Hz, 1 H). MS (ESI) $m/z$ 401 (M+1).

## Example 180

### 6-Chloro-7-cyclobutyl-N-((1s,3s)-3-hydroxy-3-methylcyclobutyl)quinoline-3-carboxamide

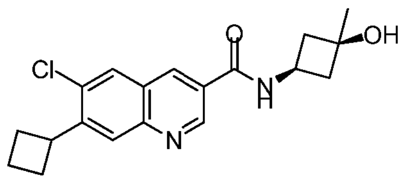

To a solution of 6-chloro-7-cyclobutylquinoline-3-carboxylic acid (Intermediate 35) (50 mg, 0.191 mmol) in DMF (10 mL) at RT, was added DIEA (0.200 mL, 1.146 mmol) and 3-amino-1-methylcyclobutanol hydrochloride (Intermediate 51) (26.3 mg, 0.191 mmol). Next, n-propylphosphonic acid anhydride ($T_3P$) (50% in EtOAc) (243 mg, 0.382 mmol) was added by a slow dropwise addition and the reaction was allowed to stir at RT overnight. The solvent was removed and saturated $NaHCO_3$ solution was added (10 mL). The mixture was repeatedly extracted with 10% THF/EtOAc until no product was in the organic layer as observed by TLC (10% MeOH/DCM). The combined extracts were absorbed onto Celite® and purified by reverse phase chromatography (10-100% acetonitrile(with 0.1% TFA)/ water(with 0.1% TFA)) to afford the title compound (30 mg, 34%). $^1H$ NMR (400 MHz, $CD_3SOCD_3$) $\delta$ ppm 1.30 (s, 3 H), 1.81 - 1.92 (m, 1 H), 2.04 - 2.18 (m, 3 H), 2.24 (m, 2 H), 2.31 - 2.40 (m, 2 H), 2.43 - 2.50 (m, 3 H), 3.88 (m, 1 H), 3.99 - 4.10 (m, 1 H), 8.00 (s, 1 H), 8.20 (s, 1 H), 8.77 (d, $J$ = 2 Hz, 1 H), 8.96 (d, $J$ = 7 Hz, 1 H), 9.28 (d, $J$ = 2 Hz, 1 H). MS (ESI) $m/z$ 345 (M+1).

## Example 181

### (S)-6-Chloro-7-cyclobutyl-N-(2-oxopyrrolidin-3-yl)quinoline-3-carboxamide

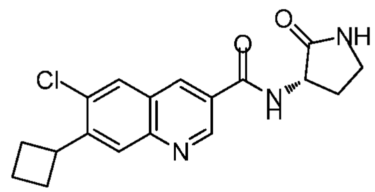

To a solution of 6-chloro-7-cyclobutylquinoline-3-carboxylic acid (Intermediate 35) (20 mg, 0.076 mmol) in DMF

(10 mL) at RT, was added DIEA (0.080 mL, 0.459 mmol) and (S)-3-aminopyrrolidin-2-one (7.65 mg, 0.076 mmol). Next, n-propylphosphonic acid anhydride ($T_3P$) (50% in EtOAc) (97 mg, 0.153 mmol) was added by a slow dropwise addition and the reaction was allowed to stir at RT overnight. The solvent was removed and saturated $NaHCO_3$ solution was added (10 mL). The mixture was repeatedly extracted with 10% THF/EtOAc until no product was in the organic layer as observed by TLC (10% MeOH/DCM). The combined extracts were absorbed onto silica gel and purified by silica gel chromatography (DCM to 10% MeOH/DCM) to afford the title compound (18 mg, 69% yield). $^1$H NMR (400 MHz, $CD_3SOCD_3$) $\delta$ ppm 1.81 - 1.92 (m, 1 H), 2.00 - 2.15 (m, 2 H), 2.20 - 2.32 (m, 2 H), 2.37 - 2.49 (m, 3 H), 3.27 (d, *J* = 12 Hz, 2 H), 3.84 - 3.94 (m, 1 H), 4.59 - 4.69 (m, 1 H), 7.92 (s, 1 H), 8.01 (s, 1 H), 8.23 (s, 1 H), 8.78 (d, *J* = 2 Hz, 1 H), 9.07 (d, *J* = 8 Hz, 1 H), 9.29 (d, *J* = 2 Hz, 1 H). MS (ESI) *m/z* 344 (M+1).

**Example 182**

**6-Chloro-7-(2-methylazetidin-1-yl)-N-((S)-2-oxopyrrolidin-3-yl)quinoline-3-carboxamide**

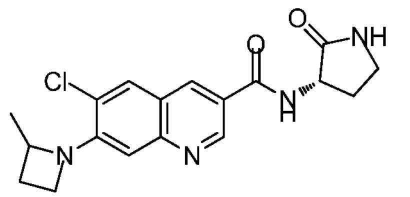

To a solution of 6-chloro-7-(2-methylazetidin-1-yl)quinoline-3-carboxylic acid (Intermediate 36) (50 mg, 0.181 mmol) in DMF (10 mL) at RT, was added DIEA (0.126 mL, 0.773 mmol) and (S)-3-aminopyrrolidin-2-one (19.90 mg, 0.0.199 mmol). Next, n-propylphosphonic acid anhydride ($T_3P$) (50% in EtOAc) (230 mg, 0.361 mmol) was added by a slow dropwise addition and the reaction was allowed to stir at RT for 48 h. Saturated $NaHCO_3$ solution was added to adjust the pH to 8. The mixture was repeatedly extracted with 10% THF/EtOAc until no product was in the organic layer as observed by TLC (10% MeOH/DCM). The combined extracts were absorbed onto silica gel and purified by silica gel chromatography (DCM to 10% MeOH/DCM) to afford the title compound (45 mg, 69%). $^1$H NMR (400 MHz, $CD_3SOCD_3$) $\delta$ ppm 1.37 (d, *J* = 6 Hz, 3 H), 1.91 - 2.09 (m, 2 H), 2.30 - 2.50 (m, 2 H), 3.20 - 3.27 (m, 2 H), 3.80 - 3.90 (m, 1 H), 4.38 (m, 1 H), 4.51 - 4.66 (m, 2 H), 7.05 (s, 1 H), 7.88 (s, 1 H), 8.05 (s, 1 H), 8.60 (d, *J* = 2 Hz, 1 H), 8.88 (d, *J* = 8 Hz, 1 H), 9.13 (d, *J* = 2 Hz, 1 H). MS (ESI) *m/z* 359 (M+1).

**Example 183**

**6-Fluoro-N-(*trans*-4-(2-hydroxypropan-2-yl)cyclohexyl)-7-(3-methylazetidin-1-yl)quinoline-3-carboxamide**

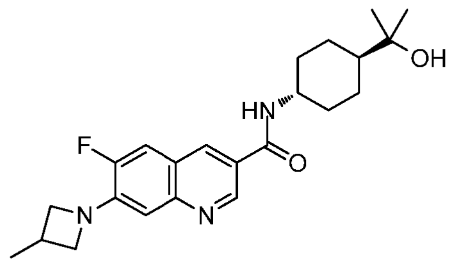

2-(trans-4-Aminocyclohexyl)propan-2-ol (0.049 g, 0.313 mmol) was added to 6-fluoro-7-(3-methylazetidin-1-yl)quinoline-3-carboxylic acid ammonia salt (Intermediate 37) (0.0724 g, 0.261 mmol) in DMF (2.61 mL) at RT. Then, DIEA (0.274 mL, 1.567 mmol) was added and the reaction mixture was stirred for 5 min. Then, n-propylphosphonic acid anhydride ($T_3P$) (0.311 mL, 0.522 mmol) was added and the reaction mixture was stirred for 16 h. The reaction mixture was concentrated. The reaction mixture was purified by RP HPLC eluting with acetonitrile:water with 0.1% ammonium hydroxide (5:95-100:0), then furthered purified by silica gel chromatography, eluting with MeOH:EtOAc (1:19) to the title compound (0.0919 g, 84 % yield). $^1$H NMR (400 MHz, $CD_3SOCD_3$) $\delta$ 1.04 (s, 6 H), 1.04-1.24 (m, 3 H), 1.26 (d, *J* = 7

Hz, 3 H), 1.30 (q, *J* = 12 Hz, 2 H), 1.83 (br d, *J* = 12 Hz, 2 H), 1.91 (br d, *J* = 10 Hz, 2 H), 2.84 (o, *J* = 7 Hz, 1 H), 3.69 (t, *J* = 6 Hz, 2 H), 3.66-3.78 (m, 1 H), 4.03 (s, 1 H), 4.24 (dt, *J* = 8, 2 Hz, 2 H), 6.84 (d, *J* = 9 Hz, 1 H), 7.68 *(d, J* = 13 Hz, 1 H), 8.36 (d, *J* = 8 Hz, 1 H), 8.53 (d, *J* = 2 Hz, 1 H), 9.05 (d, *J* = 2 Hz, 1 H); LC-MS (LC-ES) M+H = 400.

**Example 184**

**7-((Difluoromethyl)thio)-6-fluoro-N-(*trans*-4-(2-hydroxypropan-2-yl)cyclohexyl)quinoline-3-carboxamide**

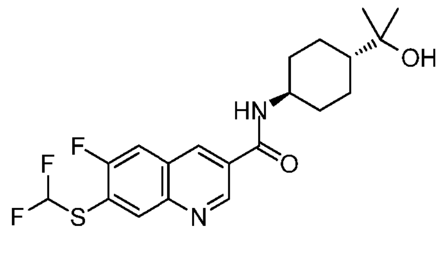

DIEA (0.136 mL, 0.776 mmol) was added to 7-((difluoromethyl)thio)-6-fluoroquinoline-3-carboxylic acid ammonia salt (Intermediate 38) (0.0563 g, 0.194 mmol) in DMF (0.647 mL) at RT. Then, 2-(trans-4-aminocyclohexyl)propan-2-ol (0.040 g, 0.252 mmol) was added and the reaction mixture was stirred for 5 min. Then, n-propylphosphonic acid anhydride ($T_3P$) (0.231 mL, 0.388 mmol) was added and the reaction mixture was stirred for 16 h. The reaction mixture was concentrated. The reaction mixture was purified by RP HPLC eluting with acetonitrile:water with 0.1% ammonium hydroxide (5:95-100:0), then further purified by silica gel chromatography, eluting with EtOAc:hexanes (9:1) to give the title compound (0.0615 g, 73.0 % yield). $^1$H NMR (400 MHz, $CD_3SOCD_3$) $\delta$ 1.04 (s, 6 H), 1.04-1.26 (m, 3 H), 1.32 (q, *J* = 12 Hz, 2 H), 1.84 (br d, *J* = 11 Hz, 2 H), 1.95 (br d, *J* = 10 Hz, 2 H), 3.68-3.84 (m, 1 H), 4.04 (s, 1 H), 7.71 (t, *J* = 55 Hz, 1 H), 8.10 (d, *J* = 9 Hz, 1 H), 8.37 (d, *J* = 7 Hz, 1 H), 8.66 (d, *J* = 8 Hz, 1 H), 8.80 (d, *J* = 2 Hz, 1 H), 9.27 (d, *J* = 2 Hz, 1 H); LC-MS (LC-ES) M+H = 413.

**Example 185**

**7-((Difluoromethyl)sulfinyl)-6-fluoro-N-(*trans*-4-(2-hydroxypropan-2-yl)cyclohexyl)quinoline-3-carboxamide**

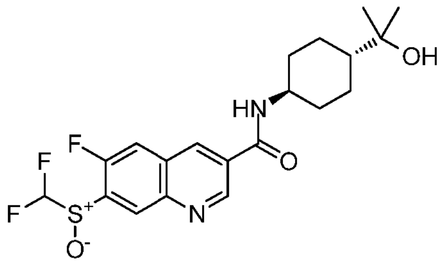

meta-Chloroperoxybenzoic acid (0.040 g, 0.178 mmol) was added to 7-((difluoromethyl)thio)-6-fluoro-N-(*trans*-4-(2-hydroxypropan-2-yl)cyclohexyl)quinoline-3-carboxamide (Example 184) (0.0565 g, 0.137 mmol) in DCM (13.70 mL) at RT. Then, the reaction mixture was stirred for 16 h. The reaction mixture was concentrated. The reaction mixture was purified by RP HPLC eluting with acetonitrile:water with 0.1% ammonium hydroxide (5:95-100:0), then further purified by RP HPLC eluting with acetonitrile:water with 0.1% ammonium hydroxide (20:80:60:40) to give the title compound (0.0205 g, 33.2 % yield). $^1$H NMR (400 MHz, $CD_3SOCD_3$) $\delta$ 1.05 (s, 6 H), 1.06-1.26 (m, 3 H), 1.33 (q, *J* = 12 Hz, 2 H), 1.85 (br d, *J* = 11 Hz, 2 H), 1.96 (br d, *J* = 10 Hz, 2 H), 3.70-3.84 (m, 1 H), 4.05 (s, 1 H), 7.27 (t, *J* = 53 Hz, 1 H), 8.20 *(d, J* = 10 Hz, 1 H), 8.40 (d, *J* = 6 Hz, 1 H), 8.71 (d, *J* = 8 Hz, 1 H), 8.88 (d, *J* = 2 Hz, 1 H), 9.34 (d, *J* = 2 Hz, 1 H); LC-MS (LC-ES) M+H = 429.

**Example 186**

**N-(trans-4-(2-Hydroxypropan-2-yl)cyclohexyl)-7-methylquinoline-3-carboxamide**

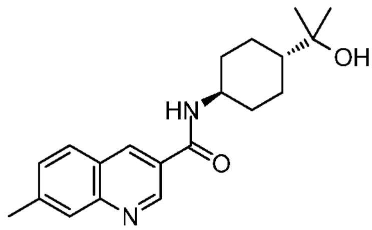

DIEA (0.391 mL, 2.240 mmol) was added to 7-methylquinoline-3-carboxylic acid (0.0699 g, 0.373 mmol) in 1,4-dioxane (1.245 mL) at RT. Then, 2-(trans-4-aminocyclohexyl)propan-2-ol (0.065 g, 0.411 mmol) was added and the reaction mixture was stirred for 5 min. Then, n-propylphosphonic acid anhydride ($T_3P$) (0.445 mL, 0.747 mmol) was added and the reaction mixture was stirred for 44 h. The reaction mixture was poured into saturated sodium bicarbonate, extracted with ethyl acetate (3X), dried over magnesium sulfate, filtered, and concentrated. The residue was purified by silica gel chromatography, eluting with MeOH:EtOAc (1:24) to give the title compound (0.0937 g, 73.0 % yield). $^1H$ NMR (400 MHz, $CD_3SOCD_3$) $\delta$ 1.04 (s, 6 H), 1.04-1.24 (m, 3 H), 1.32 (q, *J* = 12 Hz, 2 H), 1.84 (br d, *J* = 12 Hz, 2 H), 1.94 (br d, *J* = 11 Hz, 2 H), 2.54 (s, 3 H), 3.75 (qt, *J* = 8, 4 Hz, 1 H), 4.04 (s, 1 H), 7.52 (d, *J* = 8 Hz, 1 H), 7.85 (s, 1 H), 7.97 (d, *J* = 8 Hz, 1 H), 8.50 (d, *J* = 8 Hz, 1 H), 8.72 (s, 1 H), 9.20 (s, 1 H); LC-MS (LC-ES) M+H = 327.

**Example 187**

**6-Chloro-N-(trans-4-(2-hydroxypropan-2-yl)cyclohexyl-7-((S)-2-methylazetidin-1-yl)quinoline-3-carboxamide**

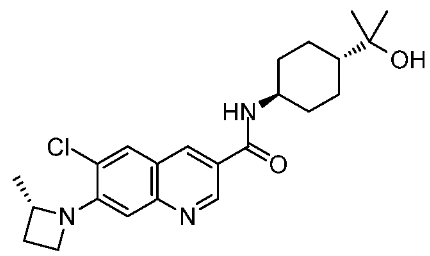

DIEA (0.159 mL, 0.909 mmol) was added to (S)-6-chloro-7-(2-methylazetidin-1-yl)quinoline-3-carboxylic acid ammonia salt (Intermediate 81) (0.0445 g, 0.151 mmol) in 1,4-dioxane (1.136 mL) and DMF (0.379 mL) at RT. Then, 2-(trans-4-aminocyclohexyl)propan-2-ol (0.026 g, 0.167 mmol) was added and the reaction mixture was stirred for 5 min. Then, n-propylphosphonic acid anhydride ($T_3P$) (0.180 mL, 0.303 mmol) was added and the reaction mixture was stirred for 16 hours. The reaction mixture was poured into saturated sodium bicarbonate, extracted with ethyl acetate (3X), dried over magnesium sulfate, filtered, and concentrated. The residue was purified by silica gel chromatography, eluting with MeOH:EtOAc (1:19) to give the title compound (0.0360 g, 54.3 % yield). $^1H$ NMR (400 MHz, $CD_3SOCD_3$) $\delta$ 1.04 (s, 6 H), 1.04-1.24 (m, 3 H), 1.31 (q, *J* = 12 Hz, 2 H), 1.37 (d, *J* = 6 Hz, 3 H), 1.83 (br d, *J* = 11 Hz, 2 H), 1.92 (br d, *J* = 10 Hz, 2 H), 1.92-2.02 (m, 1 H), 2.42-2.52 (m, 1 H), 3.73 (qt, *J* = 8, 4 Hz, 1 H), 3.83 (q, *J* = 8 Hz, 1 H), 4.03 (s, 1 H), 4.34-4.44 (m, 1 H), 4.54 (h, *J* = 6 Hz, 1 H), 7.05 (s, 1 H), 8.04 (s, 1 H), 8.40 (d, *J* = 8 Hz, 1 H), 8.56 (d, *J* = 2 Hz, 1 H), 9.11 (d, *J* = 2 Hz, 1 H); LC-MS (LC-ES) M+H = 416.

**Example 188**

**6-Chloro-7-((S)-2-methylazetidin-1-yl)-N-((S)-2-oxopyrrolidin-3-yl)quinoline-3-carboxamide**

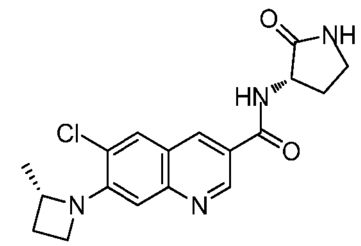

DIEA (0.142 mL, 0.813 mmol) was added to (S)-6-chloro-7-(2-methylazetidin-1-yl)quinoline-3-carboxylic acid ammonia salt (Intermediate 81) (0.0398 g, 0.135 mmol) in 1,4-dioxane (1.016 mL) and DMF (0.339 mL) at RT. Then, (S)-3-aminopyrrolidin-2-one (0.020 g, 0.203 mmol) was added and the reaction mixture was stirred for 5 min. Then, n-propylphosphonic acid anhydride ($T_3P$) (0.161 mL, 0.271 mmol) was added and the reaction mixture was stirred for 16 h. The reaction mixture was concentrated. The residue was purified by RP HPLC eluting with acetonitrile:water with 0.1% ammonium hydroxide (5:95:100:0), then further purified by silica gel chromatography, eluting with MeOH:EtOAc (1:4) to give the title compound (0.0504 g, 98 % yield). $^1H$ NMR (400 MHz, $CD_3SOCD_3$) $\delta$ 1.37 (d, *J* = 6 Hz, 3 H), 1.94-2.08 (m, 2 H), 2.34-2.52 (m, 2 H), 3.22-3.30 (m, 2 H), 3.85 (q, *J* = 7 Hz, 1 H), 4.36-4.44 (m, 1 H), 4.50-4.66 (m, 2 H), 7.06 (s, 1 H), 7.88 (s, 1 H), 8.06 (s, 1 H), 8.60 (d, *J* = 2 Hz, 1 H), 8.89 (d, *J* = 8 Hz, 1 H), 9.14 (d, *J* = 2 Hz, 1 H); LC-MS (LC-ES) M+H = 359.

**Example 189**

**N-(trans-4-(2-Hydroxypropan-2-yl)cyclohexyl)-8-methoxyquinoline-3-carboxamide**

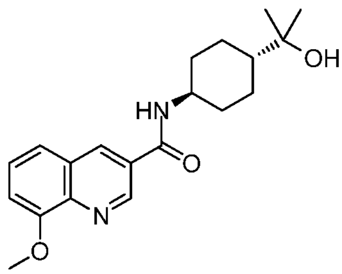

N,N-Diisopropylethylamine (0.406 mL, 2.324 mmol) was added to 8-methoxyquinoline-3-carboxylic acid (0.0787 g, 0.387 mmol) in 1,4-dioxane (1.291 mL) at room temperature. Then, 2-(trans-4-aminocyclohexyl)propan-2-ol (0.091 g, 0.581 mmol) was added and the reaction mixture was stirred for five minutes. Then, n-propylphosphonic acid anhydride (0.461 mL, 0.775 mmol, 50% in ethyl acetate) was added and the reaction mixture was stirred for sixteen hours. The reaction mixture was poured into saturated sodium bicarbonate, extracted with ethyl acetate (3X), dried over magnesium sulfate, filtered, and concentrated. The residue was purified by silica gel chromatography, eluting with methanol:ethyl acetate (1:19) to give N-(trans-4-(2-hydroxypropan-2-yl)cyclohexyl)-8-methoxyquinoline-3-carboxamide (0.0703 g, 0.195 mmol, 50.4 % yield). $^1H$ NMR (400 MHz, $CD_3SOCD_3$) $\delta$ 1.04 (s, 6 H), 1.04-1.22 (m, 3 H), 1.32 (q, *J* = 12 Hz, 2 H), 1.84 (br d, *J* = 11 Hz, 2 H), 1.94 (br d, *J* = 10 Hz, 2 H), 3.75 (qt, *J* = 8, 4 Hz, 1 H), 3.97 (s, 3 H), 4.04 (s, 1 H), 7.26-7.30 (m, 1 H), 7.54-7.60 (m, 2 H), 8.55 (d, *J* = 8 Hz, 1 H), 8.71 (d, *J* = 2 Hz, 1 H), 9.17 (d, *J* = 2 Hz, 1 H); LC-MS (LC-ES) M+H = 343.

**Example 190**

**N-(trans-4-(2-Hydroxypropan-2-yl)cyclohexyl)-7-(trifluoromethyl)quinoline-3-carboxamide**

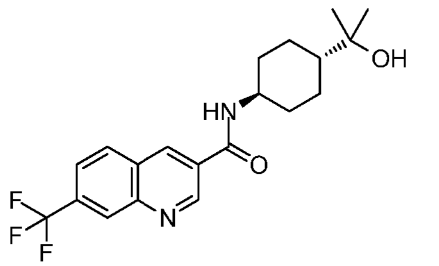

N,N-Diisopropylethylamine (0.412 mL, 2.361 mmol) was added to 7-(trifluoromethyl)quinoline-3-carboxylic acid (0.0949 g, 0.394 mmol) in 1,4-dioxane (1.312 mL) at room temperature. Then, 2-(trans-4-aminocyclohexyl)propan-2-ol (0.074 g, 0.472 mmol) was added and the reaction mixture was stirred for five minutes. Then, n-propylphosphonic acid anhydride (0.468 mL, 0.787 mmol, , 50% in ethyl acetate) was added and the reaction mixture was stirred for fourteen hours. The reaction mixture was poured into saturated sodium bicarbonate, extracted with ethyl acetate (3X), dried over magnesium sulfate, filtered, and concentrated. The residue was purified by silica gel chromatography, eluting with ethyl acetate to give N-(trans-4-(2-hydroxypropan-2-yl)cyclohexyl)-7-(trifluoromethyl)quinoline-3-carboxamide (0.0263 g, 0.066 mmol, 16.69 % yield). $^1$H NMR (400 MHz, $CD_3SOCD_3$) δ 1.05 (s, 6 H), 1.06-1.26 (m, 3 H), 1.33 (q, *J* = 13 Hz, 2 H), 1.85 (br d, *J* = 11 Hz, 2 H), 1.96 (br d, *J* = 10 Hz, 2 H), 3.77 (qt, *J* = 8, 4 Hz, 1 H), 4.05 (s, 1 H), 7.95 (dd, *J* = 8, 1 Hz, 1 H), 8.35 (d, *J* = 8 Hz, 1 H), 8.43 (s, 1 H), 8.68 (d, *J* = 8 Hz, 1 H), 8.92 (d, *J* = 2 Hz, 1 H), 9.37 (d, *J* = 2 Hz, 1 H); LC-MS (LC-ES) M+H = 381.

## Example 191

### 7-Bromo-N-(trans-4-(2-hydroxypropan-2-yl)cyclohexyl)quinoline-3-carboxamide

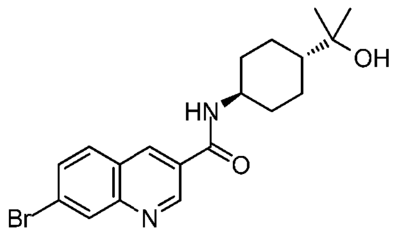

N,N-Di-*iso*-propylethylamine (0.644 mL, 3.69 mmol) was added to 7-bromoquinoline-3-carboxylic acid (0.1550 g, 0.615 mmol) in 1,4-dioxane (2.050 mL) at room temperature. Then, 2-(trans-4-aminocyclohexyl)propan-2-ol (0.116 g, 0.738 mmol) was added and the reaction mixture was stirred for five minutes. Then, n-propylphosphonic acid anhydride (0.732 mL, 1.230 mmol, 50% in ethyl acetate) was added and the reaction mixture was stirred for sixteen hours. The reaction mixture was poured into saturated sodium bicarbonate, extracted with ethyl acetate (3X), dried over magnesium sulfate, filtered, and concentrated. The residue was purified by silica gel chromatography, eluting with methanol:ethyl acetate (1:99) to give 7-bromo-N-(trans-4-(2-hydroxypropan-2-yl)cyclohexyl)quinoline-3-carboxamide (0.0684 g, 0.166 mmol, 27.0 % yield). $^1$H NMR (400 MHz, $CD_3SOCD_3$) δ 1.04 (s, 6 H), 1.06-1.24 (m, 3 H), 1.32 (q, *J* = 12 Hz, 2 H), 1.84 (br d, *J* = 11 Hz, 2 H), 1.95 (br d, *J* = 10 Hz, 2 H), 3.75 (qt, *J* = 8, 4 Hz, 1 H), 4.04 (s, 1 H), 7.83 (dd, *J* = 9, 2 Hz, 1 H), 8.07 (d, *J* = 9 Hz, 1 H), 8.30 (d, *J* = 2 Hz, 1 H), 8.59 (d, *J* = 8 Hz, 1 H), 8.82 (d, *J* = 2 Hz, 1 H), 9.26 (d, *J* = 2 Hz, 1 H); LC-MS (LC-ES) M+H = 392.

## Example 192

### 7-Cyclopropyl-N-(trans-4-(2-hydroxypropan-2-yl)cyclohexyl)quinoline-3-carboxamide

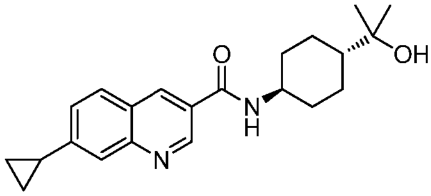

Cesium carbonate (0.070 g, 0.216 mmol) was added to 7-bromo-N-(trans-4-(2-hydroxypropan-2-yl)cyclohexyl)quinoline-3-carboxamide (0.0528 g, 0.135 mmol, Example 191) in toluene (2.159 mL) and water (0.540 mL) at room temperature, followed by potassium cyclopropyltrifluoroborate (0.024 g, 0.162 mmol) and the reaction mixture was purged with nitrogen. Then, [1,1'-bis(diphenylphosphino)ferrocene]dichloropalladium (II)-dichloromethane adduct (0.022 g, 0.027 mmol) was added and the reaction mixture was heated at 100 °C for sixteen hours. After cooling, the reaction mixture was poured into brine, extracted with ethyl acetate, dried over magnesium sulfate, filtered, and concentrated. The residue was purified by silica gel chromatography, eluting with ethyl acetate to give 7-cyclopropyl-N-(trans-4-(2-hydroxypropan-2-yl)cyclohexyl)quinoline-3-carboxamide (0.0230 g, 0.062 mmol, 45.9 % yield). $^1$H NMR (400 MHz, $CD_3SOCD_3$) δ 0.84-0.92 (m, 2 H), 1.04 (s, 6 H), 1.06-1.24 (m, 5 H), 1.32 (q, *J* = 12 Hz, 2 H), 1.84 (br d, *J* = 12 Hz, 2 H), 1.94 (br d, *J* = 10 Hz, 2 H), 2.14-2.34 (m, 1 H), 3.74 (qt, *J* = 8, 4 Hz, 1 H), 4.04 (s, 1 H), 7.37 (dd, *J* = 8, 2 Hz, 1 H), 7.75 (s, 1 H), 7.95 (d, *J* = 9 Hz, 1 H), 8.49 (d, *J* = 8 Hz, 1 H), 8.70 (d, *J* = 2 Hz, 1 H), 9.19 (d, *J* = 2 Hz, 1 H); LC-MS (LC-ES) M+H = 352.

**Example 193**

**7-Fluoro-N-(trans-4-(2-hydroxypropan-2-yl)cyclohexyl)quinoline-3-carboxamide**

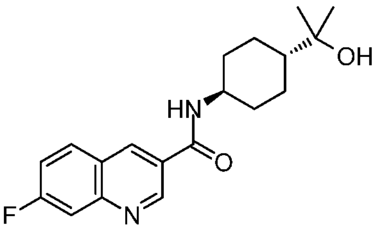

N,N-Di-*iso*-propylethylamine (0.636 mL, 3.64 mmol) was added to 7-fluoroquinoline-3-carboxylic acid (0.1160 g, 0.607 mmol) in 1,4-dioxane (2.023 mL) at room temperature. Then, 2-(trans-4-aminocyclohexyl)propan-2-ol (0.115 g, 0.728 mmol) was added and the reaction mixture was stirred for five minutes. Then, n-propylphosphonic acid anhydride (0.722 mL, 1.214 mmol, 50% in ethyl acetate) was added and the reaction mixture was stirred for sixteen hours. The reaction mixture was poured into saturated sodium bicarbonate, extracted with ethyl acetate (3X), dried over magnesium sulfate, filtered, and concentrated. The residue was purified by silica gel chromatography, eluting with ethyl acetate to give 7-fluoro-N-(trans-4-(2-hydroxypropan-2-yl)cyclohexyl)quinoline-3-carboxamide (0.1829 g, 0.526 mmol, 87 % yield). $^1$H NMR (400 MHz, $CD_3SOCD_3$) δ 1.04 (s, 6 H), 1.06-1.24 (m, 3 H), 1.32 (q, *J* = 12 Hz, 2 H), 1.84 (br d, *J* = 11 Hz, 2 H), 1.95 (br d, *J* = 10 Hz, 2 H), 3.75 (qt, *J* = 8, 4 Hz, 1 H), 4.04 (s, 1 H), 7.63 (dt, *J* = 9, 3 Hz, 1 H), 7.82 (dd, *J* = 10, 2 Hz, 1 H), 8.20 (dd, *J* = 9, 6 Hz, 1 H), 8.56 (d, *J* = 8 Hz, 1 H), 8.84 (d, *J* = 2 Hz, 1 H), 9.27 (d, *J* = 2 Hz, 1 H); LC-MS (LC-ES) M+H = 331.

**Example 194**

**7-(Azetidin-1-yl)-N-(trans-4-(2-hydroxypropan-2-yl)cyclohexyl)quinoline-3-carboxamide**

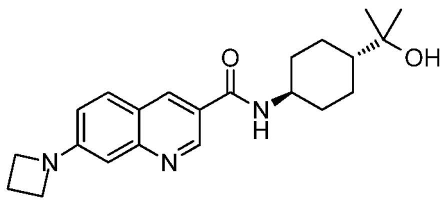

N,N-Diisopropylethylamine (0.222 mL, 1.269 mmol) was added to 7-fluoro-N-(trans-4-(2-hydroxypropan-2-yl)cyclohexyl)quinoline-3-carboxamide (0.0524 g, 0.159 mmol, Example 193) in ethanol (0.793 mL) at room temperature. Then, azetidine (0.086 mL, 1.269 mmol) was added and the reaction mixture was heated at 100 °C in a microwave for three hours and concentrated. The residue was purified by silica gel chromatography, eluting with methanol:ethyl acetate (1:9) to give 7-(azetidin-1-yl)-N-(trans-4-(2-hydroxypropan-2-yl)cyclohexyl)quinoline-3-carboxamide (0.0353 g, 0.091 mmol, 57.5 % yield). $^1$H NMR (400 MHz, $CD_3SOCD_3$) δ 1.04 (s, 6 H), 1.06-1.24 (m, 3 H), 1.30 (q, *J* = 12 Hz, 2 H), 1.83 (d, *J* = 11 Hz, 2 H), 1.91 (d, *J* = 10 Hz, 2 H), 2.38 (q, *J* = 7 Hz, 2 H), 3.73 (qt, *J* = 8, 4 Hz, 1 H), 4.00 (t, *J* = 7 Hz, 4 H), 4.03 (s, 1 H), 6.70 (d, *J* = 2 Hz, 1 H), 6.91 (dd, *J* = 9, 2 Hz, 1 H), 7.82 (d, *J* = 9 Hz, 1 H), 8.29 (d, *J* = 8 Hz, 1 H), 8.53 (d, *J* = 2 Hz, 1 H), 9.05 (d, *J* = 2 Hz, 1 H); LC-MS (LC-ES) M+H = 368.

**Example 195**

**N-(trans-4-(2-Hydroxypropan-2-yl)cyclohexyl-[1,3]dioxolo[4,5-g]quinoline-7-carboxamide**

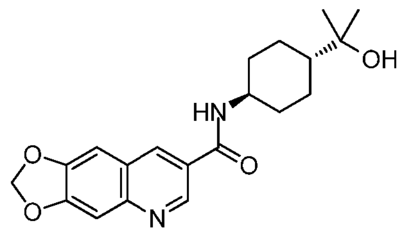

N,N-Di-*iso*-propylethylamine (0.198 mL, 1.133 mmol) was added to [1,3]dioxolo[4,5-g]quinoline-7-carboxylic acid (0.0410 g, 0.189 mmol) in 1,4-dioxane (0.629 mL) at room temperature. Then, 2-(trans-4-aminocyclohexyl)propan-2-ol (0.036 g, 0.227 mmol) was added and the reaction mixture was stirred for five minutes. Then, n-propylphosphonic acid anhydride (0.225 mL, 0.378 mmol, 50% in ethyl acetate) was added and the reaction mixture was stirred for sixteen hours. The reaction mixture was poured into saturated sodium bicarbonate, extracted with ethyl acetate (3X), dried over magnesium sulfate, filtered, and concentrated. The residue was purified by silica gel chromatography, eluting with methanol:ethyl acetate (1:49) to give N-(trans-4-(2-hydroxypropan-2-yl)cyclohexyl)-[1,3]dioxolo[4,5-g]quinoline-7-carboxamide (0.0684 g, 0.182 mmol, 97 % yield). $^1$H NMR (400 MHz, $CD_3SOCD_3$) δ 1.04 (s, 6 H), 1.06-1.24 (m, 3 H), 1.31 (q, *J* = 12 Hz, 2 H), 1.83 (br d, *J* = 12 Hz, 2 H), 1.92 (br d, *J* = 10 Hz, 2 H), 3.73 (qt, *J* = 8, 4 Hz, 1 H), 4.04 (s, 1 H), 6.24 (s, 2 H), 7.39 (s, 1 H), 7.42 (s, 1 H), 8.43 (d, *J* = 8 Hz, 1 H), 8.56 (d, *J* = 2 Hz, 1 H), 9.02 (d, *J* = 2 Hz, 1 H); LC-MS (LC-ES) M+H = 357.

**Example 196**

**7-(Dimethylamino)-N-(trans-4-(2-hydroxypropan-2-yl)cyclohexyl)quinoline-3-carboxamide**

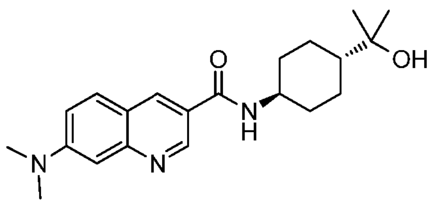

N,N-Diisopropylethylamine (0.114 mL, 0.655 mmol) was added to 7-fluoro-N-(trans-4-(2-hydroxypropan-2-yl)cyclohexyl)quinoline-3-carboxamide (0.0541 g, 0.164 mmol, Example 193) in ethanol (0.819 mL) at room temperature. Then, 2.0 M dimethylamine (0.819 mL, 1.637 mmol) in methanol was added and the reaction mixture was heated at 120 °C in a microwave for five hours and concentrated. The residue was purified by silica gel chromatography, eluting with methanol:ethyl acetate (1:9) to give 7-(dimethylamino)-N-(trans-4-(2-hydroxypropan-2-yl)cyclohexyl)quinoline-3-carboxamide (0.0399 g, 0.107 mmol, 65.1 % yield). $^1H$ NMR (400 MHz, $CD_3SOCD_3$) $\delta$ 1.04 (s, 6 H), 1.06-1.24 (m, 3 H), 1.31 (q, $J$ = 12 Hz, 2 H), 1.83 (d, $J$ = 11 Hz, 2 H), 1.92 (d, $J$ = 10 Hz, 2 H), 3.08 (s, 6 H), 3.73 (qt, $J$ = 8, 4 Hz, 1 H), 4.03 (s, 1 H), 6.99 (d, $J$ = 2 Hz, 1 H), 7.31 (dd, $J$ = 9, 3 Hz, 1 H), 7.82 (d, $J$ = 9 Hz, 1 H), 8.29 (d, $J$ = 8 Hz, 1 H), 8.53 (d, $J$ = 2 Hz, 1 H), 9.05 (d, $J$ = 2 Hz, 1 H); LC-MS (LC-ES) M+H = 356.

## Example 197

### 7-Cyclobutyl-N-(trans-4-(2-hydroxypropan-2-yl)cyclohexyl)quinoline-3-carboxamide

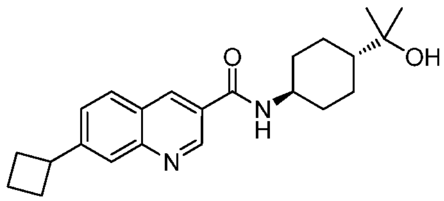

Cesium carbonate (0.078 g, 0.240 mmol) was added to 7-bromo-N-(trans-4-(2-hydroxypropan-2-yl)cyclohexyl)quinoline-3-carboxamide (0.0587 g, 0.150 mmol, Example 191) in toluene (2.400 mL) and water (0.600 mL) at room temperature, followed by potassium cyclobutyltrifluoroborate (0.029 g, 0.180 mmol) and the reaction mixture was purged with nitrogen. Then, [1,1'-bis(diphenylphosphino)ferrocene]dichloropalladium (II)-dichloromethane adduct (0.025 g, 0.030 mmol) was added and the reaction mixture was heated at 100 °C for sixteen hours. After cooling, the reaction mixture was poured into brine, extracted with ethyl acetate, dried over magnesium sulfate, filtered, and concentrated. The residue was purified by silica gel chromatography, eluting with ethyl acetate, then further purified by RP HPLC, eluting with acetonitrile:water with 0.1% ammonium hydroxide (5:95:100:0) to give 7-cyclobutyl-N-(trans-4-(2-hydroxypropan-2-yl)cyclohexyl)quinoline-3-carboxamide (0.0116 g, 0.030 mmol, 20.04 % yield). $^1H$ NMR (400 MHz, $CD_3SOCD_3$) $\delta$ 1.05 (s, 6 H), 1.06-1.24 (m, 3 H), 1.32 (q, $J$ = 12 Hz, 2 H), 1.84 (br d, $J$ = 12 Hz, 2 H), 1.86-1.96 (m, 1 H), 1.94 (br d, $J$ = 10 Hz, 2 H), 1.98-2.12 (m, 1 H), 2.16-2.28 (m, 2 H), 2.34-2.46 (m, 2 H), 3.68-3.80 (m, 2 H), 4.04 (s, 1 H), 7.57 (dd, $J$ = 8, 2 Hz, 1 H), 7.84 (s, 1 H), 8.01 (d, $J$ = 8 Hz, 1 H), 8.51 (d, $J$ = 8 Hz, 1 H), 8.73 (d, $J$ = 2 Hz, 1 H), 9.22 (d, $J$ = 2 Hz, 1 H); LC-MS (LC-ES) M+H = 367.

## Example 198

### N-((1r,4r)-4-Hydroxy-4-methylcyclohexyl)-7-methoxyquinoline-3-carboxamide

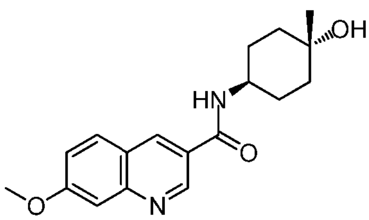

(1r,4r)-4-Amino-1-methylcyclohexanol (0.059 g, 0.454 mmol) was added to 7-methoxyquinoline-3-carboxylic acid (0.0768 g, 0.378 mmol) in 1,4-dioxane (1.890 mL) at room temperature. Then, N,N-di-*iso*-propylethylamine (0.396 mL, 2.268 mmol) was added and the reaction mixture was stirred for five minutes. Then, n-propylphosphonic acid anhydride (0.450 mL, 0.756 mmol, 50% in ethyl acetate) was added and the reaction mixture was stirred for sixty-four hours. The reaction mixture was poured into saturated sodium bicarbonate, extracted with ethyl acetate (3X), dried over magnesium sulfate, filtered, and concentrated. The reaction mixture was purified by silica gel chromatography, eluting

with methanol:ethyl acetate (1:16) to give N-((1r,4r)-4-hydroxy-4-methylcyclohexyl)-7-methoxyquinoline-3-carboxamide (0.0613 g, 0.185 mmol, 49.0 % yield). $^1$H NMR (400 MHz, $CD_3SOCD_3$) δ 1.16 (s, 3 H), 1.40-1.56 (m, 4 H), 1.56-1.66 (m, 2 H), 1.76-1.86 (m, 2 H), 3.80-3.92 (m, 1 H), 3.94 (s, 3 H), 4.29 (s, 1 H), 7.31 (dd, *J* = 9, 2 Hz, 1 H), 7.44 (d, *J* = 2 Hz, 1 H), 7.98 (d, *J* = 9 Hz, 1 H), 8.41 (d, *J* = 8 Hz, 1 H), 8.69 (d, *J* = 2 Hz, 1 H), 9.17 (d, *J* = 2 Hz, 1 H); LC-MS (LC-ES) M+H = 315.

**Example 199**

**N-(6-(2-Hydroxypropan-2-yl)spiro[3.3]heptan-2-yl)-7-methoxyquinoline-3-carboxamide**

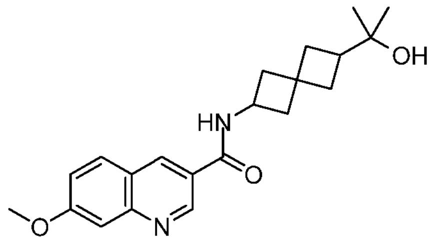

2-(6-Aminospiro[3.3]heptan-2-yl)propan-2-ol (0.102 g, 0.601 mmol, Intermediate 82) was added to 7-methoxyquinoline-3-carboxylic acid (0.1018 g, 0.501 mmol) in 1,4-dioxane (2.505 mL) at room temperature. Then, N,N-diisopropylethylamine (0.525 mL, 3.01 mmol) was added and the reaction mixture was stirred for five minutes. Then, n-propylphosphonic acid anhydride (0.596 mL, 1.002 mmol, 50% in ethyl acetate) was added and the reaction mixture was stirred for sixty-six hours. The reaction mixture was poured into saturated sodium bicarbonate, extracted with ethyl acetate (3X), dried over magnesium sulfate, filtered, and concentrated. The reaction mixture was purified by silica gel chromatography, eluting with methanol:ethyl acetate (1:49) to give N-(6-(2-hydroxypropan-2-yl)spiro[3.3]heptan-2-yl)-7-methoxyquinoline-3-carboxamide (0.1376 g, 0.369 mmol, 73.6 % yield). $^1$H NMR (400 MHz, $CD_3SOCD_3$) δ 0.94 (s, 3 H), 0.96 (s, 3 H), 1.66-1.76 (m, 1 H), 1.86-2.04 (m, 4 H), 2.11 (q, *J* = 9 Hz, 2 H), 2.12-2.22 (m, 1 H), 2.36-2.46 (m, 1 H), 3.94 (s, 3 H), 3.99 (s, 1 H), 4.33 (h, *J* = 8 Hz, 1 H), 7.31 (dd, *J* = 9, 2 Hz, 1 H), 7.44 (d, *J* = 2 Hz, 1 H), 7.97 (d, *J* = 9 Hz, 1 H), 8.70 (t, *J* = 2 Hz, 1 H), 8.77 (d, *J* = 7 Hz, 1 H), 9.18 (d, *J* = 2 Hz, 1 H); LC-MS (LC-ES) M+H = 355.

**Example 200**

**N-(trans-4-(2-Hydroxy-2-methylpropoxy)cyclohexyl)-7-methoxyquinoline-3-carboxamide**

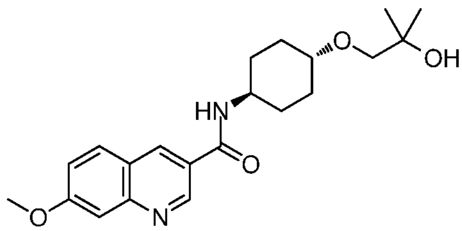

1-((trans-4-Aminocyclohexyl)oxy)-2-methylpropan-2-ol (0.085 g, 0.453 mmol, Intermediate 42) was added to 7-methoxyquinoline-3-carboxylic acid (0.0767 g, 0.377 mmol) in 1,4-dioxane (1.887 mL) at room temperature. Then, N,N-di-*iso*-propylethylamine (0.396 mL, 2.265 mmol) was added and the reaction mixture was stirred for five minutes. Then, n-propylphosphonic acid anhydride (0.449 mL, 0.755 mmol, 50% in ethyl acetate) was added and the reaction mixture was stirred for sixteen hours. The reaction mixture was poured into saturated sodium bicarbonate, extracted with ethyl acetate (3X), dried over magnesium sulfate, filtered, and concentrated. The reaction mixture was purified by silica gel chromatography, eluting with methanol:ethyl acetate (1:24) to give N-(trans-4-(2-hydroxy-2-methylpropoxy)cyclohexyl)-7-methoxyquinoline-3-carboxamide (0.0734 g, 0.187 mmol, 49.6 % yield). $^1$H NMR (400 MHz, $CD_3SOCD_3$) δ 1.06 (s, 6 H), 1.26 (q, *J* = 13 Hz, 2 H), 1.39 (q, *J* = 13 Hz, 2 H), 1.90 (br d, *J* = 10 Hz, 2 H), 2.02 (br d, *J* = 10 Hz, 2 H), 3.17 (s, 2 H), 3.18-3.28 (m, 1 H), 3.81 (qt, *J* = 8, 4 Hz, 1 H), 3.94 (s, 3 H), 4.21 (s, 1 H), 7.31 (dd, *J* = 9, 2 Hz, 1 H), 7.44

(d, $J$ = 2 Hz, 1 H), 7.98 (d, $J$ = 9 Hz, 1 H), 8.45 (d, $J$ = 8 Hz, 1 H), 8.69 (d, $J$ = 2 Hz, 1 H), 9.18 (d, $J$ = 2 Hz, 1 H); LC-MS (LC-ES) M+H = 373.

**Example 201**

**7-Methoxy-N-(trans-4-((2,2,2-trifluoroethyl)amino)cyclohexyl)quinoline-3-carboxamide**

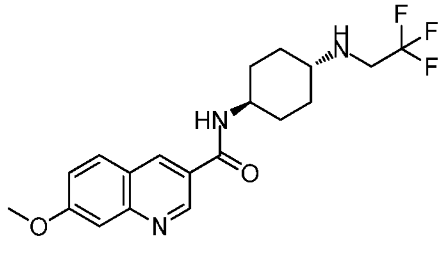

trans-N1-(2,2,2-Trifluoroethyl)cyclohexane-1,4-diamine dihydrochloride (0.080 g, 0.296 mmol, Intermediate 83) was added to 7-methoxyquinoline-3-carboxylic acid (0.0601 g, 0.296 mmol) in 1,4-dioxane (1.479 mL) at room temperature. Then, N,N-di-*iso*-propylethylamine (0.310 mL, 1.775 mmol) was added and the reaction mixture was stirred for five minutes. Then, n-propylphosphonic acid anhydride (0.352 mL, 0.592 mmol, 50% in ethyl acetate) was added and the reaction mixture was stirred for sixteen hours. The reaction mixture was poured into saturated sodium bicarbonate, extracted with ethyl acetate (3X), dried over magnesium sulfate, filtered, and concentrated. The reaction mixture was purified by silica gel chromatography, eluting with methanol:ethyl acetate (1:32) to give 7-methoxy-N-(trans-4-((2,2,2-trifluoroethyl)amino)cyclohexyl)quinoline-3-carboxamide (0.0510 g, 0.127 mmol, 42.9 % yield). $^1$H NMR (400 MHz, $CD_3SOCD_3$) $\delta$ 1.11 (q, $J$ = 14 Hz, 2 H), 1.36 (q, $J$ = 12 Hz, 2 H), 1.91 (brt, $J$ = 14 Hz, 4 H), 2.20 (q, $J$ = 8 Hz, 1 H), 2.36-2.48 (m, 1 H), 3.18-3.30 (m, 2 H), 3.78 (qt, $J$ = 8, 4 Hz, 1 H), 3.94 (s, 3 H), 7.31 (dd, $J$ = 9, 2 Hz, 1 H), 7.44 (d, $J$ = 2 Hz, 1 H), 7.97 (d, $J$ = 9 Hz, 1 H), 8.45 (d, $J$ = 8 Hz, 1 H), 8.69 (d, $J$ = 2 Hz, 1 H), 9.18 (d, $J$ = 2 Hz, 1 H); LC-MS (LC-ES) M+H = 382.

**Example 202**

**8-Fluoro-N-(trans-4-(2-hydroxvpropan-2-yl)cyclohexyl)-7-methoxyquinoline-3-carboxamide**

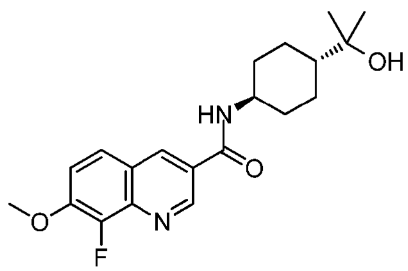

N,N-Di-*iso*-propylethylamine (0.221 mL, 1.265 mmol) was added to 8-fluoro-7-methoxyquinoline-3-carboxylic acid, N,N,N-triethylethanaminium salt (0.0741 g, 0.211 mmol) in 1,4-dioxane (0.703 mL) at room temperature. Then, 2-(trans-4-aminocyclohexyl)propan-2-ol (0.040 g, 0.253 mmol) was added and the reaction mixture was stirred for five minutes. Then, n-propylphosphonic acid anhydride (0.251 mL, 0.422 mmol, 50% in ethyl acetate) was added and the reaction mixture was stirred for sixteen hours. The reaction mixture was poured into saturated sodium bicarbonate, extracted with ethyl acetate (3X), dried over magnesium sulfate, filtered, and concentrated. The residue was purified by silica gel chromatography, eluting with methanol:ethyl acetate (1:24) to give 8-fluoro-N-(trans-4-(2-hydroxypropan-2-yl)cyclohexyl)-7-methoxyquinoline-3-carboxamide (0.0722 g, 0.190 mmol, 90 % yield). $^1$H NMR (400 MHz, $CD_3SOCD_3$) $\delta$ 1.04 (s, 6 H), 1.06-1.26 (m, 3 H), 1.32 (q, $J$ = 12 Hz, 2 H), 1.84 (br d, $J$ = 11 Hz, 2 H), 1.94 (br d, $J$ = 10 Hz, 2 H), 3.75 (qt, $J$ = 8, 4 Hz, 1 H), 4.03 (s, 3 H), 4.04 (s, 1 H), 7.72 (t, $J$ = 9 Hz, 1 H), 7.93 (dd, $J$ = 9, 1 Hz, 1 H), 8.52 (d, $J$ = 8 Hz, 1 H), 8.78 (s, 1 H), 9.23 (d, $J$ = 2 Hz, 1 H); LC-MS (LC-ES) M+H = 361.

**Example 202A**

**6,7-Dichloro-N-(trans-4-(2-hydroxypropan-2-yl)cyclohexyl)quinoline-3-carboxamide**

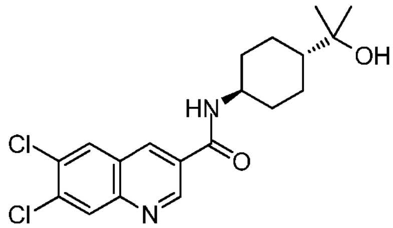

N,N-Di-*iso*-propylethylamine (0.326 mL, 1.866 mmol) was added to 6,7-dichloroquinoline-3-carboxylic acid (0.0753 g, 0.311 mmol) in 1,4-dioxane (1.037 mL) at room temperature. Then, 2-(trans-4-aminocyclohexyl)propan-2-ol (0.059 g, 0.373 mmol) was added and the reaction mixture was stirred for five minutes. Then, n-propylphosphonic acid anhydride (0.370 mL, 0.622 mmol, 50% in ethyl acetate) was added and the reaction mixture was stirred for sixteen hours. The reaction mixture was poured into saturated sodium bicarbonate, extracted with ethyl acetate (3X), dried over magnesium sulfate, filtered, and concentrated. The residue was purified by silica gel chromatography, eluting with ethyl acetate to give 6,7-dichloro-N-(trans-4-(2-hydroxypropan-2-yl)cyclohexyl)quinoline-3-carboxamide (0.0551 g, 0.137 mmol, 44.1 % yield). $^1H$ NMR (400 MHz, $CD_3SOCD_3$) $\delta$ 1.04 (s, 6 H), 1.06-1.24 (m, 3 H), 1.32 (q, *J* = 12 Hz, 2 H), 1.84 (br d, *J* = 11 Hz, 2 H), 1.95 (br d, *J* = 10 Hz, 2 H), 3.75 (qt, *J* = 8, 4 Hz, 1 H), 4.04 (s, 1 H), 8.37 (s, 1 H), 8.48 (s, 1 H), 8.63 (d, *J* = 8 Hz, 1 H), 8.79 (d, *J* = 2 Hz, 1 H), 9.28 (d, *J* = 2 Hz, 1 H); LC-MS (LC-ES) M+H = 381.

**Example 203**

**N-((1s,3s)-3-Hydroxy-3-methylcyclobutyl-7-methoxyquinoline-3-carboxamide and N-((1r,3r)-3-Hydroxy-3-methylcyclobutyl)-7-methoxyquinoline-3-carboxamide**

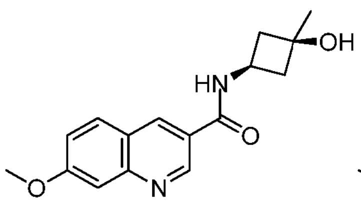

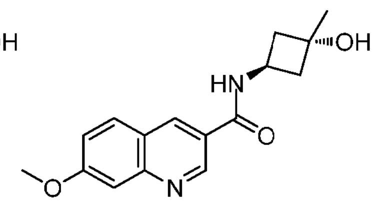

A cis/trans mixture of 3-amino-1-methylcyclobutanol (0.082 g, 0.813 mmol, Intermediate 51) was added to 7-methoxyquinoline-3-carboxylic acid (0.1376 g, 0.677 mmol) in 1,4-dioxane (3.39 mL) at room temperature. Then, N,N-di-*iso*-propylethylamine (0.710 mL, 4.06 mmol) was added and the reaction mixture was stirred for five minutes. Then, n-propylphosphonic acid anhydride (0.806 mL, 1.354 mmol, 50% in ethyl acetate) was added and the reaction mixture was stirred for sixteen hours. The reaction mixture was concentrated. The residue was purified by RP HPLC eluting with acetonitrile:water with 0.1% ammonium hydroxide (5:95:100:0), then further purified by silica gel chromatography, eluting with methanol:dichloromethane (1:10), then further purified by silica gel chromatography on a chiral ADH column, eluting with ethanol:hexanes (2:3) to give N-((1s,3s)-3-hydroxy-3-methylcyclobutyl)-7-methoxyquinoline-3-carboxamide (0.0523 g, 0.174 mmol, 25.6 % yield) and N-((1r,3r)-3-hydroxy-3-methylcyclobutyl)-7-methoxyquinoline-3-carboxamide (0.0502 g, 0.167 mmol, 24.60 % yield).

**N-((1s,3s)-3-Hydroxy-3-methylcyclobutyl)-7-methoxyquinoline-3-carboxamide**

$^1H$ NMR (400 MHz, $CD_3SOCD_3$) $\delta$ 1.28 (s, 3 H), 2.10-2.16 (m, 2 H), 2.28-2.36 (m, 2 H), 3.94 (s, 3 H), 4.02 (h, *J* = 8 Hz, 1 H), 4.98 (s, 1 H), 7.31 (dd, *J* = 9, 2 Hz, 1 H), 7.44 (d, *J* = 2 Hz, 1 H), 7.97 (d, *J* = 9 Hz, 1 H), 8.72 (d, *J* = 2 Hz, 1 H), 8.82 (d, *J* = 7 Hz, 1 H), 9.19 (d, *J* = 2 Hz, 1 H); LC-MS (LC-ES) M+H = 287.

**N-((1r,3r)-3-Hydroxy-3-methylcyclobutyl)-7-methoxyquinoline-3-carboxamide**

$^1$H NMR (400 MHz, $CD_3SOCD_3$) δ 1.29 (s, 3 H), 2.06-2.12 (m, 2 H), 2.26-2.34 (m, 2 H), 3.94 (s, 3 H), 4.54 (h, *J* = 8 Hz, 1 H), 4.86 (s, 1 H), 7.31 (dd, *J* = 9, 2 Hz, 1 H), 7.44 (d, *J* = 2 Hz, 1 H), 7.98 (d, *J* = 9 Hz, 1 H), 8.70 (d, *J* = 2 Hz, 1 H), 8.79 (d, *J* = 7 Hz, 1 H), 9.18 (d, *J* = 2 Hz, 1 H); LC-MS (LC-ES) M+H = 287.

**Example 204**

**7-Chloro-N-(trans-4-(2-hydroxvpropan-2-yl)cyclohexyl)quinoline-3-carboxamide**

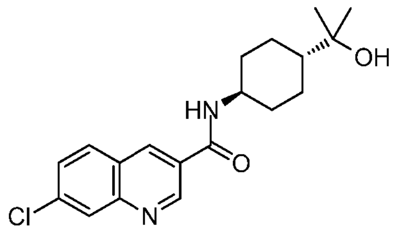

N,N-Di-*iso*-propylethylamine (0.511 mL, 2.93 mmol) was added to 7-chloroquinoline-3-carboxylic acid (0.1013 g, 0.488 mmol) in 1,4-dioxane (1.626 mL) at room temperature. Then, 2-(trans-4-aminocyclohexyl)propan-2-ol (0.092 g, 0.586 mmol) was added and the reaction mixture was stirred for five minutes. Then, n-propylphosphonic acid anhydride (0.581 mL, 0.976 mmol, 50% in ethyl acetate) was added and the reaction mixture was stirred for sixteen hours. The reaction mixture was poured into saturated sodium bicarbonate, extracted with ethyl acetate (3X), dried over magnesium sulfate, filtered, and concentrated. The residue was purified by silica gel chromatography, eluting with methanol:ethyl acetate (1:99) to give 7-chloro-N-(trans-4-(2-hydroxypropan-2-yl)cyclohexyl)quinoline-3-carboxamide (0.1265 g, 0.346 mmol, 71.0 % yield). $^1$H NMR (400 MHz, $CD_3SOCD_3$) δ 1.04 (s, 6 H), 1.04-1.26 (m, 3 H), 1.32 (q, *J* = 12 Hz, 2 H), 1.84 (br d, *J* = 11 Hz, 2 H), 1.95 (br d, *J* = 10 Hz, 2 H), 3.75 (qt, *J* = 8, 4 Hz, 1 H), 4.04 (s, 1 H), 7.72 (dd, *J* = 9, 2 Hz, 1 H), 8.14 (s, 1 H), 8.15 (d, *J* = 8 Hz, 1 H), 8.58 (d, *J* = 8 Hz, 1 H), 8.83 (d, *J* = 2 Hz, 1 H), 9.27 (d, *J* = 2 Hz, 1 H); LC-MS (LC-ES) M+H = 347.

**Example 205**

**6-Chloro-N-((3S,4R)-4-methyl-2-oxopyrrolidin-3-yl-7-((S)-2-methylazetidin-1-yl)quinoline-3-carboxamide**

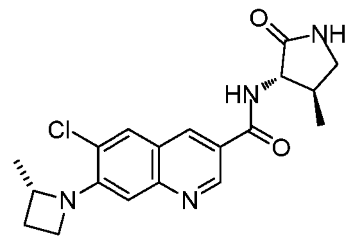

(3S,4R)-3-amino-4-methylpyrrolidin-2-one (0.019 g, 0.169 mmol, Intermediate 78) was added to (S)-6-chloro-7-(2-methylazetidin-1-yl)quinoline-3-carboxylic acid ammonia salt (0.0383 g, 0.130 mmol, Intermediate 81) in 1,4-dioxane (0.543 mL) and N,N-dimethylformamide (0.109 mL) at room temperature. Then, N,N-di-*iso*-propylethylamine (0.137 mL, 0.782 mmol) was added and the reaction mixture was stirred for five minutes. Then, n-propylphosphonic acid anhydride (0.155 mL, 0.261 mmol, 50% in ethyl acetate) was added and the reaction mixture was stirred for sixteen hours. The reaction mixture was concentrated. The residue was purified by RP HPLC eluting with acetonitrile:water with 0.1% ammonium hydroxide (5:95:100:0), then further purified by silica gel chromatography, eluting with methanol:ethyl acetate (1:4) to give 6-chloro-N-((3S,4R)-4-methyl-2-oxopyrrolidin-3-yl)-7-((S)-2-methylazetidin-1-yl)quinoline-3-carboxamide (0.0309 g, 0.079 mmol, 60.4 % yield). $^1$H NMR (400 MHz, $CD_3SOCD_3$) δ 1.09 (d, *J* = 6 Hz, 3 H), 1.38 (d, *J* = 6 Hz, 3 H), 1.92-2.04 (m, 1 H), 2.36-2.52 (m, 2 H), 2.88 (t, *J* = 9 Hz, 1 H), 3.33 (t, *J* = 9 Hz, 1 H), 3.85 (q, *J* = 7 Hz, 1 H), 4.29 (t, *J*

= 9 Hz, 1 H), 4.39 (q, *J* = 5 Hz, 1 H), 4.56 (h, *J* = 7 Hz, 1 H), 7.07 (s, 1 H), 7.83 (br s, 1 H), 8.06 (s, 1 H), 8.61 (d, *J* = 2 Hz, 1 H), 8.82 (d, *J* = 9 Hz, 1 H), 9.15 (d, *J* = 2 Hz, 1 H); LC-MS (LC-ES) M+H = 373.

**Example 206**

**6-Fluoro-7-(3-fluoroazetidin-1-yl)-N-((3S,4R)-4-methyl-2-oxopyrrolidin-3-yl)quinoline-3-carboxamide**

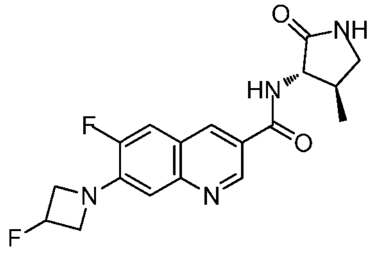

(3S,4R)-3-Amino-4-methylpyrrolidin-2-one (0.053 g, 0.466 mmol, Intermediate 78) was added to 6-fluoro-7-(3-fluoroazetidin-1-yl)quinoline-3-carboxylic acid ammonia salt (0.0820 g, 0.292 mmol, Intermediate 26) in 1,4-dioxane (1.215 mL) and N,N-dimethylformamide (0.243 mL) at room temperature. Then, N,N-di-*iso*-propylethylamine (0.306 mL, 1.749 mmol) was added and the reaction mixture was stirred for five minutes. Then, n-propylphosphonic acid anhydride (0.347 mL, 0.583 mmol, 50% in ethyl acetate) was added and the reaction mixture was stirred for sixty-four hours. The reaction mixture was concentrated. The residue was purified by RP HPLC eluting with acetonitrile:water with 0.1% ammonium hydroxide (5:95:100:0), then further purified by silica gel chromatography, eluting with methanol:ethyl acetate (1:6) to give 6-fluoro-7-(3-fluoroazetidin-1-yl)-N-((3S,4R)-4-methyl-2-oxopyrrolidin-3-yl)quinoline-3-carboxamide (0.0383 g, 0.101 mmol, 34.6 % yield). $^1$H NMR (400 MHz, $CD_3SOCD_3$) $\delta$ 1.09 (d, *J* = 7 Hz, 3 H), 2.36-2.50 (m, 1 H), 2.88 (t, *J* = 9 Hz, 1 H), 3.33 (t, *J* = 9 Hz, 1 H), 4.20 (br dd, *J* = 14, 10 Hz, 2 H), 4.29 *(dd, J* = 10, 9 Hz, 1 H), 4.40-4.54 (m, 2 H), 5.42-5.64 (m, 1 H), 6.99 (d, *J* = 13 Hz, 1 H), 7.77 (d, *J* = 13 Hz, 1 H), 7.82 (s, 1 H), 8.62 (d, *J* = 2 Hz, 1 H), 8.81 (d, *J* = 9 Hz, 1 H), 9.12 (d, *J* = 2 Hz, 1 H); LC-MS (LC-ES) M+H = 361.

**Example 207**

**6-Fluoro-N-((3S,4R)-4-methyl-2-oxopyrrolidin-3-yl)-7-(3-methylazetidin-1-yl)quinoline-3-carboxamide**

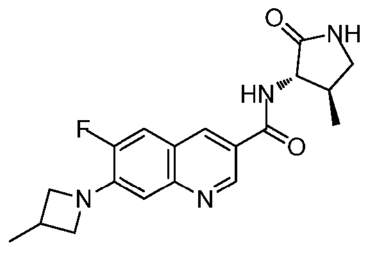

(3S,4R)-3-Amino-4-methylpyrrolidin-2-one (0.035 g, 0.304 mmol, Intermediate 78) was added to 6-fluoro-7-(3-methylazetidin-1-yl)quinoline-3-carboxylic acid ammonia salt (0.0842 g, 0.304 mmol, Intermediate 37) in 1,4-dioxane (1.265 mL) and N,N-dimethylformamide (0.253 mL) at room temperature. Then, N,N-di-*iso*-propylethylamine (0.318 mL, 1.822 mmol) was added and the reaction mixture was stirred for five minutes. Then, n-propylphosphonic acid anhydride (0.362 mL, 0.607 mmol, 50% in ethyl acetate) was added and the reaction mixture was stirred for sixty-six hours. The reaction mixture was concentrated. The residue was purified by RP HPLC eluting with acetonitrile:water with 0.1% ammonium hydroxide (5:95:100:0), then further purified by silica gel chromatography, eluting with methanol:ethyl acetate (1:4) to give 6-fluoro-N-((3S,4R)-4-methyl-2-oxopyrrolidin-3-yl)-7-(3-methylazetidin-1-yl)quinoline-3-carboxamide (0.0510 g, 0.136 mmol, 44.8 % yield). $^1$H NMR (400 MHz, $CD_3SOCD_3$) $\delta$ 1.08 (t, *J* = 7 Hz, 3 H), 1.26 (d, *J* = 7 Hz, 3 H), 2.36-2.48 (m, 1 H), 2.80-2.92 (m, 1 H), 2.88 (t, *J* = 9 Hz, 1 H), 3.33 (t, *J* = 9 Hz, 1 H), 3.70 (t, *J* = 6 Hz, 2 H), 4.25 (dt, *J* = 8, 2 Hz, 2 H), 4.29 (dd, *J* = 11, 9 Hz, 1 H), 6.85 (d, *J* = 9 Hz, 1 H), 7.70 (d, *J* = 13 Hz, 1 H), 7.82 (s, 1 H), 8.58 (d, *J* =

2 Hz, 1 H), 8.77 (d, *J* = 9 Hz, 1 H), 9.09 (d, *J* = 2 Hz, 1 H); LC-MS (LC-ES) M+H = 357.

**Example 208**

**trans-4-(6-Chloro-7-(difluoromethoxy)quinoline-3-carboxamido)-N,N-dimethylcyclohexanamine oxide**

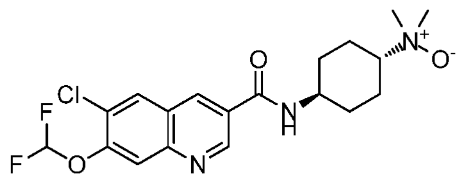

N,N-Diisopropylethylamine (0.578 mL, 3.31 mmol) was added to 6-chloro-7-(difluoromethoxy)quinoline-3-carboxylic acid (0.1509 g, 0.551 mmol, Intermediate 20) in N,N-dimethylformamide (2.76 mL) at room temperature. Then, trans-4-amino-N,N-dimethylcyclohexanamine oxide (0.140 g, 0.717 mmol, Intermediate 84) was added and the reaction mixture was stirred for five minutes. Then, n-propylphosphonic acid anhydride (0.657 mL, 1.103 mmol, 50% in ethyl acetate) was added and the reaction mixture was stirred for sixteen hours. The reaction mixture was concentrated. The resulting residue was purified by RP HPLC, eluting with acetonitrile:water with 0.1% ammonium hydroxide (5:95:100:0), then further purified by silica gel chromatography, eluting with methanol with 5% ammonium hydroxide to give trans-4-(6-chloro-7-(difluoromethoxy)quinoline-3-carboxamido)-N,N-dimethylcyclohexanamine oxide (0.0389 g, 0.089 mmol, 16.2 % yield). $^1$H NMR (400 MHz, $CD_3SOCD_3$) δ 1.42 (q, *J* = 13 Hz, 2 H), 1.57 (q, *J* = 11 Hz, 2 H), 2.02 (br d, *J* = 11 Hz, 2 H), 2.30 (br d, *J* = 12 Hz, 2 H), 2.93 (s, 6 H), 2.90-3.00 (m, 1 H), 3.76-3.88 (m, 1 H), 7.63 (t, *J* = 73 Hz, 1 H), 7.95 (s, 1 H), 8.45 (s, 1 H), 8.67 (d, *J* = 7 Hz, 1 H), 8.81 (s, 1 H), 9.31 (s, 1 H); LC-MS (LC-ES) M+H = 414.

**Example 209**

**6-Chloro-7-fluoro-N-((trans)-4-(2-hydroxypropan-2-yl)cyclohexyl)quinoline-3-carboxamide**

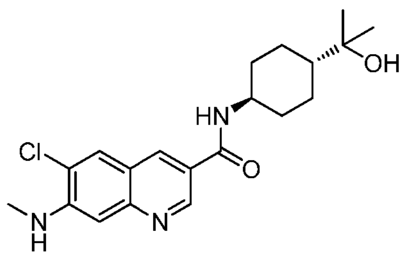

6-chloro-7-fluoro-N-((trans)-4-(2-hydroxypropan-2-yl)cyclohexyl)quinoline-3-carboxamide (Intermediate 85) (52 mg, 0.143 mmol), methanamine (0.035 mL, 0.285 mmol), and N,N-diisopropylethylamine (0.124 mL, 0.713 mmol) were suspended in acetonitrile (1.064 mL) and refluxed overnight and subsequently monitored by LC-MS. After heating overnight a solid could be observed within the solution. An excess (~20 eq) of the amine was then added to the reaction, which was then heated again at 115 °C for 15 more hours. The residue was purified using silica gel chromatography, eluting with 0-80% ethyl acetate:hexanes to give the title compound (34 mg, 0.086 mmol, 60% yield). $^1$H NMR (400 MHz, $CD_3OD$) δ 1.13-1.52 (m, 12 H), 1.90-2.18 (m, 4 H), 3.02 (s, 3 H), 3.37 (s, 1 H), 3.88 (t, *J* = 11 Hz, 1 H), 7.00 (s,1 H), 7.94 (s, 1 H), 8.51 (s, 1 H), 9.04 (s, 1 H); LC-MS (LC-ES) M+H = 376.

**Example 210**

**6-Chloro-7-(difluoromethoxy)-N-((trans)-(morpholine-4-carbonyl)cyclohexyl)quinoline-3-carboxamide**

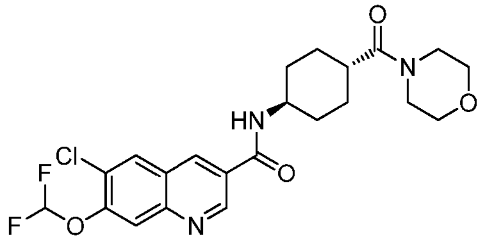

To a solution of (trans)-4-(6-chloro-7-(difluoromethoxy)quinoline-3-carboxamido)cyclohexanecarboxylic acid (Intermediate 86) (40 mg, 0.100 mmol) in N,N-dimethylformamide (1.017 mL) was added N,N-diisopropylethylamine (26.3 μL, 0.150 mmol) and O-(7-azabenzotriazol-1-yl)-N,N,N',N'-tetramethyluronium hexafluorophosphate (47.7 mg, 0.125 mmol). The mixture was stirred for 3-5 minutes prior to the addition of morpholine (26 μL, 0.301 mmol) and N,N-diisopropylethylamine (26.3 μL, 0.150 mmol). The resulting mixture was stirred at room temperature overnight and subsequently monitored by LC-MS. Water was then added to the flask and the product subsequently crashed out as a precipitate. The mixture was then filtered *via* vacuum filtration and dried to give 6-chloro-7-(difluoromethoxy)-N-((trans)-4-(morpholine-4-carbonyl)cyclohexyl)quinoline-3-carboxamide (27 mg, 0.055 mmol, 55% yield). $^1$H NMR (400 MHz, $CD_3OD$) δ 1.53 (q, *J* = 12 Hz, 2 H), 1.62-1.76 (m, 2 H), 1.90 (d, *J* = 14 Hz, 2 H), 2.15 (d, *J* = 12 Hz, 2 H), 2.70 (t, *J* = 12 Hz, 1 H), 3.54-3.76 (m, 8 H), 3.97 *(t, J* = 12 Hz, 1 H), 6.94-7.44 (m, 1 H), 7.90 (s, 1 H), 8.28 (s, 1 H), 8.73 (s, 1 H), 9.26 (s, 1 H); LC-MS (LC-ES) M+H = 468.

## Example 211

### 6-Chloro-7-(difluoromethoxy)-N-((trans)-4-(4-methylpiperazine-1-carbonyl)cyclohexyl)quinoline-3-carboxamide

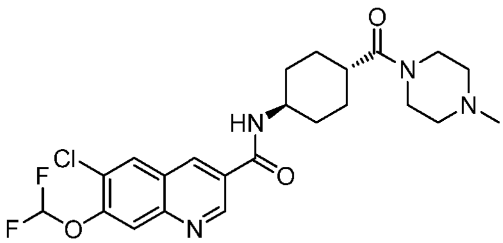

To a solution of (trans)-4-(6-chloro-7-(difluoromethoxy)quinoline-3-carboxamido)cyclohexanecarboxylic acid (43 mg, 0.108 mmol) (Intermediate 86) in N,N-dimethylformamide (1.017 mL) was added N,N-diisopropylethylamine (0.028 mL, 0.162 mmol) and O-(7-azabenzotriazol-1-yl)-N,N,N',N'-tetramethyluronium hexafluorophosphate (51.2 mg, 0.135 mmol). The mixture was stirred for 3-5 minutes prior to the addition of 1-methylpiperazine (0.036 mL, 0.323 mmol) and N,N-diisopropylethylamine (0.028 mL, 0.162 mmol). The resulting solution was stirred at room temperature and subsequently monitored by LC-MS. Water was added to the flask and the product subsequently crashed out as a precipitate. The mixture was then filtered *via* vacuum filtration and dried to give 6-chloro-7-(difluoromethoxy)-N-((trans)-4-(4-methylpiperazine-1-carbonyl)cyclohexyl)quinoline-3-carboxamide (13.6 mg, 0.028 mmol, 26% yield). $^1$H NMR (400 MHz, $CD_3OD$) δ 1.53 (q, *J* = 12 Hz, 2 H), 1.62-1.76 (m, 2 H), 1.90 (d, *J* = 14 Hz, 2 H), 2.15 (d, *J* = 12 Hz, 2 H), 2.70 (t, *J* = 12 Hz, 1 H), 3.32 (s, 3 H), 3.54-3.76 (m, 8 H), 3.97 (t, *J* = 12 Hz, 1 H), 6.94-7.44 (m, 1 H), 7.90 (s, 1 H), 8.28 (s, 1 H), 8.73 (s, 1 H), 9.26 (s, 1 H); LC-MS (LC-ES) M+H = 481.

## Example 212

### 6-Chloro-N-((trans)-4-(2-hydroxypropan-2-yl)cyclohexyl)-7-(isopropvlamino)quinoline-3-carboxamide

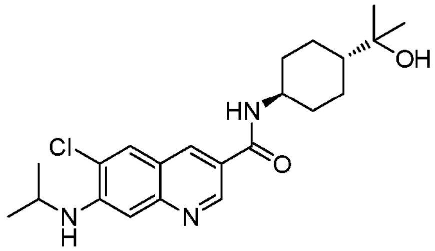

To a solution of ethyl 6-chloro-7-(isopropylamino)quinoline-3-carboxylate (Intermediate 87) (88 mg, 0.316 mmol) in a methanol (3.850 mL):tetrahydrofuran (3.85 mL) solution was added sodium hydroxide (63.1 mg, 1.579 mmol) in water (1.5 mL). The reaction was stirred at room temperature for 30 minutes, after which the solution was acidified with 1 M hydrochloric acid to a pH of ~5 and a white precipitate persisted in solution. The white precipitate was dried via vacuum filtration and added to a round bottom flask. The crude intermediate was dissolved in N,N-dimethylformamide prior to the addition of N,N-diisopropylethylamine (0.083 mL, 0.474 mmol) and O-(7-azabenzotriazol-1-yl)-N,N,N',N'-tetramethyluronium hexafluorophosphate (150 mg, 0.395 mmol). After 5 minutes of stirring, 2-((trans)-4-aminocyclohexyl)propan-2-ol (74.5 mg, 0.474 mmol) and N,N-diisopropylethylamine (0.083 mL, 0.474 mmol) were added and the reaction was subsequently monitored by LC-MS. Water was added to the reaction mixture in order to crash out the product which was vacuum filtered to give the title compound (81 mg, 0.201 mmol, 64% yield). $^1$H NMR (400 MHz, $CD_3OD$) δ 1.09-1.52 (m, 19 H), 1.89-2.20 (m, 4 H), 3.77-4.02 (m, 2 H), 7.10 (s, 1 H), 7.98 (s, 1 H), 8.51 (s, 1 H), 9.05 (s, 1 H); LC-MS (LC-ES) M+H = 404.

## Example 213

### 6-Chloro-7-(cyclopropylamino)-N-((trans)-4-(2-hydroxypropan-2 yl)cyclohexyl)quinoline-3-carboxamide

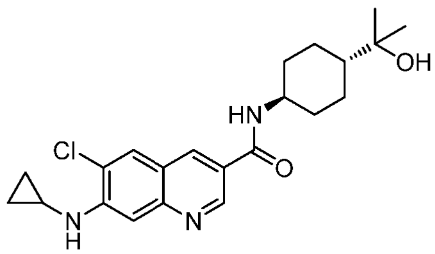

To a stirring suspension of 6-chloro-7-(cyclopropylamino)quinoline-3-carboxylic acid (54 mg, 0.206 mmol) (Intermediate 88) in N,N-dimethylformamide (0.827 mL) was added N,N-diisopropylethylamine (0.054 mL, 0.308 mmol) followed by O-(7-azabenzotriazol-1-yl)-N,N,N',N'-tetramethyluronium hexafluorophosphate (98 mg, 0.257 mmol) in one portion. After ~5 minutes, 2-((trans)-4-aminocyclohexyl)propan-2-ol (48.5 mg, 0.308 mmol) and N,N-diisopropylethylamine (0.054 mL, 0.308 mmol) were added. The reaction was stirred at room temperature and subsequently monitored by LC-MS. The N,N-dimethylformamide was removed and resulting mixture was dissolved in a acetonitrile:water mixture in which a precipitate persisted. The solid was filtered via vacuum filtration to give 6-chloro-7-(cyclopropylamino)-N-((trans)-4-(2-hydroxypropan-2-yl)cyclohexyl)quinoline-3-carboxamide (48 mg, 0.116 mmol, 56% yield). $^1$H NMR (400 MHz, $CD_3OD$) δ 0.55-0.76 (m, 2 H), 0.86-1.04 (m, 2 H), 1.14-1.55 (m, 12 H), 1.88-2.20 (m, 5 H), 2.61 (tt, $J$ = 7, 4 Hz, 1 H), 3.33 (dt, $J$ = 3, 2 Hz, 1 H), 3.79-3.98 (m, 1 H), 7.51 (s, 1 H), 7.95 (s, 1 H), 8.53 (d, $J$ = 2 Hz, 1 H), 9.06 (d, $J$ = 2 Hz, 1 H); LC-MS (LC-ES) M+H = 402.

## Example 214

### 6-Chloro-7-(difluoromethoxy)-N-((trans)-3-(3-hydroxy-3-(trifluoromethyl)azetidin-1-yl)cyclobutyl)quinoline-3-carboxamide

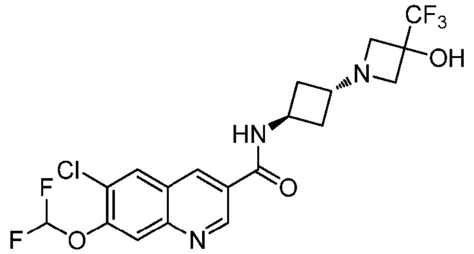

3-(Trifluoromethyl)azetidin-3-ol hydrochloride (179 mg, 1.007 mmol) was added to 6-chloro-7-(difluoromethoxy)-N-(3-oxocyclobutyl)quinoline-3-carboxamide (311.9 mg, 0.915 mmol) (Intermediate 89) in 1,2-dichloroethane (4.577 mL) at room temperature and the reaction mixture was stirred for 5 minutes, followed by the addition of N,N-diisopropylethylamine (0.160 mL, 0.915 mmol) and 4A molecular sieves (200 mg). The reaction was stirred for 3 hours at room temperature followed by the addition of sodium triacetoxyborohydride (194 mg, 0.915 mmol). The resulting solution was then stirred at room temperature and monitored subsequently by LC-MS. The reaction was filtered through Celite® and sodium bicarbonate was added to the flask. The product was then extracted with dichloromethane and dried over magnesium sulfate. Some solid in the flask would not dissolve in either the dichloromethane or water. Separately, methanol was added which dissolved the solid. LC-MS determined that the solids contained both product and undesired material. This material, now dissolved in methanol was added to an Erlenmeyer flask containing the original dichloromethane organics and magnesium sulfate. After stirring for an additional 5 minutes the solution was filtered and concentrated *in vacuo* giving the title compound (>13 mg). $^{1}$H NMR (400 MHz, $CDCl_3$) δ 1.82-1.92 (m, 2 H), 2.60-2.72 (m, 2 H), 2.98-3.10 (m, 1 H), 3.39 (br d, *J* = 10 Hz, 2 H), 3.54 (br d, *J* = 10 Hz, 2 H), 4.46-4.56 (m, 1 H), 6.68 (t, *J* = Hz, 1 H), 6.98 (br s, 1 H), 7.85 (s, 1 H), 7.92 (s, 1 H), 8.38 (s, 1 H), 9.19 (br d, *J* = 2 Hz, 1 H); LC-MS (LC-ES) M+H = 466.

**Example 215**

**8-Chloro-N-((*trans*)-4-(2-hydroxypropan-2-yl)cyclohexyl)-7-(trifluoromethoxy)quinoline-3-carboxamide and 6-Chloro-N-((*trans*)-4-(2-hydroxypropan-2-yl)cyclohexyl)-7-(trifluoromethoxy)quinoline-3-carboxamide**

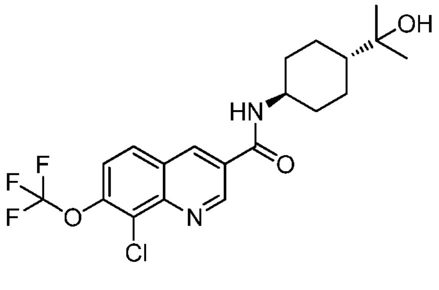

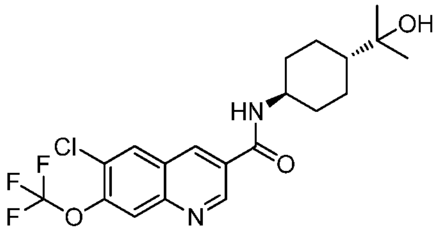

A white powder consisting of a ~1:1 mixture of 6-chloro-7-(trifluoromethoxy)quinoline-3-carboxylic acid (23.00 mg, 0.079 mmol) and 8-chloro-7-(trifluoromethoxy)quinoline-3-carboxylic acid (23 mg, 0.079 mmol) (Intermediate 90) was dissolved into N,N-dimethylformamide (3 mL) and stirred at room temperature. To the stirring solution was added N,N-diisopropylethylamine, 2-((*trans*)-4-aminocyclohexyl)propan-2-ol (8.90 mg, 0.0570 mmol), and n-propylphosphonic acid anhydride (0.250 mL, 0.786 mmol, 50% in ethyl acetate). The pale yellow solution was monitored by TLC/LC-MS and upon completion (45 min), water (1 mL) was added to the stirring solution. The resulting suspension was transferred to a separatory funnel along with ethyl acetate (30 mL). The organic layer was washed subsequently with 1 M hydrochloric acid (2X, 30 mL), saturated sodium bicarbonate (2X, 30 mL), and water (30 mL). The organic layer was collected, dried over sodium sulfate, and concentrated to dryness *in vacuo.* Further purification using reverse phase preparatory HPLC (Agilent, C18, acetonitrile:water) gave 8-chloro-N-((*trans*)-4-(2-hydroxypropan-2-yl)cyclohexyl)-7-(trifluoromethoxy)quinoline-3-carboxamide (23.0 mg, 0.0530 mmol, 67.7% yield) and 6-chloro-N-((*trans*)-4-(2-hydroxypropan-2-yl)cyclohexyl)-7-(trifluoromethoxy)quinoline-3-carboxamide (5 mg, 0.012 mmol, 14.71% yield).

**8-Chloro-N-((*trans*)-4-(2-hydroxypropan-2-yl)cyclohexyl)-7-(trifluoromethoxy)quinoline-3-carboxamide**

$^{1}$H NMR (400 MHz, $CD_3SOCD_3$) δ 0.99-1.26 (m, 9 H), 1.27-1.43 (m, 2 H), 1.87 (d, *J* = 11 Hz, 2 H), 1.98 (d, *J*

= 10 Hz, 2 H), 3.72-3.84 (m, 1 H), 4.06 (br s, 1 H), 7.90 (dd, *J* = 9, 1 Hz, 1 H), 8.28 (d, *J* = 9 Hz, 1 H), 8.71 (d, *J* = 8 Hz, 1 H), 8.98 (d, *J* = 2 Hz, 1 H), 9.43 (d, *J* = 2 Hz, 1 H); LC-MS (LC-ES) M+H = 431.

**6-Chloro-N-((*trans*)-4-(2-hydroxypropan-2-yl)cyclohexyl)-7-(trifluoromethoxy)quinoline-3-carboxamide**

$^1$H NMR (400 MHz, $CD_3SOCD_3$) δ 1.05-1.25 (m, 9 H), 1.27-1.41 (m, 2 H), 1.87 (d, *J* = 12 Hz, 2 H), 1.97 (d, *J* = 10 Hz, 2 H), 3.78 (br s, 1 H), 4.08 (s, 1 H), 8.23 (s, 1 H), 8.57 (s, 1 H), 8.70 (d, *J* = 8 Hz, 1 H), 8.86 (s, 1 H), 9.36 (s, 1 H); LC-MS (LC-ES) M+H = 431.

**Example 216**

**8-Chloro-7-(difluoromethoxy)-N-((*trans*)-4-(2-hydroxypropan-2-yl)cyclohexyl)quinoline-3-carboxamide**

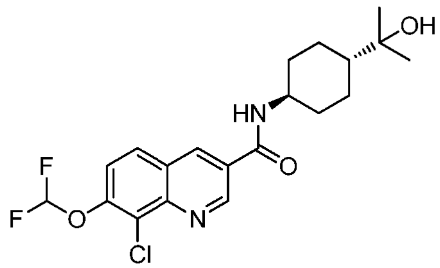

To a stirred solution of 8-chloro-7-(difluoromethoxy)quinoline-3-carboxylic acid (23 mg, 0.084 mmol) (Intermediate 91) and 2-((*trans*)-4-aminocyclohexyl)propan-2-ol (13.2 mg, 0.0840 mmol) in N,N-dimethylformamide (3 mL) was added N,N-diisopropylethylamine (10.9 mg, 0.0840 mmol), followed by n-propylphosphonic acid anhydride (0.136 mL, 0.252 mmol, 50% in ethyl acetate). After the consumption of starting material was observed by LC-MS (20 min), the reaction was subsequently quenched with water (1 mL) and stirred for an additional 10 minutes. The mixture was then diluted with ethyl acetate (75 mL) transferred to a 125 mL separatory funnel and washed with 1 M hydrochloric acid (50 mL, 2X), saturated sodium bicarbonate (50 mL, 2X), and water (50 mL, 2X). The organic layer was collected, dried over sodium sulfate, and concentrated to *in vacuo* to yield a tan solid. A further purification was achieved through column chromatography (12 g ISCO, silica, hexanes:ethyl acetate) to give the title compound as a white powder (30.0 mg, 0.0730 mmol, 86% yield). $^1$H NMR (400 MHz, $CD_3SOCD_3$) δ 1.06 (s, 6 H), 1.10-1.28 (m, 3 H), 1.34 (q, *J* = 12 Hz, 2 H), 1.86 (d, *J*= 12 Hz, 2 H), 1.98 (brd, *J*= 12 Hz, 2 H), 3.68-3.88 (m, 1 H), 4.08 (s, 1 H), 7.52 (t, *J* = 72 Hz, 1 H), 7.76 (d, *J* = 9 Hz, 1 H), 8.22 (d, *J* = 9 Hz, 1 H), 8.66 (d, *J* = 8 Hz, 1 H), 8.92 (d, *J* = 2 Hz, 1 H), 9.39 (d, *J* = 2 Hz, 1 H); LC-MS (LC-ES) M+H = 413.

**Example 217**

**6.8-Dichloro-7-(difluoromethoxy)-N-((*trans*)-4-(2-hydroxypropan-2-yl)cyclohexyl)quinoline-3-carboxamide**

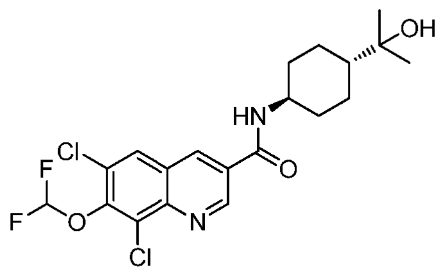

To a stirred solution of 6,8-dichloro-7-(difluoromethoxy)quinoline-3-carboxylic acid (35.0 mg, 0.114 mmol) (Intermediate 92) and 2-((trans)-4-aminocyclohexyl)propan-2-ol (17.9 mg, 0.114 mmol) in N,N-dimethylformamide (3 mL) was added n-propylphosphonic acid anhydride (0.123 mL, 0.227 mmol, 50% in ethyl acetate) followed by N,N-diisopropylethylamine (0.0400 mL, 0.227 mmol). After consumption of starting material was observed by LC-MS (15 min), the reaction was quenched with water (1 mL) and stirred for an additional 10 minutes. The mixture was then diluted with ethyl acetate (75 mL) transferred to a 125 mL separatory funnel and washed with 1 M hydrochloric acid (50 mL, 2X), saturated sodium bicarbonate (50 mL, 2X), and water (50 mL, 2X). The organic layer was collected, dried over sodium sulfate, concentrated to minimal volume *in vacuo,* and purified by silica chromatography (0-100% hexanes:ethyl acetate). The pure fractions were collected and concentrated to dryness via evaporation to yield the title compound as a white powder (37.0 mg, 0.0720 mmol, 63.3% yield). $^1$H NMR (400 MHz, $CD_3SOCD_3$) $\delta$ 1.04-1.40 (m, 9 H), 1.34 (q, *J* = 12 Hz, 2 H), 1.87 (d, *J* = 12 Hz, 2 H), 1.98 (d, *J* = 12 Hz, 2 H), 3.77 (m, 1 H), 4.06 (br s, 1 H), 7.34 (t, *J* = 72 Hz, 1 H), 8.50 (s, 1 H), 8.73 (d, *J* = 8 Hz, 1 H), 8.88 (d, *J* = 2 Hz, 1 H), 9.40 (d, *J* = 2 Hz, 1 H); LC-MS (LC-ES) M+H = 447.

**Example 218**

**8-Chloro-7-ethoxy-N-(trans-4-(2-hydroxypropan-2-yl)cyclohexyl)quinoline-3-carboxamide**

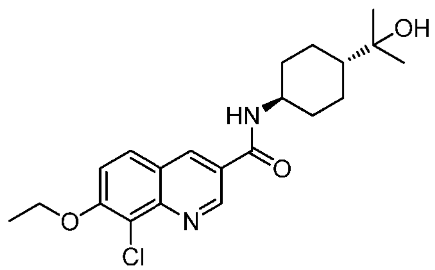

To a stirred solution of 8-chloro-7-ethoxyquinoline-3-carboxylic acid (115 mg, 0.457 mmol) (Intermediate 93) and 2-((*trans*)-4-aminocyclohexyl)propan-2-ol (71.9 mg, 0.457 mmol) in N,N-dimethylformamide (3 mL) was added n-propylphosphonic acid anhydride (0.494 mL, 0.914 mmol, 50% in ethyl acetate) followed by N,N-diisopropylethylamine (0.160 mL, 0.914 mmol). After consumption of starting material was observed by LC-MS (30 min), the reaction was subsequently quenched with water (1 mL) and stirred for an additional minute. The mixture was then diluted with ethyl acetate (75 mL) transferred to a 125 mL separatory funnel and washed with 1 M hydrochloric acid (50 mL, 2X), saturated sodium bicarbonate (50 mL, 2X), and water (50 mL, 2X). The organic layer was collected, dried over sodium sulfate, and concentrated to dryness via evaporation. A recrystallization from ethyl acetate gave the title compound as a white solid (127 mg, 0.325 mmol, 71.1% yield) $^1$H NMR (400 MHz, $CD_3SOCD_3$) $\delta$ 0.97-1.26 (m, 9 H), 1.27-1.40 (m, 2 H), 1.44 (t, *J* = 7 Hz, 3 H), 1.87 (d, *J* = 12 Hz, 2 H), 1.97 (d, *J* = 12 Hz, 2 H), 3.62-3.89 (m, 1 H), 4.08 (s, 1 H), 4.37 (q, *J* = 7 Hz, 2 H), 7.72 (d, *J* = 9.3 Hz, 1 H), 8.10 (d, *J* = 9.3 Hz, 1 H), 8.55 (d, *J* = 7.8 Hz, 1 H), 8.81 (d, *J* = 2.0 Hz, 1 H), 9.29 (d, *J* = 2.3 Hz, 1 H); LC-MS (LC-ES) M+H = 391.

**Example 220**

**6-Chloro-7-(difluoromethoxy)-N-(1-(2-hydroxy-2-methylpropanoyl)piperidin-4-yl)quinoline-3-carboxamide**

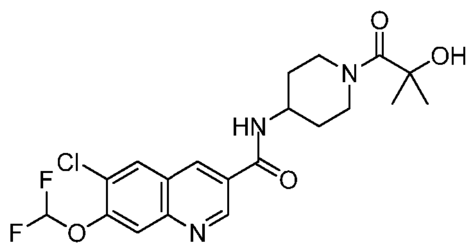

N,N-Diisopropylethylamine (0.128 mL, 0.735 mmol) was added to 6-chloro-7-(difluoromethoxy)quinoline-3-carboxylic acid (0.0670 g, 0.245 mmol, Intermediate 20) in N,N-dimethylformamide (1.224 mL) at room temperature. Then, O-(7-azabenzotriazol-1-yl)-N,N,N',N'-tetramethyluronium hexafluorophosphate (0.093 g, 0.245 mmol) was added and the reaction mixture was stirred for five minutes. Then, 1-(4-aminopiperidin-1-yl)-2-hydroxy-2-methylpropan-1-one

(0.050 g, 0.269 mmol, Intermediate 94) was added and the reaction mixture was stirred for sixteen hours. The reaction mixture was concentrated. The resulting residue was purified by RP HPLC, eluting with acetonitrile:water with 0.1% ammonium hydroxide (5:95:100:0) to give 6-chloro-7-(difluoromethoxy)-N-(1-(2-hydroxy-2-methylpropanoyl)piperidin-4-yl)quinoline-3-carboxamide (0.0760 g, 0.163 mmol, 66.7 % yield). $^1H$ NMR (400 MHz, $CD_3SOCD_3$) $\delta$ 1.33 (s, 6 H), 1.38-1.60 (m, 2 H), 1.89 (d, *J* = 12 Hz, 2 H), 2.64-3.22 (m, 2 H), 4.02-4.18 (m, 1 H), 4.20-4.90 (m, 2 H), 5.36 (s, 1 H), 7.61 (t, *J* = 73 Hz, 1 H), 7.94 (s, 1 H), 8.44 (s, 1 H), 8.68 (d, *J* = 8 Hz, 1 H), 8.80 (s, 1 H), 9.29 (s, 1 H); LC-MS (LC-ES) M+H = 442.

**Example 221**

**6-Chloro-7-(difluoromethoxy)-N-(1-(2-hydroxy-2-methylpropanoyl)azetidin-3-yl)quinoline-3-carboxamide**

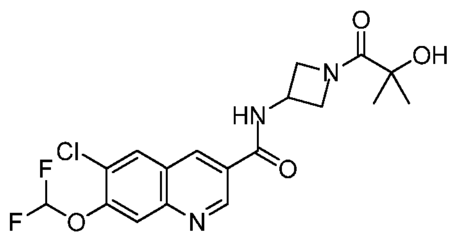

N,N-Diisopropylethylamine (0.150 mL, 0.858 mmol) was added to 6-chloro-7-(difluoromethoxy)quinoline-3-carboxylic acid (0.0783 g, 0.286 mmol, Intermediate 20) in 1,4-dioxane (1.431 mL) at room temperature. Then, O-(7-azabenzotriazol-1-yl)-N,N,N',N'-tetramethyluronium hexafluorophosphate (0.109 g, 0.286 mmol) was added and the reaction mixture was stirred for five minutes. Then, 1-(3-aminoazetidin-1-yl)-2-hydroxy-2-methylpropan-1-one (0.045 g, 0.286 mmol, Intermediate 95) was added and the reaction mixture was stirred for sixteen hours. The reaction mixture was concentrated. The resulting residue was purified by RP HPLC, eluting with acetonitrile:water with 0.1% ammonium hydroxide (5:95:100:0) to give 6-chloro-7-(difluoromethoxy)-N-(1-(2-hydroxy-2-methylpropanoyl)azetidin-3-yl)quinoline-3-carboxamide (0.0952 g, 0.219 mmol, 76 % yield). $^1H$ NMR (400 MHz, $CD_3SOCD_3$) $\delta$ 1.26 (s, 6 H), 3.90 (d, *J* = 8 Hz, 1 H), 4.17 (t, *J* = 8 Hz, 1 H), 4.32-4.44 (m, 1 H), 4.64-4.76 (m, 2 H), 5.07 (s, 1 H), 7.61 (t, *J* = 73 Hz, 1 H), 7.95 (s, 1 H), 8.45 (s, 1 H), 8.83 (s, 1 H), 9.33 (s, 1 H), 9.38 (s, 1 H); LC-MS (LC-ES) M+H = 414.

**Example 222**

**6-Chloro-7-(2,2-difluorocyclopropyl)-N-((trans)-4-(2-hydroxypropan-2-yl)cyclohexyl)quinoline-3-carboxamide**

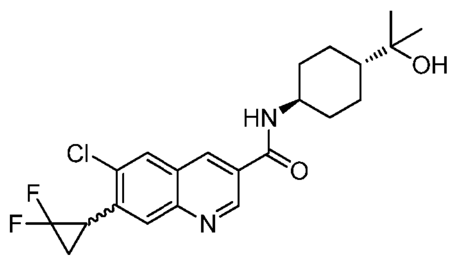

In a 5 mL vial was suspended 6-chloro-7-(2,2-difluorocyclopropyl)quinoline-3-carboxylic acid (24 mg, 0.085 mmol) (Intermediate 96) in N,N-dimethylformamide (0.564 mL) after which N,N-diisopropylethylamine (0.022 mL, 0.127 mmol) and O-(7-azabenzotriazol-1-yl)-N,N,N',N'-tetramethyluronium hexafluorophosphate (40.2 mg, 0.106 mmol) were added. After 5 minutes of stirring, 2-((trans)-4-aminocyclohexyl)propan-2-ol (19.96 mg, 0.127 mmol) and another portion of N,N-diisopropylethylamine (0.022 mL, 0.127 mmol) were added to the reaction mixture, which was stirred overnight. Water (3 mL) was added to the reaction mixture and a brown precipitate formed. The precipitate was vacuum filtered to give 6-chloro-7-(2,2-difluorocyclopropyl)-N-((trans)-4-(2-hydroxypropan-2-yl)cyclohexyl)quinoline-3-carboxamide as a brown solid (10 mg, 0.024 mmol, 28% yield). LC-MS (LC-ES) M+H = 423.

**Example 223**

**6-Chloro-7-(difluoromethoxy)-N-((cis)-4-hydroxy-4-(trifluoromethyl)cyclohexyl)quinoline-3-carboxamide**

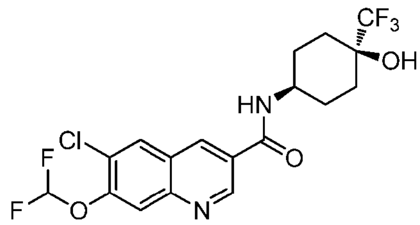

6-Chloro-7-(difluoromethoxy)quinoline-3-carboxylic acid (20 mg, 0.073 mmol) (Intermediate 20) was suspended in N,N-dimethylformamide (0.487 mL), after which N,N-diisopropylethylamine (0.019 mL, 0.110 mmol) and O-(7-azabenzotriazol-1-yl)-N,N,N',N'-tetramethyluronium hexafluorophosphate (34.7 mg, 0.091 mmol) were added. After 5 minutes of stirring, (cis)-4-amino-1-(trifluoromethyl)cyclohexanol (20.08 mg, 0.110 mmol) and another portion of N,N-diisopropylethylamine (0.019 mL, 0.110 mmol) were added to the reaction mixture. Upon consumption of the starting material the solution was stripped down to the aqueous layer and extracted with ethyl acetate (3X, 5 mL). The organic layers were combined, dried over magnesium sulfate, filtered, and concentrated *in vacuo* to give a crude orange oil. The residue was purified via silica gel chromatography, eluting with ethyl acetate:hexanes (0:1 to 1:1) to give 6-chloro-7-(difluoromethoxy)-N-((cis)-4-hydroxy-4-(trifluoromethyl)cyclohexyl)quinoline-3-carboxamide (19 mg, 0.043 mmol, 59% yield). $^1$H NMR (400 MHz, $CD_3OD$) δ 1.63-2.06 (m, 8 H), 3.86-4.07 (m, 1 H), 6.92-7.49 (m, 1 H), 7.86 (s, 1 H), 8.25 (s, 1 H), 8.72 (d, *J* = 2 Hz, 1 H), 9.25 (d, *J* = 2 Hz, 1 H); LC-MS (LC-ES) M+H = 439.

**Example 224**

**6-Chloro-7-(difluoromethoxy)-N-(4-(2-hydroxy-2-methylpropanoyl)piperazin-1-yl)quinoline-3-carboxamide**

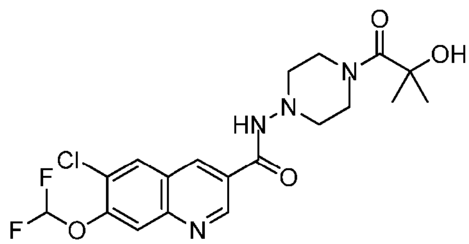

N,N-Diisopropylethylamine (0.213 mL, 1.221 mmol) was added to 6-chloro-7-(difluoromethoxy)quinoline-3-carboxylic acid (0.0835 g, 0.305 mmol, Intermediate 20) in N,N-dimethylformamide (1.526 mL) at room temperature. Then, O-(7-azabenzotriazol-1-yl)-N,N,N',N'-tetramethyluronium hexafluorophosphate (0.116 g, 0.305 mmol) was added and the reaction mixture was stirred for five minutes. Then, 1-(4-aminopiperazin-1-yl)-2-hydroxy-2-methylpropan-1-one hydrochloride (0.082 g, 0.366 mmol, Intermediate 97) was added and the reaction mixture was stirred for sixteen hours. The reaction mixture was concentrated. The resulting residue was purified by RP HPLC, eluting with acetonitrile:waterwith 0.1% ammonium hydroxide (5:95:100:0), then further purified by silica gel chromatography, eluting with methanol:ethyl acetate (0:1 to 1:4) to give 6-chloro-7-(difluoromethoxy)-N-(4-(2-hydroxy-2-methylpropanoyl)piperazin-1-yl)quinoline-3-carboxamide (0.0476 g, 0.102 mmol, 33.5 % yield). $^1$H NMR (400 MHz, $CD_3SOCD_3$) δ 1.33 (s, 6 H), 2.93 (br s, 4 H), 3.42-4.22 (m, 4 H), 5.43 (s, 1 H), 7.61 (t, *J* = 73 Hz, 1 H), 7.94 (s, 1 H), 8.45 (s, 1 H), 8.75 (s, 1 H), 9.22 (s, 1 H), 9.88 (s, 1 H); LC-MS (LC-ES) M+H = 443.

**Example 225**

**6-Chloro-N-((trans)-4-(2-hydroxypropan-2-yl)cyclohexyl)-7-vinylquinoline-3-carboxamide**

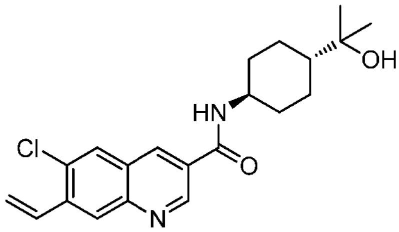

In a 5 mL vial was suspended 6-chloro-7-vinylquinoline-3-carboxylic acid (40 mg, 0.171 mmol) (Intermediate 98) in N,N-dimethylformamide (1.218 mL) after which N,N-diisopropylethylamine (0.045 mL, 0.257 mmol) and O-(7-azabenzotriazol-1-yl)-N,N,N',N'-tetramethyluronium hexafluorophosphate (81 mg, 0.214 mmol) were added. After 5 minutes of stirring, 2-((trans)-4-aminocyclohexyl)propan-2-ol (40.4 mg, 0.257 mmol) and another portion of N,N-diisopropylethylamine (0.045 mL, 0.257 mmol) were added to the reaction mixture. Upon disappearance of the starting material, the product was extracted using ethyl acetate (3X, 10 mL). The organic phases were washed with brine and water, dried over magnesium sulfate, filtered, and concentrated in vacuo to give 6-chloro-N-((trans)-4-(2-hydroxypropan-2-yl)cyclohexyl)-7-vinylquinoline-3-carboxamide as a white solid (28 mg, 0.075 mmol, 44% yield). $^1$H NMR (400 MHz, $CD_3SOCD_3$) $\delta$ 0.95-1.43 (m, 11 H), 1.77-2.02 (m, 4 H), 3.66-3.85 (m, 1 H), 4.08 (s, 1 H), 5.65 (d, *J* = 12 Hz, 1 H), 6.20(d, *J* = 18 Hz, 1 H), 7.18 (dd, *J* = 17, 11 Hz, 1 H), 8.28 (s, 1 H), 8.39 (s, 1 H), 8.62 (d, *J* = 8 Hz, 1 H), 8.74 (d, *J* = 2 Hz, 1 H), 9.27 (d, *J* = 2 Hz, 1 H); LC-MS (LC-ES) M+H = 373.

**Example 226**

**6-Chloro-7-(difluoromethoxy)-N-(trans-3-(2-hydroxy-2-methylpropanamido)cyclobutyl)quinoline-3-carboxamide**

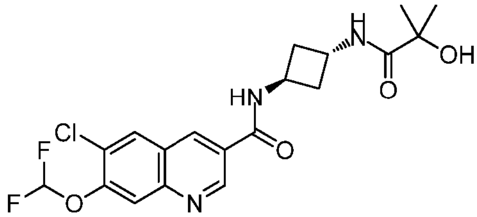

N,N-Diisopropylethylamine (0.218 mL, 1.251 mmol) was added to 6-chloro-7-(difluoromethoxy)quinoline-3-carboxylic acid (0.0856 g, 0.313 mmol, Intermediate 20) in N,N-dimethylformamide (1.564 mL) at room temperature. Then, O-(7-azabenzotriazol-1-yl)-N,N,N',N'-tetramethyluronium hexafluorophosphate (0.119 g, 0.313 mmol) was added and the reaction mixture was stirred for five minutes. Then, N-(trans-3-aminocyclobutyl)-2-hydroxy-2-methylpropanamide hydrochloride (0.065 g, 0.313 mmol, Intermediate 99) was added and the reaction mixture was stirred for sixteen hours. The reaction mixture was concentrated. The resulting residue was purified by RP HPLC, eluting with acetonitrile:waterwith 0.1% ammonium hydroxide (5:95:100:0), then further purified by silica gel chromatography, eluting with methanol:ethyl acetate (0:1 to 1:19) to give 6-chloro-7-(difluoromethoxy)-N-(trans-3-(2-hydroxy-2-methylpropanamido)cyclobutyl)quinoline-3-carboxamide (0.0584 g, 0.130 mmol, 41.5 % yield). $^1$H NMR (400 MHz, $CD_3SOCD_3$) $\delta$ 1.24 (s, 6 H), 2.28-2.46 (m, 4 H), 4.36-4.54 (m, 2 H), 5.30 (s, 1 H), 7.61 (t, *J* = 73 Hz, 1 H), 7.94 (s, 1 H), 7.95 (d, *J* = 7 Hz, 1 H), 8.44 (s, 1 H), 8.81 (s, 1 H), 9.11 (d, *J* = 7 Hz, 1 H), 9.32 (s, 1 H); LC-MS (LC-ES) M+H = 428.

**Example 227**

**6-Chloro-7-(difluoromethoxy)-N-((trans)-hydroxy-4-(trifluoromethyl)cyclohexyl)quinoline-3-carboxamide**

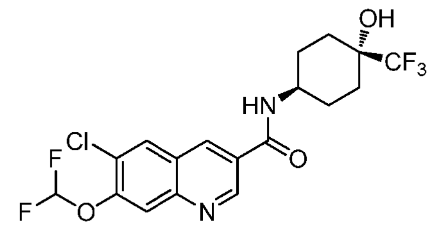

In a 5 mL vial was suspended 6-chloro-7-(difluoromethoxy)quinoline-3-carboxylic acid (50 mg, 0.183 mmol) (Intermediate 20) in N,N-dimethylformamide (1.218 mL) after which N,N-diisopropylethylamine (0.048 mL, 0.274 mmol) and O-(7-azabenzotriazol-1-yl)-N,N,N',N'-tetramethyluronium hexafluorophosphate (87 mg, 0.228 mmol) were added. After 5 minutes of stirring, (trans)-4-amino-1-(trifluoromethyl)cyclohexanol (50.2 mg, 0.274 mmol) and another portion of N,N-diisopropylethylamine (0.048 mL, 0.274 mmol) were added to the reaction mixture. After consumption of the starting material the solution was diluted with water and extracted with ethyl acetate (3X, 5 mL). The organic layers were combined, dried over magnesium sulfate, filtered, and concentrated *in vacuo* to give a crude orange oil. The residue was purified via silica gel chromatography eluting with ethyl acetate:hexanes (0:1 to 1:1) to give 6-chloro-7-(difluoromethoxy)-N-((trans)-4-hydroxy-4-(trifluoromethyl)cyclohexyl)quinoline-3-carboxamide as a white solid (69 mg, 0.157 mmol, 86% yield. $^1$H NMR (400 MHz, $CD_3SOCD_3$) δ 1.49-2.05 (m, 8 H), 4.04-4.21 (m, 1 H), 5.78 (s, 1 H), 7.37-7.87 (m, 1 H), 7.96 (s, 1 H), 8.49 (s, 1 H), 8.64 (d, *J* = 7 Hz, 1 H), 8.77 (d, *J* = 2 Hz, 1 H), 9.26 (d, *J* = 2 Hz, 1 H); LC-MS (LC-ES) M+H = 439.

**Example 228**

**8-Chloro-7-(difluoromethoxy)-N-((*trans*)-4-(2-hydroxypropan-2-yl)cyclohexyl)-6-methylquinoline-3-carboxamide**

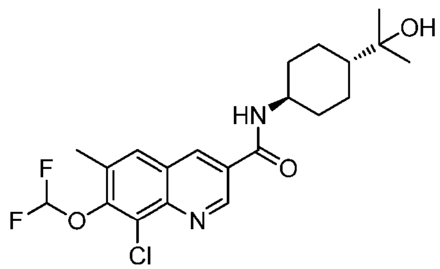

To a stirred solution of 2-((*trans*)-4-aminocyclohexyl)propan-2-ol (54.7 mg, 0.348 mmol) and 8-chloro-7-(difluoromethoxy)-6-methylquinoline-3-carboxylic acid (0.100 g, 0.348 mmol) (Intermediate 102) in N,N-dimethylformamide (3 mL) was added N,N-diisopropylethylamine (0.0610 mL, 0.348 mmol) followed by n-propylphosphonic acid anhydride (111 mg, 0.348 mmol, 50% in ethyl acetate). After consumption of starting material was observed by LC-MS (30.0 min), the reaction was quenched with water (1 mL) and stirred for an additional minute. The mixture was then diluted with ethyl acetate (75 mL) transferred to a 125 mL separatory funnel and washed with 1 M hydrochloric acid (50 mL, 2X), saturated sodium bicarbonate (50 mL, 2X), and water (50 mL, 2X). The organic layer was collected, dried over sodium sulfate, and concentrated to minimal volume via evaporation to yield a tan concentrate. This concentrate was then loaded onto an ISCO cartridge and purified by chromatography (silica, 12 g ISCO, (1:0 to 7:3) hexanes:ethyl acetate). Desired product eluted between (9:1) and (7:3) hexanes:ethyl acetate. Pure fractions were collected and concentrated to dryness *in vacuo* to give the title compound as a white powder (130.0 mg, 0.295 mmol, 85% yield). $^1$H NMR (400 MHz, $CD_3SOCD_3$) δ 0.99-1.27 (m, 9 H), 1.35 (q, *J* = 12 Hz, 2 H), 1.87 (d, *J* = 12 Hz, 2 H), 1.98 (d, *J* = 11 Hz, 2 H), 2.50 (s, 3 H), 3.64-3.86 (m, 1 H), 4.08 (s, 1 H), 7.28 (t, *J* = 72 Hz, 1 H), 8.05 (s, 1 H), 8.67 (d, J = 8 Hz, 1 H), 8.81 (d, *J* = 2 Hz, 1 H), 9.34 (d, *J* = 2 Hz, 1 H); LC-MS (LC-ES) M+H = 427.

**Example 229**

**6-Chloro-7-(difluoromethoxy)-N-((*trans*)-4-(2-hydroxypropan-2-yl)cyclohexyl)-8-methylquinoline-3-carboxamide**

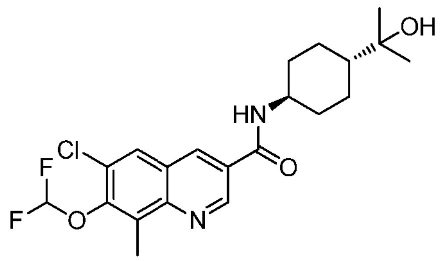

To a stirred solution of 2-((*trans*)-4-aminocyclohexyl)propan-2-ol (49.2 mg, 0.313 mmol) and 6-chloro-7-(difluoromethoxy)-8-methylquinoline-3-carboxylic acid (90.0 mg, 0.313 mmol) (Intermediate 101) in N,N-dimethylformamide (3 mL) was added N,N-diisopropylethylamine (0.057 mL, 0.344 mmol) followed by n-propylphosphonic acid anhydride (0.365 mL, 0.626 mmol, 50% in ethyl acetate). After consumption of starting material was observed by LC-MS (30 min) the reaction was quenched with water (1 mL) and stirred for an additional minutes. The mixture was then diluted with ethyl acetate (75 mL) transferred to a 125 mL separatory funnel and washed with 1 M hydrochloric acid (50 mL, 2X), saturated sodium bicarbonate (50 mL, 2X), and water (50 mL, 2X). The organic layer was collected, dried over sodium sulfate, and concentrated to minimal volume via evaporation to yield a tan concentrate. This concentrate was then loaded onto an ISCO cartridge and purified by silica chromatography (1:0 to 7:3 hexanes:ethyl acetate). Pure fractions were collected and dried via evaporation to give the title compound as a white powder (123 mg, 0.288 mmol, 92% yield). $^1$H NMR (400 MHz, $CD_3SOCD_3$) $\delta$ 0.97-1.26 (m, 9 H), 1.34 (q, *J* = 12 Hz, 2 H), 1.86 (d, *J* = 11 Hz, 2 H), 1.97 (d, *J* = 10 Hz, 2 H), 2.74 (s, 3 H), 3.67-3.85 (m, 1 H), 4.08 (s, 1 H), 7.22 (t, *J* = 72 Hz, 1 H), 8.31 (s, 1 H), 8.66 (d, *J* = 8 Hz, 1 H), 8.80 (d, *J* = 2 Hz, 1 H), 9.33 (d, *J* = 2.01 Hz, 1 H); LC-MS (LC-ES) M+H = 427.

**Example 230**

**6-Chloro-7-(2-fluoropropan-2-yl)-N-((trans)-4-(2-hydroxypropan-2-yl)cyclohexyl)quinoline-3-carboxamide**

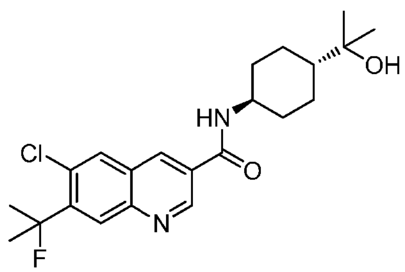

To a solution of 6-chloro-7-(2-fluoropropan-2-yl)quinoline-3-carboxylic acid (25 mg, 0.093 mmol) (Intermediate 103) in N,N-dimethylformamide (0.200 mL) was added O-(7-azabenzotriazol-1-yl)-N,N,N',N'-tetramethyluronium hexafluorophosphate (44.4 mg, 0.117 mmol) and N,N-diisopropylethylamine (0.024 mL, 0.140 mmol). The reaction was stirred for 5 minutes prior to the addition of 2-((trans)-4-aminocyclohexyl)propan-2-ol (22.03 mg, 0.140 mmol) and N,N-diisopropylethylamine (0.024 mL, 0.140 mmol). Upon consumption of the starting material the reaction was diluted in ethyl acetate and washed with water. The organics were dried over magnesium sulfate, filtered, and concentrated *in vacuo* to give a light yellow solid. The crude material was purified *via* silica gel chromatography, eluting with ethyl acetate:hexanes (0:1 to 1:0) to give 6-chloro-7-(2-fluoropropan-2-yl)-N-((trans)-4-(2-hydroxypropan-2-yl)cyclohexyl)quinoline-3-carboxamide as a light yellow/white solid (13 mg, 0.032 mmol, 34% yield). $^1$H NMR (400 MHz, $CD_3SOCD_3$) $\delta$ 0.97-1.42 (m, 11 H), 1.80-1.97 (m, 10 H), 3.70-3.84 (m, 1 H), 4.09 (s, 1 H), 8.22 (s, 1 H), 8.32 (s, 1 H), 8.66 (d, *J* = 8 Hz, 1 H), 8.78 (d, *J* = 2 Hz, 1 H), 9.30 (d, *J* = 2 Hz, 1 H); LC-MS (LC-ES) M+H = 407.

**Example 231**

**6-Chloro-7-(1,1-difluoroethyl)-N-((trans)-4-(2-hydroxypropan-2-yl)cyclohexyl)quinoline-3-carboxamide**

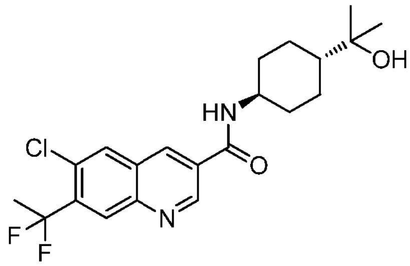

To a solution of 6-chloro-7-(1,1-difluoroethyl)quinoline-3-carboxylic acid (26.4 mg, 0.097 mmol) (Intermediate 104) in N,N-dimethylformamide (0.200 mL) was added O-(7-azabenzotriazol-1-yl)-N,N,N',N'-tetramethyluronium hexafluorophosphate (46.2 mg, 0.121 mmol) and N,N-diisopropylethylamine (0.025 mL, 0.146 mmol). The reaction was stirred for 5 minutes prior to the addition of 2-((trans)-4-aminocyclohexyl)propan-2-ol (22.92 mg, 0.146 mmol) and N,N-diisopropylethylamine (0.025 mL, 0.146 mmol). The reaction was diluted with -20 mL of ethyl acetate and washed with water (3X 5 mL). The organics were washed over magnesium sulfate, filtered, and concentrated *in vacuo* to give a crude light yellow solid. The crude mixture was purified via silica gel chromatography, eluting with ethyl acetate:hexanes (0:1 to 1:0), then further purified via silica gel chromatography, eluting with ethyl acetate:hexanes (1:1) to give 6-chloro-7-(1,1-difluoroethyl)-N-((trans)-4-(2-hydroxypropan-2-yl)cyclohexyl)quinoline-3-carboxamide as a white solid (23 mg, 0.053 mmol, 55% yield). $^1H$ NMR (400 MHz, $CD_3SOCD_3$) $\delta$ 0.95-1.43 (m, 11 H), 1.79-2.01 (m, 4 H), 2.19 (t, *J* = 19 Hz, 3 H), 3.77 (td, *J* = 8, 3 Hz, 1 H), 4.09 (s, 1 H), 8.31 (s, 1 H), 8.43 (s, 1 H), 8.71 (d, *J* = 8 Hz, 1 H), 8.83 (d, *J* = 2 Hz, 1 H), 9.35 (d, *J* = 2 Hz, 1 H); LC-MS (LC-ES) M+H = 411.

**Example 232**

**6-Chloro-7-(difluoromethoxy)-N-((1s,3s)-3-hydroxy-3-(trifluoromethyl)cyclobutyl)quinoline-3-carboxamide**

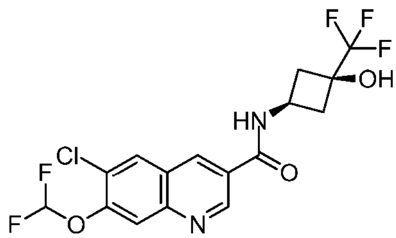

N,N-Diisopropylethylamine (0.202 mL, 1.159 mmol) was added to 6-chloro-7-(difluoromethoxy)quinoline-3-carboxylic acid (0.0793 g, 0.290 mmol, Intermediate 20) in N,N-dimethylformamide (0.966 mL) at room temperature. Then, (1s,3s)-3-amino-1-(trifluoromethyl)cyclobutan-1-ol hydrochloride (0.056 g, 0.290 mmol) was added and the reaction mixture was stirred for five minutes. Then, n-propylphosphonic acid anhydride (0.345 mL, 0.580 mmol, 50% in ethyl acetate) was added and the reaction mixture was stirred for sixteen hours. The reaction mixture was concentrated. The resulting residue was purified by RP HPLC, eluting with acetonitrile:water with 0.1% ammonium hydroxide (5:95:100:0) to give 6-chloro-7-(difluoromethoxy)-N-((1s,3s)-3-hydroxy-3-(trifluoromethyl)cyclobutyl)quinoline-3-carboxamide (0.0779 g, 0.180 mmol, 62.2 % yield). $^1H$ NMR (400 MHz, $CD_3SOCD_3$) $\delta$ 2.39 (br t, *J* = 10 Hz, 2 H), 2.76-2.86 (m, 2 H), 4.20 (h, *J* = 7 Hz, 1 H), 6.73 (s, 1 H), 7.62 (t, *J* = 73 Hz, 1 H), 7.94 (s, 1 H), 8.45 (s, 1 H), 8.82 (d, *J* = 2 Hz, 1 H), 9.23 (d, *J* = 7 Hz, 1 H), 9.31 (d, *J* = 2 Hz, 1 H); LC-MS (LC-ES) M+H = 411.

**Example 233**

**6-Chloro-7-(difluoromethoxy)-N-((1s,4s)-4-(difluoromethyl)-4-hydroxycyclohexyl)quinoline-3-carboxamide**

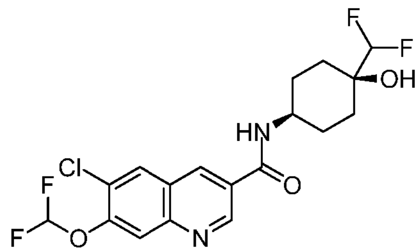

N,N-Diisopropylethylamine (0.185 mL, 1.057 mmol) was added to 6-chloro-7-(difluoromethoxy)quinoline-3-carboxylic acid (0.0723 g, 0.264 mmol, Intermediate 20) in N,N-dimethylformamide (0.881 mL) at room temperature. Then, (1s,4s)-4-amino-1-(difluoromethyl)cyclohexan-1-ol (0.061 g, 0.370 mmol, Intermediate 105) was added and the reaction mixture was stirred for five minutes. Then, n-propylphosphonic acid anhydride (0.315 mL, 0.528 mmol, 50% in ethyl acetate) was added and the reaction mixture was stirred for sixteen hours. The reaction mixture was concentrated. The resulting residue was purified by RP HPLC, eluting with acetonitrile:water with 0.1% ammonium hydroxide (5:95:100:0) to give 6-chloro-7-(difluoromethoxy)-N-((1s,4s)-4-(difluoromethyl)-4-hydroxycyclohexyl)quinoline-3-carboxamide (0.0945 g, 0.213 mmol, 81 % yield). $^1H$ NMR (400 MHz, $CD_3SOCD_3$) $\delta$ 1.40-1.52 (m, 2 H), 1.64 (br d, *J* = 12 Hz, 2 H), 1.68-1.82 (m, 4 H), 3.81 (h, *J* = 8 Hz, 1 H), 5.10 (s, 1 H), 5.69 (t, *J* = 56 Hz, 1 H), 7.62 (t, *J* = 73 Hz, 1 H), 7.93 (s, 1 H), 8.43 (s, 1 H), 8.72 (d, *J* = 8 Hz, 1 H), 8.80 (d, *J* = 2 Hz, 1 H), 9.30 (d, *J* = 2 Hz, 1 H); LC-MS (LC-ES) M+H = 421.

**Example 234**

**6-Chloro-7-(difluoromethoxy)-N-((1r,4r)-4-(difluoromethyl)-4-hydroxycyclohexyl)quinoline-3-carboxamide**

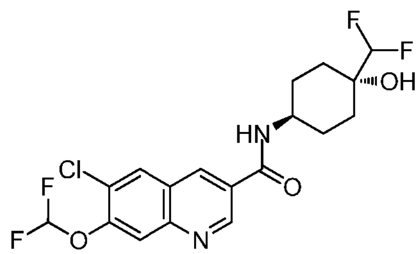

N,N-Diisopropylethylamine (0.208 mL, 1.191 mmol) was added to 6-chloro-7-(difluoromethoxy)quinoline-3-carboxylic acid (0.0815 g, 0.298 mmol, Intermediate 20) in N,N-dimethylformamide (0.993 mL) at room temperature. Then, (1r,4r)-4-amino-1-(difluoromethyl)cyclohexan-1-ol (0.059 g, 0.357 mmol, Intermediate 106) was added and the reaction mixture was stirred for five minutes. Then, n-propylphosphonic acid anhydride (0.355 mL, 0.596 mmol, 50% in ethyl acetate) was added and the reaction mixture was stirred for sixteen hours. The reaction mixture was concentrated. The resulting residue was purified by RP HPLC, eluting with acetonitrile:water with 0.1% ammonium hydroxide (5:95:100:0) to give 6-chloro-7-(difluoromethoxy)-N-((1r,4r)-4-(difluoromethyl)-4-hydroxycyclohexyl)quinoline-3-carboxamide (0.1200 g, 0.271 mmol, 91 % yield). $^1H$ NMR (400 MHz, $CD_3SOCD_3$) $\delta$ 1.38-1.52 (m, 2 H), 1.70 (br d, *J* = 9 Hz, 2 H), 1.78-1.94 (m, 4 H), 4.08 (br s, 1 H), 5.05 (s, 1 H), 5.72 (t, *J* = 56 Hz, 1 H), 7.61 (t, *J* = 73 Hz, 1 H), 7.94 (s, 1 H), 8.45 (s, 1 H), 8.50 (d, *J* = 7 Hz, 1 H), 8.76 (s, 1 H), 9.25 (s, 1 H); LC-MS (LC-ES) M+H = 421.

**Example 235**

**8-Chloro-7-(difluoromethoxv)-6-fluoro-N-(trans-4-(2-hvdroxypropan-2-yl)cyclohexyl)quinoline-3-carboxamide**

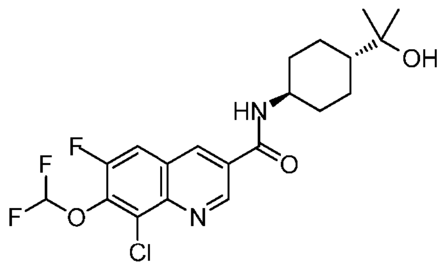

N,N-Diisopropylethylamine (0.098 mL, 0.561 mmol) was added to lithium 8-chloro-7-(difluoromethoxy)-6-fluoroquinoline-3-carboxylic acid (0.0409 g, 0.140 mmol, Intermediate 108) in N,N-dimethylformamide (1.403 mL) at room temperature. Then, 2-(trans-4-aminocyclohexyl)propan-2-ol (0.026 g, 0.168 mmol) was added and the reaction mixture was stirred for five minutes. Then, n-propylphosphonic acid anhydride (0.167 mL, 0.281 mmol, 50% in ethyl acetate) was added and the reaction mixture was stirred for sixteen hours. The reaction mixture was concentrated. The resulting residue was purified by RP HPLC, eluting with acetonitrile:water with 0.1% ammonium hydroxide (5:95:100:0) to give 8-chloro-7-(difluoromethoxy)-6-fluoro-N-(trans-4-(2-hydroxypropan-2-yl)cyclohexyl)quinoline-3-carboxamide (0.0377 g, 0.083 mmol, 59.3 % yield). $^1$H NMR (400 MHz, $CD_3SOCD_3$) δ 1.04 (s, 6 H), 1.06-1.24 (m, 3 H), 1.32 (q, *J* = 12 Hz, 2 H), 1.84 (br d, *J* = 11 Hz, 2 H), 1.95 (br d, *J* = 11 Hz, 2 H), 3.70-3.82 (m, 1 H), 4.07 (s, 1 H), 7.38 (t, *J* = 72 Hz, 1 H), 8.21 (d, *J* = 10 Hz, 1 H), 8.72 (d, *J* = 8 Hz, 1 H), 8.87 (d, *J* = 2 Hz, 1 H), 9.35 (d, *J* = 2 Hz, 1 H); LC-MS (LC-ES) M+H = 431.

## Example 236

**6-Chloro-7-(difluoromethoxy)-8-fluoro-N-(trans-4-(2-hydroxypropan-2-yl)cyclohexyl)quinoline-3-carboxamide**

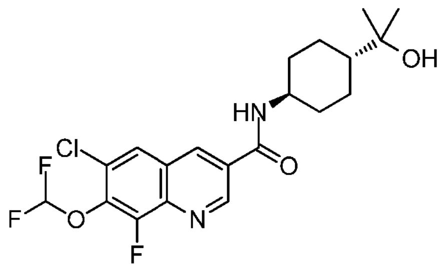

N,N-Diisopropylethylamine (0.105 mL, 0.599 mmol) was added to lithium 6-chloro-7-(difluoromethoxy)-8-fluoroquinoline-3-carboxylic acid (0.0437 g, 0.150 mmol, Intermediate 107) in N,N-dimethylformamide (1.499 mL) at room temperature. Then, 2-(trans-4-aminocyclohexyl)propan-2-ol (0.028 g, 0.180 mmol) was added and the reaction mixture was stirred for five minutes. Then, n-propylphosphonic acid anhydride (0.178 mL, 0.300 mmol, 50% in ethyl acetate) was added and the reaction mixture was stirred for sixteen hours. The reaction mixture was concentrated. The resulting residue was purified by RP HPLC, eluting with acetonitrile:water with 0.1% ammonium hydroxide (5:95:100:0) to give 6-chloro-7-(difluoromethoxy)-8-fluoro-N-(trans-4-(2-hydroxypropan-2-yl)cyclohexyl)quinoline-3-carboxamide (0.0374 g, 0.082 mmol, 55.0 % yield). $^1$H NMR (400 MHz, $CD_3SOCD_3$) δ 1.04 (s, 6 H), 1.06-1.24 (m, 3 H), 1.32 (q, *J* = 12 Hz, 2 H), 1.84 (br d, *J* = 12 Hz, 2 H), 1.95 (br d, *J* = 10 Hz, 2 H), 3.70-3.82 (m, 1 H), 4.07 (s, 1 H), 7.39 (t, *J* = 72 Hz, 1 H), 8.32 (d, *J* = 2 Hz, 1 H), 8.72 (d, *J* = 8 Hz, 1 H), 8.85 (s, 1 H), 9.33 (d, *J* = 2 Hz, 1 H); LC-MS (LC-ES) M+H = 431.

## Example 237

**6-Chloro-7-(difluoromethoxy)-N-(2-oxo-2,3,4,5-tetrahydro-1H-benzo[b]azepin-3-yl)quinoline-3-carboxamide**

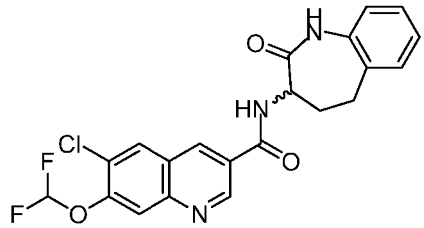

N,N-Diisopropylethylamine (0.298 mL, 1.705 mmol) was added to 6-chloro-7-(difluoromethoxy)quinoline-3-carboxylic acid (0.1166 g, 0.426 mmol, Intermediate 20) in N,N-dimethylformamide (1.420 mL) at room temperature. Then, 3-amino-1,3,4,5-tetrahydro-2H-benzo[b]azepin-2-one (0.090 g, 0.511 mmol) was added and the reaction mixture was stirred for five minutes. Then, n-propylphosphonic acid anhydride (0.507 mL, 0.852 mmol, 50% in ethyl acetate) was added and the reaction mixture was stirred for sixty-six hours. The reaction mixture was concentrated. The resulting residue was purified by RP HPLC, eluting with acetonitrile:water with 0.1% ammonium hydroxide (5:95:100:0), then further purified by silica gel chromatography, eluting with methanol:dichloromethane (0:1 to 1:9), then recrystallized from ethyl acetate to give 6-chloro-7-(difluoromethoxy)-N-(2-oxo-2,3,4,5-tetrahydro-1H-benzo[b]azepin-3-yl)quinoline-3-carboxamide (0.1705 g, 0.375 mmol, 88 % yield). $^1H$ NMR (400 MHz, $CD_3SOCD_3$) δ 2.28-2.44 (m, 2 H), 2.70-2.82 (m, 2 H), 4.42-4.54 (m, 1 H), 7.06 (d, *J* = 8 Hz, 1 H), 7.15 (t, *J* = 7 Hz, 1 H), 7.29 (t, *J* = 8 Hz, 1 H), 7.32 (d, *J* = 8 Hz, 1 H), 7.63 (t, *J* = 73 Hz, 1 H), 7.94 (s, 1 H), 8.47 (s, 1 H), 8.81 (d, *J* = 2 Hz, 1 H), 9.01 (d, *J* = 8 Hz, 1 H), 9.30 (d, *J* = 2 Hz, 1 H), 9.92 (s, 1 H); LC-MS (LC-ES) M+H = 431.

**Example 238 - Capsule Composition**

An oral dosage form for administering a compound of the present invention is produced by filing a standard two piece hard gelatin capsule with the ingredients in the proportions shown in Table 1, below.

Table 1

| INGREDIENTS | AMOUNTS |
|---|---|
| 7-Methoxy-N-(1-(1-methyl-1*H*-tetrazol-5-yl)piperidin-4-yl)quinoline-3-carboxamide (Compound of Example 1) | 7 mg |
| Lactose | 53 mg |
| Talc | 16 mg |
| Magnesium Stearate | 4 mg |

**Example 239 - Injectable Parenteral Composition**

An injectable form for administering a compound of the present invention is produced by stirring 1.7% by weight of (S)-7-Methoxy-N-(2-oxopyrrolidin-3-yl)quinoline-3-carboxamide (Compound of Example 2) in 10% by volume propylene glycol in water.

**Example 240 Tablet Composition**

The sucrose, calcium sulfate dihydrate and a H-GPDS inhibitor of the present invention as shown in Table 2 below, are mixed and granulated in the proportions shown with a 10% gelatin solution. The wet granules are screened, dried, mixed with the starch, talc and stearic acid;, screened and compressed into a tablet.

Table 2

| INGREDIENTS | AMOUNTS |
|---|---|
| 7-(Difluoromethoxy)-N-(1-(1-methyl-1H-tetrazol-5-yl)piperidin-4-yl)quinoline-3-carboxamide (Compound of Example 4) | 12 mg |
| calcium sulfate dihydrate | 30 mg |
| sucrose | 4 mg |
| Starch | 2 mg |
| Talc | 1 mg |

(continued)

| INGREDIENTS | AMOUNTS |
|---|---|
| stearic acid | 0.5 mg |

**BIOLOGICAL ASSAYS**

H-PGDS RapidFire™ High Throughput Mass Spectrometry Assay

The H-PGDS RapidFire™ mass spectrometric assay monitors conversion of prostaglandin $H_2$ ($PGH_2$) to prostaglandin $D_2$ ($PGD_2$) by haematopoietic prostaglandin D synthase (H-PGDS). In the assay format described here, the substrate ($PGH_2$) is formed *in situ* by the action of cyclooxygenase-2 on arachidonic acid. This first step is set up to be fast, and generates a burst of $PGH_2$ at ~10 $\mu$M. The $PGH_2$ is then further converted to $PGD_2$ by the H-PGDS enzyme. The reaction is quenched with tin (II) chloride in citric acid, which converts any remaining $PGH_2$ to the more stable $PGF_2\alpha$. Plates are then read on the RapidFire™ high throughput solid phase extraction system (Agilent) which incorporates a solid phase extraction step coupled to a triple quadrupole mass spectrometer (AB SCIEX). Relative levels of $PGD_2$ and $PGF_2\alpha$, which acts as a surrogate for substrate, are measured and a percent conversion calculated. Inhibitors are characterised as compounds which lower the conversion of $PGH_2$ to $PGD_2$.

Expression and purification of H-PGDS protein

Full length human H-PGDS cDNA (Invitrogen Ultimate ORF IOH13026) was amplified by PCR with the addition of a 5' 6-His tag and TEV protease cleavage site. The PCR product was digested with Ndel and Xhol and ligated into pET22b+ (Merck Novagen®). Expression was carried out in *E. coli* strain BL21 (DE3*) using auto-induction Overnight Express™ Instant TB medium (Merck Novagen®) supplemented with 1 % glycerol. The culture was first grown at 37 °C and the temperature was reduced to 25 °C when $OD_{600}$ reached 2.0. Cells were harvested by centrifugation after a further 18 hr. 10 g of *E. coli* cell pellet was suspended to a total volume of 80 mL in lysis buffer (20 mM Tris-Cl pH 7.5, 300 mM NaCl, 20 mM imidazole, 5 mM $\beta$-mercaptoethanol, 10 % glycerol). 1 mg/mL protease inhibitors (Protease Inhibitor Cocktail Set III, Merck Calbiochem®) and 1 mg/mL lysozyme were added to the cell suspension. The suspension was then sonicated for 5 min (UltraSonic Processor VCX 750, Cole-Parmer Instrument Co.) with a micro probe (50 % amplitude, 10 sec on/off) and then centrifuged at 100,000 g for 90 min (at 4 °C). The supernatant was loaded onto a Ni-NTA HiTrap column (5 mL, GE Heathcare, pre-equilibrated in lysis buffer). The column was washed with 10 column volumes of lysis buffer and eluted with lysis buffer containing 500 mM imidazole. The pooled protein peak fractions were concentrated using a 10 kDa centrifugal filter at 3500 g and 4 °C (Amicon Ultra-15 centrifugal filter unit with Ultracel-10 membrane from Millipore). Further purification of the concentrated protein was carried out using gel filtration chromatography on a HiLoad 26/600 Superdex 75 preparative grade column (GE Healthcare Life Sciences) using 50 mM Tris pH 7.5, 50 mM NaCl, 1 mM dithiothreitol, 1 mM $MgCl_2$. Fractions containing the protein were pooled, concentrated as described above, and stored at -80 °C.

Expression and purification of cyclooxygenase-2 (COX-2) protein

The full length human COX-2 gene (accession number L15326) was amplified by PCR to generate an EcoRI - HindIII fragment containing an in-frame FLAG tag. This was subcloned into pFastBac 1 (Invitrogen). The COX2 FLAG plasmid was recombined into the baculovirus genome according to the BAC-to-BAC protocol described by Invitrogen. Transfection into *Spodoptera frugiperda* (Sf9) insect cells was performed using Cellfectin (Invitrogen), according to the manufacturer's protocol. Super Sf9 cells were cultured in EX420 media (SAFC Biosciences) to a density of approximately 1.5 x $10^6$ cells/mL within a wave bioreactor. Recombinant virus was added at a Multiplicity of Infection (MOI) of 5 and the culture was allowed to continue for 3 days. Cells were harvested using a continuous feed centrifuge run at 2500 g at a rate of approximately 2 L/min with cooling. The resultant cell slurry was re-centrifuged in pots (2500 g, 20 min, 4 °C) and the cell paste was stored at -80 °C. 342 g of cell paste was re-suspended to a final volume of 1600 mL in a buffer of 20 mM Tris-Cl pH 7.4, 150 mM NaCl, 0.1 mM EDTA, 1.3 % w/v n-octyl-p-D-glucopyranoside containing 20 Complete EDTA-free Protease Inhibitor Cocktail tablets (Roche Applied Science). The suspension was sonicated in 500 mL batches for 8 x 5 seconds at 10 u amplitude with the medium tip of an MSE probe sonicator and subsequently incubated at 4 °C for 90 min with gentle stirring. The lysate was centrifuged at 12000 rpm for 45 min at 4 °C in a Sorvall SLA1500 rotor. The supernatant (1400 mL) was added to 420 mL of 20 mM Tris-Cl pH 7.4, 150 mM NaCl, 0.1 mM EDTA to reduce the concentration of n-octyl-$\beta$-D-glucopyranoside to 1% w/v. The diluted supernatant was incubated overnight at 4 °C on a roller with 150 mL of anti-FLAG M2 agarose affinity gel (Aldrich-Sigma) which had been pre-equilibrated with 20 mM Tris-Cl pH 7.4, 150 mM NaCl, 0.1 mM EDTA, 1 % w/v n-octyl-$\beta$-D-glucopyranoside (purification buffer). The

anti-Flag M2 agarose beads were pelleted by centrifugation in 500 mL conical Corning centrifuge pots at 2000 rpm for 10 min at 4 °C in a Sorvall RC3 swing-out rotor. The supernatant (unbound fraction) was discarded and the beads were re-suspended to half the original volume in purification buffer and re-centrifuged as above. The beads were then packed into a BioRad Econo Column (5 cm diameter) and washed with 1500 mL of purification buffer at 4 °C. Bound proteins were eluted with 100 μg/mL triple FLAG peptide (Aldrich-Sigma) in purification buffer. Six fractions each of 0.5 column volume were collected. After each 0.5 column volume of purification buffer was added into the column the flow was held for 10 min before elution. Fractions containing COX-2 were pooled resulting in a protein concentration of ~ 1 mg/mL. The protein was further concentrated on Vivaspin 20 centrifugal concentrators (10 kDa cut-off) to 2.4 mg/mL and then stored at -80 °C.

Test compound plate preparation

Test compounds were diluted to 1 mM in DMSO and a 1:3, 11 point serial dilution was performed across a 384 well HiBase plate (Greiner Bio-one). 100 nL of this dilution series was then transferred into a 384 well v-base plate (Greiner Bio-one) using an Echo™ acoustic dispenser (Labcyte Inc) to create the assay plate. 100 nL of DMSO was added to each well in columns 6 and 18 for use as control columns.

Assay Method

5 μL of an enzyme solution containing 10 nM H-PGDS enzyme, 1.1 μM COX-2 enzyme and 2 mM reduced glutathione (Sigma-Aldrich), diluted in a buffer of 50 mM Tris-Cl pH 7.4, 10 mM $MgCl_2$ and 0.1 % Pluronic F-127 (all Sigma-Aldrich) was added to each well of the plate except column 18 using a Multidrop Combi® dispenser (Thermo Fisher Scientific). 5 μL of enzyme solution without H-PGDS was added to each well in column 18 of the assay plate to generate 100 % inhibition control wells.

Immediately after the addition of enzyme solution, 2.5 μL of a co-factor solution containing 4 μM Hemin (Sigma-Aldrich) diluted in buffer of 50 mM Tris-Cl pH 7.4 and 10 mM $MgCl_2$ (all Sigma-Aldrich), was added to each well using a Multidrop Combi® dispenser. 2.5 μL of substrate solution containing 80 μM arachidonic acid (Sigma-Aldrich) and 1 mM sodium hydroxide (Sigma-Aldrich) diluted in HPLC grade water (Sigma-Aldrich) was then added to each well using a Multidrop Combi® dispenser, to initiate the reaction.

The assay plates were incubated at room temperature for the duration of the linear phase of the reaction (usually 1 min 30 s - 2 min, this timing should be checked on a regular basis). Precisely after this time, the reaction was quenched by the addition of 30 μL of quench solution containing 32.5 mM $SnCl_2$ (Sigma-Aldrich) in 200 mM citric acid (adjusted to pH 3.0 with 0.1 mM NaOH solution) to all wells using a Multidrop Combi® dispenser (Thermo Fisher Scientific). The $SnCl_2$ was initially prepared as a suspension at an equivalent of 600 mM in HPLC water (Sigma-Aldrich) and sufficient concentrated hydrochloric acid (Sigma-Aldrich) was added in small volumes until dissolved. The assay plates were centrifuged at 1000 rpm for 5 min prior to analysis.

The assay plates were analysed using a RapidFire™ high throughput solid phase extraction system (Agilent) coupled to a triple quadrupole mass spectrometer (AB SCIEX) to measure relative peak areas of $PGF_{2\alpha}$ and $PGD_2$ product. Peaks were integrated using the RapidFire™ integrator software before percentage conversion of substrate to $PGD_2$ product was calculated as shown below:

$$\% \text{ Conversion} = ((PGD_2 \text{ peak area}) / (PGD_2 \text{ peak area} + PGF_{2\alpha} \text{ peak area})) \times 100.$$

Data were further analysed within Activitybase software (IDBS) using a four parameter curve fit of the following form:

$$y = \frac{a-d}{1+\left(x/c\right)^{b}} + d$$

where a is the minimum, b is the Hill slope, c is the $IC_{50}$ and d is the maximum. Data are presented as the mean $pIC_{50}$ in Table 3 below.

Table 3

| Example # | Potency Range |
|---|---|
| 1 | ** |
| 2 | ** |
| 3 | ** |
| 4 | ** |
| 5 | ** |
| 6 | ** |
| 7 | ** |
| 8 | ** |
| 9 | ** |
| 10 | ** |
| 11 | ** |
| 12 | ** |
| 13 | ** |
| 14 | *** |
| 15 | *** |
| 16 | ** |
| 17 | ** |
| 18 | *** |
| 19 | ** |
| 20 | *** |
| 21 | *** |
| 22 | ** |
| 23 | ** |
| 24 | ** |
| 25 | ** |
| 26 | *** |
| 27 | ** |
| 28 | ** |
| 29 | ** |
| 30 | ** |
| 31 | * |
| 32 | ** |
| 33 | ** |
| 34 | *** |
| 35 | *** |
| 36 | ** |
| 37 | *** |

(continued)

| Example # | Potency Range |
|---|---|
| 38 | *** |
| 39 | *** |
| 40 | ** |
| 41 | ** |
| 42 | *** |
| 43 | *** |
| 44 | *** |
| 45 | ** |
| 46 | * |
| 47 | ** |
| 48 | ** |
| 49 | ** |
| 50 | ** |
| 51 | ** |
| 52 | * |
| 53 | ** |
| 54 | ** |
| 55 | ** |
| 56 | ** |
| 57 | ** |
| 58 | ** |
| 59 | ** |
| 60 | ** |
| 61 | ** |
| 62 | ** |
| 63 | *** |
| 64 | ** |
| 65 | ** |
| 66 | *** |
| 67 | ** |
| 68 | ** |
| 69 | ** |
| 70 | *** |
| 71 | ** |
| 72 | ** |
| 73 | ** |
| 74 | ** |
| 75 | ** |

(continued)

| Example # | Potency Range |
|---|---|
| 76 | ** |
| 77 | ** |
| 78 | ** |
| 79 | ** |
| 80 | ** |
| 81 | ** |
| 82 | ** |
| 83 | ** |
| 84 | ** |
| 85 | ** |
| 86 | *** |
| 87 | ** |
| 88 | ** |
| 89 | * |
| 90 | ** |
| 91 | * |
| 92 | ** |
| 93 | * |
| 94 | ** |
| 95 | *** |
| 96 | ** |
| 97 | ** |
| 98 | ** |
| 99 | ** |
| 100 | ** |
| 101 | *** |
| 102 | ** |
| 103 | ** |
| 104 | * |
| 105 | * |
| 106 | * |
| 107 | * |
| 108 | ** |
| 109 | * |
| 110 | ** |
| 111 | ** |
| 112 | ** |
| 113 | ** |

(continued)

| Example # | Potency Range |
|---|---|
| 114 | * |
| 115 | * |
| 116 | *** |
| 117 | *** |
| 118 | *** |
| 119 | ** |
| 120 | ** |
| 121 | *** |
| 122 | *** |
| 123 | *** |
| 124 | *** |
| 125 | *** |
| 126 | ** |
| 127 | *** |
| 128 | ** |
| 129 | *** |
| 130 | *** |
| 131 | *** |
| 132 | *** |
| 133 | ** |
| 134 | *** |
| 135 | *** |
| 136 | *** |
| 137 | ** |
| 138 | ** |
| 139 | *** |
| 140 | ** |
| 141 | *** |
| 142 | ** |
| 143 | *** |
| 144 | *** |
| 145 | ** |
| 146 | ** |
| 147 | ** |
| 148 | ** |
| 149 | ** |
| 150 | ** |
| 151 | ** |

(continued)

| Example # | Potency Range |
|---|---|
| 152 | *** |
| 153 | *** |
| 154 | *** |
| 155 | *** |
| 156 | *** |
| 157 | ** |
| 158 | *** |
| 159 | *** |
| 160 | *** |
| 161 | *** |
| 162 | *** |
| 163 | * |
| 164 | *** |
| 165 | ** |
| 166 | *** |
| 167 | *** |
| 168 | ** |
| 169 | *** |
| 170 | ** |
| 171 | ** |
| 172 | *** |
| 173 | * |
| 174 | ** |
| 175 | * |
| 176 | ** |
| 177 | ** |
| 178 | ** |
| 179 | *** |
| 180 | ** |
| 181 | *** |
| 182 | ** |
| 183 | ** |
| 184 | ** |
| 185 | ** |
| 186 | * |
| 187 | *** |
| 188 | ** |
| 189 | * |

(continued)

| Example # | Potency Range |
|---|---|
| 190 | * |
| 191 | ** |
| 192 | *** |
| 193 | - |
| 194 | *** |
| 195 | * |
| 196 | *** |
| 197 | ** |
| 198 | * |
| 199 | *** |
| 200 | ** |
| 201 | ** |
| 202 | ** |
| 202A | ** |
| 203A | * |
| 203B | * |
| 204 | * |
| 205 | *** |
| 206 | ** |
| 207 | * |
| 208 | ** |
| 209 | *** |
| 210 | *** |
| 211 | ** |
| 212 | *** |
| 213 | *** |
| 214 | *** |
| 215A | * |
| 215B | *** |
| 216 | ** |
| 217 | *** |
| 218 | ** |
| 220 | *** |
| 221 | * |
| 222 | ** |
| 223 | ** |
| 224 | ** |
| 225 | ** |

(continued)

| Example # | Potency Range |
|---|---|
| 226 | ** |
| 227 | ** |
| 228 | ** |
| 229 | ** |
| 230 | *** |
| 231 | *** |
| 232 | *** |
| 233 | ** |
| 234 | ** |
| 235 | ** |
| 236 | *** |
| 237 | *** |
| Legend * = $IC_{50}$ 6.0 - 7.0, ** = $pIC_{50}$ 7.1 - 8.0, *** = $pIC_{50}$ >8.0 | |

The compound of Example 2 was tested generally according to the above H-PGDS assay and in at least one experimental run exhibited an H-PGDS $pIC_{50}$ value of 7.2 against H-PGDS.

The compound of Example 15 was tested generally according to the above H-PGDS assay and in at least one experimental run exhibited an H-PGDS $pIC_{50}$ value of 8.1 against H-PGDS.

The compound of Example 20 was tested generally according to the above H-PGDS assay and in at least one experimental run exhibited an H-PGDS $pIC_{50}$ value of 8.1 against H-PGDS.

The compound of Example 25 was tested generally according to the above H-PGDS assay and in at least one experimental run exhibited an H-PGDS $pIC_{50}$ value of 7.6 against H-PGDS.

The compound of Example 36 was tested generally according to the above H-PGDS assay and in at least one experimental run exhibited an H-PGDS $pIC_{50}$ value of 7.8 against H-PGDS.

The compound of Example 46 was tested generally according to the above H-PGDS assay and in at least one experimental run exhibited an H-PGDS $pIC_{50}$ value of 6.9 against H-PGDS.

The compound of Example 59 was tested generally according to the above H-PGDS assay and in at least one experimental run exhibited an H-PGDS $pIC_{50}$ value of 7.3 against H-PGDS.

The compound of Example 116 was tested generally according to the above H-PGDS assay and in at least one experimental run exhibited an H-PGDS $pIC_{50}$ value of 8.6 against H-PGDS.

The compound of Example 118 was tested generally according to the above H-PGDS assay and in at least one experimental run exhibited an H-PGDS $pIC_{50}$ value of 8.0 against H-PGDS.

The compound of Example 138 was tested generally according to the above H-PGDS assay and in at least one experimental run exhibited an H-PGDS $pIC_{50}$ value of 7.7 against H-PGDS.

The compound of Example 142 was tested generally according to the above H-PGDS assay and in at least one experimental run exhibited an H-PGDS $pIC_{50}$ value of 7.4 against H-PGDS.

The compound of Example 150 was tested generally according to the above H-PGDS assay and in at least one experimental run exhibited an H-PGDS $pIC_{50}$ value of 7.4 against H-PGDS.

The compound of Example 160 was tested generally according to the above H-PGDS assay and in at least one experimental run exhibited an H-PGDS $pIC_{50}$ value of 8.4 against H-PGDS.

The compound of Example 165 was tested generally according to the above H-PGDS assay and in at least one experimental run exhibited an H-PGDS $pIC_{50}$ value of 7.2 against H-PGDS.

The compound of Example 177 was tested generally according to the above H-PGDS assay and in at least one experimental run exhibited an H-PGDS $pIC_{50}$ value of 7.7 against H-PGDS.

The compound of Example 187 was tested generally according to the above H-PGDS assay and in at least one experimental run exhibited an H-PGDS $pIC_{50}$ value of 8.2 against H-PGDS.

The compound of Example 193 was tested generally according to the above H-PGDS assay and in at least one experimental run exhibited an H-PGDS $pIC_{50}$ value of 5.7 against H-PGDS.

The compound of Example 204 was tested generally according to the above H-PGDS assay and in at least

one experimental run exhibited an H-PGDS $pIC_{50}$ value of 6.8 against H-PGDS.

The compound of Example 214 was tested generally according to the above H-PGDS assay and in at least one experimental run exhibited an H-PGDS $pIC_{50}$ value of 8.2 against H-PGDS.

The compound of Example 222 was tested generally according to the above H-PGDS assay and in at least one experimental run exhibited an H-PGDS $pIC_{50}$ value of 7.2 against H-PGDS.

**In Vivo Assays for Functional Response to Muscle Injury**

Under anesthesia, the right hind limb of a mouse is restrained at the knee and the foot attached to a motorized footplate/force transducer. Needle electrodes are inserted into the upper limb, either side of the sciatic nerve and a current sufficient to elicit a maximal muscle contraction is applied. Muscle tension is produced by moving the footplate to lengthen the plantarflexor muscles while the limb is under maximal stimulation. This is repeated 60 times to fatigue the muscles of the lower limb. Anesthesia, limb immobilization and limb stimulation are then repeated at regular intervals to measure maximal isometric force in the recovering limb. 7 to 9 animals are tested for each test condition.

Eccentric contraction-induced muscle fatigue in vehicle-treated male C57Bl/6N mice, 10-12 weeks of age, significantly reduced (~35%) maximal isometric torque 24-hours after injury and took~5 weeks for full functional restoration. In contrast, animals (PO) dosed with 1, 3, and 10 mg/kg BID of the compound of Example 6 beginning 10 min prior to eccentric contraction challenge exhibited an acceleration in the kinetics of recovery. Both 3 and 10 mg/kg BID of the compound of Example 6 also reduced the initial magnitude of the injury, as determined by isometric limb force 24-hours following protocol initiation. See Figure 1.

The force deficit and recovery kinetics following eccentric contraction-induced muscle damage was also evaluated in middle-aged (8 month-old) male *C57BL/mdx* mice, a preclinical model of Duchenne Muscular Dystrophy. In this dystrophin-null background, the protocol elicited a greater functional deficit (~75%) and a protracted recovery interval, as shown in the eccentric vehicle controls. Treatment (30 mg/kg BID) with the H-PGDS inhibitor, the compound of Example 48, significantly blunted the force deficit at 24-hours, and improved both the rate and extent of functional recovery in these fragile muscles. See Figure 2.

## Claims

**1.** A compound according to Formula (XI)

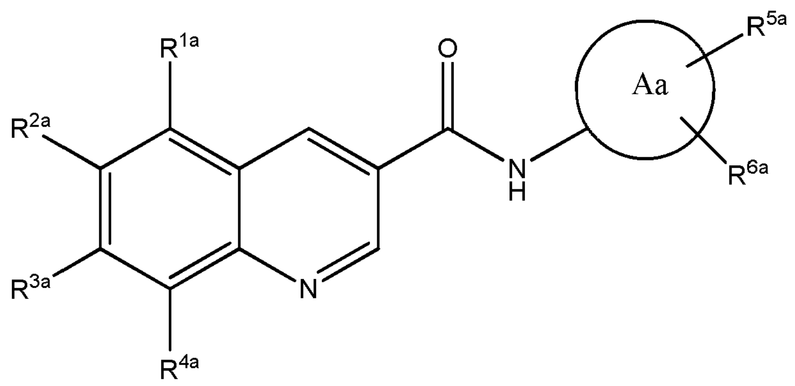

(XI)

wherein:

$R^{1a}$ is selected from:

H,
F,
Cl,
-OH,
$-OCH_3$, and
$C_1$alkoxy substituted 1 to 3 times with fluoro;

$R^{2a}$ is selected from:

H,
F,
Cl,
Br,
I,
-OH,
$-C(O)OC(CH_3)_3$,
-COOH,
$-C(O)C_{1-4}$alkyl,
$-C(O)C_{1-4}$alkyl substituted with from 1 to 5 substituents independently selected from: fluoro, chloro, bromo, oxo, -OH and -CN,
$-SC_{1-4}$alkyl,
$-SC_{1-4}$alkyl, substituted with from 1 to 5 substituents independently selected from: fluoro, chloro, bromo, oxo, -OH and -CN,
$-S(O)C_{1-4}$alkyl,
$-S(O)C_{1-4}$alkyl substituted with from 1 to 5 substituents independently selected from: fluoro, chloro, bromo, oxo, -OH and -CN,
$-N_3$,
-C≡N,
$C_{1-4}$alkoxy,
$C_{1-4}$alkoxy substituted with from 1 to 5 substituents independently selected from: fluoro, chloro, bromo, oxo, -OH and -CN,
$C_{1-6}$alkyl,
$C_{1-6}$alkyl substituted with from 1 to 5 substituents Independently selected from: fluoro, chloro, bromo, iodo, oxo, $C_{1-4}$alkoxy, -OH, -COOH, $-NH_2$, $-N(H)C_{1-4}$alkyl, $-N(C_{1-4}alkyl)_2$ and -CN,
cyclopropyl,
cyclobutyl,
2,2-difluorocyclopropyl,
pyrrolidinyl,
azetidinyl, and
azetidinyl substituted with one or two substituents independently selected from halogen and methyl;

$R^{3a}$ is a difluoromethoxy group;
$R^{4a}$ is a hydrogen or fluorine atom or a methoxy group;
Aa is selected from:

$C_{4-7}$cycloalkyl,
a 4-, 5-, or 6-membered heterocycloalkyl containing one or two heteroatoms independently selected from O and N,
and
a 5-12 membered heteroaryl containing one or two heteroatoms, wherein at least one heteroatom is nitrogen and the second heteroatom, if present, is selected from N and S;

$R^{5a}$ and $R^{6a}$ are independently selected from:

hydrogen,
$-OS(O)_2NH_2$,
$-S(O)_2CH_3$,
-OH,
-C≡N,
F,
Cl,
Br,
I,
tetrazolyl,
methyl-tetrazolyl,
ethyl-tetrazolyl,
cycloalkyl,

cyclopropyl substituted with one or two substituents independently selected from; -OH, $-OCH_3$, and $-CH_3$,
morpholinyl,
azetidinyl,
azetidinyl substituted with one or two substituents independently selected from: fluoro, chloro, bromo, iodo, -OH, $-CF_3$, and $-CH_3$,
pyridinyl,
pyridinyl substituted with -C≡N,
oxazolyl,
oxazolyl substituted with $-C(O)OCH_2CH_3$,
oxazolyl substituted with -C≡N,
-N(H)oxazolyl,
-N(H)oxazolyl substituted with $-C(O)OCH_2CH_3$,
-N(H)oxazolyl substituted with -C≡N,
$-N(H)S(O)_2CH_3$,
oxo,
$C_{1-8}$alkyl,
$C_{1-8}$alkyl substituted with from one to six substituents independently selected from: -OH, oxo, fluoro, chloro, bromo, iodo, $C_{1-4}$alkoxy, cycloalkyl, morpholinyl, methylpiperazinyl, $-NH_2$, $-N(H)C_{1-4}$alkyl, $-N(H)C_{1-4}$alkyl where alkyl is subitituted with from 1 to 5 fluoro, $-N(C_{1-4}alkyl)_2$, and $-N(C_{1-4}alkyl)_2$ where the alkyls are independently subitituted with from 1 to 7 fluoro,
$C_{1-8}$alkoxy,
$C_{1-8}$alkoxy subsitituted with from one to six substituents independently selected from: -OH, oxo, fluoro, chloro, bromo, iodo, $C_{1-4}$alkoxy, cycloalkyl, $-NH_2$, $-N(H)C_{1-4}$alkyl, $-N(H)C_{1-4}$alkyl where the alkyl is subitituted with from 1 to 5 fluoro, $-N(C_{1-4}alkyl)_2$, $-N(C_{1-4}alkyl)_2$ where the alkyls are independently subitituted with from 1 to 7 fluoro, $-S(O)_2CH_3$, $-S(O)_2NH_2$, and $-S(O)_2N(H)C_{1-4}$alkyl,
dimethylamine oxide,
$N(C_{1-6}alkyl)_2$, where each alkyl is optionally subsitituted with from one to six substituents independently selected from: -OH, oxo, fluoro, chloro, bromo, iodo, and $-S(O)_2CH_3$,
$N(H)C_{1-6}$alkyl,
$N(H)C_{1-6}$alkyl subsitituted with from one to six substituents independently selected from: -OH, oxo, fluoro, chloro, bromo, iodo, and
$-S(O)_2CH_3$;

or a pharmaceutically acceptable salt thereof.

2. The compound of Claim 1, wherein the compound of Formula (XI) is represented by the following Formula (XII):

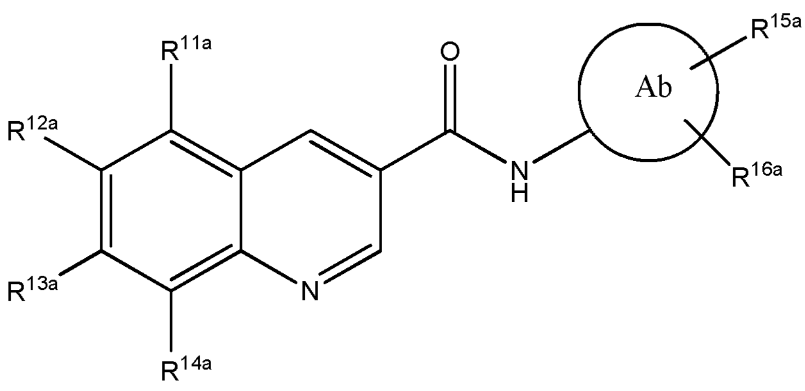

(XII)

wherein:

$R^{11a}$ is selected from:

H,
F,
Cl,

-OH, and
$-OCH_3$;

$R^{12a}$ is selected from:

H,
$C_{1-6}$alkyl,
F,
Cl,
Br,
-C≡N, and
$C_{1-4}$alkoxy;

$R^{13a}$ is a difluoromethoxy group;
$R^{14a}$ is a hydrogen or fluorine atom or a methoxy group;
Ab is selected from:

$C_{4-7}$cycloalkyl,
a 4-, 5-, or 6-membered heterocycloalkyl containing one or two heteroatoms independently selected from O and N,
and
a 5-12 membered heteroaryl containing one or two heteroatoms, wherein at least one heteroatom is nitrogen and the second heteroatom, if present, is selected from N and S;

$R^{15a}$ and $R^{16a}$ are independently selected from:

H,
$-OS(O)_2NH_2$,
$-S(O)_2CH_3$,
-OH,
-CN,
F,
tetrazolyl,
methyl-tetrazolyl,
ethyl-tetrazolyl,
cyclopropyl,
cyclopropyl substituted with one or two substituents independently selected from; -OH, $-OCH_3$, and $-CH_3$,
morpholinyl,
azetidinyl,
azetidinyl substituted with one or two substituents independently selected from: fluoro, chloro, bromo, iodo, -OH, $-CF_3$, and $-CH_3$,
pyridinyl,
pyridinyl substituted with -C≡N,
oxazolyl,
oxazolyl substituted with $-C(O)OCH_2CH_3$,
oxazolyl substituted with -C≡N,
-N(H)oxazolyl,
-N(H)oxazolyl substituted with $-C(O)OCH_2CH_3$,
-N(H)oxazolyl substituted with -C≡N,
$-N(H)S(O)_2CH_3$,
oxo,
$C_{1-8}$alkyl,
$C_{1-8}$alkyl substituted with from one to six substituents independently selected from: -OH, oxo, fluoro, chloro, bromo, iodo, $C_{1-4}$alkoxy, cyclopropyl cyclopentyl, cyclobutyl, morpholinyl, methylpiperazinyl, $-NH_2$, $-N(H)C_{1-4}$alkyl, $-N(H)C_{1-4}$alkyl where alkyl is subitituted with from 1 to 5 fluoro, $-N(C_{1-4}alkyl)_2$, and $-N(C_{1-4}alkyl)_2$ where the alkyls are independently subitituted with from 1 to 7 fluoro,
$C_{1-8}$alkoxy,
$C_{1-8}$alkoxy subsitituted with from one to six substituents independently selected from: -OH, oxo, fluoro,

chloro, bromo, iodo, $C_{1-4}$alkoxy, cycloalkyl, $-NH_2$, $-N(H)C_{1-4}$alkyl, $-N(H)C_{1-4}$alkyl where the alkyl is subitituted with from 1 to 5 fluoro, $-N(C_{1-4}alkyl)_2$, $-N(C_{1-4}alkyl)_2$ where the alkyls are independently subitituted with from 1 to 7 fluoro, $-S(O)_2CH_3$, $-S(O)_2NH_2$, and $-S(O)_2N(H)C_{1-4}$alkyl,
dimethylamine oxide,
$N(H)C_{1-6}$alkyl,
$N(H)C_{1-6}$alkyl subsitituted with from one to six substituents independently selected from: -OH, oxo, fluoro, chloro, bromo, iodo, and $-S(O)_2CH_3$;

or a pharmaceutically acceptable salt thereof.

**3.** The compound of claim 1 or claim 2 wherein the compound of Formula (XI) is represented by the following Formula (XIII):

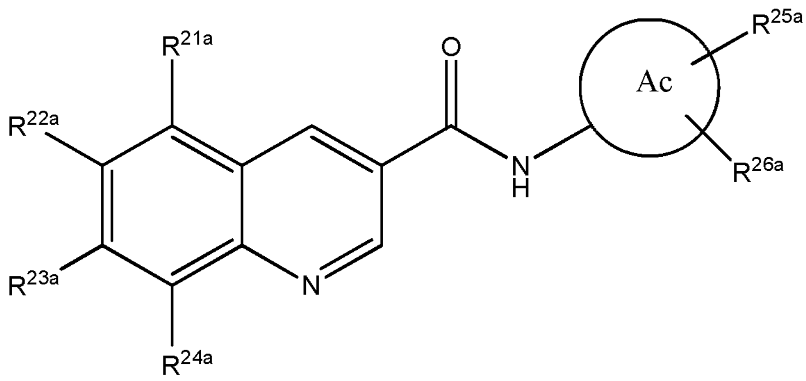

(XIII)

wherein:

$R^{21a}$ is selected from:

H,
F,
-OH, and
$-OCH_3$;

$R^{22a}$ is selected from:

H,
$C_{1-6}$alkyl,
F,
Cl,
Br,
-C≡N, and

$C_{1-4}$alkoxy;
$R^{23a}$ is a difluoromethoxy group;
$R^{24a}$ is a hydrogen or fluorine atom or a methoxy group;
Ac is selected from:

$C_{4-7}$cycloalkyl,
a 4-, 5-, or 6-membered heterocycloalkyl containing one or two heteroatoms independently selected from O and N,
and
a 5-12 membered heteroaryl containing one or two heteroatoms, wherein at least one heteroatom is nitrogen and the second heteroatom, if present, is selected from N and S;

$R^{25a}$ and $R^{26a}$ are independently selected from:

H,
$-OS(O)_2NH_2$,
$-S(O)_2CH_3$,
-OH,
-CN,
F,
methyl-tetrazolyl,
ethyl-tetrazolyl,
cyclopropyl,
cyclopropyl substituted with one or two substituents independently selected from; -OH, and $-OCH_3$,
morpholinyl,
azetidinyl,
azetidinyl substituted with one or two substituents independently selected from: fluoro, chloro, bromo, -OH, $-CF_3$, and $-CH_3$,
pyridinyl,
pyridinyl substituted with -C≡N,
oxazolyl,
oxazolyl substituted with $-C(O)OCH_2CH_3$,
oxazolyl substituted with -C≡N,
-N(H)oxazolyl,
-N(H)oxazolyl substituted with $-C(O)OCH_2CH_3$,
-N(H)oxazolyl substituted with -C≡N,
$-N(H)S(O)_2CH_3$,
oxo,
$C_{1-8}$alkyl,
$C_{1-8}$alkyl substituted with from one to six substituents independently selected from: -OH, oxo, fluoro, chloro, bromo, iodo, $C_{1-4}$alkoxy, cyclopropyl, cyclopentyl, morpholinyl, methylpiperazinyl, $-NH_2$, $-N(H)C_{1-4}$alkyl, $-N(H)C_{1-4}$alkyl where alkyl is subitituted with from 1 to 5 fluoro, $-N(C_{1-4}alkyl)_2$, and $-N(C_{1-4}alkyl)_2$ where the alkyls are independently subitituted with from 1 to 7 fluoro,
$C_{1-8}$alkoxy,
$C_{1-8}$alkoxy subsitituted with from one to six substituents independently selected from: -OH, oxo, fluoro, chloro, bromo, iodo, $C_{1-4}$alkoxy, cyclopropyl, $-NH_2$, $-N(H)C_{1-4}$alkyl, $-N(H)C_{1-4}$alkyl where the alkyl is subitituted with from 1 to 5 fluoro, $-N(C_{1-4}alkyl)_2$, $-N(C_{1-4}alkyl)_2$ where the alkyls are independently subitituted with from 1 to 7 fluoro, $-S(O)_2CH_3$, $-S(O)_2NH_2$, and $-S(O)_2N(H)C_{1-4}$alkyl,
dimethylamine oxide,
$N(H)C_{1-6}$alkyl,
$N(H)C_{1-6}$alkyl subsitituted with from one to six substituents independently selected from: -OH, oxo, fluoro, chloro, bromo, iodo, and $-S(O)_2CH_3$;

or a pharmaceutically acceptable salt thereof.

**4.** The compound of Claim 1, wherein the compound of Formula (XI) is represented by the following Formula (XIV):

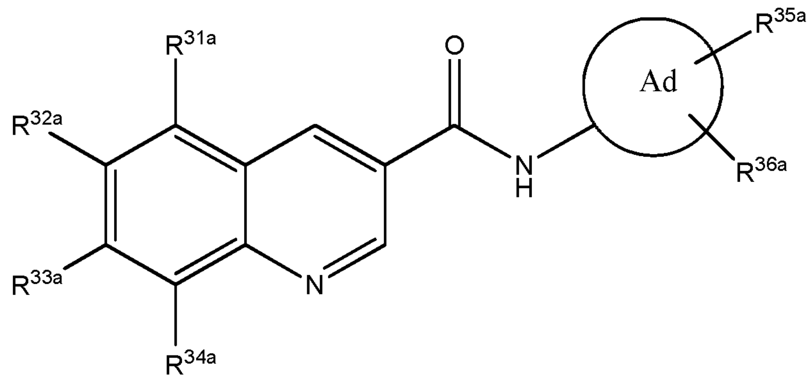

(XIV)

wherein:

$R^{31a}$ is selected from: H, F, and $-OCH_3$;
$R^{32a}$ is selected from: H, -C≡N, F, Cl, Br, $-OCH_3$, and $-CH_3$;
$R^{13a}$ is a difluoromethoxy group;
$R^{14a}$ is a hydrogen or fluorine atom or a methoxy group;
and
either:

Ad is $C_{4-7}$cycloalkyl,
$R^{35a}$ is selected from: H, methyl, ethyl, -C≡N, $-CH_2OCH_2C{\equiv}CH$, $-C(CH_3)_2OH$, $-CH(CH_3)OH$, $-CH(CF_3)OH$, $-CH_2C(O)OCH_2CH_3$, $-CH_2NHCH(CH_3)CHF_2$, $-C(O)N(CH_3)_2$, $-C(O)OCH_3$, =O, $-OCH_3$, $-OCH_2C(O)NH_2$, $-CF_3$, $-CF_2$, $-OCH_2CH(CH_3)OH$, $-OCH_2C(CH_3)_2OH$, $-OCH_2C(CH_3)(CF_3)OH$, $-OCH_2CH(OH)CH(CH_3)_2$, $-OCH_2CH_2S(O)_2CH_3$, $-OCH_2C(O)NHCH_2C{\equiv}CH$, $-OCH_2C(O)NHCH_2CH_2C{\equiv}CH$, $-OCH_2C(CH_3)(CF_3)OH$, $-O(CH_2)_3NH_2$, $-NHS(O)_2CH_3$, $-NHCH(CH_3)CF_3$, $-NHCH(CH_3)CHF_2$, $-NHCH(CF_3)CH_2OH$, $-NHC(CH_3)_2CF_3$, $-NHCH_2CF_3$, $-NHCH_2CHF_2$, $-OS(O)_2NH_2$, -C(O)morpholinyl, -C(O)methylpiperazinyl,dimethylamine oxide, cyclopropanol, cyclopropanolmethoxy, morpholinyl, azetidinyl, azetidinyl substituted with one or two groups independently selected from: halogen, -OH, and $-CF_3$, $-C(OH)R^7R^8$ where $R^7$ is H and $R^8$ is selected from: cyclopropyl, $-CH_3$, and $-CF_3$, or $R^7$ and $R^8$ join together to form cyclopropyl ring, and $-NHR^9$ where $R^9$ is oxazolyl substituted with $-C(O)OCH_2CH_3$, and
$R^{36a}$ is selected from: H, and -OH,
where:
at least one substituent selected from $R^{35a}$, and $R^{36a}$ is not H;
or
Ad is a 4-, 5-, or 6-membered heterocycloalkyl containing one or two heteroatoms independently selected from O and N;
$R^{35a}$ is selected from: H, methyl, $-CH_2CF_3$, $-CH_2CHF_2$, $-C(CH_3)_2OH$, $-CH(CH_3)OH$, $-CH(CF_3)OH$, $-C(O)N(CH_3)_2$, =O, $-S(O)_2CH_3$, $-OS(O)_2NH_2$, ethyl-1*H*-tetrazolyl, methyl-1*H*-tetrazolyl, pyridinyl, pyridinyl substituted with -C≡N, and oxazolyl substituted with $-C(O)OCH_2CH_3$ or -C≡N, and
$R^{36a}$ is selected from: H, methyl, isopropyl, $-CH_2CH(CH_3)_2$, cyclopropylmethylene, or cyclopentylmethylene;
or
Ad is a 5-12 membered heteroaryl containing one or two heteroatoms, wherein at least one heteroatom is nitrogen and the second heteroatom, if present, is selected from N and S,
$R^{35a}$ is selected from: H, methyl or =O, and
$R^{36a}$ is selected from: H, and cyclopropyl;

or a pharmaceutically acceptable salt thereof.

**5.** The compound according to claim 1 or a pharmaceutically acceptable salt thereof wherein $R^{1a}$ represents a hydrogen atom.

**6.** A compound or pharmaceutically acceptable salt thereof according to claim 1 claim 2 wherein $R^{2a}$ represents a hydrogen atom or a halogen atom.

**7.** A compound according to any one of claims 1 to 3 or a pharmaceutically acceptable salt thereof wherein $R^{2a}$ represents a fluorine or chlorine atom.

**8.** A compound according to claim 7 or a pharmaceutically acceptable salt thereof wherein $R^{4a}$ represents a hydrogen atom.

**9.** The compound of claim 1 selected from:

7-(Difluoromethoxy)-N-(1-(1-methyl-1H-tetrazol-5-yl)piperidin-4-yl)quinoline-3-carboxamide;
(S)-7-(Difluoromethoxy)-N-(2-oxopyrrolidin-3-yl)quinoline-3-carboxamide;
7-(Difluoromethoxy)-N-((trans)-4-(dimethylcarbamoyl)cyclohexyl)quinoline-3-carboxamide;
7-(Difluoromethoxy)-N-((trans)-4-(2-hydroxypropan-2-yl)cyclohexyl)quinoline-3-carboxamide;
7-(Difluoromethoxy)-N-(1-(methylsulfonyl)piperidin-4-yl)quinoline-3-carboxamide;
7-(Difluoromethoxy)-N-(3-methyl-1H-pyrazol-5-yl)quinoline-3-carboxamide;
7-(Difluoromethoxy)-N-(2-oxo-1,2,3,4-tetrahydroquinolin-4-yl)quinoline-3-carboxamide;
7-(Difluoromethoxy)-N-(thiazol-2-yl)quinoline-3-carboxamide;

7-(Difluoromethoxy)-N-(trans-3-(2-hydroxypropan-2-yl)cyclobutyl)quinoline-3-carboxamide;
7-(Difluoromethoxy)-N-(trans-4-(2-hydroxy-2-methylpropoxy)cyclohexyl)quinoline-3-carboxamide;
7-(Difluoromethoxy)-N-(trans-4-(-1-hydroxyethyl)cyclohexyl)quinoline-3-carboxamide;
N-(trans-4-(-Cyclopropyl(hydroxy)methyl)cyclohexyl)-7-(difluoromethoxy)quinoline-3-carboxamide;
7-(Difluoromethoxy)-N-(trans-4-(2,2,2-trifluoro-1-hydroxyethyl)cyclohexyl)quinoline-3-carboxamide;
7-(Difluoromethoxy)-N-(trans-4-(2-hydroxypropoxy)cyclohexyl)quinoline-3-carboxamide;
7-(Difluoromethoxy)-N-((3*R*,6S)-6-(2,2,2-trifluoro-1-hydroxyethyl)tetrahydro-2H-pyran-3-yl)quinoline-3-carboxamide;
7-(Difluoromethoxy)-N-(trans-4-(1-hydroxycyclopropyl)cyclohexyl)quinoline-3-carboxamide;
7-(Difluoromethoxy)-N-(trans-4-(3,3,3-trifluoro-2-hydroxy-2-methylpropoxy)cyclohexyl)quinoline-3-carboxamide;
7-(Difluoromethoxy)-N-(trans-4-((R)-2,2,2-trifluoro-1-hydroxyethyl)cyclohexyl)quinoline-3-carboxamide;
7-(Difluoromethoxy)-N-(trans-4-((S)-2,2,2-trifluoro-1-hydroxyethyl)cyclohexyl)quinoline-3-carboxamide;
7-(Difluoromethoxy)-N-((3*s*,6*r*)-6-(2-hydroxypropan-2-yl)tetrahydro-2H-pyran-3-yl)quinoline-3-carboxamide;
N-(trans-4-((3-Aminopropoxy)methyl)cyclohexyl)-7-(difluoromethoxy)quinoline-3-carboxamide;
(S)-7-(Difluoromethoxy)-6-fluoro-N-(2-oxopyrrolidin-3-yl)quinoline-3-carboxamide;
N-(*trans*-4-((2,2-Difluoroethyl)amino)cyclohexyl)-7-(difluoromethoxy)-6-fluoroquinoline-3-carboxamide;
(S)-7-(Difluoromethoxy)-6-fluoro-N-(1-isobutyl-2-oxopyrrolidin-3-yl)quinoline-3-carboxamide;
7-(Difluoromethoxy)-6-fluoro-N-(trans-4-(2-hydroxypropan-2-yl)cyclohexyl)quinoline-3-carboxamide;
7-(Difluoromethoxy)-6-fluoro-N-((1S,2R)-2-hydroxycyclopentyl)quinoline-3-carboxamide;
(S)-N-(1-(Cyclopropylmethyl)-2-oxopyrrolidin-3-yl)-7-(difluoromethoxy)-6-fluoroquinoline-3-carboxamide;
7-(Difluoromethoxy)-6-fluoro-N-(2-oxo-1,2,3,4-tetrahydroquinolin-4-yl)quinoline-3-carboxamide;
7-(Difluoromethoxy)-6-fluoro-N-(trans-4-methoxycyclohexyl)quinoline-3-carboxamide;
7-(Difluoromethoxy)-6-fluoro-N-(trans-4-(methylsulfonamido)cyclohexyl)quinoline-3-carboxamide;
7-(Difluoromethoxy)-6-fluoro-N-(*trans*-4-((1,1,1-trifluoropropan-2-yl)amino)cyclohexyl)quinoline-3-carboxamide;
7-(Difluoromethoxy)-6-fluoro-N-(1-(2,2,2-trifluoroethyl)piperidin-4-yl)quinoline-3-carboxamide;
7-(Difluoromethoxy)-6-fluoro-N-(*trans*-3-((2,2,2-trifluoroethyl)amino)cyclobutyl)quinoline-3-carboxamide;
7-(Difluoromethoxy)-6-fluoro-N-(trans-4-(2-hydroxy-2-methylpropoxy)cyclohexyl)quinoline-3-carboxamide;
7-(Difluoromethoxy)-6-fluoro-N-(6-((2,2,2-trifluoroethyl)amino)spiro[3.3]heptan-2-yl)quinoline-3-carboxamide;
7-(Difluoromethoxy)-6-fluoro-N-(trans-4-(((R)-1,1,1-trifluoropropan-2-yl)amino)cyclohexyl)quinoline-3-carboxamide;
7-(Difluoromethoxy)-6-fluoro-N-(trans-4-(((S)-1,1,1-trifluoropropan-2-yl)amino)cyclohexyl)quinoline-3-carboxamide;
7-(Difluoromethoxy)-6-fluoro-N-((1R,3R)-3-((2,2,2-trifluoroethyl)amino)cyclopentyl)quinoline-3-carboxamide;
N-(1-(2,2-Difluoroethyl)piperidin-4-yl)-7-(difluoromethoxy)-6-fluoroquinoline-3-carboxamide;
7-(Difluoromethoxy)-6-fluoro-N-(*trans*-4-((1,1,1-trifluoro-2-methylpropan-2-yl)amino)cyclohexyl)quinoline-3-carboxamide;
7-(Difluoromethoxy)-N-(*trans*-4-((1,1-difluoropropan-2-yl)amino)cyclohexyl)-6-fluoroquinoline-3-carboxamide;
7-(Difluoromethoxy)-6-fluoro-N-(4-morpholinocyclohexyl)quinoline-3-carboxamide;
7-(Difluoromethoxy)-N-(trans-4-(((R)-1,1-difluoropropan-2-yl)amino)cyclohexyl)-6-fluoroquinoline-3-carboxamide;
7-(Difluoromethoxy)-N-(trans-4-(((S)-1,1-difluoropropan-2-yl)amino)cyclohexyl)-6-fluoroquinoline-3-carboxamide;
N-(*trans*-4-(3,3-Difluoroazetidin-1-yl)cyclohexyl)-7-(difluoromethoxy)-6-fluoroquinoline-3-carboxamide;
Ethyl 2-(4-(7-(difluoromethoxy)-6-fluoroquinoline-3-carboxamido)piperidin-1-yl)oxazole-5-carboxylate;
7-(Difluoromethoxy)-N-((1S,3r)-3-(((S)-1,1-difluoropropan-2-yl)amino)cyclobutyl)-6-fluoroquinoline-3-carboxamide;
7-(Difluoromethoxy)-N-((1R,3r)-3-(((R)-1,1-difluoropropan-2-yl)amino)cyclobutyl)-6-fluoroquinoline-3-carboxamide;
7-(Difluoromethoxy)-N-(*trans*-4-(((1,1-difluoropropan-2-yl)amino)methyl)cyclohexyl)-6-fluoroquinoline-3-carboxamide;
7-(Difluoromethoxy)-6-fluoro-N-(trans-4-(3,3,3-trifluoro-2-hydroxy-2-methylpropoxy)cyclohexyl)quinoline-3-carboxamide;
7-(Difluoromethoxy)-6-fluoro-N-(*trans*-4-(((R)-1,1,1-trifluoro-3-hydroxypropan-2-yl)amino)cyclohexyl)quinoline-3-carboxamide;
(S)-N-(1-(Cyclopentylmethyl)-2-oxopyrrolidin-3-yl)-7-(difluoromethoxy)-6-fluoroquinoline-3-carboxamide;
7-(Difluoromethoxy)-6-fluoro-N-(trans-4-(2-hydroxy-3-methylbutoxy)cyclohexyl)quinoline-3-carboxamide;

(S)-Ethyl 2-(3-(7-(difluoromethoxy)-6-fluoroquinoline-3-carboxamido)pyrrolidin-1-yl)oxazole-5-carboxylate;
7-(Difluoromethoxy)-N-((1r,4r)-4-ethyl-4-hydroxycyclohexyl)-6-fluoroquinoline-3-carboxamide;
Ethyl 2-(3-(7-(difluoromethoxy)-6-fluoroquinoline-3-carboxamido)azetidin-1-yl)oxazole-4-carboxylate;
Ethyl 2-((trans-3-(7-(difluoromethoxy)-6-fluoroquinoline-3-carboxamido)cyclobutyl)amino)oxazole-5-carboxylate;
Ethyl 2-(3-(7-(difluoromethoxy)-6-fluoroquinoline-3-carboxamido)azetidin-1-yl)oxazole-5-carboxylate;
7-(Difluoromethoxy)-6-fluoro-N-(*trans*-4-(((S)-1,1,1-trifluoro-3-hydroxypropan-2-yl)amino)cyclohexyl)quinoline-3-carboxamide
*trans*-Methyl 4-(7-(difluoromethoxy)-6-fluoroquinoline-3-carboxamido) cyclohexanecarboxylate;
N-(*trans*-4-Cyanocyclohexyl)-7-(difluoromethoxy)-6-fluoroquinoline-3-carboxamide;
Ethyl 2-(*trans*-4-(7-(difluoromethoxy)-6-fluoroquinoline-3-carboxamido)cyclohexyl) acetate;
7-(Difluoromethoxy)-6-fluoro-N-(*cis*-4-(3-fluoroazetidin-1-yl)cyclohexyl)quinoline-3-carboxamide;
7-(Difluoromethoxy)-6-fluoro-N-(*trans*-4-(3-fluoroazetidin-1-yl)cyclohexyl)quinoline-3-carboxamide;
7-(Difluoromethoxy)-6-fluoro-N-(*cis*-4-(3-hydroxy-3-(trifluoromethyl)azetidin-1-yl)cyclohexyl)quinoline-3-carboxamide;
7-(Difluoromethoxy)-6-fluoro-N-(*trans*-4-(3-hydroxy-3-(trifluoromethyl)azetidin-1-yl)cyclohexyl)quinoline-3-carboxamide;
7-(Difluoromethoxy)-6-fluoro-N-((1s,3s)-3-hydroxy-3-methylcyclobutyl)quinoline-3-carboxamide;
7-(Difluoromethoxy)-6-fluoro-N-(trans-4-(2-(methylsulfonyl)ethoxy)cyclohexyl)quinoline-3-carboxamide;
7-(Difluoromethoxy)-6-fluoro-N-(1-(pyridin-2-yl)piperidin-4-yl)quinoline-3-carboxamide;
7-(Difluoromethoxy)-6-fluoro-N-(1-(pyridin-3-yl)piperidin-4-yl)quinoline-3-carboxamide;
N-(1-(5-Cyanooxazol-2-yl)piperidin-4-yl)-7-(difluoromethoxy)-6-fluoroquinoline-3-carboxamide;
N-(1-(5-Cyanopyridin-2-yl)azetidin-3-yl)-7-(difluoromethoxy)-6-fluoroquinoline-3-carboxamide;
7-(Difluoromethoxy)-N-(frans-4-((((R)-1,1-difluoropropan-2-yl)amino)methyl)cyclohexyl)-6-fluoroquinoline-3-carboxamide;
7-(Difluoromethoxy)-N-(trans-4-((((S)-1,1-difluoropropan-2-yl)amino)methyl)cyclohexyl)-6-fluoroquinoline-3-carboxamide;
7-(Difluoromethoxy)-6-fluoro-N-(trans-4-((prop-2-yn-1-yloxy)methyl)cyclohexyl)quinoline-3-carboxamide;
7-(Difluoromethoxy)-6-fluoro-N-(trans-4-(2-oxo-2-(prop-2-yn-1-ylamino)ethoxy)cyclohexyl)quinoline-3-carboxamide;
N-(trans-4-(2-(But-3-yn-1-ylamino)-2-oxoethoxy)cyclohexyl)-7-(difluoromethoxy)-6-fluoroquinoline-3-carboxamide;
(6-Chloro-7-(difluoromethoxy)-N-(trans-4-(2-hydroxypropan-2-yl)cyclohexyl)quinoline-3-carboxamide;
(S)-6-Chloro-7-(difluoromethoxy)-N-(2-oxopyrrolidin-3-yl)quinoline-3-carboxamide;
6-Chloro-7-(difluoromethoxy)-N-(trans-3-(2-hydroxypropan-2-yl)cyclobutyl)quinoline-3-carboxamide;
(S)-6-Chloro-7-(difluoromethoxy)-N-(1-isopropyl-2-oxopyrrolidin-3-yl)quinoline-3-carboxamide;
(S)-6-Chloro-N-(1-(cyclopropylmethyl)-2-oxopyrrolidin-3-yl)-7-(difluoromethoxy)quinoline-3-carboxamide;
6-Chloro-7-(difluoromethoxy)-N-(trans-4-(1-hydroxycyclopropyl)cyclohexyl)quinoline-3-carboxamide;
6-Chloro-7-(difluoromethoxy)-N-(trans-4-(2-hydroxy-2-methylpropoxy)cyclohexyl)quinoline-3-carboxamide;
6-Chloro-7-(difluoromethoxy)-N-(trans-4-((1-hydroxycyclopropyl)methoxy)cyclohexyl)quinoline-3-carboxamide;
6-Chloro-7-(difluoromethoxy)-N-((3*r*,6s)-6-(2-hydroxypropan-2-yl)tetrahydro-2H-pyran-3-yl)quinoline-3-carboxamide;
6-Chloro-7-(difluoromethoxy)-N-(*trans*-4-((1,1-difluoropropan-2-yl)amino)cyclohexyl)quinoline-3-carboxamide;
6-Chloro-7-(difluoromethoxy)-N-((3*r*,6*s*)-6-(dimethylcarbamoyl)tetrahydro-2H-pyran-3-yl)quinoline-3-carboxamide;
6-Chloro-7-(difluoromethoxy)-N-(trans-4-(2-hydroxy-3-methylbutoxy)cyclohexyl)quinoline-3-carboxamide;
6-Chloro-N-(3-cyclopropyl-1-methyl-1H-pyrazol-5-yl)-7-(difluoromethoxy)quinoline-3-carboxamide;
6-Chloro-7-(difluoromethoxy)-N-(*trans*-4-(((R)-1,1-difluoropropan-2-yl)amino)cyclohexyl)quinoline-3-carboxamide;
6-Chloro-7-(difluoromethoxy)-N-(*trans*-4-(((S)-1,1-difluoropropan-2-yl)amino)cyclohexyl)quinoline-3-carboxamide;
6-Chloro-7-(difluoromethoxy)-N-(*trans*-4-(((S)-1,1,1-trifluoro-3-hydroxypropan-2-yl)amino)cyclohexyl)quinoline-3-carboxamide;
6-Chloro-7-(difluoromethoxy)-N-(*trans*-4-(3-fluoroazetidin-1-yl)cyclohexyl)quinoline-3-carboxamide;
6-Chloro-7-(difluoromethoxy)-N-((3S)-5-methyl-2-oxopyrrolidin-3-yl)quinoline-3-carboxamide;
6-Chloro-7-(difluoromethoxy)-N-(*trans*-4-(3-hydroxy-3-(trifluoromethyl)azetidin-1-yl)cyclohexyl)quinoline-3-carboxamide;

6-Chloro-7-(difluoromethoxy)-N-(*trans*-4-(((R)-1,1,1-trifluoro-3-hydroxypropan-2-yl)amino)cyclohexyl)quinoline-3-carboxamide;
N-(trans-4-(2-Amino-2-oxoethoxy)cyclohexyl)-6-chloro-7-(difluoromethoxy)quinoline-3-carboxamide;
6-Chloro-7-(difluoromethoxy)-N-((1r,3r)-3-hydroxy-3-methylcyclobutyl)quinoline-3-carboxamide;
6-Chloro-7-(difluoromethoxy)-N-((1s,3s)-3-hydroxy-3-methylcyclobutyl)quinoline-3-carboxamide;
6-Chloro-7-(difluoromethoxy)-N-(trans-4-(2-(methylsulfonyl)ethoxy)cyclohexyl)quinoline-3-carboxamide;
6-Chloro-7-(difluoromethoxy)-N-(*trans*-3-hydroxycyclobutyl)quinoline-3-carboxamide;
trans-4-(6-Chloro-7-(difluoromethoxy)quinoline-3-carboxamido)cyclohexyl sulfamate;
6-Chloro-7-(difluoromethoxy)-N-(3-oxocyclobutyl)quinoline-3-carboxamide;
6-Chloro-7-(difluoromethoxy)-N-((1r,4r)-4-hydroxy-4-methylcyclohexyl)quinoline-3-carboxamide;
6-Chloro-7-(difluoromethoxy)-N-((3S,4R)-4-methyl-2-oxopyrrolidin-3-yl)quinoline-3-carboxamide;
6-Chloro-7-(difluoromethoxy)-N-(3-hydroxy-3-methylcyclopentyl)quinoline-3-carboxamide;
6-Chloro-7-(difluoromethoxy)-N-((1S,3S)-3-hydroxy-3-methylcyclopentyl)quinoline-3-carboxamide;
6-Chloro-7-(difluoromethoxy)-N-((1s,4s)-4-hydroxy-4-methylcyclohexyl)quinoline-3-carboxamide;
6-Chloro-7-(difluoromethoxy)-N-(tetrahydro-2H-pyran-4-yl)quinoline-3-carboxamide;
6-Chloro-7-(difluoromethoxy)-N-(tetrahydro-2H-pyran-3-yl)quinoline-3-carboxamide;
6-Chloro-7-(difluoromethoxy)-N-(2,2-dimethyltetrahydro-2H-pyran-4-yl)quinoline-3-carboxamide;
6-Bromo-7-(difluoromethoxy)-N-(*trans*-4-(2-hydroxypropan-2-yl)cyclohexyl)quinoline-3-carboxamide;
6-Bromo-7-(difluoromethoxy)-N-(*trans*-4-(((S)-1,1,1-trifluoro-3-hydroxypropan-2-yl)amino)cyclohexyl)quinoline-3-carboxamide;
(S)-6-Bromo-7-(difluoromethoxy)-N-(2-oxopyrrolidin-3-yl)quinoline-3-carboxamide;
6-Bromo-7-(difluoromethoxy)-N-(*trans*-4-(((R)-1,1,1-trifluoro-3-hydroxypropan-2-yl)amino)cyclohexyl)quinoline-3-carboxamide;
6-Bromo-7-(difluoromethoxy)-N-(*trans*-4-((1,1-difluoropropan-2-yl)amino)cyclohexyl)quinoline-3-carboxamide;
6-Bromo-7-(difluoromethoxy)-N-((1s,3s)-3-hydroxy-3-methylcyclobutyl)quinoline-3-carboxamide;
6-Bromo-7-(difluoromethoxy)-N-(*trans*-4-(((R)-1,1-difluoropropan-2-yl)amino)cyclohexyl)quinoline-3-carboxamide;
6-Bromo-7-(difluoromethoxy)-N-(*trans*-4-(((S)-1,1-difluoropropan-2-yl)amino)cyclohexyl)quinoline-3-carboxamide;
trans-4-(6-Chloro-7-(difluoromethoxy)quinoline-3-carboxamido)-N, N-dimethylcyclohexanamine oxide;
6-Chloro-7-(difluoromethoxy)-N-((trans)-4-(morpholine-4-carbonyl)cyclohexyl)quinoline-3-carboxamide;
6-Chloro-7-(difluoromethoxy)-N-((trans)-4-(4-methylpiperazine-1-carbonyl)cyclohexyl)quinoline-3-carboxamide;
6-Chloro-7-(difluoromethoxy)-N-((trans)-3-(3-hydroxy-3-(trifluoromethyl)azetidin-1-yl)cyclobutyl)quinoline-3-carboxamide;
8-Chloro-7-(difluoromethoxy)-N-((*trans*)-4-(2-hydroxypropan-2-yl)cyclohexyl)quinoline-3-carboxamide;
6,8-Dichloro-7-(difluoromethoxy)-N-((*trans*)-4-(2-hydroxypropan-2-yl)cyclohexyl)quinoline-3-carboxamide;
6-Chloro-7-(difluoromethoxy)-N-(1-(2-hydroxy-2-methylpropanoyl)piperidin-4-yl)quinoline-3-carboxamide;
6-Chloro-7-(difluoromethoxy)-N-(1-(2-hydroxy-2-methylpropanoyl)azetidin-3-yl)quinoline-3-carboxamide;
6-Chloro-7-(difluoromethoxy)-N-((cis)-4-hydroxy-4-(trifluoromethyl)cyclohexyl)quinoline-3-carboxamide;
6-Chloro-7-(difluoromethoxy)-N-(4-(2-hydroxy-2-methylpropanoyl)piperazin-1-yl)quinoline-3-carboxamide;
6-Chloro-7-(difluoromethoxy)-N-(trans-3-(2-hydroxy-2-methylpropanamido)cyclobutyl)quinoline-3-carboxamide;
6-Chloro-7-(difluoromethoxy)-N-((trans)-4-hydroxy-4-(trifluoromethyl)cyclohexyl)quinoline-3-carboxamide;
8-Chloro-7-(difluoromethoxy)-N-((*trans*)-4-(2-hydroxypropan-2-yl)cyclohexyl)-6-methylquinoline-3-carboxamide;
6-Chloro-7-(difluoromethoxy)-N-((*trans*)-4-(2-hydroxypropan-2-yl)cyclohexyl)-8-methylquinoline-3-carboxamide;
6-Chloro-7-(difluoromethoxy)-N-((1s,3s)-3-hydroxy-3-(trifluoromethyl)cyclobutyl)quinoline-3-carboxamide;
6-Chloro-7-(difluoromethoxy)-N-((1s,4s)-4-(difluoromethyl)-4-hydroxycyclohexyl)quinoline-3-carboxamide;
6-Chloro-7-(difluoromethoxy)-N-((1r,4r)-4-(difluoromethyl)-4-hydroxycyclohexyl)quinoline-3-carboxamide;
8-Chloro-7-(difluoromethoxy)-6-fluoro-N-(trans-4-(2-hydroxypropan-2-yl)cyclohexyl)quinoline-3-carboxamide;
6-Chloro-7-(difluoromethoxy)-8-fluoro-N-(trans-4-(2-hydroxypropan-2-yl)cyclohexyl)quinoline-3-carboxamide;
and
6-Chloro-7-(difluoromethoxy)-N-(2-oxo-2,3,4,5-tetrahydro-1H-benzo[b]azepin-3-yl)quinoline-3-carboxamide;
or a pharmaceutically acceptable salt thereof.

**10.** The compound of claim 1, which is 6-chloro-7-(difluoromethoxy)-N-((1s,3s)-3-hydroxy-3-methylcyclobutyl)quinoline-3-carboxamide or a pharmaceutically acceptable salt thereof.

**11.** A compound of Formula (XI) or a pharmaceutically acceptable salt thereof according to any one of claims 1 to 10 for use in therapy.

**12.** A compound of Formula (XI) or a pharmaceutically acceptable salt thereof according to any one claims 1 to 10 for use in the treatment of muscle injury, muscle lacerations, muscle damage due to knee replacement, asthma, chronic obstructive pulmonary disease or Duchenne Muscular Dystrophy.

**Patentansprüche**

**1.** Verbindung mit der Formel (XI)

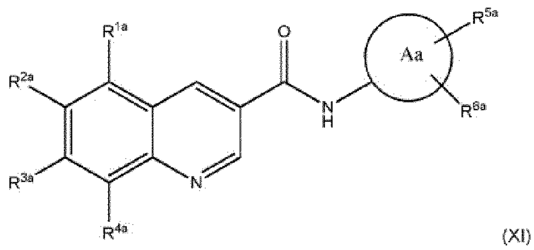

(XI)

worin

$R^{1a}$ ausgewählt ist aus:

H,
F,
Cl,
-OH,
$-OCH_3$ und
$C_1$-Alkoxy, 1- bis 3-mal mit Fluor substituiert,

$R^{2a}$ ausgewählt ist aus:

H,
F,
Cl,
Br,
I,
-OH,
$-C(O)OC(CH3)_3$,
-COOH,
$-C(O)C_{1-4}$-Alkyl,
$-C(O)C_{1-4}$-Alkyl, substituiert mit 1 bis 5 Substituenten, unabhängig ausgewählt aus: Fluor, Chlor, Brom, Oxo, -OH und -CN,
$-SC_{1-4}$-Alkyl,
$-SC_{1-4}$-Alkyl, substituiert mit 1 bis 5 Substituenten, unabhängig ausgewählt aus: Fluor, Chlor, Brom, Oxo, -OH und -CN,
$-S(O)C_{1-4}$-Alkyl,
$-S(O)C_{1-4}$-Alkyl, substituiert mit 1 bis 5 Substituenten, unabhängig ausgewählt aus: Fluor, Chlor, Brom, Oxo, -OH und -CN,
$-N_3$,
-C≡N,
$C_{1-4}$-Alkoxy,
$C_{1-4}$-Alkoxy, substituiert mit 1 bis 5 Substituenten, unabhängig ausgewählt aus: Fluor, Chlor, Brom, Oxo, -OH und -CN,

$C_{1-6}$-Alkyl,
$C_{1-6}$-Alkyl, substituiert mit 1 bis 5 Substituenten, unabhängig ausgewählt aus: Fluor, Chlor, Brom, Iod, Oxo, $C_{1-4}$-Alkoxy, -OH, -COOH, $-NH_2$, $-N(H)C_{1-4}$-Alkyl, $-N(C_{1-4}\text{-Alkyl})_2$ und -CN,
Cyclopropyl,
Cyclobutyl,
2,2-Difluorcyclopropyl,
Pyrrolidinyl,
Azetidinyl und
Azetidinyl, ist, substituiert mit einem oder zwei Substituenten, unabhängig ausgewählt aus Halogen und Methyl,

$R^{3a}$ Difluormethoxygruppe ist,
$R^{4a}$ Wasserstoff oder Fluoratom oder eine Methoxygruppe ist,
Aa ausgewählt ist aus:

$C_{4-7}$-Cycloalkyl,
einem 4-, 5- oder 6-gliedrigen Heterocycloalkyl mit einem oder zwei Heteroatomen, unabhängig ausgewählt aus 0 und N, und
einem 5-12-gliedrigen Heteroaryl, enthaltend ein oder zwei Heteroatome, worin zumindest ein Heteroatom Stickstoff ist und das zweite Heteroatom, falls vorhanden, ausgewählt ist aus N und S,

$R^{5a}$ und $R^{6a}$ unabhängig ausgewählt sind aus:

Wasserstoff,
$-OS(O)_2NH_2$,
$-S(O)_2CH_3$,
-OH,
-C≡N,
F,
Cl,
Br,
I,
Tetrazolyl,
Methyl-tetrazolyl,
Ethyl-tetrazolyl,
Cycloalkyl,
Cyclopropyl, substituiert mit einem oder zwei Substituenten, unabhängig ausgewählt aus: -OH, $-OCH_3$ und $-CH_3$,
Morpholinyl,
Azetidinyl,
Azetidinyl, substituiert mit einem oder zwei Substituenten, unabhängig ausgewählt aus: Fluor, Chlor, Brom, Iod, -OH, $-CH_3$ und $-CH_3$,
Pyridinyl,
Pyridinyl, substituiert mit -C≡N,
Oxazolyl,
Oxazolyl, substituiert mit $-C(O)OCH_2CH_3$,
Oxazolyl, substituiert mit -C≡N,
-N(H)Oxazolyl,
-N(H)Oxazolyl, substituiert mit $-C(O)OCH_2CH_3$,
-N(H)Oxazolyl, substituiert mit -C≡N,
$-N(H)S(O)_2CH_3$,
Oxo,
$C_{1-8}$-Alkyl,
$C_{1-8}$-Alkyl, substituiert mit einem bis sechs Substituierten, unabhängig ausgewählt aus: OH, Oxo, Fluor, Chlor, Brom, Iod, $C_{1-4}$-Alkoxy, Cycloalkyl, Morpholinyl, Methylpiperazinyl, $-NH_2$, $-N(H)C_{1-4}$-Alkyl, worin Alkyl mit 1 bis 5 Fluor substituiert ist, $-N(C_{1-4}\text{-Alkyl})_2$ und $-N(C_{1-4}\text{-Alkyl})_2$ und worin die Alkylgruppen unabhängig mit 1 bis 7 Fluor substituiert sind,
$C_{1-8}$-Alkoxy,

$C_{1-8}$-Alkoxy, substituiert mit einem bis sechs Substituenten, unabhängig ausgewählt aus: -OH, Oxo, Fluor, Chlor, Brom, Iod, $C_{1-4}$-Alkoxy,
Cycloalkyl, $-NH_2$, $-N(H)C_{1-4}$-Alkyl, $-N(H)C_{1-4}$-Alkyl, worin das Alkyl mit 1 bis 5 Fluor substituiert ist, $-N(C_{1-4}$-Alkyl$)_2$, $-N(C_{1-4}$-Alkyl$)_2$, worin die Alkylgruppen unabhängig mit 1 bis 7 Fluor substituiert sind, $-S(O)_2CH_2$, $-S(O)_2NH_2$ und $-S(O)_2N(H)C_{1-4}$-Alkyl, Dimethylaminoxid,
$N(C_{1-6}$-Alkyl$)_2$, worin jedes Alkyl wahlweise mit einem bis sechs Substituenten substituiert ist, unabhängig ausgewählt aus: -OH, Oxo, Fluor, Chlor, Brom, Iod und $-S(O)_2CH_3$,
N (H) $C_{1-6}$-Alkyl,
$N(H)C_{1-6}$-Alkyl, substituiert mit einem bis sechs Substituenten, unabhängig ausgewählt aus: -OH, Oxo, Fluor, Chlor, Brom, Iod und $-S(O)_2CH_2$,
oder ein pharmazeutisch akzeptables Salz davon.

**2.** Verbindung nach Anspruch 1, worin die Verbindung der Formel (XI) durch die folgende Formel (XII) dargestellt ist:

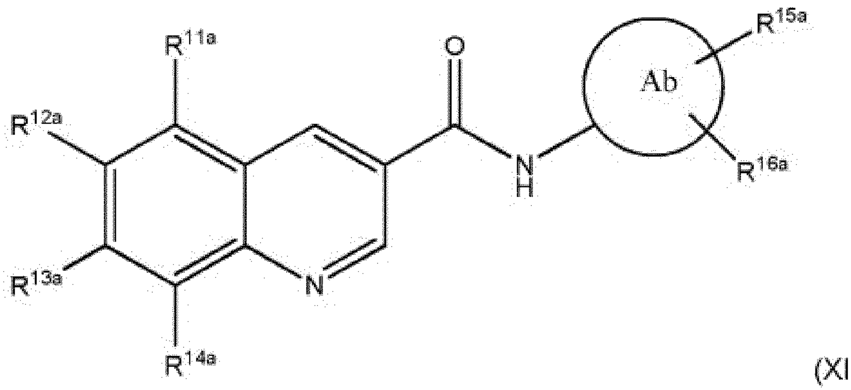

(XII)

worin:

$R^{11a}$ ausgewählt ist aus:

H,
F,
Cl,
-OH und
$-OCH_3$,

$R^{12a}$ ausgewählt ist aus:

H,
$C_{1-6}$-Alkyl,
F,
Cl,
Br,
-C≡N und
$C_{1-4}$-Alkoxy,

$R^{13a}$ eine Difluormethoxygruppe ist,
$R^{14a}$ ein Wasserstoff- oder Fluoratom oder eine Methoxygruppe ist,
Ab ausgewählt ist aus:

$C_{4-7}$-Cycloalkyl,
einem 4-, 5- oder 6-gliedrigen Heterocycloalkyl mit einem oder zwei Heteroatomen, unabhängig ausgewählt aus 0 und N, und
einem 5-12-gliedrigem Heteroaryl mit einem oder zwei Heteroatomen, worin zumindest ein Heteroatom Stickstoff und das zweite Heteroatom, falls vorhanden, ausgewählt ist aus N und S,

$R^{15a}$ und $R^{16a}$ unabhängig ausgewählt sind aus:

H,
$-OS(O)_2NH_2$,
$-S(O)_2CH_3$,
-OH,
-CN,
F,
Tetrazolyl,
Methyl-tetrazolyl,
Ethyl-tetrazolyl,
Cycopropyl,
Cyclopropyl, substituiert mit einem oder zwei Substituenten, unabhängig ausgewählt aus: OH, $-OCH_3$ und $-CH_3$,
Morpholinyl,
Azetidinyl,
Azetidinyl, substituiert mit einem oder zwei Substituenten, unabhängig ausgewählt aus: Fluor, Chlor, Brom, Iod, -OH, $-CF_3$ und $-CH_3$,
Pyridinyl
Pyridinyl, substituiert mit -C≡N, Oxazolyl,
Oxazolyl, substiutiert mit $-C(O)OCH_2CH_3$, Oxazolyl, substituiert mit -C≡N,
-N(H)Oxazolyl,
-N(H)Oxazolyl, substituiert mit $-C(O)OCH_2CH_3$,
-N(H)Oxazolyl, substituiert mit -C≡N,
$-N(H)S(O)_2CH_3$,
Oxo,
$C_{1-8}$-Alkyl,
$C_{1-8}$-Alkyl, substituiert mit einem bis sechs Substituenten, unabhängig ausgewählt aus: -OH, Oxo, Fluor, Chlor, Brom, Iod, $C_{1-4}$-Alkoxy, Cyclopropyl, Cyclopentyl, Cyclobutyl, Morpholinyl, Methylpiperazinyl, $-NH_2$, $-N(H)C_{1-4}$-Alkyl, $-N(H)C_{1-4}$-Alkyl, worin Alkyl mit 1 bis 5 Fluor substituiert ist, $-N(C_{1-4}$-Alkyl$)_2$ und $-N(C_{1-4}$-Alkyl$)_2$, worin die Alkylgruppen unabhängig mit 1 bis 7 Fluor substituiert sind,

$C_{1-8}$-Alkoxy,
$C_{1-8}$-Alkoxy, substituiert mit einem bis sechs Substituenten, unabhängig ausgewählt aus: -OH, Oxo, Fluor, Chlor, Brom, Iod, $C_{1-4}$-Alkoxy, Cycloalkyl, $-NH_2$, $-N(H)C_{1-4}$-Alkyl, $-N(H)C_{1-4}$-Alkyl,
worin das Alkyl mit 1 bis 5 Fluor substituiert ist, $-N(C_{1-4}$-Alkyl$)_2$, $-N(C_{1-4}$-Alkyl$)_2$, worin die Alkylgruppen unabhängig mit 1 bis 7 Fluor substituiert sind,
Dimethylaminoxid,
N (H) $C_{1-6}$-Alkyl,
$N(H)C_{1-6}$-Alkyl, substituiert mit ein bis sechs Substituenten, unabhängig ausgewählt aus: -OH, Oxo, Fluor, Chlor, Brom, Iod,
$-S(O)_2CH_3$, $-S(O)_2NH_2$ und $-S(O)_2N(H)C_{1-4}$-Alkyl oder ein pharmazeutisch akzeptables Salz davon.

3. Verbindung nach Anspruch 1 oder Anspruch 2, worin die Verbindung der Formel (XI) durch die folgende Formel (XIII) dargestellt ist:

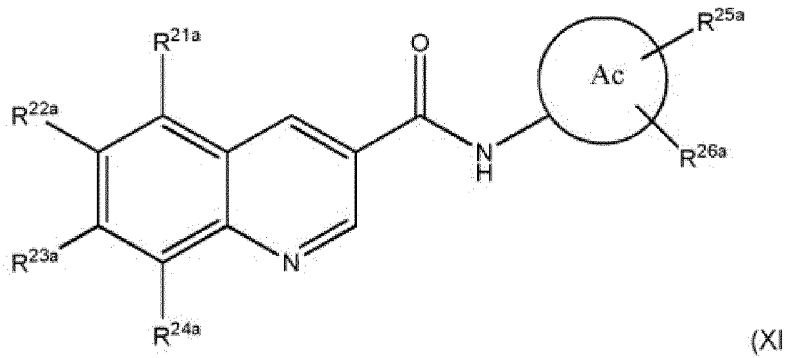

(XIII)

worin

$R^{21a}$ ausgewählt ist aus:

H,
F,
-OH und
$-OCH_3$,

$R^{22a}$ ausgewählt ist aus:

H,
$C_{1-6}$-Alkyl,
F,
Cl,
Br,
-C≡N und
$C_{1-4}$-Alkoxy,

$R^{23a}$ eine Difluormethoxygruppe ist,
$R^{24a}$ Wasserstoff oder Fluoratom oder eine Methoxygruppe ist,
Ac ausgewählt ist aus:

$C_{4-7}$-Cycloalkyl,
einem 4-, 5- oder 6-gliedrigen Heterocycloalkyl mit einem oder zwei Heteroatomen, unabhängig ausgewählt aus 0 und N, und
einem 5-12-gliedrigen Heteroaryl mit einem oder zwei Heteroatomen, worin zumindest ein Heteroatom Stickstoff und das zweite Heteroatom, falls vorhanden, ausgewählt ist aus N und S,

$R^{25a}$ und $R^{26a}$ unabhängig ausgewählt sind aus:

H,
$-OS(O)_2NH_2$,
$-S(O)_2CH_3$,
-OH,
-CN,
F,
Methyl-tetrazolyl,
Ethyl-tetrazolyl,
Cyclopropyl,
Cyclopropyl, substituiert mit einem oder zwei Substituenten, unabhängig ausgewählt aus: -OH und $-OCH_3$,
Morpholinyl,
Azetidinyl,
Azetidinyl, substituiert mit einem oder zwei Substituenten, unabhängig ausgewählt aus: Fluor, Chlor, Brom, -OH, $-CF_3$ und $-CH_3$,
Pyridinyl,
Pyridinyl, substituiert mit -C≡N,
Oxazolyl,
Oxazolyl, substituiert mit $-C(O)OCH_2CH_3$,
Oxazolyl, substituiert mit -C≡N,
-N(H)Oxazolyl,
-N(H)Oxazolyl, substituiert mit $-C(O)OCH_2CH_3$,
-N(H)Oxazolyl, substituiert mit -C≡N,
$-N(H)S(O)_2CH_3$,
Oxo,
$C_{1-8}$-Alkyl,
$C_{1-8}$-Alkyl, substituiert mit einem bis sechs Substituenten, unabhängig ausgewählt aus -OH, Oxo, Fluor, Chlor, Brom, Iod, $C_{1-4}$-Alkoxy, Cyclopropyl,

Cyclopentyl, Morpholinyl, Methylpiperazinyl,
$-NH_2$, $-N(H)C_{1-4}$-Alkyl, $-N(H)C_{1-4}$-Alkyl, worin Alkyl substituiert ist mit 1 bis 5 Fluor, $-N(C_{1-4}\text{-Alkyl})_2$ und $-N(C_{1-4}\text{-Alkyl})_2$, worin die Alkylgruppen unabhängig mit 1 bis 7 Fluor substituiert sind,
$C_{1-8}$-Alkoxy,
$C_{1-8}$-Alkoxy, substituiert mit einem bis sechs Substituenten, unabhängig ausgewählt aus: -OH, Oxo, Fluor, Chlor, Brom, Iod, $C_{1-4}$-Alkoxy,
Cyclopropyl, $-NH_2$, $-N(H)C_{1-4}$-Alkyl, $-N(H)C_{1-4}$-Alkyl, worin das Alkyl mit 1 bis 5 Fluor substituiert ist, $-N(C_{1-4}\text{-Alkyl})_2$, $-N(C_{1-4}\text{-Alkyl})_2$, worin die Alkylgruppen unabhängig mit 1 bis 7 Fluor substituiert sind,
$-S(O)_2CH_3)$, $-S(O_2)NH_2$ und $-S(O)_2N(H)C_{1-4}$-Alkyl Dimethylaminoxid,
N (H) $C_{1-6}$-Alkyl,
$N(H)C_{1-6}$-Alkyl, substituiert mit einem bis sechs Substituenten, unabhängig ausgewählt aus: -OH, Oxo, Fluor, Chlor, Brom, Iod und $-S(O)_2CH_3$,

oder ein pharmazeutisch akzeptables Salz davon.

**4.** Verbindung nach Anspruch 1, worin die Verbindung der Formel (XI) durch die folgende Formel (XIV) dargestellt ist:

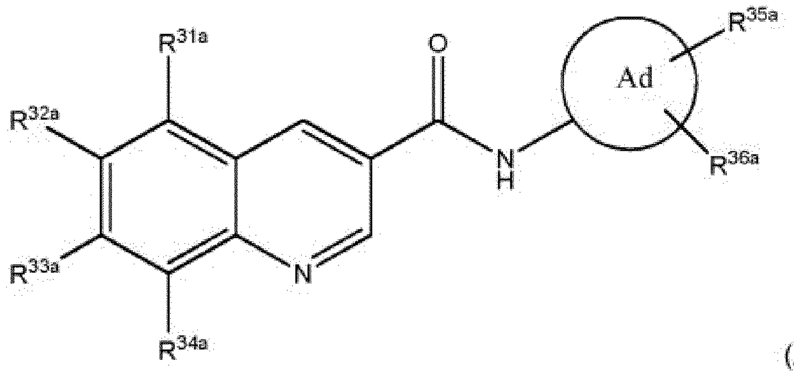

(XIV)

worin

$R^{31a}$ ausgewählt ist aus: H, F und $-OCH_3$,
$R^{32a}$ ausgewählt ist aus: H, -C≡N, F, Cl, Br, $-OCH_3$ und $-CH_3$,
$R^{13a}$ eine Difluormethoxygruppe ist,
$R^{14a}$ Wasserstoff oder Fluoratom oder eine Methoxygruppe ist, und

entweder:

Ad $C_{4-7}$-Cycloalkyl ist,
$R^{35a}$ ausgewählt ist aus:
H, Methyl, Ethyl, -C≡N, $-CH_2OCH_2C{\equiv}CH$, $-C(CH_3)_2OH$, $-CCH(CH_3)OH$, $-CH(CF_3)OH$, $-CH_2C(O)OCH_2CH_3$, $-CH_2NHCH(CH_3)CHF_2$, $-C(O)N(CH_3)_2$, $-C(O)OCH_3$, =0, $-OCH_3$, $-OCH_2C(O)NH_2$, $-CF_3$, $-CF_2$, $-OCH_2CH(CH_3)OH$, $-OCH_2C(CH_3)_2OH$, $-OCH_2C(CH_3)(CF_3)OH$, $-OCH_2CH(OH)CH(CH_3)_2$, $-OCH_2CH_2S(O)_2CH3$, $OCH_2C(O)NHCH_2C{\equiv}CH$, $-OCH_2C(O)NHCH_2CH_2C{\equiv}CH$, $-OCH_2C(CH_3(CF_3)OH$, $-O(CH_2)_3NH_2$, $-NHS(O)_2CH_3$, $-NHCH(CH_3)CF_3$, $-NHCH(CH_3)CHF_2$, $-NHCH(CF_3)CH_2OH$, $-NHC(CH_3)_2CF_3$, $-NHCH_2CF_3$, $-NHCH_2CHF_2$, $-OS(O)_2NH_2$, -C(O)Morpholinyl, C(O)Methylpiperazinyl, Dimethylaminoxid, Cyclopropanol, Cyclopropanolmethoxy, Morpholinyl, Azetidinyl, Azetidinyl, substituiert mit einer oder zwei Gruppen, unabhängig ausgewählt aus: Halogen, -OH und $-CF_3$, $-C(OH)R^7R^8$, worin $R^7$ H und $R^8$ ausgewählt ist aus: Cyclopropyl, $-CH_3$ und $-CF_3$ oder $R^7$ und $R^8$ zusammen binden, zur Bildung eines Cyclopropylrings, und $-NHR^9$, worin $R^9$ Oxazolyl ist, substituiert mit $-C(O)OCH_2CH_3$, und
$R^{36a}$ ausgewählt ist aus: H und -OH,
worin: zumindest ein Substituent, ausgewählt aus $R^{35a}$, und $R^{36a}$ nicht H ist, oder
Ad ein 4-, 5- oder 6-gliedriges Heterocycloalkyl mit einem oder zwei Heteroatomen ist, unabhängig ausgewählt aus 0 und N,
$R^{35a}$ ausgewählt ist: H, Methyl, $-CH_2CF_3$, $-CH_2CHF_2$, $-C(CH_3)_2OH$, $-CH(CH_3)OH$, $-CH(CF_3)OH$, $-C(O)N(CH_3)_2$, =O, $-S(O)_2CH_3$, $-OS(O)_2NH_2$, Ethyl-1H-tetrazolyl, Methyl-1H-tetrazolyl, Pyridinyl, Pyridinyl, substituiert mit -C≡N,

und Oxazolyl, substituiert mit $-C(O)OCH_2CH_3$ oder $-C\equiv N$ und
$R^{36a}$ ausgewählt ist aus: H, Methyl, Isopropyl, $-CH_2CH(CH_3)_2$, Cyclopropylmethylen, oder Cyclopentylmethylen, oder
Ad ein 5- bis 12-gliedriges Heteroaryl mit einem oder zwei Heteroatomen ist, worin zumindest ein Heteroatom Stickstoff und das zweite Heteroatom, falls vorhanden, ausgewählt ist aus N und S,
$R^{35a}$ ausgewählt ist aus: H, Methyl oder =O, und
$R^{36a}$ ausgewählt aus: H und Cyclopropyl,

oder ein pharmazeutisch akzeptables Salz davon.

5. Verbindung nach Anspruch 1 oder ein pharmazeutisch akzeptables Salz davon, worin $R^{1a}$ ein Wasserstoffatom ist.

6. Verbindung oder pharmazeutisch akzeptables Salz davon nach Anspruch 1 oder 2, worin $R^{2a}$ ein Wasserstoffatom oder ein Halogenatom ist.

7. Verbindung nach einem der Anspruch 1 bis 3 oder ein pharmazeutisch akzeptables Salz davon, worin $R^{2a}$ ein Fluoratom oder Chloratom ist.

8. Verbindung nach Anspruch 7 oder ein pharmazeutisch akzeptables Salz davon, worin $R^{4a}$ ein Wasserstoffatom ist.

9. Verbindung nach Anspruch 1, ausgewählt aus:

7-(Difluormethoxy)-N-(1-(1-methyl-1H-tetrazol-5-yl)piperidin-4-yl)chinolin-3-carboxamid,

(S)-7-(Difluormethoxy)-N-(2-oxopyrrolidin-3-yl)chinolin-3-carboxamid,
7-(Difluormethoxy)-N-((trans)-4-(dimethylcarbamoyl)-(cyclohexyl)chinolin-3-carboxamid,
7-(Difluormethoxy)-N-((trans)-4-(2-hydroxypropan-2-yl)cyclohexyl)chinolin-3-carboxamid,
7-(Difluormethoxy)-N-(1-(methylsulfonyl)piperidin-4-yl)chinolin-3-carboxamid,
7-(Difluormethoxy)-N-(3-methyl-1H-pyrazol-5-yl)chinolin-3-carboxamid,
7-(Difluormethoxy)-N-(2-oxo-1,2,3,4-tetrahydrochinolin-4-yl)chinolin-3-carboxamid,
7-(Difluormethoxy)-N-(thiazol-2-yl)chinolin-3-carboxamid,
7-(Difluormethoxy)-N-(trans-3-(2-hydroxypropan-2-yl)cyclobutyl)chinolin-3-carboxamid,
7-(Difluormethoxy)-N-(trans-4-(2-hydroxy-2-methylpropoxy)cyclohexyl)chinolin-3-carboxamid,
7-(Difluormethoxy)-N-(trans-4-(1-hydroxyethyl)-cyclohexyl)chinolin-3-carboxamid,
N-(trans-4-(Cyclopropyl(hydroxy)methyl)cyclohexyl)-7-(difluormethoxy)chinolin-3-carboxamid,
7-(Difluormethoxy)-N-(trans-4-(2,2,2-trifluor-1-hydroxyethyl)cyclohexyl)chinolin-3-carboxamid,
7-(Difluormethoxy)-N-(trans-4-(2-hydroxypropoxy)-cyclohexyl)chinolin-3-carboxamid,
7-(Difluormethoxy)-N-((3R,6S)-6-(2,2,2-trifluor-1-hydroxyethyl)tetrahydro-2H-pyran-3-yl)chinolin-3-carboxamid,
7-(Difluormethoxy)-N-(trans-4-(1-hydroxycyclo-propyl)cyclohexyl)chinolin-3-carboxamid,
7-(Difluormethoxy)-N-(trans-4-(3,3,3-trifluor-2-hydroxy-2-methylpropoxy)cyclohexyl)chinolin-3-carboxamid,
7-(Difluormethoxy)-N-(trans-4-((R)-2,2,2-trifluor-1-hydroxyethyl)cyclohexyl)chinolin-3-carboxamid,
7-(Difluormethoxy)-N-(trans-4-((S)-2,2,2-trifluor-1-hydroxyethyl)cyclohexyl)chinolin-3-carboxamid,
7-(Difluormethoxy)-N-((3s,6r)-6-(2-hydroxypropan-2-yl)tetrahydro-2H-pyran-3-yl)chinolin-3-carboxamid,
N-(trans-4-((3-Aminopropoxy)methyl)cyclohexyl)-(difluormethoxy)chinolin-3-carboxamid,
(S)-7-(Difluormethoxy)-6-fluor-N-(2-oxopyrrolidin-3-yl)chinolin-3-carboxamid,
N-(trans-4-(2,2-Difluorethyl)amino)cyclohexyl)-7-(difluormethoxy)-6-fluorchinolin-3-carboxamid,
(S)-7-(Difluormethoxy)-6-fluor-N-(1-isobutyl-2-oxopyrrolidin-3-yl)chinolin-3-carboxamid,
7-(Difluormethoxy)-6-fluor-N-(trans-4-(2-hydroxypropan-2-yl)cyclohexyl)chinolin-3-carboxamid,
7-(Difluormethoxy)-6-fluor-N-((1S,2R)-2-hydroxycyclopentyl)chinolin-3-carboxamid,
(S)-N-(1-(Cyclopropylmethyl)-2-oxopyrrolidin-3-yl)-7-(difluormethoxy)-6-fluorchinolin-3-carboxamid,
7-(Difluormethoxy)-6-fluor-N-(2-oxo-1,2,3,4-tetrahydrochinolin-4-yl)chinolin-3-carboxamid,
7-(Difluormethoxy)-6-fluor-N-(trans-4-methoxycyclohexyl)chinolin-3-carboxamid,
7-(Difluormethoxy)-6-fluor-N-(trans-4-(methylsulfonamido)cyclohexyl)chinolin-3-carboxamid,
7-(Difluormethoxy)-6-fluor-N-(trans-4-((1,1,1-trifluorpropan-2-yl)amino)cyclohexyl)chinolin-3-carboxamid,
7-(Difluormethoxy)-6-fluor-N-(1-(2,2,2-trifluorethyl)-piperidin-4-yl)chinolin-3-carboxamid,
7-(Difluormethoxy)-6-fluor-N-(trans-3-((2,2,2-trifluorethyl)amino)cyclobutyl)chinolin-3-carboxamid,

7-(Difluormethoxy)-6-fluor-N-(trans-4-(2-hydroxy-2-methylpropoxy)cyclohexyl)chinolin-3-carboxamid,
7-(Difluormethoxy)-6-fluor-N-(6-((2,2,2-trifluorethyl)amino)spiro[3.3]heptan-2-yl)chinolin-3-carboxamid,
7-(Difluormethoxy)-6-fluor-N-(trans-4-(((R)-1,1,1-trifluorpropan-2-yl)amino)cyclohexyl)chinolin-3-carboxamid,
7-(Difluormethoxy)-6-fluor-N-(trans-4-(((S)-1,1,1-trifluorpropan-2-yl)amino)cyclohexyl)chinolin-3-carboxamid,
7-(Difluormethoxy)-6-fluor-N-((1R,3R)-3-((2,2,2-trifluorethyl)amino)cyclopentyl)chinolin-3-carboxamid,
N-(1-(2,2Difluorethyl)piperidin-4-yl)-7-(difluormethoxy)-6-fluorchinolin-3-carboxamid,
7-(Difluormethoxy)-6-fluor-N-(trans-4-((1,1,1-trifluor-2-methylpropan-2-yl)amino)cyclohexyl)chinolin-3-carboxamid,
7-(Difluormethoxy)-N-(trans-4-((1,1-difluorpopran-2-yl)amino)cyclohexyl)-6-fluorchinolin-3-carboxamid,
7-(Difluormethoxy)-6-fluor-N-(4-morpholino-cyclohexyl)chinolin-3-carboxamid,
7-(Difluormethoxy)-(trans-4-(((R)-1,1-difluorpropan-2-yl)amino)cyclohexyl)-6-fluorchinolin-3-carboxamid,
7-(Difluormethoxy)-N-(trans-4-(((S)-1,1-difluorpropan-2-yl)amino)cyclohexyl)-6-fluorchinolin-3-carboxamid,
N-(trans-4-(3,3-Difluorazetidin-1-yl)cyclohexyl)-7-(difluormethoxy)-6-fluorchinolin-3-carboxamid,
Etyhl-2-(4-(7-(difluormethoxy)-6-fluorchinolin-3-carboxamido)piperidin-1-yl)oxazol-5-carboxylat,
7-(Difluormethoxy)-N-((1S,3r)-3-(((S)-1,1-difluorpropan-2-yl)amino)cyclobutyl)-6-fluorchinolin-3-carboxamid,
7-(Difluormethoxy)-N-((1R,3r)-3-(((R)-1,1-difluorpropan-2-yl)amino)cyclobutyl)6-fluorchinolin-3-carboxamid,
7-(Difluormethoxy)-N-(trans-4-(((1,1-difluorpropan-2-yl)amino)methyl)cyclohexyl)-6-fluorchinilin-3-carboxamid,
7-(Difluormethoxy)-6-fluor-N-(trans-4-(3,3,3-trifluor-2-hydroxy-2-methylpropoxy)cyclohexyl)chinolin-3-carboxamid,
7-(Difluormethoxy)-6-fluor-N-(trans-4-(((R)-1,1,1-trifluor-3-hydroxypropan-2-yl)amino)cyclohexyl)chinolin-3-carboxamid,
(S)-N-(1-(Cyclopentylmethyl)-2-oxopyrrolidin-3-yl)-7-(difluormethoxy)-6-fluorchinolin-3-carboxamid,
7-(Difluormethoxy)-6-fluor-N-(trans-4-(2-hydroxy-3-methylbutoxy)cyclohexyl)chinolin-3-carboxamid,
(S)-Ethyl-2-(3-(7-difluormethoxy)-6-fluorchinolin-3-carboxamido)pyrrolidin-1-yl)oxazol-5-carboxylat,
7-(Difluormethoxy)-N-((1r,4r)-4-ethyl-4-hydroxycyclohexyl)-6-fluorchinolin-3-carboxamid,
Ethyl-2-(3-(7-(difluormethoxy)-6-fluorchinolin-3-carboxamido)azetidin-1-yl)oxazol-4-carboxylat,
Ethyl-2-((trans-3-(7-(difluormethoxy)-6-fluorchinolin-3-carboxamido)cyclobutyl)amino)oxazol-5-carboxylat,
Ethyl-2-)3-(7-(difluormethoxy)-6-fluorchinolin-3-carboxamido)azetidin-1-yl)oxazol-5-carboxylat,
7-(Difluormethoxy)-6-fluor-N-(trans-4-(((S)-1,1,1-trifluor-3-hydroxypropan-2-yl)amino)cyclohexyl)chinolin-3-carboxamid,
trans-Methyl-4-(7-(difluormethoxy)-6-fluorchinolin-3-carboxamido)cyclohexancarboxylat,
N-(trans-4-Cyanocyclohexyl)-7-(difluormethoxy)-6-fluorchinolin-3-carboamid,
Ethyl-2-(trans-4-(7-(difluormethoxy)-6-fluorchinolin-3-carboxamido)cyclohexyl)acetat,
7-(Difluormethoxy)-6-fluor-N-(cis-4-(3-fluorazetidin-1-yl)cyclohexyl)chinolin-3-carboxamid,
7-(Difluormethoxy)-6-fluor-N-(trans-4-(3-fluorazetidin-1-yl)cyclohexyl)chinolin-3-carboxamid,
7-(Difluormethoxy)-6-fluor-N-(cis-4-(3-hydroxy-3-(trifluormethyl)azetidin-1-yl)cyclohexyl)chinolin-3-carboxamid,
7-(Difluormethoxy)-6-fluor-N-(trans-4-(3-hydroxy-3-(trifluormethyl)azetidin-1-yl)cyclohexyl)chinolin-3-carboxamid,
7-(Difluormethoxy)-6-fluor-N-((1s,3s)-3-hydroxy-3-methylyclobutyl)chinolin-3-carboxamid,
7-(Difluormethoxy)-6-fluor-N-(trans-4-(2-(methylsulfonyl)ethoxy)cyclohexyl)chinolin-3-carboxamid,
7-(Difluormethoxy)-6-fluor-N-(1-)piperidin-4-yl)chinolin-3-carboxamid,
7-(Difluormethoxy)-6-fluor-N-(1-(pyridin-3-yl)piperidin-4-yl)chinolin-3-carboxamid, N-(1-(5-Cyanooxazol-2-yl)piperidin-4-yl)-7-(difluormethoxy)-6-fluorchinolin-3-carboxamid,
N-(1-(5-Cyanopyridin-2-yl)azetidin-3-yl)-7-(difluormethoxy)-6-fluorchinolin-3-carboxamid,
7-(Difluormethoxy)-N-(trans-4-((((R)-1,1-difluorpropan-2-yl)amino)methyl)cyclohexyl)-6-fluorchinolin-3-carboxamid,
7-(Difluormethoxy)-N-(trans-4-((((S)-1,1-difluorproan-2-yl)amino)methyl)cyclohexyl)-6-fluorchinolin-3-carboxamid,
7-(Difluormethoxy)-6-fluor-N-(trans-4-((prop-2-in-1-yl)oxyl)methyl)cyclohexyl)chinolin-3-carboxamid,
7-(Difluormethoxy)-6-fluor-N-(trans-4-(2-oxo-2-)prop-2-in-1-ylamino)ethoxy)cyclohexyl)chinolin-3-carboxamid,
N-(trans-4-(2-(But-3-in-1-ylamino)-2-oxoethoxy)-cyclohexyl)-7-(difluormethoxy)-6-fluorchinolin-3-carboxamid,
(6-Chlor-7-(difluormethoxy)-N-(trans-4-(2-hydroxypropan-2-yl)cyclohexyl)chinolin-3-carboxamid,
(S)-6-Chlor-7-(difluormethoxy)-N-(2-oxopyrrolidin-3-yl)-chinolin-3-carboxamid,

6-Chlor-7-(difluormethoxy)-N-(trans-3-(2-hydroxypropan-2-yl)cyclobutyl)chinolin-3-carboxamid,

(S)-6-Chlor-7-(difluormethoxy)-N-(1-isopropyl-2-oxopyrrolidin-3-yl)quinolin-3-carboxamid,

(S)-6-Chlor-N-(1-(cyclopropylmethyl)-2-oxopyrrolidin-3-yl)-7-(difluormethoxy)quinolin-3-carboxamid,
6-Chlor-7-(difluormethoxy)-N-(trans-4-(1-hydroxycyclopropyl)cyclohexyl)chinolin-3-carboxamid,
6-Chlor-7-(difluormethoxy)-N-(trans-4-(2-hydroxy-2-methylpropoxy)cyclohexyl)chinolin-3-carboxamid,
6-Chlor-7-(difluormethoxy)-N-(trans-4-((1-hydroxycyclopropyl)methoxy)cyclohexyl)chinolin-3-carboxamid,
6-Chlor-7-(difluormethoxy)-N-((3r,6s)-6-(2-hydroxypropan-2-yl)tetrahydro-2H-pyran-3-yl)chinolin-3-carboxamid,
6-Chlor-7-(difluormethoxy)-N-(trans-4-((1,1-difluorpropan-2-yl)amino)cyclohexyl)chinolin-3-carboxamid,
6-Chlor-7-(difluormethoxy)-N-((3r,6s)-6-(dimethylcarbamoyl)tetrahydro-2H-pyran-3-yl)chinolin-3-carboxamid,
6-Chlor-7-(difluormethoxy)-N-(trans-4-(2-hydroxy-3-methylbutoxy)cyclohexyl)chinolin-3-carboxamid,
6-Chlor-N-(3-cyclopropyl-1-methyl-1H-pyrazol-5-yl)-7-(difluormethoxy)chinolin-3-carboxamid,
6-Chlor-7-(difluormethoxy)-N-(trans-4-(((R)-1,1-difluorpropan-2-yl)amino)cyclohexyl)chinolin-3-carboxamid,
6-Chlor-7-(difluormethoxy)-N-(trans-4-(((S)-1,1-difluorpropan-2-yl)amino)cyclohexyl)chinolin-3-carboxamid,
6-Chlor-7-(difluormethoxy)-N-(trans-4-(((S)-1,1,1-trifluor-3-hydroxypropan-2-yl)amino)cyclohexyl)chinolin-3-carboxamid,
6-Chlor-7-(difluormethoxy)-N-(trans-4-(3-fluorazetidin-1-yl)cyclohexyl)chinolin-3-carboxamid,
6-Chlor-7-(difluormethoxy)-N-((3S)-5-methyl-2-oxopyrrolidin-3-yl)chinolin-3-carboxamid,

6-Chlor-7-(difluormethoxy)-N-trans-4-(3-hydroxy-3-(trifluormethyl)azetidin-1-yl)cyclohexyl)chinolin-3-carboxamid,
6-Chlor-7-(difluormethoxy)-N-(trans-4-(((R)-1,1,1-trifluor-3-hydroxypropan-2-yl)amino)cyclohexyl)chinolin-3-carboxamid,
N-(trans-4-(2-Amino-2-oxoethoxy)cyclohexyl)-6-chlor-7-(difluormethoxy)chinolin-3-carboxamid,
6-Chlor-7-(difluormethoxy)-N-((1r,3r)-3-hydroxy-3-methylcyclobutyl)chinolin-3-carboxamid,
6-Chlor-7-(difluormethoxy)-N-((1s,3s)-3-hydroxy-3-methylcyclobutyl)chinolin-3-carboxamid,
6-Chlor-7-(difluormethoxy)-N-(trans-4-(2-(methylsulfonyl)-ethoxy)cyclohexyl)chinolin-3-carboxamid,
6-Chlor-7-(difluormethoxy)-N-(trans-3-hydroxycyclobutyl)chinolin-3-carboxamid,
trans-4-(6-Chlor-7-(difluormethoxy)chinolin-3-carboxamido)cyclohexylsulfamat,
6-Chlor-7-(difluormethoxy)-N-(3-oxocyclobutyl)-chinolin-3-carboxamid,
6-Chlor-7-(difluormethoxy)-N-((1r,4r)-4-hydroxy-4-methylcyclohexyl)chinolin-3-carboxamid,
6-Chlor-7-(difluormethoxy)-N-((3S,4R)-4-methyl-2-oxopyrrolidin-3-yl)chinolin-3-carboxamid,
6-Chlor-7-(difluormethoxy)-N-(3-hydroxy-3-methylcyclopentyl)chinolin-3-carboxamid,
6-Chlor-7-(difluormethoxy)-N-((1S,3S)-3-hydroxy-3-methylcyclopentyl)chinolin-3-carboxamid,
6-Chlor-7-(difluormethoxy)-N-((1s,4s)-4-hydroxy-4-methylcyclohexyl)chinolin-3-carboxamid,
6-Chlor-7-(difluormethoxy)-N-(tetrahydro-2H-pyran-4-yl)chinolin-3-carboxamid,
6-Chlor-7-(difluormethoxy)-N-(tetrahydro-2H-pyran-3-yl)chinolin-3-carboxamid,
6-Chlor-7-(difluormethoxy)-N-(2,2-dimethyl-tetrahydro-2H-pyran-4-yl)chinolin-3-carboxamid,

6-Brom-7-(difluormethoxy)-N-(trans-4-(2-hydroxypropan-2-yl)cyclohexyl)chinolin-3-carboxamid,
6-Brom-7-(difluormethoxy)-N-(trans-4-(((S)-1,1,1-trifluor-3-hydroxypropan-2-yl)amino)cyclohexyl)chinolin-3-carboxamid,
(S)-6-Brom-7-(difluormethoxy)-N-(2-oxopyrrolidin-3-yl)chinolin-3-carboxamid,
6-Brom-7-(difluormethoxy)-N-(trans-4-(((R)-1,1,1-trifluor-3-hydroxypropan-2 yl)amino)cyclohexyl)chinolin-3-carboxamid,
6-Brom-7-(difluormethoxy)-N-(trans-4-((1,1-difluorpropan-2-yl)amino)cyclohexyl)chinolin-3-carboxamid,
6-Brom-7-(difluormethoxy)-N-((1s,3s)-3-hydroxy-3-methylcyclobutyl)chinolin-3-carboxamid,
6-Brom-7-(difluormethoxy)-N-(trans-4-(((R)-1,1-difluorpropan-2-yl)amino)cyclohexyl)chinolin-3-carboxamid;
6-Brom-7-(difluormethoxy)-N-(trans-4-(((S)-1,1-difluorpropan-2-yl)amino)cyclohexyl)chinolin-3-carboxamid;
trans-4-(6-Chlor-7-(difluormethoxy)chinolin-3-carboxamido)-N,N-dimethylcyclohexanaminoxid,
6-Chlor-7-(difluormethoxy)-N-((trans)-4-(morpholin-4-carbonyl)cyclohexyl)chinolin-3-carboxamid,
6-Chlor-7-(difluormethoxy)-N-((trans)-4-(4-methylpiperazin-1-carbonyl)cyclohexyl)chinolin-3-carboxamid;
6-Chlor-7-(difluormethoxy)-N-((trans)-3-(3-hydroxy-3-(trifluormethyl)azetidin-1-yl)cyclobutyl)chinolin-3-carboxamid,
8-Chlor-7-(difluormethoxy)-N-((trans)-4-(2-hydroxypropan-2-yl)cyclohexyl)chinolin-3-carboxamid,
6,8-Dichlor-7-(difluormethoxy)-N-((trans)-4-(2-hydroxypropan-2-yl)cyclohexyl)chinolin-3-carboxamid,
6-Chlor-7-(difluormethoxy)-N-(1-(2-hydroxys-2-methylpropanoyl)piperidin-4-yl)chinolin-3-carboxamid,
6-Chlor-7-(difluormethoxy)-N-(1-(2-hydroxy-2-methylpropanoyl)azetidin-3-yl)chinolin-3-carbosamid,
6-Chlor-7-(difluormethoxy)-N-(cis)-4-hydroxy-4-(trifluormethyl)cyclohexyl)chinolin-3-carboxamid,
6-Chlor-7-(difluormethoxy)-N-(4-(2-hydroxy-2-methylpropanoyl)piperazin-1-yl)chinolin-3-carboxamid,
6-Chlor-7-difluormethoxy)-N-(trans-3-(2-hydroxy-2-methylpropanamido)cyclobutyl)chinolin-3-carboxamid,
6-Chlor-7-(difluormethoxy)-N-((trans)-4-hydroxy-4-(trifluormethyl)cyclohexyl)chinolin-3-carboxamid,
8-Chlor-7-(difluormethoxy)-N-((trans)-4-(2-hydroxypropan-2-yl)cyclohexyl)-6-methylchinolin-3-carboxamid,
6-Chlor-7-(difluormethoxy)-N-((trans)-4-(2-hydroxypropan-2-yl)cyclohexyl)-8-methylchinolin-3-carboxamid,
6-Chlor-7-(difluormethoxy)-N-((1s,3s)-3-hydroxy-3-(trifluormethyl)cyclobutyl)chinolin-3-carboxamid,
6-Chlor-7-(difluormethoxy)-N-((1s,4s)-4-(difluormethyl)-4-hydroxycyclohexyl)chinolin-3-carboxamid,
6-Chlor-7-(difluormethoxy)-N-((1r,4r)-4-(difluormethyl)-4-hydroxycyclohexyl)chinolin-3-carboxamid,
8-Chlor-7-(difluormethoxy)-6-fluor-N-(trans-4-(2-hydroxypropan-2-yl)cyclohexyl)chinolin-3-carboxamid,
6-Chlor-7-(difluormethoxy)-8-fluor-N-(trans-4-(2-hydroxypropan-2-yl)cyclohexyl)chinolin-3-carboxamid, und
6-Chlor-7-(difluormethoxy)-N-(2-oxo-2,3,4,5-tetrahydro-1H-benzo[b]azepin-3-yl)chinolin-3-carboxamid,

oder ein pharmazeutisch akzeptables Salz davon.

**10.** Verbindung nach Anspruch 1, die 6-Chlor-7-(difluormethoxy)-N-((1s,3s)-3-hydroxy-3-methylcyclobutyl)chinolin-3-carboxamid oder ein pharmazeutisch akzeptables Salz davon ist.

**11.** Verbindung der Formel (XI) oder ein pharmazeutisch akzeptables Salz davon nach einem der Ansprüche 1 bis 10 zur Verwendung in der Therapie.

**12.** Verbindung der Formel (XI) oder ein pharmazeutisch akzeptables Salz davon nach einem der Ansprüche 1 bis 10 zur Verwendung bei der Behandlung von Muskelschädigung, Muskellazerationen, Muskelschädigung aufgrund eines Knieersatzes, Asthma, chronisch obstruktiver Pulmonarerkrankung oder muskulärer Duchenne-Dystrophie.

## Revendications

**1.** Composé selon la formule (XI)

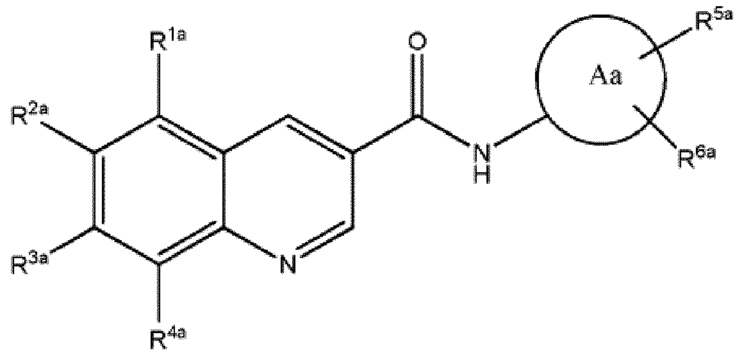

(XI)

dans laquelle :

$R^{1a}$ est sélectionné parmi :

H,
F,
Cl,
-OH,
$-OCH_3$, et
un alcoxy en $C_1$ substitué 1 à 3 fois avec un fluoro ;

$R^{2a}$ est sélectionné parmi :

H,
F,
Cl,
Br,
I,
-OH,
$-C(O)OC(CH_3)_3$,
-COOH,
un -C(O)alkyle en $C_1$ à $C_4$,
un -C(O)alkyle en $C_1$ à $C_4$ substitué avec 1 à 5 substituants sélectionnés indépendamment parmi : un fluoro, un chloro, un bromo, un oxo, -OH et -CN, un -Salkyle en $C_1$ à $C_4$,
un -Salkyle en $C_1$ à $C_4$, substitué avec 1 à 5 substituants sélectionnés indépendamment parmi : un fluoro, un chloro, un bromo, un oxo, -OH et -CN,
un -S(O)alkyle en $C_1$ à $C_4$,
un -S(O)alkyle en $C_1$ à $C_4$ substitué avec 1 à 5 substituants sélectionnés indépendamment parmi : un fluoro, un chloro, un bromo, un oxo, -OH et -CN,
$-N_3$,
-C≡N,
un alcoxy en $C_1$ à $C_4$,
un alcoxy en $C_1$ à $C_4$ substitué avec 1 à 5 substituants sélectionnés indépendamment parmi : un fluoro, un chloro, un bromo, un oxo, -OH et -CN,
un alkyle en $C_1$ à $C_6$,
un alkyle en $C_1$ à $C_6$ substitué avec 1 à 5 substituants sélectionnés indépendamment parmi : un fluoro, un chloro, un bromo, un iodo, un oxo, un alcoxy en $C_1$ à $C_4$, -OH, - COOH, $-NH_2$, un -N(H)alkyle en $C_1$ à $C_4$, un -N(alkyle en $C_1$ à $C_4)_2$ et -CN,
un cyclopropyle,
un cyclobutyle,
un 2,2-difluorocyclopropyle,
un pyrrolidinyle,
un azétidinyle, et
un azétidinyle substitué avec un ou deux substituants sélectionnés indépendamment parmi un halogène et un méthyle ;

$R^{3a}$ est un groupe difluorométhoxy ;
$R^{4a}$ est un atome d'hydrogène ou de fluor ou un groupe méthoxy ;
Aa est sélectionné parmi :

un cycloalkyle en $C_4$ à $C_7$,
un hétérocycloalkyle à 4, 5 ou 6 chaînons contenant un ou deux hétéroatomes sélectionnés indépendamment parmi O et N,
et
un hétéroaryle de 5 à 12 chaînons contenant un ou deux hétéroatomes, dans lequel au moins un hétéroatome est un azote et le second hétéroatome, s'il est présent, est sélectionné parmi N et S ;

$R^{5a}$ et $R^{6a}$ sont sélectionnés indépendamment parmi :

un hydrogène,
$-OS(O)_2NH_2$,
$-S(O)_2CH_3$,
-OH,
-C≡N,
F,
Cl,
Br,
I,
un tétrazolyle,
un méthyl-tétrazolyle,
un éthyl-tétrazolyle,
un cycloalkyle,
un cyclopropyle substitué avec un ou deux substituants sélectionnés indépendamment parmi ; -OH, $-OCH_3$, et $-CH_3$,
un morpholinyle,
un azétidinyle,
un azétidinyle substitué avec un ou deux substituants sélectionnés indépendamment parmi : un fluoro, un chloro, un bromo, un iodo, -OH, $-CF_3$, et $-CH_3$,
un pyridinyle,
un pyridinyle substitué avec -C≡N,
un oxazolyle,
un oxazolyle substitué avec $-C(O)OCH_2CH_3$,
un oxazolyle substitué avec -C≡N,
un -N(H)oxazolyle,
un -N(H)oxazolyle substitué avec $-C(O)OCH_2CH_3$,
un -N(H)oxazolyle substitué avec -C≡N,
$-N(H)S(O)_2CH_3$,
un oxo,
un alkyle en $C_1$ à $C_8$,
un alkyle en $C_1$ à $C_8$ substitué avec un à six substituants sélectionnés indépendamment parmi : -OH, un oxo, un fluoro, un chloro, un bromo, un iodo, un alcoxy en $C_1$ à $C_4$, un cycloalkyle, un morpholinyle, un méthylpipérazinyle, $-NH_2$, un -N(H)alkyle en $C_1$ à $C_4$, un -N(H)alkyle en $C_1$ à $C_4$ où l'alkyle est substitué avec 1 à 5 fluoro, un -N(alkyle en $C_1$ à $C_4)_2$, et un -N(alkyle en $C_1$ à $C_4)_2$ où les alkyles sont substitués indépendamment avec 1 à 7 fluoro,
un alcoxy en $C_1$ à $C_8$,
un alcoxy en $C_1$ à $C_8$ substitué avec un à six substituants sélectionnés indépendamment parmi : -OH, un oxo, un fluoro, un chloro, un bromo, un iodo, un alcoxy en $C_1$ à $C_4$, un cycloalkyle, $-NH_2$, un -N(H)alkyle en $C_1$ à $C_4$, un -N(H)alkyle en $C_1$ à $C_4$ où l'alkyle est substitué avec 1 à 5 fluoro, un -N(alkyle en $C_1$ à $C_4)_2$, un -N(alkyle en $C_1$ à $C_4)_2$ où les alkyles sont substitués indépendamment avec 1 à 7 fluoro, $-S(O)_2CH_3$, $-S(O)_2NH_2$, et un $-S(O)_2N(H)$alkyle en $C_1$ à $C_4$,
un oxyde de diméthylamine,
un N(alkyle en $C_1$ à $C_6)_2$, où chaque alkyle est facultativement substitué avec un à six substituants sélectionnés indépendamment parmi : -OH, un oxo, un fluoro, un chloro, un bromo, un iodo et $-S(O)_2CH_3$,
un N(H)alkyle en $C_1$ à $C_6$,

un N(H)alkyle en $C_1$ à $C_6$ substitué avec un à six substituants sélectionnés indépendamment parmi : -OH, un oxo, un fluoro, un chloro, un bromo, un iodo et $-S(O)_2CH_3$ ;
ou un sel pharmaceutiquement acceptable de celui-ci.

**2.** Composé selon la revendication 1, dans lequel le composé de formule (XI) est représenté par la formule (XII) suivante :

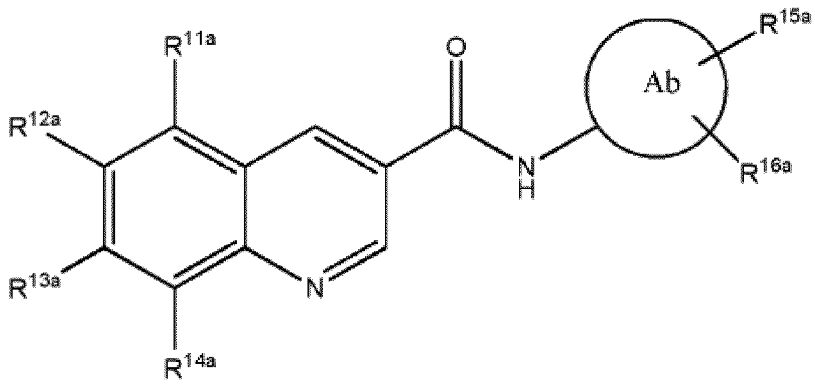

(XII)

dans laquelle :

$R^{11a}$ est sélectionné parmi :

H,
F,
Cl,
-OH, et
$-OCH_3$ ;

$R^{12a}$ est sélectionné parmi :

H,
un alkyle en $C_1$ à $C_6$,
F,
Cl,
Br,
-C≡N, et
un alcoxy en $C_1$ à $C_4$ ;
$R^{13a}$ est un groupe difluorométhoxy ;
$R^{14a}$ est un atome d'hydrogène ou de fluor ou un groupe méthoxy ;
Ab est sélectionné parmi :

un cycloalkyle en $C_4$ à $C_7$,
un hétérocycloalkyle à 4, 5 ou 6 chaînons contenant un ou deux hétéroatomes sélectionnés indépendamment parmi O et N,
et
un hétéroaryle de 5 à 12 chaînons contenant un ou deux hétéroatomes, dans lequel au moins un hétéroatome est un azote et le second hétéroatome, s'il est présent, est sélectionné parmi N et S ;
$R^{15a}$ et $R^{16a}$ sont sélectionnés indépendamment parmi :

H,
$-OS(O)_2NH_2$,
$-S(O)_2CH_3$,
-OH,
-CN,
F,

un tétrazolyle,
un méthyl-tétrazolyle,
un éthyl-tétrazolyle,
un cyclopropyle,
un cyclopropyle substitué avec un ou deux substituants sélectionnés indépendamment parmi ; -OH, $-OCH_3$, et $-CH_3$,
un morpholinyle,
un azétidinyle,
un azétidinyle substitué avec un ou deux substituants sélectionnés indépendamment parmi : un fluoro, un chloro, un bromo, un iodo, -OH, $-CF_3$, et $-CH_3$,
un pyridinyle,
un pyridinyle substitué avec -C≡N,
un oxazolyle,
un oxazolyle substitué avec $-C(O)OCH_2CH_3$,
un oxazolyle substitué avec -C≡N,
un -N(H)oxazolyle,
un -N(H)oxazolyle substitué avec $-C(O)OCH_2CH_3$,
un -N(H)oxazolyle substitué avec -C≡N,
$-N(H)S(O)_2CH_3$,
un oxo,
un alkyle en $C_1$ à $C_8$,
un alkyle en $C_1$ à $C_8$ substitué avec un à six substituants sélectionnés indépendamment parmi : -OH, un oxo, un fluoro, un chloro, un bromo, un iodo, un alcoxy en $C_1$ à $C_4$, un cyclopropyle, un cyclopentyle, un cyclobutyle, un morpholinyle, un méthylpipérazinyle, $-NH_2$, un -N(H)alkyle en $C_1$ à $C_4$, un -N(H)alkyle en $C_1$ à $C_4$ où l'alkyle est substitué avec 1 à 5 fluoro, un -N(alkyle en $C_1$ à $C_4)_2$, et un -N(alkyle en $C_1$ à $C_4)_2$ où les alkyles sont substitués indépendamment avec 1 à 7 fluoro,
un alcoxy en $C_1$ à $C_8$,
un alcoxy en $C_1$ à $C_8$ substitué avec un à six substituants sélectionnés indépendamment parmi : -OH, un oxo, un fluoro, un chloro, un bromo, un iodo, un alcoxy en $C_1$ à $C_4$, un cycloalkyle, $-NH_2$, un -N(H)alkyle en $C_1$ à $C_4$, un -N(H)alkyle en $C_1$ à $C_4$ où l'alkyle est substitué avec 1 à 5 fluoro, un -N(alkyle en $C_1$ à $C_4)_2$, un -N(alkyle en $C_1$ à $C_4)_2$ où les alkyles sont substitués indépendamment avec 1 à 7 fluoro, $-S(O)_2CH_3$, $-S(O)_2NH_2$,
et un $-S(O)_2N(H)$alkyle en $C_1$ à $C_4$,
un oxyde de diméthylamine,
un N(H)alkyle en $C_1$ à $C_6$,
un N(H)alkyle en $C_1$ à $C_6$ substitué avec un à six substituants sélectionnés indépendamment parmi : -OH, un oxo, un fluoro, un chloro, un bromo, un iodo et $-S(O)_2CH_3$ ;
ou un sel pharmaceutiquement acceptable de celui-ci.

**3.** Composé selon la revendication 1 ou la revendication 2, dans lequel le composé de formule (XI) est représenté par la formule (XIII) suivante :

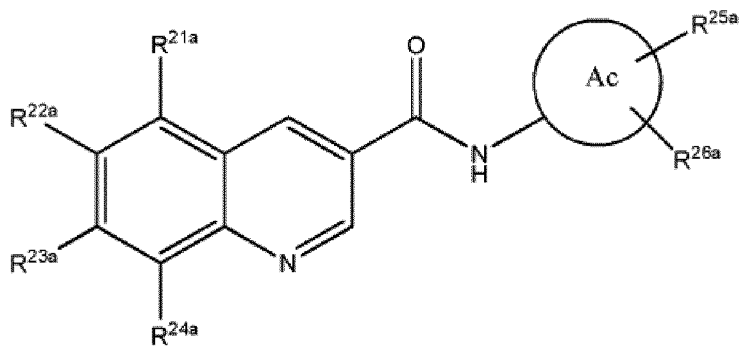

(XIII)

dans laquelle :

$R^{21a}$ est sélectionné parmi :

H,
F,
-OH, et
$-OCH_3$ ;

$R^{22a}$ est sélectionné parmi :

H,
un alkyle en $C_1$ à $C_6$,
F,
Cl,
Br,
-C≡N, et
un alcoxy en $C_1$ à $C_4$ ;
$R^{23a}$ est un groupe difluorométhoxy ;
$R^{24a}$ est un atome d'hydrogène ou de fluor ou un groupe méthoxy ;
Ac est sélectionné parmi :
un cycloalkyle en $C_4$ à $C_7$,
un hétérocycloalkyle à 4, 5 ou 6 chaînons contenant un ou deux hétéroatomes sélectionnés indépendamment parmi O et N,
et
un hétéroaryle de 5 à 12 chaînons contenant un ou deux hétéroatomes, dans lequel au moins un hétéroatome est un azote et le second hétéroatome, s'il est présent, est sélectionné parmi N et S ;

$R^{25a}$ et $R^{26a}$ sont sélectionnés indépendamment parmi :

H,
$-OS(O)_2NH_2$,
$-S(O)_2CH_3$,
-OH,
-CN,
F,
un méthyl-tétrazolyle,
un éthyl-tétrazolyle,
un cyclopropyle,
un cyclopropyle substitué avec un ou deux substituants sélectionnés indépendamment parmi : -OH et $-OCH_3$,
un morpholinyle,
un azétidinyle,
un azétidinyle substitué avec un ou deux substituants sélectionnés indépendamment parmi : un fluoro, un chloro, un bromo, -OH, $-CF_3$, et $-CH_3$,
un pyridinyle,
un pyridinyle substitué avec -C≡N,
un oxazolyle,
un oxazolyle substitué avec $-C(O)OCH_2CH_3$,
un oxazolyle substitué avec -C≡N,
un -N(H)oxazolyle,
un -N(H)oxazolyle substitué avec $-C(O)OCH_2CH_3$,
un -N(H)oxazolyle substitué avec -C≡N,
$-N(H)S(O)_2CH_3$,
un oxo,
un alkyle en $C_1$ à $C_8$,
un alkyle en $C_1$ à $C_8$ substitué avec un à six substituants sélectionnés indépendamment parmi : -OH, un oxo, un fluoro, un chloro, un bromo, un iodo, un alcoxy en $C_1$ à $C_4$, un cyclopropyle, un cyclopentyle, un morpholinyle, un méthylpipérazinyle, $-NH_2$, un -N(H)alkyle en $C_1$ à $C_4$, un -N(H)alkyle en $C_1$ à $C_4$ où l'alkyle est substitué avec 1 à 5 fluoro, un -N(alkyle en $C_1$ à $C_4$)$_2$, et un -N(alkyle en $C_1$ à $C_4$)$_2$ où les alkyles sont substitués indépendamment avec 1 à 7 fluoro,
un alcoxy en $C_1$ à $C_8$,

un alcoxy en $C_1$ à $C_8$ substitué avec un à six substituants sélectionnés indépendamment parmi : -OH, un oxo, un fluoro, un chloro, un bromo, un iodo, un alcoxy en $C_1$ à $C_4$, un cyclopropyle, $-NH_2$, un -N(H)alkyle en $C_1$ à $C_4$, un -N(H)alkyle en $C_1$ à $C_4$ où l'alkyle est substitué avec 1 à 5 fluoro, un -N(alkyle en $C_1$ à $C_4)_2$, un -N(alkyle en $C_1$ à $C_4)_2$ où les alkyles sont substitués indépendamment avec 1 à 7 fluoro, $-S(O)_2CH_3$, -$S(O)_2NH_2$, et un $-S(O)_2N(H)$alkyle en $C_1$ à $C_4$,
un oxyde de diméthylamine,
un N(H)alkyle en $C_1$ à $C_6$,
un N(H)alkyle en $C_1$ à $C_6$ substitué avec un à six substituants sélectionnés indépendamment parmi : -OH, un oxo, un fluoro, un chloro, un bromo, un iodo et $-S(O)_2CH_3$ ;
ou un sel pharmaceutiquement acceptable de celui-ci.

**4.** Composé selon la revendication 1, dans lequel le composé de formule (XI) est représenté par la formule (XIV) suivante :

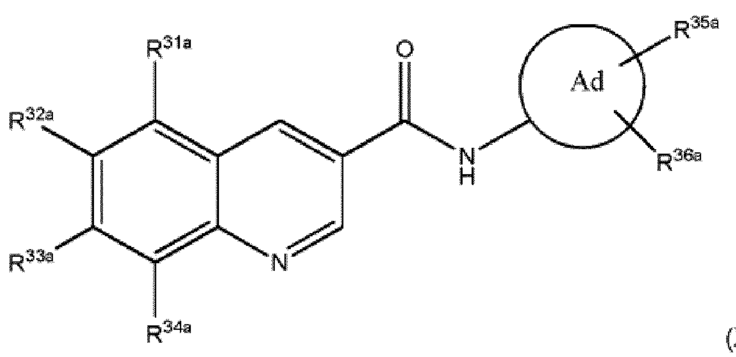

(XIV)

dans laquelle :

$R^{31a}$ est sélectionné parmi : H, F, et $-OCH_3$ ;
$R^{32a}$ est sélectionné parmi : H, -C≡N, F, Cl, Br, $-OCH_3$ et $-CH_3$ ;
$R^{13a}$ est un groupe difluorométhoxy ;
$R^{14a}$ est un atome d'hydrogène ou de fluor ou un groupe méthoxy ;
et
soit
Ad est un cycloalkyle en $C_4$ à $C_7$,
$R^{35a}$ est sélectionné parmi : H, un méthyle, un éthyle, -C≡N, $-CH_2OCH_2C≡CH$, $-C(CH_3)_2OH$, $-CH(CH_3)OH$, $-CH(CF_3)OH$, $-CH_2C(O)OCH_2CH_3$, $-CH_2NHCH(CH_3)CHF_2$, $-C(O)N(CH_3)_2$, $-C(O)OCH_3$, =O, $-OCH_3$, $-OCH_2C(O)NH_2$, $-CF_3$, $-CF_2$, $-OCH_2CH(CH_3)OH$, $-OCH_2C(CH_3)_2OH$, $-OCH_2C(CH_3)(CF_3)OH$, $-OCH_2CH(OH)CH(CH_3)_2$, $-OCH_2CH_2S(O)_2CH_3$, $-OCH_2C(O)NHCH_2C≡CH$, $-OCH_2C(O)NHCH_2CH_2C≡CH$, $-OCH_2C(CH_3)(CF_3)OH$, $-O(CH_2)_3NH_2$, $-NHS(O)_2CH_3$, $-NHCH(CH_3)CF_3$, $-NHCH(CH_3)CHF_2$, $-NHCH(CF_3)CH_2OH$, $-NHC(CH_3)_2CF_3$, $-NHCH_2CF_3$, $-NHCH_2CHF_2$, $-OS(O)_2NH_2$, un -C(O)morpholinyle, un -C(O)méthylpipérazinyle, un oxyde de diméthylamine, un cyclopropanol, un cyclopropanolméthoxy, un morpholinyle, un azétidinyle, un azétidinyle substitué avec un ou deux groupes sélectionnés indépendamment parmi : un halogène, -OH et $-CF_3$, $-C(OH)R^7R^8$ où $R^7$ est H et $R^8$ est sélectionné parmi : un cyclopropyle, $-CH_3$ et $-CF_3$, ou $R^7$ et $R^8$ se lie l'un à l'autre pour former un cycle cyclopropyle, et $-NHR^9$ où $R^9$ est un oxazolyle substitué avec $-C(O)OCH_2CH_3$, et
$R^{36a}$ est sélectionné parmi : H et -OH,
où :

au moins un substituant sélectionné parmi $R^{35a}$ et $R^{36a}$ n'est pas H ;
soit
Ad est un hétérocycloalkyle à 4, 5 ou 6 chaînons contenant un ou deux hétéroatomes sélectionnés indépendamment parmi O et N ;
$R^{35a}$ est sélectionné parmi : H, un méthyle, $-CH_2CF_3$, $-CH_2CHF_2$, $-C(CH_3)_2OH$, $-CH(CH_3)OH$, $-CH(CF_3)OH$, $-C(O)N(CH_3)_2$, =O, $-S(O)_2CH_3$, $-OS(O)_2NH_2$, un éthyl-1*H*-tétrazolyle, un méthyl-1*H*-tétrazolyle, un pyridinyle, un pyridinyle substitué avec -C≡N, et un oxazolyle substitué avec $-C(O)OCH_2CH_3$ ou -C≡N, et
$R^{36a}$ est sélectionné parmi : H, un méthyle, un isopropyle, $-CH_2CH(CH_3)_2$, un cyclopropylméthylène, ou un

cyclopentylméthylène ;
soit
Ad est un hétéroaryle de 5 à 12 chaînons contenant un ou deux hétéroatomes, dans lequel au moins un hétéroatome est un azote et le second hétéroatome, s'il est présent, est sélectionné parmi N et S,
$R^{35a}$ est sélectionné parmi : H, un méthyle ou =O, et
$R^{36a}$ est sélectionné parmi : H et un cyclopropyle ;
ou un sel pharmaceutiquement acceptable de celui-ci.

5. Composé selon la revendication 1 ou un sel pharmaceutiquement acceptable de celui-ci, dans lequel $R^{1a}$ représente un atome d'hydrogène.

6. Composé ou sel pharmaceutiquement acceptable de celui-ci selon la revendication 1 la revendication 2, dans lequel $R^{2a}$ représente un atome d'hydrogène ou un atome d'halogène.

7. Composé selon l'une quelconque des revendications 1 à 3 ou un sel pharmaceutiquement acceptable de celui-ci, dans lequel $R^{2a}$ représente un atome de fluor ou de chlore.

8. Composé selon la revendication 7 ou un sel pharmaceutiquement acceptable de celui-ci, dans lequel $R^{4a}$ représente un atome d'hydrogène.

9. Composé selon la revendication 1 sélectionné parmi :

le 7-(difluorométhoxy)-N-(1-(1-méthyl-1H-tétrazol-5-yl)pipéridin-4-yl)quinoléine-3-carboxamide ;
le (S)-7-(difluorométhoxy)-N-(2-oxopyrrolidin-3-yl)quinoléine-3-carboxamide ;
le 7-(difluorométhoxy)-N-((trans)-4-(diméthylcarbamoyl)cyclohexyl)quinoléine-3-carboxamide ;
le 7-(difluorométhoxy)-N-((trans)-4-(2-hydroxypropan-2-yl)cyclohexyl)quinoléine-3-carboxamide ;
le 7-(difluorométhoxy)-N-(1-(méthylsulfonyl)pipéridin-4-yl)quinoléine-3-carboxamide ;
le 7-(difluorométhoxy)-N-(3-méthyl-1H-pyrazol-5-yl)quinoléine-3-carboxamide ;
le 7-(difluorométhoxy)-N-(2-oxo-1,2,3,4-tétrahydroquinoléin-4-yl)quinoléine-3-carboxamide ;
le 7-(difluorométhoxy)-N-(thiazol-2-yl)quinoléine-3-carboxamide ;
le 7-(difluorométhoxy)-N-(trans-3-(2-hydroxypropan-2-yl)cyclobutyl)quinoléine-3-carboxamide ;
le 7-(difluorométhoxy)-N-(trans-4-(2-hydroxy-2-méthylpropoxy)cyclohexyl)-quinoléine-3-carboxamide ;
le 7-(difluorométhoxy)-N-(trans-4-(-1-hydroxyéthyl)cyclohexyl)quinoléine-3-carboxamide ;
le N-(trans-4-(cyclopropyl(hydroxy)méthyl)cyclohexyl)-7-(difluorométhoxy)-quinoléine-3-carboxamide ;
le 7-(difluorométhoxy)-N-(trans-4-(2,2,2-trifluoro-1-hydroxyéthyl)cyclohexyl)-quinoléine-3-carboxamide ;
le 7-(difluorométhoxy)-N-(trans-4-(2-hydroxypropoxy)cyclohexyl)quinoléine-3-carboxamide ;
le 7-(difluorométhoxy)-N-(3*R*,6*S*)-6-(2,2,2-trifluoro-1-hydroxyéthyl)tétrahydro-2H-pyran-3-yl)quinoléine-3-carboxamide ;
le 7-(difluorométhoxy)-N-(trans-4-(1-hydroxycyclopropyl)cyclohexyl)quinoléine-3-carboxamide ;
le 7-(difluorométhoxy)-N-(trans-4-(3,3,3-trifluoro-2-hydroxy-2-méthylpropoxy)cyclohexyl)quinoléine-3-carboxamide ;
le 7-(difluorométhoxy)-N-(trans-4-((*R*)-2,2,2-trifluoro-1-hydroxyéthyl)cyclohexyl)-quinoléine-3-carboxamide ;
le 7-(difluorométhoxy)-N-(trans-4-((*S*)-2,2,2-trifluoro-1-hydroxyéthyl)cyclohexyl)-quinoléine-3-carboxamide ;
le 7-(difluorométhoxy)-N-((3*s*,6*r*)-6-(2-hydroxypropan-2-yl)tétrahydro-2H-pyran-3-yl)quinoléine-3-carboxamide ;
le N-(trans-4-((3-aminopropoxy)méthyl)cyclohexyl)-7-(difluorométhoxy)quinoléine-3-carboxamide ;
le (S)-7-(difluorométhoxy)-6-fluoro-N-(2-oxopyrrolidin-3-yl)quinoléine-3-carboxamide ;
le N-(*trans*-4-((2,2-difluoroéthyl)amino)cyclohexyl)-7-(difluorométhoxy)-6-fluoro-quinoléine-3-carboxamide ;
le (S)-7-(difluorométhoxy)-6-fluoro-N-(1-isobutyl-2-oxopyrrolidin-3-yl)quinoléine-3-carboxamide ;
le 7-(difluorométhoxy)-6-fluoro-N-(trans-4-(2-hydroxypropan-2-yl)cyclohexyl)-quinoléine-3-carboxamide ;
le 7-(difluorométhoxy)-6-fluoro-N-((1S,2R)-2-hydroxycyclopentyl)quinoléine-3-carboxamide ;
le (S)-N-(1-(cyclopropylméthyl)-2-oxopyrrolidin-3-yl)-7-(difluorométhoxy)-6-fluoroquinoléine-3-carboxamide ;
le 7-(difluorométhoxy)-6-fluoro-N-(2-oxo-1,2,3,4-tétrahydroquinoléin-4-yl)-quinoléine-3-carboxamide ;
le 7-(difluorométhoxy)-6-fluoro-N-(trans-4-méthoxycyclohexyl)quinoléine-3-carboxamide ;
le 7-(difluorométhoxy)-6-fluoro-N-(trans-4-(méthylsulfonamido)cyclohexyl)-quinoléine-3-carboxamide ;
le 7-(difluorométhoxy)-6-fluoro-N-(*trans*-4-((1,1,1-trifluoropropan-2-yl)amino)-cyclohexyl)quinoléine-3-carboxamide ;
le 7-(difluorométhoxy)-6-fluoro-N-(1-(2,2,2-trifluoroéthyl)pipéridin-4-yl)quinoléine-3-carboxamide ;

le 7-(difluorométhoxy)-6-fluoro-N-(*trans*-3-((2,2,2-trifluoroéthyl)amino)cyclobutyl)-quinoléine-3-carboxamide ;
le 7-(difluorométhoxy)-6-fluoro-N-(trans-4-(2-hydroxy-2-méthylpropoxy)-cyclohexyl)quinoléine-3 -carboxamide ;
le 7-(difluorométhoxy)-6-fluoro-N-(6-((2,2,2-trifluoroéthyl)amino)spiro[3,3]heptan-2-yl)quinoléine-3-carboxamide ;
le 7-(difluorométhoxy)-6-fluoro-N-(trans-4-(((R)-1,1,1-trifluoropropan-2-yl)amino)-cyclohexyl)quinoléine-3-carboxamide ;
le 7-(difluorométhoxy)-6-fluoro-N-(trans-4-(((S)-1,1,1-trifluoropropan-2-yl)amino)-cyclohexyl)quinoléine-3-carboxamide ;
le 7-(difluorométhoxy)-6-fluoro-N-((1R,3R)-3-((2,2,2-trifluoroéthyl)amino)-cyclopentyl)quinoléine-3-carboxamide ;
le N-(1-(2,2-difluoroéthyl)pipéridin-4-yl)-7-(difluorométhoxy)-6-fluoroquinoléine-3-carboxamide ;
le 7-(difluorométhoxy)-6-fluoro-N-(*trans*-4-((1,1,1-trifluoro-2-méthylpropan-2-yl)-amino)cyclohexyl)quinoléine-3 -carboxamide ;
le 7-(difluorométhoxy)-N-(*trans*-4-((1,1-difluoropropan-2-yl)amino)cyclohexyl)-6-fluoroquinoléine-3-carboxamide ;
le 7-(difluorométhoxy)-6-fluoro-N-(4-morpholinocyclohexyl)quinoléine-3-carboxamide ;
le 7-(difluorométhoxy)-N-(trans-4-(((R)-1,1-difluoropropan-2-yl)amino)cyclohexyl)-6-fluoroquinoléine-3-carboxamide ;
le 7-(difluorométhoxy)-N-(trans-4-(((S)-1,1-difluoropropan-2-yl)amino)cyclohexyl)-6-fluoroquinoléine-3-carboxamide ;
le N-(*trans*-4-(3,3-difluoroazétidin-1-yl)cyclohexyl)-7-(difluorométhoxy)-6-fluoro-quinoléine-3-carboxamide ;
le 2-(4-(7-(difluorométhoxy)-6-fluoroquinoléine-3-carboxamido)pipéridin-1-yl)-oxazole-5-carboxylate d'éthyle ;
le 7-(difluorométhoxy)-N-((1S,3r)-3-(((S)-1,1-difluoropropan-2-yl)amino)-cyclobutyl)-6-fluoroquinoléine-3-carboxamide ;
le 7-(difluorométhoxy)-N-((1R,3r)-3-(((R)-1,1-difluoropropan-2-yl)amino)-cyclobutyl)-6-fluoroquinoléine-3-carboxamide ;
le 7-(difluorométhoxy)-N-(*trans*-4-(((1,1-difluoropropan-2-yl)amino)méthyl)-cyclohexyl)-6-fluoroquinoléine-3-carboxamide ;
le 7-(difluorométhoxy)-6-fluoro-N-(trans-4-(3,3,3-trifluoro-2-hydroxy-2-méthyl-propoxy)cyclohexyl)quinoléine-3-carboxamide ;
le 7-(difluorométhoxy)-6-fluoro-N-(*trans*-4-(((R)-1,1,1-trifluoro-3-hydroxypropan-2-yl)amino)cyclohexyl)quinoléine-3-carboxamide ;
le (S)-N-(1-(cyclopentylméthyl)-2-oxopyrrolidin-3-yl)-7-(difluorométhoxy)-6-fluoroquinoléine-3-carboxamide ;
le 7-(difluorométhoxy)-6-fluoro-N-(trans-4-(2-hydroxy-3-méthylbutoxy)cyclohexyl)-quinoléine-3-carboxamide ;
le (*S*)-2-(3-(7-(difluorométhoxy)-6-fluoroquinoléine-3-carboxamido)pyrrolidin-1-yl)-oxazole-5-carboxylate d'éthyle ;
le 7-(difluorométhoxy)-N-((1r,4r)-4-éthyl-4-hydroxycyclohexyl)-6-fluoroquinoléine-3-carboxamide ;
le 2-(3-(7-(difluorométhoxy)-6-fluoroquinoléine-3-carboxamido)azétidin-1-yl)-oxazole-4-carboxylate d'éthyle ;
le 2-((trans-3-(7-(difluorométhoxy)-6-fluoroquinoléine-3-carboxamido)cyclobutyl)-amino)oxazole-5-carboxylate d'éthyle ;
le 2-(3-(7-(difluorométhoxy)-6-fluoroquinoléine-3-carboxamido)azétidin-1-yl)-oxazole-5-carboxylate d'éthyle ;
le 7-(difluorométhoxy)-6-fluoro-N-(*trans*-4-(((S)-1,1,1-trifluoro-3-hydroxypropan-2-yl)amino)cyclohexyl)quinoléine-3-carboxamide
le *trans*-4-(7-(difluorométhoxy)-6-fluoroquinoléine-3-carboxamido)cyclohexane-carboxylate de méthyle ;
le N-(*trans*-4-cyanocyclohexyl)-7-(difluorométhoxy)-6-fluoroquinoléine-3-carboxamide ;
le 2-(*trans*-4-(7-(difluorométhoxy)-6-fluoroquinoléine-3-carboxamido)cyclohexyl)-acétate d'éthyle;
le 7-(difluorométhoxy)-6-fluoro-N-(*cis*-4-(3-fluoroazétidin-1-yl)cyclohexyl)-quinoléine-3-carboxamide ;
le 7-(difluorométhoxy)-6-fluoro-N-(*trans*-4-(3-fluoroazétidin-1-yl)cyclohexyl)-quinoléine-3-carboxamide ;
le 7-(difluorométhoxy)-6-fluoro-N-(*cis*-4-(3-hydroxy-3-(trifluorométhyl)azétidin-1-yl)cyclohexyl)quinoléine-3-carboxamide ;
le 7-(difluorométhoxy)-6-fluoro-N-(*trans*-4-(3-hydroxy-3-(trifluorométhyl)azétidin-1-yl)cyclohexyl)quinoléine-3-carboxamide ;
le 7-(difluorométhoxy)-6-fluoro-N-(1s,3s)-3-hydroxy-3-méthylcyclobutyl)-quinoléine-3-carboxamide ;
le 7-(difluorométhoxy)-6-fluoro-N-(trans-4-(2-(méthylsulfonyl)éthoxy)cyclohexyl)-quinoléine-3-carboxamide ;
le 7-(difluorométhoxy)-6-fluoro-N-(1-(pyridin-2-yl)pipéridin-4-yl)quinoléine-3-carboxamide ;
le 7-(difluorométhoxy)-6-fluoro-N-(1-(pyridin-3-yl)pipéridin-4-yl)quinoléine-3-carboxamide ;
le N-(1-(5-cyanooxazol-2-yl)pipéridin-4-yl)-7-(difluorométhoxy)-6-fluoro-quinoléine-3-carboxamide ;

le N-(1-(5-cyanopyridin-2-yl)azétidin-3-yl)-7-(difluorométhoxy)-6-fluoroquinoléine-3-carboxamide ;
le 7-(difluorométhoxy)-N-(*trans*-4-((((R)-1,1-difluoropropan-2-yl)amino)méthyl)-cyclohexyl)-6-fluoroquinoléine-3-carboxamide ;
le 7-(difluorométhoxy)-N-(*trans*-4-((((S)-1,1-difluoropropan-2-yl)amino)méthyl)-cyclohexyl)-6-fluoroquinoléine-3-carboxamide ;
le 7-(difluorométhoxy)-6-fluoro-N-(trans-4-((prop-2-yn-1-yloxy)méthyl)cyclohexyl)-quinoléine-3-carboxamide ;
le 7-(difluorométhoxy)-6-fluoro-N-(trans-4-(2-oxo-2-(prop-2-yn-1-ylamino)éthoxy)-cyclohexyl)quinoléine-3-carboxamide ;
le N-(trans-4-(2-(but-3-yn-1-ylamino)-2-oxoéthoxy)cyclohexyl)-7-(difluorométhoxy)-6-fluoroquinoléine-3-carboxamide ;
le (6-chloro-7-(difluorométhoxy)-N-(trans-4-(2-hydroxypropan-2-yl)cyclohexyl)-quinoléine-3-carboxamide ;
le (S)-6-chloro-7-(difluorométhoxy)-N-(2-oxopyrrolidin-3-yl)quinoléine-3-carboxamide ;
le 6-chloro-7-(difluorométhoxy)-N-(trans-3-(2-hydroxypropan-2-yl)cyclobutyl)-quinoléine-3-carboxamide ;
le (S)-6-chloro-7-(difluorométhoxy)-N-(1-isopropyl-2-oxopyrrolidin-3-yl)-quinoléine-3-carboxamide ;
le (S)-6-chloro-N-(1-(cyclopropylméthyl)-2-oxopyrrolidin-3-yl)-7-(difluorométhoxy)quinoléine-3-carboxamide ;
le 6-chloro-7-(difluorométhoxy)-N-(trans-4-(1-hydroxycyclopropyl)cyclohexyl)-quinoléine-3-carboxamide ;
le 6-chloro-7-(difluorométhoxy)-N-(trans-4-(2-hydroxy-2-méthylpropoxy)-cyclohexyl)quinoléine-3-carboxamide ;
le 6-chloro-7-(difluorométhoxy)-N-(trans-4-((1-hydroxycyclopropyl)méthoxy)-cyclohexyl)quinoléine-3-carboxamide ;
le 6-chloro-7-(difluorométhoxy)-N-((3*r*,6*s*)-6-(2-hydroxypropan-2-yl)tétrahydro-2H-pyran-3-yl)quinoléine-3-carboxamide ;
le 6-chloro-7-(difluorométhoxy)-N-(*trans*-4-((1,1-difluoropropan-2-yl)amino)-cyclohexyl)quinoléine-3-carboxamide ;
le 6-chloro-7-(difluorométhoxy)-N-((3*r*,6*s*)-6-(diméthylcarbamoyl)tétrahydro-2H-pyran-3-yl)quinoléine-3-carboxamide ;
le 6-chloro-7-(difluorométhoxy)-N-(trans-4-(2-hydroxy-3-méthylbutoxy)-cyclohexyl)quinoléine-3-carboxamide ;
le 6-chloro-N-(3-cyclopropyl-1-méthyl-1H-pyrazol-5-yl)-7-(difluorométhoxy)-quinoléine-3-carboxamide ;
le 6-chloro-7-(difluorométhoxy)-N-(*trans*-4-(((R)-1,1-difluoropropan-2-yl)amino)-cyclohexyl)quinoléine-3-carboxamide ;
le 6-chloro-7-(difluorométhoxy)-N-(*trans*-4-(((S)-1,1-difluoropropan-2-yl)amino)-cyclohexyl)quinoléine-3-carboxamide ;
le 6-chloro-7-(difluorométhoxy)-N-(*trans*-4-(((S)-1,1,1-trifluoro-3-hydroxypropan-2-yl)amino)cyclohexyl)quinoléine-3-carboxamide ;
le 6-chloro-7-(difluorométhoxy)-N-(*trans*-4-(3-fluoroazétidin-1-yl)cyclohexyl)-quinoléine-3-carboxamide ;
le 6-chloro-7-(difluorométhoxy)-N-((3S)-5-méthyl-2-oxopyrrolidin-3-yl)quinoléine-3-carboxamide ;
le 6-chloro-7-(difluorométhoxy)-N-(*trans*-4-(3-hydroxy-3-(trifluorométhyl)azétidin-1-yl)cyclohexyl)quinoléine-3-carboxamide ;
le 6-chloro-7-(difluorométhoxy)-N-(*trans*-4-(((R)-1,1,1-trifluoro-3-hydroxypropan-2-yl)amino)cyclohexyl)quinoléine-3-carboxamide ;
le N-(trans-4-(2-amino-2-oxoéthoxy)cyclohexyl)-6-chloro-7-(difluorométhoxy)-quinoléine-3-carboxamide ;
le 6-chloro-7-(difluorométhoxy)-N-((lr,3r)-3-hydroxy-3-méthylcyclobutyl)-quinoléine-3-carboxamide ;
le 6-chloro-7-(difluorométhoxy)-N-((ls,3s)-3-hydroxy-3-méthylcyclobutyl)-quinoléine-3-carboxamide ;
le 6-chloro-7-(difluorométhoxy)-N-(trans-4-(2-(méthylsulfonyl)éthoxy)cyclohexyl)-quinoléine-3-carboxamide ;
le 6-chloro-7-(difluorométhoxy)-N-(*trans*-3-hydroxycyclobutyl)quinoléine-3-carboxamide ;
le sulfamate de trans-4-(6-chloro-7-(difluorométhoxy)quinoléine-3-carboxamido)-cyclohexyle ;
le 6-chloro-7-(difluorométhoxy)-N-(3-oxocyclobutyl)quinoléine-3-carboxamide ;
le 6-chloro-7-(difluorométhoxy)-N-((lr,4r)-4-hydroxy-4-méthylcyclohexyl)-quinoléine-3-carboxamide ;
le 6-chloro-7-(difluorométhoxy)-N-((3S,4R)-4-méthyl-2-oxopyrrolidin-3-yl)-quinoléine-3-carboxamide ;
le 6-chloro-7-(difluorométhoxy)-N-(3-hydroxy-3-méthylcyclopentyl)quinoléine-3-carboxamide ;
le 6-chloro-7-(difluorométhoxy)-N-((1S,3S)-3-hydroxy-3-méthylcyclopentyl)-quinoléine-3-carboxamide ;
le 6-chloro-7-(difluorométhoxy)-N-((1s,4s)-4-hydroxy-4-méthylcyclohexyl)-quinoléine-3-carboxamide ;
le 6-chloro-7-(difluorométhoxy)-N-(tétrahydro-2H-pyran-4-yl)quinoléine-3-carboxamide ;
le 6-chloro-7-(difluorométhoxy)-N-(tétrahydro-2H-pyran-3-yl)quinoléine-3-carboxamide ;
le 6-chloro-7-(difluorométhoxy)-N-(2,2-diméthyltétrahydro-2H-pyran-4-yl)-quinoléine-3-carboxamide ;
le 6-bromo-7-(difluorométhoxy)-N-(trans-4-(2-hydroxypropan-2-yl)cyclohexyl)-quinoléine-3-carboxamide ;
le 6-bromo-7-(difluorométhoxy)-N-(*trans*-4-(((S)-1,1,1-trifluoro-3-hydroxypropan-2-yl)amino)cyclohexyl)quinoléine-3-carboxamide ;

le (S)-6-bromo-7-(difluorométhoxy)-N-(2-oxopyrrolidin-3-yl)quinoléine-3-carboxamide ;
le 6-bromo-7-(difluorométhoxy)-N-(*trans*-4-(((R)-1,1,1-trifluoro-3-hydroxypropan-2-yl)amino)cyclohexyl)quinoléine-3-carboxamide ;
le 6-bromo-7-(difluorométhoxy)-N-(*trans*-4-((1,1-difluoropropan-2-yl)amino)-cyclohexyl)quinoléine-3-carboxamide ;
le 6-bromo-7-(difluorométhoxy)-N-((ls,3s)-3-hydroxy-3-méthylcyclobutyl)-quinoléine-3-carboxamide ;
le 6-bromo-7-(difluorométhoxy)-N-(*trans*-4-(((R)-1,1-difluoropropan-2-yl)amino)-cyclohexyl)quinoléine-3-carboxamide ;
le 6-bromo-7-(difluorométhoxy)-N-(*trans*-4-(((S)-1,1-difluoropropan-2-yl)amino)-cyclohexyl)quinoléine-3-carboxamide ;
l'oxyde de trans-4-(6-chloro-7-(difluorométhoxy)quinoléine-3-carboxamido)-N,N-diméthylcyclohexanamine ;
le 6-chloro-7-(difluorométhoxy)-N-((trans)-4-(morpholine-4-carbonyl)cyclohexyl)-quinoléine-3-carboxamide ;
le 6-chloro-7-(difluorométhoxy)-N-((trans)-4-(4-méthylpipérazine-1-carbonyl)-cyclohexyl)quinoléine-3-carboxamide ;
le 6-chloro-7-(difluorométhoxy)-N-((trans)-3-(3-hydroxy-3-(trifluorométhyl)-azétidin-1-yl)cyclobutyl)quinoléine-3-carboxamide ;
le 8-chloro-7-(difluorométhoxy)-N-((trans)-4-(2-hydroxypropan-2-yl)cyclohexyl)-quinoléine-3-carboxamide ;
le 6,8-dichloro-7-(difluorométhoxy)-N-((*trans*)-4-(2-hydroxypropan-2-yl)-cyclohexyl)quinoléine-3-carboxamide ;
le 6-chloro-7-(difluorométhoxy)-N-(1-(2-hydroxy-2-méthylpropanoyl)pipéridin-4-yl)quinoléine-3-carboxamide ;
le 6-chloro-7-(difluorométhoxy)-N-(1-(2-hydroxy-2-méthylpropanoyl)azétidin-3-yl)quinoléine-3-carboxamide ;
le 6-chloro-7-(difluorométhoxy)-N-((cis)-4-hydroxy-4-(trifluorométhyl)cyclohexyl)-quinoléine-3-carboxamide ;
le 6-chloro-7-(difluorométhoxy)-N-(4-(2-hydroxy-2-méthylpropanoyl)pipérazin-1-yl)quinoléine-3-carboxamide ;
le 6-chloro-7-(difluorométhoxy)-N-(trans-3-(2-hydroxy-2-méthylpropanamido)-cyclobutyl)quinoléine-3-carboxamide ;
le 6-chloro-7-(difluorométhoxy)-N-((trans)-4-hydroxy-4-(trifluorométhyl)-cyclohexyl)quinoléine-3-carboxamide ;
le 8-chloro-7-(difluorométhoxy)-N-((*trans*)-4-(2-hydroxypropan-2-yl)cyclohexyl)-6-méthylquinoléine-3-carboxamide ;
le 6-chloro-7-(difluorométhoxy)-N-((*trans*)-4-(2-hydroxypropan-2-yl)cyclohexyl)-8-méthylquinoléine-3-carboxamide ;
le 6-chloro-7-(difluorométhoxy)-N-((ls,3s)-3-hydroxy-3-(trifluorométhyl)-cyclobutyl)quinoléine-3-carboxamide ;
le 6-chloro-7-(difluorométhoxy)-N-((1s,4s)-4-(difluorométhyl)-4-hydroxy-cyclohexyl)quinoléine-3-carboxamide ;
le 6-chloro-7-(difluorométhoxy)-N-((lr,4r)-4-(difluorométhyl)-4-hydroxy-cyclohexyl)quinoléine-3-carboxamide ;
le 8-chloro-7-(difluorométhoxy)-6-fluoro-N-(trans-4-(2-hydroxypropan-2-yl)-cyclohexyl)quinoléine-3-carboxamide ;
le 6-chloro-7-(difluorométhoxy)-8-fluoro-N-(trans-4-(2-hydroxypropan-2-yl)-cyclohexyl)quinoléine-3-carboxamide ; et
le 6-chloro-7-(difluorométhoxy)-N-(2-oxo-2,3,4,5-tétrahydro-1H-benzo[b]azépin-3-yl)quinoléine-3-carboxamide ;
ou un sel pharmaceutiquement acceptable de ceux-ci.

**10.** Composé selon la revendication 1, qui est le 6-chloro-7-(difluorométhoxy)-N-(1s,3s)-3-hydroxy-3-méthylcyclobutyl)quinoléine-3-carboxamide ou un sel pharmaceutiquement acceptable de celui-ci.

**11.** Composé de formule (XI) ou un sel pharmaceutiquement acceptable de celui-ci selon l'une quelconque des revendications 1 à 10 pour une utilisation en thérapie.

**12.** Composé de formule (XI) ou un sel pharmaceutiquement acceptable de celui-ci selon l'une quelconque des revendications 1 à 10 pour une utilisation dans le traitement d'une lésion musculaire, des déchirures musculaires, d'un dommage musculaire dû à une arthroplastie du genou, de l'asthme, d'une bronchopneumopathie chronique obstructive ou de la myopathie de Duchenne.

# Figure 1

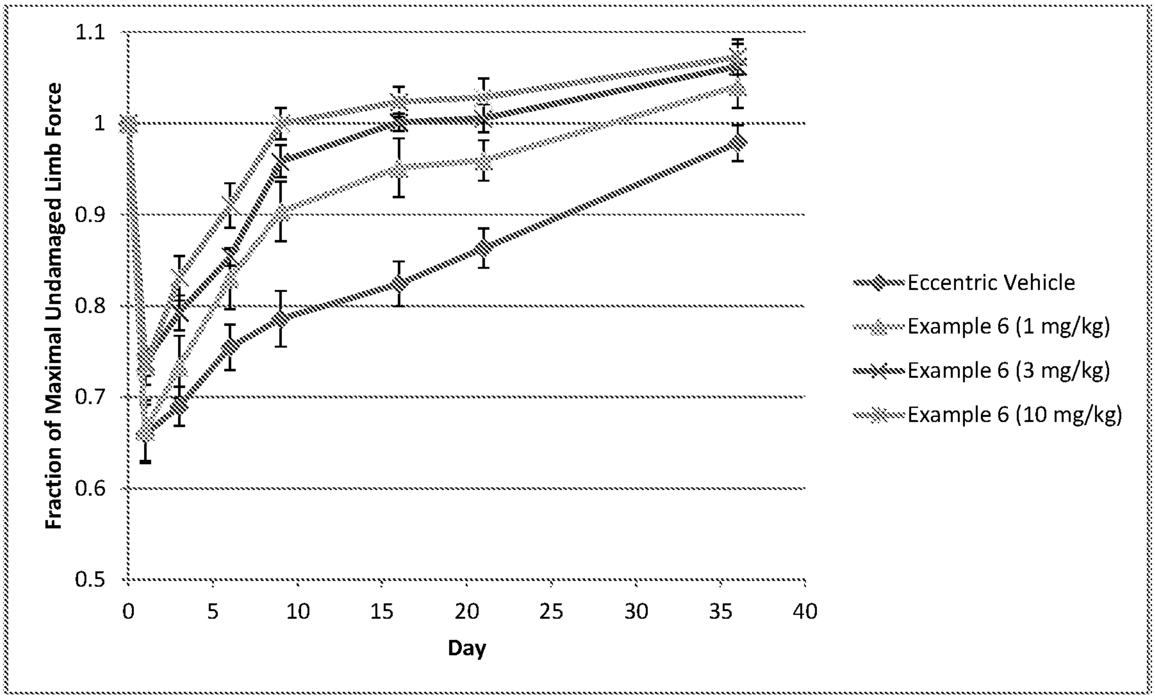
Fraction of Maximal Undamaged Limb Force
1.1
1
0.9
0.8
0.7
0.6
0.5
0
5
10
15
20
25
30
35
40
Day
Eccentric Vehicle
Example 6 (1 mg/kg)
Example 6 (3 mg/kg)
Example 6 (10 mg/kg)

# Figure 2

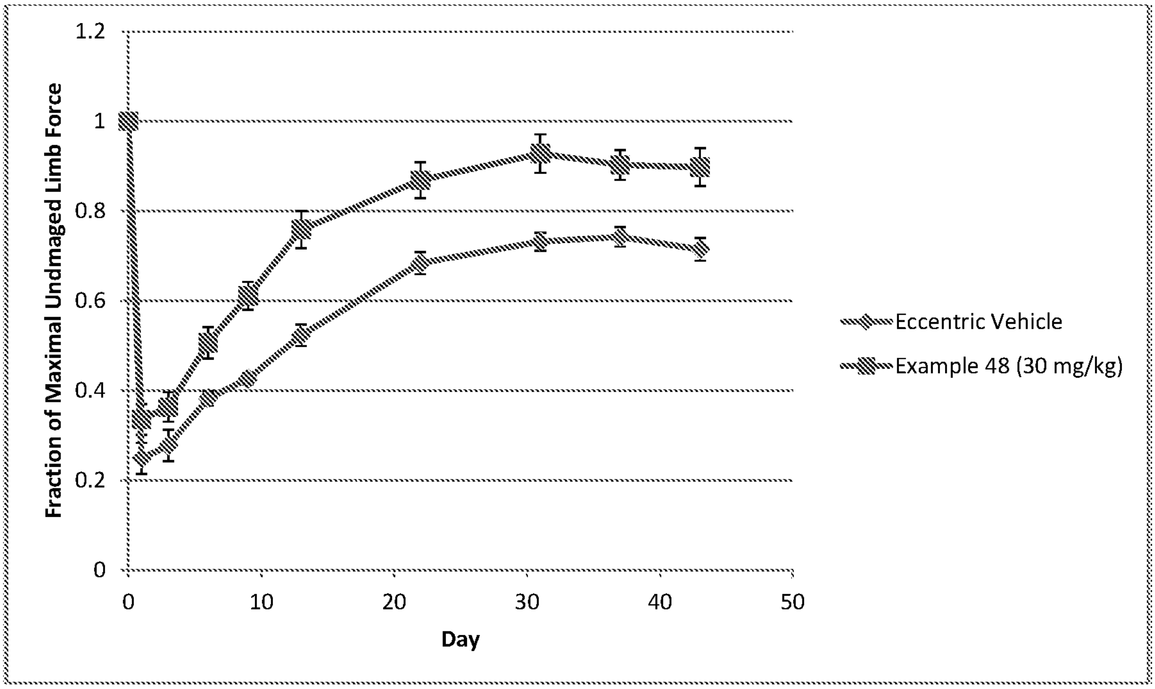
1.2
1
0.8
0.6
0.4
0.2
0
Fraction of Maximal Undmaged Limb Force
0
10
20
30
40
50
Day
Eccentric Vehicle
Example 48 (30 mg/kg)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005094805 A
- WO 2007007778 A
- WO 2007041634 A
- WO 2008121670 A
- WO 2008122787 A
- WO 2009153720 A
- WO 2009153721 A
- WO 2010033977 A
- WO 2010104024 A
- WO 2011043359 A
- WO 2011044307 A
- WO 2011090062 A
- JP 2007051121 A
- US 20080146569 A
- US 6576265 B
- WO 9421229 A
- WO 9834596 A
- WO 2010027097 A
- WO 2011156610 A


### Non-patent literature cited in the description


- **LEWIS et al.** Prostaglandin D2 generation after activation of rat and human mast cells with anti-IgE. *J Immunol,* 1982, vol. 129, 1627-1631
- **BOIE et al.** Molecular cloning and characterization of the human prostanoid DP receptor. *J Biol Chem,* 1995, vol. 270, 18910-18916
- **ABE et al.** Molecular cloning, chromosome mapping and characterization of the mouse CRTH2 gene, a putative member of the leukocyte chemo-attractant receptor family. *Gene,* 1999, vol. 227, 71-77
- **URADE et al.** Prostaglandin D synthase structure and function. *Vitamins and hormones,* 2000, vol. 58, 89-120
- **NAKAGAWA et al.** A prostaglandin D2 metabolite is elevated in the urine of Duchenne muscular dystrophy subjects and increases further from 8 years old. *Clinica Chimica Acta,* 2013, vol. 423, 10-14
- **MOHRI et al.** Inhibition of prostaglandin D synthase suppresses muscular necrosis. *Am J Pathol,* 2009, vol. 174, 1735-1744
- **OKINAGA et al.** Induction of hematopoietic prostaglandin D synthase in hyalinated necrotic muscle fibers: its implication in grouped necrosis. *Acta Neuropathologica,* 2002, vol. 104, 377-84
- **REDENSEK et al.** Expression and detrimental role of hematopoietic prostaglandin D synthase in spinal cord contusion injury. *Glia,* 2011, vol. 59, 603-614
- **MOHRI et al.** Prostaglandin D2-mediated microglia/astrocyte interaction enhances astrogliosis and demyelination in twitcher. *J Neurosci,* 2006, vol. 26, 4383-4393
- **IKUKO et al.** Hematopoietic prostaglandin D synthase and DP1 receptor are selectively upregulated in microglia and astrocytes within senile plaques from human subjects and in a mouse model of Alzheimer disease. *J Neuropath Exp Neur,* 2007, vol. 66, 469-480
- **TANAKA et al.** Mast cells function as an alternative modulator of adipogenesis through 15-deoxy-delta-12, 14-prostaglandin J. *Am J Physiol Cell Physiol,* 2011, vol. 301, C1360-C1367
- **PAPALIODIS et al.** Niacin-induced "flush" involves release of prostaglandin D2 from mast cells and serotonin from platelets: Evidence from human cells in vitro and an animal model. *JPET,* 2008, vol. 327, 665-672
- **WEBER et al.** Identification and characterisation of new inhibitors for the human hematopoietic prostaglandin D2 synthase. *European Journal of Medicinal Chemistry,* 2010, vol. 45, 447-454
- **CARRON et al.** Discovery of an Oral Potent Selective Inhibitor of Hematopoietic Prostaglandin D Synthase (H-PGDS). *ACS Med Chem Lett.,* 2010, vol. 1, 59-63
- **CHRIST et al.** Development and Characterization of New Inhibitors of the Human and Mouse Hematopoietic Prostaglandin D2 Synthases. *J Med Chem,* 2010, vol. 53, 5536-5548
- **HOHWY et al.** Novel Prostaglandin D Synthase Inhibitors Generated by Fragment-Based Drug Design. *J Med Chem,* 2008, vol. 51, 2178-2186
- **T. GREENE ; P. WUTS.** Protecting Groups in Organic Synthesis. John Wiley & Sons, 2006
- **TURNER ; BADYLACK.** *Cell Tissue Res.,* 2012, vol. 347 (3), 759-74
- *Organic Letters,* 2010, vol. 12 (10), 2322-2325

- *Bioogranic and Medicinal Chemistry,* 2006, vol. 14, 3953